# EUROPEAN PATENT SPECIFICATION

(11) **EP 2 344 672 B1**
(45) Date of publication and mention of the grant of the patent: **12.11.2014**
(21) Application number: 09816583.0
(22) Date of filing: 25.09.2009
(51) Int. Cl.: C12Q 1/68, C12N 15/11

(54) **GENETIC MARKERS FOR OPTIMIZING TREATMENT FOR SCHIZOPHRENIA**
GENETISCHE MARKER ZUR OPTIMIERUNG EINER SCHIZOPHRENIEBEHANDLUNG
MARQUEURS GÉNÉTIQUES POUR OPTIMISER LE TRAITEMENT DE LA SCHIZOPHRÉNIE

(30) Priority: 25.09.2008 US 100176 P
(43) Date of publication of application: 20.07.2011
(73) Proprietor: Suregene Llc, Louisville, KY 40204 (US)
(72) Inventor: BRENNAN, Mark, David, Jeffersonville, Indiana 47130 (US); RAMSEY, Timothy, Lynn, Shelbyville, KY 40065 (US)
(74) Representative: Coehn, Markus
(86) International application number: PCT/US2009/005318
(87) International publication number: WO 2010/036353

(56) References cited:
- WO-A1-2008/055341
- US-A1- 2008 206 768
- LEUCHT S ET AL: "What does the PANSS mean?", SCHIZOPHRENIA RESEARCH, ELSEVIER, NETHERLANDS, vol. 79, no. 2-3, 15 November 2005 (2005-11-15), pages 231-238, XP027835369, ISSN: 0920-9964 [retrieved on 2005-11-15]
- RILEY BRIEN ET AL: "Molecular genetic studies of schizophrenia", EUROPEAN JOURNAL OF HUMAN GENETICS, KARGER, BASEL, CH, vol. 14, no. 6, 1 June 2006 (2006-06-01), pages 669-680, XP002519427, ISSN: 1018-4813, DOI: 10.1038/SJ.EJHG.5201571
- BERTOLINO ET AL: "COMT Val<158>Met polymorphism predicts negative symptoms response to treatment with olanzapine in schizophrenia", SCHIZOPHRENIA RESEARCH, ELSEVIER, NETHERLANDS, vol. 95, no. 1-3, 29 August 2007 (2007-08-29), pages 253-255, XP022217143, ISSN: 0920-9964, DOI: 10.1016/J.SCHRES.2007.06.014

## Description

### TECHNICAL FIELD

This invention relates to genetic markers that are associated with response to treatments for schizophrenia (SZ) or schizophrenia spectrum disorders (SSDs), which includes SZ, schizotypal personality disorder (SPD), and schizoaffective disorder (SD). For example, this document provides methods for using such genetic markers to stratify patient populations in order to select optimal treatments.

### BACKGROUND

The schizophrenia spectrum disorders include schizophrenia (SZ), schizotypal personality disorder (SPD), and/or schizoaffective disorder (SD). Schizophrenia (SZ) is considered a clinical syndrome, and is probably a constellation of several pathologies. Substantial heterogeneity is seen between cases, which is thought to reflect multiple overlapping etiologic factors, including both genetic and environmental contributions. SD is characterized by the presence of affective (depressive or manic) symptoms and schizophrenic symptoms within the same, uninterrupted episode of illness. SPD is characterized by a pervasive pattern of social and interpersonal deficits marked by acute discomfort with, and reduced capacity for, close relationships as well as by cognitive or perceptual distortions and eccentricities of behavior, beginning by early adulthood and present in a variety of contexts.

Various genes and chromosomes have been implicated in etiology of SZ. Many studies have suggested the presence of one or more important genes relating to SZ on most or all of the autosomes (Williams et al., Hum. Mol. Genet. 8:1729-1739 (1999);;; Fallin et al., Am. J. Hum. Genet. 77:918-936 (2005); ; Badner et al., Mol. Psychiatry 7:405-411 (2002);; Cooper-Casey et al., Mol. Psychiatry 10:651-656 (2005); Devlin et al., Mol. Psychiatry 7:689-694 (2002); Fallin et al., Am. J. Hum. Genet. 73:601-611 (2003);; Jablensky, Mol. Psychiatry 11: 815-836 (2006); Kirov et al., J. Clin. Invest. 115:1440-1448 (2005); Norton et al., Curr. Opin. Psychiatry 19:158-164 (2006); Owen et al., Mol. Psychiatry 9:14-27 (2004)). However, none of these prior studies have used high resolution genetic association methods to systematically compare genes involved in response to treatments for SSD, e.g., using anti-psychotics. Neither have any of these studies demonstrated that genetic polymorphisms in the genes defined herein are important in response to anti-psychotics.

US 20080206768 A1 relates to the relative effect of specific genetic polymorphisms in predicting the clinical outcome of olanzapine therapy in patients suffering from a psychiatric disease such as schizophrenia.

Due to the severity of these disorders, especially the negative impact of a psychotic episode on a patient, and the diminishing recovery after each psychotic episode, there is a need to more conclusively identify individuals who will respond best to specific therapies, and/or who is likely to suffer the most severe side effects, to determine appropriate therapies based on genotypic subtype.

### SUMMARY

This disclosure provides methods of identifying, selecting, and administering optimal treatments for SZ or SSDs, based on the presence of variations in genes involved in a number of pathways including: glutamate signaling and metabolism, cell adhesion, cytoskeletal architecture, vesicle formation, and trafficking, G-protein coupled receptors, carrier proteins and transporters, cell cycle modulators, neuronal development, calcium/calmodulin signaling, neuropeptide signaling, and several additional genes identified by virtue of their interaction with genes in high impact pathways and their expression in the central nervous system. This disclosure provides methods and claims relating to predicting treatment response (e.g., efficacy and likelihood of side effects) for these conditions based on their underlying genetic architecture. As described herein, methods for predicting response in order to select optimal treatments include evaluation of SNPs for genes relating to response to treatments for SZ. In some embodiments, specific exemplary SNPs are given that can be used to capture significant allelic variation in these genes. The allelic and genotypic variants thus identified can be used to stratify a patient population in order to select optimal treatments (e.g., atypical antipsychotics, typical antipsychotics, antidepressants, lithium) for patients.

In one aspect, this document features methods for predicting a human patient's likely response to a treatment for schizophrenia (SZ). These ethods are described in the appended claims.

Determining the identity of an allele can include obtaining a sample comprising DNA from the subject, and determining identity of the nucleotide at the polymorphic site. Determining the identity of the nucleotide can include contacting the sample with a probe specific for a selected allele of the polymorphism, and detecting the formation of complexes between the probe and the selected allele of the polymorphism, wherein the formation of complexes between the probe and the test marker indicates the presence of the selected allele in the sample. Determining the identity of an allele can include determining the identity of a nucleotide at position 31 of one of SEQ ID NOs: 1-4617. Methods can further include selecting or excluding a subject for enrollment in a clinical trial based on the identity of the allele. Methods can further include stratifying a subject population for analysis of a clinical trial based on the identity of the allele in the subjects.

A response can be selected from the group consisting of a change in a PANNS score; length of time until a discontinuation of therapy; likelihood of discontinuing therapy due to lack of efficacy; and likelihood of discontinuing therapy due to a serious adverse event. A positive response can be a decrease in a PANSS score relative to baseline; a long time until discontinuation of therapy; a low likelihood of discontinuing therapy due to lack of efficacy; and a low likelihood of discontinuing therapy due to a serious adverse event. Methods can further include recording the response allele in a tangible medium. A tangible medium can include a computer-readable disk, a solid state memory device, or an optical storage device.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DETAILED DESCRIPTION

Described herein are methods for predicting response to treatments for SSDs (and optionally stratifying patient populations), e.g., in order to select optimal treatments, based on evaluation of single nucleotide polymorphisms (SNPs) for genes on chromosomes 1-22. As described herein, bioinformatic and genetic analyses provided evidence of association of the disclosed SNP alleles associated with response, e.g., efficacy (positive response) or the occurrence of side effects to certain treatments for these disorders.

### Definitions

As used herein, an "allele" is one of a pair or series of genetic variants of a polymorphism at a specific genomic location. A "response allele" is an allele that is associated with altered response to a treatment. Where a SNP is biallelic, both alleles will be response alleles (e.g., one will be associated with a positive response, while the other allele is associated with no or a negative response, or some variation thereof).

As used herein, "genotype" refers to the diploid combination of alleles for a given genetic polymorphism. A homozygous subject carries two copies of the same allele and a heterozygous subject carries two different alleles.

As used herein, a "haplotype" is one or a set of signature genetic changes (polymorphisms) that are normally grouped closely together on the DNA strand, and are usually inherited as a group; the polymorphisms are also referred to herein as "markers." A "haplotype" as used herein is information regarding the presence or absence of one or more genetic markers in a given chromosomal region in a subject. A haplotype can consist of a variety of genetic markers, including indels (insertions or deletions of the DNA at particular locations on the chromosome); single nucleotide polymorphisms (SNPs) in which a particular nucleotide is changed; microsatellites; and minisatellites.

Microsatellites (sometimes referred to as a variable number of tandem repeats or VNTRs) are short segments of DNA that have a repeated sequence, usually about 2 to 5 nucleotides long (e.g., CACACA), that tend to occur in non-coding DNA. Changes in the microsatellites sometimes occur during the genetic recombination of sexual reproduction, increasing or decreasing the number of repeats found at an allele, changing the length of the allele. Microsatellite markers are stable, polymorphic, easily analyzed and occur regularly throughout the genome, making them especially suitable for genetic analysis.

"Copy number variation" (CNV), as used herein, refers to variation from the normal diploid condition for a gene or polymorphism. Individual segments of human chromosomes can be deleted or duplicated such that the subject's two chromosome carry fewer than two copies of the gene or polymorphism (a deletion or deficiency) or two or more copies (a duplication).

"Linkage disequilibrium" refers to when the observed frequencies of haplotypes in a population does not agree with haplotype frequencies predicted by multiplying together the frequency of individual genetic markers in each haplotype.

The term "chromosome" as used herein refers to a gene carrier of a cell that is derived from chromatin and comprises DNA and protein components (e.g., histones). The conventional internationally recognized individual human genome chromosome numbering identification system is employed herein. The size of an individual chromosome can vary from one type to another with a given multi-chromosomal genome and from one genome to another. In the case of the human genome, the entire DNA mass of a given chromosome is usually greater than about 100,000,000 base pairs. For example, the size of the entire human genome is about 3 X 10⁹ base pairs.

The term "gene" refers to a DNA sequence in a chromosome that codes for a product (either RNA or its translation product, a polypeptide). A gene contains a coding region and includes regions preceding and following the coding region (termed respectively "leader" and "trailer"). The coding region is comprised of a plurality of coding segments ("exons") and intervening sequences ("introns") between individual coding segments.

The term "probe" refers to an oligonucleotide. A probe can be single stranded at the time of hybridization to a target. As used herein, probes include primers, i.e., oligonucleotides that can be used to prime a reaction, e.g., a PCR reaction.

The term "label" or "label containing moiety" refers in a moiety capable of detection, such as a radioactive isotope or group containing same, and nonisotopic labels, such as enzymes, biotin, avidin, streptavidin, digoxygenin, luminescent agents, dyes, haptens, and the like. Luminescent agents, depending upon the source of exciting energy, can be classified as radioluminescent, chemiluminescent, bioluminescent, and photoluminescent (including fluorescent and phosphorescent). A probe described herein can be bound, e.g., chemically bound to label-containing moieties or can be suitable to be so bound. The probe can be directly or indirectly labeled.

The term "direct label probe" (or "directly labeled probe") refers to a nucleic acid probe whose label after hybrid formation with a target is detectable without further reactive processing of hybrid. The term "indirect label probe" (or "indirectly labeled probe") refers to a nucleic acid probe whose label after hybrid formation with a target is further reacted in subsequent processing with one or more reagents to associate therewith one or more moieties that finally result in a detectable entity.

The terms "target," "DNA target," or "DNA target region" refers to a nucleotide sequence that occurs at a specific chromosomal location. Each such sequence or portion is preferably at least partially, single stranded (e.g., denatured) at the time of hybridization. When the target nucleotide sequences are located only in a single region or fraction of a given chromosome, the term "target region" is sometimes used. Targets for hybridization can be derived from specimens which include, but are not limited to, chromosomes or regions of chromosomes in normal, diseased or malignant human cells, either interphase or at any state of meiosis or mitosis, and either extracted or derived from living or postmortem tissues, organs or fluids; germinal cells including sperm and egg cells, or cells from zygotes, fetuses, or embryos, or chorionic or amniotic cells, or cells from any other germinating body; cells grown in vitro, from either long-term or short-term culture, and either normal, immortalized or transformed; inter- or intraspecific hybrids of different types of cells or differentiation states of these cells; individual chromosomes or portions of chromosomes, or translocated, deleted or other damaged chromosomes, isolated by any of a number of means known to those with skill in the art, including libraries of such chromosomes cloned and propagated in prokaryotic or other cloning vectors, or amplified in vitro by means well known to those with skill; or any forensic material, including but not limited to blood, or other samples.

The term "hybrid" refers to the product of a hybridization procedure between a probe and a target.

The term "hybridizing conditions" has general reference to the combinations of conditions that are employable in a given hybridization procedure to produce hybrids, such conditions typically involving controlled temperature, liquid phase, and contact between a probe (or probe composition) and a target. Conveniently and preferably, at least one denaturation step precedes a step wherein a probe or probe composition is contacted with a target. Guidance for performing hybridization reactions can be found in Ausubel et al., Current Protocols in Molecular Biology, John Wiley & Sons, N.Y. (2003), 6.3.1-6.3.6. Aqueous and nonaqueous methods are described in that reference and either can be used. Hybridization conditions referred to herein are a 50% formamide, 2X SSC wash for 10 minutes at 45°C followed by a 2X SSC wash for 10 minutes at 37°C.

Calculations of "identity" between two sequences can be performed as follows. The sequences are aligned for optimal comparison purposes (e.g., gaps can be introduced in one or both of a first and a second nucleic acid sequence for optimal alignment and non-identical sequences can be disregarded for comparison purposes). The length of a sequence aligned for comparison purposes is at least 30% (e.g., at least 40%, 50%, 60%, 70%, 80%, 90% or 100%) of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In some embodiments, the percent identity between two nucleotide sequences is determined using the GAP program in the GCG software package, using a Blossum 62 scoring matrix with a gap penalty of 12, a gap extend penalty of 4, and a frameshift gap penalty of 5.

As used herein, the term "substantially identical" is used to refer to a first nucleotide sequence that contains a sufficient number of identical nucleotides to a second nucleotide sequence such that the first and second nucleotide sequences have similar activities. Nucleotide sequences that are substantially identical are at least 80% (e.g., 85%, 90%, 95%, 97% or more) identical.

The term "nonspecific binding DNA" refers to DNA which is complementary to DNA segments of a probe, which DNA occurs in at least one other position in a genome, outside of a selected chromosomal target region within that genome. An example of nonspecific binding DNA comprises a class of DNA repeated segments whose members commonly occur in more than one chromosome or chromosome region. Such common repetitive segments tend to hybridize to a greater extent than other DNA segments that are present in probe composition.

As used herein, the term "stratification" refers to the creation of a distinction between subjects on the basis of a characteristic or characteristics of the subjects. Generally, in the context of clinical trials, the distinction is used to distinguish responses or effects in different sets of patients distinguished according to the stratification parameters. In some embodiments, stratification includes distinction of subject groups based on the presence or absence of particular markers or alleles described herein. The stratification can be performed, e.g., in the course of analysis, or can be used in creation of distinct groups or in other ways.

### Methods of Predicting Response and Selecting Optimal Treatment

Described herein are a variety of methods for predicting a subject's response, or selecting and optimizing (and optionally administering) a treatment for a subject having an SSD (e.g., SZ) based on the presence or absence of a response allele.

As used herein, "determining the identity of an allele" includes obtaining information regarding the identity (i.e., of a specific nucleotide), presence or absence of one or more specific alleles in a subject. Determining the identity of an allele can, but need not, include obtaining a sample comprising DNA from a subject, and/or assessing the identity, presence or absence of one or more genetic markers in the sample. The individual or organization who determines the identity of the allele need not actually carry out the physical analysis of a sample from a subject; the methods can include using information obtained by analysis of the sample by a third party. Thus the methods can include steps that occur at more than one site. For example, a sample can be obtained from a subject at a first site, such as at a health care provider, or at the subject's home in the case of a self-testing kit. The sample can be analyzed at the same or a second site, e.g., at a laboratory or other testing facility.

Determining the identity of an allele can also include or consist of reviewing a subject's medical history, where the medical history includes information regarding the identity, presence or absence of one or more response alleles in the subject, e.g., results of a genetic test.

In some embodiments, to determine the identity of an allele described herein, a biological sample that includes nucleated cells (such as blood, a cheek swab or mouthwash) is prepared and analyzed for the presence or absence of preselected markers. Such diagnoses may be performed by diagnostic laboratories, or, alternatively, diagnostic kits can be manufactured and sold to health care providers or to private individuals for self-diagnosis. Diagnostic or prognostic tests can be performed as described herein or using well known techniques, such as described in U.S. Pat. No. 5,800,998.

Results of these tests, and optionally interpretive information, can be returned to the subject, the health care provider or to a third party payor. The results can be used in a number of ways. The information can be, e.g., communicated to the tested subject, e.g., with a prognosis and optionally interpretive materials that help the subject understand the test results and prognosis. The information can be used, e.g., by a health care provider, to determine whether to administer a specific drug, or whether a subject should be assigned to a specific category, e.g., a category associated with a specific disease endophenotype, or with drug response or non-response. The information can be used, e.g., by a third party payor such as a healthcare payer (e.g., insurance company or HMO) or other agency, to determine whether or not to reimburse a health care provider for services to the subject, or whether to approve the provision of services to the subject. For example, the healthcare payer may decide to reimburse a health care provider for treatments for an SSD if the subject has a particular response allele. As another example, a drug or treatment may be indicated for individuals with a certain allele, and the insurance company would only reimburse the health care provider (or the insured individual) for prescription or purchase of the drug if the insured individual has that response allele. The presence or absence of the response allele in a patient may be ascertained by using any of the methods described herein.

### Response Alleles

This document provides methods for predicting response and selecting an optimal treatment based on evaluation of one or more single nucleotide polymorphisms (SNPs) associated with specific treatment responses in subjects having SZ or SZ-spectrum disorders including SZ, SPD, or SD. Tables 1-20 and Table A list specific SNPs, variation of which is associated with altered response. One of skill in the art will appreciate that other variants can be identified and verified by Case/Control comparisons using the SNP markers presented herein. Using SNP markers that are identical to or in linkage disequilibrium with the exemplary SNPs, one can determine additional alleles of the genes, such as haplotypes, relating to response to treatment of an SSD (e.g., SZ). The allelic variants thus identified can be used, e.g., to select optimal treatments (e.g., pharmaceutical and/or psychosocial intervention) for patients.

### Markers in Linkage disequilibrium (LD)

Linkage disequilibrium (LD) is a measure of the degree of association between alleles in a population. One of skill in the art will appreciate that alleles involving markers in LD with the polymorphisms described herein can also be used in a similar manner to those described herein. Methods of calculating LD are known in the art (see, e.g., Morton et al., Proc. Natl. Acad. Sci. USA 98(9):5217-21 (2001); Tapper et al., Proc. Natl. Acad. Sci. USA 102(33):11835-11839 (2005); Maniatis et al., Proc. Natl. Acad. Sci. USA 99:2228-2233 (2002)). Thus, in some cases, the methods can include analysis of polymorphisms that are in LD with a polymorphism described herein. Methods are known in the art for identifying such polymorphisms; for example, the International HapMap Project provides a public database that can be used, see hapmap.org, as well as The International HapMap Consortium, Nature 426:789-796 (2003), and The International HapMap Consortium, Nature 437:1299-1320 (2005). Generally, it will be desirable to use a HapMap constructed using data from individuals who share ethnicity with the subject. For example, a HapMap for African Americans would ideally be used to identify markers in LD with an exemplary marker described herein for use in genotyping a subject of African American descent.

Alternatively, methods described herein can include analysis of polymorphisms that show a correlation coefficient (r²) of value ≥ 0.5 with the markers described herein. Results can be obtained from on line public resources such as HapMap.org on the World Wide Web. The correlation coefficient is a measure of LD, and reflects the degree to which alleles at two loci (for example, two SNPs) occur together, such that an allele at one SNP position can predict the correlated allele at a second SNP position, in the case where r² is > 0.5.

### Identifying Additional Genetic Markers

In general, genetic markers can be identified using any of a number of methods well known in the art. For example, numerous polymorphisms in the regions described herein are known to exist and are available in public databases, which can be searched using methods and algorithms known in the art. Alternately, polymorphisms can be identified by sequencing either genomic DNA or cDNA in the region in which it is desired to find a polymorphism. According to one approach, primers are designed to amplify such a region, and DNA from a subject is obtained and amplified. The DNA is sequenced, and the sequence (referred to as a "subject sequence" or "test sequence") is compared with a reference sequence, which can represent the "normal" or "wild type" sequence, or the "affected" sequence. In some embodiments, a reference sequence can be from, for example, the human draft genome sequence, publicly available in various databases, or a sequence deposited in a database such as GenBank. In some embodiments, the reference sequence is a composite of ethnically diverse individuals.

In general, if sequencing reveals a difference between the sequenced region and the reference sequence, a polymorphism has been identified. The fact that a difference in nucleotide sequence is identified at a particular site that determines that a polymorphism exists at that site. In most instances, particularly in the case of SNPs, only two polymorphic variants will exist at any location. However, in the case of SNPs, up to four variants may exist since there are four naturally occurring nucleotides in DNA. Other polymorphisms, such as insertions and deletions, may have more than four alleles.

In some embodiments, the methods include determining the presence or absence of one or more other markers that are or may be associated with treatment response, e.g., in one or more genes, e.g., as described in WO 2009/092032, WO 2009/089120, WO 2009/082743, US2006/0177851, or US2009/0012371. See also, e.g., OMIM entry no. 181500 (SCZD).

### Methods of Determining Identity of a Subject's Response Allele

The methods described herein include determining the identity, e.g., the specific nucleotide, presence or absence, of alleles associated with a predicted response to a treatment for an SSD, e.g., SZ. In some embodiments, a predicted response to a method of treating an SSD is determined by detecting the presence of an identical allele in both the subject and an individual with a known response to a method of treating an SSD, e.g., in an unrelated reference subject or a first or second-degree relation of the subject, and, in some cases, the absence of the allele in an reference individual having a known but opposite response. Thus the methods can include obtaining and analyzing a sample from a suitable reference individual. Samples that are suitable for use in the methods described herein contain genetic material, e.g., genomic DNA (gDNA). Genomic DNA is typically extracted from biological samples such as blood or mucosal scrapings of the lining of the mouth, but can be extracted from other biological samples including urine or expectorant. The sample itself will typically include nucleated cells (e.g., blood or buccal cells) or tissue removed from the subject. The subject can be an adult, child, fetus, or embryo. In some embodiments, the sample is obtained prenatally, either from a fetus or embryo or from the mother (e.g., from fetal or embryonic cells in the maternal circulation). Methods and reagents are known in the art for obtaining, processing, and analyzing samples. In some embodiments, the sample is obtained with the assistance of a health care provider, e.g., to draw blood. In some embodiments, the sample is obtained without the assistance of a health care provider, e.g., where the sample is obtained non-invasively, such as a sample comprising buccal cells that is obtained using a buccal swab or brush, or a mouthwash sample.

In some cases, a biological sample may be processed for DNA isolation. For example, DNA in a cell or tissue sample can be separated from other components of the sample. Cells can be harvested from a biological sample using standard techniques known in the art. For example, cells can be harvested by centrifuging a cell sample and resuspending the pelleted cells. The cells can be resuspended in a buffered solution such as phosphate-buffered saline (PBS). After centrifuging the cell suspension to obtain a cell pellet, the cells can be lysed to extract DNA, e.g., gDNA. See, e.g., Ausubel et al., 2003, supra. The sample can be concentrated and/or purified to isolate DNA. All samples obtained from a subject, including those subjected to any sort of further processing, are considered to be obtained from the subject. Routine methods can be used to extract genomic DNA from a biological sample, including, for example, phenol extraction. Alternatively, genomic DNA can be extracted with kits such as the QIAamp® Tissue Kit (Qiagen, Chatsworth, CA) and the Wizard® Genomic DNA purification kit (Promega). Nonlimiting examples of sources of samples include urine, blood, and tissue.

The presence or absence of an allele or genotype associated with a predicted response to treatment for an SPD (e.g., SZ) as described herein can be determined using methods known in the art. For example, gel electrophoresis, capillary electrophoresis, size exclusion chromatography, sequencing, and/or arrays can be used to detect the presence or absence of specific response alleles. Amplification of nucleic acids, where desirable, can be accomplished using methods known in the art, e.g., PCR. In one example, a sample (e.g., a sample comprising genomic DNA), is obtained from a subject. The DNA in the sample is then examined to determine the identity of an allele as described herein, i.e., by determining the identity of one or more alleles associated with a selected response. The identity of an allele can be determined by any method described herein, e.g., by sequencing or by hybridization of the gene in the genomic DNA, RNA, or cDNA to a nucleic acid probe, e.g., a DNA probe (which includes cDNA and oligonucleotide probes) or an RNA probe. The nucleic acid probe can be designed to specifically or preferentially hybridize with a particular polymorphic variant.

Other methods of nucleic acid analysis can include direct manual sequencing (Church and Gilbert, Proc. Natl. Acad. Sci. USA 81:1991-1995 (1988); Sanger et al., Proc. Natl. Acad. Sci. USA 74:5463-5467 (1977); Beavis et al., U.S. Pat. No. 5,288,644); automated fluorescent sequencing; single-stranded conformation polymorphism assays (SSCP) (Schafer et al., Nat. Biotechnol. 15:33-39 (1995)); clamped denaturing gel electrophoresis (CDGE); two-dimensional gel electrophoresis (2DGE or TDGE); conformational sensitive gel electrophoresis (CSGE); denaturing gradient gel electrophoresis (DGGE) (Sheffield et al., Proc. Natl. Acad. Sci. USA 86:232-236 (1989)); denaturing high performance liquid chromatography (DHPLC, Underhill et al., Genome Res. 7:996-1005 (1997)); infrared matrix-assisted laser desorption/ionization (IR-MALDI) mass spectrometry (WO 99/57318); mobility shift analysis (Orita et al., Proc. Natl. Acad. Sci. USA 86:2766-2770 (1989)); restriction enzyme analysis (Flavell et al., Cell 15:25 (1978); Geever et al., Proc. Natl. Acad. Sci. USA 78:5081 (1981)); quantitative real-time PCR (Raca et al., Genet Test 8(4):387-94 (2004)); heteroduplex analysis; chemical mismatch cleavage (CMC) (Cotton et al., Proc. Natl. Acad. Sci. USA 85:4397-4401 (1985)); RNase protection assays (Myers et al., Science 230:1242 (1985)); use of polypeptides that recognize nucleotide mismatches, e.g., *E*. *coli* mutS protein; allele-specific PCR, and combinations of such methods. See, e.g., Gerber et al., U.S. Patent Publication No. 2004/0014095.

Sequence analysis can also be used to detect specific polymorphic variants. For example, polymorphic variants can be detected by sequencing exons, introns, 5' untranslated sequences, or 3' untranslated sequences. A sample comprising DNA or RNA is obtained from the subject. PCR or other appropriate methods can be used to amplify a portion encompassing the polymorphic site, if desired. The sequence is then ascertained, using any standard method, and the presence of a polymorphic variant is determined. Real-time pyrophosphate DNA sequencing is yet another approach to detection of polymorphisms and polymorphic variants (Alderbom et al., Genome Research 10(8):1249-1258 (2000)). Additional methods include, for example, PCR amplification in combination with denaturing high performance liquid chromatography (dHPLC) (Underhill et al., Genome Research 7(10):996-1005 (1997)).

In order to detect polymorphisms and/or polymorphic variants, it may be desirable to amplify a portion of genomic DNA (gDNA) encompassing the polymorphic site. Such regions can be amplified and isolated by PCR using oligonucleotide primers designed based on genomic and/or cDNA sequences that flank the site. PCR refers to procedures in which target nucleic acid (e.g., genomic DNA) is amplified in a manner similar to that described in U.S. Patent No. 4,683,195, and subsequent modifications of the procedure described therein. Generally, sequence information from the ends of the region of interest or beyond are used to design oligonucleotide primers that are identical or similar in sequence to opposite strands of a potential template to be amplified. See e.g., PCR Primer: A Laboratory Manual, Dieffenbach and Dveksler, (Eds.); McPherson et al., PCR Basics: From Background to Bench (Springer Verlag, 2000); Mattila et al., Nucleic Acids Res., 19:4967 (1991); Eckert et al., PCR Methods and Applications, 1:17 (1991); PCR (eds. McPherson et al., IRL Press, Oxford); and U.S. Pat. No. 4,683,202. Other amplification methods that may be employed include the ligase chain reaction (LCR) (Wu and Wallace, Genomics 4:560 (1989), Landegren et al., Science 241:1077 (1988), transcription amplification (Kwoh et al., Proc. Natl. Acad. Sci. USA 86:1173 (1989)), self-sustained sequence replication (Guatelli et al., Proc. Nat. Acad. Sci. USA 87:1874 (1990)), and nucleic acid based sequence amplification (NASBA). Guidelines for selecting primers for PCR amplification are well known in the art. See, e.g., McPherson et al., PCR Basics: From Background to Bench, Springer-Verlag, 2000. A variety of computer programs for designing primers are available, e.g., 'Oligo' (National Biosciences, Inc, Plymouth Minn.), MacVector (Kodak/IBI), and the GCG suite of sequence analysis programs (Genetics Computer Group, Madison, Wis. 53711).

In some cases, PCR conditions and primers can be developed that amplify a product only when the variant allele is present or only when the wild type allele is present (MSPCR or allele-specific PCR). For example, patient DNA and a control can be amplified separately using either a wild type primer or a primer specific for the variant allele. Each set of reactions is then examined for the presence of amplification products using standard methods to visualize the DNA. For example, the reactions can be electrophoresed through an agarose gel and the DNA visualized by staining with ethidium bromide or other DNA intercalating dye. In DNA samples from heterozygous patients, reaction products would be detected in each reaction.

Real-time quantitative PCR can also be used to determine copy number. Quantitative PCR permits both detection and quantification of specific DNA sequence in a sample as an absolute number of copies or as a relative amount when normalized to DNA input or other normalizing genes. A key feature of quantitative PCR is that the amplified DNA product is quantified in real-time as it accumulates in the reaction after each amplification cycle. Methods of quantification can include the use of fluorescent dyes that intercalate with double-stranded DNA, and modified DNA oligonucleotide probes that fluoresce when hybridized with a complementary DNA. Methods of quantification can include determining the intensity of fluorescence for fluorescently tagged molecular probes attached to a solid surface such as a microarray.

The first report of extensive copy number variation (CNV) in the human genome used intensity analysis of microarray data to document numerous examples of genes that vary in copy number (Redon et al., Nature 444(7118):444-54 (2006)). Subsequent studies have shown that certain copy number variants are associated with complex genetic diseases such as SZ (Walsh et al., Science 320(5875):539-43 (2008); and Stone et al., Nature 455(7210):237-41 (2008)).

In some embodiments, a peptide nucleic acid (PNA) probe can be used instead of a nucleic acid probe in the hybridization methods described above. PNA is a DNA mimetic with a peptide-like, inorganic backbone, e.g., N-(2-aminoethyl)glycine units, with an organic base (A, G, C, T or U) attached to the glycine nitrogen via a methylene carbonyl linker (see, e.g., Nielsen et al., Bioconjugate Chemistry, The American Chemical Society, 5:1 (1994)). The PNA probe can be designed to specifically hybridize to a nucleic acid comprising a polymorphic variant.

In some cases, allele-specific oligonucleotides can also be used to detect the presence of a polymorphic variant. For example, polymorphic variants can be detected by performing allele-specific hybridization or allele-specific restriction digests. Allele specific hybridization is an example of a method that can be used to detect sequence variants, including complete genotypes of a subject (e.g., a mammal such as a human). See Stoneking et al., Am. J. Hum. Genet. 48:370-382 (1991); and Prince et al., Genome Res. 11:152-162 (2001). An "allele-specific oligonucleotide" (also referred to herein as an "allele-specific oligonucleotide probe") is an oligonucleotide that is specific for particular a polymorphism can be prepared using standard methods (see Ausubel et al., Current Protocols in Molecular Biology*,* supra). Allele-specific oligonucleotide probes typically can be approximately 10-50 base pairs, preferably approximately 15-30 base pairs, that specifically hybridizes to a nucleic acid region that contains a polymorphism. Hybridization conditions are selected such that a nucleic acid probe can specifically bind to the sequence of interest, e.g., the variant nucleic acid sequence. Such hybridizations typically are performed under high stringency as some sequence variants include only a single nucleotide difference. In some cases, dot-blot hybridization of amplified oligonucleotides with allele-specific oligonucleotide (ASO) probes can be performed. See, for example, Saiki et al., Nature (London) 324:163-166 (1986).

In some embodiments, allele-specific restriction digest analysis can be used to detect the existence of a polymorphic variant of a polymorphism, if alternate polymorphic variants of the polymorphism result in the creation or elimination of a restriction site. Allele-specific restriction digests can be performed in the following manner. A sample containing genomic DNA is obtained from the individual and genomic DNA is isolated for analysis. For nucleotide sequence variants that introduce a restriction site, restriction digest with the particular restriction enzyme can differentiate the alleles. In some cases, polymerase chain reaction (PCR) can be used to amplify a region comprising the polymorphic site, and restriction fragment length polymorphism analysis is conducted (see Ausubel et al., Current Protocols in Molecular Biology*,* supra). The digestion pattern of the relevant DNA fragment indicates the presence or absence of a particular polymorphic variant of the polymorphism and is therefore indicative of the subject's response allele. For sequence variants that do not alter a common restriction site, mutagenic primers can be designed that introduce a restriction site when the variant allele is present or when the wild type allele is present. For example, a portion of a nucleic acid can be amplified using the mutagenic primer and a wild type primer, followed by digest with the appropriate restriction endonuclease.

In some embodiments, fluorescence polarization template-directed dye-terminator incorporation (FP-TDI) is used to determine which of multiple polymorphic variants of a polymorphism is present in a subject (Chen et al., Genome Research 9(5):492-498 (1999)). Rather than involving use of allele-specific probes or primers, this method employs primers that terminate adjacent to a polymorphic site, so that extension of the primer by a single nucleotide results in incorporation of a nucleotide complementary to the polymorphic variant at the polymorphic site.

In some cases, DNA containing an amplified portion may be dot-blotted, using standard methods (see Ausubel et al., Current Protocols in Molecular Biology*,* supra), and the blot contacted with the oligonucleotide probe. The presence of specific hybridization of the probe to the DNA is then detected. Specific hybridization of an allele-specific oligonucleotide probe (specific for a polymorphic variant indicative of a predicted response to a method of treating an SSD) to DNA from the subject is indicative of a subject's response allele.

The methods can include determining the genotype of a subject with respect to both copies of the polymorphic site present in the genome (i.e., both alleles). For example, the complete genotype may be characterized as -/-, as -/+, or as +/+, where a minus sign indicates the presence of the reference or wild type sequence at the polymorphic site, and the plus sign indicates the presence of a polymorphic variant other than the reference sequence. If multiple polymorphic variants exist at a site, this can be appropriately indicated by specifying which ones are present in the subject. Any of the detection means described herein can be used to determine the genotype of a subject with respect to one or both copies of the polymorphism present in the subject's genome.

Methods of nucleic acid analysis to detect polymorphisms and/or polymorphic variants can include, e.g., microarray analysis. Hybridization methods, such as Southern analysis, Northern analysis, or *in situ* hybridizations, can also be used (see Ausubel et al., Current Protocols in Molecular Biology, eds., John Wiley & Sons (2003)). To detect microdeletions, fluorescence in situ hybridization (FISH) using DNA probes that are directed to a putatively deleted region in a chromosome can be used. For example, probes that detect all or a part of a microsatellite marker can be used to detect microdeletions in the region that contains that marker.

In some embodiments, it is desirable to employ methods that can detect the presence of multiple polymorphisms (e.g., polymorphic variants at a plurality of polymorphic sites) in parallel or substantially simultaneously. Oligonucleotide arrays represent one suitable means for doing so. Other methods, including methods in which reactions (e.g., amplification, hybridization) are performed in individual vessels, e.g., within individual wells of a multi-well plate or other vessel may also be performed so as to detect the presence of multiple polymorphic variants (e.g., polymorphic variants at a plurality of polymorphic sites) in parallel or substantially simultaneously according to the methods provided herein.

Nucleic acid probes can be used to detect and/or quantify the presence of a particular target nucleic acid sequence within a sample of nucleic acid sequences, e.g., as hybridization probes, or to amplify a particular target sequence within a sample, e.g., as a primer. Probes have a complimentary nucleic acid sequence that selectively hybridizes to the target nucleic acid sequence. In order for a probe to hybridize to a target sequence, the hybridization probe must have sufficient identity with the target sequence, i.e., at least 70% (e.g., 80%, 90%, 95%, 98% or more) identity to the target sequence. The probe sequence must also be sufficiently long so that the probe exhibits selectivity for the target sequence over non-target sequences. For example, the probe will be at least 20 (e.g., 25, 30, 35, 50, 100, 200, 300, 400, 500, 600, 700, 800, 900 or more) nucleotides in length. In some embodiments, the probes are not more than 30, 50, 100, 200, 300, 500, 750, or 1000 nucleotides in length. Probes are typically about 20 to about 1 x 10⁶ nucleotides in length. Probes include primers, which generally refers to a single-stranded oligonucleotide probe that can act as a point of initiation of template-directed DNA synthesis using methods such as PCR (polymerase chain reaction), LCR (ligase chain reaction), etc., for amplification of a target sequence.

The probe can be a test probe such as a probe that can be used to detect polymorphisms in a region described herein (e.g., an allele associated with treatment response as described herein). In some embodiments, the probe can bind to another marker sequence associated with SZ, SPD, or SD as described herein or known in the art.

Control probes can also be used. For example, a probe that binds a less variable sequence, e.g., repetitive DNA associated with a centromere of a chromosome, can be used as a control. Probes that hybridize with various centromeric DNA and locus-specific DNA are available commercially, for example, from Vysis, Inc. (Downers Grove, Ill.), Molecular Probes, Inc. (Eugene, Oreg.), or from Cytocell (Oxfordshire, UK). Probe sets are available commercially such from Applied Biosystems, e.g., the Assays-on-Demand SNP kits Alternatively, probes can be synthesized, e.g., chemically or in vitro, or made from chromosomal or genomic DNA through standard techniques. For example, sources of DNA that can be used include genomic DNA, cloned DNA sequences, somatic cell hybrids that contain one, or a part of one, human chromosome along with the normal chromosome complement of the host, and chromosomes purified by flow cytometry or microdissection. The region of interest can be isolated through cloning, or by site-specific amplification via the polymerase chain reaction (PCR). See, for example, Nath and Johnson, Biotechnic. Histochem. 73(1):6-22 (1998); Wheeless et al., Cytometry 17:319-326 (1994); and U.S. Pat. No. 5,491,224.

In some embodiments, the probes are labeled, e.g., by direct labeling, with a fluorophore, an organic molecule that fluoresces after absorbing light of lower wavelength/higher energy. A directly labeled fluorophore allows the probe to be visualized without a secondary detection molecule. After covalently attaching a fluorophore to a nucleotide, the nucleotide can be directly incorporated into the probe with standard techniques such as nick translation, random priming, and PCR labeling. Alternatively, deoxycytidine nucleotides within the probe can be transaminated with a linker. The fluorophore then is covalently attached to the transaminated deoxycytidine nucleotides. See, e.g., U.S. Pat. No. 5,491,224.

Fluorophores of different colors can be chosen such that each probe in a set can be distinctly visualized. For example, a combination of the following fluorophores can be used: 7-amino-4-methylcoumarin-3-acetic acid (AMCA), TEXAS RED™ (Molecular Probes, Inc., Eugene, OR), 5-(and-6)-carboxy-X-rhodamine, lissamine rhodamine B, 5-(and-6)-carboxyfluorescein, fluorescein-5-isothiocyanate (FITC), 7-diethylaminocoumarin-3-carboxylic acid, tetramethylrhodamine-5-(and-6)-isothiocyanate, 5-(and-6)-carboxytetramethylrhodamine, 7-hydroxycoumarin-3-carboxylic acid, 6-[fluorescein 5-(and-6)-carboxamido]hexanoic acid, N-(4,4-difluoro-5,7-dimethyl-4-bora-3a,4a diaza-3-indacenepropionic acid, eosin-5-isothiocyanate, erythrosin-5-isothiocyanate, and CASCADE™ blue acetylazide (Molecular Probes, Inc., Eugene, OR). Fluorescently labeled probes can be viewed with a fluorescence microscope and an appropriate filter for each fluorophore, or by using dual or triple band-pass filter sets to observe multiple fluorophores. See, for example, U.S. Pat. No. 5,776,688. Alternatively, techniques such as flow cytometry can be used to examine the hybridization pattern of the probes. Fluorescence-based arrays are also known in the art.

In other embodiments, the probes can be indirectly labeled with, e.g., biotin or digoxygenin, or labeled with radioactive isotopes such as ³²P and ³H. For example, a probe indirectly labeled with biotin can be detected by avidin conjugated to a detectable marker. For example, avidin can be conjugated to an enzymatic marker such as alkaline phosphatase or horseradish peroxidase. Enzymatic markers can be detected in standard colorimetric reactions using a substrate and/or a catalyst for the enzyme. Catalysts for alkaline phosphatase include 5-bromo-4-chloro-3-indolylphosphate and nitro blue tetrazolium. Diaminobenzoate can be used as a catalyst for horseradish peroxidase.

In another aspect, this document features arrays that include a substrate having a plurality of addressable areas, and methods of using them. At least one area of the plurality includes a nucleic acid probe that binds specifically to a sequence comprising a polymorphism listed in Table A, and can be used to detect the absence or presence of said polymorphism, e.g., one or more SNPs, microsatellites, minisatellites, or indels, as described herein, to determine a response allele. For example, the array can include one or more nucleic acid probes that can be used to detect a polymorphism listed in Table A. In some embodiments, the array further includes at least one area that includes a nucleic acid probe that can be used to specifically detect another marker associated with a predicted response to a method of treating an SSD (e.g., SZ), as described herein. In some embodiments, the probes are nucleic acid capture probes.

Generally, microarray hybridization is performed by hybridizing a nucleic acid of interest (e.g., a nucleic acid encompassing a polymorphic site) with the array and detecting hybridization using nucleic acid probes. In some cases, the nucleic acid of interest is amplified prior to hybridization. Hybridization and detecting are generally carried out according to standard methods. See, e.g., Published PCT Application Nos. WO 92/10092 and WO 95/11995, and U.S. Pat. No. 5,424,186. For example, the array can be scanned to determine the position on the array to which the nucleic acid hybridizes. The hybridization data obtained from the scan is typically in the form of fluorescence intensities as a function of location on the array.

Arrays can be formed on substrates fabricated with materials such as paper, glass, plastic (e.g., polypropylene, nylon, or polystyrene), polyacrylamide, nitrocellulose, silicon, optical fiber, or any other suitable solid or semisolid support, and can be configured in a planar (e.g., glass plates, silicon chips) or three dimensional (e.g., pins, fibers, beads, particles, microtiter wells, capillaries) configuration. Methods for generating arrays are known in the art and include, e.g., photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145). The array typically includes oligonucleotide hybridization probes capable of specifically hybridizing to different polymorphic variants. Oligonucleotide probes that exhibit differential or selective binding to polymorphic sites may readily be designed by one of ordinary skill in the art. For example, an oligonucleotide that is perfectly complementary to a sequence that encompasses a polymorphic site (i.e., a sequence that includes the polymorphic site, within it or at one end) will generally hybridize preferentially to a nucleic acid comprising that sequence, as opposed to a nucleic acid comprising an alternate polymorphic variant.

Oligonucleotide probes forming an array may be attached to a substrate by any number of techniques, including, without limitation, (i) *in situ* synthesis (e.g., high-density oligonucleotide arrays) using photolithographic techniques; (ii) spotting/printing at medium to low density on glass, nylon or nitrocellulose; (iii) by masking, and (iv) by dot-blotting on a nylon or nitrocellulose hybridization membrane. Oligonucleotides can be immobilized via a linker, including by covalent, ionic, or physical linkage. Linkers for immobilizing nucleic acids and polypeptides, including reversible or cleavable linkers, are known in the art. See, for example, U.S. Patent No. 5,451,683 and WO98/20019. Alternatively, oligonucleotides can be non-covalently immobilized on a substrate by hybridization to anchors, by means of magnetic beads, or in a fluid phase such as in microtiter wells or capillaries. Immobilized oligonucleotide probes are typically about 20 nucleotides in length, but can vary from about 10 nucleotides to about 1000 nucleotides in length.

Arrays can include multiple detection blocks (i.e., multiple groups of probes designed for detection of particular polymorphisms). Such arrays can be used to analyze multiple different polymorphisms. Detection blocks may be grouped within a single array or in multiple, separate arrays so that varying conditions (e.g., conditions optimized for particular polymorphisms) may be used during the hybridization. For example, it may be desirable to provide for the detection of those polymorphisms that fall within G-C rich stretches of a genomic sequence, separately from those falling in A-T rich segments. General descriptions of using oligonucleotide arrays for detection of polymorphisms can be found, for example, in U.S. Pat. Nos. 5,858,659 and 5,837,832. In addition to oligonucleotide arrays, cDNA arrays may be used similarly in certain embodiments.

The methods described herein can include providing an array as described herein; contacting the array with a sample (e.g., all or a portion of genomic DNA that includes at least a portion of a human chromosome comprising a response allele) and/or optionally, a different portion of genomic DNA (e.g., a portion that includes a different portion of one or more human chromosomes), and detecting binding of a nucleic acid from the sample to the array. Optionally, the method includes amplifying nucleic acid from the sample, e.g., genomic DNA that includes a portion of a human chromosome described herein, and, optionally, a region that includes another region associated with a predicted response to a method of treating SZ, SD, or SPD, prior to or during contact with the array.

In some aspects, the methods described herein can include using an array that can ascertain differential expression patterns or copy numbers of one or more genes in samples from normal and affected individuals (see, e.g., Redon et al., Nature 444(7118):444-54 (2006)). For example, arrays of probes to a marker described herein can be used to measure polymorphisms between DNA from a subject having an SSD (e.g., SZ) and having a predicted response to a treatment for an SSD (e.g., SZ), and control DNA, e.g., DNA obtained from an individual that has SZ, SPD, or SD, and has a known response to a form of treatment for an SSD (e.g., SZ). Since the clones on the array contain sequence tags, their positions on the array are accurately known relative to the genomic sequence. Different hybridization patterns between DNA from an individual afflicted with an SSD (e.g., SZ) and DNA from a control individual at areas in the array corresponding to markers as described herein, and, optionally, one or more other regions associated with an SSD (e.g., SZ), are indicative of a predicted response to a treatment for an SSD (e.g., SZ). Methods for array production, hybridization, and analysis are described, e.g., in Snijders et al., Nat. Genetics 29:263-264 (2001); Klein et al., Proc. Natl Acad. Sci. USA 96:4494-4499 (1999); Albertson et al., Breast Cancer Research and Treatment 78:289-298 (2003); and Snijders et al., "BAC microarray based comparative genomic hybridization," in: Zhao et al. (eds), Bacterial Artificial Chromosomes: Methods and Protocols, Methods in Molecular Biology, Humana Press, 2002.

In another aspect, this document provides methods of determining the absence or presence of a response allele associated with a predicted response to treatment for an SSD (e.g., SZ) as described herein, using an array described above. The methods can include providing a two dimensional array having a plurality of addresses, each address of the plurality being positionally distinguishable from each other address of the plurality having a unique nucleic acid capture probe, contacting the array with a first sample from a test subject who is has an SSD (e.g., SZ), and comparing the binding of the first sample with one or more references, e.g., binding of a sample from a subject who is known to have an SSD (e.g., SZ), and/or binding of a sample from a subject who has an SSD (e.g., SZ) and a known response to treatment for an SSD (e.g., SZ); and comparing the binding of the first sample with the binding of the second sample. In some embodiments, the methods can include contacting the array with a third sample from a cell or subject that does not have SZ; and comparing the binding of the first sample with the binding of the third sample. In some embodiments, the second and third samples are from first or second-degree relatives of the test subject. In the case of a nucleic acid hybridization, binding with a capture probe at an address of the plurality, can be detected by any method known in the art, e.g., by detection of a signal generated from a label attached to the nucleic acid.

### Schizophrenia Spectrum Disorders

The methods described herein can be used to determine an individual predicted response to a method of treating a schizophrenia spectrum disorder (SSD). The SSDs include schizophrenia (SZ), schizotypal personality disorder (SPD), and schizoaffective disorder (SD). Methods for diagnosing SSDs are known in the art, see, e.g., the DSM-IV. See, e.g., WO 2009/092032.

### Methods of Selecting and Optimizing Treatment

In some embodiments, the methods described herein include the administration of one or more treatments, e.g., antipsychotic medications, to a person identified as having or being at risk of developing an SSD (e.g., SZ). The methods can also include selecting a treatment regimen for a subject who has an SSD or is determined to be at risk for developing an SSD (e.g., SZ), based upon the absence or presence of an allele or genotype associated with response as described herein. The determination of a treatment regimen can also be based upon the absence or presence of other risk factors, e.g., as known in the art or described herein. A treatment regimen can include the administration of antipsychotic medications to a subject identified as having or at risk of developing an SSD (e.g., SZ) before the onset of any psychotic episodes.

As used herein, the term "treat" or "treatment" is defined as the application or administration of a treatment regimen, e.g., a therapeutic agent or modality, to a subject, e.g., a patient. The subject can be a patient having an SSD (e.g., SZ), a symptom of an SSD (e.g., SZ), or at risk of developing (i.e., a predisposition toward) an SSD (e.g., SZ). The treatment can be to cure, heal, alleviate, relieve, alter, remedy, ameliorate, palliate, improve or affect an SSD (e.g., SZ), the symptoms of an SSD (e.g., SZ) or the predisposition toward an SSD (e.g., SZ). For example, a standard treatment regimen for schizophrenia is the administration of antipsychotic medications, which are typically antagonists acting at postsynaptic D2 dopamine receptors in the brain and can include neuroleptics and/or atypical antipsychotics. Antipsychotic medications substantially reduce the risk of relapse in the stable phase of illness. Currently accepted treatments for SZ are described in greater detail in the Practice Guideline for the Treatment of Patients With Schizophrenia American Psychiatric Association, Second Edition, American Psychiatric Association, 2004, which is incorporated herein by reference in its entirety.

The methods of determining a treatment regimen as described herein can further include the step of monitoring the subject, e.g., for a change (e.g., an increase or decrease) in one or more of the diagnostic criteria for an SSD listed herein, or any other parameter related to clinical outcome. The subject can be monitored in one or more of the following periods: prior to beginning of treatment; during the treatment; or after one or more elements of the treatment have been administered. Monitoring can be used to evaluate the need for further treatment with the same or a different therapeutic agent or modality. Generally, a decrease in one or more of the parameters described above is indicative of the improved condition of the subject, although with red blood cell and platelet levels, an increase can be associated with the improved condition of the subject.

The methods can be used, for example, to choose between alternative treatments (e.g., a particular dosage, mode of delivery, time of delivery, inclusion of adjunctive therapy, e.g., administration in combination with a second agent) based on the subject's probable drug response.

With regards to both prophylactic and therapeutic methods of treatment of SSDs, according to the present diagnostic methods, a treatment can be specifically tailored or modified, based on knowledge obtained from pharmacogenomics. "Pharmacogenomics," as used herein, refers to the application of genomics technologies such as structural chromosomal analysis, to drugs in clinical development and on the market. See, for example, Eichelbaum et al., Clin. Exp. Pharmacol. Physiol. 23:983-985 (1996); Linder et al., Clin. Chem. 43:254-266 (1997); Wang et al. (2003) Pharmacogenetics 13:555-64; Weinshilboum and Wang (2004) Nature Rev. Drug Discovery 3:739-748; Guttmacher and Collins (2005) JAMA 294:1399-402; and Weinshilboum and Wang, Annu. Rev. Genomics Hum. Genet. 7:223-45 (2006). Specifically, as used herein, the term refers the study of how a patient's genes determine his or her response to a drug (e.g., a patient's "drug response phenotype," or "drug response allele"). Drug response phenotypes that are influenced by inheritance and can vary from potentially life-threatening adverse reactions at one of the spectrum to lack of therapeutic efficacy at the other. The ability to determine whether and how a subject will respond to a particular drug can assist medical professionals in determining whether the drug should be administered to the subject, and at what dose. Thus, this documents provides methods for tailoring an individual's prophylactic or therapeutic treatment according to the presence of specific drug response alleles in that individual.

Standard pharmacologic therapies for SSDs include the administration of one or more antipsychotic medications, which are typically antagonists acting at postsynaptic D2 dopamine receptors in the brain. Antipsychotic medications include conventional, or first generation, antipsychotic agents, which are sometimes referred to as neuroleptics because of their neurologic side effects, and second generation antipsychotic agents, which are less likely to exhibit neuroleptic effects and have been termed atypical antipsychotics. Typical antipsychotics can include chlorpromazine, fluphenazine, haloperidol, thiothixene, trifluoperazine, perphenazine, and thioridazine; atypical antipsychotics can include aripiprazole, risperidone, clozapine, olanzapine, quetiapine, or ziprasidone.

Information generated from pharmacogenomic research using a method described herein can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when administering a therapeutic composition (e.g., a cytotoxic agent or combination of cytotoxic agents) to a patient as a means of treating or preventing progression of SSDs.

In some cases, a physician or clinician may consider applying knowledge obtained in relevant pharmacogenomics studies (e.g., using a method described herein) when determining whether to administer a pharmaceutical composition such as an antipsychotic agent or a combination of antipsychotic agents to a subject. In other cases, a physician or clinician may consider applying such knowledge when determining the dosage or frequency of treatments (e.g., administration of antipsychotic agent or combination of antipsychotic agents to a patient). As one example, a physician or clinician can determine (or have determined by, for example, a laboratory) the presence of one or more response alleles in a subject as described herein, and optionally one or more other markers associated with an SSD (e.g., SZ) or response to a treatment, of one or a group of subjects, e.g., clinical patients, or subjects who may be participating in a clinical trial, e.g., a trial designed to test the efficacy of a pharmaceutical composition (e.g., an antipsychotic or combination of antipsychotic agents); the physician or clinician can then correlate the genetic makeup of the subjects with their response to the pharmaceutical composition.

As another example, information regarding a response allele as described herein can be used to stratify or select a subject population for a clinical trial. The information can, in some embodiments, be used to stratify individuals that exhibit or are likely to exhibit a toxic response to a treatment from those that will not. In other cases, the information can be used to separate those that will be non-responders from those who will be responders. The allele s described herein can be used in pharmacogenomics-based design and manage the conduct of a clinical trial, e.g., as described in U.S. Pat. Pub. No. 2003/0108938.

As another example, information regarding a response allele as described herein, can be used to stratify or select human cells or cell lines for drug testing purposes. Human cells are useful for studying the effect of a polymorphism on physiological function, and for identifying and/or evaluating potential therapeutic agents for the treatment of SSDs, e.g., anti-psychotics. Thus the methods can include performing the present methods on genetic material from a cell line. The information can, in some embodiments, be used to separate cells that respond or are expected to respond to particular drugs from those that do not respond, e.g. which cells show altered second messenger signaling.

Also disclosed herein are compositions and methods for the identification and treatment of subjects who have a predicted response to a treatment for an SSD (e.g., SZ), such that a theranostic approach can be taken to test such individuals to predict the effectiveness of a particular therapeutic intervention (e.g., a pharmaceutical or non-pharmaceutical intervention as described herein) and to alter the intervention to (1) reduce the risk of developing adverse outcomes and (2) enhance the effectiveness of the intervention. Thus, the methods and compositions described herein also provide a means of optimizing the treatment of a subject having such a disorder. Provided herein is a theranostic approach to treating and preventing SSDs, by integrating diagnostics and therapeutics to improve the real-time treatment of a subject. Practically, this means creating tests that can identify which patients are most suited to a particular therapy, and providing feedback on how well a drug is working to optimize treatment regimens.

Within the clinical trial setting, a theranostic method as described herein can provide key information to optimize trial design, monitor efficacy, and enhance drug safety. For instance, "trial design" theranostics can be used for patient stratification, determination of patient eligibility (inclusion/exclusion), creation of homogeneous treatment groups, and selection of patient samples that are representative of the general population. Such theranostic tests can therefore provide the means for patient efficacy enrichment, thereby minimizing the number of individuals needed for trial recruitment. "Efficacy" theranostics are useful for monitoring therapy and assessing efficacy criteria. Finally, "safety" theranostics can be used to prevent adverse drug reactions or avoid medication error.

The methods described herein can include retrospective analysis of clinical trial data as well, both at the subject level and for the entire trial, to detect correlations between an allele as described herein and any measurable or quantifiable parameter relating to the outcome of the treatment, e.g., efficacy (the results of which may be binary (i.e., yes and no) as well as along a continuum), side-effect profile (e.g., weight gain, metabolic dysfunction, lipid dysfunction, movement disorders, or extrapyramidal symptoms), treatment maintenance and discontinuation rates, return to work status, hospitalizations, suicidality, total healthcare cost, social functioning scales, response to non-pharmacological treatments, and/or dose response curves. The results of these correlations can then be used to influence decision-making, e.g., regarding treatment or therapeutic strategies, provision of services, and/or payment. For example, a correlation between a positive outcome parameter (e.g., high efficacy, low side effect profile, high treatment maintenance/low discontinuation rates, good return to work status, low hospitalizations, low suicidality, low total healthcare cost, high social function scale, favorable response to non-pharmacological treatments, and/or acceptable dose response curves) and a selected allele or genotype can influence treatment such that the treatment is recommended or selected for a subject having the selected allele or genotype.

This document also provides methods and materials to assist medical or research professionals in determining whether a particular treatment regimen is optimal. Medical professionals can be, for example, doctors, nurses, medical laboratory technologists, and pharmacists. Research professionals can be, for example, principle investigators, research technicians, postdoctoral trainees, and graduate students. A professional can be assisted by (1) determining whether specific polymorphic variants are present in a biological sample from a subject, and (2) communicating information about polymorphic variants to that professional.

Using information about specific polymorphic variants determined using a method described herein, a medical professional can take one or more actions that can affect patient care. For example, a medical professional can record information in the patient's medical record regarding the patient's likely response to a given treatment for an SSD (e.g., SZ). In some cases, a medical professional can record information regarding a treatment assessment, or otherwise transform the patient's medical record, to reflect the patient's current treatment and response allele(s). In some cases, a medical professional can review and evaluate a patient's entire medical record and assess multiple treatment strategies for clinical intervention of a patient's condition.

A medical professional can initiate or modify treatment after receiving information regarding a patient's response allele(s), for example. In some cases, a medical professional can recommend a change in therapy based on the subject's response allele(s). In some cases, a medical professional can enroll a patient in a clinical trial for, by way of example, detecting correlations between an allele or genotype as described herein and any measurable or quantifiable parameter relating to the outcome of the treatment as described above.

A medical professional can communicate information regarding a patient's expected response to a treatment to a patient or a patient's family. In some cases, a medical professional can provide a patient and/or a patient's family with information regarding SSDs and response assessment information, including treatment options, prognosis, and referrals to specialists. In some cases, a medical professional can provide a copy of a patient's medical records to a specialist.

A research professional can apply information regarding a subject's response allele(s) to advance scientific research. For example, a researcher can compile data on specific polymorphic variants. In some cases, a research professional can obtain a subject's response allele(s) as described herein to evaluate a subject's enrollment, or continued participation, in a research study or clinical trial. In some cases, a research professional can communicate information regarding a subject's response allele(s) to a medical professional. In some cases, a research professional can refer a subject to a medical professional.

Any appropriate method can be used to communicate information to another person (e.g., a professional). For example, information can be given directly or indirectly to a professional. For example, a laboratory technician can input a patient's polymorphic variant alleles as described herein into a computer-based record. In some cases, information is communicated by making a physical alteration to medical or research records. For example, a medical professional can make a permanent notation or flag a medical record for communicating the response allele determination to other medical professionals reviewing the record. In addition, any type of communication can be used to communicate allelic, genotypic, and/or treatment information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

### Articles of Manufacture

Also disclosed herein are articles of manufacture comprising a probe that hybridizes with a region of human chromosome as described herein and can be used to detect a polymorphism described herein. For example, any of the probes for detecting polymorphisms described herein can be combined with packaging material to generate articles of manufacture or kits. The kit can include one or more other elements including: instructions for use; and other reagents such as a label or an agent useful for attaching a label to the probe. Instructions for use can include instructions for diagnostic applications of the probe for predicting response to a treatment for SSDs in a method described herein. Other instructions can include instructions for attaching a label to the probe, instructions for performing *in situ* analysis with the probe, and/or instructions for obtaining a sample to be analyzed from a subject. In some cases, the kit can include a labeled probe that hybridizes to a region of human chromosome as described herein.

The kit can also include one or more additional reference or control probes that hybridize to the same chromosome or another chromosome or portion thereof that can have an abnormality associated with a particular response. A kit that includes additional probes can further include labels, e.g., one or more of the same or different labels for the probes. In other examples, the additional probe or probes provided with the kit can be a labeled probe or probes. When the kit further includes one or more additional probe or probes, the kit can further provide instructions for the use of the additional probe or probes. Kits for use in self-testing can also be provided. Such test kits can include devices and instructions that a subject can use to obtain a biological sample (e.g., buccal cells, blood) without the aid of a health care provider. For example, buccal cells can be obtained using a buccal swab or brush, or using mouthwash.

Kits as provided herein can also include a mailer (e.g., a postage paid envelope or mailing pack) that can be used to return the sample for analysis, e.g., to a laboratory. The kit can include one or more containers for the sample, or the sample can be in a standard blood collection vial. The kit can also include one or more of an informed consent form, a test requisition form, and instructions on how to use the kit in a method described herein. Methods for using such kits are also included herein. One or more of the forms (e.g., the test requisition form) and the container holding the sample can be coded, for example, with a bar code for identifying the subject who provided the sample.

### Databases and Reports

Also disclosed herein are databases that include a list of polymorphisms as described herein, and wherein the list is largely or entirely limited to polymorphisms identified as useful for predicting a subject's response to a treatment for an SSD (e.g., SZ) as described herein. The list is stored, e.g., on a flat file or computer-readable medium. The databases can further include information regarding one or more subjects, e.g., whether a subject is affected or unaffected, clinical information such as endophenotype, age of onset of symptoms, any treatments administered and outcomes (e.g., data relevant to pharmacogenomics, diagnostics or theranostics), and other details, e.g., about the disorder in the subject, or environmental or other genetic factors. The databases can be used to detect correlations between a particular allele or genotype and the information regarding the subject.

The methods described herein can also include the generation of reports, e.g., for use by a patient, care giver, payor, or researcher, that include information regarding a subject's response allele(s), and optionally further information such as treatments administered, treatment history, medical history, predicted response, and actual response. The reports can be recorded in a tangible medium, e.g., a computer-readable disk, a solid state memory device, or an optical storage device.

### Engineered Cells

Also disclosed herein are engineered cells that harbor one or more polymorphisms described herein, e.g., one or more response alleles. Such cells are useful for studying the effect of a polymorphism on physiological function, and for identifying and/or evaluating potential therapeutic agents such as anti-psychotics for the treatment of an SSD (e.g., SZ).

As one example, included herein are cells in which one or more of the various alleles of the genes described herein has be re-created that is associated with a response to a specific treatment. Methods are known in the art for generating cells, e.g., by homologous recombination between the endogenous gene and an exogenous DNA molecule introduced into a cell, e.g., a cell of an animal. In some cases, the cells can be used to generate transgenic animals using methods known in the art.

The cells are preferably mammalian cells (e.g., neuronal type cells) in which an endogenous gene has been altered to include a polymorphism as described herein. Techniques such as targeted homologous recombinations, can be used to insert the heterologous DNA as described in, e.g., Chappel, US 5,272,071; WO 91/06667, published in May 16, 1991.

The invention is further described in the following examples, which do not limit the scope of the invention described in the claims.

### EXAMPLES

### Example 1: Markers Associated with Drug Response

The Clinical Antipsychotic Trials of Intervention Effectiveness (CATIE), a large federally funded clinical trial designed to assess the efficacy of antipsychotics in a real world setting, is a valuable resource for determining the role of genes in drug response (Stroup et al., Schizophr. Bull. 29:15-31 (2003); Lieberman et al., N. Engl. J. Med. 353:1209-1223 (2005)). As part of the CATIE trial, SNP genotyping was performed for roughly half of the trial participants (Sullivan et al., Mol. Psychiatry 13:570-584 (2008)). When combined with disease status, PANSS scores, and clinical drug response data, the genotyping data allows the identification of genetic variants (e.g., SNPs) that are statistically associated with specific responses to selected treatments.

The design of the CATIE study has been described in detail by others (see, e.g., Stroup et al., Schizophr. Bull. 29:15-31 (2003); Lieberman et al., N. Engl. J. Med. 353:1209-1223 (2005)). Briefly, 1460 subjects were randomly assigned one of several antipsychotics and those who did not respond or chose to quit their current medication were re-randomized to another drug. Details regarding SNP genotyping and quality control have been recently published (Sullivan et al., Mol. Psychiatry 13:570-584 (2008)).

Genotype and phenotype data for the CATIE trial were made available to qualified researchers through the NIMH Center for Collaborative Genetic Studies on Mental Disorders. Data for 417 patients with schizophrenia and 419 unaffected controls self reported as having exclusively European ancestry were evaluated. This same patient population was described in a recent study by Sullivan and coworkers, which confirmed that there is no hidden stratification in the sample (Sullivan et al., Mol. Psychiatry 13:570-584 (2008)).

For the CATIE study, individual cases were diagnosed as having SZ based on DSM-III/IV criteria. All patients analyzed herein, either were taking the medication as their first antipsychotic medication or were randomly assigned at the start of the trial to a drug different from the one they were taking prior to enrollment in the CATIE Trial. This approach reflects a real world situation in which patients initiate therapy with a new drug, and eliminates the uncontrolled variable of time on the same drug prior to initiation of the study.

Treatment response for all patients was assessed using the the Positive and Negative Syndrome Scale (PANSS) (Kay et al., Schizophr. Bull. 13:261-276 (1987); Kay et al., Br. J. Psychiatry Suppl 59-67 (1989); Leucht et al., Schizophr. Res. 79:231-238 (2005)). PANSS rating was performed at baseline (after a minimum 7 day drug free period) and at various time points throughout the study. The change in PANSS (delta PANSS) used here is the percent (%) change in PANSS is defined as [(PANSS_last observation - PANSS_baseline)/PANSS_basline] X 100. To assess drug response, the last observation for each patient in treatment Phase 1 of the CATIE trial was used as a primary time point at which assessment of efficacy occurred.

An independent assessment of efficacy and tolerability is the time until discontinuation of drug treatment for all causes. This was the primary endpoint of the CATIE Trial (Lieberman et al., N Engl J Med 353(12):1209-23 (2005)). The longer the time to discontinuation for a given drug, the better the efficacy of the drug and its tolerability. The duration between a patient's first visit and last visit in Phase I of CATIE defined the time until discontinuation.

In addition to the quantitative time to discontinuation and delta PANSS, the CATIE consortium assigned dichotomous outcomes for patients who discontinued due to lack of efficacy due to adverse events.

Genetic analysis to document the influence of allelic variants on response to administration of olanzapine, risperidone, quetiapine, perphenazine, and ziprasidone, was performed using the PLINK 1.03 whole genome analysis toolset developed by Purcell and coworkers (Purcell et al., Am. J. Hum. Genet. 81:559-575 (2007)). For quantitative variables, PLINK calculates P values use a linear regression and an additive model for the minor allele. For dichotomous comparisons, PLINK calculates P values for the allele-specific chi-squared test and the odds ratio (OR; or relative risk) associated with the minor allele.

### Confirmation of SNP effects on DELTA PANSS for various drugs:

Tables 1-5 provide numerous examples of SNP-based alleles that predict altered response to olanzapine, risperidone, quetiapine, perphenazine and ziprasidone. Tables 1-5 report the test allele, P values, and Beta weights (in % PANSS) from the linear regression for SNPs that affect DELTA PANSS for each of olanzapine, risperidone, quetiapine, perphenazine and ziprasidone. A negative beta weight indicates that the allele is associated with a decrease in DELTA PANSS score, corresponding to greater improvement (or lowering) in symptom burden. A positive beta weight indicates that the allele is associated with an increase in DELTA PANSS score, corresponding to less improvement in symptom burden.

### Confirmation of SNP effects on time to discontinuation of therapy:

Tables 6-10 provide numerous examples of SNP-based alleles that predict altered length of time until a discontinuation of various antipschotics (olanzapine, risperidone, quetiapine, perphenazine and ziprasidone). Tables 6-10 report the test allele, P values, and Beta weight from the linear regression for SNPs that affect Time to Discontinuation for each of olanzapine, risperidone, quetiapine, perphenazine and ziprasidone. A negative beta weight indicates a quicker time to discontinuation and a positive beta weight indicates an increased time to discontinuation.

### Confirmation of SNP effects on likelihood of discontinuing therapy due to lack of efficacy:

Tables 11-15 provide numerous examples of SNP-based alleles that predict altered likelihood of discontinuing therapy due to lack of efficacy for olanzapine, risperidone, quetiapine, perphenazine and ziprasidone. Tables 11-15 report the test allele, frequencies for discontinuers, P values, and ORs for SNPs that affect likelihood of discontinuing due to lack of efficacy for olanzapine, risperidone, quetiapine, perphenazine and ziprasidone. For Tables 11-15, ORs of > 1.0 indicate that the minor SNP allele reported in the table is associated with greater likelihood of discontinuing due to lack of efficacy, and ORs of < 1.0 indicate that the minor SNP allele is associated with lesser likelihood of discontinuing due to lack of efficacy.

### Confirmation of SNP effects on likelihood of discontinuing therapy due to serious adverse events:

Tables 16-20 provide numerous examples of SNP-based alleles that predict altered likelihood of discontinuing therapy due to serious adverse event for olanzapine, risperidone, quetiapine, perphenazine and ziprasidone. Tables 16-20 report the test allele, frequencies for discontinuers, P values, and ORs for SNPs that affect likelihood of discontinuing due to serious adverse event for olanzapine, risperidone, quetiapine, perphenazine and ziprasidone. For Tables 16-20, ORs of > 1.0 indicate that the minor SNP allele is associated with greater likelihood of discontinuing due to serious adverse event, and ORs of < 1.0 indicate that the minor SNP allele is associated with lesser likelihood of discontinuing due to serious adverse event.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

| **TABLE 1: Alleles Influencing Response to Olanzapine** | | | | | |
|---|---|---|---|---|---|
| **Gene** | **Seq ID** | **NCBI RS #** | **Allele** | **Beta (PANSS)** | **P** |
| AGBL4-C1ORF165 | 25 | 11205517 | C | 26.81 | 0.008637 |
| AGBL4-C1ORF165 | 26 | 17373849 | A | 26.81 | 0.008637 |
| LRP8 | 54 | 12035926 | G | 10.76 | 0.035950 |
| PTGFRN | 90 | 17036573 | G | 57.40 | 0.008632 |
| PTGFRN | 91 | 12078461 | A | 57.40 | 0.008632 |
| SEC16B | 129 | 869286 | G | 10.78 | 0.048400 |
| USH2A | 155 | 6665313 | A | -12.77 | 0.022990 |
| USH2A | 156 | 12403674 | T | -12.77 | 0.022990 |
| ESRRG | 181 | 2820879 | A | 15.61 | 0.027360 |
| GNG4 | 193 | 508208 | G | -12.26 | 0.005302 |
| GNG4 | 195 | 2774327 | A | -12.57 | 0.002177 |
| FMN2 | 251 | 882869 | G | -10.90 | 0.037300 |
| RGS7 | 252 | 1970533 | C | -14.29 | 0.007357 |
| RGS7 | 254 | 4659581 | G | -16.79 | 0.002922 |
| BRE | 314 | 7558946 | T | 11.16 | 0.006172 |
| BRE | 315 | 6705961 | T | 11.05 | 0.008115 |
| BRE | 320 | 17006590 | C | 12.04 | 0.013590 |
| HAAO | 357 | 6738169 | G | 8.46 | 0.034550 |
| PLEKHH2 | 358 | 12623703 | G | -11.12 | 0.049400 |
| PLEKHH2 | 361 | 7570252 | A | -10.91 | 0.022780 |
| PRKCE | 378 | 1464572 | T | 11.40 | 0.031800 |
| PRKCE | 383 | 4953294 | A | -10.50 | 0.023400 |
| PRKCE | 384 | 4953297 | A | -10.42 | 0.024550 |
| PRKCE | 388 | 13024907 | C | -12.16 | 0.004725 |
| EPAS1 | 391 | 17034950 | A | 9.49 | 0.035380 |
| LRP1B | 513 | 9283437 | C | -10.37 | 0.031560 |
| LRP1B | 516 | 10178963 | A | -9.13 | 0.036270 |
| KYNU | 528 | 351673 | T | -11.00 | 0.019100 |
| KYNU | 536 | 6732786 | G | 12.56 | 0.018150 |
| KCNJ3 | 592 | 2971899 | C | 13.11 | 0.023670 |
| PDE1A | 642 | 16823124 | A | 19.39 | 0.001305 |
| PDE1A | 643 | 10497597 | T | 14.07 | 0.015900 |
| PDE1A | 648 | 9332425 | T | 16.21 | 0.002216 |
| PDE1A | 649 | 2082136 | G | -11.32 | 0.011860 |
| PDE1A | 650 | 2887208 | C | -10.20 | 0.018620 |
| ERBB4 | 681 | 7563759 | A | -11.08 | 0.009072 |
| ERBB4 | 682 | 4672666 | G | -8.45 | 0.031710 |
| ERBB4 | 683 | 4672668 | G | -8.37 | 0.036590 |
| ERBB4 | 684 | 4553767 | G | -17.12 | 0.000231 |
| COL4A4 | 692 | 4423583 | T | 9.79 | 0.021630 |
| COL4A4 | 695 | 10166736 | A | 9.65 | 0.022100 |
| COL4A3 | 710 | 11681636 | C | -10.03 | 0.029610 |
| ECEL1 | 729 | 12616972 | C | 9.24 | 0.025260 |
| CNTN6 | 742 | 265799 | G | -10.12 | 0.009883 |
| CNTN6 | 743 | 3821429 | G | -14.99 | 0.002884 |
| CNTN4 | 760 | 1153486 | T | 12.01 | 0.003993 |
| ITPR1 | 783 | 7432768 | G | -13.75 | 0.001128 |
| CLASP2 | 817 | 6781846 | G | -9.20 | 0.041530 |
| CLASP2 | 818 | 9311026 | C | -12.35 | 0.033540 |
| FHIT | 926 | 1470035 | A | -9.95 | 0.020820 |
| CADPS | 950 | 968808 | T | -11.00 | 0.014620 |
| FOXP1 | 985 | 2597312 | C | 9.24 | 0.032610 |
| CPNE4 | 1049 | 6792146 | A | 12.84 | 0.033130 |
| PLD1 | 1109 | 2178532 | A | 27.10 | 0.000304 |
| NLGN1 | 1111 | 10936764 | G | -10.27 | 0.045880 |
| NLGN1 | 1116 | 12635897 | C | 12.61 | 0.010760 |
| LRRC15 | 1149 | 4974536 | T | 11.58 | 0.017400 |
| LDB2 | 1175 | 189988 | T | -15.99 | 0.025250 |
| LDB2 | 1176 | 150260 | G | -15.99 | 0.025250 |
| LDB2 | 1177 | 157613 | G | -15.99 | 0.025250 |
| LDB2 | 1178 | 157611 | A | -13.58 | 0.044860 |
| SLIT2 | 1188 | 10516357 | A | 10.25 | 0.040900 |
| KIAA1239 | 1204 | 3910363 | C | 13.97 | 0.036390 |
| LOC389207 | 1229 | 13142149 | G | 9.34 | 0.043370 |
| LOC389207 | 1230 | 7656477 | G | -9.98 | 0.036830 |
| SHROOM3 | 1260 | 9307184 | G | -10.00 | 0.032290 |
| SCD5 | 1268 | 6852540 | C | 13.92 | 0.003715 |
| PDLIM5 | 1295 | 2452600 | T | -8.37 | 0.042030 |
| PDLIM5 | 1302 | 998071 | C | -9.77 | 0.025140 |
| TSPAN5 | 1303 | 2037797 | A | 12.68 | 0.049990 |
| COL25A1 | 1317 | 2305438 | A | 10.70 | 0.014390 |
| COL25A1 | 1322 | 1405145 | A | 11.70 | 0.011360 |
| COL25A1 | 1323 | 11729511 | C | 10.72 | 0.019110 |
| COL25A1 | 1324 | 3844177 | C | 10.72 | 0.019110 |
| COL25A1 | 1326 | 3851416 | T | 9.29 | 0.033010 |
| COL25A1 | 1332 | 2526456 | G | -11.06 | 0.018890 |
| COL25A1 | 1333 | 2704102 | C | -10.70 | 0.024410 |
| COL25A1 | 1334 | 987695 | G | -12.86 | 0.005477 |
| COL25A1 | 1335 | 7675657 | A | -13.36 | 0.003300 |
| COL25A1 | 1336 | 4256292 | G | -17.74 | 0.000304 |
| ANK2 | 1343 | 1989930 | G | -10.41 | 0.043040 |
| CAMK2D | 1366 | 7438925 | G | 10.60 | 0.022010 |
| NDST3 | 1371 | 6534079 | A | 12.83 | 0.018010 |
| NDST3 | 1375 | 4576085 | G | 18.13 | 0.005461 |
| PRSS12 | 1378 | 4834679 | T | 27.32 | 0.007368 |
| PRSS12 | 1379 | 1514657 | A | 27.32 | 0.007368 |
| POU4F2 | 1425 | 7680105 | G | 17.52 | 0.013670 |
| POU4F2 | 1429 | 1394279 | G | 15.00 | 0.043980 |
| POU4F2 | 1430 | 1979903 | C | 22.91 | 0.045640 |
| PALLD | 1496 | 2710827 | A | -8.79 | 0.033930 |
| PALLD | 1498 | 4425335 | G | 16.65 | 0.000779 |
| PALLD | 1499 | 12511925 | T | 20.22 | 0.006582 |
| PALLD | 1501 | 1039385 | G | 11.26 | 0.014000 |
| PALLD | 1502 | 1039386 | G | -10.24 | 0.034310 |
| DNAH5 | 1550 | 6554811 | G | 9.18 | 0.047670 |
| CDH10 | 1576 | 983446 | T | 14.13 | 0.006220 |
| SLC45A2 | 1578 | 10461928 | G | 11.86 | 0.048340 |
| SLC1A3 | 1583 | 3776569 | G | -9.80 | 0.032610 |
| EGFLAM | 1594 | 10214135 | C | 10.91 | 0.036670 |
| ITGA1 | 1601 | 11886 | G | 11.51 | 0.049160 |
| ITGA1 | 1608 | 7731949 | C | 10.39 | 0.048900 |
| ITGA1 | 1609 | 16880453 | C | 9.24 | 0.044500 |
| ITGA1 | 1611 | 923837 | T | 9.03 | 0.046160 |
| PDE4D | 1624 | 12654005 | G | -9.77 | 0.034230 |
| PDE4D | 1626 | 2910642 | C | -9.77 | 0.034230 |
| CMYA5 | 1653 | 259064 | C | 15.38 | 0.040600 |
| GPR98 | 1689 | 1878878 | A | -8.54 | 0.041940 |
| GPR98 | 1690 | 2222244 | A | -8.80 | 0.035060 |
| SNCA1P | 1751 | 17149141 | A | 15.51 | 0.011780 |
| VDAC1 | 1770 | 6878448 | A | -9.79 | 0.034530 |
| VDAC1 | 1771 | 13173424 | T | 10.59 | 0.012670 |
| ODZ2 | 1787 | 2337017 | C | 18.93 | 0.000877 |
| ODZ2 | 1789 | 1421989 | G | 18.93 | 0.000877 |
| SLC22A23 | 1812 | 17136575 | G | -12.07 | 0.045960 |
| ATXN1 | 1835 | 235147 | A | 11.75 | 0.004224 |
| SLC17A4 | 1849 | 4712972 | A | -19.00 | 0.033590 |
| SLC17A4 | 1851 | 1892253 | T | -19.00 | 0.033590 |
| MSH5 | 1875 | 707937 | G | 12.11 | 0.040310 |
| RIMS1 | 1901 | 482736 | C | 15.14 | 0.015910 |
| RIMS1 | 1902 | 1010326 | A | 12.38 | 0.035570 |
| RIMS1 | 1912 | 1339226 | G | -10.43 | 0.049680 |
| STX11 | 1995 | 6935462 | A | 31.15 | 0.016300 |
| TFB1M | 2011 | 7775163 | A | 9.98 | 0.016750 |
| PARK2 | 2017 | 2315321 | T | -8.99 | 0.036560 |
| PARK2 | 2019 | 992037 | T | -18.66 | 0.000191 |
| PARK2 | 2020 | 12055483 | T | -12.27 | 0.012430 |
| PARK2 | 2021 | 6924602 | C | -11.78 | 0.016730 |
| PARK2 | 2026 | 12528179 | C | -9.17 | 0.020990 |
| PARK2 | 2028 | 11752805 | A | -9.16 | 0.025170 |
| PARK2 | 2029 | 2155486 | T | -7.81 | 0.043870 |
| PARK2 | 2030 | 9295173 | G | 8.65 | 0.041320 |
| ETV1 | 2096 | 9639168 | C | -10.29 | 0.014260 |
| STK31 | 2111 | 12532929 | T | -18.18 | 0.002355 |
| STK31 | 2113 | 2390813 | G | -8.82 | 0.038370 |
| CHN2 | 2127 | 245927 | G | -9.77 | 0.021040 |
| SCRN1 | 2130 | 6976421 | A | 9.15 | 0.046930 |
| PLEKHA8 | 2134 | 10275475 | C | 12.76 | 0.011990 |
| SCRN1 | 2136 | 1465327 | T | 11.68 | 0.023910 |
| VPS41 | 2173 | 2286105 | A | -17.06 | 0.004040 |
| VPS41 | 2174 | 2074655 | T | -17.06 | 0.004040 |
| VPS41 | 2175 | 17768153 | A | -17.06 | 0.004040 |
| VPS41 | 2176 | 3801140 | C | -10.04 | 0.026070 |
| VPS41 | 2178 | 4723793 | G | 10.82 | 0.028270 |
| ABCA13 | 2205 | 10273407 | C | 19.32 | 0.002663 |
| ABCA13 | 2213 | 10216298 | G | 16.64 | 0.015780 |
| WBSCR17 | 2236 | 12666361 | A | 9.68 | 0.046600 |
| HIP1 | 2256 | 17352471 | A | 18.55 | 0.000415 |
| MAGI2 | 2268 | 3807797 | T | 14.32 | 0.015060 |
| MAGI2 | 2269 | 3779305 | A | -9.96 | 0.041220 |
| MAGI2 | 2279 | 848913 | C | -13.15 | 0.031560 |
| MAGI2 | 2289 | 6971099 | T | -10.77 | 0.048300 |
| MAGI2 | 2292 | 10280554 | T | 13.89 | 0.045990 |
| MAGI2 | 2293 | 848934 | C | -15.70 | 0.008896 |
| MAGI2 | 2300 | 10248584 | G | -13.24 | 0.030650 |
| GNAI1 | 2302 | 2462131 | G | 9.84 | 0.047780 |
| SEMA3E | 2335 | 2713154 | G | -11.74 | 0.006444 |
| SEMA3E | 2339 | 2713174 | C | -14.11 | 0.000976 |
| SEMA3E | 2340 | 2249188 | G | 12.81 | 0.001285 |
| SEMA3E | 2341 | 10270844 | T | 10.15 | 0.019010 |
| ABCB4 | 2350 | 31658 | T | 16.07 | 0.022220 |
| ABCB4 | 2351 | 31662 | A | 15.43 | 0.022530 |
| ADAM22 | 2357 | 2282949 | C | -15.09 | 0.000817 |
| ADAM22 | 2358 | 6951172 | T | -9.25 | 0.034180 |
| ADAM22 | 2359 | 9632709 | C | -9.25 | 0.034180 |
| PON1 | 2366 | 662 | G | -13.91 | 0.004692 |
| PPP1R9A | 2370 | 4729174 | A | 10.06 | 0.031200 |
| PPP1R9A | 2371 | 7806304 | C | 8.99 | 0.049990 |
| PPP1R9A | 2375 | 12690919 | T | -9.58 | 0.030100 |
| PPP1R9A | 2376 | 854520 | T | -10.75 | 0.010670 |
| PON1 | 2382 | 2057681 | G | -14.04 | 0.004888 |
| PON1 | 2383 | 2299257 | C | -10.20 | 0.032230 |
| EMID2 | 2402 | 6947185 | C | -11.40 | 0.017800 |
| CUX1 | 2404 | 2960266 | A | 9.89 | 0.028890 |
| KCND2 | 2414 | 7795646 | G | -13.25 | 0.037660 |
| TBXAS1 | 2485 | 41723 | G | 10.56 | 0.041190 |
| CNTNAP2 | 2499 | 826824 | C | -11.70 | 0.028820 |
| CNTNAP2 | 2502 | 2022226 | C | -10.70 | 0.031970 |
| CSMD1 | 2541 | 13272021 | C | -14.73 | 0.026830 |
| CSMD1 | 2543 | 13260434 | A | -13.61 | 0.034730 |
| CSMD1 | 2545 | 13254344 | T | 15.79 | 0.000870 |
| CSMD1 | 2546 | 895696 | T | 15.79 | 0.000870 |
| CSMD1 | 2547 | 895695 | A | 15.79 | 0.000870 |
| CSMD1 | 2550 | 4493928 | A | 13.62 | 0.038940 |
| MCPH1 | 2551 | 1530408 | C | -11.75 | 0.031210 |
| MCPH1 | 2552 | 3020270 | T | -17.26 | 0.005508 |
| MCPH1 | 2554 | 2083914 | T | -16.11 | 0.010990 |
| MCPH1 | 2555 | 1054073 | T | -12.38 | 0.022760 |
| MCPH1 | 2556 | 2034143 | T | -13.36 | 0.023840 |
| MCPH1 | 2557 | 2922818 | T | -12.53 | 0.042330 |
| MCPH1 | 2561 | 2442496 | G | -11.11 | 0.044010 |
| ATP6V1B2 | 2616 | 13252207 | A | -12.69 | 0.017010 |
| ATP6V1B2 | 2617 | 11204102 | G | -10.93 | 0.014960 |
| GFRA2 | 2619 | 4506202 | G | 9.31 | 0.030150 |
| UNC5D | 2637 | 16884099 | G | -19.56 | 0.010900 |
| UNC5D | 2638 | 16884109 | G | -19.56 | 0.010900 |
| UNC5D | 2639 | 6989128 | A | -15.88 | 0.035710 |
| SNTG1 | 2661 | 12545915 | T | 11.48 | 0.018550 |
| NKAIN3 | 2707 | 16929516 | T | -13.15 | 0.031760 |
| NKAIN3 | 2716 | 4739023 | C | -9.81 | 0.045840 |
| NKAIN3 | 2723 | 10957263 | C | -18.48 | 0.012950 |
| KCNB2 | 2736 | 349358 | G | 12.83 | 0.046360 |
| KCNB2 | 2737 | 349355 | T | 12.83 | 0.046360 |
| MMP16 | 2764 | 6994019 | T | 10.12 | 0.041750 |
| NCALD | 2782 | 7830291 | C | 10.43 | 0.042310 |
| FER1L6 | 2840 | 9987271 | T | 14.30 | 0.003106 |
| PER1L6 | 2845 | 2069078 | C | -13.52 | 0.030900 |
| MTSS1 | 2854 | 4870913 | T | -13.67 | 0.049320 |
| DDEF1 | 2859 | 7003309 | C | -10.13 | 0.014770 |
| ADCY8 | 2889 | 17248206 | G | -9.75 | 0.034640 |
| SMARCA2 | 2928 | 7872216 | T | -11.12 | 0.045320 |
| PTPRD | 2939 | 3858060 | T | -14.90 | 0.001771 |
| PTPRD | 2940 | 3843579 | C | -14.90 | 0.001771 |
| KIAA1797 | 2960 | 7848159 | G | 18.05 | 0.019460 |
| KIAA1797 | 2961 | 7021708 | G | -11.87 | 0.007420 |
| KIAA1797 | 2962 | 2151002 | A | 14.39 | 0.002859 |
| KIAA1797 | 2963 | 10511687 | G | -10.33 | 0.032580 |
| KIAA1797 | 2967 | 6475483 | T | 18.99 | 0.000501 |
| KIAA1797 | 2968 | 10738569 | A | 18.08 | 0.001234 |
| KIAA1797 | 2969 | 7049134 | G | 18.24 | 0.001512 |
| KIAA1797 | 2970 | 2383162 | G | 16.26 | 0.000090 |
| KIAA1797 | 2971 | 4246849 | A | 19.56 | 0.000004 |
| KIAA1797 | 2972 | 4977823 | T | 19.25 | 0.000004 |
| KIAA1797 | 2973 | 6475490 | G | -9.06 | 0.032340 |
| KIAA1797 | 2975 | 4579612 | C | -9.49 | 0.036220 |
| KIAA1797 | 2976 | 4977848 | A | 17.87 | 0.000012 |
| KIAA1797 | 2977 | 7025851 | G | -8.64 | 0.041590 |
| KIAA1797 | 2981 | 4272463 | C | -9.98 | 0.019590 |
| TEK | 2990 | 1322051 | A | 23.07 | 0.014890 |
| PIP5K1B | 2999 | 872077 | A | -12.84 | 0.023790 |
| TMC1 | 3027 | 6560285 | T | -10.98 | 0.022740 |
| TMC1 | 3030 | 6560293 | A | 11.46 | 0.005511 |
| TMC1 | 3035 | 1417614 | T | -10.15 | 0.048480 |
| TRPM6 | 3045 | 7045338 | T | -10.21 | 0.033640 |
| TRPM6 | 3046 | 7859201 | C | -9.95 | 0.047940 |
| DIRAS2 | 3088 | 7028171 | A | 10.80 | 0.041780 |
| FKTN | 3111 | 2518128 | A | 9.00 | 0.049110 |
| PALM2 | 3117 | 17202329 | C | -11.60 | 0.025810 |
| PALM2 | 3118 | 736502 | T | -11.60 | 0.025810 |
| PALM2 | 3119 | 7849027 | G | -11.60 | 0.025810 |
| PALM2 | 3120 | 10980027 | A | -11.42 | 0.026120 |
| SVEP1 | 3124 | 963143 | A | 9.53 | 0.026250 |
| RALGPS1 | 3151 | 546493 | G | -10.21 | 0.049650 |
| RALGPS1 | 3160 | 10987576 | A | 13.92 | 0.030100 |
| SLC25A25 | 3161 | 7873492 | T | -15.90 | 0.018590 |
| SLC25A25 | 3162 | 9695803 | C | -12.88 | 0.042060 |
| VAV2 | 3187 | 7038256 | T | -17.52 | 0.018840 |
| VAV2 | 3188 | 12001180 | G | -14.19 | 0.022620 |
| OLFM1 | 3193 | 10776933 | T | 10.53 | 0.030380 |
| OLFM1 | 3194 | 2031825 | A | 9.90 | 0.042720 |
| PRKCQ | 3229 | 1889001 | A | -9.33 | 0.041640 |
| SLC16A9 | 3294 | 10128501 | G | -9.64 | 0.026350 |
| CDH23 | 3304 | 1417207 | G | 11.74 | 0.032910 |
| KCNMA1 | 3322 | 1907729 | T | -11.52 | 0.048920 |
| KCNMA1 | 3325 | 10509378 | T | 10.47 | 0.032570 |
| VTI1A | 3399 | 7918958 | G | -11.04 | 0.017060 |
| VTI1A | 3400 | 7342028 | T | -11.33 | 0.019300 |
| VTI1A | 3403 | 11196083 | T | -11.18 | 0.022540 |
| VTI1A | 3404 | 7068695 | C | -8.61 | 0.045770 |
| VTI1A | 3405 | 4145776 | G | -9.41 | 0.043560 |
| ATE1 | 3448 | 2420970 | T | 10.04 | 0.016610 |
| ATE1 | 3449 | 11200251 | T | 11.70 | 0.017210 |
| CTBP2 | 3455 | 9422765 | G | 10.58 | 0.029640 |
| CTBP2 | 3456 | 7896555 | G | 10.58 | 0.029640 |
| ARNTL | 3492 | 10832020 | C | 10.48 | 0.034040 |
| SPON1 | 3499 | 1528668 | C | -9.30 | 0.029390 |
| SPON1 | 3501 | 11023054 | A | -9.41 | 0.030010 |
| SPON1 | 3502 | 10832165 | A | -9.02 | 0.034340 |
| SPON1 | 3503 | 7112850 | A | -9.02 | 0.034340 |
| SPON1 | 3504 | 10766134 | G | -9.02 | 0.034340 |
| ELMOD1 | 3559 | 683266 | A | -10.43 | 0.019420 |
| TSPAN9 | 3594 | 4304861 | G | 10.42 | 0.019420 |
| TMEM16B | 3606 | 11063913 | G | -10.24 | 0.047260 |
| CNOT2 | 3643 | 1595410 | C | 8.95 | 0.043750 |
| KCNC2 | 3655 | 6582260 | C | -11.97 | 0.031940 |
| KCNC2 | 3658 | 1526821 | C | -15.91 | 0.003126 |
| KCNC2 | 3659 | 10785167 | T | -10.21 | 0.031070 |
| MTIF3 | 3691 | 9805452 | A | -18.01 | 0.001309 |
| MTIF3 | 3692 | 7334690 | A | -16.39 | 0.001186 |
| TRPC4 | 3725 | 7336008 | A | 9.27 | 0.017370 |
| TRPC4 | 3726 | 4943529 | C | 9.43 | 0.028090 |
| TRPC4 | 3727 | 7319926 | A | -12.39 | 0.010750 |
| TRPC4 | 3728 | 9532107 | A | -13.34 | 0.003121 |
| TRPC4 | 3732 | 1538146 | T | 13.40 | 0.015790 |
| GPC5 | 3777 | 1537027 | T | -10.08 | 0.036720 |
| GPC5 | 3780 | 2352195 | T | 11.44 | 0.010230 |
| GPC5 | 3781 | 2352199 | C | 11.68 | 0.009241 |
| GPC5 | 3782 | 7321674 | A | 11.68 | 0.009241 |
| GPC6 | 3804 | 3848066 | C | 12.06 | 0.018980 |
| GPC6 | 3805 | 4085921 | G | 13.48 | 0.005165 |
| GPC6 | 3806 | 1330620 | A | 14.43 | 0.002196 |
| GPC6 | 3807 | 9589816 | A | 13.61 | 0.002511 |
| GPC6 | 3810 | 7993501 | C | 12.29 | 0.003483 |
| NPAS3 | 3873 | 6571605 | A | 13.29 | 0.012170 |
| NPAS3 | 3874 | 10150948 | A | -11.54 | 0.003906 |
| NPAS3 | 3876 | 2183276 | G | 10.81 | 0.004129 |
| SAMD4A | 3913 | 709939 | C | -8.97 | 0.045570 |
| SAMD4A | 3914 | 3813402 | A | -11.64 | 0.032790 |
| PSMC1 | 3970 | 2236403 | T | -8.85 | 0.039710 |
| RPS6KA5 | 3972 | 1152423 | C | -10.75 | 0.026310 |
| BCL11B | 4000 | 2793321 | T | -16.67 | 0.001161 |
| BCL11B | 4001 | 1257446 | C | -17.33 | 0.000895 |
| BCL11B | 4002 | 1257416 | G | -15.04 | 0.007450 |
| BCL11B | 4003 | 2614463 | T | -9.46 | 0.023430 |
| C15ORF41 | 4042 | 16964046 | T | -28.30 | 0.001232 |
| C15ORF41 | 4043 | 16964049 | T | -28.01 | 0.001237 |
| C15ORF41 | 4044 | 1210459 | T | -13.82 | 0.018830 |
| C15ORF41 | 4045 | 16964103 | T | -28.01 | 0.001237 |
| C15ORF41 | 4046 | 16964104 | G | -28.01 | 0.001237 |
| C15ORF41 | 4047 | 17508461 | T | -28.01 | 0.001237 |
| GLDN | 4057 | 16964316 | A | 41.39 | 0.001055 |
| GLDN | 4059 | 16964319 | T | 41.39 | 0.001055 |
| GLDN | 4060 | 10163098 | G | 41.39 | 0.001055 |
| GLDN | 4061 | 12443092 | C | 41.39 | 0.001055 |
| A2BP1 | 4132 | 12922392 | T | -10.91 | 0.021710 |
| TMC5 | 4139 | 1985395 | C | -9.92 | 0.043410 |
| KIFC3 | 4149 | 874503 | A | -20.34 | 0.049910 |
| CRISPLD2 | 4214 | 2934477 | A | 10.01 | 0.022330 |
| ALOX12B | 4218 | 9914077 | G | 9.66 | 0.040330 |
| SDK2 | 4262 | 1846334 | A | 12.03 | 0.015780 |
| DNAH17 | 4268 | 9302876 | A | 10.92 | 0.033350 |
| PTPRM | 4291 | 1893224 | C | 21.61 | 0.036710 |
| CHST9 | 4317 | 4800797 | G | 12.16 | 0.012590 |
| RNF24 | 4393 | 6084530 | C | 9.94 | 0.026400 |
| FERMT1 | 4409 | 6085406 | A | 9.69 | 0.031810 |
| PLCB1 | 4416 | 6055678 | T | 12.32 | 0.023540 |
| MACROD2 | 4452 | 6043402 | A | -25.18 | 0.014090 |
| KIF16B | 4462 | 6043875 | C | -12.97 | 0.017340 |
| MIRN155 | 4553 | 9977556 | C | 14.13 | 0.007401 |
| ASPHD2 | 4573 | 16982107 | T | -22.28 | 0.018950 |
| TTLL1 | 4591 | 5759120 | T | -22.96 | 0.045150 |
| TTLL1 | 4594 | 135001 | C | -9.57 | 0.027900 |
| TTLL1 | 4595 | 135002 | C | -9.57 | 0.027900 |
| EFCAB6 | 4598 | 9614382 | G | -10.77 | 0.037170 |
| EFCAB6 | 4600 | 2374773 | A | -11.83 | 0.016230 |
| EFCAB6 | 4605 | 137794 | A | 22.20 | 0.001550 |
| EFCAB6 | 4608 | 137821 | G | 13.40 | 0.015660 |
| EFCAB6 | 4613 | 5764302 | G | 18.51 | 0.003064 |
| EFCAB6 | 4614 | 5764310 | C | 18.29 | 0.002464 |

| **TABLE 2: Alleles Influencing Response to Risperidone** | | | | | |
|---|---|---|---|---|---|
| **Gene** | **Seq ID** | **NCBI RS#** | **Allele** | **Beta (PANSS)** | **P** |
| AGBL4-C1ORF165 | 27 | 1934395 | G | -16.73 | 0.0003 |
| AGBL4-C1ORF165 | 30 | 1934368 | C | -15.54 | 0.0012 |
| AGBL4-C1ORF165 | 32 | 5017079 | T | -17.26 | 0.0015 |
| AGBL4-C1ORF165 | 34 | 3121522 | A | -11.55 | 0.0067 |
| AGBL4-C1ORF165 | 35 | 12043418 | T | -10.43 | 0.0180 |
| AGBL4-C1ORF165 | 36 | 2355693 | T | -15.94 | 0.0016 |
| SYT6 | 79 | 500739 | T | -9.31 | 0.0175 |
| SLC22A15 | 85 | 7547928 | T | 9.84 | 0.0242 |
| SLC22A15 | 86 | 2051060 | C | 9.84 | 0.0242 |
| RALGPS2 | 131 | 4652319 | T | 8.61 | 0.0445 |
| KCNK2 | 140 | 10779634 | T | 8.49 | 0.0478 |
| USH2A | 169 | 700024 | G | 13.89 | 0.0332 |
| ESRRG | 175 | 10495027 | A | -11.08 | 0.0345 |
| SLC35F3 | 189 | 480993 | T | -8.61 | 0.0305 |
| RYR2 | 211 | 651250 | C | -11.65 | 0.0071 |
| KCNF1 | 287 | 16856887 | T | -10.77 | 0.0096 |
| PLEKHH2 | 360 | 12621654 | T | 12.51 | 0.0072 |
| C2ORF34 | 366 | 1067402 | T | -10.97 | 0.0289 |
| C2ORF34 | 367 | 698792 | A | -10.07 | 0.0428 |
| C2ORF34 | 375 | 11679997 | T | -17.19 | 0.0050 |
| EPAS1 | 392 | 4953340 | C | 8.83 | 0.0194 |
| CCDC85A | 428 | 1030091 | C | 15.59 | 0.0367 |
| CTNNA2 | 445 | 2566542 | C | 10.44 | 0.0477 |
| CTNNA2 | 452 | 1319241 | A | -11.61 | 0.0458 |
| CTNNA2 | 455 | 1443895 | A | -7.74 | 0.0479 |
| CTNNA2 | 469 | 731359 | C | 8.22 | 0.0347 |
| INPP4A | 474 | 17032120 | T | 10.14 | 0.0148 |
| INPP4A | 475 | 2278206 | C | 8.72 | 0.0332 |
| NXPH2 | 498 | 10928662 | T | 12.92 | 0.0120 |
| LRP1B | 511 | 1949871 | C | -7.89 | 0.0442 |
| ARHGAP15 | 538 | 11895997 | A | -8.99 | 0.0195 |
| ARHGAP15 | 541 | 6719609 | A | 12.89 | 0.0112 |
| ARHGAP15 | 543 | 9677486 | G | 12.52 | 0.0140 |
| ARHGAP15 | 545 | 7605182 | C | 12.36 | 0.0200 |
| ARHGAP15 | 546 | 17814502 | A | 12.14 | 0.0187 |
| KIF5C | 564 | 6755213 | A | 15.31 | 0.0185 |
| FMNL2 | 588 | 2272535 | G | -13.74 | 0.0376 |
| SCN3A | 615 | 10930148 | G | -9.99 | 0.0333 |
| SCN3A | 618 | 11677254 | C | -10.12 | 0.0125 |
| PDE1A | 638 | 2623422 | G | -9.80 | 0.0371 |
| PDE1A | 647 | 7601804 | T | -10.61 | 0.0400 |
| PARD3B | 664 | 1100393 | G | 10.79 | 0.0134 |
| IRS1 | 688 | 2288587 | C | 24.29 | 0.0270 |
| IRS1 | 689 | 16822570 | G | 24.29 | 0.0270 |
| COL4A3 | 710 | 11681636 | C | -11.42 | 0.0034 |
| SAG | 731 | 3792096 | T | -11.29 | 0.0093 |
| CNTN6 | 734 | 4362705 | A | 9.55 | 0.0285 |
| CNTN6 | 735 | 6798321 | C | 8.70 | 0.0271 |
| CNTN6 | 736 | 11925058 | C | 8.74 | 0.0412 |
| CNTN6 | 752 | 3915436 | A | -12.04 | 0.0034 |
| CNTN4 | 756 | 1523320 | C | -9.80 | 0.0174 |
| CNTN4 | 760 | 1153486 | T | -10.35 | 0.0368 |
| CNTN4 | 769 | 9823500 | C | 13.42 | 0.0087 |
| ITPR1 | 774 | 304073 | G | -11.23 | 0.0042 |
| ITPR1 | 775 | 304038 | G | -7.95 | 0.0479 |
| DAZL | 800 | 12233472 | G | 9.07 | 0.0376 |
| DAZL | 803 | 13064112 | G | 8.86 | 0.0446 |
| ULK4 | 847 | 1795346 | C | 19.72 | 0.0143 |
| GPX1 | 855 | 9814873 | G | 14.07 | 0.0013 |
| GPX1 | 856 | 1801143 | T | 14.07 | 0.0013 |
| GPX1 | 857 | 9827708 | G | 13.53 | 0.0022 |
| GPX1 | 858 | 2271961 | C | -8.06 | 0.0278 |
| GPX1 | 859 | 2352974 | T | -7.77 | 0.0396 |
| CACNA2D3 | 873 | 17054586 | C | 13.91 | 0.0260 |
| ERC2 | 881 | 7617962 | T | 13.09 | 0.0069 |
| ERC2 | 882 | 1993539 | T | 17.98 | 0.0004 |
| ERC2 | 883 | 9810436 | A | 9.80 | 0.0301 |
| ERC2 | 886 | 7614271 | G | 19.29 | 0.0012 |
| ERC2 | 887 | 4974168 | C | 15.79 | 0.0084 |
| ERC2 | 889 | 2316481 | A | 18.68 | 0.0171 |
| IL17RD | 905 | 747089 | T | 12.09 | 0.0073 |
| FLIT | 927 | 9858223 | A | 11.91 | 0.0029 |
| FHIT | 928 | 4688167 | C | 11.91 | 0.0029 |
| PRICKLE2 | 957 | 17069879 | A | -12.65 | 0.0047 |
| MAGI1 | 972 | 4234687 | A | 11.95 | 0.0219 |
| MAGI1 | 975 | 7612644 | A | 8.94 | 0.0493 |
| GBE1 | 989 | 17019088 | T | 12.40 | 0.0161 |
| GBE1 | 991 | 13324454 | G | -11.00 | 0.0147 |
| GBE1 | 994 | 9871474 | T | -9.70 | 0.0394 |
| EPHA6 | 1007 | 6439203 | G | 16.23 | 0.0104 |
| EPHA6 | 1009 | 2317212 | A | 10.35 | 0.0420 |
| PLCXD2 | 1011 | 6788543 | C | 8.27 | 0.0455 |
| KALRN | 1044 | 333354 | A | -8.82 | 0.0406 |
| CPNE4 | 1048 | 1385969 | C | -10.36 | 0.0283 |
| SPSB4 | 1082 | 11915229 | G | 16.56 | 0.0428 |
| SERPTN11 | 1091 | 13090569 | A | -10.04 | 0.0277 |
| SLC7A14 | 1093 | 6788596 | G | 11.57 | 0.0108 |
| SLC7A14 | 1096 | 17289598 | G | 16.07 | 0.0156 |
| TNIK | 1099 | 4955765 | G | 7.70 | 0.0422 |
| TNIK | 1102 | 17217552 | G | -29.57 | 0.0346 |
| IL1RAP | 1141 | 3773999 | C | 10.31 | 0.0280 |
| 1L1RAP | 1142 | 9990231 | C | 9.40 | 0.0467 |
| SLC2A9 | 1156 | 2139243 | C | -9.60 | 0.0425 |
| SLIT2 | 1185 | 7655084 | G | 9.48 | 0.0438 |
| PCDH7 | 1199 | 10517215 | A | -13.99 | 0.0252 |
| NPFFR2 | 1235 | 11936367 | G | -9.41 | 0.0401 |
| NPFFR2 | 1248 | 9790741 | G | 8.50 | 0.0480 |
| PAPSS1 | 1308 | 4956020 | T | 11.46 | 0.0266 |
| PAPSS1 | 1309 | 12502059 | A | 11.82 | 0.0226 |
| COL25A1 | 1321 | 1563004 | G | 14.34 | 0.0420 |
| ANK2 | 1348 | 362496 | G | -13.64 | 0.0032 |
| GPR103 | 1382 | 2013332 | A | -10.22 | 0.0382 |
| MAML3 | 1392 | 10024138 | T | 8.89 | 0.0486 |
| MAML3 | 1393 | 12512994 | T | 8.99 | 0.0500 |
| FSTL5 | 1460 | 2082669 | G | 14.31 | 0.0077 |
| TLL1 | 1479 | 3756016 | C | -10.74 | 0.0146 |
| TLL1 | 1480 | 2292083 | A | -10.46 | 0.0167 |
| ENPP6 | 1527 | 6830766 | G | 8.79 | 0.0263 |
| ENPP6 | 1528 | 10020224 | G | 11.04 | 0.0089 |
| SEMA5A | 1541 | 11134354 | A | 9.74 | 0.0454 |
| DNAH5 | 1553 | 7715811 | T | 10.04 | 0.0404 |
| DNAH5 | 1554 | 1502050 | G | 9.91 | 0.0456 |
| CDH10 | 1575 | 3822429 | T | -8.78 | 0.0313 |
| SLC45A2 | 1579 | 35389 | G | 16.41 | 0.0135 |
| SLC1A3 | 1586 | 6451305 | C | 10.80 | 0.0389 |
| ITGA1 | 1615 | 1047481 | G | 8.75 | 0.0404 |
| TNPO1 | 1644 | 2548331 | A | -10.68 | 0.0039 |
| FCHO2 | 1645 | 478575 | T | 9.86 | 0.0094 |
| FCHO2 | 1647 | 6895786 | G | 9.44 | 0.0173 |
| FCHO2 | 1648 | 7727222 | C | 9.44 | 0.0173 |
| FCHO2 | 1649 | 7709974 | A | 9.51 | 0.0184 |
| VCAN | 1669 | 309559 | T | 9.16 | 0.0447 |
| VCAN | 1670 | 160277 | A | -8.27 | 0.0453 |
| CAST | 1694 | 754615 | C | 8.91 | 0.0479 |
| FBXL17 | 1701 | 288172 | C | -13.10 | 0.0099 |
| FBXL17 | 1702 | 288144 | A | -13.10 | 0.0099 |
| FBXL17 | 1712 | 1849012 | A | -13.48 | 0.0085 |
| FBXL17 | 1713 | 17161232 | T | -12.07 | 0.0179 |
| FBXL17 | 1714 | 6594298 | A | -12.07 | 0.0179 |
| KCNN2 | 1729 | 6889371 | G | -14.35 | 0.0154 |
| KCNN2 | 1730 | 4435855 | A | 9.87 | 0.0478 |
| SEMA6A | 1732 | 26591 | A | 8.14 | 0.0346 |
| SEMA6A | 1735 | 254231 | G | -8.30 | 0.0446 |
| ODZ2 | 1799 | 1422421 | T | -11.09 | 0.0130 |
| NEDD9 | 1815 | 3798729 | T | -15.48 | 0.0046 |
| PHACTR1 | 1817 | 6458439 | C | 9.22 | 0.0253 |
| JARID2 | 1827 | 707833 | C | -10.12 | 0.0155 |
| PPP2R5D | 1882 | 6901782 | C | 12.17 | 0.0380 |
| RIMS1 | 1907 | 1015945 | G | 9.23 | 0.0430 |
| RIMS1 | 1908 | 9351903 | G | -9.48 | 0.0287 |
| RIMS1 | 1910 | 10498882 | G | -8.78 | 0.0330 |
| RIMS1 | 1912 | 1339226 | G | -12.54 | 0.0079 |
| RIMS1 | 1913 | 1546914 | T | -10.86 | 0.0165 |
| TRDN | 1935 | 13190807 | C | 10.08 | 0.0325 |
| TRDN | 1939 | 1431284 | G | -8.49 | 0.0331 |
| TRDN | 1940 | 17686735 | G | -8.91 | 0.0282 |
| PDE7B | 1987 | 6938424 | T | 27.27 | 0.0252 |
| PLAGL1 | 1992 | 9376803 | G | 9.95 | 0.0087 |
| PLAGL1 | 1993 | 9484836 | A | 12.76 | 0.0044 |
| UTRN | 1997 | 601873 | A | 9.86 | 0.0196 |
| UTRN | 2000 | 9390166 | G | 12.61 | 0.0018 |
| UTRN | 2001 | 7765923 | A | 11.35 | 0.0097 |
| PACRG | 2051 | 742956 | G | 10.81 | 0.0086 |
| PACRG | 2053 | 761625 | A | -9.55 | 0.0303 |
| PDE10A | 2057 | 1706123 | C | 9.07 | 0.0198 |
| CARD11 | 2069 | 4722277 | T | -9.49 | 0.0333 |
| SDK1 | 2072 | 11766511 | G | 8.33 | 0.0324 |
| SDK1 | 2075 | 649186 | C | -11.40 | 0.0470 |
| SDK1 | 2082 | 1044701 | A | -8.12 | 0.0443 |
| ETV1 | 2098 | 4721291 | C | 10.36 | 0.0126 |
| ETV1 | 2099 | 2237295 | G | -11.17 | 0.0181 |
| STK31 | 2110 | 10264967 | T | -12.28 | 0.0397 |
| CREB5 | 2125 | 4722864 | A | -11.11 | 0.0436 |
| SCRN1 | 2130 | 6976421 | A | -13.32 | 0.0059 |
| SCRN1 | 2131 | 6976789 | T | -10.14 | 0.0331 |
| SCRN1 | 2132 | 4662 | T | -10.14 | 0.0331 |
| PLEKHA8 | 2134 | 10275475 | C | -10.28 | 0.0454 |
| PDE1C | 2156 | 7790003 | C | -7.65 | 0.0332 |
| BMPER | 2164 | 6975236 | T | -10.77 | 0.0092 |
| VPS41 | 2173 | 2286105 | A | 16.65 | 0.0095 |
| VPS41 | 2176 | 3801140 | C | 10.11 | 0.0201 |
| VPS41 | 2177 | 3801139 | T | 10.11 | 0.0201 |
| VPS41 | 2180 | 12674115 | G | 14.84 | 0.0052 |
| GRB10 | 2220 | 2074777 | G | 8.95 | 0.0190 |
| GRB10 | 2221 | 757774 | G | 13.84 | 0.0021 |
| GRB10 | 2222 | 6593164 | C | 13.84 | 0.0021 |
| GRB10 | 2223 | 10281065 | A | 13.84 | 0.0021 |
| GRB10 | 2225 | 6943901 | G | 13.84 | 0.0021 |
| GRB10 | 2226 | 6971834 | T | 13.84 | 0.0021 |
| WBSCR17 | 2241 | 740099 | C | 13.86 | 0.0052 |
| WBSCR17 | 2243 | 7794574 | A | 14.82 | 0.0070 |
| HIP1 | 2260 | 1015877 | C | -9.21 | 0.0165 |
| HIP1 | 2261 | 2696213 | T | -13.55 | 0.0006 |
| HIP1 | 2263 | 2253020 | G | -12.68 | 0.0013 |
| HIP1 | 2264 | 11770686 | T | 11.72 | 0.0028 |
| MAGI2 | 2275 | 38110 | C | -9.60 | 0.0162 |
| MAGI2 | 2285 | 2191806 | C | -11.70 | 0.0465 |
| CACNA2D1 | 2313 | 12531682 | T | 8.70 | 0.0412 |
| CACNA2D1 | 2315 | 7788848 | A | 8.92 | 0.0360 |
| CACNA2D1 | 2317 | 6952949 | G | -9.80 | 0.0201 |
| PCLO | 2319 | 10232216 | T | 10.76 | 0.0080 |
| SEMA3E | 2336 | 2713151 | T | -8.37 | 0.0470 |
| SEMA3E | 2337 | 2709890 | A | -8.37 | 0.0470 |
| ADAM22 | 2355 | 1688886 | G | -8.72 | 0.0436 |
| ADAM22 | 2356 | 2282948 | C | -8.42 | 0.0456 |
| ADAM22 | 2357 | 2282949 | C | -8.42 | 0.0456 |
| KCND2 | 2413 | 4727911 | G | -10.75 | 0.0226 |
| KCND2 | 2414 | 7795646 | G | -10.75 | 0.0226 |
| GRM8 | 2442 | 6950264 | G | 10.70 | 0.0116 |
| GRM8 | 2452 | 1361956 | A | 9.55 | 0.0178 |
| EXOC4 | 2467 | 10247014 | T | 8.60 | 0.0370 |
| EXOC4 | 2468 | 10279261 | G | 8.90 | 0.0241 |
| EXOC4 | 2471 | 4731992 | A | 11.91 | 0.0148 |
| DGKI | 2475 | 16874961 | C | 10.64 | 0.0245 |
| DGK1 | 2476 | 12535157 | A | 9.39 | 0.0383 |
| PTPRN2 | 2515 | 10263303 | T | 11.16 | 0.0202 |
| CSMD1 | 2539 | 12541278 | T | -9.41 | 0.0194 |
| MCPH1 | 2568 | 2959799 | C | -8.76 | 0.0245 |
| MCPH1 | 2570 | 2454517 | C | -9.94 | 0.0121 |
| MCPH1 | 2573 | 3020264 | A | -9.65 | 0.0221 |
| DLC1 | 2576 | 569510 | A | 13.54 | 0.0026 |
| DLC1 | 2577 | 2203838 | C | 13.22 | 0.0042 |
| DLC1 | 2578 | 536147 | G | 11.16 | 0.0130 |
| SLC7A2 | 2601 | 7002951 | G | 15.96 | 0.0059 |
| PSD3 | 2606 | 11990818 | G | -8.48 | 0.0438 |
| UNC5D | 2644 | 3015796 | G | 9.15 | 0.0321 |
| LYN | 2676 | 17812677 | T | -12.56 | 0.0137 |
| LYN | 2677 | 16922508 | C | -12.56 | 0.0137 |
| LYN | 2679 | 6988853 | A | -11.16 | 0.0170 |
| LYN | 2681 | 2668003 | C | -9.98 | 0.0335 |
| NKAIN3 | 2694 | 896489 | G | -10.97 | 0.0132 |
| NKAIN3 | 2713 | 10504356 | G | -16.39 | 0.0144 |
| NKAIN3 | 2716 | 4739023 | C | 10.11 | 0.0115 |
| NKAIN3 | 2718 | 1350059 | C | 9.86 | 0.0148 |
| DEPDC2 | 2725 | 4737868 | A | 9.72 | 0.0154 |
| DEPDC2 | 2728 | 1434764 | A | -8.84 | 0.0352 |
| KCNB2 | 2739 | 349350 | G | -9.58 | 0.0359 |
| FER1L6 | 2839 | 10094533 | C | 14.91 | 0.0079 |
| FER1L6 | 2842 | 7832499 | C | 15.37 | 0.0093 |
| FER1L6 | 2843 | 9643188 | A | 15.84 | 0.0100 |
| FER1L6 | 2844 | 13254498 | A | 14.73 | 0.0143 |
| FER1L6 | 2846 | 13269044 | G | 14.53 | 0.0164 |
| MTSS1 | 2856 | 6470298 | A | 16.81 | 0.0413 |
| ADCY8 | 2897 | 17327900 | A | -13.26 | 0.0424 |
| ST3GAL1 | 2903 | 2978015 | T | 7.73 | 0.0476 |
| COL22A1 | 2909 | 7844188 | G | 9.41 | 0.0499 |
| SMARCA2 | 2927 | 10757205 | G | -18.00 | 0.0334 |
| SLC1A1 | 2937 | 159914 | C | 9.23 | 0.0417 |
| KIAA1797 | 2966 | 7025868 | G | -10.35 | 0.0382 |
| KIAA1797 | 2967 | 6475483 | T | -10.47 | 0.0443 |
| KIAA1797 | 2973 | 6475490 | G | 8.73 | 0.0267 |
| KIAA1797 | 2974 | 6475491 | G | 7.76 | 0.0481 |
| KIAA1797 | 2977 | 7025851 | G | 9.13 | 0.0222 |
| KIAA1797 | 2979 | 4977881 | A | -9.69 | 0.0307 |
| APBA1 | 3004 | 11139084 | A | 8.90 | 0.0254 |
| APBA1 | 3005 | 10867730 | T | 8.90 | 0.0254 |
| MAMDC2 | 3008 | 3015182 | C | 19.84 | 0.0102 |
| MAMDC2 | 3009 | 2975866 | A | 19.84 | 0.0102 |
| MAMDC2 | 3010 | 3015189 | G | 19.84 | 0.0102 |
| MAMDC2 | 3011 | 3015213 | A | 19.84 | 0.0102 |
| TMC1 | 3034 | 2487471 | T | 12.41 | 0.0181 |
| TMC1 | 3036 | 1663740 | T | 10.93 | 0.0398 |
| TMC1 | 3037 | 1796991 | G | 10.64 | 0.0419 |
| TMC1 | 3038 | 2589609 | T | 10.64 | 0.0419 |
| PCSKS | 3052 | 2789608 | G | -13.11 | 0.0012 |
| ZNF169 | 3098 | 7037298 | T | -13.98 | 0.0138 |
| GABBR2 | 3105 | 16914811 | A | 16.40 | 0.0163 |
| GABBR2 | 3106 | 3780439 | G | 9.53 | 0.0342 |
| CDK5RAP2 | 3136 | 3780679 | G | 27.19 | 0.0129 |
| NTNG2 | 3176 | 3739930 | A | -14.54 | 0.0221 |
| NTNG2 | 3177 | 3739929 | G | -13.48 | 0.0315 |
| NPDC1 | 3202 | 4880195 | C | 9.93 | 0.0436 |
| PITRM1 | 3219 | 3814596 | G | 11.27 | 0.0236 |
| CACNB2 | 3240 | 10828726 | G | -9.12 | 0.0263 |
| JMJD1C | 3301 | 9629895 | A | 10.26 | 0.0222 |
| CDH23 | 3306 | 7896061 | C | -10.49 | 0.0479 |
| KCNMA1 | 3334 | 2619636 | A | 10.03 | 0.0109 |
| KCNMA1 | 3335 | 2719986 | T | 8.31 | 0.0369 |
| SORBS1 | 3368 | 564370 | A | 9.22 | 0.0329 |
| ATRNL1 | 3413 | 7922670 | C | -7.08 | 0.0482 |
| STIM1 | 3459 | 10835337 | T | 10.45 | 0.0270 |
| STIM1 | 3461 | 7924984 | A | 9.80 | 0.0372 |
| STIM1 | 3465 | 2959068 | T | 8.64 | 0.0443 |
| STIM1 | 3466 | 1372805 | A | 8.64 | 0.0443 |
| SPON1 | 3499 | 1528668 | C | -9.76 | 0.0341 |
| SPONI | 3512 | 3935919 | C | -12.74 | 0.0051 |
| SPONI | 3513 | 4757244 | A | -10.81 | 0.0129 |
| TMEM16B | 3606 | 11063913 | G | -10.79 | 0.0279 |
| TMEM16B | 3607 | 3741903 | G | -11.41 | 0.0369 |
| LOC729025 | 3628 | 11056858 | T | 14.05 | 0.0421 |
| LOC729025 | 3629 | 11056862 | A | 14.05 | 0.0421 |
| PIK3C2G | 3631 | 4764383 | G | -10.17 | 0.0186 |
| CNOT2 | 3648 | 10506586 | A | 15.30 | 0.0359 |
| CNOT2 | 3650 | 3817486 | G | 15.30 | 0.0359 |
| GAS2L3 | 3680 | 35711 | G | -8.17 | 0.0381 |
| GAS2L3 | 3681 | 35706 | A | -7.81 | 0.0441 |
| UBL3 | 3696 | 17502901 | G | - 9.52 | 0.0364 |
| PCDH20 | 3737 | 17059750 | T | 25.34 | 0.0381 |
| LMO7 | 3745 | 7326714 | T | 8.95 | 0.0234 |
| LMO7 | 3748 | 2273996 | T | 10.31 | 0.0255 |
| LMO7 | 3750 | 920701 | C | 10.01 | 0.0159 |
| LMO7 | 3751 | 9530473 | G | 11.42 | 0.0048 |
| LMO7 | 3752 | 7986131 | T | 12.23 | 0.0072 |
| GPC5 | 3770 | 9652104 | A | 13.42 | 0.0357 |
| GPC6 | 3804 | 3848066 | C | -9.52 | 0.0232 |
| GPC6 | 3813 | 9524268 | T | -9.69 | 0.0373 |
| NALCN | 3836 | 9300662 | G | -9.36 | 0.0232 |
| NALCN | 3837 | 3916910 | C | 9.28 | 0.0411 |
| SLC25A21 | 3878 | 2073248 | C | 21.08 | 0.0004 |
| SLC25A21 | 3879 | 12185044 | G | 17.59 | 0.0033 |
| LRFN5 | 3894 | 862480 | T | 10.55 | 0.0117 |
| SAMD4A | 3911 | 1307289 | G | -10.32 | 0.0307 |
| SAMD4A | 3912 | 1212968 | C | -10.73 | 0.0405 |
| SAMD4A | 3917 | 2057369 | A | 15.81 | 0.0011 |
| SAMD4A | 3918 | 17128004 | A | 15.81 | 0.0011 |
| SAMD4A | 3919 | 10483639 | C | 14.84 | 0.0021 |
| PPP2R5E | 3923 | 4899113 | C | -10.38 | 0.0337 |
| RGS6 | 3953 | 917426 | G | -8.66 | 0.0294 |
| RPS6KA5 | 3976 | 1286148 | C | -10.74 | 0.0234 |
| ATP10A | 4011 | 2930630 | C | 15.19 | 0.0036 |
| ATP10A | 4014 | 4906750 | C | 10.98 | 0.0198 |
| C15ORF41 | 4045 | 16964103 | T | -17.91 | 0.0344 |
| C15ORF41 | 4047 | 17508461 | T | -17.91 | 0.0344 |
| ME1S2 | 4048 | 716218 | A | 8.24 | 0.0431 |
| TBC1D2B | 4094 | 12593545 | G | -11.85 | 0.0090 |
| ARNT2 | 4105 | 7180510 | G | 9.09 | 0.0407 |
| A2BP1 | 4113 | 17822719 | A | -11.25 | 0.0084 |
| GDE1 | 4143 | 3751821 | T | 13.88 | 0.0376 |
| MPHOSPH6 | 4164 | 8047857 | C | 11.70 | 0.0380 |
| CRISPLD2 | 4215 | 8049317 | G | 9.70 | 0.0146 |
| ALOX12B | 4218 | 9914077 | G | 11.27 | 0.0096 |
| CA10 | 4226 | 11867674 | G | -7.32 | 0.0384 |
| CA10 | 4227 | 8076197 | G | 7.32 | 0.0384 |
| CA10 | 4228 | 8076496 | G | 7.32 | 0.0384 |
| CA10 | 4230 | 4794295 | T | -7.32 | 0.0384 |
| DNAH17 | 4264 | 16971269 | C | 8.81 | 0.0441 |
| DNAH17 | 4270 | 9907431 | A | -8.20 | 0.0466 |
| LOXHD1 | 4318 | 11082530 | T | -8.69 | 0.0452 |
| CCBE1 | 4337 | 12969965 | A | -14.12 | 0.0041 |
| CCBE1 | 4343 | 1560299 | T | 10.34 | 0.0082 |
| CCBE1 | 4344 | 4608425 | A | 9.12 | 0.0207 |
| DOK6 | 4374 | 7243624 | T | 8.19 | 0.0338 |
| LDLR | 4384 | 2116897 | T | 14.54 | 0.0065 |
| PRNT | 4396 | 2236284 | G | -13.88 | 0.0016 |
| PRNT | 4405 | 968433 | C | -13.43 | 0.0019 |
| PRNT | 4406 | 6084875 | A | -10.99 | 0.0132 |
| PRNT | 4407 | 6084876 | T | -10.77 | 0.0144 |
| FERMT1 | 4411 | 8121939 | G | 10.70 | 0.0173 |
| MACROD2 | 4457 | 6043648 | C | -10.48 | 0.0059 |
| KIF16B | 4476 | 6034509 | G | 8.63 | 0.0294 |
| KIF16B | 4477 | 6135769 | A | 8.63 | 0.0294 |
| KIF16B | 4478 | 6044044 | A | 8.63 | 0.0294 |
| KIF16B | 4480 | 6135784 | G | 8.63 | 0.0294 |
| KIF16B | 4481 | 720592 | T | 8.63 | 0.0294 |
| MAPRE1 | 4491 | 2070090 | T | 14.58 | 0.0015 |
| PTPRT | 4503 | 6065484 | C | -9.71 | 0.0339 |
| PTPRT | 4504 | 230153 | T | -10.49 | 0.0439 |
| PTPRT | 4507 | 6065525 | A | -8.79 | 0.0444 |
| PTPRT | 4511 | 2223540 | T | -9.31 | 0.0483 |
| SNAI1 | 4528 | 6020187 | G | -9.54 | 0.0367 |
| PDE9A | 4562 | 2269145 | T | 10.22 | 0.0117 |
| PDE9A | 4564 | 12626774 | C | -8.69 | 0.0391 |
| PDE9A | 4565 | 2284976 | A | 10.74 | 0.0106 |
| ARVCF | 4570 | 887203 | A | 34.48 | 0.0147 |
| PACSIN2 | 4590 | 2072883 | G | 10.39 | 0.0126 |
| TTLL1 | 4592 | 1052160 | T | -10.20 | 0.0133 |

| **TABLE 3: Alleles Influencing Response to Quetiapine** | | | | | |
|---|---|---|---|---|---|
| **Gene** | **Seq ID** | **NCBI RS#** | **Allele** | **Beta (PANSS)** | **P** |
| SCP2 | 39 | 7528867 | A | 28.75 | 0.00361 |
| SCP2 | 40 | 11206061 | C | 28.75 | 0.00361 |
| SCP2 | 41 | 7546714 | T | 14.16 | 0.03148 |
| SCP2 | 42 | 6588458 | A | 14.87 | 0.03712 |
| SCP2 | 44 | 11588200 | T | 20.34 | 0.01544 |
| SCP2 | 45 | 2896792 | T | 19.99 | 0.01707 |
| SCP2 | 46 | 17107767 | C | 19.99 | 0.01707 |
| SCP2 | 47 | 12096165 | G | 19.99 | 0.01707 |
| LRP8 | 50 | 869987 | T | -9.58 | 0.03659 |
| LRP8 | 51 | 869988 | C | -9.58 | 0.03659 |
| PRKACB | 68 | 3915908 | T | -10.70 | 0.04731 |
| PRKACB | 69 | 3903924 | G | 10.57 | 0.04814 |
| PRKACB | 74 | 2642186 | G | 10.31 | 0.03302 |
| OLFML2B | 110 | 7537366 | T | 14.04 | 0.01786 |
| ESRRG | 183 | 12033378 | A | 12.78 | 0.03923 |
| RYR2 | 215 | 4659819 | T | -13.39 | 0.00224 |
| FMN2 | 251 | 882869 | G | -10.78 | 0.04162 |
| KLHL29 | 299 | 9261 | T | 12.04 | 0.01064 |
| KLHL29 | 300 | 12478701 | C | 11.64 | 0.01200 |
| KLHL29 | 301 | 12478744 | A | -10.03 | 0.03478 |
| DPYSL5 | 311 | 12618836 | A | -14.78 | 0.00154 |
| DPYSL5 | 312 | 3769138 | G | -8.96 | 0.04317 |
| CRIM1 | 327 | 4670549 | T | -9.16 | 0.04585 |
| CDC42EP3 | 340 | 4670745 | G | -10.78 | 0.03597 |
| CDC42EP3 | 341 | 10694 | T | -13.56 | 0.02699 |
| SLC8A1 | 345 | 417614 | A | -10.33 | 0.02748 |
| SLC8A1 | 347 | 11885401 | C | -13.70 | 0.03826 |
| C2ORF34 | 363 | 1584885 | C | -8.90 | 0.04586 |
| PRKCE | 382 | 4953288 | T | -10.21 | 0.02069 |
| ACYP2 | 415 | 6744662 | T | 16.39 | 0.00683 |
| ACYP2 | 416 | 10439478 | C | 13.05 | 0.01119 |
| CCDC85A | 422 | 8179803 | T | -12.78 | 0.00709 |
| CCDC85A | 425 | 2111470 | C | -13.67 | 0.01014 |
| CCDC85A | 426 | 1990758 | C | -20.48 | 0.00591 |
| CCDC85A | 427 | 10460558 | A | -13.67 | 0.01014 |
| OTX1 | 431 | 11125946 | A | 14.15 | 0.00223 |
| LRP1B | 516 | 10178963 | A | 9.55 | 0.03033 |
| KYNU | 530 | 7561260 | G | -12.92 | 0.04210 |
| KYNU | 532 | 1371516 | C | -15.60 | 0.00474 |
| KYNU | 533 | 6430001 | A | -15.14 | 0.00433 |
| KYNU | 534 | 3768844 | C | -16.13 | 0.01182 |
| FMNL2 | 583 | 16823809 | C | -13.92 | 0.04578 |
| PLA2R1 | 603 | 6432570 | T | 10.93 | 0.02808 |
| PLA2R1 | 612 | 2175416 | T | -10.05 | 0.02269 |
| PLA2R1 | 614 | 16844715 | T | -10.05 | 0.02269 |
| CERKL | 634 | 1967351 | C | 9.94 | 0.04738 |
| NRP2 | 668 | 849540 | T | 9.96 | 0.04191 |
| COL4A4 | 694 | 2276593 | A | 15.58 | 0.00053 |
| COL4A4 | 695 | 10166736 | A | 14.86 | 0.00088 |
| COL4A4 | 697 | 4566357 | A | -10.71 | 0.02458 |
| DNER | 712 | 207671 | G | 11.26 | 0.01097 |
| DNER | 713 | 6733289 | G | 12.62 | 0.01409 |
| TRIP12 | 724 | 6707272 | T | 10.22 | 0.04130 |
| TRIP12 | 725 | 6687 | T | -15.08 | 0.02818 |
| TRIP12 | 726 | 1372086 | C | -12.27 | 0.04566 |
| CNTN6 | 750 | 155390 | G | 13.19 | 0.00341 |
| CNTN6 | 751 | 2291100 | C | 11.89 | 0.01478 |
| CNTN6 | 752 | 3915436 | A | -11.03 | 0.00983 |
| CNTN4 | 762 | 1502582 | T | 12.72 | 0.04388 |
| SLC6A6 | 795 | 4684216 | G | -25.78 | 0.00067 |
| ULK4 | 838 | 7627972 | A | -8.82 | 0.03737 |
| ULK4 | 840 | 6794510 | T | -8.52 | 0.04954 |
| ULK4 | 842 | 11129908 | A | -9.91 | 0.02216 |
| CACNA2D3 | 871 | 2141034 | A | -14.13 | 0.02467 |
| CACNA2D3 | 872 | 6804643 | A | -12.38 | 0.04995 |
| FLNB | 912 | 7632986 | T | -9.96 | 0.04182 |
| FLNB | 913 | 7622687 | A | -13.23 | 0.00550 |
| PTPRG | 940 | 9813294 | A | 10.41 | 0.01004 |
| CADPS | 942 | 1376917 | C | -21.12 | 0.03049 |
| FAM19A1 | 982 | 6801913 | A | 10.15 | 0.03078 |
| FOXP1 | 985 | 2597312 | C | 9.79 | 0.03127 |
| FOXP1 | 987 | 1290499 | T | -16.99 | 0.01410 |
| STX19 | 1000 | 9810986 | T | 29.68 | 0.00262 |
| STXBP5L | 1036 | 2169302 | G | 14.91 | 0.04740 |
| RAB6B | 1061 | 6765093 | G | 10.16 | 0.03627 |
| EPHB1 | 1064 | 4955460 | C | -11.99 | 0.01041 |
| EPHB1 | 1065 | 185257 | T | -10.65 | 0.03061 |
| EPHB1 | 1066 | 9825380 | T | -10.80 | 0.03441 |
| EPHB1 | 1067 | 936323 | T | -10.08 | 0.04468 |
| EPHB1 | 1068 | 1870196 | T | -12.03 | 0.01370 |
| TNIK | 1097 | 6781167 | A | 26.27 | 0.00163 |
| TNIK | 1098 | 12486611 | T | 25.75 | 0.00178 |
| PLD1 | 1106 | 1316415 | G | -10.17 | 0.02517 |
| PLD1 | 1107 | 7626674 | C | 13.50 | 0.02594 |
| PLD1 | 1109 | 2178532 | A | 13.50 | 0.02594 |
| PCDH7 | 1199 | 10517215 | A | 12.34 | 0.04410 |
| PCDH7 | 1202 | 2310225 | A | 12.78 | 0.01941 |
| LIMCH1 | 1223 | 6821712 | C | -9.50 | 0.04144 |
| NPFFR2 | 1249 | 17775309 | G | 9.68 | 0.04822 |
| SHROOM3 | 1259 | 10022778 | G | 8.13 | 0.04828 |
| SCD5 | 1263 | 17006038 | C | 13.50 | 0.03407 |
| SCD5 | 1271 | 3897960 | A | -12.82 | 0.03552 |
| HERC3 | 1275 | 3796660 | A | -19.26 | 0.03462 |
| FAM13A1 | 1283 | 6814344 | G | -10.80 | 0.04153 |
| FAM13A1 | 1284 | 13131633 | T | -10.80 | 0.04153 |
| PAPSS1 | 1307 | 2672487 | A | -9.55 | 0.04604 |
| COL25A1 | 1315 | 17039326 | T | 13.56 | 0.01265 |
| COL25A1 | 1316 | 9996734 | A | 12.84 | 0.01446 |
| COL25A1 | 1334 | 987695 | G | 10.43 | 0.02112 |
| COL25A1 | 1335 | 7675657 | A | 9.44 | 0.04234 |
| ANK2 | 1350 | 29355 | G | -12.76 | 0.03517 |
| ANK2 | 1352 | 29319 | A | -12.67 | 0.03804 |
| CAMK2D | 1359 | 17046126 | T | 9.37 | 0.04666 |
| CAMK2D | 1361 | 1859148 | C | -11.78 | 0.02900 |
| CAMK2D | 1362 | 7675804 | T | -11.78 | 0.02900 |
| CAMK2D | 1363 | 10023113 | G | 19.04 | 0.01080 |
| MAML3 | 1392 | 10024138 | T | -9.54 | 0.04145 |
| POU4F2 | 1425 | 7680105 | G | -18.46 | 0.00196 |
| POU4F2 | 1428 | 6835151 | A | -17.97 | 0.00525 |
| POU4F2 | 1429 | 1394279 | G | -18.05 | 0.00551 |
| FSTL5 | 1452 | 4501178 | C | 13.98 | 0.03848 |
| FSTL5 | 1473 | 4691039 | T | 18.45 | 0.00375 |
| CASP3 | 1529 | 2696056 | G | -12.54 | 0.04554 |
| AHRR | 1531 | 2721004 | T | -9.04 | 0.04234 |
| AHRR | 1532 | 2721012 | G | -10.04 | 0.03557 |
| SLC1A3 | 1583 | 3776569 | G | -9.41 | 0.04957 |
| EGFLAM | 1596 | 2731979 | C | 14.60 | 0.03298 |
| ITGA1 | 1607 | 2169782 | T | 20.56 | 0.00163 |
| VCAN | 1668 | 2287926 | A | 15.56 | 0.01299 |
| VCAN | 1671 | 2445874 | T | 12.62 | 0.04828 |
| PJA2 | 1723 | 2963034 | A | 10.47 | 0.01709 |
| KCNN2 | 1726 | 1072922 | A | 11.72 | 0.02454 |
| KCNN2 | 1728 | 13163662 | A | 12.26 | 0.01715 |
| ADAMTS19 | 1769 | 4835975 | A | 11.67 | 0.01495 |
| ODZ2 | 1793 | 1529682 | G | -13.20 | 0.04506 |
| SLC22A23 | 1807 | 3813487 | A | 14.38 | 0.01646 |
| BRD2 | 1879 | 194677 | T | -26.51 | 0.01459 |
| ELOVL5 | 1888 | 2562898 | A | 12.58 | 0.02447 |
| RIMS1 | 1902 | 1010326 | A | -13.37 | 0.00906 |
| RIMS1 | 1905 | 2496495 | G | -8.96 | 0.04892 |
| RIMS1 | 1906 | 2250128 | C | -9.54 | 0.03816 |
| RIMS1 | 1908 | 9351903 | G | 10.79 | 0.01570 |
| RIMS1 | 1910 | 10498882 | G | 10.13 | 0.02668 |
| RIMS1 | 1911 | 2807531 | A | 9.63 | 0.04062 |
| STX11 | 1996 | 4896708 | A | -10.18 | 0.02725 |
| PDE10A | 2063 | 484594 | C | -13.01 | 0.03420 |
| SDK1 | 2070 | 10265630 | G | 10.87 | 0.03561 |
| SDK1 | 2071 | 1062213 | C | 10.61 | 0.04465 |
| SNX13 | 2101 | 10216315 | C | 9.58 | 0.03782 |
| STK31 | 2112 | 6957981 | G | 13.72 | 0.01342 |
| CREB5 | 2119 | 2237361 | C | 9.31 | 0.04837 |
| CREB5 | 2124 | 17669844 | C | 10.09 | 0.04720 |
| SCRN1 | 2129 | 9912 | T | -11.55 | 0.02516 |
| PDE1C | 2155 | 6952633 | G | -10.82 | 0.03590 |
| PDE1C | 2160 | 4551232 | A | -9.19 | 0.04338 |
| PDE1C | 2161 | 10225488 | A | -9.19 | 0.04338 |
| BMPER | 2163 | 11978741 | T | 10.68 | 0.04675 |
| ABCA13 | 2194 | 17132289 | T | 24.20 | 0.00500 |
| GRB10 | 2216 | 3779072 | G | 13.39 | 0.03125 |
| GRB10 | 2219 | 1978208 | G | 13.13 | 0.03586 |
| GRB10 | 2224 | 12536500 | T | 12.05 | 0.04430 |
| WBSCR17 | 2242 | 4585627 | T | 10.71 | 0.02053 |
| GTF2IRD1 | 2252 | 2267834 | G | -15.82 | 0.00219 |
| MAGI2 | 2278 | 1918930 | C | -19.68 | 0.00211 |
| MAGI2 | 2279 | 848913 | C | -17.50 | 0.01199 |
| MAGI2 | 2282 | 10259665 | A | 14.84 | 0.00299 |
| MAGI2 | 2285 | 2191806 | C | 14.60 | 0.04286 |
| MAGI2 | 2288 | 6466535 | C | 16.79 | 0.02393 |
| MAGI2 | 2293 | 848934 | C | -19.68 | 0.00211 |
| MAGI2 | 2301 | 4730829 | C | 12.19 | 0.02884 |
| CACNA2D1 | 2304 | 17262683 | C | -10.61 | 0.04476 |
| CACNA2D1 | 2305 | 10215394 | G | -10.68 | 0.02153 |
| CACNA2D1 | 2317 | 6952949 | G | -9.39 | 0.04936 |
| SEMA3E | 2339 | 2713174 | C | 9.61 | 0.04165 |
| SEMA3A | 2347 | 1989964 | T | 8.01 | 0.04934 |
| ABCB4 | 2352 | 31669 | A | -25.48 | 0.00291 |
| PPP1R9A | 2365 | 6465455 | T | -12.31 | 0.00459 |
| PPP1R9A | 2367 | 854738 | T | -14.64 | 0.00212 |
| PPP1R9A | 2369 | 961262 | C | -12.06 | 0.00394 |
| PON1 | 2381 | 3917538 | T | 13.46 | 0.02265 |
| PON1 | 2382 | 2057681 | G | 11.09 | 0.03250 |
| PON1 | 2383 | 2299257 | C | 17.35 | 0.00010 |
| EXOC4 | 2463 | 2345943 | T | 9.28 | 0.04423 |
| EXOC4 | 2464 | 7776986 | C | -8.93 | 0.04321 |
| EXOC4 | 2465 | 10808271 | C | -8.93 | 0.04321 |
| PTPRN2 | 2535 | 2878355 | A | -11.30 | 0.04363 |
| CSMD1 | 2544 | 7829968 | T | 14.04 | 0.01998 |
| CSMD1 | 2548 | 1161518 | T | 11.24 | 0.03612 |
| MCPH1 | 2571 | 1057090 | T | -11.34 | 0.01720 |
| MCPH1 | 2572 | 2911968 | C | -13.11 | 0.00694 |
| SGCZ | 2594 | 4831307 | G | 10.73 | 0.03295 |
| XPO7 | 2620 | 10101881 | T | 11.83 | 0.00703 |
| XPO7 | 2622 | 2306645 | T | 11.83 | 0.00703 |
| XPO7 | 2624 | 7013323 | C | 10.33 | 0.01964 |
| SFRP1 | 2648 | 7833518 | T | 11.63 | 0.01928 |
| SNTG1 | 2651 | 318861 | G | 33.83 | 0.00334 |
| SNTG1 | 2652 | 10504093 | G | 26.47 | 0.01475 |
| SNTG1 | 2657 | 9298278 | A | 9.08 | 0.03616 |
| SNTG1 | 2659 | 4873135 | A | 8.75 | 0.04379 |
| LYN | 2673 | 1546518 | C | 10.00 | 0.02711 |
| TOX | 2685 | 11777927 | A | -13.16 | 0.02972 |
| CRISPLDI | 2758 | 13248650 | G | 14.91 | 0.00199 |
| MMP16 | 2761 | 2616499 | G | 11.95 | 0.02270 |
| ZFPM2 | 2806 | 7007352 | G | 24.36 | 0.00854 |
| ZFPM2 | 2808 | 1372614 | G | 24.36 | 0.00854 |
| SAMD12 | 2818 | 4876815 | G | -12.26 | 0.01965 |
| FBXO32 | 2830 | 6990407 | G | 16.67 | 0.00922 |
| FER1L6 | 2832 | 13279726 | G | -9.02 | 0.04900 |
| COL22A1 | 2913 | 6991720 | C | -12.69 | 0.02068 |
| COL22A1 | 2914 | 13279213 | A | -13.13 | 0.01377 |
| COL22A1 | 2915 | 1879047 | T | 15.41 | 0.03227 |
| COL22A1 | 2916 | 2318348 | G | 13.41 | 0.04622 |
| SMARCA2 | 2923 | 13301640 | A | -17.56 | 0.01897 |
| KIAA1797 | 2968 | 10738569 | A | -11.42 | 0.04514 |
| TEK | 2987 | 4601425 | T | -11.59 | 0.02295 |
| TEK | 2989 | 10511803 | C | -13.99 | 0.03164 |
| PIP5K1B | 2992 | 4744683 | T | 15.46 | 0.00132 |
| PIP5K1B | 2993 | 4534183 | A | -15.26 | 0.00152 |
| PIP5K1B | 2994 | 7853748 | G | 12.87 | 0.00748 |
| TRPM3 | 3026 | 1329776 | G | -14.14 | 0.01573 |
| NFIL3 | 3089 | 2440589 | T | 8.33 | 0.04871 |
| GABBR2 | 3105 | 16914811 | A | 14.03 | 0.03112 |
| SVEP1 | 3126 | 10759422 | C | 12.55 | 0.01872 |
| RALGPS1 | 3160 | 10987576 | A | -13.98 | 0.04220 |
| KCNT1 | 3195 | 497547 | G | 32.82 | 0.00928 |
| PITRM1 | 3225 | 6601755 | C | -9.59 | 0.04178 |
| CACNB2 | 3233 | 7090118 | A | -9.09 | 0.04983 |
| ARMC3 | 3245 | 10828374 | G | -15.07 | 0.00074 |
| ARMC3 | 3246 | 7919349 | G | -12.60 | 0.03190 |
| ARMC3 | 3248 | 17440393 | A | -13.21 | 0.02520 |
| ARMC3 | 3249 | 11013210 | T | -13.29 | 0.02262 |
| ARMC3 | 3251 | 10828395 | A | -14.16 | 0.03000 |
| JMJD1C | 3303 | 2893922 | C | 12.36 | 0.03509 |
| KCNMA1 | 3330 | 10509386 | C | 11.01 | 0.02073 |
| KCNMA1 | 3331 | 11002120 | T | 11.01 | 0.02073 |
| KCNMA1 | 3334 | 2619636 | A | -12.24 | 0.00287 |
| KCNMA1 | 3335 | 2719986 | T | -11.93 | 0.00328 |
| KCNMA1 | 3336 | 12780841 | A | -16.00 | 0.02220 |
| KCNMA1 | 3337 | 1465865 | C | -16.00 | 0.02220 |
| KCNMA1 | 3338 | 1978868 | A | -11.20 | 0.03732 |
| NRG3 | 3348 | 7078771 | A | -30.75 | 0.02918 |
| NRG3 | 3349 | 17107795 | T | -30.75 | 0.02918 |
| NRG3 | 3351 | 17101125 | A | -30.75 | 0.02918 |
| NRG3 | 3352 | 10509462 | T | -30.75 | 0.02918 |
| SORBS1 | 3358 | 11188287 | G | 14.94 | 0.00368 |
| SORCS3 | 3373 | 7900775 | T | 9.46 | 0.03682 |
| SORCS3 | 3375 | 7912102 | A | -12.13 | 0.01927 |
| SORCS3 | 3376 | 6584631 | T | -11.62 | 0.02475 |
| SORCS3 | 3380 | 7085496 | A | -10.57 | 0.04469 |
| SORCS3 | 3381 | 4918143 | T | 12.80 | 0.04764 |
| ATE1 | 3438 | 10510104 | G | -15.97 | 0.01579 |
| ATE1 | 3440 | 11598265 | C | -47.04 | 0.01634 |
| ATE1 | 3441 | 10788208 | A | -47.04 | 0.01634 |
| ATE1 | 3443 | 4471357 | A | -47.04 | 0.01634 |
| ATE1 | 3444 | 10749429 | T | -46.22 | 0.01723 |
| CTBP2 | 3454 | 769160 | C | 17.95 | 0.00445 |
| CTBP2 | 3455 | 9422765 | G | 14.93 | 0.00756 |
| GALNTL4 | 3471 | 7122801 | T | 14.69 | 0.00173 |
| NELL1 | 3516 | 12799803 | A | -13.94 | 0.03618 |
| DLG2 | 3538 | 4944461 | A | 18.63 | 0.04652 |
| DLG2 | 3552 | 1940128 | C | 15.62 | 0.02873 |
| DLG2 | 3554 | 582652 | A | 20.77 | 0.00323 |
| DLG2 | 3555 | 483220 | G | 14.75 | 0.03123 |
| OPCML | 3560 | 10894536 | A | -16.26 | 0.02002 |
| OPCML | 3567 | 7119286 | C | -14.35 | 0.00460 |
| OPCML | 3568 | 3018406 | A | -10.99 | 0.01738 |
| OPCML | 3571 | 3016500 | T | -10.21 | 0.04733 |
| OPCML | 3572 | 6590684 | G | -8.83 | 0.04804 |
| OPCML | 3585 | 7936263 | G | 14.05 | 0.00248 |
| OPCML | 3587 | 7108670 | C | 13.33 | 0.00699 |
| OPCML | 3588 | 7108751 | T | 12.85 | 0.00999 |
| LOC729025 | 3617 | 7955194 | T | -10.71 | 0.01769 |
| LOC729025 | 3620 | 6488827 | T | 9.52 | 0.02910 |
| LOC729025 | 3622 | 10772915 | A | -10.51 | 0.02419 |
| LOC729025 | 3623 | 1913253 | T | 9.48 | 0.03730 |
| LOC729025 | 3627 | 10734895 | A | 16.50 | 0.00600 |
| ITPR2 | 3640 | 2230375 | G | -15.33 | 0.00116 |
| NAV3 | 3667 | 7976374 | A | 10.13 | 0.03246 |
| NAV3 | 3674 | 1731757 | C | -23.93 | 0.02158 |
| ATXN2 | 3686 | 1544396 | G | -10.58 | 0.04357 |
| MTIF3 | 3690 | 17085657 | C | -34.65 | 0.00588 |
| MTIF3 | 3692 | 7334690 | A | -12.77 | 0.02160 |
| PCDH20 | 3737 | 17059750 | T | -55.74 | 0.00040 |
| PCDH20 | 3739 | 3812874 | T | -55.74 | 0.00040 |
| ITGBL1 | 3843 | 7323330 | A | 9.34 | 0.02946 |
| ITGBL1 | 3844 | 4772403 | T | 10.87 | 0.01544 |
| ITGBL1 | 3851 | 1469855 | G | -18.66 | 0.03401 |
| NPAS3 | 3865 | 1290203 | T | -10.28 | 0.02936 |
| NPAS3 | 3867 | 243284 | G | -9.91 | 0.03912 |
| SLC25A21 | 3880 | 10138134 | A | -21.38 | 0.03937 |
| LRFN5 | 3893 | 12884090 | A | -10.48 | 0.04179 |
| PPP2RSE | 3929 | 7154718 | G | -36.65 | 0.01356 |
| KCNK10 | 3966 | 12185033 | T | 11.24 | 0.02219 |
| KCNK13 | 3969 | 769044 | A | -9.72 | 0.03184 |
| ATP10A | 4005 | 11853308 | C | -9.26 | 0.03688 |
| ATP10A | 4009 | 12442216 | T | -9.93 | 0.04477 |
| ATP10A | 4012 | 4906747 | G | 17.70 | 0.04997 |
| ATP10A | 4013 | 10873606 | A | -12.27 | 0.00657 |
| ATP10A | 4014 | 4906750 | C | 12.86 | 0.03028 |
| RYR3 | 4021 | 1980102 | A | 9.38 | 0.03360 |
| C15ORF41 | 4038 | 3784678 | C | 9.30 | 0.03009 |
| CGNL1 | 4067 | 12595188 | G | 13.54 | 0.01546 |
| CGNL1 | 4068 | 4774257 | G | 11.11 | 0.02124 |
| CGNL1 | 4072 | 8034215 | G | -12.17 | 0.00844 |
| RORA | 4078 | 12914584 | G | 9.52 | 0.04840 |
| KIFC3 | 4149 | 874503 | A | -29.26 | 0.03832 |
| WWOX | 4151 | 8061908 | C | 13.29 | 0.00239 |
| WWOX | 4152 | 1922620 | C | 12.60 | 0.00368 |
| WWOX | 4154 | 16947095 | A | -10.87 | 0.00821 |
| WWOX | 4155 | 7184271 | C | -9.30 | 0.03705 |
| WWOX | 4156 | 4887935 | C | 12.10 | 0.00692 |
| MPHOSPH6 | 4165 | 2081257 | G | -21.33 | 0.00325 |
| MPHOSPH6 | 4167 | 7192444 | G | 10.16 | 0.03567 |
| MPHOSPH6 | 4168 | 11645604 | A | -18.83 | 0.00409 |
| MPHOSPH6 | 4169 | 11647563 | C | -18.06 | 0.01012 |
| MPHOSPH6 | 4171 | 10514533 | C | -14.84 | 0.03018 |
| USP10 | 4211 | 8060725 | A | -9.05 | 0.04662 |
| DNAH9 | 4222 | 11078034 | C | -10.61 | 0.02917 |
| DNAH9 | 4223 | 9903258 | G | -15.15 | 0.00214 |
| MS12 | 4260 | 8065120 | A | 15.62 | 0.01491 |
| PTPRM | 4293 | 1866854 | G | 10.26 | 0.03005 |
| LOXHD1 | 4319 | 986117 | C | 11.42 | 0.01869 |
| NEDD4L | 4326 | 9962106 | T | 13.10 | 0.01472 |
| NEDD4L | 4329 | 10513914 | G | 12.79 | 0.01662 |
| NEDD4L | 4330 | 2075405 | G | 13.40 | 0.01607 |
| CCBE1 | 4342 | 2564495 | G | 10.79 | 0.02069 |
| CDH7 | 4363 | 9953248 | T | -28.26 | 0.04726 |
| TXNDC10 | 4373 | 309242 | G | -16.46 | 0.00943 |
| DOK6 | 4374 | 7243624 | T | 11.80 | 0.01762 |
| ZNF667 | 4387 | 10423617 | T | 13.70 | 0.01557 |
| MACROD2 | 4450 | 708975 | G | 8.64 | 0.02963 |
| MAPRE1 | 4490 | 410488 | A | 10.74 | 0.02545 |
| PTPRT | 4498 | 746413 | C | -9.32 | 0.02404 |
| PTPRT | 4502 | 6124439 | A | 15.34 | 0.00336 |
| SNAI1 | 4524 | 6020157 | A | 21.42 | 0.00127 |
| CDH4 | 4531 | 6121764 | G | -12.08 | 0.01213 |
| CDH4 | 4532 | 1317385 | C | -11.06 | 0.02082 |
| CDH4 | 4536 | 17811544 | G | 8.81 | 0.04843 |
| MIRN155 | 4555 | 8134718 | T | 12.63 | 0.04551 |
| SGSM1 | 4571 | 5760752 | C | -17.90 | 0.00009 |
| TTLL1 | 4592 | 1052160 | T | 9.39 | 0.02651 |
| EFCAB6 | 4596 | 7286735 | T | 13.61 | 0.01095 |
| EFCAB6 | 4599 | 12628583 | C | -20.37 | 0.01186 |
| EFCAB6 | 4612 | 2187887 | G | 11.66 | 0.01390 |
| EFCAB6 | 4615 | 138102 | T | 9.60 | 0.04333 |

| **TABLE 4: Alleles Influencing Response to Perphenazine** | | | | | |
|---|---|---|---|---|---|
| **Gene** | **Seq ID** | **NCBI RS#** | **Allele** | **Beta (PANSS)** | **P** |
| EPHB2 | 20 | 10799762 | C | -18.09 | 0.00658 |
| EPHB2 | 23 | 309499 | T | 10.52 | 0.01061 |
| LRP8 | 48 | 5174 | A | 13.42 | 0.00025 |
| LRP8 | 52 | 2297660 | A | 12.62 | 0.00101 |
| LRP8 | 53 | 2297657 | A | 8.84 | 0.02742 |
| RALGPS2 | 132 | 6662050 | A | 25.81 | 0.00196 |
| RALGPS2 | 133 | 3766637 | C | 21.70 | 0.00747 |
| SLC35F3 | 186 | 7515801 | G | 18.89 | 0.00369 |
| SLC35F3 | 187 | 479040 | T | 18.67 | 0.00518 |
| SLC35F3 | 190 | 12118979 | C | -22.78 | 0.00162 |
| SLC35F3 | 191 | 10495346 | T | -23.53 | 0.00174 |
| FMN2 | 230 | 6698474 | A | 10.10 | 0.02189 |
| FMN2 | 231 | 12123407 | A | 10.06 | 0.02354 |
| FMN2 | 235 | 10926168 | G | -8.03 | 0.04313 |
| FMN2 | 236 | 7520065 | G | -8.03 | 0.04313 |
| RGS7 | 253 | 6429223 | G | 8.63 | 0.03151 |
| PLD5 | 271 | 1548160 | C | -10.35 | 0.01789 |
| C2ORF46 | 275 | 6747626 | C | -25.38 | 0.00733 |
| KLHL29 | 295 | 4665612 | T | 18.32 | 0.02099 |
| ASXL2 | 302 | 10180842 | G | 11.21 | 0.04149 |
| BRE | 317 | 1948922 | G | -10.64 | 0.02541 |
| SLC8A1 | 349 | 385561 | G | -10.88 | 0.03408 |
| PLEKHH2 | 362 | 10165660 | C | 11.78 | 0.02521 |
| C2ORF34 | 374 | 3738980 | C | -13.72 | 0.02998 |
| CTNNA2 | 448 | 3755095 | G | 13.53 | 0.02058 |
| CTNNA2 | 449 | 2974169 | C | -8.15 | 0.03658 |
| CTNNA2 | 455 | 1443895 | A | -7.70 | 0.04037 |
| LRP1B | 501 | 1486963 | C | -10.66 | 0.02393 |
| LRP1B | 503 | 1492388 | T | -9.32 | 0.01963 |
| LRP1B | 504 | 10469593 | C | -9.79 | 0.02421 |
| LRP1B | 505 | 12479163 | A | -9.98 | 0.02848 |
| LRP1B | 513 | 9283437 | C | -8.88 | 0.04509 |
| LRP1B | 515 | 4611596 | C | 8.49 | 0.04455 |
| LRP1B | 518 | 13418027 | C | -7.67 | 0.03880 |
| LRP1B | 521 | 4662370 | T | -10.09 | 0.04936 |
| LRP1B | 524 | 13382235 | G | -17.23 | 0.04795 |
| KYNU | 526 | 2043944 | T | -9.30 | 0.03330 |
| ARHGAP15 | 551 | 11694505 | C | 11.08 | 0.01213 |
| ARHGAP15 | 552 | 6745691 | G | 9.77 | 0.03162 |
| ARHGAP15 | 553 | 6750323 | C | 9.09 | 0.04200 |
| KCNJ3 | 593 | 2971904 | T | 17.32 | 0.00371 |
| PDE1A | 637 | 4666828 | A | 17.89 | 0.00084 |
| NRP2 | 670 | 849526 | A | 10.56 | 0.01259 |
| NRP2 | 671 | 849525 | A | 10.56 | 0.01259 |
| COL4A3 | 708 | 7587228 | C | 13.78 | 0.04754 |
| DNER | 716 | 2052303 | T | -8.93 | 0.03447 |
| TRIP12 | 727 | 475525 | T | 14.81 | 0.00593 |
| CLASP2 | 813 | 4679039 | C | 9.32 | 0.03495 |
| ULK4 | 838 | 7627972 | A | 9.28 | 0.03329 |
| ULK4 | 839 | 7649806 | A | 11.03 | 0.01291 |
| SEMA3F | 860 | 2624837 | T | 14.68 | 0.03126 |
| CACNA2D2 | 861 | 2298955 | C | 15.46 | 0.00237 |
| CACNA2D2 | 863 | 754298 | C | 15.81 | 0.00344 |
| IL17RD | 906 | 6788981 | T | 12.41 | 0.04164 |
| PTPRG | 933 | 1320173 | G | -9.36 | 0.02371 |
| MAGI1 | 976 | 264705 | A | 10.96 | 0.03322 |
| PLCXD2 | 1017 | 1913495 | T | -10.27 | 0.04585 |
| RAB6B | 1061 | 6765093 | G | -10.03 | 0.01598 |
| SPSB4 | 1084 | 4683550 | C | 11.95 | 0.03625 |
| NLGN1 | 1121 | 692457 | A | -8.44 | 0.04605 |
| NLGN1 | 1124 | 12634066 | A | -17.68 | 0.02450 |
| NLGN1 | 1126 | 1546685 | T | -9.77 | 0.04822 |
| NLGN1 | 1127 | 9828801 | T | -17.25 | 0.03279 |
| IL1RAP | 1143 | 2193877 | G | 9.10 | 0.04257 |
| SLC2A9 | 1159 | 3733591 | A | 10.07 | 0.04856 |
| NPFFR2 | 1250 | 2137735 | A | 11.74 | 0.01270 |
| NPFFR2 | 1252 | 2365797 | T | 9.08 | 0.04986 |
| SCD5 | 1266 | 7682126 | G | 9.52 | 0.03709 |
| SCD5 | 1267 | 13106297 | G | 9.52 | 0.03709 |
| COL25A1 | 1328 | 711865 | A | 8.61 | 0.03855 |
| ANK2 | 1340 | 13134375 | C | 10.05 | 0.00796 |
| ANK2 | 1341 | 13107082 | G | -8.30 | 0.02672 |
| ANK2 | 1342 | 413019 | C | -7.48 | 0.04296 |
| CAMK2D | 1364 | 6853484 | T | 22.47 | 0.03898 |
| PRSS12 | 1377 | 883686 | C | -8.78 | 0.04436 |
| MAML3 | 1386 | 6819304 | A | -14.75 | 0.01125 |
| MAML3 | 1392 | 10024138 | T | 9.42 | 0.02936 |
| IL15 | 1403 | 6841939 | A | 9.02 | 0.03908 |
| IL15 | 1406 | 4254850 | C | -7.95 | 0.04822 |
| IL15 | 1407 | 1493015 | A | 8.06 | 0.04924 |
| IL15 | 1408 | 6821171 | G | -10.89 | 0.01052 |
| IL15 | 1409 | 1389098 | C | -10.56 | 0.01099 |
| INPP4B | 1411 | 2667108 | C | 42.73 | 0.01254 |
| INPP4B | 1414 | 336329 | G | 42.73 | 0.01254 |
| INPP4B | 1415 | 336330 | T | 59.30 | 0.01366 |
| DCLK2 | 1441 | 12513356 | G | -20.95 | 0.02896 |
| DCLK2 | 1442 | 6535725 | A | -11.73 | 0.00199 |
| DCLK2 | 1443 | 4696645 | A | -16.65 | 0.00794 |
| ODZ3 | 1515 | 6820811 | G | 9.79 | 0.03884 |
| DNAH5 | 1554 | 1502050 | G | 14.21 | 0.00425 |
| DNAH5 | 1556 | 7709692 | C | 12.80 | 0.01798 |
| CDH10 | 1577 | 1835231 | A | -11.12 | 0.03042 |
| PDE4D | 1625 | 929820 | T | -10.43 | 0.01773 |
| PDE4D | 1626 | 2910642 | C | -8.65 | 0.03770 |
| CMYA5 | 1651 | 6880680 | C | 18.93 | 0.04811 |
| THBS4 | 1659 | 1866389 | C | 12.59 | 0.00821 |
| VCAN | 1666 | 11749904 | C | 8.13 | 0.04789 |
| FBXL17 | 1697 | 12519388 | T | 12.44 | 0.02375 |
| FBXL17 | 1698 | 12189428 | G | 12.44 | 0.02375 |
| FBXL17 | 1699 | 17160706 | C | 10.03 | 0.04472 |
| HSD17B4 | 1739 | 17453681 | C | -12.17 | 0.03437 |
| SNCA1P | 1748 | 1990879 | C | -10.98 | 0.02646 |
| ODZ2 | 1791 | 11952245 | G | 8.80 | 0.04092 |
| SLC22A23 | 1809 | 11967828 | T | 14.26 | 0.00461 |
| SLC22A23 | 1810 | 17136440 | T | 11.88 | 0.01855 |
| SLC22A23 | 1811 | 7747259 | T | 11.98 | 0.01871 |
| ATXN1 | 1836 | 720006 | A | 9.92 | 0.01942 |
| GABRR2 | 1920 | 6900958 | T | -11.49 | 0.02760 |
| KLHL32 | 1921 | 6922974 | A | 13.21 | 0.03354 |
| KLHL32 | 1923 | 998195 | C | -10.41 | 0.01262 |
| KLHL32 | 1924 | 1997933 | G | 9.90 | 0.02072 |
| SLC22A16 | 1932 | 7768422 | C | 15.08 | 0.04785 |
| HS3ST5 | 1934 | 1334900 | T | 9.23 | 0.03155 |
| TRDN | 1939 | 1431284 | G | -10.87 | 0.01718 |
| TRDN | 1940 | 17686735 | G | -11.08 | 0.01349 |
| TRDN | 1942 | 12190077 | A | -11.03 | 0.01496 |
| NKAIN2 | 1962 | 11753035 | A | 13.79 | 0.04159 |
| PDE7B | 1987 | 6938424 | T | 24.87 | 0.00239 |
| PDE7B | 1989 | 4475338 | T | 37.18 | 0.03146 |
| PARK2 | 2037 | 12192200 | C | 8.57 | 0.03638 |
| PARK2 | 2038 | 10945823 | A | 11.85 | 0.01815 |
| PARK2 | 2039 | 10945825 | A | 11.00 | 0.02079 |
| PARK2 | 2041 | 2105643 | C | 11.00 | 0.02079 |
| PARK2 | 2042 | 4708961 | T | 8.59 | 0.04701 |
| PDE10A | 2060 | 585208 | C | 12.17 | 0.02777 |
| PDE10A | 2061 | 566759 | G | 11.62 | 0.03342 |
| FERD3L | 2103 | 10228726 | G | -9.79 | 0.04191 |
| FERD3L | 2105 | 11766540 | C | -11.46 | 0.01666 |
| FERD3L | 2108 | 6967671 | T | 9.66 | 0.04524 |
| CREB5 | 2119 | 2237361 | C | -10.99 | 0.01522 |
| CREB5 | 2123 | 12538892 | A | -10.24 | 0.04037 |
| CHN2 | 2128 | 7781003 | T | 8.81 | 0.04292 |
| BMPER | 2165 | 7806522 | C | 9.75 | 0.04375 |
| BMPER | 2167 | 4140982 | A | 9.54 | 0.02586 |
| WBSCR17 | 2238 | 6971383 | A | 10.51 | 0.01012 |
| WBSCR17 | 2240 | 1859526 | T | 15.00 | 0.00083 |
| WBSCR17 | 2242 | 4585627 | T | 11.71 | 0.00419 |
| WBSCR17 | 2245 | 10279551 | G | 12.40 | 0.01521 |
| WBSCR17 | 2246 | 7783170 | C | 10.80 | 0.02237 |
| MAGI2 | 2274 | 758710 | C | -8.60 | 0.04552 |
| MAGI2 | 2279 | 848913 | C | -18.78 | 0.00215 |
| MAGI2 | 2297 | 10261439 | A | -16.51 | 0.00118 |
| MAGI2 | 2300 | 10248584 | G | -18.74 | 0.00178 |
| CACNA2D1 | 2308 | 258728 | A | 13.63 | 0.01824 |
| PCLO | 2316 | 7781142 | A | -9.66 | 0.02069 |
| PCLO | 2323 | 2371214 | C | 8.95 | 0.04471 |
| PCLO | 2324 | 2522843 | A | 8.57 | 0.04213 |
| PCLO | 2329 | 7799260 | G | -8.87 | 0.02973 |
| DYNC111 | 2386 | 1382620 | A | -8.27 | 0.04927 |
| DYNC111 | 2392 | 6968143 | G | -14.28 | 0.03029 |
| DYNC111 | 2393 | 2073984 | A | -8.92 | 0.04580 |
| ZNF3 | 2399 | 4424195 | G | 10.18 | 0.03333 |
| DGKI | 2473 | 834054 | T | 58.87 | 0.01393 |
| CNTNAP2 | 2498 | 7803992 | A | 10.51 | 0.01398 |
| CNTNAP2 | 2500 | 1525218 | T | 11.87 | 0.00588 |
| CNTNAP2 | 2501 | 1525259 | C | 8.85 | 0.04879 |
| CNTNAP2 | 2505 | 4726875 | A | 11.26 | 0.01023 |
| CNTNAP2 | 2506 | 1528507 | G | 11.26 | 0.01023 |
| CSMD1 | 2540 | 17065933 | A | 9.85 | 0.03080 |
| CSMD1 | 2542 | 17065959 | C | 11.01 | 0.02651 |
| MCPH1 | 2563 | 3020213 | T | 8.54 | 0.04516 |
| MCPH1 | 2565 | 3824312 | A | 10.12 | 0.04141 |
| XPO7 | 2621 | 1809498 | C | 10.91 | 0.00804 |
| XPO7 | 2623 | 3816786 | T | 10.90 | 0.00819 |
| SLC25A37 | 2632 | 10503726 | C | 10.65 | 0.02414 |
| SLC25A37 | 2633 | 7826247 | G | 14.80 | 0.00146 |
| NKAIN3 | 2704 | 4737609 | A | 15.16 | 0.02969 |
| NKAIN3 | 2716 | 4739023 | C | -9.90 | 0.01595 |
| NKAIN3 | 2719 | 4382471 | T | 9.45 | 0.04753 |
| NKAIN3 | 2721 | 10100456 | T | 9.45 | 0.04753 |
| KCNB2 | 2735 | 1431659 | T | 9.66 | 0.02773 |
| KCNB2 | 2737 | 349355 | T | 11.11 | 0.04899 |
| KCNB2 | 2740 | 7010480 | G | 8.56 | 0.03633 |
| GRHL2 | 2771 | 17397776 | T | 9.02 | 0.03416 |
| GRHL2 | 2772 | 17397811 | A | 8.91 | 0.03600 |
| ZFPM2 | 2801 | 12546300 | G | 8.80 | 0.04189 |
| ZFPM2 | 2807 | 2028553 | A | 11.63 | 0.01401 |
| SAMD12 | 2827 | 2514602 | A | 18.08 | 0.01693 |
| SAMD12 | 2829 | 2460983 | G | 24.02 | 0.00040 |
| FBXO32 | 2831 | 2294089 | C | -14.45 | 0.03866 |
| FER1L6 | 2834 | 6981430 | G | -8.38 | 0.04590 |
| FER1L6 | 2837 | 7831422 | G | -17.07 | 0.02009 |
| FER1L6 | 2838 | 12375254 | A | -16.10 | 0.02425 |
| FER1L6 | 2841 | 7012186 | T | -12.64 | 0.04795 |
| FER1L6 | 2847 | 4871463 | G | -10.19 | 0.03115 |
| COL22A1 | 2907 | 4475485 | T | 14.66 | 0.01144 |
| PTPRD | 2940 | 3843579 | C | 9.03 | 0.03728 |
| PTPRD | 2941 | 12555585 | G | 10.69 | 0.04498 |
| PTPRD | 2949 | 942176 | A | 8.57 | 0.04125 |
| PTPRD | 2956 | 7868093 | T | -13.29 | 0.00215 |
| TMC1 | 3039 | 10481772 | C | -15.33 | 0.01611 |
| PCSK5 | 3050 | 7862766 | T | -15.54 | 0.01957 |
| PCSKS | 3051 | 2789610 | C | -11.58 | 0.02452 |
| PCSK5 | 3052 | 2789608 | G | -13.59 | 0.00380 |
| PCSK5 | 3053 | 4745488 | C | -9.53 | 0.04628 |
| GNAQ | 3059 | 1030828 | A | -11.43 | 0.00528 |
| GNAQ | 3060 | 11145565 | C | -10.32 | 0.01455 |
| GNAQ | 3070 | 6560625 | C | 10.71 | 0.03768 |
| NTRK2 | 3080 | 1036914 | C | 10.00 | 0.02856 |
| DAPK1 | 3082 | 6560011 | C | -8.82 | 0.03047 |
| PTGS1 | 3150 | 7864901 | G | -13.40 | 0.01478 |
| ABL1 | 3168 | 10901294 | T | 20.09 | 0.01102 |
| ABL1 | 3169 | 3824400 | A | 12.54 | 0.03932 |
| ABLI | 3170 | 7021981 | T | 15.63 | 0.04125 |
| ABL1 | 3171 | 11792273 | G | 15.63 | 0.04125 |
| PNPLA7 | 3204 | 10867123 | G | -11.06 | 0.01613 |
| CACNA1B | 3210 | 1123190 | A | -11.33 | 0.03162 |
| CACNB2 | 3241 | 10764566 | G | -9.41 | 0.01861 |
| MYO3A | 3281 | 3758447 | C | -10.14 | 0.01847 |
| MYO3A | 3283 | 16926660 | A | 16.56 | 0.00830 |
| MYO3A | 3286 | 16926667 | A | 12.83 | 0.03051 |
| JMJD1C | 3301 | 9629895 | A | 11.33 | 0.00735 |
| CDH23 | 3309 | 9415039 | T | 9.60 | 0.01842 |
| KCNMA1 | 3319 | 2288840 | G | -8.37 | 0.04573 |
| KCNMA1 | 3320 | 7067873 | C | -8.67 | 0.04617 |
| KCNMA1 | 3326 | 548216 | C | 9.26 | 0.02968 |
| SORCS3 | 3370 | 752558 | C | 11.23 | 0.02285 |
| SORCS3 | 3371 | 10884026 | G | 10.72 | 0.04684 |
| VTI1A | 3389 | 9971308 | A | -12.82 | 0.01260 |
| VTI1A | 3390 | 6585156 | A | -12.82 | 0.01260 |
| ATE1 | 3450 | 11200257 | A | -15.96 | 0.00720 |
| ATE1 | 3452 | 7906462 | A | -11.96 | 0.01936 |
| CTBP2 | 3453 | 4962718 | C | 10.32 | 0.04535 |
| IGSF22 | 3515 | 12789252 | A | -9.95 | 0.01529 |
| KCNA4 | 3517 | 7931190 | T | 7.63 | 0.04487 |
| C11ORF49 | 3522 | 3740691 | A | -9.52 | 0.03894 |
| ELMOD1 | 3558 | 684901 | T | -11.84 | 0.01420 |
| OPCML | 3571 | 3016500 | T | 9.47 | 0.04326 |
| OPCML | 3589 | 4936195 | G | -13.29 | 0.03592 |
| TMEM16B | 3605 | 10466913 | A | 14.47 | 0.01253 |
| STYK1 | 3616 | 10743918 | C | -8.54 | 0.03431 |
| NAV3 | 3679 | 7137676 | T | 10.08 | 0.01847 |
| NBEA | 3715 | 7333195 | T | -14.55 | 0.00033 |
| NBEA | 3719 | 7320580 | G | 12.53 | 0:00547 |
| NBEA | 3720 | 7988705 | A | 10.21 | 0.03388 |
| TRPC4 | 3729 | 1556541 | C | -10.31 | 0.01559 |
| LMO7 | 3753 | 9573672 | T | 17.25 | 0.03651 |
| GPC5 | 3790 | 2148226 | G | -8.06 | 0.04086 |
| NALCN | 3828 | 11069434 | C | 8.70 | 0.02327 |
| NALCN | 3829 | 9518353 | C | 8.09 | 0.03943 |
| NOVA1 | 3856 | 178205 | A | -10.11 | 0.04524 |
| NOVA1 | 3861 | 1955850 | G | -8.98 | 0.03229 |
| NPAS3 | 3866 | 243291 | G | -8.30 | 0.04225 |
| NPAS3 | 3869 | 1579702 | G | -13.16 | 0.00059 |
| NPAS3 | 3871 | 10133530 | A | 12.90 | 0.01481 |
| SLC25A21 | 3878 | 2073248 | C | 17.11 | 0.02495 |
| SLC25A21 | 3879 | 12185044 | G | 17.49 | 0.04144 |
| SLC25A21 | 3882 | 2022601 | A | -12.40 | 0.00297 |
| SLC25A21 | 3883 | 10872897 | T | -12.33 | 0.00329 |
| SLC25A21 | 3885 | 1367029 | A | -8.06 | 0.04998 |
| PPP2R5E | 3922 | 7140704 | G | -15.31 | 0.01036 |
| RGS6 | 3944 | 2681749 | C | -8.81 | 0.03842 |
| RGS6 | 3952 | 2238192 | A | -11.04 | 0.00721 |
| NRXN3 | 3955 | 4020132 | T | 22.49 | 0.01259 |
| KCNK10 | 3960 | 411627 | C | 9.62 | 0.03242 |
| CCDC88C | 3983 | 8015982 | C | -9.12 | 0.04057 |
| CCDC88C | 3984 | 10131741 | G | -9.12 | 0.04057 |
| CCDC88C | 3985 | 8008996 | T | -9.12 | 0.04057 |
| CCDC88C | 3986 | 8007791 | A | -9.12 | 0.04057 |
| BCL11B | 3999 | 807567 | C | -13.61 | 0.01511 |
| ATP10A | 4016 | 2066704 | A | -21.04 | 0.01347 |
| RYR3 | 4028 | 16957065 | G | -9.77 | 0.03092 |
| MEIS2 | 4049 | 1562804 | T | 8.07 | 0.02567 |
| CGNL1 | 4068 | 4774257 | G | 9.24 | 0.03544 |
| CGNL1 | 4069 | 4774940 | C | -10.55 | 0.01298 |
| CGNL1 | 4070 | 7182648 | T | -10.00 | 0.02481 |
| CLK3 | 4084 | 12441932 | G | 18.04 | 0.00243 |
| CLK3 | 4085 | 2068982 | A | 15.44 | 0.00974 |
| A2BP1 | 4114 | 1420058 | C | -8.62 | 0.04650 |
| A2BP1 | 4120 | 1492382 | C | 11.69 | 0.01883 |
| WWOX | 4152 | 1922620 | C | 8.47 | 0.04436 |
| MPHOSPH6 | 4163 | 8052512 | T | -15.17 | 0.00973 |
| MPHOSPH6 | 4168 | 11645604 | A | -11.69 | 0.04876 |
| CRISPLD2 | 4212 | 2641698 | T | -9.24 | 0.03290 |
| CA10 | 4235 | 1501253 | G | -8.04 | 0.03701 |
| CA10 | 4238 | 2970016 | G | 9.50 | 0.02212 |
| CA10 | 4240 | 1503058 | A | -8.63 | 0.03181 |
| ZFP161 | 4286 | 12604181 | T | 22.46 | 0.02818 |
| PTPRM | 4303 | 2509550 | G | -10.26 | 0.01680 |
| CHST9 | 4317 | 4800797 | G | -10.74 | 0.02272 |
| NEDD4L | 4320 | 7243662 | G | -14.91 | 0.00869 |
| NEDD4L | 4323 | 292448 | C | -12.19 | 0.01301 |
| CCBE1 | 4340 | 2851865 | T | 10.87 | 0.01560 |
| CCBE1 | 4342 | 2564495 | G | 8.94 | 0.03843 |
| CCBE1 | 4343 | 1560299 | T | 8.11 | 0.03784 |
| CCBE1 | 4345 | 9954124 | T | 9.08 | 0.03681 |
| DOK6 | 4378 | 8097743 | G | -7.73 | 0.04573 |
| ATRN | 4391 | 2670300 | G | -8.57 | 0.04216 |
| FERMT1 | 4411 | 8121939 | G | 8.87 | 0.03128 |
| PLCB1 | 4432 | 6118375 | C | 11.83 | 0.00946 |
| MACROD2 | 4458 | 7509422 | T | -8.81 | 0.03627 |
| PTPRT | 4494 | 2866940 | C | 9.51 | 0.02822 |
| PTGIS | 4523 | 515633 | A | -15.94 | 0.03724 |
| SNAI1 | 4524 | 6020157 | A | 13.30 | 0.02592 |
| CDH4 | 4529 | 4347903 | C | 10.21 | 0.04356 |
| CDH4 | 4530 | 4812313 | G | 8.52 | 0.04953 |
| NCAM2 | 4549 | 2826852 | C | 8.43 | 0.03889 |
| NCAM2 | 4550 | 973045 | A | -8.31 | 0.04192 |
| NCAM2 | 4551 | 986917 | A | 8.13 | 0.04547 |
| PCP4 | 4556 | 398802 | G | -7.92 | 0.04088 |
| PDE9A | 4562 | 2269145 | T | 13.28 | 0.00086 |
| PDE9A | 4564 | 12626774 | C | -13.28 | 0.00269 |

| **TABLE 5: Alleles Influencing Response to Ziprasidone** | | | | | |
|---|---|---|---|---|---|
| **Gene** | **Seq ID** | **NCBI RS#** | **Allele** | **Beta (PANSS)** | **P** |
| KIF1B | 3 | 4240911 | C | 9.08 | 0.03275 |
| KIF1B | 5 | 17401966 | G | 10.00 | 0.02342 |
| KIF1B | 6 | 12734551 | G | 9.47 | 0.02466 |
| RP1-21018.1 | 9 | 7539545 | T | -7.22 | 0.02811 |
| AGBL4-C1ORF165 | 27 | 1934395 | G | -11.79 | 0.01637 |
| LRP8 | 59 | 1416095 | C | 8.99 | 0.02855 |
| ELTD1 | 61 | 2200810 | G | -14.72 | 0.00145 |
| ELTD1 | 62 | 7552978 | A | 12.31 | 0.00046 |
| ELTD1 | 63 | 12754818 | C | 9.47 | 0.00493 |
| ELTD1 | 64 | 1352555 | A | 11.66 | 0.00104 |
| ELTD1 | 66 | 10493622 | A | 12.10 | 0.00123 |
| PTGFRN | 92 | 1998922 | A | 10.85 | 0.01631 |
| ATF6 | 99 | 1875762 | T | 13.32 | 0.04286 |
| OLFML2B | 111 | 12025136 | C | 9.46 | 0.03799 |
| DPT | 121 | 6427142 | G | -12.75 | 0.02446 |
| RYR2 | 204 | 4376724 | C | 9.25 | 0.00789 |
| C2ORF46 | 277 | 2562011 | C | 12.31 | 0.04666 |
| C2ORF46 | 279 | 10210006 | C | -6.98 | 0.04505 |
| KLHL29 | 297 | 17045574 | A | 39.49 | 0.00049 |
| BRE | 316 | 9309659 | G | -10.32 | 0.01956 |
| CRIM1 | 333 | 11681392 | T | -8.28 | 0.03510 |
| FEZ2 | 335 | 3770811 | T | -7.78 | 0.04220 |
| FEZ2 | 336 | 1533946 | G | -7.68 | 0.04228 |
| FEZ2 | 339 | 1179494 | C | 8.62 | 0.03384 |
| C2ORF34 | 364 | 1067378 | A | 9.91 | 0.01158 |
| C2ORF34 | 365 | 1067404 | A | 8.35 | 0.02429 |
| C2ORF34 | 367 | 698792 | A | 8.35 | 0.02429 |
| C2ORF34 | 373 | 1965789 | C | 8.02 | 0.02728 |
| AAK1 | 441 | 12622388 | G | -9.39 | 0.02387 |
| NAP5 | 477 | 1437909 | G | 8.12 | 0.03817 |
| ZRANB3 | 490 | 11893424 | A | 19.63 | 0.04721 |
| ZRANB3 | 491 | 1898524 | T | 19.63 | 0.04721 |
| ARHGAP15 | 549 | 12478952 | A | -13.03 | 0.01912 |
| ARHGAP15 | 560 | 10928200 | T | -7.07 | 0.03531 |
| ARHGAP15 | 561 | 13031917 | C | -7.07 | 0.03531 |
| FMNL2 | 587 | 1561267 | C | 12.60 | 0.03280 |
| SCN1A | 620 | 6432860 | A | 8.55 | 0.04669 |
| CERKL | 633 | 993650 | G | 9.92 | 0.02526 |
| PDE1A | 643 | 10497597 | T | -10.66 | 0.00616 |
| PDE1A | 645 | 11689230 | T | -9.45 | 0.01172 |
| NRP2 | 673 | 13396083 | C | 9.55 | 0.01084 |
| NRP2 | 674 | 7596827 | C | 8.96 | 0.01293 |
| NRP2 | 675 | 6435306 | A | 8.36 | 0.02085 |
| ERBB4 | 681 | 7563759 | A | 8.41 | 0.02528 |
| ERBB4 | 683 | 4672668 | G | 7.81 | 0.02546 |
| DNER | 718 | 208791 | A | -14.15 | 0.01544 |
| DNER | 719 | 208784 | G | -14.15 | 0.01544 |
| DNER | 720 | 208785 | G | -14.15 | 0.01544 |
| DNER | 721 | 1323595 | T | -14.15 | 0.01544 |
| DNER | 722 | 208787 | G | -14.15 | 0.01544 |
| TRIP12 | 723 | 6723005 | T | -14.15 | 0.01544 |
| CNTN4 | 763 | 1032783 | A | 12.09 | 0.02697 |
| ITPRI | 776 | 3792490 | A | 8.08 | 0.04869 |
| SLC6A6 | 794 | 1156567 | C | -11.62 | 0.03731 |
| DAZL | 799 | 17029179 | C | -10.70 | 0.03316 |
| ULK4 | 828 | 4973875 | C | 8.31 | 0.02284 |
| ULK4 | 831 | 6790569 | A | 10.17 | 0.01511 |
| ULK4 | 832 | 9827383 | C | 9.98 | 0.00929 |
| ULK4 | 833 | 10510719 | A | 9.64 | 0.01466 |
| ULK4 | 836 | 9311275 | C | 12.05 | 0.00193 |
| ULK4 | 837 | 9818955 | C | 12.24 | 0.00284 |
| ULK4 | 841 | 6809441 | C | 9.93 | 0.00808 |
| ULK4 | 842 | 11129908 | A | 7.58 | 0.04141 |
| ULK4 | 843 | 7618902 | G | 10.22 | 0.00563 |
| ULK4 | 848 | 9825741 | A | 13.26 | 0.02411 |
| ULK4 | 849 | 17062109 | T | 12.68 | 0.02961 |
| ULK4 | 850 | 7648578 | T | 12.68 | 0.02961 |
| ULK4 | 851 | 3774372 | C | 12.68 | 0.02961 |
| ULK4 | 852 | 1716685 | T | 12.68 | 0.02961 |
| ULK4 | 853 | 2272007 | A | 12.68 | 0.02961 |
| CACNA2D3 | 874 | 3773580 | T | -12.03 | 0.00211 |
| CACNA2D3 | 875 | 3773569 | A | -10.79 | 0.00484 |
| FLNB | 910 | 12488636 | C | 9.12 | 0.00750 |
| FLNB | 911 | 12632456 | A | 8.96 | 0.00820 |
| FLNB | 912 | 7632986 | T | 10.69 | 0.00179 |
| PRICKLE2 | 964 | 696017 | A | 19.24 | 0.01331 |
| PRICKLE2 | 969 | 704398 | G | 16.10 | 0.02652 |
| PRICKLE2 | 970 | 697288 | T | 14.55 | 0.03537 |
| CDGAP | 1018 | 7649930 | G | -7.50 | 0.03792 |
| CDGAP | 1019 | 1448434 | C | -7.50 | 0.03792 |
| CDGAP | 1020 | 6438523 | A | -7.50 | 0.03792 |
| CDGAP | 1022 | 6773050 | C | 7.89 | 0.04425 |
| CPNE4 | 1060 | 2178383 | G | 9.24 | 0.00878 |
| TNIK | 1099 | 4955765 | G | 7.90 | 0.04965 |
| PLD1 | 1104 | 181715 | T | 14.25 | 0.00070 |
| LRRC15 | 1150 | 6799698 | A | 9.95 | 0.04529 |
| SLC2A9 | 1162 | 13101772 | A | -16.65 | 0.03403 |
| LIMCH1 | 1218 | 10026397 | A | -13.48 | 0.01236 |
| LIMCH1 | 1219 | 4386634 | G | -12.75 | 0.01513 |
| LIMCH1 | 1220 | 10006669 | A | -12.75 | 0.01513 |
| LIMCH1 | 1221 | 10026359 | T | -11.52 | 0.01534 |
| HERC3 | 1275 | 3796660 | A | -17.26 | 0.04705 |
| HERC3 | 1276 | 2924942 | T | -9.92 | 0.02467 |
| HERC3 | 1277 | 3017925 | T | -10.56 | 0.01611 |
| HERC3 | 1278 | 2972021 | A | -9.92 | 0.02467 |
| HERC3 | 1279 | 2972038 | T | -9.95 | 0.04291 |
| FAM13A1 | 1285 | 17817631 | T | 13.13 | 0.01292 |
| PAPSS1 | 1310 | 2522421 | G | -19.38 | 0.00665 |
| PAPSS1 | 1311 | 2522422 | A | -19.38 | 0.00665 |
| PAPSS1 | 1312 | 2522428 | C | -15.11 | 0.02685 |
| PAPSS1 | 1313 | 4956136 | C | -14.48 | 0.00984 |
| PAPSS1 | 1314 | 2158179 | G | -13.68 | 0.02076 |
| ANK2 | 1351 | 29306 | G | 9.03 | 0.02869 |
| GPR103 | 1380 | 17371804 | T | 8.54 | 0.03946 |
| GPR103 | 1382 | 2013332 | A | -15.60 | 0.00293 |
| GPR103 | 1385 | 17438900 | C | 8.54 | 0.03946 |
| MAML3 | 1395 | 11729794 | T | -11.65 | 0.01147 |
| MAML3 | 1398 | 1350036 | T | -10.40 | 0.01532 |
| IL15 | 1402 | 7671019 | A | -7.91 | 0.03444 |
| IL15 | 1403 | 6841939 | A | -8.22 | 0.03983 |
| IL15 | 1404 | 2139383 | C | 7.33 | 0.04215 |
| INPP4B | 1418 | 3775655 | C | 14.59 | 0.04575 |
| INPP4B | 1419 | 7697702 | C | 23.21 | 0.00625 |
| FSTL5 | 1461 | 45426 | T | -7.95 | 0.03181 |
| SEMA5A | 1535 | 985723 | A | 10.49 | 0.03673 |
| SEMA5A | 1545 | 150631 | A | -8.47 | 0.02109 |
| DNAH5 | 1563 | 16902955 | G | -8.35 | 0.04972 |
| DNAH5 | 1564 | 16902956 | C | -8.35 | 0.04972 |
| DNAH5 | 1565 | 16902957 | T | -8.35 | 0.04972 |
| DNAH5 | 1566 | 9312854 | C | -8.35 | 0.04972 |
| SLC1A3 | 1586 | 6451305 | C | 7.61 | 0.03994 |
| ITGA1 | 1615 | 1047481 | G | -9.04 | 0.00979 |
| ITGA1 | 1616 | 6882655 | A | -11.32 | 0.00790 |
| ITGA2 | 1617 | 989073 | A | -10.83 | 0.01968 |
| TNPO1 | 1641 | 34653 | T | -11.03 | 0.03445 |
| TNPO1 | 1643 | 266444 | C | -10.77 | 0.04440 |
| TNPO1 | 1644 | 2548331 | A | 9.45 | 0.01692 |
| FCHO2 | 1646 | 185435 | G | -10.77 | 0.04440 |
| FBXL17 | 1707 | 6596770 | C | 8.67 | 0.02289 |
| FBXL17 | 1709 | 2966805 | C | 8.32 | 0.03874 |
| FBXL17 | 1715 | 12521975 | T | 8.32 | 0.03874 |
| FBXL17 | 1716 | 6875297 | A | 8.40 | 0.03232 |
| KCNN2 | 1724 | 1457762 | A | -9.30 | 0.04162 |
| KCNN2 | 1725 | 4705504 | T | -8.88 | 0.04674 |
| KCNN2 | 1731 | 10519388 | C | -12.61 | 0.00346 |
| FTMT | 1747 | 10447274 | C | -9.11 | 0.02638 |
| TRPC7 | 1775 | 3777145 | T | 16.55 | 0.00264 |
| TRPC7 | 1778 | 950714 | G | 16.55 | 0.00264 |
| TRPC7 | 1779 | 2649691 | A | 13.42 | 0.01087 |
| ODZ2 | 1789 | 1421989 | G | -9.39 | 0.03638 |
| ODZ2 | 1798 | 3733988 | T | -8.71 | 0.01095 |
| ODZ2 | 1799 | 1422421 | T | -10.86 | 0.00222 |
| WWC1 | 1801 | 6893982 | C | 12.00 | 0.01232 |
| ELOVL5 | 1889 | 1429146 | T | -10.60 | 0.00409 |
| ELOVL5 | 1892 | 209500 | T | 9.45 | 0.00823 |
| ELOVL5 | 1893 | 9357760 | G | -7.45 | 0.04511 |
| ELOVL5 | 1894 | 9370196 | C | -7.45 | 0.04511 |
| RIMS1 | 1917 | 12525154 | A | 9.94 | 0.04287 |
| GABRR2 | 1919 | 4707535 | T | 8.96 | 0.04355 |
| GABRR2 | 1920 | 6900958 | T | -8.72 | 0.02592 |
| KLHL32 | 1921 | 6922974 | A | 9.93 | 0.01589 |
| SLC22A16 | 1926 | 9400390 | T | 9.54 | 0.01475 |
| NKAIN2 | 1957 | 173067 | A | -7.30 | 0.04805 |
| PLAGL1 | 1990 | 9321951 | T | -10.19 | 0.02081 |
| PLAGL1 | 1991 | 9390148 | A | 8.51 | 0.03238 |
| SYNE1 | 2006 | 1844356 | G | 8.52 | 0.04995 |
| PARK2 | 2044 | 7750033 | T | 9.93 | 0.01042 |
| PARK2 | 2045 | 9364662 | A | -12.54 | 0.00244 |
| PARK2 | 2046 | 9458610 | G | -12.54 | 0.00244 |
| PARK2 | 2048 | 2803045 | T | -8.51 | 0.03522 |
| PARK2 | 2049 | 9456810 | C | 9.23 | 0.01868 |
| SCIN | 2092 | 2240571 | G | -9.01 | 0.00416 |
| SCIN | 2093 | 1034856 | T | 7.28 | 0.04436 |
| FERD3L | 2106 | 6963269 | G | -9.84 | 0.02537 |
| FERD3L | 2107 | 6967799 | T | -9.58 | 0.04433 |
| FERD3L | 2109 | 6461422 | G | -9.35 | 0.04636 |
| FLJ22374 | 2148 | 10229281 | A | 14.63 | 0.02392 |
| FLJ22374 | 2149 | 10235270 | C | 10.38 | 0.03228 |
| PDEIC | 2157 | 17336016 | G | -8.89 | 0.03168 |
| EEPD1 | 2171 | 11765336 | C | 6.82 | 0.04860 |
| ABCA13 | 2214 | 6958356 | A | -8.36 | 0.03159 |
| HIP1 | 2255 | 1167802 | C | -11.66 | 0.00744 |
| MAGI2 | 2287 | 1990432 | A | -11.65 | 0.01842 |
| MAGI2 | 2288 | 6466535 | C | -14.03 | 0.00601 |
| ABCB4 | 2351 | 31662 | A | -15.48 | 0.02305 |
| ABCB4 | 2352 | 31669 | A | -17.13 | 0.01857 |
| CUX1 | 2404 | 2960266 | A | 8.19 | 0.04899 |
| CUX1 | 2406 | 10277680 | A | 7.91 | 0.04274 |
| CUX1 | 2407 | 12537077 | T | 7.18 | 0.04337 |
| DGK1 | 2477 | 4728414 | C | -7.79 | 0.04962 |
| CNTNAP2 | 2508 | 1637864 | T | 11.76 | 0.01832 |
| CNTNAP2 | 2509 | 1637840 | T | 11.76 | 0.01832 |
| PTPRN2 | 2528 | 6459879 | C | -11.04 | 0.00677 |
| PTPRN2 | 2529 | 1377374 | T | -11.04 | 0.00677 |
| PTPRN2 | 2530 | 11765319 | G | 13.12 | 0.00710 |
| PTPRN2 | 2531 | 4909222 | G | 13.91 | 0.00809 |
| CSMD1 | 2549 | 2938236 | T | -15.96 | 0.02797 |
| MCPH1 | 2559 | 2916741 | G | 13.30 | 0.00549 |
| MCPH1 | 2560 | 2442497 | G | 14.30 | 0.02113 |
| MCPH1 | 2561 | 2442496 | G | 14.28 | 0.00299 |
| MCPH1 | 2562 | 2442492 | T | 14.28 | 0.00299 |
| PSD3 | 2609 | 11782622 | C | -11.34 | 0.03407 |
| GFRA2 | 2618 | 17427734 | A | 13.63 | 0.00068 |
| PPP3CC | 2625 | 13271367 | G | -7.38 | 0.04313 |
| LYN | 2673 | 1546518 | C | -10.47 | 0.00218 |
| NKAIN3 | 2722 | 11989852 | A | 13.50 | 0.02102 |
| NKAIN3 | 2724 | 17279355 | G | 11.60 | 0.03751 |
| DEPDC2 | 2732 | 7007570 | G | 8.82 | 0.01437 |
| DEPDC2 | 2733 | 13262547 | G | -7.34 | 0.03639 |
| SAMD12 | 2828 | 16891338 | G | 22.53 | 0.02160 |
| FBXO32 | 2830 | 6990407 | G | 10.85 | 0.02815 |
| FER1L6 | 2835 | 7819813 | A | -7.58 | 0.04488 |
| FER1L6 | 2836 | 11784115 | A | -7.58 | 0.04488 |
| MTSS1 | 2856 | 6470298 | A | -39.87 | 0.01587 |
| SMARCA2 | 2930 | 7875236 | C | -13.17 | 0.00109 |
| SMARCA2 | 2931 | 2147263 | T | 7.25 | 0.04908 |
| SMARCA2 | 2932 | 4741650 | T | -10.01 | 0.01459 |
| PTPRD | 2943 | 10124689 | T | 9.95 | 0.01535 |
| PTPRD | 2944 | 7036240 | A | -9.33 | 0.01486 |
| PTPRD | 2946 | 10977089 | C | 8.82 | 0.03318 |
| PTPRD | 2947 | 2031216 | G | 9.27 | 0.01691 |
| PTPRD | 2948 | 4742502 | C | -8.17 | 0.02984 |
| PTPRD | 2951 | 7027844 | C | -8.34 | 0.03640 |
| MAMDC2 | 3014 | 1927143 | T | -15.03 | 0.00891 |
| MAMDC2 | 3017 | 2773362 | T | -11.08 | 0.04760 |
| TRPM3 | 3022 | 10868855 | C | 12.72 | 0.00762 |
| TRPM3 | 3024 | 12552572 | C | -8.80 | 0.04786 |
| GNAQ | 3062 | 4641131 | A | -10.73 | 0.01174 |
| GNAQ | 3063 | 4745687 | G | -10.16 | 0.01341 |
| GNAQ | 3064 | 11145653 | A | -10.16 | 0.01341 |
| GNAQ | 3066 | 964423 | T | -10.16 | 0.01341 |
| GNAQ | 3067 | 4744858 | A | -9.59 | 0.02990 |
| GNAQ | 3068 | 10781482 | T | -9.59 | 0.02990 |
| GNAQ | 3069 | 7038083 | G | 8.74 | 0.01344 |
| NFIL3 | 3092 | 7038686 | A | 14.05 | 0.00033 |
| NFIL3 | 3093 | 4743835 | A | 10.03 | 0.01351 |
| NFIL3 | 3094 | 4743836 | T | 12.65 | 0.00373 |
| NFIL3 | 3095 | 4743837 | A | 12.65 | 0.00373 |
| HIATL1 | 3099 | 9409765 | A | -9.40 | 0.01148 |
| HIATL1 | 3100 | 9409552 | G | -9.40 | 0.01148 |
| GABBR2 | 3103 | 3824451 | G | -12.63 | 0.02597 |
| SVEP1 | 3124 | 963143 | A | 9.18 | 0.00632 |
| SVEP1 | 3125 | 1571236 | T | -8.54 | 0.02390 |
| CDKSRAP2 | 3137 | 4837771 | T | -10.50 | 0.01603 |
| CDK5RAP2 | 3138 | 3750494 | G | -10.11 | 0.03530 |
| CDK5RAP2 | 3139 | 10818467 | C | -12.93 | 0.04769 |
| NPDC1 | 3202 | 4880195 | C | -9.44 | 0.03379 |
| PITRM1 | 3222 | 6602036 | A | 8.85 | 0.04411 |
| PRKCQ | 3227 | 582052 | G | 8.68 | 0.01225 |
| PRKCQ | 3229 | 1889001 | A | 8.68 | 0.01225 |
| CACNB2 | 3238 | 7923191 | G | 8.88 | 0.04690 |
| ARMC3 | 3243 | 10764350 | T | 10.37 | 0.00789 |
| ARMC3 | 3244 | 7899968 | A | 9.89 | 0.00865 |
| ARMC3 | 3246 | 7919349 | G | 12.06 | 0.00295 |
| ARMC3 | 3247 | 1171136 | A | 9.81 | 0.01694 |
| ARMC3 | 3250 | 10828389 | T | 8.40 | 0.03457 |
| MYO3A | 3283 | 16926660 | A | -20.85 | 0.01378 |
| SLC16A9 | 3294 | 10128501 | G | -7.21 | 0.03515 |
| CDH23 | 3305 | 17635709 | T | -9.49 | 0.02234 |
| KCNMA1 | 3319 | 2288840 | G | -8.61 | 0.04504 |
| KCNMA1 | 3321 | 7069982 | A | -8.37 | 0.04942 |
| NRG3 | 3339 | 17099789 | G | -9.84 | 0.02486 |
| NRG3 | 3344 | 342361 | G | 8.89 | 0.02602 |
| SORBS1 | 3363 | 11188299 | C | 48.52 | 0.00262 |
| SORCS3 | 3384 | 10160134 | A | 9.25 | 0.02013 |
| SORCS3 | 3385 | 697189 | C | 9.21 | 0.02224 |
| SORCS3 | 3386 | 697190 | C | 8.60 | 0.03054 |
| SORCS3 | 3387 | 7092104 | C | -8.26 | 0.04870 |
| HSPA12A | 3424 | 12777585 | C | -13.87 | 0.00037 |
| GALNTL4 | 3468 | 4910288 | A | 8.82 | 0.01247 |
| GALNTL4 | 3469 | 4910289 | C | 10.74 | 0.00517 |
| ARNTL | 3487 | 10832012 | C | 10.79 | 0.00913 |
| ARNTL | 3488 | 900144 | G | 10.54 | 0.00720 |
| ARNTL | 3491 | 10766073 | A | 11.30 | 0.00573 |
| ARNTL | 3492 | 10832020 | C | 14.35 | 0.00676 |
| ARNTL | 3493 | 9633835 | A | 10.92 | 0.00651 |
| DLG2 | 3526 | 11233565 | C | 18.75 | 0.03009 |
| DLG2 | 3527 | 3793946 | T | 7.93 | 0.03160 |
| ELMODI | 3557 | 494957 | C | -10.69 | 0.01804 |
| OPCML | 3568 | 3018406 | A | -10.82 | 0.01137 |
| LOC729025 | 3626 | 1671486 | T | -13.53 | 0.01429 |
| LOC729025 | 3627 | 10734895 | A | -12.68 | 0.01666 |
| CNOT2 | 3641 | 2118427 | T | -13.61 | 0.03659 |
| CNOT2 | 3647 | 11178177 | T | -10.06 | 0.00931 |
| CNOT2 | 3648 | 10506586 | A | -14.16 | 0.03891 |
| CNOT2 | 3650 | 3817486 | G | -14.16 | 0.03891 |
| CNOT2 | 3652 | 3817487 | A | -7.20 | 0.04872 |
| NAV3 | 3663 | 11106732 | G | 10.46 | 0.01720 |
| NAV3 | 3664 | 939458 | G | 9.28 | 0.03656 |
| NAV3 | 3675 | 10859738 | A | -8.05 | 0.04335 |
| NBEA | 3704 | 2322289 | C | 11.51 | 0.02846 |
| NBEA | 3705 | 867503 | C | 17.05 | 0.00137 |
| NBEA | 3706 | 9600364 | C | 13.81 | 0.04421 |
| NBEA | 3707 | 2243774 | C | 12.69 | 0.00924 |
| NBEA | 3708 | 1160720 | G | 12.69 | 0.00924 |
| NBEA | 3712 | 9544444 | A | -7.51 | 0.04242 |
| NBEA | 3713 | 1381476 | A | -7.12 | 0.04902 |
| FNDC3A | 3734 | 7321178 | G | -9.01 | 0.04261 |
| LMO7 | 3743 | 9530467 | G | -7.04 | 0.04602 |
| GPC6 | 3798 | 995810 | C | 10.54 | 0.03120 |
| GPC6 | 3799 | 1323974 | C | 10.54 | 0.03120 |
| NALCN | 3826 | 655365 | A | 10.44 | 0.03287 |
| WDR23 | 3854 | 6573565 | A | -8.66 | 0.03293 |
| WDR23 | 3855 | 11574503 | C | -8.66 | 0.03293 |
| SLC25A21 | 3878 | 2073248 | C | -19.35 | 0.02491 |
| LRFN5 | 3898 | 1241781 | G | -9.72 | 0.00583 |
| LRFN5 | 3900 | 4143896 | A | -10.07 | 0.00549 |
| LRFN5 | 3901 | 8020273 | G | -9.22 | 0.01337 |
| LRFN5 | 3903 | 17112319 | A | -8.32 | 0.02770 |
| RGS6 | 3930 | 2332699 | C | 8.96 | 0.01512 |
| NRXN3 | 3955 | 4020132 | T | 15.65 | 0.01514 |
| KCNK10 | 3965 | 17698533 | C | 8.48 | 0.01786 |
| KCNK10 | 3966 | 12185033 | T | 8.33 | 0.02138 |
| RYR3 | 4021 | 1980102 | A | -8.62 | 0.03936 |
| RYR3 | 4030 | 1390159 | T | 28.21 | 0.01695 |
| RYR3 | 4031 | 2132208 | A | 18.48 | 0.01782 |
| C15ORF41 | 4037 | 4923719 | G | -9.69 | 0.04662 |
| GLDN | 4056 | 17524318 | A | 9.41 | 0.04870 |
| GLDN | 4063 | 17648140 | G | 10.39 | 0.02065 |
| CGNL1 | 4074 | 2131730 | T | -12.53 | 0.01317 |
| CGNL1 | 4075 | 2131731 | G | -12.53 | 0.01317 |
| CGNL1 | 4077 | 2932209 | G | -12.10 | 0.01505 |
| ARNT2 | 4104 | 4778836 | T | 9.58 | 0.01514 |
| ARNT2 | 4105 | 7180510 | G | 8.54 | 0.03190 |
| A2BP1 | 4135 | 11648047 | C | 12.29 | 0.02677 |
| A2BP1 | 4136 | 2079753 | T | 10.95 | 0.04625 |
| TMC5 | 4141 | 1023446 | C | -7.95 | 0.02869 |
| MPHOSPH6 | 4162 | 7405231 | C | -8.41 | 0.01341 |
| MPHOSPH6 | 4168 | 11645604 | A | 9.19 | 0.03174 |
| MPHOSPH6 | 4170 | 2967355 | A | -8.48 | 0.04369 |
| CDH13 | 4202 | 889491 | T | -9.24 | 0.02597 |
| CRISPLD2 | 4217 | 11862286 | A | 12.81 | 0.04030 |
| ZFP161 | 4282 | 990072 | T | 9.74 | 0.03711 |
| PTPRM | 4297 | 2230601 | C | 9.94 | 0.00699 |
| PTPRM | 4299 | 507445 | T | 10.76 | 0.00511 |
| PTPRM | 4300 | 630778 | G | 8.63 | 0.02079 |
| PTPRM | 4301 | 6506572 | T | 7.69 | 0.02980 |
| PTPRM | 4302 | 9949149 | C | 7.69 | 0.02980 |
| NEDD4L | 4328 | 4940385 | C | -8.10 | 0.02725 |
| NEDD4L | 4333 | 12373325 | T | -8.73 | 0.03815 |
| CDH20 | 4348 | 1509128 | T | -7.19 | 0.03575 |
| FERMT1 | 4408 | 6117065 | A | 9.07 | 0.02962 |
| JAG1 | 4439 | 3748477 | T | 22.35 | 0.00872 |
| JAG1 | 4440 | 3748478 | T | 21.96 | 0.01027 |
| SNAI1 | 4524 | 6020157 | A | 9.47 | 0.03580 |
| SNAI1 | 4526 | 16995010 | C | 11.56 | 0.04852 |
| SNAI1 | 4528 | 6020187 | G | 12.58 | 0.01606 |
| NCAM2 | 4542 | 2826349 | G | -11.69 | 0.01381 |
| NCAM2 | 4543 | 2826351 | A | -11.69 | 0.01381 |
| NCAM2 | 4551 | 986917 | A | 9.05 | 0.02234 |
| PCP4 | 4557 | 2837279 | C | 10.32 | 0.04030 |
| PCP4 | 4559 | 2837283 | T | 10.32 | 0.04030 |
| ARVCF | 4567 | 1110477 | C | 34.11 | 0.00323 |
| ARVCF | 4568 | 887200 | C | 25.02 | 0.01002 |
| ARVCF | 4570 | 887203 | A | 34.11 | 0.00323 |
| HPS4 | 4574 | 5761552 | T | -7.93 | 0.02600 |
| HPS4 | 4577 | 5761557 | A | -11.17 | 0.04242 |
| EFCAB6 | 4604 | 6006521 | G | -8.28 | 0.03345 |
| EFCAB6 | 4607 | 1894489 | A | -8.28 | 0.03345 |

| **TABLE 6: Alleles Influencing Time to Discontinuation for Olanzapine** | | | | | |
|---|---|---|---|---|---|
| **Gene** | **Seq ID** | **NCBI RS#** | **Allele** | **Beta (Months)** | **P** |
| SCP2 | 39 | 7528867 | A | -5.09 | 0.03956 |
| PRKACB | 74 | 2642186 | G | -3.69 | 0.02840 |
| NGF | 82 | 7513144 | C | -6.44 | 0.00532 |
| NGF | 84 | 17033706 | A | -5.62 | 0.01618 |
| ATF6 | 100 | 2089083 | T | 5.20 | 0.02796 |
| FAM78B | 114 | 4072242 | T | 3.71 | 0.01635 |
| FAM78B | 115 | 4339838 | A | 3.71 | 0.01635 |
| FAM78B | 116 | 6426968 | T | 3.46 | 0.02396 |
| FAM78B | 117 | 1002160 | A | 3.74' | 0.02369 |
| FAM78B | 118 | 7521995 | C | 3.59 | 0.03082 |
| USH2A | 157 | 11120703 | C | -4.96 | 0.03071 |
| GNG4 | 192 | 10925976 | A | -3.29 | 0.02209 |
| GNG4 | 193 | 508208 | G | 3.22 | 0.02748 |
| RYR2 | 203 | 4659786 | A | -3.34 | 0.02006 |
| RYR2 | 204 | 4376724 | C | 3.24 | 0.03034 |
| RYR2 | 205 | 3924864 | C | -4.71 | 0.01844 |
| ASXL2 | 302 | 10180842 | G | -3.88 | 0.03025 |
| ASXL2 | 303 | 1076000 | A | -3.88 | 0.03025 |
| ASXL2 | 304 | 6758088 | A | -3.61 | 0.04493 |
| ASXL2 | 305 | 10182811 | T | -3.61 | 0.04493 |
| BRE | 323 | 6747817 | G | -3.45 | 0.02410 |
| SLC8A1 | 350 | 4952404 | T | -3.88 | 0.03259 |
| PRKCE | 376 | 666334 | T | 3.33 | 0.04249 |
| EPAS1 | 393 | 7589621 | A | 3.87 | 0.02990 |
| EPAS1 | 394 | 6707241 | C | -3.33 | 0.03095 |
| EPAS1 | 396 | 7594278 | T | -3.47 | 0.03104 |
| CCDC85A | 421 | 2869529 | C | -3.33 | 0.01914 |
| CTNNA2 | 442 | 6719738 | T | 5.42 | 0.00368 |
| CTNNA2 | 443 | 2566539 | T | 3.67 | 0.02132 |
| CTNNA2 | 444 | 1971766 | A | 4.50 | 0.00543 |
| CTNNA2 | 464 | 318362 | C | 3.50 | 0.02918 |
| LRP1B | 508 | 2171169 | A | 6.96 | 0.00601 |
| LRP1B | 511 | 1949871 | C | 3.23 | 0.03086 |
| LRP1B | 514 | 4954907 | T | 3.73 | 0.04161 |
| KYNU | 525 | 2278587 | A | 5.77 | 0.03983 |
| ARHGAP15 | 538 | 11895997 | A | 4.21 | 0.00457 |
| ARHGAP15 | 559 | 16823114 | A | 4.94 | 0.00164 |
| ARHGAP15 | 560 | 10928200 | T | 5.03 | 0.00232 |
| ARHGAP15 | 561 | 13031917 | C | 5.03 | 0.00232 |
| CACNB4 | 573 | 10497094 | G | 5.55 | 0.01135 |
| PKP4 | 594 | 4664963 | G | -7.01 | 0.03893 |
| SCN9A | 628 | 4447616 | C | 4.22 | 0.00360 |
| SCN9A | 629 | 6732627 | A | 4.07 | 0.00572 |
| CERKL | 631 | 10490688 | T | -3.69 | 0.02450 |
| CERKL | 632 | 895901 | A | -3.69 | 0.02450 |
| CERKL | 634 | 1967351 | C | 3.94 | 0.01038 |
| PARD3B | 657 | 2878473 | C | 3.87 | 0.02264 |
| NRP2 | 672 | 849565 | C | 4.93 | 0.00871 |
| CNTN4 | 761 | 1685456 | C | 3.44 | 0.02879 |
| ARPP-21 | 819 | 3772395 | T | 4.58 | 0.04977 |
| ARPP-21 | 822 | 2305234 | A | 4.58 | 0.04977 |
| ULK4 | 829 | 6763756 | T | 3.89 | 0.00302 |
| ULK4 | 832 | 9827383 | C | 4.75 | 0.00401 |
| ULK4 | 833 | 10510719 | A | 3.83 | 0.01676 |
| ULK4 | 834 | 9311270 | C | 4.11 | 0.00161 |
| ULK4 | 836 | 9311275 | C | 3.61 | 0.00782 |
| ULK4 | 842 | 11129908 | A | 2.77 | 0.03492 |
| FHIT | 918 | 7621056 | A | -3.75 | 0.02014 |
| FHIT | 919 | 11709440 | C | -3.72 | 0.04271 |
| SYNPR | 954 | 1505600 | C | 4.67 | 0.00992 |
| SYNPR | 955 | 4616634 | G | 4.67 | 0.00992 |
| PRICKLE2 | 965 | 696019 | A | 3.12 | 0.04836 |
| EPHA6 | 1004 | 2873617 | G | 6.10 | 0.03693 |
| PLCXD2 | 1010 | 1513331 | C | -6.77 | 0.00092 |
| CDGAP | 1021 | 10934491 | C | -3.51 | 0.03857 |
| CPNE4 | 1049 | 6792146 | A | -4.53 | 0.02011 |
| CPNE4 | 1050 | 16836991 | T | -4.69 | 0.04000 |
| SERPINI2 | 1089 | 17246389 | C | -4.35 | 0.00680 |
| HTR3D | 1128 | 939334 | G | -3.36 | 0.03207 |
| LDB2 | 1172 | 10755217 | G | 3.92 | 0.02075 |
| LDB2 | 1173 | 2135442 | A | 3.14 | 0.04385 |
| LDB2 | 1181 | 1384606 | T | 4.18 | 0.00961 |
| SLIT2 | 1189 | 609535 | A | 2.98 | 0.03598 |
| SLIT2 | 1191 | 9992591 | C | 3.01 | 0.04079 |
| PDLIM5 | 1301 | 951613 | A | -2.88 | 0.04914 |
| COL25A1 | 1317 | 2305438 | A | -4.35 | 0.00204 |
| COL25A1 | 1319 | 13104307 | T | 4.25 | 0.00418 |
| COL25A1 | 1334 | 987695 | G | 3.78 | 0.01371 |
| COL25A1 | 1335 | 7675657 | A | 3.79 | 0.01212 |
| ANK2 | 1344 | 10488904 | A | -3.45 | 0.04253 |
| ANK2 | 1351 | 29306 | G | 3.42 | 0.04294 |
| ANK2 | 1353 | 29311 | A | 3.79 | 0.02417 |
| NDST3 | 1369 | 2291416 | G | 4.80 | 0.02019 |
| NDST3 | 1370 | 2254215 | A | 4.80 | 0.02019 |
| NDST3 | 1372 | 2389498 | T | 4.80 | 0.02019 |
| GPR103 | 1381 | 13110738 | G | -4.32 | 0.01034 |
| MAML3 | 1387 | 2246759 | G | 2.68 | 0.04943 |
| MAML3 | 1388 | 13105452 | T | -2.96 | 0.04884 |
| DCLK2 | 1441 | 12513356 | G | 7.25 | 0.02007 |
| FSTL5 | 1452 | 4501178 | C | -3.91 | 0.03044 |
| FSTL5 | 1457 | 10013537 | T | -3.59 | 0.02595 |
| FSTL5 | 1458 | 1560925 | A | -3.56 | 0.03737 |
| FSTL5 | 1459 | 7679398 | C | -3.18 | 0.04707 |
| PALLD | 1500 | 13122019 | A | -4.08 | 0.00577 |
| PALLD | 1501 | 1039385 | G | -3.76 | 0.01217 |
| ENPP6 | 1520 | 11132220 | A | -3.91 | 0.00434 |
| ENPP6 | 1521 | 6829678 | C | -3.38 | 0.01190 |
| ENPP6 | 1523 | 6831986 | C | -3.32 | 0.01562 |
| ENPP6 | 1524 | 17679120 | A | -3.10 | 0.02310 |
| AHRR | 1530 | 3734145 | T | -4.20 | 0.00618 |
| AHRR | 1531 | 2721004 | T | -3.75 | 0.01586 |
| SEMA5A | 1536 | 17235037 | T | -5.14 | 0.04437 |
| DNAH5 | 1548 | 6862469 | C | -3.26 | 0.04034 |
| DNAH5 | 1549 | 6876673 | T | -3.38 | 0.04722 |
| DNAH5 | 1554 | 1502050 | G | -4.88 | 0.00553 |
| DNAH5 | 1555 | 10513155 | A | 3.55 | 0.01611 |
| DNAH5 | 1563 | 16902955 | G | -3.64 | 0.01359 |
| DNAH5 | 1564 | 16902956 | C | -3.64 | 0.01359 |
| DNAH5 | 1566 | 9312854 | C | -3.64 | 0.01359 |
| CDH10 | 1575 | 3822429 | T | 3.02 | 0.01856 |
| ITGA1 | 1603 | 6883810 | A | -3.09 | 0.04953 |
| ITGA1 | 1606 | 10513001 | G | -3.93 | 0.01402 |
| GPR98 | 1682 | 168743 | G | 3.61 | 0.04407 |
| FBXL17 | 1709 | 2966805 | C | -3.16 | 0.02699 |
| FBXL17 | 1710 | 2922434 | A | -3.16 | 0.02699 |
| FBXL17 | 1715 | 12521975 | T | -2.83 | 0.04529 |
| SNCAIP | 1749 | 7444144 | G | -4.03 | 0.02367 |
| SNCAIP | 1751 | 17149141 | A | -5.25 | 0.00917 |
| ADAMTS19 | 1767 | 30651 | G | 4.12 | 0.01213 |
| ADAMTS19 | 1769 | 4835975 | A | 4.28 | 0.01770 |
| SLC22A23 | 1808 | 7775219 | C | 4.84 | 0.01188 |
| SLC17A4 | 1847 | 4712969 | A | 6.26 | 0.03299 |
| SLC17A4 | 1849 | 4712972 | A | 6.26 | 0.03299 |
| SLC17A4 | 1851 | 1892253 | T | 6.26 | 0.03299 |
| SLC17A1 | 1853 | 1324082 | A | 7.45 | 0.00016 |
| SLC17A1 | 1855 | 13200784 | T | 5.96 | 0.00298 |
| SLC17A3 | 1860 | 1165158 | T | 5.74 | 0.00507 |
| SLC17A2 | 1864 | 13207673 | T | 8.13 | 0.03010 |
| BTN3A1 | 1873 | 10946826 | G | 4.06 | 0.04974 |
| BTNL2 | 1876 | 3806156 | T | 3.92 | 0.01504 |
| BRD2 | 1879 | 194677 | T | 7.97 | 0.03348 |
| ABCC10 | 1885 | 4714684 | C | -3.28 | 0.02282 |
| GABRR2 | 1920 | 6900958 | T | 4.85 | 0.00568 |
| KLHL32 | 1922 | 1766519 | C | 4.36 | 0.04226 |
| SLC22A16 | 1927 | 9320331 | A | -2.98 | 0.03190 |
| SLC22A16 | 1928 | 9481067 | G | -3.15 | 0.02376 |
| TRDN | 1949 | 2317707 | T | 3.44 | 0.04047 |
| NKAIN2 | 1956 | 4897531 | T | -5.56 | 0.00561 |
| PDE7B | 1979 | 9385741 | A | -9.79 | 0.02169 |
| PDE7B | 1981 | 7772608 | G | -9.79 | 0.02169 |
| SYNE1 | 2005 | 2763015 | A | -3.04 | 0.04326 |
| PDE10A | 2058 | 220771 | T | -4.31 | 0.03248 |
| PDE10A | 2065 | 9459474 | C | 3.29 | 0.04340 |
| ETV1 | 2098 | 4721291 | C | -3.57 | 0.00685 |
| SNX13 | 2100 | 2177652 | T | -3.01 | 0.03839 |
| PLEKHA8 | 2135 | 10951257 | G | -5.03 | 0.01445 |
| FLJ22374 | 2145 | 7781249 | A | -3.16 | 0.04856 |
| BMPER | 2166 | 2110856 | C | 3.31 | 0.03886 |
| ABCA13 | 2194 | 17132289 | T | 5.86 | 0.04658 |
| ABCA13 | 2196 | 17548783 | C | 4.09 | 0.00678 |
| ABCA13 | 2198 | 17729647 | G | 4.10 | 0.02276 |
| ABCA13 | 2199 | 7778020 | A | 5.30 | 0.03759 |
| ABCA13 | 2213 | 10216298 | G | 4.48 | 0.04991 |
| WBSCR17 | 2231 | 12538186 | G | -3.31 | 0.02926 |
| WBSCR17 | 2232 | 757841 | C | -3.31 | 0.02926 |
| WBSCR17 | 2234 | 17593300 | G | -3.31 | 0.02926 |
| WBSCR17 | 2235 | 2190223 | C | -3.03 | 0.04859 |
| GTF2IRD1 | 2252 | 2267834 | G | -3.25 | 0.03665 |
| MAGI2 | 2266 | 6466021 | G | 3.05 | 0.04279 |
| MAGI2 | 2281 | 7803949 | A | 3.75 | 0.04130 |
| MAGI2 | 2299 | 2364920 | G | 3.77 | 0.03832 |
| CACNA2D1 | 2308 | 258728 | A | 4.06 | 0.01520 |
| PCLO | 2310 | 10954689 | T | 4.69 | 0.00249 |
| PCLO | 2320 | 9656532 | A | -4.15 | 0.01316 |
| PCLO | 2321 | 2715148 | A | 3.39 | 0.01526 |
| PCLO | 2322 | 2522833 | C | 3.54 | 0.02043 |
| PCLO | 2324 | 2522843 | A | 3.15 | 0.03569 |
| PCLO | 2326 | 1986742 | G | -3.01 | 0.04113 |
| SEMA3E | 2344 | 169992 | C | 3.18 | 0.02776 |
| ABCB4 | 2352 | 31669 | A | -7.80 | 0.00472 |
| PONI | 2366 | 662 | G | 3.57 | 0.02993 |
| PONI | 2382 | 2057681 | G | 3.61 | 0.03020 |
| DYNC1I1 | 2390 | 4357216 | C | -7.82 | 0.00017 |
| DYNC1I1 | 2391 | 10243540 | G | -4.51 | 0.02003 |
| DYNC1I1 | 2396 | 42066 | A | -5.13 | 0.03555 |
| KCND2 | 2414 | 7795646 | G | 4.45 | 0.03295 |
| KCND2 | 2416 | 5009999 | T | 7.17 | 0.00030 |
| CNTNAP2 | 2510 | 2717829 | C | -4.67 | 0.04813 |
| PTPRN2 | 2514 | 11771772 | A | 2.76 | 0.04281 |
| PTPRN2 | 2516 | 221242 | A | -7.62 | 0.00810 |
| PTPRN2 | 2523 | 10277112 | A | 4.39 | 0.00576 |
| CSMD1 | 2536 | 2930282 | C | -4.67 | 0.02689 |
| CSMD1 | 2537 | 622765 | A | -4.67 | 0.02689 |
| SGCZ | 2580 | 2898389 | C | -3.13 | 0.03705 |
| SGCZ | 2581 | 7000337 | T | 3.79 | 0.01891 |
| SGCZ | 2582 | 10092634 | C | 4.52 | 0.00250 |
| SGCZ | 2583 | 1551816 | G | 3.71 | 0.02480 |
| SGCZ | 2592 | 17119601 | G | 3.63 | 0.02307 |
| SGCZ | 2595 | 7817295 | A | -3.49 | 0.01689 |
| GFRA2 | 2618 | 17427734 | A | -3.28 | 0.02327 |
| SNTG1 | 2661 | 12545915 | T | -3.21 | 0.04681 |
| NKAIN3 | 2710 | 1383154 | A | 3.65 | 0.02232 |
| CRISPLD1 | 2757 | 1455811 | C | 4.21 | 0.03212 |
| CRISPLD1 | 2758 | 13248650 | G | 3.64 | 0.04136 |
| CRISPLD1 | 2759 | 16939030 | C | -3.63 | 0.04272 |
| MMP16 | 2768 | 10504845 | T | -3.96 | 0.03978 |
| BAALC | 2785 | 1845430 | A | -3.09 | 0.04456 |
| ZFPM2 | 2792 | 12677825 | T | 2.94 | 0.04721 |
| SAMD12 | 2819 | 10955887 | C | -3.37 | 0.02118 |
| SAMD12 | 2820 | 4876841 | A | -3.37 | 0.02118 |
| SAMD12 | 2821 | 10096317 | A | -3.37 | 0.02118 |
| SAMD12 | 2822 | 7830423 | C | -3.37 | 0.02118 |
| DDEF1 | 2860 | 10956511 | T | -3.24 | 0.03378 |
| DDEF1 | 2862 | 12546206 | A | -3.05 | 0.03773 |
| DDEF1 | 2877 | 1123205 | G | -3.70 | 0.01429 |
| ADCY8 | 2889 | 17248206 | G | 4.16 | 0.00473 |
| ADCY8 | 2891 | 7464362 | G | -3.53 | 0.00509 |
| ADCY8 | 2892 | 11781997 | A | -3.21 | 0.01501 |
| ADCY8 | 2893 | 7459573 | G | -2.80 | 0.03771 |
| ADCY8 | 2894 | 4736722 | C | 3.54 | 0.01032 |
| SMARCA2 | 2922 | 16937123 | C | -6.17 | 0.01464 |
| IFT74 | 2985 | 2095405 | C | -4.04 | 0.03292 |
| PIP5K1B | 2999 | 872077 | A | 4.48 | 0.01597 |
| TMC1 | 3030 | 6560293 | A | -2.93 | 0.04807 |
| TMC1 | 3032 | 13285999 | A | -3.63 | 0.01250 |
| TMC1 | 3033 | 10869190 | A | 3.29 | 0.02338 |
| TMC1 | 3042 | 2501914 | T | -2.98 | 0.04993 |
| NTRK2 | 3071 | 11140721 | C | -4.71 | 0.03889 |
| DIRAS2 | 3088 | 7028171 | A | -3.45 | 0.04791 |
| NFIL3 | 3090 | 7869771 | C | 3.78 | 0.01190 |
| GABBR2 | 3102 | 10985811 | C | 4.14 | 0.01432 |
| GABBR2 | 3104 | 13286336 | C | 3.72 | 0.02693 |
| GABBR2 | 3105 | 16914811 | A | 3.68 | 0.03133 |
| ZNF618 | 3131 | 1965753 | T | 3.40 | 0.02344 |
| ZNF618 | 3132 | 10121424 | T | -4.06 | 0.01265 |
| ZNF618 | 3133 | 1408115 | G | 3.40 | 0.02344 |
| CDK5RAP2 | 3140 | 4837782 | T | 5.54 | 0.04600 |
| LHX6 | 3149 | 7849627 | G | -3.07 | 0.04970 |
| ABL1 | 3165 | 2855160 | T | 2.78 | 0.03940 |
| EXOSC2 | 3166 | 4740366 | G | 4.17 | 0.01924 |
| PNPLA7 | 3204 | 10867123 | G | 3.89 | 0.03144 |
| ARHGAP21 | 3253 | 10764482 | G | -4.60 | 0.00968 |
| ARHGAP21 | 3254 | 7070456 | C | -5.75 | 0.00076 |
| ARHGAP21 | 3255 | 10828686 | C | -4.58 | 0.00507 |
| ARHGAP21 | 3256 | 4415644 | T | -4.61 | 0.01621 |
| ARHGAP21 | 3257 | 7897303 | A | -4.61 | 0.01621 |
| MYO3A | 3267 | 993397 | A | -3.33 | 0.02291 |
| MYO3A | 3269 | 17666079 | C | 3.13 | 0.01616 |
| MYO3A | 3280 | 1999240 | C | -2.89 | 0.04492 |
| MYO3A | 3281 | 3758447 | C | 2.96 | 0.02617 |
| JMJD1C | 3296 | 10761739 | C | -5.56 | 0.00016 |
| JMJD1C | 3297 | 10995505 | A | 3.25 | 0.03575 |
| JMJD1C | 3298 | 10740118 | C | -5.56 | 0.00016 |
| JMJD1C | 3300 | 12355784 | A | -4.21 | 0.00317 |
| JMJD1C | 3301 | 9629895 | A | 3.28 | 0.04259 |
| JMJD1C | 3302 | 3999089 | C | -4.22 | 0.00356 |
| NRG3 | 3339 | 17099789 | G | 3.87 | 0.03706 |
| NRG3 | 3346 | 512064 | A | -3.52 | 0.01230 |
| NRG3 | 3347 | 495978 | A | -3.24 | 0.03001 |
| NRG3 | 3353 | 17763320 | A | -5.32 | 0.04684 |
| SORBS1 | 3358 | 11188287 | G | -3.73 | 0.04151 |
| SORBS1 | 3362 | 11188298 | C | 2.87 | 0.04700 |
| ATRNL1 | 3421 | 180675 | A | -4.33 | 0.01804 |
| ATE1 | 3431 | 1693688 | C | -3.07 | 0.03457 |
| ATE1 | 3435 | 1219505 | T | -2.99 | 0.04504 |
| SPON1 | 3501 | 11023054 | A | 3.15 | 0.02514 |
| SPON1 | 3502 | 10832165 | A | 2.89 | 0.03895 |
| SPON1 | 3503 | 7112850 | A | 2.89 | 0.03895 |
| SPON1 | 3504 | 10766134 | G | 2.89 | 0.03895 |
| KCNA4 | 3517 | 7931190 | T | 3.98 | 0.01256 |
| KCNA4 | 3518 | 605765 | G | 3.44 | 0.01298 |
| LOC729025 | 3626 | 1671486 | T | -4.36 | 0.02401 |
| ITPR2 | 3640 | 2230375 | G | -3.52 | 0.03207 |
| CNOT2 | 3644 | 11178116 | C | 6.30 | 0.00528 |
| CNOT2 | 3645 | 7312236 | C | 3.56 | 0.03545 |
| CNOT2 | 3648 | 10506586 | A | -6.01 | 0.03028 |
| CNOT2 | 3650 | 3817486 | G | -6.01 | 0.03028 |
| CNOT2 | 3654 | 1495345 | C | -3.23 | 0.01523 |
| NAV3 | 3678 | 1375287 | C | -3.04 | 0.04760 |
| PLA2G1B | 3687 | 5637 | A | 4.83 | 0.00749 |
| N4BP2L2 | 3700 | 3742318 | C | -3.64 | 0.04574 |
| NBEA | 3722 | 3818423 | C | 4.64 | 0.04656 |
| LMO7 | 3752 | 7986131 | T | 3.01 | 0.04927 |
| SLAIN1 | 3756 | 1279445 | G | 6.48 | 0.01371 |
| SLAIN1 | 3757 | 1279446 | C | 3.81 | 0.02101 |
| GPC5 | 3771 | 553717 | A | -6.18 | 0.00873 |
| GPC5 | 3772 | 342678 | A | -6.02 | 0.01104 |
| GPC5 | 3773 | 342677 | A | -4.13 | 0.04743 |
| GPC5 | 3792 | 17267453 | C | 5.64 | 0.01743 |
| GPC6 | 3803 | 3899317 | A | -3.95 | 0.01952 |
| GPC6 | 3809 | 7987964 | T | -3.70 | 0.01325 |
| LRFN5 | 3895 | 1597045 | T | -3.81 | 0.01313 |
| LRFN5 | 3897 | 4904776 | C | 3.58 | 0.02275 |
| SAMD4A | 3912 | 1212968 | C | -3.87 | 0.03653 |
| RPS6KA5 | 3973 | 941847 | T | 3.22 | 0.04847 |
| RPS6KA5 | 3975 | 1286060 | C | 3.55 | 0.02952 |
| RPS6KA5 | 3978 | 1075014 | C | 3.50 | 0.01889 |
| RPS6KA5 | 3980 | 7492628 | G | 3.63 | 0.01120 |
| BCL11B | 4000 | 2793321 | T | 4.42 | 0.01012 |
| BCL11B | 4001 | 1257446 | C | 5.33 | 0.00168 |
| BCL11B | 4002 | 1257416 | G | 4.49 | 0.01570 |
| C15ORF41 | 4042 | 16964046 | T | 6.14 | 0.03662 |
| C15ORF41 | 4045 | 16964103 | T | 5.94 | 0.04342 |
| C15ORF41 | 4046 | 16964104 | G | 5.94 | 0.04342 |
| C15ORF41 | 4047 | 17508461 | T | 5.94 | 0.04342 |
| RASGRP1 | 4054 | 6492847 | G | 5.25 | 0.00540 |
| CLK3 | 4086 | 11072496 | T | -4.09 | 0.03208 |
| CLK3 | 4087 | 7161903 | G | -4.09 | 0.03208 |
| ARNT2 | 4101 | 4778800 | T | 3.64 | 0.03664 |
| A2BP1 | 4137 | 8054765 | G | 4.12 | 0.01511 |
| CDH13 | 4182 | 1000078 | C | 4.19 | 0.00832 |
| CDH13 | 4183 | 4783266 | A | 3.21 | 0.04507 |
| CDH13 | 4184 | 11646849 | A | 2.86 | 0.03936 |
| CDH13 | 4186 | 4598906 | T | 3.25 | 0.03776 |
| ALOX12B | 4218 | 9914077 | G | -4.28 | 0.00474 |
| CA10 | 4247 | 4635383 | C | 3.15 | 0.04481 |
| CA10 | 4250 | 9303606 | C | -3.34 | 0.02638 |
| CA10 | 4251 | 12945099 | C | -2.91 | 0.03607 |
| DLGAP1 | 4279 | 9948987 | A | -5.08 | 0.00652 |
| ZFP161 | 4281 | 581144 | T | 3.23 | 0.04853 |
| PTPRM | 4291 | 1893224 | C | -7.33 | 0.03051 |
| KIAA0802 | 4307 | 16954483 | C | 4.30 | 0.03846 |
| NEDD4L | 4334 | 9966665 | G | 4.16 | 0.03637 |
| ZNF544 | 4388 | 260454 | T | -2.55 | 0.04837 |
| ATRN | 4392 | 2670314 | A | -3.66 | 0.01608 |
| PRNT | 4396 | 2236284 | G | 4.15 | 0.00569 |
| PRNT | 4397 | 6139537 | T | -4.72 | 0.00574 |
| PRNT | 4400 | 6052824 | A | -3.47 | 0.02246 |
| PRNT | 4402 | 6107527 | G | -3.95 | 0.00329 |
| PRNT | 4407 | 6084876 | T | 3.85 | 0.00683 |
| PLCB1 | 4414 | 6055577 | A | -7.21 | 0.00012 |
| PLCB1 | 4415 | 2235212 | A | -5.36 | 0.00098 |
| PLCB1 | 4417 | 16994533 | G | -4.89 | 0.02509 |
| PLCB1 | 4418 | 6077339 | C | 3.21 | 0.02841 |
| PLCB1 | 4419 | 6055697 | A | 3.21 | 0.02841 |
| MACROD2 | 4454 | 2236008 | T | 3.63 | 0.02258 |
| MACROD2 | 4456 | 11697488 | T | 4.22 | 0.00955 |
| NCAM2 | 4546 | 2826830 | G | -4.76 | 0.00790 |
| NCAM2 | 4547 | 3787603 | G | -4.40 | 0.01385 |
| NCAM2 | 4548 | 2826841 | T | -4.20 | 0.01788 |
| MIRN155 | 4554 | 2828908 | T | 4.72 | 0.02933 |
| PDE9A | 4561 | 2269139 | T | -5.29 | 0.02419 |
| PACSIN2 | 4590 | 2072883 | G | 3.22 | 0.04840 |

| **TABLE 7: Alleles Influencing Time to Discontinuation for Risperidone** | | | | | |
|---|---|---|---|---|---|
| **Gene** | **Seq ID** | **NCBI RS#** | **Allele** | **Beta (Months)** | **P** |
| KIFIB | 4 | 912962 | G | 2.65 | 0.02340000 |
| RP1-21O18.1 | 7 | 6663699 | G | 3.33 | 0.01853000 |
| RP1-21O18.1 | 8 | 7518989 | C | 3.97 | 0.04221000 |
| RP1-21O18.1 | 15 | 4501834 | C | 2.90 | 0.03302000 |
| PRKACB | 74 | 2642186 | G | -4.52 | 0.00893200 |
| SYT6 | 78 | 611514 | A | -2.76 | 0.03642000 |
| NGF | 80 | 6327 | A | 3.57 | 0.00304100 |
| NGF | 81 | 12145726 | G | -3.10 | 0.02638000 |
| SEC16B | 128 | 2862300 | G | -3.05 | 0.02147000 |
| RYR2 | 212 | 3766884 | T | 5.09 | 0.00242100 |
| CHRM3 | 227 | 663927 | A | 3.23 | 0.02766000 |
| FMN2 | 232 | 10926147 | G | -2.63 | 0.03394000 |
| PLD5 | 266 | 400904 | T | 2.77 | 0.04121000 |
| C2ORF46 | 276 | 3102945 | C | 3.27 | 0.01632000 |
| C2ORF46 | 279 | 10210006 | C | -2.96 | 0.03525000 |
| KLHL29 | 298 | 7560892 | A | 2.44 | 0.04522000 |
| SLC8A1 | 348 | 389109 | T | 2.98 | 0.03861000 |
| OTX1 | 431 | 11125946 | A | -2.44 | 0.04789000 |
| CTNNA2 | 445 | 2566542 | C | -3.25 | 0.04001000 |
| CTNNA2 | 457 | 2165975 | T | 6.80 | 0.00024050 |
| CTNNA2 | 460 | 160864 | T | 2.92 | 0.00717700 |
| CTNNA2 | 463 | 318369 | G | 2.92 | 0.00935100 |
| CTNNA2 | 465 | 895388 | T | 6.59 | 0.00305500 |
| CTNNA2 | 466 | 1930 | T | 3.67 | 0.01004000 |
| CTNNA2 | 467 | 12464777 | T | 2.70 | 0.04571000 |
| NXPH2 | 499 | 11686876 | T | 3.66 | 0.00602200 |
| LRP1B | 502 | 1486958 | G | 2.17 | 0.04586000 |
| LRP1B | 503 | 1492388 | T | 2.17 | 0.04586000 |
| LRP1B | 507 | 4447563 | G | 2.56 | 0.03286000 |
| KYNU | 527 | 10176234 | A | 2.91 | 0.01925000 |
| FMNL2 | 585 | 11682487 | C | 2.87 | 0.03337000 |
| PKP4 | 599 | 2711066 | C | -3.38 | 0.03270000 |
| SCN3A | 618 | 11677254 | C | 2.99 | 0.01443000 |
| PDE1A | 638 | 2623422 | G | 3.22 | 0.02246000 |
| PDE1A | 641 | 6736414 | T | -2.90 | 0.02767000 |
| ERBB4 | 680 | 1992029 | G | -2.89 | 0.04336000 |
| COL4A3 | 698 | 6741679 | A | 3.61 | 0.00245000 |
| COL4A3 | 701 | 6756898 | C | -3.20 | 0.03855000 |
| CNTN6 | 736 | 11925058 | C | -3.10 | 0.01551000 |
| CNTN6 | 741 | 1353825 | T | 2.59 | 0.04167000 |
| CNTN6 | 742 | 265799 | G | -2.51 | 0.03496000 |
| CNTN6 | 746 | 3772290 | T | -2.52 | 0.03244000 |
| DAZL | 801 | 4685357 | T | -2.92 | 0.01258000 |
| DAZL | 802 | 6787063 | G | -2.58 | 0.02359000 |
| RARB | 806 | 1286738 | T | -3.52 | 0.01698000 |
| RARB | 808 | 1656463 | A | -3.42 | 0.01978000 |
| ERC2 | 896 | 885211 | A | 3.24 | 0.01495000 |
| MAGI1 | 973 | 17073075 | T | 2.85 | 0.03110000 |
| FOXP1 | 984 | 2196356 | C | 3.67 | 0.01155000 |
| FOXP1 | 985 | 2597312 | C | 2.41 | 0.04753000 |
| EPHA6 | 1008 | 6439265 | T | 3.21 | 0.03918000 |
| EPHA6 | 1009 | 2317212 | A | 3.07 | 0.04515000 |
| CPNE4 | 1053 | 13319291 | G | 2.70 | 0.03769000 |
| CPNE4 | 1054 | 9812534 | C | -3.54 | 0.00697100 |
| EPHB1 | 1067 | 936323 | T | 2.37 | 0.03698000 |
| EPHB1 | 1070 | 4894286 | A | -2.48 | 0.04769000 |
| CLSTN2 | 1079 | 9875409 | G | 3.68 | 0.00858000 |
| CLSTN2 | 1080 | 347975 | G | 3.61 | 0.00676700 |
| CLSTN2 | 1081 | 347973 | A | 3.66 | 0.00883400 |
| NLGN1 | 1120 | 12636180 | T | 3.65 | 0.00836300 |
| LEPREL1 | 1139 | 893054 | T | 3.07 | 0.02936000 |
| LEPREL1 | 1140 | 573533 | A | 3.07 | 0.02936000 |
| UBXD7 | 1154 | 7374260 | C | -2.34 | 0.03482000 |
| SLIT2 | 1188 | 10516357 | A | -3.39 | 0.01791000 |
| SLIT2 | 1190 | 2168802 | T | -3.23 | 0.03660000 |
| LPHN3 | 1231 | 995447 | C | 6.10 | 0.02237000 |
| SCD5 | 1268 | 6852540 | C | -2.73 | 0.02726000 |
| FAM13A1 | 1280 | 7657817 | T | -3.41 | 0.03091000 |
| COL25A1 | 1334 | 987695 | G | 3.06 | 0.01774000 |
| COL25A1 | 1337 | 6816017 | G | 3.38 | 0.01769000 |
| COL25A1 | 1338 | 7673867 | A | 3.38 | 0.01769000 |
| MAML3 | 1392 | 10024138 | T | -2.94 | 0.02936000 |
| MAML3 | 1394 | 2271391 | T | -2.92 | 0.03381000 |
| MAML3 | 1396 | 11733737 | T | 2.48 | 0.04880000 |
| MAML3 | 1397 | 17051001 | T | -2.46 | 0.04833000 |
| DCLK2 | 1432 | 4405992 | A | 3.21 | 0.02212000 |
| DCLK2 | 1435 | 12503886 | A | 3.20 | 0.02214000 |
| DCLK2 | 1437 | 10001651 | C | 4.30 | 0.00120900 |
| DCLK2 | 1438 | 11735949 | C | 3.92 | 0.00246700 |
| DCLK2 | 1439 | 17503402 | A | 5.94 | 0.00000006 |
| DCLK2 | 1440 | 11727182 | G | 6.09 | 0.00000015 |
| DCLK2 | 1442 | 6535725 | A | 5.25 | 0.00000354 |
| FSTL5 | 1455 | 6825091 | A | -3.98 | 0.00353700 |
| FSTL5 | 1456 | 7442468 | C | -3.72 | 0.00854400 |
| TLL1 | 1475 | 719753 | G | -4.17 | 0.02645000 |
| TLL1 | 1481 | 1391334 | G | -2.66 | 0.03191000 |
| DNAH5 | 1562 | 13154455 | G | 2.55 | 0.03762000 |
| MYO10 | 1570 | 11750538 | G | 3.76 | 0.00464500 |
| MYO10 | 1571 | 1560089 | T | 4.19 | 0.00092940 |
| MYO10 | 1572 | 890910 | A | 4.06 | 0.00143400 |
| MYO10 | 1573 | 11133860 | G | 3.49 | 0.03594000 |
| EGFLAM | 1587 | 4869576 | T | 5.29 | 0.04848000 |
| ITGA2 | 1617 | 989073 | A | -3.08 | 0.01383000 |
| PDE4D | 1629 | 193447 | T | -3.44 | 0.01478000 |
| VCAN | 1663 | 28296 | A | -3.19 | 0.02343000 |
| VCAN | 1667 | 4558964 | G | -2.81 | 0.03877000 |
| KCNN2 | 1727 | 3857431 | T | -2.53 | 0.04948000 |
| LOC340069 | 1745 | 919306 | C | -2.90 | 0.03084000 |
| LOC340069 | 1746 | 328694 | A | -2.67 | 0.03825000 |
| ODZ2 | 1790 | 13158058 | T | -2.88 | 0.04543000 |
| ODZ2 | 1797 | 7708354 | G | -3.87 | 0.00204100 |
| BTNL2 | 1877 | 3763309 | A | -2.83 | 0.04171000 |
| ELOVL5 | 1889 | 1429146 | T | -2.51 | 0.04782000 |
| ELOVL5 | 1893 | 9357760 | G | -3.32 | 0.01840000 |
| ELOVL5 | 1894 | 9370196 | C | -3.11 | 0.02945000 |
| ELOVL5 | 1895 | 9474507 | A | -4.09 | 0.01209000 |
| ELOVL5 | 1896 | 1429143 | G | -3.50 | 0.02476000 |
| SLC22A16 | 1933 | 2428175 | A | 3.37 | 0.01885000 |
| TRDN | 1937 | 17737379 | T | -3.81 | 0.03039000 |
| EYA4 | 1978 | 6929938 | A | 2.76 | 0.04420000 |
| PARK2 | 2020 | 12055483 | T | 2.74 | 0.03047000 |
| PARK2 | 2021 | 6924602 | C | 2.82 | 0.02018000 |
| CREBS | 2119 | 2237361 | C | 3.06 | 0.00892500 |
| PLEKHA8 | 2139 | 7806238 | A | -3.85 | 0.04894000 |
| PDE1C | 2152 | 10224888 | T | 2.91 | 0.01164000 |
| PDE1C | 2153 | 3213709 | G | 3.98 | 0.01262000 |
| ABCA13 | 2196 | 17548783 | C | 2.62 | 0.02556000 |
| WBSCR17 | 2230 | 7808758 | C | -2.62 | 0.03618000 |
| WBSCR17 | 2242 | 4585627 | T | 3.41 | 0.01041000 |
| WBSCR17 | 2244 | 12699040 | A | 2.98 | 0.04337000 |
| WBSCR17 | 2246 | 7783170 | C | 3.19 | 0.03021000 |
| HIP1 | 2264 | 11770686 | T | -2.72 | 0.02246000 |
| HIP1 | 2265 | 12533075 | G | -3.14 | 0.01805000 |
| CACNA2D1 | 2306 | 17155665 | T | 5.33 | 0.00732400 |
| SEMA3A | 2347 | 1989964 | T | 2.66 | 0.03028000 |
| ABCB4 | 2349 | 2097937 | G | 3.50 | 0.02621000 |
| ADAM22 | 2361 | 10268574 | T | 4.89 | 0.00466500 |
| PPP1R9A | 2380 | 854542 | G | 2.88 | 0.02997000 |
| DYNC1I1 | 2394 | 2299277 | T | 4.64 | 0.00090580 |
| DYNC1I1 | 2395 | 12672406 | T | -2.95 | 0.02744000 |
| CADPS2 | 2421 | 1476898 | T | -4.91 | 0.00274300 |
| CADPS2 | 2422 | 2471194 | A | -4.91 | 0.00274300 |
| CADPS2 | 2423 | 17144752 | T | -4.31 | 0.00489500 |
| GRM8 | 2441 | 1008907 | T | -2.63 | 0.02952000 |
| GRM8 | 2443 | 2299500 | A | -2.52 | 0.04283000 |
| GRM8 | 2444 | 2299505 | G | 2.83 | 0.01773000 |
| GRM8 | 2447 | 6951643 | G | 2.43 | 0.03151000 |
| GRM8 | 2449 | 6975541 | T | 2.25 | 0.04225000 |
| EXOC4 | 2455 | 7778778 | G | -3.22 | 0.04050000 |
| EXOC4 | 2471 | 4731992 | A | -3.35 | 0.02291000 |
| EXOC4 | 2472 | 6950383 | C | 2.53 | 0.02763000 |
| DGKI | 2474 | 1731951 | T | 3.28 | 0.00756500 |
| CNTNAP2 | 2489 | 1948489 | T | -3.44 | 0.03683000 |
| CNTNAP2 | 2493 | 1405179 | C | -3.56 | 0.00672800 |
| CNTNAP2 | 2494 | 12535047 | T | -3.43 | 0.01179000 |
| CNTNAP2 | 2510 | 2717829 | C | -4.38 | 0.01829000 |
| PTPRN2 | 2512 | 221258 | C | -3.35 | 0.01476000 |
| PTPRN2 | 2513 | 221300 | C | -2.85 | 0.03487000 |
| SLC7A2 | 2600 | 2285295 | G | -8.29 | 0.01167000 |
| PSD3 | 2604 | 13260536 | G | -2.81 | 0.01525000 |
| PSD3 | 2605 | 1038606 | G | -2.98 | 0.01539000 |
| SNTG1 | 2660 | 12548940 | C | -3.22 | 0.03371000 |
| NKAIN3 | 2704 | 4737609 | A | -4.14 | 0.00871100 |
| NKAIN3 | 2706 | 4739003 | A | -4.54 | 0.01948000 |
| NKAIN3 | 2707 | 16929516 | T | -4.54 | 0.01948000 |
| NKAIN3 | 2708 | 16929568 | A | -4.54 | 0.01948000 |
| NKAIN3 | 2723 | 10957263 | C | -3.69 | 0.02326000 |
| DEPDC2 | 2734 | 10808759 | C | 3.22 | 0.01531000 |
| ZFPM2 | 2790 | 7845734 | G | 3.12 | 0.04689000 |
| CSMD3 | 2815 | 4354335 | G | 3.50 | 0.02607000 |
| CSMD3 | 2816 | 1513524 | C | 3.25 | 0.03740000 |
| CSMD3 | 2817 | 2954892 | G | 3.20 | 0.03834000 |
| SAMD12 | 2825 | 1607924 | G | 2.85 | 0.03947000 |
| SAMD12 | 2826 | 10505340 | A | 3.96 | 0.03913000 |
| DDEF1 | 2869 | 11774947 | A | 2.62 | 0.03014000 |
| ADCY8 | 2884 | 3793387 | T | -2.74 | 0.02021000 |
| ADCY8 | 2885 | 4339660 | G | -2.74 | 0.02021000 |
| ADCY8 | 2886 | 10956553 | G | -2.74 | 0.02021000 |
| ADCY8 | 2890 | 7016933 | A | -2.75 | 0.03555000 |
| ST3GAL1 | 2904 | 2945779 | A | -2.71 | 0.01933000 |
| SMARCA2 | 2931 | 2147263 | T | 2.36 | 0.04745000 |
| SMARCA2 | 2932 | 4741650 | T | -2.77 | 0.03500000 |
| PTPRD | 2944 | 7036240 | A | -2.49 | 0.03516000 |
| ADAMTSL1 | 2958 | 563805 | C | -3.71 | 0.02012000 |
| TRPM3 | 3025 | 2993010 | A | -3.11 | 0.01876000 |
| TRPM6 | 3047 | 1012710 | C | 2.72 | 0.03664000 |
| SHC3 | 3085 | 9410299 | G | 3.81 | 0.00244200 |
| SVEP1 | 3127 | 7875389 | C | -2.44 | 0.02914000 |
| STOM | 3142 | 2196311 | G | 3.53 | 0.03063000 |
| NTNG2 | 3173 | 883318 | G | 2.92 | 0.00982200 |
| NTNG2 | 3175 | 11793404 | T | 3.51 | 0.00590600 |
| NTNG2 | 3176 | 3739930 | A | 4.69 | 0.01391000 |
| NTNG2 | 3177 | 3739929 | G | 3.93 | 0.03744000 |
| NTNG2 | 3178 | 7026497 | A | 3.30 | 0.00725800 |
| VAV2 | 3191 | 2519799 | G | 2.40 | 0.03307000 |
| VAV2 | 3192 | 2073829 | G | 2.48 | 0.01996000 |
| NOTCH1 | 3200 | 10870085 | T | 3.06 | 0.03949000 |
| NPDC1 | 3202 | 4880195 | C | -3.54 | 0.01601000 |
| GRIN1 | 3203 | 7856589 | A | 3.98 | 0.00724500 |
| CACNB2 | 3235 | 16917281 | G | -3.05 | 0.04812000 |
| CACNB2 | 3236 | 10828679 | G | -5.17 | 0.00414500 |
| CACNB2 | 3237 | 10764483 | A | -4.26 | 0.03404000 |
| CDH23 | 3314 | 4747197 | T | -3.32 | 0.02496000 |
| CDH23 | 3315 | 2070968 | A | -3.18 | 0.03747000 |
| NRG3 | 3340 | 1937959 | G | 3.69 | 0.04772000 |
| SORBS1 | 3365 | 17456435 | C | 3.83 | 0.02197000 |
| VTI1A | 3391 | 3740143 | G | 3.37 | 0.00990100 |
| VTI1A | 3392 | 716168 | C | 3.63 | 0.00395900 |
| VTI1A | 3393 | 10509963 | A | 3.58 | 0.00434700 |
| VTI1A | 3394 | 10885352 | T | 3.46 | 0.00581100 |
| C11ORF49 | 3520 | 11039112 | T | 2.71 | 0.04288000 |
| DLG2 | 3529 | 12275254 | C | -2.32 | 0.04812000 |
| DLG2 | 3541 | 1943695 | T | 3.69 | 0.01248000 |
| DLG2 | 3552 | 1940128 | C | 4.46 | 0.00891200 |
| DLG2 | 3554 | 582652 | A | 4.10 | 0.02499000 |
| OPCML | 3561 | 882584 | A | -3.34 | 0.01873000 |
| OPCML | 3562 | 7120180 | G | -3.36 | 0.02284000 |
| OPCML | 3563 | 12284699 | A | -3.21 | 0.02482000 |
| OPCML | 3564 | 10791230 | G | -2.85 | 0.03642000 |
| OPCML | 3565 | 4937700 | T | -2.43 | 0.04986000 |
| TSPAN9 | 3592 | 4766079 | C | -3.33 | 0.01468000 |
| STYK1 | 3610 | 1979268 | T | 2.67 | 0.03062000 |
| CNOT2 | 3642 | 10879096 | G | -4.21 | 0.01091000 |
| CNOT2 | 3646 | 17107924 | T | -4.73 | 0.01533000 |
| KCNC2 | 3656 | 4131955 | T | -3.44 | 0.01155000 |
| KCNC2 | 3657 | 7952767 | C | -3.33 | 0.01928000 |
| KCNC2 | 3658 | 1526821 | C | -3.44 | 0.01155000 |
| NAV3 | 3665 | 1677923 | G | -4.36 | 0.03007000 |
| GAS2L3 | 3680 | 35711 | G | 3.71 | 0.00138100 |
| GAS2L3 | 3681 | 35706 | A | 3.34 | 0.00359800 |
| NBEA | 3716 | 1381479 | C | 3.95 | 0.02518000 |
| GPC5 | 3788 | 7997883 | G | 3.22 | 0.05511000 |
| GPC5 | 3792 | 17267453 | C | -5.90 | 0.00952600 |
| GPC6 | 3796 | 9516222 | G | 4.40 | 0.00968200 |
| GPC6 | 3799 | 1323974 | C | 3.69 | 0.01787000 |
| NPAS3 | 3870 | 1315139 | T | 3.82 | 0.00383200 |
| LRFN5 | 3902 | 10131436 | A | 3.35 | 0.01160000 |
| SAMD4A | 3910 | 11626634 | A | 4.01 | 0.03693000 |
| RGS6 | 3939 | 1568404 | T | 2.76 | 0.01410000 |
| RGS6 | 3940 | 847358 | A | -2.97 | 0.00824600 |
| RGS6 | 3945 | 2681754 | A | -2.56 | 0.03572000 |
| RGS6 | 3947 | 10136263 | T | -3.02 | 0.02383000 |
| RGS6 | 3949 | 10149207 | G | -2.73 | 0.03271000 |
| RGS6 | 3950 | 2239221 | C | -3.62 | 0.00400400 |
| RGS6 | 3952 | 2238192 | A | -2.56 | 0.04299000 |
| RASGRP1 | 4051 | 12324402 | G | 2.79 | 0.02325000 |
| CDH13 | 4184 | 11646849 | A | -3.00 | 0.01034000 |
| CDH13 | 4187 | 192598 | A | 2.59 | 0.03428000 |
| CDH13 | 4188 | 7201088 | G | 2.74 | 0.02699000 |
| CDH13 | 4189 | 9933935 | T | 2.55 | 0.04489000 |
| CRISPLD2 | 4213 | 16974822 | G | 4.82 | 0.00014050 |
| CRISPLD2 | 4214 | 2934477 | A | -2.63 | 0.03089000 |
| CRISPLD2 | 4215 | 8049317 | G | -3.04 | 0.01094000 |
| CRISPLD2 | 4216 | 2646107 | C | 3.85 | 0.00145500 |
| CA10 | 4249 | 8069850 | A | 2.50 | 0.04822000 |
| MS12 | 4257 | 8066677 | G | 2.80 | 0.02093000 |
| PTPRM | 4288 | 535937 | A | 4.44 | 0.01262000 |
| PTPRM | 4289 | 524833 | C | 3.62 | 0.02496000 |
| PTPRM | 4291 | 1893224 | C | 3.98 | 0.02152000 |
| PTPRM | 4292 | 1893223 | C | 3.65 | 0.02243000 |
| NEDD4L | 4333 | 12373325 | T | 3.15 | 0.03253000 |
| DOK6 | 4377 | 12967871 | A | -2.57 | 0.03545000 |
| ZNF667 | 4387 | 10423617 | T | 3.65 | 0.02665000 |
| MACROD2 | 4452 | 6043402 | A | -6.49 | 0.02172000 |
| MACROD2 | 4459 | 1225890 | C | 2.83 | 0.04854000 |
| PTPRT | 4511 | 2223540 | T | 3.40 | 0.01583000 |
| PTGIS | 4523 | 515633 | A | -6.58 | 0.01351000 |
| CDH4 | 4534 | 2427144 | A | -3.20 | 0.01535000 |
| NCAM2 | 4544 | 9978969 | T | 5.05 | 0.00126100 |
| PCP4 | 4558 | 16998864 | A | 5.20 | 0.01318000 |
| PCP4 | 4559 | 2837283 | T | 4.41 | 0.03250000 |
| ASPHD2 | 4573 | 16982107 | T | -6.38 | 0.02428000 |
| EFCAB6 | 4598 | 9614382 | G | -2.97 | 0.02951000 |
| EFCAB6 | 4606 | 5764214 | C | -2.94 | 0.01969000 |

| **TABLE 8: Alleles Influencing Time to Discontinuation for Quetiapine** | | | | | |
|---|---|---|---|---|---|
| **Gene** | **Seq ID** | **NCBI RS#** | **Allele** | **Beta (Months)** | **P** |
| LRP8 | 57 | 1288519 | C | 2.99 | 0.015790 |
| LRP8 | 58 | 1416096 | A | 2.49 | 0.041320 |
| LRP8 | 60 | 2782497 | C | 2.49 | 0.041320 |
| PTGFRN | 87 | 2251406 | G | -6.30 | 0.039310 |
| PTGFRN | 88 | 2250457 | G | -6.06 | 0.048080 |
| PTGFRN | 89 | 2806864 | G | -6.06 | 0.048080 |
| PTGFRN | 94 | 12090536 | G | 3.62 | 0.009760 |
| KCNN3 | 97 | 11264248 | C | 2.58 | 0.031130 |
| FAM78B | 113 | 10800181 | G | -2.51 | 0.036160 |
| RYR2 | 208 | 2169902 | A | 4.98 | 0.049730 |
| CHRM3 | 221 | 6663632 | A | 2.60 | 0.031420 |
| RGS7 | 257 | 4659585 | A | -3.25 | 0.012550 |
| PLD5 | 262 | 2252440 | T | 2.76 | 0.021420 |
| PLD5 | 263 | 10926660 | C | 2.94 | 0.040370 |
| PLD5 | 264 | 4658772 | G | 2.81 | 0.045860 |
| PLD5 | 265 | 10926665 | A | 2.81 | 0.045860 |
| C2ORF46 | 273 | 7577544 | G | 5.38 | 0.027520 |
| BRE | 322 | 10209422 | T | -3.26 | 0.035550 |
| PRKCE | 390 | 281473 | A | 4.72 | 0.037500 |
| FBXO11 | 398 | 874869 | C | 3.48 | 0.003725 |
| FBXO11 | 399 | 12463595 | T | 3.51 | 0.012290 |
| CTNNA2 | 458 | 6547299 | A | 2.65 | 0.032160 |
| NAP5 | 483 | 12991216 | A | -3.06 | 0.009380 |
| NAP5 | 487 | 11695364 | T | 3.66 | 0.002984 |
| NXPH2 | 499 | 11686876 | T | 2.32 | 0.044570 |
| LRP1B | 512 | 10188773 | G | -3.16 | 0.005816 |
| ARHGAP15 | 540 | 10496944 | A | 3.54 | 0.023370 |
| ARHGAP15 | 557 | 11693600 | C | -2.59 | 0.039310 |
| CACNB4 | 565 | 2345160 | G | -6.78 | 0.001175 |
| CACNB4 | 567 | 13421383 | T | 4.59 | 0.002738 |
| CACNB4 | 569 | 12693235 | C | 3.79 | 0.009396 |
| CACNB4 | 570 | 1806702 | G | 5.39 | 0.000368 |
| CACNB4 | 572 | 17326195 | T | 5.44 | 0.000998 |
| FMNL2 | 574 | 2164402 | G | 5.02 | 0.000140 |
| FMNL2 | 575 | 6434028 | C | 3.42 | 0.003645 |
| FMNL2 | 576 | 11684450 | G | 3.83 | 0.003748 |
| FMNL2 | 577 | 12471183 | T | 4.46 | 0.000252 |
| FMNL2 | 578 | 17327752 | A | 2.72 | 0.045250 |
| FMNL2 | 580 | 2577180 | G | -3.10 | 0.040890 |
| FMNL2 | 581 | 10497108 | C | 5.57 | 0.002176 |
| FMNL2 | 582 | 16823807 | G | 5.57 | 0.002176 |
| FMNL2 | 583 | 16823809 | C | 4.97 | 0.005751 |
| FMNL2 | 584 | 10497111 | A | 5.42 | 0.005860 |
| PLA2R1 | 604 | 949753 | G | -3.60 | 0.036320 |
| PLA2R1 | 611 | 17241282 | C | 2.50 | 0.032790 |
| SCN3A | 617 | 1439807 | G | 2.94 | 0.035790 |
| PDE1A | 636 | 3769789 | G | 3.23 | 0.042350 |
| ALS2 | 654 | 4675226 | T | 3.32 | 0.013680 |
| PARD3B | 666 | 698906 | T | 3.78 | 0.028000 |
| PIP5K3 | 676 | 920218 | A | 6.52 | 0.022000 |
| PIP5K3 | 677 | 10497899 | T | 6.52 | 0.022000 |
| COL4A4 | 695 | 10166736 | A | -4.64 | 0.000053 |
| COL4A4 | 696 | 2272202 | C | -3.15 | 0.010140 |
| CNTN6 | 737 | 3772339 | T | 2.67 | 0.025420 |
| CNTN6 | 751 | 2291100 | C | -2.61 | 0.042380 |
| CNTN6 | 753 | 17038510 | T | 3.17 | 0.040590 |
| IRAK2 | 785 | 708035 | T | -2.93 | 0.006203 |
| IRAK2 | 786 | 11717636 | C | -2.92 | 0.007807 |
| SLC6A6 | 797 | 2241780 | C | 3.33 | 0.038100 |
| ARPP-21 | 821 | 4678802 | C | 2.75 | 0.020300 |
| ERC2 | 884 | 11707255 | A | -2.78 | 0.035190 |
| ERC2 | 885 | 11713228 | C | -2.62 | 0.047500 |
| PTPRG | 936 | 6785764 | G | -3.45 | 0.045750 |
| PRICKLE2 | 957 | 17069879 | A | 3.56 | 0.030630 |
| PRICKLE2 | 959 | 17664296 | T | -3.22 | 0.007006 |
| PRICKLE2 | 961 | 13068250 | C | -3.13 | 0.010680 |
| GBE1 | 992 | 3772899 | G | 2.37 | 0.048300 |
| GBE1 | 995 | 3772891 | A | -2.92 | 0.031570 |
| EPHA6 | 1009 | 2317212 | A | 3.51 | 0.043670 |
| PLCXD2 | 1016 | 9288930 | T | 2.56 | 0.037560 |
| KALRN | 1045 | 16835902 | C | 2.37 | 0.032410 |
| ZXDC | 1047 | 1687462 | C | 2.56 | 0.045120 |
| CLSTN2 | 1077 | 4683509 | G | -2.69 | 0.046860 |
| SPSB4 | 1083 | 7651293 | T | 9.76 | 0.020290 |
| TNIK | 1102 | 17217552 | G | -7.02 | 0.034640 |
| PLD1 | 1108 | 4243419 | G | -3.46 | 0.031100 |
| PLD1 | 1109 | 2178532 | A | -3.39 | 0.032730 |
| NLGN1 | 1113 | 16828952 | A | -2.57 | 0.019490 |
| NLGN1 | 1114 | 9870677 | C | -2.57 | 0.030240 |
| NLGN1 | 1115 | 993298 | C | -2.42 | 0.039420 |
| NLGN1 | 1117 | 9874225 | G | -3.20 | 0.013690 |
| NLGN1 | 1118 | 9878812 | C | -4.01 | 0.027250 |
| LEPREL1 | 1136 | 17424551 | G | 2.67 | 0.046710 |
| LEPREL1 | 1137 | 692909 | T | -2.78 | 0.048120 |
| LEPREL1 | 1138 | 2667709 | G | -2.78 | 0.048120 |
| LEPREL1 | 1140 | 573533 | A | -2.78 | 0.048120 |
| LDB2 | 1170 | 9993371 | T | 2.66 | 0.019920 |
| LDB2 | 1171 | 7690190 | A | 2.38 | 0.034790 |
| LDB2 | 1174 | 283027 | T | -6.80 | 0.026000 |
| PPARGCIA | 1194 | 2290604 | G | -4.85 | 0.047300 |
| LIMCH1 | 1227 | 17444879 | T | -4.57 | 0.009328 |
| LIMCH1 | 1228 | 6850846 | A | -2.80 | 0.038240 |
| SCD5 | 1265 | 6811012 | T | -3.95 | 0.023890 |
| FAM13A1 | 1285 | 17817631 | T | 3.42 | 0.049890 |
| PDLIM5 | 1290 | 2438142 | A | -3.06 | 0.012230 |
| PDLIM5 | 1291 | 1353044 | T | -2.93 | 0.016380 |
| PDLIM5 | 1292 | 2433325 | A | -2.93 | 0.016380 |
| DKK2 | 1304 | 419764 | T | 3.47 | 0.014350 |
| DKK2 | 1305 | 399087 | C | 3.29 | 0.039250 |
| COL25A1 | 1332 | 2526456 | G | -2.96 | 0.025690 |
| COL25A1 | 1333 | 2704102 | C | -2.85 | 0.032310 |
| INPP4B | 1421 | 17015779 | C | 3.04 | 0.046570 |
| ENPP6 | 1521 | 6829678 | C | 2.84 | 0.008260 |
| ENPP6 | 1522 | 7696176 | C | 3.00 | 0.005635 |
| ENPP6 | 1524 | 17679120 | A | 2.77 | 0.012380 |
| ENPP6 | 1525 | 4444870 | T | -3.02 | 0.007302 |
| ENPP6 | 1526 | 11132224 | C | 2.90 | 0.012170 |
| SEMA5A | 1542 | 4702625 | G | 4.02 | 0.019640 |
| SEMA5A | 1543 | 12520210 | A | 2.76 | 0.035120 |
| SEMA5A | 1544 | 13174956 | T | 2.74 | 0.036440 |
| ITGA1 | 1607 | 2169782 | T | -3.85 | 0.025750 |
| PDE4D | 1628 | 35274 | G | 2.89 | 0.019050 |
| PDE4D | 1629 | 193447 | T | 4.22 | 0.004303 |
| PDE4D | 1630 | 165940 | T | 3.12 | 0.021810 |
| VCAN | 1668 | 2287926 | A | -3.46 | 0.035700 |
| VCAN | 1671 | 2445874 | T | -3.76 | 0.023730 |
| VCAN | 1672 | 3096172 | C | -2.83 | 0.038250 |
| CAST | 1695 | 731827 | T | 2.55 | 0.032430 |
| CAST | 1696 | 17401719 | C | 2.40 | 0.044380 |
| WWC1 | 1800 | 6555802 | A | -2.76 | 0.044570 |
| SLC22A23 | 1813 | 9392497 | T | -6.80 | 0.040850 |
| SLC17A1 | 1855 | 13200784 | T | -3.56 | 0.010700 |
| SLC17A3 | 1858 | 1165162 | A | -3.87 | 0.003778 |
| SLC17A3 | 1859 | 1165161 | A | -3.69 | 0.005748 |
| SLC17A3 | 1860 | 1165158 | T | -3.64 | 0.009834 |
| LRFN2 | 1880 | 1433721 | G | 2.82 | 0.032090 |
| ELOVL5 | 1888 | 2562898 | A | -2.97 | 0.042410 |
| RIMS1 | 1914 | 9446625 | T | -3.04 | 0.010870 |
| RIMS1 | 1915 | 2815743 | T | -2.60 | 0.026540 |
| RIMS1 | 1916 | 7738055 | C | -2.99 | 0.013230 |
| SYNE1 | 2008 | 7776230 | T | -3.88 | 0.003824 |
| PARK2 | 2033 | 4708953 | C | -3.17 | 0.018980 |
| PARK2 | 2034 | 6930880 | C | -2.81 | 0.029210 |
| PARK2 | 2040 | 9365421 | A | -2.57 | 0.037820 |
| PACRG | 2050 | 7740626 | G | -3.31 | 0.010930 |
| PDE10A | 2059 | 9459418 | A | -2.72 | 0.045300 |
| CREB5 | 2125 | 4722864 | A | 2.94 | 0.049710 |
| CRHR2 | 2140 | 975537 | A | -4.04 | 0.009416 |
| CRHR2 | 2141 | 973002 | G | -3.90 | 0.018480 |
| PDE1C | 2158 | 30568 | C | 4.00 | 0.017390 |
| PDE1C | 2160 | 4551232 | A | 3.05 | 0.009675 |
| PDE1C | 2161 | 10225488 | A | 2.93 | 0.013060 |
| PDE1C | 2162 | 2079373 | C | 5.72 | 0.000135 |
| VPS41 | 2179 | 2286080 | G | 2.66 | 0.032950 |
| ABCA13 | 2194 | 17132289 | T | -4.82 | 0.034590 |
| ABCA13 | 2214 | 6958356 | A | 2.68 | 0.023710 |
| MAGI2 | 2279 | 848913 | C | 3.77 | 0.038580 |
| MAGI2 | 2294 | 860246 | A | 3.44 | 0.010730 |
| CACNA2D1 | 2304 | 17262683 | C | 2.93 | 0.034100 |
| ABCB4 | 2354 | 2888611 | C | -3.65 | 0.038890 |
| EMID2 | 2402 | 6947185 | C | 2.65 | 0.039810 |
| CUX1 | 2410 | 712839 | A | 2.72 | 0.044540 |
| DGKI | 2474 | 1731951 | T | -2.34 | 0.044930 |
| CREB3L2 | 2481 | 11972734 | A | 2.86 | 0.024020 |
| CNTNAP2 | 2490 | 6963627 | T | 2.61 | 0.022370 |
| CNTNAP2 | 2491 | 1639484 | T | 2.27 | 0.039910 |
| CNTNAP2 | 2497 | 12703906 | T | -2.84 | 0.026380 |
| CNTNAP2 | 2502 | 2022226 | C | 2.70 | 0.037400 |
| PTPRN2 | 2517 | 4716882 | T | 2.47 | 0.044150 |
| PTPRN2 | 2520 | 2335167 | C | 3.44 | 0.001937 |
| PTPRN2 | 2531 | 4909222 | G | 3.33 | 0.004042 |
| MCPH1 | 2551 | 1530408 | C | -4.05 | 0.031370 |
| DLC1 | 2578 | 536147 | G | 3.57 | 0.047570 |
| SGCZ | 2587 | 7813614 | T | 2.67 | 0.036620 |
| SGCZ | 2592 | 17119601 | G | 3.42 | 0.009225 |
| SGCZ | 2593 | 11990657 | T | -2.88 | 0.034220 |
| TOX | 2685 | 11777927 | A | 3.34 | 0.035790 |
| KCNB2 | 2745 | 2196904 | C | 2.81 | 0.043680 |
| KCNB2 | 2746 | 2255053 | A | -4.29 | 0.028690 |
| KCNB2 | 2750 | 2053421 | G | 2.80 | 0.028860 |
| GRHL2 | 2773 | 4734553 | G | -3.15 | 0.015480 |
| GRHL2 | 2775 | 13275653 | C | -2.64 | 0.044440 |
| CSMD3 | 2810 | 17640721 | C | 3.26 | 0.029800 |
| CSMD3 | 2812 | 11996690 | A | 5.85 | 0.030080 |
| ADCY8 | 2887 | 12548835 | T | -2.65 | 0.040850 |
| COL22A1 | 2918 | 1316335 | A | -3.79 | 0.008787 |
| COL22A1 | 2919 | 9657434 | T | -3.78 | 0.009091 |
| SMARCA2 | 2931 | 2147263 | T | 3.91 | 0.002293 |
| SLC1A1 | 2938 | 3087879 | C | -2.51 | 0.034470 |
| PTPRD | 2939 | 3858060 | T | -2.83 | 0.043940 |
| IFT74 | 2982 | 17694549 | C | 5.94 | 0.010800 |
| IFT74 | 2983 | 3429 | A | 3.89 | 0.022060 |
| IFT74 | 2985 | 2095405 | C | 4.01 | 0.011780 |
| TEK | 2990 | 1322051 | A | 6.03 | 0.014760 |
| TRPM3 | 3023 | 718447 | A | 2.92 | 0.030650 |
| TMC1 | 3028 | 7857300 | T | -2.77 | 0.024030 |
| TMC1 | 3029 | 7866185 | T | -2.40 | 0.046170 |
| NTRK2 | 3076 | 13289538 | G | 4.89 | 0.039800 |
| NFIL3 | 3092 | 7038686 | A | -4.58 | 0.001379 |
| NFIL3 | 3093 | 4743835 | A | -3.96 | 0.002948 |
| NFIL3 | 3094 | 4743836 | T | -3.96 | 0.009878 |
| NFIL3 | 3096 | 4743838 | A | -3.92 | 0.010470 |
| ZNF169 | 3097 | 12236219 | T | -5.74 | 0.032780 |
| RP11-33N6.1 | 3108 | 7853653 | A | -3.01 | 0.041430 |
| FKTN | 3112 | 885954 | C | -2.95 | 0.014930 |
| FKTN | 3113 | 17251166 | C | -2.62 | 0.039910 |
| FKTN | 3114 | 7851777 | T | -2.62 | 0.039910 |
| CDK5RAP2 | 3135 | 4836819 | A | 2.34 | 0.033730 |
| SLC25A25 | 3162 | 9695803 | C | 3.47 | 0.043050 |
| ABL1 | 3172 | 10901297 | A | -5.46 | 0.033810 |
| VAV2 | 3184 | 10993826 | T | -2.93 | 0.042390 |
| CACNA1B | 3209 | 7860423 | A | 3.28 | 0.039010 |
| CACNA1B | 3212 | 10867105 | C | 3.74 | 0.027430 |
| PITRM1 | 3224 | 2129440 | G | 3.37 | 0.004872 |
| PITRM1 | 3225 | 6601755 | C | 3.06 | 0.012500 |
| MYO3A | 3272 | 11014933 | A | 2.40 | 0.040000 |
| MYO3A | 3282 | 6482535 | C | 4.86 | 0.005334 |
| MYO3A | 3287 | 3781117 | C | 3.76 | 0.042520 |
| SLC16A9 | 3293 | 10993988 | T | -2.70 | 0.039340 |
| KCNMA1 | 3323 | 3781158 | T | -2.66 | 0.038640 |
| KCNMA1 | 3324 | 2131216 | T | -2.64 | 0.039310 |
| NRG3 | 3345 | 474496 | G | -2.51 | 0.028110 |
| NRG3 | 3350 | 11812533 | T | 9.92 | 0.002431 |
| NRG3 | 3351 | 17101125 | A | 9.70 | 0.007997 |
| NRG3 | 3352 | 10509462 | T | 9.70 | 0.007997 |
| NRG3 | 3353 | 17763320 | A | 5.71 | 0.045740 |
| SORCS3 | 3372 | 10786808 | T | 3.41 | 0.048340 |
| SORCS3 | 3378 | 7099674 | A | 3.35 | 0.022300 |
| SORCS3 | 3383 | 2692331 | G | 3.11 | 0.035640 |
| HSPA12A | 3424 | 12777585 | C | 2.33 | 0.040560 |
| HSPA12A | 3425 | 1900500 | A | -2.71 | 0.018550 |
| HSPA12A | 3426 | 3010476 | G | -2.53 | 0.030490 |
| HSPA12A | 3428 | 1622217 | C | -2.29 | 0.047440 |
| GALNTL4 | 3468 | 4910288 | A | -2.65 | 0.045940 |
| GALNTL4 | 3469 | 4910289 | C | -3.20 | 0.027850 |
| ARNTL | 3482 | 7107711 | C | 3.16 | 0.049040 |
| ARNTL | 3483 | 7112233 | T | 3.16 | 0.049040 |
| DLG2 | 3542 | 11234157 | T | -2.49 | 0.027170 |
| DLG2 | 3544 | 1943696 | C | -2.52 | 0.025200 |
| DLG2 | 3545 | 1943698 | G | -2.45 | 0.030540 |
| DLG2 | 3546 | 11234194 | C | -2.41 | 0.030760 |
| TSPAN9 | 3591 | 10848789 | A | -3.40 | 0.019300 |
| TSPAN9 | 3593 | 544668 | G | -2.92 | 0.025560 |
| TMEM16B | 3604 | 7298357 | A | 2.76 | 0.027500 |
| PIK3C2G | 3632 | 7136674 | G | 3.28 | 0.016110 |
| CHST11 | 3683 | 2141857 | T | 2.94 | 0.046000 |
| CHST11 | 3684 | 2055861 | C | 2.74 | 0.021310 |
| NBEA | 3719 | 7320580 | G | -3.32 | 0.026280 |
| NBEA | 3723 | 17769661 | T | -4.29 | 0.019870 |
| FNDC3A | 3734 | 7321178 | G | -3.47 | 0.005594 |
| LMO7 | 3741 | 502171 | G | -2.99 | 0.011840 |
| SLAIN1 | 3754 | 9530678 | A | 4.05 | 0.009498 |
| SLAIN1 | 3755 | 1343911 | A | 3.28 | 0.038850 |
| SLAIN1 | 3757 | 1279446 | C | 2.85 | 0.022380 |
| GPC5 | 3761 | 7992120 | T | -3.69 | 0.039590 |
| NALCN | 3836 | 9300662 | G | 2.35 | 0.042360 |
| ITGBL1 | 3851 | 1469855 | G | 5.12 | 0.024900 |
| ITGBL1 | 3852 | 1469853 | G | 3.96 | 0.039950 |
| WDR23 | 3853 | 3742499 | T | -6.86 | 0.024560 |
| GNG2 | 3909 | 12882906 | G | 2.64 | 0.026700 |
| PPP2R5E | 3925 | 8008684 | G | 4.04 | 0.008795 |
| PPP2R5E | 3926 | 2754223 | G | 3.45 | 0.009768 |
| PPP2R5E | 3928 | 1255741 | A | 2.51 | 0.049010 |
| RGS6 | 3950 | 2239221 | C | 2.34 | 0.041930 |
| NRXN3 | 3957 | 759233 | G | -6.25 | 0.005320 |
| NRXN3 | 3958 | 8011654 | C | -4.84 | 0.027210 |
| KCNK13 | 3967 | 3850395 | C | 2.63 | 0.032570 |
| CCDC88C | 3987 | 11160006 | C | 2.40 | 0.047130 |
| RYR3 | 4027 | 937302 | G | 2.47 | 0.035410 |
| RYR3 | 4028 | 16957065 | G | 2.44 | 0.047940 |
| KIAA1370 | 4064 | 4776065 | C | 3.03 | 0.010740 |
| KIAA1370 | 4066 | 1863512 | G | 2.93 | 0.013390 |
| CGNL1 | 4071 | 4774941 | G | 5.44 | 0.006506 |
| TBC1D2B | 4088 | 1108478 | C | 3.02 | 0.035190 |
| THC1D2B | 4094 | 12593545 | G | 2.92 | 0.044670 |
| TBC1D2B | 4095 | 2867985 | A | 3.08 | 0.015030 |
| TBC1D2B | 4098 | 12903461 | T | -3.20 | 0.031500 |
| ARNT2 | 4102 | 4778835 | G | 3.49 | 0.023440 |
| A2BP1 | 4118 | 1155959 | C | -2.43 | 0.044040 |
| A2BP1 | 4126 | 1922585 | T | -3.14 | 0.009143 |
| WWOX | 4157 | 4129721 | G | 3.86 | 0.028820 |
| MPHOSPH6 | 4172 | 1862820 | G | -2.45 | 0.032610 |
| USP10 | 4206 | 1541699 | G | -3.07 | 0.018270 |
| USP10 | 4207 | 7188512 | C | -3.29 | 0.017400 |
| USP10 | 4208 | 964453 | G | -2.72 | 0.032580 |
| CA10 | 4250 | 9303606 | C | -3.96 | 0.001921 |
| CA10 | 4251 | 12945099 | C | -3.25 | 0.010160 |
| MSI2 | 4260 | 8065120 | A | 3.41 | 0.042170 |
| DNAH17 | 4267 | 7211232 | T | -5.00 | 0.022530 |
| DNAH17 | 4269 | 1515022 | A | -2.97 | 0.025710 |
| NEDD4L | 4329 | 10513914 | G | -2.97 | 0.034390 |
| NEDD4L | 4330 | 2075405 | G | -3.56 | 0.014510 |
| CCBE1 | 4336 | 17065761 | C | 4.89 | 0.006636 |
| DOK6 | 4381 | 4438406 | C | -2.47 | 0.041000 |
| MBP | 4383 | 470181 | G | -2.87 | 0.020090 |
| ZNF667 | 4387 | 10423617 | T | -3.23 | 0.029650 |
| FERMT1 | 4410 | 10373 | T | -2.44 | 0.038370 |
| PLCB1 | 4414 | 6055577 | A | 5.37 | 0.000740 |
| PLCB1 | 4415 | 2235212 | A | 3.82 | 0.009620 |
| MACROD2 | 4452 | 6043402 | A | 5.61 | 0.007932 |
| MACROD2 | 4459 | 1225890 | C | 3.82 | 0.005841 |
| PTPRT | 4505 | 6065520 | T | -4.12 | 0.034490 |
| KCNB1 | 4519 | 496511 | C | -3.32 | 0.006536 |
| CDH4 | 4539 | 6089573 | A | 2.78 | 0.023000 |
| NCAM2 | 4552 | 1599228 | C | -2.85 | 0.044800 |
| EFCAB6 | 4614 | 5764310 | C | 4.48 | 0.030870 |

| **TABLE 9: Alleles Influencing Time to Discontinuation for Perphenazine** | | | | | |
|---|---|---|---|---|---|
| **Gene** | **Seq ID** | **NCBI RS#** | **Allele** | **Beta (Months)** | **P** |
| EPHB2 | 19 | 4655108 | A | 5.27 | 0.0053 |
| EPHB2 | 20 | 10799762 | C | 5.09 | 0.0135 |
| EPHB2 | 23 | 309499 | T | -3.07 | 0.0122 |
| LRP8 | 48 | 5174 | A | -2.95 | 0.0079 |
| LRP8 | 49 | 11206127 | A | -2.80 | 0.0148 |
| LRP8 | 50 | 869987 | T | -2.77 | 0.0219 |
| LRP8 | 52 | 2297660 | A | -2.63 | 0.0219 |
| LRP8 | 53 | 2297657 | A | -2.37 | 0.0440 |
| LRP8 | 54 | 12035926 | G | -2.45 | 0.0485 |
| NGF | 81 | 12145726 | G | 2.77 | 0.0321 |
| NGF | 83 | 11102925 | T | 3.53 | 0.0167 |
| NGF | 84 | 17033706 | A | 4.94 | 0.0025 |
| PTGFRN | 93 | 4641299 | G | -3.48 | 0.0100 |
| ATF6 | 98 | 2070150 | C | 4.57 | 0.0372 |
| ATF6 | 102 | 12401299 | G | 4.24 | 0.0396 |
| ATF6 | 106 | 12028953 | A | 4.57 | 0.0372 |
| ATF6 | 108 | 12040523 | T | 4.57 | 0.0372 |
| ESRRG | 172 | 12757165 | G | -2.51 | 0.0409 |
| ESRRG | 173 | 6658528 | T | -2.49 | 0.0397 |
| SLC35F3 | 191 | 10495346 | T | 5.44 | 0.0161 |
| GNG4 | 192 | 10925976 | A | -2.92 | 0.0139 |
| GNG4 | 193 | 508208 | G | 2.73 | 0.0302 |
| GNG4 | 194 | 488618 | G | -2.93 | 0.0133 |
| RYR2 | 210 | 1402803 | G | 3.55 | 0.0212 |
| CHRM3 | 222 | 1431719 | G | 2.41 | 0.0430 |
| FMN2 | 228 | 12118961 | T | -2.68 | 0.0336 |
| RGS7 | 260 | 10737861 | T | 3.34 | 0.0252 |
| PLD5 | 271 | 1548160 | C | 3.31 | 0.0106 |
| PLD5 | 272 | 12128054 | A | 2.56 | 0.0403 |
| C2ORF46 | 274 | 182971 | C | -2.82 | 0.0464 |
| C2ORF46 | 277 | 2562011 | C | 3.78 | 0.0393 |
| KCNK3 | 307 | 11126666 | A | 2.83 | 0.0397 |
| CRIM1 | 333 | 11681392 | T | 2.93 | 0.0201 |
| CRIM1 | 334 | 10189344 | C | 3.02 | 0.0207 |
| FEZ2 | 337 | 17488036 | C | 3.62 | 0.0052 |
| FEZ2 | 338 | 10197570 | T | 3.66 | 0.0053 |
| CDC42EP3 | 342 | 15628 | T | -7.37 | 0.0088 |
| SLC8A1 | 352 | 967351 | A | -2.41 | 0.0493 |
| C2ORF34 | 374 | 3738980 | C | 5.69 | 0.0021 |
| PRKCE | 386 | 13003856 | G | 2.26 | 0.0393 |
| PRKCE | 387 | 6748375 | C | -2.23 | 0.0489 |
| CCDC85A | 419 | 11696109 | A | -2.67 | 0.0228 |
| CCDC85A | 420 | 1159916 | G | -3.44 | 0.0084 |
| CCDC85A | 424 | 214041 | T | 3.48 | 0.0031 |
| CTNNA2 | 446 | 13033608 | C | -2.58 | 0.0239 |
| CTNNA2 | 447 | 2861914 | G | -2.38 | 0.0444 |
| CTNNA2 | 470 | 216616 | G | 2.87 | 0.0331 |
| CTNNA2 | 471 | 216630 | C | 2.78 | 0.0216 |
| CTNNA2 | 472 | 216657 | C | 2.75 | 0.0227 |
| NAP5 | 482 | 13432172 | G | 6.27 | 0.0270 |
| KYNU | 533 | 6430001 | A | -3.01 | 0.0214 |
| KYNU | 535 | 3816193 | A | -4.57 | 0.0067 |
| KCNJ3 | 591 | 2937609 | C | -2.78 | 0.0192 |
| SCN1A | 622 | 1461197 | A | 2.62 | 0.0354 |
| PARD3B | 658 | 1510765 | T | 3.17 | 0.0114 |
| PARD3B | 660 | 6760872 | C | 2.63 | 0.0356 |
| PARD3B | 661 | 4675490 | G | 2.55 | 0.0466 |
| PARD3B | 664 | 1100393 | G | 2.79 | 0.0410 |
| NRP2 | 675 | 6435306 | A | 2.41 | 0.0449 |
| ERBB4 | 680 | 1992029 | G | 3.43 | 0.0141 |
| CNTN4 | 757 | 11129074 | G | 2.64 | 0.0455 |
| RARB | 807 | 17526942 | T | -5.76 | 0.0101 |
| CLASP2 | 816 | 13098051 | C | 5.32 | 0.0041 |
| STAC | 826 | 3749279 | T | 3.88 | 0.0499 |
| FHIT | 916 | 212016 | C | 3.63 | 0.0128 |
| PTPRG | 940 | 9813294 | A | -2.60 | 0.0178 |
| PRICKLE2 | 962 | 153729 | A | -4.68 | 0.0105 |
| PRICKLE2 | 963 | 697280 | G | -4.01 | 0.0327 |
| PRICKLE2 | 965 | 696019 | A | -2.59 | 0.0456 |
| PRICKLE2 | 968 | 11924614 | C | -2.69 | 0.0317 |
| GBE1 | 989 | 17019088 | T | 4.05 | 0.0034 |
| GBE1 | 990 | 7618973 | G | 3.90 | 0.0039 |
| GBE1 | 993 | 3821548 | G | 3.09 | 0.0169 |
| GBE1 | 998 | 3860595 | C | 2.45 | 0.0491 |
| CDGAP | 1023 | 9855065 | A | -2.90 | 0.0225 |
| CDGAP | 1024 | 3732413 | G | 3.25 | 0.0285 |
| CLSTN2 | 1079 | 9875409 | G | 3.88 | 0.0046 |
| CLSTN2 | 1080 | 347975 | G | 3.25 | 0.0081 |
| CLSTN2 | 1081 | 347973 | A | 2.74 | 0.0271 |
| SPSB4 | 1084 | 4683550 | C | -4.38 | 0.0093 |
| SPSB4 | 1086 | 2086180 | G | -3.88 | 0.0221 |
| TNIK | 1102 | 17217552 | G | 7.19 | 0.0176 |
| NLGN1 | 1123 | 692486 | G | 2.58 | 0.0299 |
| NLGN1 | 1125 | 11713211 | C | 2.58 | 0.0299 |
| LDB2 | 1171 | 7690190 | A | 2.89 | 0.0202 |
| SLIT2 | 1188 | 10516357 | A | -3.02 | 0.0155 |
| PCDH7 | 1200 | 4611883 | G | 2.29 | 0.0357 |
| COL25A1 | 1318 | 7666215 | G | 2.64 | 0.0383 |
| COL25A1 | 1325 | 17596971 | G | 3.10 | 0.0355 |
| COL25A1 | 1328 | 711865 | A | -3.32 | 0.0063 |
| COL25A1 | 1329 | 794149 | G | -3.15 | 0.0090 |
| COL25A1 | 1330 | 1863293 | G | -3.11 | 0.0108 |
| COL25A1 | 1331 | 3096490 | A | -3.11 | 0.0108 |
| ANK2 | 1343 | 1989930 | G | 2.77 | 0.0243 |
| GPR103 | 1384 | 11732033 | A | 3.44 | 0.0256 |
| POU4F2 | 1431 | 6839918 | T | 4.31 | 0.0428 |
| DCLK2 | 1443 | 4696645 | A | 4.63 | 0.0132 |
| CTSO | 1446 | 6536124 | A | -3.12 | 0.0135 |
| CTSO | 1447 | 4608743 | C | -3.70 | 0.0171 |
| CTSO | 1448 | 17033891 | C | -3.56 | 0.0305 |
| FSTL5 | 1466 | 13110363 | G | -3.94 | 0.0334 |
| TLL1 | 1480 | 2292083 | A | 2.37 | 0.0457 |
| PALLD | 1483 | 6552873 | C | -3.67 | 0.0028 |
| PALLD | 1484 | 6552875 | T | -3.67 | 0.0028 |
| PALLD | 1485 | 6818921 | C | -3.67 | 0.0028 |
| PALLD | 1487 | 17054309 | A | 3.24 | 0.0301 |
| PALLD | 1488 | 10031058 | G | -3.67 | 0.0028 |
| PALLD | 1489 | 10009813 | G | 3.01 | 0.0274 |
| PALLD | 1490 | 6840767 | C | -4.05 | 0.0035 |
| PALLD | 1492 | 2712118 | C | -3.47 | 0.0063 |
| PALLD | 1493 | 2319900 | T | -3.22 | 0.0103 |
| ODZ3 | 1517 | 7668885 | T | -3.08 | 0.0248 |
| DNAH5 | 1556 | 7709692 | C | -3.22 | 0.0440 |
| CDH10 | 1574 | 7731953 | T | 4.20 | 0.0194 |
| SLC1A3 | 1581 | 4869676 | A | 3.00 | 0.0341 |
| SLC1A3 | 1582 | 1645651 | A | -2.74 | 0.0254 |
| ITGA1 | 1604 | 2860025 | T | 2.99 | 0.0223 |
| ITGA1 | 1605 | 1820167 | A | -2.70 | 0.0308 |
| PDE4D | 1624 | 12654005 | G | 2.75 | 0.0365 |
| THBS4 | 1660 | 2288395 | G | 3.16 | 0.0178 |
| VCAN | 1667 | 4558964 | G | 3.60 | 0.0156 |
| GPR98 | 1680 | 666659 | T | -2.63 | 0.0226 |
| GPR98 | 1681 | 688956 | C | -2.43 | 0.0314 |
| GPR98 | 1682 | 168743 | G | 2.42 | 0.0441 |
| PJA2 | 1720 | 1428931 | G | -4.01 | 0.0498 |
| SEMA6A | 1737 | 682684 | G | 2.89 | 0.0325 |
| ADAMTS19 | 1766 | 1363143 | C | 6.33 | 0.0011 |
| TRPC7 | 1777 | 3777150 | A | -3.15 | 0.0116 |
| CTNNA1 | 1781 | 10043478 | G | -2.31 | 0.0398 |
| PHACTR1 | 1818 | 1537340 | A | -3.07 | 0.0382 |
| PHACTR1 | 1820 | 13219256 | T | -2.85 | 0.0451 |
| PARC | 1884 | 17209372 | G | -4.86 | 0.0459 |
| RIMS1 | 1900 | 575472 | G | -3.10 | 0.0216 |
| RIMS1 | 1902 | 1010326 | A | -3.32 | 0.0227 |
| RIMS1 | 1905 | 2496495 | G | -2.71 | 0.0399 |
| TRDN | 1935 | 13190807 | C | -3.11 | 0.0268 |
| NKAIN2 | 1966 | 1871329 | C | 3.76 | 0.0136 |
| NKAIN2 | 1968 | 6906607 | A | 4.13 | 0.0181 |
| NKAIN2 | 1969 | 609783 | T | 3.59 | 0.0394 |
| EYA4 | 1972 | 533729 | T | 4.30 | 0.0065 |
| EYA4 | 1973 | 211443 | G | 4.09 | 0.0107 |
| EYA4 | 1975 | 6941879 | G | 3.47 | 0.0225 |
| PARK2 | 2023 | 10945767 | T | -2.92 | 0.0091 |
| PARK2 | 2024 | 9365311 | C | 2.45 | 0.0278 |
| PARK2 | 2027 | 4279411 | G | 2.59 | 0.0448 |
| PARK2 | 2031 | 6930668 | T | 2.85 | 0.0368 |
| PARK2 | 2043 | 9347659 | G | -3.36 | 0.0239 |
| PARK2 | 2047 | 9365473 | T | -3.18 | 0.0319 |
| SKAP2 | 2117 | 774256 | G | 2.99 | 0.0437 |
| SKAP2 | 2118 | 774250 | T | 3.60 | 0.0218 |
| CREB5 | 2122 | 1544469 | G | 2.84 | 0.0433 |
| BMPER | 2170 | 1420335 | C | -3.46 | 0.0063 |
| CDC2L5 | 2186 | 3800802 | T | -2.98 | 0.0245 |
| CDC2L5 | 2187 | 10277422 | C | -4.12 | 0.0072 |
| ABCA13 | 2214 | 6958356 | A | 3.71 | 0.0041 |
| GRB10 | 2215 | 2244347 | C | 2.91 | 0.0347 |
| GRB10 | 2217 | 2244351 | G | 3.12 | 0.0218 |
| HIP1 | 2256 | 17352471 | A | 3.39 | 0.0212 |
| HIP1 | 2257 | 1018945 | G | 2.99 | 0.0177 |
| HIP1 | 2258 | 757364 | T | -3.45 | 0.0049 |
| HIP1 | 2259 | 2525467 | C | -2.57 | 0.0458 |
| PCLO | 2311 | 17156675 | T | -3.73 | 0.0064 |
| PCLO | 2324 | 2522843 | A | -3.12 | 0.0120 |
| SEMA3E | 2346 | 1972459 | C | 2.59 | 0.0372 |
| ABCB4 | 2348 | 31659 | C | -4.54 | 0.0327 |
| ABCB4 | 2349 | 2097937 | G | -3.25 | 0.0299 |
| CUX1 | 2408 | 2694158 | T | 2.77 | 0.0311 |
| CUX1 | 2409 | 803064 | C | -4.69 | 0.0009 |
| TBXAS1 | 2482 | 6464451 | C | 9.45 | 0.0094 |
| TBXAS1 | 2484 | 8192813 | T | 9.45 | 0.0094 |
| CNTNAP2 | 2496 | 7802708 | G | 2.38 | 0.0388 |
| PTPRN2 | 2532 | 10265417 | T | 3.88 | 0.0014 |
| PTPRN2 | 2534 | 10266729 | T | 3.86 | 0.0015 |
| PTPRN2 | 2535 | 2878355 | A | 3.10 | 0.0216 |
| SGCZ | 2588 | 6651381 | A | -3.01 | 0.0186 |
| SLC25A37 | 2632 | 10503726 | C | -3.38 | 0.0156 |
| SLC25A37 | 2633 | 7826247 | G | -4.07 | 0.0034 |
| SLC25A37 | 2634 | 11778179 | T | -3.44 | 0.0171 |
| SFRP1 | 2646 | 7843510 | G | -2.71 | 0.0223 |
| SFRP1 | 2648 | 7833518 | T | -2.48 | 0.0353 |
| SNTG1 | 2666 | 1542615 | T | -5.07 | 0.0370 |
| SNTG1 | 2667 | 2623207 | C | -5.03 | 0.0388 |
| SNTG1 | 2669 | 2623224 | A | -5.03 | 0.0388 |
| DEPDC2 | 2729 | 16934215 | A | -4.41 | 0.0099 |
| DEPDC2 | 2730 | 3793379 | A | -3.94 | 0.0133 |
| DEPDC2 | 2731 | 3793381 | A | -3.92 | 0.0158 |
| ZFPM2 | 2791 | 10464844 | G | 2.77 | 0.0428 |
| ZFPM2 | 2793 | 16873003 | C | 2.88 | 0.0433 |
| ZFPM2 | 2800 | 1442326 | G | -2.57 | 0.0442 |
| CSMD3 | 2816 | 1513524 | C | -2.53 | 0.0343 |
| CSMD3 | 2817 | 2954892 | G | -2.53 | 0.0343 |
| MTSS1 | 2849 | 1988734 | A | 2.83 | 0.0177 |
| MTSS1 | 2851 | 4871503 | C | -4.01 | 0.0122 |
| FAM84B | 2857 | 16901667 | T | -3.61 | 0.0132 |
| SMARCA2 | 2924 | 7048496 | A | -4.12 | 0.0265 |
| SMARCA2 | 2925 | 6475520 | G | -4.49 | 0.0214 |
| SMARCA2 | 2926 | 6475522 | A | -4.61 | 0.0185 |
| SMARCA2 | 2927 | 10757205 | G | -5.56 | 0.0105 |
| PTPRD | 2954 | 10815933 | C | 3.07 | 0.0230 |
| PTPRD | 2955 | 1036328 | C | 2.96 | 0.0251 |
| PTPRD | 2956 | 7868093 | T | 2.67 | 0.0418 |
| KIAA1797 | 2962 | 2151002 | A | 2.78 | 0.0241 |
| KIAA1797 | 2978 | 7030990 | G | -3.49 | 0.0127 |
| KIAA1797 | 2980 | 4468020 | T | -2.72 | 0.0375 |
| APBA1 | 3002 | 10867656 | C | -4.77 | 0.0119 |
| APBA1 | 3003 | 7866199 | C | -4.58 | 0.0182 |
| APBA1 | 3006 | 7872660 | T | -4.19 | 0.0195 |
| TMC1 | 3042 | 2501914 | T | -2.41 | 0.0329 |
| PCSK5 | 3056 | 10869698 | A | -2.65 | 0.0398 |
| PCSK5 | 3057 | 10512054 | C | -3.57 | 0.0050 |
| DIRAS2 | 3087 | 532591 | C | -3.61 | 0.0054 |
| DIRAS2 | 3088 | 7028171 | A | 2.71 | 0.0483 |
| GRIN3A | 3110 | 2786716 | C | 3.05 | 0.0134 |
| DAB2IP | 3143 | 10985431 | T | 4.78 | 0.0496 |
| TTLL11 | 3148 | 10985529 | T | 2.93 | 0.0247 |
| EXOSC2 | 3164 | 4740358 | C | -3.92 | 0.0222 |
| KCNT1 | 3197 | 11103150 | A | -6.26 | 0.0182 |
| CACNA1B | 3211 | 11137363 | T | 3.35 | 0.0220 |
| PITRM1 | 3221 | 1127047 | T | -7.85 | 0.0320 |
| CUGBP2 | 3232 | 2938015 | A | 2.92 | 0.0466 |
| CACNB2 | 3236 | 10828679 | G | -3.89 | 0.0440 |
| ARMC3 | 3252 | 12252340 | T | 3.43 | 0.0210 |
| MYO3A | 3262 | 10741104 | C | -2.79 | 0.0308 |
| MYO3A | 3264 | 10828912 | A | -2.79 | 0.0308 |
| MYO3A | 3268 | 4749090 | C | 3.69 | 0.0332 |
| MYO3A | 3280 | 1999240 | C | -2.54 | 0.0339 |
| MYO3A | 3285 | 12764197 | G | 3.37 | 0.0483 |
| JMJD1C | 3301 | 9629895 | A | -3.04 | 0.0157 |
| JMJD1C | 3303 | 2893922 | C | -3.79 | 0.0063 |
| CDH23 | 3312 | 3802711 | T | -3.75 | 0.0039 |
| CDH23 | 3315 | 2070968 | A | -2.88 | 0.0352 |
| SORCS3 | 3374 | 2491377 | T | 2.75 | 0.0337 |
| SORCS3 | 3382 | 2177744 | C | -3.25 | 0.0375 |
| STIM1 | 3463 | 10835407 | G | 2.62 | 0.0416 |
| STIM1 | 3467 | 725519 | T | 2.53 | 0.0438 |
| MICAL2 | 3473 | 1159649 | C | 3.41 | 0.0069 |
| MICAL2 | 3474 | 10765929 | T | 2.98 | 0.0178 |
| MICAL2 | 3480 | 1493955 | A | 5.19 | 0.0169 |
| ARNTL | 3486 | 10766066 | G | 2.75 | 0.0358 |
| ARNTL | 3489 | 4414197 | T | 2.72 | 0.0378 |
| SPON1 | 3505 | 12283632 | A | -2.95 | 0.0322 |
| SPONI | 3508 | 7950377 | T | -3.71 | 0.0082 |
| SPONI | 3509 | 7116296 | T | 2.86 | 0.0252 |
| SPON1 | 3510 | 11023088 | T | 2.82 | 0.0301 |
| C11ORF49 | 3519 | 11039105 | T | 3.25 | 0.0110 |
| C11ORF49 | 3521 | 747650 | A | 3.18 | 0.0128 |
| ELMOD1 | 3559 | 683266 | A | -3.00 | 0.0216 |
| OPCML | 3584 | 4937782 | T | -3.82 | 0.0085 |
| OPCML | 3585 | 7936263 | G | -3.49 | 0.0222 |
| OPCML | 3588 | 7108751 | T | -3.85 | 0.0055 |
| N4BP2L2 | 3699 | 206336 | G | 2.50 | 0.0347 |
| TRPC4 | 3731 | 1924378 | A | 2.43 | 0.0413 |
| LMO7 | 3742 | 7335067 | A | 2.76 | 0.0321 |
| GPC5 | 3764 | 7983063 | G | 3.01 | 0.0317 |
| GPC5 | 3765 | 653774 | T | -2.68 | 0.0254 |
| GPC5 | 3766 | 655169 | T | -2.63 | 0.0288 |
| GPC5 | 3771 | 553717 | A | -5.43 | 0.0124 |
| GPC5 | 3772 | 342678 | A | -5.43 | 0.0124 |
| GPC5 | 3773 | 342677 | A | -4.32 | 0.0426 |
| GPC5 | 3775 | 7986797 | A | -6.13 | 0.0455 |
| GPC5 | 3776 | 9515971 | G | -2.89 | 0.0214 |
| GPC5 | 3787 | 913005 | T | -2.55 | 0.0349 |
| GPC6 | 3818 | 10492635 | C | 3.14 | 0.0230 |
| NPAS3 | 3866 | 243291 | G | 2.62 | 0.0307 |
| NPAS3 | 3869 | 1579702 | G | 2.43 | 0.0374 |
| SLC25A21 | 3881 | 712377 | C | 2.69 | 0.0419 |
| SLC25A21 | 3883 | 10872897 | T | 2.56 | 0.0433 |
| SAMD4A | 3913 | 709939 | C | -3.20 | 0.0128 |
| SAMD4A | 3915 | 8021957 | T | -3.05 | 0.0150 |
| SAMD4A | 3916 | 8021151 | G | -3.09 | 0.0166 |
| SAMD4A | 3917 | 2057369 | A | 3.17 | 0.0198 |
| SAMD4A | 3918 | 17128004 | A | 3.17 | 0.0198 |
| SAMD4A | 3919 | 10483639 | C | 3.17 | 0.0198 |
| RGS6 | 3949 | 10149207 | G | 3.04 | 0.0149 |
| RGS6 | 3952 | 2238192 | A | 3.31 | 0.0066 |
| RGS6 | 3954 | 2803947 | A | 2.52 | 0.0484 |
| KCNK10 | 3964 | 1950279 | T | 3.82 | 0.0017 |
| KCNK10 | 3965 | 17698533 | C | 3.89 | 0.0020 |
| KCNK10 | 3966 | 12185033 | T | 3.34 | 0.0074 |
| RYR3 | 4019 | 2670947 | G | 3.30 | 0.0046 |
| RYR3 | 4020 | 2676083 | T | 3.19 | 0.0079 |
| RYR3 | 4022 | 2670944 | G | 2.61 | 0.0394 |
| RYR3 | 4035 | 11072734 | G | -2.88 | 0.0293 |
| RYR3 | 4036 | 891343 | T | -3.09 | 0.0184 |
| CGNL1 | 4070 | 7182648 | T | 2.83 | 0.0323 |
| RORA | 4082 | 11071548 | G | -3.38 | 0.0061 |
| CLK3 | 4084 | 12441932 | G | -3.89 | 0.0293 |
| CLK3 | 4085 | 2068982 | A | -3.81 | 0.0329 |
| HYDIN | 4150 | 9939194 | T | 2.61 | 0.0470 |
| WWOX | 4157 | 4129721 | G | 4.71 | 0.0449 |
| CDH13 | 4203 | 8049546 | A | -3.05 | 0.0406 |
| CDH13 | 4204 | 33121 | G | -3.39 | 0.0108 |
| DNAH17 | 4264 | 16971269 | C | -3.26 | 0.0104 |
| DNAH17 | 4265 | 647727 | C | -2.96 | 0.0235 |
| DNAH17 | 4267 | 7211232 | T | -4.14 | 0.0113 |
| DLGAP1 | 4276 | 9635857 | G | -3.43 | 0.0267 |
| DLGAP1 | 4278 | 7245298 | G | -3.38 | 0.0315 |
| ZFP161 | 4281 | 581144 | T | -3.30 | 0.0135 |
| ZFP161 | 4282 | 990072 | T | -3.87 | 0.0005 |
| ZFP161 | 4283 | 11665417 | T | 3.81 | 0.0009 |
| ZFP161 | 4284 | 9945680 | A | 3.81 | 0.0009 |
| KIAA0802 | 4306 | 17408213 | T | 4.07 | 0.0092 |
| OSBPL1A | 4310 | 275857 | C | -5.38 | 0.0462 |
| CDH7 | 4349 | 7241038 | T | -2.16 | 0.0470 |
| DOK6 | 4378 | 8097743 | G | 2.79 | 0.0145 |
| PLCB4 | 4437 | 13036731 | G | -6.85 | 0.0383 |
| SEC23B | 4486 | 1555354 | C | 3.49 | 0.0226 |
| SEC23B | 4488 | 2273526 | G | 3.63 | 0.0424 |
| PTPRT | 4502 | 6124439 | A | 2.99 | 0.0413 |
| PTPRT | 4506 | 6016864 | A | 2.49 | 0.0475 |
| C21ORF37 | 4541 | 2824301 | G | -2.61 | 0.0235 |
| PDE9A | 4564 | 12626774 | C | 3.75 | 0.0044 |
| ARFGAP3 | 4578 | 5758951 | G | -3.09 | 0.0101 |
| ARFGAP3 | 4579 | 2024645 | T | -2.94 | 0.0174 |
| ARFGAP3 | 4580 | 5758965 | A | -2.87 | 0.0186 |
| ARFGAP3 | 4581 | 5758972 | T | -2.50 | 0.0288 |
| ARFGAP3 | 4582 | 738536 | A | -2.27 | 0.0448 |
| PACSIN2 | 4584 | 12166809 | A | -2.77 | 0.0222 |
| PACSIN2 | 4588 | 7286299 | A | -2.58 | 0.0245 |
| PACSIN2 | 4589 | 737782 | G | -2.47 | 0.0291 |
| EFCAB6 | 4597 | 137160 | G | -2.60 | 0.0245 |
| EFCAB6 | 4598 | 9614382 | G | -2.70 | 0.0478 |
| EFCAB6 | 4600 | 2374773 | A | -2.78 | 0.0336 |

| **TABLE 10: Alleles Influencing Time to Discontinuation for Ziprasidone** | | | | | |
|---|---|---|---|---|---|
| **Gene** | **Seq ID** | **NCBI RS#** | **Allele** | **Beta (Months)** | **P** |
| AGBL4-C1ORF165 | 27 | 1934395 | G | 4.80 | 0.0271 |
| LRP8 | 57 | 1288519 | C | -4.06 | 0.0307 |
| ESRRG | 180 | 11117642 | T | 5.44 | 0.0114 |
| ESRRG | 184 | 7529655 | A | -5.33 | 0.0260 |
| GNG4 | 194 | 488618 | G | -3.52 | 0.0290 |
| RYR2 | 201 | 10802596 | T | 3.43 | 0.0307 |
| FMN2 | 235 | 10926168 | G | -5.41 | 0.0044 |
| FMN2 | 236 | 7520065 | G | -5.41 | 0.0044 |
| FMN2 | 238 | 10926188 | G | -4.04 | 0.0337 |
| RGS7 | 255 | 191735 | A | 3.32 | 0.0496 |
| C2ORF46 | 277 | 2562011 | C | -5.56 | 0.0407 |
| DDEF2 | 283 | 10593 | G | -6.24 | 0.0151 |
| CRIM1 | 334 | 10189344 | C | 4.39 | 0.0074 |
| FEZ2 | 335 | 3770811 | T | 4.59 | 0.0047 |
| FEZ2 | 336 | 1533946 | G | 4.50 | 0.0056 |
| FEZ2 | 338 | 10197570 | T | 4.45 | 0.0070 |
| SLC8A1 | 347 | 11885401 | C | 6.21 | 0.0065 |
| SLC8A1 | 353 | 13017237 | A | 9.79 | 0.0090 |
| SLC8A1 | 354 | 9789739 | A | 9.63 | 0.0258 |
| EPAS1 | 395 | 1530632 | C | -3.98 | 0.0417 |
| EPAS1 | 397 | 7571218 | A | -3.93 | 0.0196 |
| FBXO11 | 398 | 874869 | C | -3.81 | 0.0418 |
| PSME4 | 410 | 1363061 | T | -5.57 | 0.0106 |
| CCDC85A | 427 | 10460558 | A | -3.89 | 0.0467 |
| CTNNA2 | 453 | 17018760 | T | -6.17 | 0.0027 |
| CTNNA2 | 454 | 1006607 | G | -6.68 | 0.0035 |
| CTNNA2 | 456 | 2120408 | C | -6.68 | 0.0035 |
| CTNNA2 | 457 | 2165975 | T | -6.68 | 0.0035 |
| CTNNA2 | 458 | 6547299 | A | 3.53 | 0.0346 |
| CTNNA2 | 461 | 17018918 | G | -7.13 | 0.0170 |
| CTNNA2 | 473 | 3770369 | C | -3.46 | 0.0238 |
| NAP5 | 483 | 12991216 | A | 4.15 | 0.0189 |
| KCNJ3 | 590 | 3111017 | T | 3.80 | 0.0377 |
| SCN3A | 616 | 1550385 | G | -4.19 | 0.0206 |
| CERKL | 632 | 895901 | A | 3.69 | 0.0310 |
| CERKL | 635 | 4018756 | A | -3.55 | 0.0116 |
| PDE1A | 641 | 6736414 | T | -4.86 | 0.0300 |
| DNER | 715 | 17677612 | T | 7.75 | 0.0422 |
| CHRND | 730 | 2697800 | A | -4.77 | 0.0138 |
| CNTN4 | 769 | 9823500 | C | -4.07 | 0.0428 |
| ITPR1 | 780 | 3804993 | G | -3.82 | 0.0375 |
| ATP2B2 | 788 | 1438168 | G | -6.00 | 0.0003 |
| ATP2B2 | 789 | 4684687 | T | -3.58 | 0.0282 |
| ATP2B2 | 793 | 34923 | G | -4.59 | 0.0037 |
| SLC6A6 | 798 | 11713355 | A | -4.79 | 0.0330 |
| RARB | 809 | 6774124 | C | 4.25 | 0.0199 |
| RARB | 810 | 1881707 | A | 3.94 | 0.0439 |
| GPXI | 857 | 9827708 | G | 4.68 | 0.0220 |
| CACNA2D3 | 864 | 11130396 | T | 4.97 | 0.0058 |
| CACNA2D3 | 870 | 4384925 | G | -4.10 | 0.0229 |
| ERC2 | 877 | 3796232 | G | 4.66 | 0.0023 |
| ERC2 | 879 | 2132237 | C | 3.16 | 0.0406 |
| FLNB | 911 | 12632456 | A | -4.43 | 0.0025 |
| PTPRG | 932 | 6794009 | G | -4.98 | 0.0014 |
| PRICKLE2 | 964 | 696017 | A | -9.34 | 0.0058 |
| PRICKLE2 | 969 | 704398 | G | -8.11 | 0.0103 |
| PRICKLE2 | 970 | 697288 | T | -6.36 | 0.0336 |
| KALRN | 1037 | 2008839 | A | -6.67 | 0.0189 |
| KALRN | 1038 | 2008847 | T | -6.19 | 0.0402 |
| SERPINI2 | 1090 | 13062550 | C | 3.53 | 0.0244 |
| TNIK | 1101 | 9824475 | G | -3.43 | 0.0478 |
| LEPREL1 | 1132 | 1719607 | A | 3.67 | 0.0454 |
| LEPREL1 | 1136 | 17424551 | G | 3.83 | 0.0145 |
| UBXD7 | 1151 | 6774540 | T | -3.77 | 0.0304 |
| UBXD7 | 1152 | 9841810 | C | -3.61 | 0.0370 |
| UBXD7 | 1153 | 9847223 | T | -3.61 | 0.0370 |
| LDB2 | 1169 | 1501142 | G | 3.95 | 0.0325 |
| LDB2 | 1171 | 7690190 | A | 3.03 | 0.0453 |
| SLIT2 | 1191 | 9992591 | C | -3.09 | 0.0484 |
| UBE2K | 1213 | 2054718 | T | -5.34 | 0.0261 |
| N4BP2 | 1215 | 4974970 | A | 3.25 | 0.0476 |
| LIMCH1 | 1218 | 10026397 | A | 4.76 | 0.0464 |
| LIMCH1 | 1222 | 10805090 | G | 4.01 | 0.0423 |
| LPHN3 | 1232 | 950313 | C | 5.23 | 0.0143 |
| LPHN3 | 1233 | 1124974 | A | 4.55 | 0.0330 |
| FAM13A1 | 1283 | 6814344 | G | 3.74 | 0.0143 |
| FAM13A1 | 1284 | 13131633 | T | 3.74 | 0.0143 |
| PDLIM5 | 1293 | 2510777 | C | -4.41 | 0.0135 |
| PDLIM5 | 1294 | 3805274 | A | 6.81 | 0.0142 |
| PDLIM5 | 1298 | 11735212 | A | 4.69 | 0.0150 |
| ANK2 | 1354 | 17045935 | C | -4.22 | 0.0377 |
| ANK2 | 1355 | 2272229 | T | -4.22 | 0.0377 |
| GPR103 | 1381 | 13110738 | G | 3.78 | 0.0275 |
| IL15 | 1403 | 6841939 | A | 5.35 | 0.0018 |
| IL15 | 1408 | 6821171 | G | -4.73 | 0.0174 |
| CTSO | 1449 | 10517625 | C | -5.06 | 0.0091 |
| FSTL5 | 1461 | 45426 | T | 3.95 | 0.0145 |
| TLL1 | 1474 | 6844820 | G | 3.85 | 0.0233 |
| AHRR | 1533 | 4956936 | C | 3.62 | 0.0435 |
| SEMA5A | 1535 | 985723 | A | -5.71 | 0.0138 |
| SEMA5A | 1537 | 2962699 | T | -4.97 | 0.0339 |
| SEMA5A | 1538 | 786843 | G | -4.42 | 0.0143 |
| SEMA5A | 1539 | 257098 | G | -4.34 | 0.0443 |
| SEMA5A | 1540 | 786846 | C | -4.34 | 0.0443 |
| DNAH5 | 1547 | 1354191 | G | 3.62 | 0.0256 |
| DNAH5 | 1549 | 6876673 | T | 3.21 | 0.0475 |
| EGFLAM | 1590 | 1469421 | A | 9.90 | 0.0216 |
| EGFLAM | 1596 | 2731979 | C | -5.55 | 0.0305 |
| ITGA2 | 1619 | 3212527 | G | 3.66 | 0.0459 |
| PDE4D | 1632 | 294494 | A | -5.74 | 0.0162 |
| PDE4D | 1633 | 295954 | G | -6.72 | 0.0179 |
| PDE4D | 1634 | 295955 | G | -7.16 | 0.0182 |
| PDE4D | 1635 | 295963 | C | -6.62 | 0.0286 |
| ODZ2 | 1787 | 2337017 | C | 9.01 | 0.0001 |
| ODZ2 | 1789 | 1421989 | G | 5.38 | 0.0057 |
| ODZ2 | 1795 | 6555784 | T | 6.44 | 0.0110 |
| ODZ2 | 1797 | 7708354 | G | -3.99 | 0.0203 |
| WWC1 | 1802 | 10038727 | A | 3.19 | 0.0493 |
| JARID2 | 1831 | 4085876 | T | -4.30 | 0.0230 |
| JARID2 | 1832 | 9476827 | T | -4.08 | 0.0325 |
| JARID2 | 1833 | 2282821 | A | 3.86 | 0.0389 |
| SLC17A4 | 1845 | 4712963 | T | 3.38 | 0.0369 |
| SLC17A1 | 1853 | 1324082 | A | 4.13 | 0.0301 |
| SLC17A1 | 1854 | 1165196 | C | -4.07 | 0.0066 |
| SLC17A1 | 1856 | 1165177 | T | -3.47 | 0.0187 |
| SLC17A1 | 1857 | 4712976 | T | 4.23 | 0.0349 |
| SLC17A3 | 1862 | 1165205 | A | -3.47 | 0.0187 |
| SLC17A2 | 1865 | 3799371 | T | 3.94 | 0.0071 |
| SLC17A2 | 1866 | 6938233 | T | 3.94 | 0.0071 |
| SLC17A2 | 1867 | 2071297 | C | 3.94 | 0.0071 |
| SLC17A2 | 1868 | 442601 | C | -2.92 | 0.0380 |
| SLC17A2 | 1869 | 9467646 | G | 3.94 | 0.0071 |
| SLC17A2 | 1870 | 9467652 | G | 3.49 | 0.0171 |
| BTN3A3 | 1874 | 2237236 | G | 3.62 | 0.0471 |
| TJAP1 | 1886 | 1106841 | C | -4.56 | 0.0076 |
| TJAP1 | 1887 | 1096699 | A | -4.00 | 0.0275 |
| RIMS1 | 1909 | 11754022 | T | -3.89 | 0.0342 |
| RIMS1 | 1910 | 10498882 | G | -3.20 | 0.0349 |
| RIMS1 | 1911 | 2807531 | A | -3.17 | 0.0402 |
| RIMS1 | 1912 | 1339226 | G | -5.22 | 0.0120 |
| RIMS1 | 1913 | 1546914 | T | -5.22 | 0.0120 |
| RIMS1 | 1917 | 12525154 | A | -4.53 | 0.0353 |
| KLHL32 | 1925 | 1206149 | A | -4.01 | 0.0143 |
| SLC22A16 | 1926 | 9400390 | T | -4.52 | 0.0082 |
| SLC22A16 | 1927 | 9320331 | A | -6.63 | 0.0032 |
| SLC22A16 | 1928 | 9481067 | G | -3.29 | 0.0485 |
| UTRN | 1997 | 601873 | A | 3.65 | 0.0261 |
| UTRN | 1998 | 622718 | T | 3.37 | 0.0385 |
| UTRN | 2001 | 7765923 | A | 4.18 | 0.0359 |
| SYNE1 | 2003 | 2295190 | T | -4.67 | 0.0485 |
| SLC22A3 | 2013 | 12194182 | C | -5.11 | 0.0149 |
| SLC22A3 | 2014 | 3123636 | C | -4.62 | 0.0170 |
| SLC22A3 | 2015 | 2048327 | G | -3.96 | 0.0307 |
| PDE10A | 2056 | 486002 | G | -3.05 | 0.0426 |
| SDK1 | 2076 | 10951446 | A | 3.06 | 0.0398 |
| SDK1 | 2077 | 4723434 | A | -3.39 | 0.0217 |
| ETV1 | 2097 | 4721286 | T | -3.89 | 0.0465 |
| ETV1 | 2099 | 2237295 | G | 3.36 | 0.0426 |
| FERD3L | 2107 | 6967799 | T | 4.25 | 0.0382 |
| FLJ22374 | 2144 | 6956143 | G | 3.42 | 0.0350 |
| PDEIC | 2155 | 6952633 | G | 3.89 | 0.0259 |
| BMPER | 2164 | 6975236 | T | -4.38 | 0.0064 |
| VPS41 | 2181 | 859522 | G | 6.61 | 0.0299 |
| WBSCR17 | 2237 | 11768233 | A | -3.51 | 0.0257 |
| CACNA2D1 | 2303 | 12535840 | G | 3.56 | 0.0387 |
| CACNA2D1 | 2309 | 2059037 | C | 8.11 | 0.0023 |
| CACNA2D1 | 2314 | 1468400 | A | -4.44 | 0.0254 |
| PCLO | 2318 | 1407640 | C | -3.56 | 0.0428 |
| SEMA3E | 2335 | 2713154 | G | -4.36 | 0.0077 |
| SEMA3E | 2336 | 2713151 | T | -4.07 | 0.0145 |
| SEMA3E | 2337 | 2709890 | A | -4.07 | 0.0145 |
| SEMA3E | 2339 | 2713174 | C | -4.07 | 0.0145 |
| SEMA3E | 2341 | 10270844 | T | 3.96 | 0.0349 |
| SEMA3A | 2347 | 1989964 | T | -3.31 | 0.0281 |
| DYNC1II | 2384 | 1227517 | A | -2.90 | 0.0453 |
| PON1 | 2385 | 13223537 | C | -4.34 | 0.0067 |
| ZNF3 | 2398 | 7778571 | G | 4.12 | 0.0161 |
| CNTNAP2 | 2496 | 7802708 | G | 4.92 | 0.0048 |
| PTPRN2 | 2528 | 6459879 | C | 4.31 | 0.0174 |
| PTPRN2 | 2531 | 4909222 | G | -5.59 | 0.0163 |
| PTPRN2 | 2532 | 10265417 | T | -4.51 | 0.0057 |
| PTPRN2 | 2534 | 10266729 | T | -4.37 | 0.0092 |
| CSMD1 | 2538 | 4875250 | G | -4.92 | 0.0215 |
| CSMD1 | 2541 | 13272021 | C | 4.75 | 0.0182 |
| CSMD1 | 2543 | 13260434 | A | 4.76 | 0.0089 |
| MCPH1 | 2571 | 1057090 | T | -3.19 | 0.0322 |
| MCPH1 | 2572 | 2911968 | C | -4.02 | 0.0109 |
| PEBP4 | 2630 | 2457426 | C | -3.90 | 0.0127 |
| UNC5D | 2642 | 7826624 | T | 5.58 | 0.0043 |
| SNTG1 | 2670 | 7832799 | A | -4.52 | 0.0142 |
| SNTG1 | 2671 | 1484128 | T | -5.06 | 0.0039 |
| SNTG1 | 2672 | 1484129 | T | -5.13 | 0.0054 |
| DEPDC2 | 2726 | 2053140 | A | 3.90 | 0.0168 |
| DEPDC2 | 2732 | 7007570 | G | -4.38 | 0.0052 |
| KCNB2 | 2752 | 13279934 | G | -3.76 | 0.0390 |
| MMP16 | 2763 | 2139481 | T | 4.90 | 0.0059 |
| ZFPM2 | 2798 | 9643020 | A | 5.63 | 0.0111 |
| ZFPM2 | 2803 | 16873677 | A | 6.33 | 0.0134 |
| ZFPM2 | 2808 | 1372614 | G | 7.38 | 0.0015 |
| SAMD12 | 2824 | 2514588 | T | -3.76 | 0.0162 |
| SAMD12 | 2825 | 1607924 | G | -3.76 | 0.0162 |
| PIP5K1B | 2996 | 17392931 | C | 3.56 | 0.0417 |
| TMC1 | 3030 | 6560293 | A | 3.57 | 0.0306 |
| TMC1 | 3032 | 13285999 | A | 4.15 | 0.0109 |
| TMC1 | 3040 | 1663763 | T | -4.70 | 0.0060 |
| TMC1 | 3042 | 2501914 | T | 3.32 | 0.0311 |
| PCSK5 | 3054 | 1029035 | C | 3.30 | 0.0423 |
| DAPKI | 3084 | 3128499 | G | 3.41 | 0.0403 |
| ZNF169 | 3097 | 12236219 | T | 8.59 | 0.0235 |
| ZNF169 | 3098 | 7037298 | T | -5.08 | 0.0053 |
| SVEP1 | 3124 | 963143 | A | -3.23 | 0.0363 |
| SVEP1 | 3126 | 10759422 | C | -5.04 | 0.0091 |
| CDK5RAP2 | 3135 | 4836819 | A | 3.96 | 0.0491 |
| RALGPS1 | 3156 | 12551115 | C | 3.82 | 0.0205 |
| RALGPS1 | 3157 | 12350252 | A | 3.20 | 0.0430 |
| NTNG2 | 3174 | 10901133 | C | 4.14 | 0.0224 |
| VAV2 | 3189 | 2810473 | T | -4.20 | 0.0474 |
| VAV2 | 3190 | 2519109 | A | 3.49 | 0.0413 |
| VAV2 | 3192 | 2073829 | G | 3.74 | 0.0186 |
| NOTCH1 | 3200 | 10870085 | T | 5.69 | 0.0157 |
| CACNA1B | 3208 | 10867093 | T | 5.27 | 0.0472 |
| MY03A | 3275 | 7097387 | C | 3.33 | 0.0364 |
| MY03A | 3285 | 12764197 | G | 5.89 | 0.0029 |
| MY03A | 3287 | 3781117 | C | 4.39 | 0.0263 |
| KCNMA1 | 3338 | 1978868 | A | 4.83 | 0.0121 |
| GALNTL4 | 3469 | 4910289 | C | -3.44 | 0.0476 |
| ARNTL | 3484 | 2054605 | T | -3.48 | 0.0262 |
| SPON1 | 3498 | 1528667 | G | 3.58 | 0.0461 |
| DLG2 | 3531 | 11233708 | T | -4.18 | 0.0137 |
| OPCML | 3569 | 3016388 | C | 3.76 | 0.0460 |
| OPCML | 3570 | 3016382 | T | -4.13 | 0.0410 |
| STYK1 | 3608 | 2900467 | G | -3.66 | 0.0111 |
| LOC729025 | 3618 | 10219568 | A | -4.68 | 0.0029 |
| LOC729025 | 3625 | 3915237 | C | -4.68 | 0.0080 |
| NAV3 | 3672 | 1012088 | G | -3.44 | 0.0335 |
| NAV3 | 3678 | 1375287 | C | -5.59 | 0.0013 |
| NBEA | 3715 | 7333195 | T | 3.68 | 0.0157 |
| GPC5 | 3771 | 553717 | A | 3.65 | 0.0358 |
| GPC5 | 3774 | 4773644 | A | 6.73 | 0.0096 |
| GPC5 | 3775 | 7986797 | A | 6.50 | 0.0160 |
| NOVA1 | 3860 | 8008779 | C | 3.44 | 0.0450 |
| NPAS3 | 3872 | 6571604 | C | 6.10 | 0.0117 |
| NPAS3 | 3873 | 6571605 | A | 6.10 | 0.0117 |
| SLC25A21 | 3882 | 2022601 | A | 3.94 | 0.0181 |
| SLC25A21 | 3884 | 1154121 | T | 3.52 | 0.0409 |
| SLC25A21 | 3885 | 1367029 | A | 3.22 | 0.0410 |
| KCNK10 | 3963 | 4424855 | T | -6.33 | 0.0265 |
| CCDC88C | 3988 | 10139287 | A | -3.24 | 0.0460 |
| BCL11B | 4003 | 2614463 | T | 3.51 | 0.0375 |
| ATP10A | 4005 | 11853308 | C | -3.10 | 0.0485 |
| C15ORF41 | 4042 | 16964046 | T | 10.11 | 0.0025 |
| CGNL1 | 4070 | 7182648 | T | 5.08 | 0.0094 |
| CGNL1 | 4072 | 8034215 | G | 4.18 | 0.0146 |
| RORA | 4078 | 12914584 | G | -4.14 | 0.0276 |
| RORA | 4080 | 1657792 | A | 3.67 | 0.0364 |
| TBC1D2B | 4096 | 3803380 | G | -4.30 | 0.0444 |
| A2BP1 | 4123 | 7197955 | C | -3.54 | 0.0262 |
| A2BP1 | 4124 | 7199748 | C | -3.51 | 0.0286 |
| A2BP1 | 4125 | 11077158 | C | -3.51 | 0.0286 |
| A2BP1 | 4129 | 1922593 | T | -4.92 | 0.0152 |
| CDH13 | 4192 | 9926620 | C | 3.61 | 0.0222 |
| TGIF1 | 4275 | 1662805 | G | 5.52 | 0.0101 |
| CHST9 | 4311 | 4093026 | G | -4.26 | 0.0147 |
| CHST9 | 4312 | 720282 | G | -3.86 | 0.0269 |
| CHST9 | 4313 | 8084423 | A | -3.91 | 0.0304 |
| CCBE1 | 4346 | 10503032 | A | 3.58 | 0.0278 |
| CCBE1 | 4347 | 12454887 | G | 3.58 | 0.0278 |
| TXNDC10 | 4371 | 1477990 | A | 6.75 | 0.0350 |
| TXNDC10 | 4372 | 1477992 | G | 6.75 | 0.0350 |
| ATRN | 4390 | 151511 | A | -5.40 | 0.0111 |
| RNF24 | 4394 | 2143235 | A | -7.25 | 0.0361 |
| PLCB1 | 4415 | 2235212 | A | -3.45 | 0.0464 |
| PLCB1 | 4435 | 6056252 | A | 4.16 | 0.0265 |
| PLCB4 | 4436 | 6086690 | G | 4.46 | 0.0115 |
| MACROD2 | 4451 | 2193028 | C | 5.14 | 0.0481 |
| MACROD2 | 4460 | 775122 | A | 4.21 | 0.0339 |
| MAPRE1 | 4491 | 2070090 | T | 5.65 | 0.0275 |
| PTPRT | 4497 | 6065432 | T | -3.36 | 0.0395 |
| PTPRT | 4508 | 4812631 | A | -4.79 | 0.0169 |
| PTPRT | 4509 | 4810366 | C | -4.50 | 0.0258 |
| PTPRT | 4510 | 2017914 | G | -4.50 | 0.0258 |
| ZSWIM3 | 4516 | 6130953 | A | -3.92 | 0.0180 |
| CDH4 | 4537 | 6121844 | C | 9.29 | 0.0321 |
| ASPHD2 | 4572 | 8140344 | A | -3.39 | 0.0246 |
| HPS4 | 4574 | 5761552 | T | 3.33 | 0.0341 |
| EFCAB6 | 4599 | 12628583 | C | -7.08 | 0.0408 |
| EFCAB6 | 4609 | 2401408 | T | -4.60 | 0.0443 |

| **TABLE 11: Alleles Influencing Discontinuation for Lack of Efficacy for Olanzapine** | | | | | | |
|---|---|---|---|---|---|---|
| **Gene** | **Seq ID** | **NCBI RS#** | **Allele** | **Freq. in Discontinuers** | **P** | **Odds Ratio** |
| SCP2 | 43 | 13376501 | C | 0.167 | 0.017420 | 4.03 |
| SCP2 | 46 | 17107767 | C | 0.133 | 0.041620 | 3.69 |
| SEC16B | 129 | 869286 | G | 0.367 | 0.028920 | 2.52 |
| USH2A | 161 | 659875 | G | 0.467 | 0.001919 | 3.50 |
| USH2A | 163 | 62906 | G | 0.467 | 0.003684 | 3.23 |
| USH2A | 164 | 386654 | T | 0.467 | 0.004272 | 3.17 |
| USH2A | 166 | 700021 | A | 0.567 | 0.023670 | 2.46 |
| USH2A | 168 | 680410 | G | 0.467 | 0.005763 | 3.05 |
| ESRRG | 172 | 12757165 | G | 0.200 | 0.023670 | 0.35 |
| ESRRG | 173 | 6658528 | T | 0.231 | 0.025770 | 0.34 |
| ESRRG | 175 | 10495027 | A | 0.200 | 0.023670 | 0.35 |
| SLC35F3 | 188 | 538272 | A | 0.133 | 0.018040 | 0.28 |
| SLC35F3 | 189 | 480993 | T | 0.133 | 0.029100 | 0.31 |
| RYR2 | 196 | 12140458 | C | 0.600 | 0.036750 | 2.31 |
| CHRM3 | 216 | 16838637 | G | 0.233 | 0.010800 | 0.32 |
| CHRM3 | 217 | 1867265 | A | 0.233 | 0.025670 | 0.37 |
| CHRM3 | 218 | 2355237 | A | 0.667 | 0.016160 | 2.69 |
| CHRM3 | 224 | 6657343 | A | 0.667 | 0.010980 | 2.84 |
| FEZ2 | 339 | 1179494 | C | 0.167 | 0.033890 | 0.35 |
| SLC8A1 | 347 | 11885401 | C | 0.300 | 0.013010 | 3.10 |
| PRKCE | 382 | 4953288 | T | 0.200 | 0.037970 | 0.38 |
| COMMD1 | 430 | 11125907 | A | 0.179 | 0.019890 | 0.31 |
| AAK1 | 434 | 9789387 | A | 0.200 | 0.010040 | 0.30 |
| AAK1 | 435 | 6546534 | T | 0.100 | 0.005196 | 0.20 |
| AAK1 | 436 | 2311837 | C | 0.167 | 0.001543 | 0.22 |
| AAK1 | 437 | 6712370 | C | 0.100 | 0.003597 | 0.19 |
| AAK1 | 438 | 12713679 | G | 0.167 | 0.004392 | 0.25 |
| AAK1 | 439 | 10779953 | G | 0.133 | 0.009626 | 0.25 |
| AAK1 | 440 | 12471316 | G | 0.200 | 0.005740 | 0.28 |
| CTNNA2 | 466 | 1930 | T | 0.667 | 0.010980 | 2.84 |
| CTNNA2 | 467 | 12464777 | T | 0.667 | 0.016160 | 2.69 |
| RAB3GAP1 | 489 | 935614 | G | 0.200 | 0.013440 | 3.81 |
| ZRANB3 | 492 | 16831601 | T | 0.200 | 0.011320 | 3.92 |
| ZRANB3 | 493 | 4953951 | T | 0.200 | 0.012330 | 3.86 |
| ZRANB3 | 494 | 16831751 | G | 0.200 | 0.019150 | 3.50 |
| ZRANB3 | 495 | 6430579 | T | 0.167 | 0.046440 | 3.13 |
| LRP1B | 511 | 1949871 | C | 0.133 | 0.021220 | 0.29 |
| ARHGAP15 | 542 | 6747717 | T | 0.000 | 0.008333 | 0.00 |
| ARHGAP15 | 544 | 16858944 | A | 0.033 | 0.012420 | 0.11 |
| ARHGAP15 | 552 | 6745691 | G | 0.600 | 0.012320 | 2.75 |
| ARHGAP15 | 555 | 10197021 | T | 0.600 | 0.014310 | 2.67 |
| ARHGAP15 | 556 | 13415550 | T | 0.433 | 0.045800 | 2.25 |
| FMNL2 | 580 | 2577180 | G | 0.300 | 0.023410 | 2.79 |
| PLA2R1 | 606 | 2667023 | C | 0.033 | 0.043040 | 0.16 |
| SCN1A | 621 | 3812718 | C | 0.300 | 0.023380 | 0.39 |
| SCN9A | 627 | 4128577 | T | 0.467 | 0.043790 | 2.25 |
| PARD3B | 659 | 6736058 | G | 0.100 | 0.005196 | 0.20 |
| PARD3B | 660 | 6760872 | C | 0.100 | 0.005196 | 0.20 |
| PARD3B | 661 | 4675490 | G | 0.200 | 0.010040 | 0.30 |
| NRP2 | 673 | 13396083 | C | 0.300 | 0.032720 | 0.41 |
| NRP2 | 674 | 7596827 | T | 0.321 | 0.039530 | 0.41 |
| CUL3 | 685 | 11891006 | G | 0.033 | 0.040830 | 0.15 |
| CUL3 | 687 | 1542789 | A | 0.033 | 0.043040 | 0.16 |
| DNER | 717 | 6743564 | C | 0.567 | 0.012770 | 2.70 |
| CNTN4 | 756 | 1523320 | C | 0.300 | 0.032770 | 0.41 |
| ITPR1 | 774 | 304073 | G | 0.214 | 0.028180 | 0.35 |
| ITPR1 | 775 | 304038 | G | 0.233 | 0.030860 | 0.38 |
| ITPR1 | 777 | 2322830 | T | 0.633 | 0.013910 | 2.71 |
| ATP2B2 | 792 | 6763274 | C | 0.100 | 0.025400 | 0.26 |
| SLC6A6 | 794 | 1156567 | C | 0.000 | 0.033890 | 0.00 |
| ULK4 | 845 | 1495698 | C | 0.533 | 0.045530 | 2.22 |
| FLNB | 913 | 7622687 | A | 0:607 | 0.024250 | 2.54 |
| PTPRG | 939 | 875423 | T | 0.667 | 0.038750 | 2.35 |
| CADPS | 947 | 186236 | C | 0.433 | 0.041620 | 2.29 |
| PRICKLE2 | 958 | 161661 | A | 0.600 | 0.017500 | 2.59 |
| FAMI9A1 | 979 | 12486136 | A | 0.633 | 0.010330 | 2.82 |
| FOXP1 | 985 | 2597312 | C | 0.567 | 0.006420 | 2.96 |
| CDGAP | 1021 | 10934491 | C | 0.433 | 0.008361 | 2.94 |
| CDGAP | 1025 | 16829838 | T | 0.267 | 0.036970 | 2.67 |
| KALRN | 1046 | 16835912 | C | 0.700 | 0.005012 | 3.22 |
| CLSTN2 | 1077 | 4683509 | G | 0.067 | 0.036600 | 0.23 |
| SPSB4 | 1085 | 7620622 | A | 0.100 | 0.032370 | 0.28 |
| NLGN1 | 1112 | 16828652 | A | 0.000 | 0.029220 | 0.00 |
| NLGN1 | 1113 | 16828952 | A | 0.200 | 0.027800 | 0.35 |
| NLGN1 | 1114 | 9870677 | C | 0.167 | 0.045810 | 0.37 |
| SLIT2 | 1188 | 10516357 | A | 0.567 | 0.009526 | 2.81 |
| PPARGC1A | 1192 | 10938963 | A | 0.200 | 0.020080 | 0.34 |
| PPARGC1A | 1193 | 8192678 | A | 0.167 | 0.039460 | 0.36 |
| PCDH7 | 1200 | 4611883 | G | 0.233 | 0.004096 | 0.28 |
| PCDH7 | 1201 | 6812005 | G | 0.233 | 0.015450 | 0.34 |
| PCDH7 | 1203 | 12331633 | C | 0.767 | 0.000856 | 4.30 |
| LIMCH1 | 1216 | 4339249 | C | 0.533 | 0.031450 | 2.36 |
| LIMCH1 | 1222 | 10805090 | G | 0.467 | 0.029100 | 2.41 |
| DKK2 | 1306 | 81299 | T | 0.000 | 0.045500 | 0.00 |
| PAPSS1 | 1308 | 4956020 | T | 0.467 | 0.001350 | 3.65 |
| PAPSS1 | 1309 | 12502059 | A | 0.464 | 0.000634 | 4.13 |
| CAMK2D | 1357 | 4834340 | A | 0.600 | 0.010420 | 2.79 |
| CAMK2D | 1358 | 11098193 | T | 0.433 | 0.045800 | 2.25 |
| CAMK2D | 1360 | 13144613 | G | 0.500 | 0.049510 | 2.19 |
| DCLK2 | 1440 | 11727182 | G | 0.633 | 0.037460 | 2.33 |
| CTSO | 1444 | 11946572 | G | 0.500 | 0.028480 | 2.42 |
| FSTL5 | 1460 | 2082669 | G | 0.367 | 0.026200 | 2.58 |
| PALLD | 1492 | 2712118 | C | 0.167 | 0.039460 | 0.36 |
| PALLD | 1494 | 4389538 | G | 0.667 | 0.022930 | 2.55 |
| PALLD | 1496 | 2710827 | A | 0.167 | 0.021100 | 0.32 |
| ODZ3 | 1519 | 10028158 | A | 0.300 | 0.016790 | 2.96 |
| SEMASA | 1543 | 12520210 | A | 0.233 | 0.041230 | 0.40 |
| EGFLAM | 1588 | 16903920 | G | 0.200 | 0.000669 | 0.21 |
| EGFLAM | 1589 | 2434504 | A | 0.300 | 0.032770 | 0.41 |
| EGFLAM | 1591 | 1428263 | C | 0.167 | 0.045810 | 0.37 |
| EGFLAM | 1593 | 2561111 | T | 0.200 | 0.045500 | 2.88 |
| PDE4D | 1626 | 2910642 | C | 0.233 | 0.018370 | 0.35 |
| PDE4D | 1627 | 2910641 | A | 0.267 | 0.036970 | 2.67 |
| PDE4D | 1632 | 294494 | A | 0.067 | 0.034110 | 0.23 |
| ELOVL7 | 1636 | 6449505 | C | 0.067 | 0.022930 | 0.21 |
| CMYAS | 1650 | 11960229 | C | 0.400 | 0.000488 | 4.33 |
| GPR98 | 1686 | 4916684 | G | 0.633 | 0.022560 | 2.52 |
| FBXL 17 | 1719 | 2193976 | T | 0.321 | 0.039530 | 0.41 |
| KCNN2 | 1730 | 4435855 | A | 0.267 | 0.012550 | 3.27 |
| LOC340069 | 1745 | 919306 | C | 0.233 | 0.027380 | 0.37 |
| LOC340069 | 1746 | 328694 | A | 0.233 | 0.035330 | 0.39 |
| SNCAIP | 1752 | 10066937 | A | 0.367 | 0.016750 | 2.76 |
| SNCAIP | 1754 | 3811879 | C | 0.393 | 0.015460 | 2.82 |
| SNCAIP | 1755 | 1841972 | C | 0.367 | 0.016750 | 2.76 |
| SNCAIP | 1756 | 11241644 | G | 0.367 | 0.028920 | 2.52 |
| SNCAIP | 1757 | 2407209 | A | 0.567 | 0.041230 | 2.26 |
| ADAMTS19 | 1767 | 30651 | G | 0.133 | 0.009170 | 0.25 |
| ADAMTS19 | 1768 | 30645 | T | 0.233 | 0.001087 | 0.24 |
| ADAMTS19 | 1769 | 4835975 | A | 0.107 | 0.003065 | 0.18 |
| TRPC7 | 1775 | 3777145 | T | 0.267 | 0.005054 | 3.83 |
| TRPC7 | 1778 | 950714 | G | 0.267 | 0.012550 | 3.27 |
| TRPC7 | 1779 | 2649691 | A | 0.267 | 0.020420 | 3.00 |
| NEDD9 | 1814 | 8180658 | C | 0.733 | 0.001341 | 3.90 |
| PHACTR1 | 1819 | 9296512 | G | 0.600 | 0.011620 | 2.75 |
| PHACTR1 | 1822 | 4714990 | C | 0.567 | 0.034670 | 2.33 |
| PHACTR1 | 1825 | 202066 | G | 0.200 | 0.047540 | 0.39 |
| ATXN1 | 1835 | 235147 | A | 0.700 | 0.002481 | 3.52 |
| SLC17A1 | 1854 | 1165196 | C | 0.700 | 0.023310 | 2.60 |
| SLC17A1 | 1856 | 1165177 | T | 0.700 | 0.038470 | 2.40 |
| SLC17A3 | 1861 | 1165207 | A | 0.733 | 0.016160 | 2.82 |
| SLC17A3 | 1862 | 1165205 | T | 0.733 | 0.019520 | 2.75 |
| SLC17A2 | 1868 | 442601 | C | 0.533 | 0.008603 | 2.84 |
| SLC17A2 | 1871 | 199739 | T | 0.500 | 0.022460 | 2.49 |
| BTN3A1 | 1872 | 12208788 | T | 0.267 | 0.043820 | 2.59 |
| KLC4 | 1883 | 3793017 | C | 0.033 | 0.002680 | 0.08 |
| TRDN | 1935 | 13190807 | C | 0.500 | 0.027710 | 2.41 |
| TRDN | 1945 | 7452290 | C | 0.233 | 0.031710 | 2.91 |
| TRDN | 1948 | 6931183 | A | 0.233 | 0.031710 | 2.91 |
| TFB1M | 2010 | 9371369 | A | 0.600 | 0.021260 | 2.52 |
| SLC22A3 | 2013 | 12194182 | C | 0.433 | 0.037180 | 2.34 |
| NXPH1 | 2089 | 11489426 | C | 0.600 | 0.025670 | 2.45 |
| SCRN1 | 2133 | 11973169 | T | 0.300 | 0.023410 | 2.79 |
| PLEKHA8 | 2139 | 7806238 | A | 0.300 | 0.010990 | 3.20 |
| ABCA13 | 2211 | 10243683 | T | 0.167 | 0.043370 | 0.36 |
| ABCA 13 | 2212 | 3923511 | G | 0.133 | 0.045800 | 0.34 |
| WBSCR17 | 2238 | 6971383 | A | 0.200 | 0.025240 | 0.35 |
| WBSCR17 | 2247 | 7806531 | T | 0.267 | 0.043590 | 0.42 |
| WBSCR17 | 2248 | 4717604 | T | 0.200 | 0.044160 | 0.39 |
| LIMK1 | 2249 | 810532 | G | 0.033 | 0.018760 | 0.13 |
| LIMK1 | 2250 | 2855726 | A | 0.167 | 0.024790 | 0.33 |
| GTF2IRD1 | 2251 | 37612 | C | 0.533 | 0.037970 | 2.29 |
| HIP1 | 2255 | 1167802 | C | 0.033 | 0.010560 | 0.11 |
| MAGI2 | 2285 | 2191806 | C | 0.067 | 0.049510 | 0.25 |
| MAGI2 | 2286 | 10255710 | C | 0.067 | 0.045500 | 0.24 |
| MAGI2 | 2291 | 1528267 | C | 0.033 | 0.037030 | 0.15 |
| ABCB4 | 2353 | 31671 | G | 0.067 | 0.009367 | 0.17 |
| GTPBP10 | 2362 | 11972149 | T | 0.400 | 0.025630 | 2.53 |
| GTPBP10 | 2363 | 17863999 | A | 0.400 | 0.023390 | 2.56 |
| GTPBP10 | 2364 | 13222379 | A | 0.433 | 0.023800 | 2.52 |
| CADPS2 | 2420 | 2074589 | C | 0.200 | 0.044160 | 0.39 |
| SLC13A1 | 2426 | 1404836 | C | 0.100 | 0.010640 | 0.22 |
| SLC13A1 | 2429 | 6963735 | C | 0.133 | 0.012940 | 0.27 |
| SLC13A1 | 2431 | 10253693 | T | 0.567 | 0.004182 | 3.13 |
| SLC13A1 | 2432 | 2204296 | T | 0.500 | 0.016480 | 2.61 |
| EXOC4 | 2459 | 1946303 | T | 0.367 | 0.013940 | 2.85 |
| EXOC4 | 2461 | 6975109 | T | 0.367 | 0.020950 | 2.67 |
| PTPRN2 | 2519 | 4909289 | A | 0.433 | 0.033540 | 2.38 |
| SLC7A2 | 2601 | 7002951 | G | 0.133 | 0.009654 | 5.62 |
| NKAIN3 | 2712 | 10102831 | A | 0.233 | 0.000917 | 5.78 |
| NKAIN3 | 2713 | 10504356 | G | 0.167 | 0.046440 | 3.13 |
| NKAIN3 | 2714 | 10504358 | G | 0.167 | 0.046440 | 3.13 |
| KCNB2 | 2744 | 1972888 | C | 0.067 | 0.005024 | 0.15 |
| KCNB2 | 2745 | 2196904 | C | 0.067 | 0.005024 | 0.15 |
| KCNB2 | 2747 | 1899077 | T | 0.067 | 0.005590 | 0.16 |
| KCNB2 | 2748 | 1440355 | C | 0.067 | 0.008509 | 0.17 |
| KCNB2 | 2749 | 1965805 | G | 0.067 | 0.008509 | 0.17 |
| KCNB2 | 2750 | 2053421 | G | 0.067 | 0.013060 | 0.18 |
| KCNB2 | 2753 | 1026869 | T | 0.600 | 0.021260 | 2.52 |
| GRHL2 | 2774 | 682769 | A | 0.100 | 0.032370 | 0.28 |
| ZFPM2 | 2803 | 16873677 | A | 0.233 | 0.045200 | 2.70 |
| ZFPM2 | 2804 | 16873707 | T | 0.200 | 0.048840 | 2.83 |
| ZFPM2 | 2806 | 7007352 | G | 0.233 | 0.048850 | 2.66 |
| CSMD3 | 2811 | 10088226 | G | 0.133 | 0.000006 | 2.83 |
| CSMD3 | 2812 | 11996690 | A | 0.133 | 0.000006 | 2.75 |
| CSMD3 | 2813 | 7012235 | C | 0.133 | 0.000006 | 2.75 |
| CSMD3 | 2814 | 10505203 | A | 0.100 | 0.008367 | 8.22 |
| FER1L6 | 2834 | 6981430 | G | 0.533 | 0.024230 | 2.46 |
| KIAA1797 | 2960 | 7848159 | G | 0.267 | 0.008146 | 3.53 |
| KIAA1797 | 2962 | 2151002 | A | 0.567 | 0.031480 | 2.36 |
| KIAA1797 | 2964 | 2039390 | A | 0.267 | 0.003735 | 4.06 |
| KlAA1797 | 2965 | 7858506 | G | 0.267 | 0.018600 | 3.05 |
| PIPSK1B | 2995 | 963707 | G | 0.167 | 0.039460 | 0.36 |
| TMC1 | 3041 | 13298704 | C | 0.300 | 0.038290 | 2.55 |
| GNAQ | 3061 | 7048811 | G | 0.400 | 0.032850 | 2.43 |
| DIRAS2 | 3086 | 690232 | A | 0.071 | 0.037160 | 0.23 |
| DIRAS2 | 3087 | 532591 | C | 0.100 | 0.031340 | 0.27 |
| HIATL1 | 3099 | 9409765 | A | 0.333 | 0.038170 | 0.42 |
| HIATL1 | 3100 | 9409552 | G | 0.333 | 0.045500 | 0.44 |
| TTLLI1 | 3144 | 13299260 | C | 0.300 | 0.038470 | 0.42 |
| TSC1 | 3179 | 1073123 | G | 0.367 | 0.028920 | 2.52 |
| TSC1 | 3180 | 7858160 | T | 0.367 | 0.032120 | 2.48 |
| TSC1 | 3182 | 7035308 | A | 0.500 | 0.035810 | 2.32 |
| VAV2 | 3186 | 7023551 | G | 0.367 | 0.046930 | 2.32 |
| CACNA1B | 3213 | 10867107 | C | 0.400 | 0.037670 | 2.36 |
| CACNB2 | 3239 | 729245 | A | 0.067 | 0.045500 | 0.24 |
| IMJD1C | 3297 | 10995505 | A | 0.067 | 0.019550 | 0.20 |
| JMJD1C | 3299 | 10761747 | G | 0.067 | 0.021170 | 0.20 |
| JMJD1C | 3301 | 9629895 | A | 0.067 | 0.039190 | 0.23 |
| JMJD1C | 3303 | 2893922 | C | 0.033 | 0.043040 | 0.16 |
| SORCS3 | 3388 | 7072425 | T | 0.000 | 0.032590 | 0.00 |
| VTI1A | 3398 | 7097587 | A | 0.067 | 0.019510 | 0.20 |
| ATRNL1 | 3411 | 10885719 | T | 0.200 | 0.040790 | 0.38 |
| ATRNL1 | 3412 | 4364974 | G | 0.067 | 0.005590 | 0.16 |
| ATRNL1 | 3414 | 2804207 | C | 0.357 | 0.022000 | 0.38 |
| ATRNL1 | 3416 | 10787584 | C | 0.367 | 0.025060 | 0.40 |
| ATRNL1 | 3417 | 2420098 | G | 0.367 | 0.027030 | 0.41 |
| ATRNL1 | 3421 | 180675 | A | 0.036 | 0.030580 | 0.14 |
| ATRNL1 | 3422 | 180635 | T | 0.100 | 0.043730 | 0.30 |
| ATE1 | 3438 | 10510104 | G | 0.000 | 0.039290 | 0.00 |
| ATE1 | 3445 | 4752611 | A | 0.533 | 0.024230 | 2.46 |
| ATE1 | 3449 | 11200251 | T | 0.500 | 0.007236 | 2.94 |
| CTBP2 | 3455 | 9422765 | G | 0.400 | 0.010300 | 2.91 |
| CTBP2 | 3456 | 7896555 | G | 0.400 | 0.010300 | 2.91 |
| STIM1 | 3457 | 7952083 | C | 0.233 | 0.004090 | 0.28 |
| STIM1 | 3458 | 10835249 | G | 0.233 | 0.025670 | 0.37 |
| SPON1 | 3496 | 1876427 | G | 0.100 | 0.037670 | 0.29 |
| SPON1 | 3497 | 2049723 | C | 0.067 | 0.022930 | 0.21 |
| 81.99 mb | 3525 | 1939631 | G | 0.567 | 0.028670 | 2.39 |
| DLG2 | 3553 | 624493 | G | 0.300 | 0.009859 | 3.25 |
| DLG2 | 3554 | 582652 | A | 0.286 | 0.014700 | 3.20 |
| DLG2 | 3555 | 483220 | G | 0.300 | 0.023410 | 2.79 |
| TSPAN9 | 3595 | 10774140 | C | 0.500 | 0.033890 | 2.33 |
| TMEM16B | 3597 | 4930809 | T | 0.433 | 0.021070 | 2.56 |
| ITPR2 | 3638 | 7955049 | G | 0.033 | 0.017100 | 0.12 |
| KCNC2 | 3660 | 10879888 | G | 0.133 | 0.045800 | 0.34 |
| NAV3 | 3675 | 10859738 | A | 0.667 | 0.019110 | 2.63 |
| N4BP2L2 | 3701 | 1081796 | A | 0.400 | 0.001406 | 3.82 |
| N4BP2L2 | 3702 | 208431 | G | 0.393 | 0.011630 | 2.95 |
| NBEA | 3705 | 867503 | C | 0.000 | 0.045500 | 0.00 |
| NBEA | 3709 | 669933 | A | 0.033 | 0.017100 | 0.12 |
| NBEA | 3710 | 2105174 | T | 0.033 | 0.017100 | 0.12 |
| NBEA | 3711 | 9530540 | C | 0.167 | 0.029030 | 0.34 |
| NBEA | 3717 | 1536634 | T | 0.267 | 0.016160 | 0.35 |
| NBEA | 3718 | 2798337 | A | 0.167 | 0.045810 | 0.37 |
| LMO7 | 3741 | 502171 | G | 0.267 | 0.031820 | 0.39 |
| LMO7 | 3742 | 7335067 | A | 0.433 | 0.033540 | 2.38 |
| LMO7 | 3744 | 7321748 | C | 0.100 | 0.001027 | 0.16 |
| LMO7 | 3745 | 7326714 | T | 0.100 | 0.001107 | 0.16 |
| LMO7 | 3746 | 9318373 | A | 0.633 | 0.000265 | 4.30 |
| LMO7 | 3749 | 11617789 | G | 0.000 | 0.006411 | 0.00 |
| LMO7 | 3751 | 9530473 | G | 0.133 | 0.012940 | 0.27 |
| SLAIN1 | 3758 | 9318496 | T | 0.033 | 0.023440 | 0.13 |
| SLAIN1 | 3759 | 1146920 | C | 0.033 | 0.002680 | 0.08 |
| SLAIN1 | 3760 | 10507874 | G | 0.000 | 0.021650 | 0.00 |
| GPC5 | 3767 | 9560813 | T | 0.533 | 0.000872 | 3.76 |
| GPC5 | 3768 | 9560814 | C | 0.533 | 0.001213 | 3.62 |
| GPC5 | 3784 | 10507993 | G | 0.367 | 0.045470 | 2.34 |
| GPC5 | 3786 | 1933197 | C | 0.333 | 0.022930 | 0.39 |
| GPC6 | 3793 | 2209880 | A | 0.400 | 0.018060 | 2.67 |
| GPC6 | 3813 | 9524268 | T | 0.567 | 0.000671 | 3.85 |
| GPC6 | 3820 | 9524371 | C | 0.100 | 0.018410 | 0.25 |
| GPC6 | 3821 | 7337873 | C | 0.133 | 0.039470 | 0.33 |
| NALCN | 3827 | 588083 | C | 0.300 | 0.045020 | 0.43 |
| NPAS3 | 3870 | 1315139 | T | 0.214 | 0.015640 | 0.32 |
| SLC25A21 | 3890 | 1950367 | G | 0.067 | 0.019110 | 10.64 |
| LRFN5 | 3891 | 7148640 | G | 0.467 | 0.018600 | 2.58 |
| RGS6 | 3932 | 11158926 | T | 0.633 | 0.001891 | 3.49 |
| RGS6 | 3933 | 7156200 | C | 0.433 | 0.029870 | 2.42 |
| RGS6 | 3934 | 11623548 | T | 0.333 | 0.045500 | 0.44 |
| RGS6 | 3943 | 2214965 | T | 0.233 | 0.047940 | 0.41 |
| RGS6 | 3950 | 2239221 | C | 0.233 | 0.035330 | 0.39 |
| ATP10A | 4005 | 11853308 | C | 0.233 | 0.007423 | 0.30 |
| ATP10A | 4006 | 7179423 | G | 0.600 | 0.030800 | 2.38 |
| ATP10VA | 4007 | 2066707 | G | 0.167 | 0.004267 | 0.25 |
| ATP10A | 4008 | 7178018 | A | 0.533 | 0.037970 | 2.29 |
| MEIS2 | 4050 | 6495883 | G | 0.167 | 0.045810 | 0.37 |
| CGNL1 | 4071 | 4774941 | G | 0.200 | 0.012330 | 3.86 |
| CGNL1 | 4073 | 16977507 | A | 0.167 | 0.049540 | 3.09 |
| CGNL1 | 4076 | 16977597 | T | 0.033 | 0.024940 | 3.86 |
| RORA | 4078 | 12914584 | G | 0.067 | 0.011070 | 0.18 |
| RORA | 4079 | 4774370 | C | 0.067 | 0.028920 | 0.22 |
| RORA | 4082 | 11071548 | G | 0.667 | 0.004725 | 3.17 |
| TBC1D2B | 4096 | 3803380 | G | 0.033 | 0.014590 | 0.12 |
| SH3GL3 | 4108 | 2244838 | C | 0.367 | 0.005772 | 3.26 |
| A2BP1 | 4113 | 17822719 | A | 0.214 | 0.018470 | 0.33 |
| A2BP1 | 4115 | 11077043 | G | 0.321 | 0.028620 | 0.39 |
| A2BP1 | 4128 | 9934782 | G | 0.300 | 0.005930 | 0.32 |
| WWOX | 4158 | 3764342 | C | 0.300 | 0.002226 | 4.10 |
| WWOX | 4160 | 12051258 | T | 0.167 | 0.049540 | 3.09 |
| MPHOSPH6 | 4162 | 7405231 | C | 0.667 | 0.027720 | 2.48 |
| MPHOSPH6 | 4175 | 2967339 | T | 0.300 | 0.034910 | 2.59 |
| MPHOSPH6 | 4178 | 1011433 | C | 0.300 | 0.020520 | 2.87 |
| MPHOSPH6 | 4181 | 2932812 | A | 0.300 | 0.031820 | 2.63 |
| CDH13 | 4205 | 11862993 | A | 0.167 | 0.028590 | 3.55 |
| CA10 | 4239 | 9893317 | A | 0.633 | 0.030830 | 2.41 |
| CA10 | 4240 | 1503058 | A | 0.300 | 0.016270 | 0.36 |
| MS12 | 4255 | 1007462 | C | 0.233 | 0.002145 | 0.26 |
| PTPRM | 4294 | 11081352 | T | 0.500 | 0.014360 | 2.66 |
| PTPRM | 4295 | 649598 | T | 0.467 | 0.035860 | 2.33 |
| PTPRM | 4296 | 623258 | T | 0.467 | 0.043790 | 2.25 |
| PTPRM | 4298 | 619379 | C | 0.467 | 0.049190 | 2.21 |
| OSBPL1A | 4310 | 275857 | C | 0.100 | 0.025860 | 5.44 |
| CCBE1 | 4338 | 4558500 | C | 0.200 | 0.024430 | 0.35 |
| CCBE1 | 4339 | 1557406 | G | 0.200 | 0.037970 | 0.38 |
| CDH7 | 4353 | 10871590 | A | 0.143 | 0.015220 | 0.27 |
| CDH7 | 4354 | 10460125 | T | 0.133 | 0.033930 | 0.32 |
| CDH7 | 4355 | 7237421 | T | 0.133 | 0.039470 | 0.33 |
| CDH7 | 4356 | 8092259 | G | 0.133 | 0.044400 | 0.33 |
| CDH7 | 4358 | 1484725 | T | 0.133 | 0.045800 | 0.34 |
| DOK6 | 4382 | 9962570 | T | 0.533 | 0.003976 | 3.14 |
| LDLR | 4384 | 2116897 | T | 0.400 | 0.023390 | 2.56 |
| PLCB1 | 4420 | 2294259 | C | 0.133 | 0.039470 | 0.33 |
| PLCB1 | 4435 | 6056252 | A | 0.533 | 0.015830 | 2.62 |
| PLCB4 | 4436 | 6086690 | G | 0.533 | 0.015830 | 2.62 |
| MACROD2 | 4445 | 11908639 | G | 0.667 | 0.004725 | 3.17 |
| MACROD2 | 4446 | 1743471 | A | 0.667 | 0.005888 | 3.09 |
| MACROD2 | 4448 | 459322 | G | 0.643 | 0.011440 | 2.87 |
| MACROD2 | 4449 | 459874 | C | 0.633 | 0.012670 | 2.74 |
| MACROD2 | 4453 | 175805 | T | 0.400 | 0.041920 | 2.32 |
| MACROD2 | 4455 | 6043616 | C | 0.267 | 0.047700 | 2.55 |
| KIF16B | 4484 | 6080359 | C | 0.067 | 0.045500 | 0.24 |
| PTPRT | 4497 | 6065432 | T | 0.467 | 0.003684 | 3.23 |
| PTPRT | 4498 | 746413 | C | 0.633 | 0.007999 | 2.92 |
| PTPRT | 4499 | 1126101 | G | 0.233 | 0.042280 | 0.40 |
| PTPRT | 4500 | 6065434 | T | 0.500 | 0.008152 | 2.89 |
| PTPRT | 4501 | 6016688 | T | 0.433 | 0.002313 | 3.48 |
| CDH4 | 4538 | 6142884 | A | 0.333 | 0.038750 | 0.43 |
| NCAM2 | 4543 | 2826351 | A | 0.567 | 0.006420 | 2.96 |
| NCAM2 | 4545 | 232518 | C | 0.133 | 0.015300 | 0.27 |
| EFCAB6 | 4599 | 12628583 | C | 0.233 | 0.029220 | 2.96 |
| EFCAB6 | 4602 | 470083 | C | 0.367 | 0.008227 | 3.10 |
| EFCAB6 | 4603 | 137733 | T | 0.167 | 0.000139 | 0.16 |
| EFCAB6 | 4606 | 5764214 | C | 0.200 | 0.001341 | 0.23 |
| EFCAB6 | 4609 | 2401408 | T | 0.367 | 0.011470 | 2.94 |
| EFCAB6 | 4612 | 2187887 | G | 0.133 | 0.004437 | 0.22 |
| EFCAB6 | 4613 | 5764302 | G | 0.200 | 0.020750 | 3.45 |
| EFCAB6 | 4614 | 5764310 | C | 0.200 | 0.011320 | 3.92 |

| **TABLE 12: Alleles Influencing Discontinuation for Lack of Efficacy for Risperidone** | | | | | | |
|---|---|---|---|---|---|---|
| **Gene** | **Seq ID** | **NCBI RS#** | **Allele** | **Freq. in Discontinuers** | **P** | **Odds Ratio** |
| RP1-21O18.1 | 14 | 1000313 | G | 0.18 | 0.0402 | 0.46 |
| RP1-21O18.1 | 17 | 4661575 | T | 0.30 | 0.0461 | 0.52 |
| LRP8 | 55 | 17108202 | G | 0.50 | 0.0262 | 2.00 |
| DPT | 121 | 6427142 | G | 0.21 | 0.0378 | 2.39 |
| DPT | 125 | 1018453 | C | 0.39 | 0.0311 | 0.51 |
| SEC16B | 126 | 1889981 | T | 0.33 | 0.0087 | 2.58 |
| SEC16B | 128 | 2862300 | G | 0.40 | 0.0023 | 2.76 |
| SEC16B | 129 | 869286 | G | 0.37 | 0.0017 | 2.95 |
| SEC16B | 130 | 1934650 | C | 0.26 | 0.0105 | 2.71 |
| PLA2G4A | 135 | 12128551 | A | 0.12 | 0.0353 | 0.39 |
| PLA2G4A | 137 | 12720662 | T | 0.16 | 0.0449 | 0.46 |
| KCNK2 | 150 | 11120527 | T | 0.37 | 0.0169 | 2.25 |
| USH2A | 161 | 659875 | G | 0.37 | 0.0065 | 2.56 |
| USH2A | 163 | 62906 | G | 0.37 | 0.0093 | 2.40 |
| USH2A | 165 | 10864235 | A | 0.48 | 0.0212 | 2.08 |
| USH2A | 168 | 680410 | G | 0.37 | 0.0096 | 2.43 |
| ESRRG | 179 | 12023399 | G | 0.37 | 0.0265 | 2.10 |
| ESRRG | 182 | 12037068 | C | 0.07 | 0.0247 | 0.30 |
| ESRRG | 184 | 7529655 | A | 0.08 | 0.0301 | 0.34 |
| RYR2 | 196 | 12140458 | C | 0.32 | 0.0423 | 0.52 |
| RYR2 | 215 | 4659819 | T | 0.58 | 0.0419 | 1.88 |
| FMN2 | 234 | 11801848 | C | 0.47 | 0.0073 | 2.34 |
| FMN2 | 236 | 1520065 | G | 0.55 | 0.0078 | 2.29 |
| FMN2 | 237 | 897662 | T | 0.56 | 0.0041 | 2.45 |
| FMN2 | 239 | 1542400 | G | 0.32 | 0.0051 | 0.41 |
| FMN2 | 240 | 4659569 | T | 0.15 | 0.0237 | 0.40 |
| FMN2 | 241 | 10754696 | T | 0.15 | 0.0386 | 0.43 |
| FMN2 | 242 | 10754697 | A | 0.15 | 0.0390 | 0.44 |
| FMN2 | 243 | 16832176 | C | 0.13 | 0.0427 | 0.43 |
| DDEF2 | 283 | 10593 | G | 0.27 | 0.0199 | 2.39 |
| BRE | 325 | 1546029 | C | 0.16 | 0.0500 | 0.47 |
| PLEKHH2 | 359 | 4952676 | G | 0.35 | 0.0465 | 0.53 |
| PLEKHH2 | 360 | 12621654 | T | 0.40 | 0.0232 | 2.15 |
| CCDC85A | 421 | 2869529 | C | 0.45 | 0.0431 | 1.89 |
| COMMD1 | 429 | 7569442 | C | 0.19 | 0.0075 | 0.38 |
| COMMD1 | 430 | 11125907 | A | 0.23 | 0.0096 | 0.41 |
| CTNNA2 | 463 | 318369 | G | 0.29 | 0.0061 | 0.41 |
| CTNNA2 | 464 | 318362 | C | 0.19 | 0.0168 | 0.42 |
| NAP5 | 482 | 13432172 | G | 0.00 | 0.0261 | 0.00 |
| KYNU | 525 | 2278587 | A | 0.00 | 0.0142 | 0.00 |
| KYNU | 529 | 16858404 | T | 0.05 | 0.0164 | 0.24 |
| ARHGAP15 | 559 | 16823114 | A | 0.53 | 0.0004 | 3.03 |
| ARHGAP15 | 561 | 13031917 | C | 0.53 | 0.0007 | 2.92 |
| PKP4 | 596 | 1990815 | C | 0.31 | 0.0333 | 0.50 |
| PKP4 | 597 | 10933492 | A | 0.27 | 0.0490 | 2.09 |
| PKP4 | 600 | 2528582 | G | 0.29 | 0.0231 | 0.48 |
| CERKL | 635 | 4018756 | A | 0.59 | 0.0461 | 1.90 |
| PDE1A | 639 | 11895090 | A | 0.40 | 0.0307 | 2.03 |
| PDE1A | 641 | 6736414 | T | 0.39 | 0.0085 | 2.41 |
| PARD3B | 662 | 7559302 | T | 0.42 | 0.0125 | 2.26 |
| COL4A3 | 699 | 6758076 | C | 0.05 | 0.0164 | 0.24 |
| CNTN4 | 770 | 12497768 | T | 0.60 | 0.0391 | 1.91 |
| ERC2 | 883 | 9810436 | A | 0.37 | 0.0265 | 2.10 |
| ERC2 | 897 | 1878270 | C | 0.27 | 0.0141 | 0.44 |
| ERC2 | 899 | 9881216 | T | 0.27 | 0.0167 | 0.45 |
| ERC2 | 900 | 7633140 | A | 0.29 | 0.0180 | 0.46 |
| ERC2 | 901 | 7627759 | C | 0.31 | 0.0316 | 0.50 |
| IL17RD | 903 | 2035654 | C | 0.08 | 0.0175 | 0.31 |
| IL17RD | 905 | 747089 | T | 0.27 | 0.0109 | 2.64 |
| FHIT | 914 | 2253211 | C | 0.68 | 0.0007 | 2.94 |
| PTPRG | 931 | 7427981 | A | 0.47 | 0.0044 | 2.52 |
| PTPRG | 934 | 1713531 | C | 0.40 | 0.0493 | 0.54 |
| CADPS | 943 | 9843019 | T | 0.10 | 0.0281 | 0.36 |
| CADPS | 949 | 11925708 | A | 0.28 | 0.0409 | 0.50 |
| MAGI1 | 972 | 4234687 | A | 0.15 | 0.0387 | 0.43 |
| MAGI1 | 973 | 17073075 | T | 0.19 | 0.0279 | 0.45 |
| EPHA6 | 1006 | 6438925 | G | 0.03 | 0.0303 | 0.22 |
| CPNE4 | 1059 | 2178385 | A | 0.44 | 0.0422 | 1.91 |
| CPNE4 | 1060 | 2178383 | G | 0.26 | 0.0402 | 0.50 |
| CLSTN2 | 1074 | 7632885 | G | 0.31 | 0.0048 | 0.40 |
| CLSTN2 | 1075 | 4683499 | G | 0.42 | 0.0149 | 0.47 |
| CLSTN2 | 1076 | 6439927 | C | 0.26 | 0.0325 | 0.49 |
| HTR3D | 1128 | 939334 | G | 0.18 | 0.0247 | 0.43 |
| LEPREL1 | 1133 | 9835230 | A | 0.31 | 0.0301 | 2.17 |
| PCDH7 | 1199 | 10517215 | A | 0.05 | 0.0260 | 0.26 |
| PCDH7 | 1202 | 2310225 | A | 0.16 | 0.0500 | 0.47 |
| LIMCH1 | 1223 | 6821712 | C | 0.40 | 0.0224 | 2.11 |
| LIMCH1 | 1224 | 11734372 | T | 0.34 | 0.0352 | 0.51 |
| LPHN3 | 1231 | 995447 | C | 0.02 | 0.0271 | 0.14 |
| PDLIM5 | 1296 | 2452601 | G | 0.29 | 0.0296 | 0.49 |
| PDLIM5 | 1299 | 7690296 | G | 0.29 | 0.0376 | 0.51 |
| PDLIM5 | 1301 | 951613 | A | 0.29 | 0.0390 | 0.51 |
| NDST3 | 1371 | 6534070 | A | 0.08 | 0.0072 | 0.27 |
| NDST3 | 1374 | 6820851 | T | 0.23 | 0.0346 | 0.48 |
| NDST3 | 1376 | 6534084 | C | 0.23 | 0.0437 | 0.49 |
| MAML3 | 1397 | 17051001 | T | 0.47 | 0.0307 | 1.98 |
| POU4F2 | 1427 | 1104532 | A | 0.05 | 0.0414 | 0.29 |
| FSTL5 | 1454 | 4352428 | G | 0.42 | 0.0358 | 1.98 |
| FSTLS | 1468 | 7699959 | A | 0.40 | 0.0487 | 0.54 |
| FSTL5 | 1471 | 1994770 | A | 0.60 | 0.0060 | 2.35 |
| TLL1 | 1477 | 9637646 | T | 0.24 | 0.0181 | 0.45 |
| PALLD | 1491 | 2319901 | G | 0.58 | 0.0139 | - 2.17 |
| ODZ3 | 1514 | 2675532 | C | 0.23 | 0.0242 | 0.46 |
| MYO10 | 1569 | 388887 | T | 0.53 | 0.0255 | 2.02 |
| MYO10 | 1570 | 11750539 | G | 0.24 | 0.0477 | 0.51 |
| MYO10 | 1572 | 890910 | A | 0.22 | 0.0083 | 0.39 |
| CDH10 | 1575 | 3822429 | T | 0.34 | 0.0166 | 0.47 |
| CIQTNF3 | 1580 | 6898170 | G | 0.30 | 0.0096 | 0.43 |
| EGFLAM | 1591 | 1428263 | C | 0.14 | 0.0476 | 0.43 |
| EGFLAM | 1595 | 16903966 | C | 0.22 | 0.0410 | 2.33 |
| ELOVL7 | 1637 | 4700394 | T | 0.68 | 0.0207 | 2.10 |
| VCAN | 1669 | 309559 | T | 0.56 | 0.0189 | 2.07 |
| MEF2C | 1674 | 652660 | G | 0.13 | 0.0427 | 0.43 |
| GPR98 | 1688 | 2366926 | G | 0.19 | 0.0168 | 0.42 |
| GPR98 | 1689 | 1878878 | A | 0.44 | 0.0321 | 1.98 |
| GPR98 | 1691 | 2438359 | T | 0.29 | 0.0024 | 0.37 |
| SEMA6A | 1735 | 254231 | G | 0.42 | 0.0328 | 0.52 |
| SNX24 | 1761 | 10066638 | G | 0.63 | 0.0301 | 1.97 |
| TRPC7 | 1775 | 3777145 | T | 0.02 | 0.0063 | 0.10 |
| TRPC7 | 1776 | 11748766 | C | 0.35 | 0.0082 | 2.48 |
| TRPC7 | 1778 | 950714 | G | 0.02 | 0.0079 | 0.10 |
| TRPC7 | 1779 | 2649691 | A | 0.05 | 0.0219 | 0.25 |
| ODZ2 | 1792 | 1529681 | A | 0.40 | 0.0165 | 2.23 |
| ODZ2 | 1795 | 6555784 | T | 0.13 | 0.0153 | 0.37 |
| ODZ2 | 1797 | 7708354 | G | 0.42 | 0.0103 | 2.31 |
| RNF144B | 1841 | 494248 | C | 0.26 | 0.0326 | 2.27 |
| LRFN2 | 1881 | 846505 | T | 0.31 | 0.0266 | 0.49 |
| SLC22A16 | 1929 | 723685 | C | 0.11 | 0.0431 | 3.23 |
| SLC22A16 | 1930 | 7740547 | C | 0.30 | 0.0487 | 2.03 |
| STX11 | 1995 | 6935462 | A | 0.00 | 0.0460 | 0.00 |
| PARK2 | 2019 | 992037 | T | 0.18 | 0.0316 | 0.45 |
| PARK2 | 2033 | 4708953 | C | 0.68 | 0.0262 | 2.04 |
| PARK2 | 2038 | 10945823 | A | 0.32 | 0.0334 | 2.10 |
| PARK2 | 2039 | 10945825 | A | 0.32 | 0.0404 | 2.04 |
| PARK2 | 2041 | 2105643 | C | 0.34 | 0.0435 | 1.99 |
| PDE10A | 2062 | 7752880 | G | 0.13 | 0.0226 | 0.39 |
| SDK1 | 2081 | 4723505 | T | 0.52 | 0.0118 | 2.21 |
| SDK1 | 2082 | 1044701 | A | 0.24 | 0.0013 | 0.34 |
| NXPH1 | 2084 | 6970999 | G | 0.13 | 0.0088 | 0.34 |
| NXPH1 | 2085 | 2189464 | T | 0.11 | 0.0093 | 0.33 |
| SCIN | 2093 | 1034856 | T | 0.55 | 0.0078 | 2.29 |
| SNX13 | 2102 | 4470902 | T | 0.48 | 0.0441 | 1.88 |
| NPY | 2114 | 16129 | T | 0.35 | 0.0370 | 0.52 |
| NPY | 2115 | 16124 | A | 0.37 | 0.0381 | 0.52 |
| PDE1C | 2153 | 3213709 | G | 0.08 | 0.0036 | 0.25 |
| EEPD1 | 2172 | 196618 | A | 0.24 | 0.0324 | 2.31 |
| ABCA13 | 2190 | 17132163 | T | 0.02 | 0.0158 | 0.12 |
| ABCA13 | 2191 | 12113721 | A | 0.02 | 0.0158 | 0.12 |
| GRB10 | 2215 | 2244347 | C | 0.21 | 0.0010 | 0.32 |
| GRB10 | 2217 | 2244351 | G | 0.33 | 0.0112 | 0.44 |
| GRB10 | 2218 | 2237477 | T | 0.24 | 0.0233 | 0.46 |
| HIP1 | 2264 | 11770686 | T | 0.55 | 0.0085 | 2.29 |
| MAGI2 | 2272 | 1860538 | G | 0.35 | 0.0368 | 0.52 |
| MAGI2 | 2273 | 2075013 | C | 0.35 | 0.0368 | 0.52 |
| MAGI2 | 2277 | 12531031 | T | 0.10 | 0.0199 | 0.34 |
| MAGI2 | 2296 | 10953851 | T | 0.06 | 0.0229 | 0.30 |
| MAGI2 | 2298 | 10269174 | G | 0.29 | 0.0105 | 0.43 |
| SEMA3E | 2346 | 1972459 | C | 0.42 | 0.0274 | 2.04 |
| PPP1R9A | 2372 | 7806202 | G | 0.53 | 0.0063 | 2.37 |
| PPP1R9A | 2373 | 1918482 | A | 0.29 | 0.0025 | 0.37 |
| PPP1R9A | 2374 | 6465460 | G | 0.31 | 0.0090 | 0.42 |
| PPP1R9A | 2376 | 854520 | T | 0.55 | 0.0262 | 1.99 |
| PPP1R9A | 2377 | 854532 | T | 0.52 | 0.0200 | 2.06 |
| PPP1R9A | 2378 | 854535 | C | 0.56 | 0.0335 | 1.93 |
| EXOC4 | 2458 | 10488165 | C | 0.26 | 0.0118 | 0.43 |
| EXOC4 | 2471 | 4731992 | A | 0.29 | 0.0471 | 2.05 |
| DGK1 | 2474 | 1731951 | T | 0.40 | 0.0409 | 0.53 |
| CNTNAP2 | 2508 | 1637864 | T | 0.27 | 0.0130 | 2.56 |
| CNTNAP2 | 2509 | 1637840 | T | 0.27 | 0.0130 | 2.56 |
| SGCZ | 2596 | 2898413 | A | 0.21 | 0.0420 | 2.35 |
| PSD3 | 2607 | 7837572 | T | 0.24 | 0.0408 | 2.23 |
| PSD3 | 2610 | 11781914 | G | 0.55 | 0.0012 | 2.77 |
| PSD3 | 2611 | 1492289 | C | 0.58 | 0.0032 | 2.50 |
| UNC5D | 2636 | 2589344 | T | 0.10 | 0.0130 | 0.32 |
| UNC5D | 2643 | 6468338 | T | 0.05 | 0.0383 | 0.28 |
| NKAIN3 | 2686 | 4297032 | T | 0.19 | 0.0268 | 2.64 |
| NKAIN3 | 2687 | 16928845 | A | 0.19 | 0.0268 | 2.64 |
| NKAIN3 | 2688 | 16928883 | C | 0.20 | 0.0378 | 2.46 |
| NKAIN3 | 2689 | 16928902 | C | 0.19 | 0.0429 | 2.40 |
| NKAIN3 | 2703 | 16929381 | T | 0.23 | 0.0269 | 2.46 |
| NKAIN3 | 2704 | 4737609 | A | 0.24 | 0.0324 | 2.31 |
| NKAIN3 | 2710 | 1383154 | A | 0.29 | 0.0231 | 2.29 |
| NKAIN3 | 2724 | 17279355 | G | 0.16 | 0.0458 | 2.59 |
| MMP16 | 2762 | 2248057 | G | 0.56 | 0.0113 | 2.20 |
| GRHL2 | 2775 | 13275653 | C | 0.27 | 0.0366 | 0.50 |
| GRHL2 | 2777 | 4734573 | C | 0.18 | 0.0271 | 0.44 |
| NCALD | 2780 | 2387620 | G | 0.18 | 0.0113 | 0.39 |
| ZFPM2 | 2790 | 7845734 | G | 0.06 | 0.0395 | 0.33 |
| ZFPM2 | 2802 | 6469014 | T | 0.08 | 0.0175 | 0.31 |
| ZFPM2 | 2805 | 11986654 | G | 0.02 | 0.0366 | 0.15 |
| FER1L6 | 2845 | 2069078 | C | 0.22 | 0.0013 | 4.33 |
| PER1L6 | 2846 | 13269044 | G | 0.21 | 0.0081 | 3.18 |
| DDEF1 | 2875 | 2791349 | C | 0.37 | 0.0362 | 2.01 |
| DDEF1 | 2883 | 4504679 | A | 0.45 | 0.0097 | 2.28 |
| ST3GAL1 | 2902 | 2978014 | A | 0.43 | 0.0241 | 2.10 |
| ST3GAL1 | 2903 | 2978015 | T | 0.44 | 0.0321 | 1.98 |
| ST3GAL1 | 2904 | 2945779 | A | 0.46 | 0.0406 | 1.96 |
| SMARCA2 | 2933 | 10116703 | A | 0.37 | 0.0301 | 0.51 |
| PTPRD | 2955 | 1036328 | C | 0.21 | 0.0397 | 0.48 |
| PIP5K1B | 2998 | 1541082 | A | 0.13 | 0.0359 | 3.11 |
| TMC1 | 3043 | 348478 | G | 0.15 | 0.0497 | 0.45 |
| PCSK5 | 3052 | 2789608 | G | 0.26 | 0.0068 | 0.40 |
| ZNF169 | 3097 | 12236219 | T | 0.11 | 0.0463 | 3.18 |
| FKTN | 3111 | 2518128 | A | 0.53 | 0.0264 | 1.99 |
| ABL1 | 3165 | 2855160 | T | 0.48 | 0.0441 | 1.88 |
| CUGBP2 | 3232 | 2938015 | A | 0.15 | 0.0031 | 0.31 |
| CACNB2 | 3242 | 10828879 | A | 0.32 | 0.0290 | 2.14 |
| SORBS1 | 3354 | 2296966 | A | 0.11 | 0.0096 | 5.30 |
| SORBS1 | 3361 | 10882584 | G | 0.12 | 0.0166 | 4.26 |
| VTI1A | 3391 | 3740143 | G | 0.23 | 0.0242 | 0.46 |
| VTI1A | 3393 | 10509963 | A | 0.23 | 0.0126 | 0.42 |
| VTI1A | 3394 | 10885352 | T | 0.23 | 0.0187 | 0.44 |
| GRK5 | 3429 | 10510055 | C | 0.32 | 0.0091 | 0.43 |
| MICAL2 | 3481 | 2270511 | G | 0.19 | 0.0429 | 2.40 |
| SPON1 | 3511 | 11023098 | T | 0.27 | 0.0460 | 0.51 |
| DLG2 | 3528 | 2060147 | G | 0.32 | 0.0109 | 2.47 |
| DLG2 | 3529 | 12275254 | C | 0.48 | 0.0060 | 2.40 |
| DLG2 | 3530 | 983590 | T | 0.29 | 0.0334 | 2.16 |
| DLG2 | 3531 | 11233708 | T | 0.45 | 0.0248 | 2.04 |
| TSPAN9 | 3592 | 4766079 | C | 0.55 | 0.0056 | 2.37 |
| STYK1 | 3613 | 6488316 | T | 0.37 | 0.0214 | 0.48 |
| STYKI | 3614 | 7350597 | A | 0.39 | 0.0498 | 0.54 |
| LOC729025 | 3614 | 1624759 | G | 0.43 | 0.0165 | 2.20 |
| LOC729025 | 3625 | 3915237 | C | 0.15 | 0.0020 | 0.30 |
| PIK3C2G | 3633 | 11043979 | C | 0.30 | 0.0447 | 0.52 |
| NAV3 | 3668 | 10859620 | G | 0.37 | 0.0484 | 0.54 |
| NAV3 | 3670 | 11107419 | T | 0.19 | 0.0056 | 0.37 |
| NAV3 | 3673 | 1677893 | A | 0.56 | 0.0113 | 2.20 |
| NAV3 | 3675 | 10859738 | A | 0.55 | 0.0249 | 2.01 |
| ATXN2 | 3685 | 616559 | G | 0.27 | 0.0481 | 2.08 |
| TRPC4 | 3730 | 1855253 | A | 0.60 | 0.0487 | 1.86 |
| NPAS3 | 3864 | 11851667 | C | 0.15 | 0.0183 | 0.39 |
| NPAS3 | 3875 | 7144144 | C | 0.58 | 0.0112 | 2.20 |
| NPAS3 | 3877 | 11156807 | T | 0.35 | 0.0465 | 0.53 |
| LRFN5 | 3892 | 10149327 | T | 0.48 | 0.0340 | 1.94 |
| LRFN5 | 3900 | 4143896 | A | 0.37 | 0.0138 | 0.46 |
| LRFN5 | 3902 | 10131436 | A | 0.21 | 0.0121 | 0.41 |
| LRFN5 | 3903 | 17112319 | A | 0.31 | 0.0205 | 0.47 |
| SAMD4A | 3911 | 1307289 | G | 0.12 | 0.0272 | 0.38 |
| SAMD4A | 3912 | 1212968 | C | 0.11 | 0.0412 | 0.41 |
| PPP2R5E | 3922 | 7140704 | G | 0.24 | 0.0477 | 2.16 |
| RPS6KA5 | 3977 | 7156252 | T | 0.40 | 0.0259 | 2.07 |
| CCDC88C | 3981 | 1970911 | A | 0.19 | 0.0045 | 3.72 |
| CCDC88C | 3988 | 10139287 | A | 0.22 | 0.0390 | 0.48 |
| GLDN | 4055 | 17647970 | T | 0.29 | 0.0390 | 0.51 |
| GLDN | 4058 | 2470172 | C | 0.37 | 0.0447 | 0.53 |
| RORA | 4081 | 12594561 | T | 0.53 | 0.0342 | 1.93 |
| RORA | 4083 | 12595483 | G | 0.60 | 0.0243 | 2.02 |
| TBC1D2B | 4093 | 11634607 | T | 0.50 | 0.0198 | 2.07 |
| ARNT2 | 4103 | 11072931 | T | 0.15 | 0.0390 | 0.44 |
| A2BP1 | 4118 | 1155959 | C | 0.29 | 0.0473 | 0.52 |
| CDH13 | 4192 | 9926620 | C | 0.26 | 0.0325 | 0.49 |
| CDH13 | 4199 | 10514574 | G | 0.06 | 0.0098 | 0.26 |
| MSI2 | 4257 | 8066677 | G | 0.18 | 0.0092 | 0.38 |
| SDK2 | 4263 | 7219778 | A | 0.31 | 0.0148 | 0.46 |
| HRNBP3 | 4272 | 7212305 | A | 0.19 | 0.0429 | 2.40 |
| PTPRM | 4289 | 524833 | C | 0.06 | 0.0395 | 0.33 |
| CHST9 | 4314 | 17703962 | G | 0.26 | 0.0376 | 2.20 |
| CCBE1 | 4340 | 2851865 | T | 0.50 | 0.0007 | 2.94 |
| CCBE1 | 4343 | 1560299 | T | 0.65 | 0.0041 | 2.47 |
| CCBE1 | 4344 | 4608425 | A | 0.58 | 0.0011 | 2.77 |
| CDH7 | 4359 | 7244664 | G | 0.11 | 0.0204 | 4.07 |
| CDH7 | 4360 | 8089669 | A | 0.48 | 0.0314 | 1.96 |
| CDH7 | 4369 | 2587427 | A | 0.44 | 0.0240 | 2.06 |
| DOK6 | 4375 | 1550597 | C | 0.62 | 0.0014 | 2.75 |
| DOK6 | 4376 | 1550598 | A | 0.61 | 0.0023 | 2.60 |
| DOK6 | 4377 | 12967871 | A | 0.55 | 0.0044 | 2.43 |
| MBP | 4383 | 470181 | G | 0.24 | 0.0317 | 0.48 |
| ZNF667 | 4386 | 7251105 | G | 0.48 | 0.0441 | 1.88 |
| ZNF544 | 4389 | 12462307 | T | 0.48 | 0.0106 | 2.24 |
| KIF16B | 4461 | 4814465 | T | 0.61 | 0.0238 | 2.03 |
| KIF16B | 4464 | 2144883 | T | 0.52 | 0.0264 | 1.99 |
| KIF16B | 4465 | 2328019 | C | 0.52 | 0.0264 | 1.99 |
| KIF16B | 4472 | 6034464 | A | 0.55 | 0.0339 | 1.93 |
| KIF168 | 4473 | 2876428 | C | 0.53 | 0.0264 | 1.99 |
| KCNB1 | 4517 | 2057077 | C | 0.48 | 0.0237 | 2.04 |
| SNAI1 | 4528 | 6020187 | G | 0.08 | 0.0175 | 0.31 |
| CDH4 | 4538 | 6142884 | A | 0.37 | 0.0447 | 0.53 |
| C21ORF37 | 4540 | 12483129 | C | 0.28 | 0.0066 | 0.41 |
| NCAM2 | 4550 | 973045 | A | 0.61 | 0.0059 | 2.36 |
| NCAM2 | 4551 | 986917 | A | 0.34 | 0.0352 | 0.51 |
| PDE9A | 4564 | 12626774 | C | 0.18 | 0.0071 | 0.37 |
| PDE9A | 4566 | 11702778 | A | 0.60 | 0.0023 | 2.59 |
| ASPHD2 | 4573 | 16982107 | T | 0.10 | 0.0239 | 4.54 |
| PACSIN2 | 4587 | 1071960 | C | 0.19 | 0.0356 | 0.46 |
| EFCAB6 | 4598 | 9614382 | G | 0.44 | 0.0003 | 3.30 |
| EFCAB6 | 4600 | 2374773 | A | 0.48 | 0.0038 | 2.50 |
| EFCAB6 | 4606 | 5764214 | C | 0.55 | 0.0339 | 1.93 |

| **TABLE 13: Alleles Influencing Discontinuation for Lack of Efficacy for Quetiapine** | | | | | | |
|---|---|---|---|---|---|---|
| **Gene** | **Seq ID** | **NCBI RS#** | **Allele** | **Freq. in Discontinuers** | **P** | **Odds Ratio** |
| RP1-21O18.1 | 16 | 2221926 | T | 0.19 | 0.04706 | 2.46 |
| EPHB2 | 21 | 7536195 | T | 0.19 | 0.04706 | 2.46 |
| LRP8 | 53 | 2297657 | A | 0.24 | 0.00457 | 0.36 |
| LRP8 | 54 | 12035926 | G | 0.27 | 0.02170 | 0.44 |
| LRP8 | 55 | 17108202 | G | 0.25 | 0.00468 | 0.36 |
| LRP8 | 56 | 11206139 | G | 0.73 | 0.00009 | 3.88 |
| PRKACB | 70 | 648856 | G | 0.13 | 0.02387 | 0.37 |
| PRKACB | 71 | 599686 | T | 0.13 | 0.02995 | 0.39 |
| PRKACB | 72 | 6701486 | A | 0.13 | 0.02995 | 0.39 |
| PRKACB | 74 | 2642186 | G | 0.13 | 0.03741 | 0.40 |
| PTGFRN | 90 | 17036573 | G | 0.08 | 0.00800 | 11.25 |
| PTGFRN | 91 | 12078461 | A | 0.08 | 0.00800 | 11.25 |
| ATF6 | 103 | 16856629 | C | 0.02 | 0.00704 | 0.10 |
| ATF6 | 105 | 10918104 | A | 0.02 | 0.01105 | 0.11 |
| ATF6 | 107 | 2880056 | A | 0.02 | 0.01281 | 0.11 |
| ATF6 | 108 | 12040523 | T | 0.02 | 0.03532 | 0.15 |
| ATF6 | 109 | 2499856 | T | 0.23 | 0.00880 | 3.10 |
| FAM78B | 113 | 10800181 | G | 0.40 | 0.04051 | 2.04 |
| SEC16B | 129 | 869286 | G | 0.29 | 0.02588 | 2.35 |
| SEC16B | 130 | 1934650 | C | 0.18 | 0.00622 | 3.98 |
| MR1 | 134 | 3845422 | A | 0.10 | 0.02526 | 0.33 |
| KCNK2 | 145 | 7547655 | A | 0.38 | 0.00990 | 2.48 |
| KCNK2 | 149 | 1112101 | G | 0.40 | 0.01024 | 2.48 |
| KCNK2 | 150 | 11120527 | T | 0.33 | 0.04745 | 2.06 |
| USH2A | 152 | 4655413 | T | 0.46 | 0.00426 | 2.62 |
| USH2A | 153 | 17025030 | C | 0.46 | 0.00696 | 2.47 |
| ESRRG | 176 | 1984137 | C | 0.58 | 0.02649 | 2.07 |
| ESRRG | 177 | 10218694 | A | 0.23 | 0.02617 | 0.44 |
| ESRRG | 178 | 12088947 | A | 0.23 | 0.02912 | 0.45 |
| RYR2 | 213 | 2685297 | G | 0.40 | 0.03807 | 2.03 |
| PLD5 | 268 | 12063701 | G | 0.15 | 0.03267 | 0.39 |
| DDEF2 | 282 | 10204214 | T | 0.21 | 0.00189 | 4.29 |
| DDEF2 | 283 | 10593 | G | 0.21 | 0.00383 | 3.79 |
| KLHL29 | 296 | 6544949 | G | 0.13 | 0.00720 | 4.98 |
| CRIM1 | 326 | 6757374 | T | 0.17 | 0.01455 | 3.35 |
| CRIM1 | 330 | 3770876 | C | 0.56 | 0.01400 | 2.24 |
| CRIM1 | 331 | 2160367 | C | 0.58 | 0.01239 | 2.27 |
| CRIM1 | 332 | 1017154 | T | 0.50 | 0.04593 | 1.93 |
| SLC8A1 | 351 | 12611602 | C | 0.58 | 0.00890 | 2.37 |
| PRKCE | 380 | 6732900 | T | 0.29 | 0.00898 | 0.41 |
| PRKCE | 381 | 4953270 | G | 0.31 | 0.02235 | 0.46 |
| AAK1 | 440 | 12471316 | G | 0.54 | 0.04224 | 1.94 |
| NAP5 | 483 | 12991216 | A | 0.50 | 0.00267 | 2.72 |
| NAP5 | 487 | 11695364 | T | 0.18 | 0.02907 | 0.42 |
| LRP1B | 523 | 1900936 | T | 0.25 | 0.04561 | 0.48 |
| LRP1B | 524 | 13382235 | G | 0.14 | 0.01726 | 3.94 |
| ARHGAP15 | 554 | 4334442 | C | 0.25 | 0.04842 | 2.25 |
| KIF5C | 563 | 4130558 | A | 0.19 | 0.04706 | 2.46 |
| FMNL2 | 578 | 17327752 | A | 0.15 | 0.04849 | 0.44 |
| FMNL2 | 581 | 10497108 | C | 0.00 | 0.00447 | 0.00 |
| FMNL2 | 582 | 16823807 | G | 0.00 | 0.00447 | 0.00 |
| FMNL2 | 583 | 16823809 | C | 0.00 | 0.00447 | 0.00 |
| FMNL2 | 584 | 10497111 | A | 0.02 | 0.01655 | 0.12 |
| SCN1A | 619 | 4667862 | A | 0.42 | 0.04132 | 2.00 |
| SCN1A | 620 | 6432860 | A | 0.42 | 0.04594 | 1.96 |
| SCN9A | 623 | 4303728 | A | 0.15 | 0.03987 | 2.86 |
| SCN7A | 630 | 3791251 | G | 0.15 | 0.01036 | 3.94 |
| PARD3B | 663 | 919936 | C | 0.27 | 0.03201 | 0.47 |
| TRIP12 | 724 | 6707272 | T | 0.42 | 0.03547 | 2.04 |
| SAG | 732 | 3828307 | T | 0.12 | 0.00222 | 8.74 |
| CNTN6 | 740 | 17271519 | G | 0.44 | 0.04244 | 1.97 |
| CNTN6 | 747 | 3772284 | A | 0.31 | 0.02855 | 0.47 |
| CNTN6 | 748 | 1479530 | G | 0.25 | 0.01613 | 0.42 |
| CNTN6 | 749 | 155900 | T | 0.65 | 0.00160 | 2.87 |
| CNTN6 | 750 | 155390 | G | 0.54 | 0.00161 | 2.84 |
| CNTN6 | 754 | 17038701 | G | 0.00 | 0.01986 | 0.00 |
| CNTN4 | 755 | 10510226 | G | 0.58 | 0.02551 | 2.08 |
| CNTN4 | 764 | 6785626 | A | 0.58 | 0.00711 | 2.42 |
| CNTN4 | 769 | 9823500 | C | 0.33 | 0.04745 | 2.06 |
| ITPR1 | 778 | 3804992 | A | 0.29 | 0.00983 | 0.40 |
| ITPR1 | 779 | 7637793 | T | 0.31 | 0.01401 | 0.43 |
| ITPR1 | 781 | 9880562 | T | 0.25 | 0.03955 | 0.48 |
| ITPR1 | 782 | 3792512 | C | 0.27 | 0.03678 | 2.27 |
| DAZL | 800 | 12233472 | G | 0.37 | 0.04928 | 2.00 |
| RARB | 806 | 1286738 | T | 0.06 | 0.00514 | 0.20 |
| RARB | 808 | 1656463 | A | 0.06 | 0.02341 | 0.26 |
| STAC | 824 | 17035045 | T | 0.31 | 0.03607 | 2.18 |
| STAC | 825 | 4678878 | C | 0.48 | 0.02767 | 2.07 |
| ULK4 | 828 | 4973875 | C | 0.54 | 0.03348 | 2.01 |
| ULK4 | 830 | 9311268 | A | 0.54 | 0.02627 | 2.07 |
| ULK4 | 839 | 7649806 | A | 0.52 | 0.02926 | 2.05 |
| ULK4 | 844 | 1495696 | C | 0.56 | 0.02230 | 2.12 |
| ULK4 | 846 | 9834824 | G | 0.56 | 0.02988 | 2.04 |
| PTPRG | 937 | 1388612 | A | 0.25 | 0.02461 | 2.50 |
| PTPRG | 939 | 875423 | T | 0.35 | 0.01178 | 0.43 |
| PTPRG | 940 | 9813294 | A | 0.58 | 0.01594 | 2.23 |
| PTPRG | 941 | 2242352 | G | 0.44 | 0.02509 | 2.12 |
| CADPS | 946 | 9830865 | A | 0.12 | 0.04366 | 0.39 |
| CADPS | 949 | 11925708 | A | 0.29 | 0.04142 | 0.49 |
| FAM19A1 | 982 | 6801913 | A | 0.42 | 0.00550 | 2.59 |
| GBE1 | 996 | 4411908 | G | 0.24 | 0.04031 | 0.47 |
| GBE1 | 998 | 3860595 | C | 0.25 | 0.04872 | 0.49 |
| CDGAP | 1021 | 10934491 | C | 0.42 | 0.00171 | 2.96 |
| CPNE4 | 1058 | 6806898 | T | 0.17 | 0.04662 | 2.60 |
| EPHB1 | 1071 | 4894251 | A | 0.65 | 0.00909 | 2.39 |
| EPHB1 | 1072 | 750543 | A | 0.65 | 0.01178 | 2.32 |
| SERPINI2 | 1087 | 4955673 | C | 0.37 | 0.02729 | 0.48 |
| SERPINI1 | 1092 | 1492022 | C | 0.28 | 0.02449 | 0.45 |
| TNIK | 1099 | 4955765 | G | 0.54 | 0.04224 | 1.94 |
| TNIK | 1103 | 9861012 | G | 0.46 | 0.03900 | 1.99 |
| PLD1 | 1106 | 1316415 | G | 0.31 | 0.02235 | 0.46 |
| NLGN1 | 1111 | 10936764 | G | 0.40 | 0.01864 | 2.25 |
| NLGN1 | 1119 | 4894645 | T | 0.24 | 0.01686 | 2.75 |
| NLGN1 | 1120 | 12636180 | T | 0.38 | 0.03547 | 2.08 |
| ILIRAP | 1143 | 2193877 | G | 0.10 | 0.01230 | 0.30 |
| LDB2 | 1169 | 1501142 | G | 0.12 | 0.04366 | 0.39 |
| LDB2 | 1171 | 7690190 | A | 0.27 | 0.03088 | 0.47 |
| SLIT2 | 1187 | 621806 | C | 0.21 | 0.01399 | 0.40 |
| SLIT2 | 1189 | 609535 | A | 0.21 | 0.02111 | 0.42 |
| SLIT2 | 1191 | 9992591 | C | 0.21 | 0.04085 | 0.46 |
| CCKAR | 1197 | 2040342 | C | 0.08 | 0.02797 | 0.31 |
| KIAA1239 | 1206 | 17575883 | A | 0.23 | 0.04319 | 2.34 |
| PDLIM5 | 1293 | 2510777 | C | 0.60 | 0.04428 | 1.96 |
| PAPSSI | 1307 | 2672487 | A | 0.25 | 0.03955 | 0.48 |
| PAPSSI | 1313 | 4956136 | C | 0.17 | 0.04662 | 2.60 |
| CAMK2D | 1364 | 6853484 | T | 0.21 | 0.00215 | 4.23 |
| CAMK2D | 1365 | 10488959 | C | 0.17 | 0.02586 | 2.95 |
| CAMK2D | 1367 | 7680434 | T | 0.17 | 0.02586 | 2.95 |
| MAML3 | 1387 | 2246759 | G | 0.60 | 0.00827 | 2.39 |
| MAML3 | 1390 | 6831959 | A | 0.38 | 0.04067 | 0.51 |
| INPP4B | 1416 | 17015544 | T | 0.31 | 0.00038 | 4.23 |
| INPP4B | 1417 | 3775654 | G | 0.21 | 0.00215 | 4.23 |
| POU4F2 | 1426 | 10010958 | T | 0.02 | 0.00412 | 0.09 |
| POU4F2 | 1430 | 1979903 | C | 0.02 | 0.04533 | 0.16 |
| FSTL5 | 1450 | 17041096 | C | 0.13 | 0.02590 | 0.38 |
| FSTLS | 1451 | 7657675 | A | 0.13 | 0.02942 | 0.38 |
| FSTL5 | 1467 | 13117227 | T | 0.25 | 0.00608 | 3.15 |
| FSTL5 | 1472 | 13101933 | T | 0.25 | 0.01610 | 2.69 |
| ODZ3 | 1516 | 6841520 | G | 0.10 | 0.00833 | 7.13 |
| ODZ3 | 1518 | 9312304 | A | 0.37 | 0.03183 | 2.14 |
| DNAH5 | 1549 | 6876673 | T | 0.56 | 0.03635 | 2.01 |
| DNAH5 | 1557 | 895321 | C | 0.44 | 0.02509 | 2.12 |
| DNAH5 | 1558 | 895317 | T | 0.44 | 0.02509 | 2.12 |
| DNAH5 | 1559 | 895316 | G | 0.44 | 0.02509 | 2.12 |
| DNAH5 | 1568 | 339428 | T | 0.37 | 0.02245 | 0.47 |
| EGFLAM | 1590 | 1469421 | A | 0.06 | 0.04830 | 0.30 |
| ITGA1 | 1613 | 1421927 | C | 0.46 | 0.02134 | 2.19 |
| ITGA1 | 1614 | 10940281 | A | 0.44 | 0.04406 | 1.99 |
| ITGA2 | 1618 | 1421937 | C | 0.38 | 0.04744 | 2.05 |
| ELOVL7 | 1637 | 4700394 | T | 0.42 | 0.04081 | 0.51 |
| VCAN | 1663 | 28296 | A | 0.42 | 0.02028 | 2.20 |
| FBXL17 | 1700 | 10463584 | G | 0.25 | 0.01012 | 2.91 |
| PJA2 | 1721 | 11738695 | C | 0.56 | 0.01318 | 2.26 |
| PJA2 | 1722 | 1045706 | A | 0.56 | 0.01628 | 2.21 |
| SEMA6A | 1733 | 17139820 | T | 0.29 | 0.04179 | 2.22 |
| LOC340069 | 1744 | 2561513 | A | 0.62 | 0.02586 | 2.09 |
| LOC340069 | 1746 | 328694 | A | 0.60 | 0.03713 | 1.99 |
| TRPC7 | 1773 | 3734125 | G | 0.42 | 0.01815 | 2.26 |
| ATXN1 | 1835 | 235147 | A | 0.63 | 0.00049 | 3.18 |
| SLC17A4 | 1852 | 1165157 | C | 0.15 | 0.03146 | 0.41 |
| TRDN | 1945 | 7452290 | C | 0.10 | 0.04044 | 0.36 |
| NKAIN2 | 1954 | 342623 | T | 0.06 | 0.03778 | 0.29 |
| NKAIN2 | 1956 | 4897531 | T | 0.06 | 0.03778 | 0.29 |
| NKAIN2 | 1970 | 781487 | C | 0.17 | 0.04280 | 2.64 |
| PDE7B | 1979 | 9385741 | A | 0.08 | 0.00800 | 11.25 |
| PDE7B | 1980 | 6570049 | T | 0.10 | 0.00833 | 7.13 |
| PDE7B | 1981 | 7772608 | G | 0.08 | 0.03174 | 5.50 |
| PDE7B | 1982 | 4382284 | T | 0.21 | 0.00112 | 0.29 |
| PDE7B | 1983 | 6570055 | C | 0.29 | 0.00693 | 0.39 |
| PDE7B | 1984 | 2327665 | G | 0.52 | 0.01748 | 2.18 |
| PDE7B | 1985 | 2018448 | G | 0.52 | 0.01748 | 2.18 |
| PDE7B | 1986 | 11154835 | A | 0.52 | 0.02118 | 2.14 |
| SYNE1 | 2004 | 718527 | A | 0.50 | 0.01101 | 2.32 |
| TFB1M | 2011 | 7775163 | A | 0.23 | 0.00614 | 0.37 |
| PARK2 | 2036 | 1555011 | G | 0.37 | 0.02007 | 2.28 |
| PARK2 | 2049 | 9456810 | C | 0.29 | 0.04545 | 0.50 |
| PDE10A | 2061 | 566759 | G | 0.06 | 0.02341 | 0.26 |
| FERD3L | 2107 | 6967799 | T | 0.14 | 0.01716 | 0.35 |
| FERD3L | 2109 | 6461422 | G | 0.13 | 0.01893 | 0.36 |
| CRHR2 | 2142 | 2190242 | C | 0.37 | 0.01229 | 2.44 |
| CRHR2 | 2143 | 4723003 | T | 0.21 | 0.00721 | 3.38 |
| PDE1C | 2162 | 2079373 | C | 0.04 | 0.01102 | 0.18 |
| CDC2L5 | 2182 | 3735135 | C | 0.00 | 0.02684 | 0.00 |
| CDC2L5 | 2183 | 12531761 | G | 0.00 | 0.02684 | 0.00 |
| CDC2L5 | 2185 | 12536726 | G | 0.00 | 0.02863 | 0.00 |
| ABCA13 | 2206 | 4072503 | G | 0.00 | 0.02684 | 0.00 |
| ABCA13 | 2207 | 1317162 | C | 0.00 | 0.02684 | 0.00 |
| ABCA13 | 2208 | 12113920 | T | 0.00 | 0.02684 | 0.00 |
| ABCA13 | 2210 | 1918602 | T | 0.00 | 0.03652 | 0.00 |
| WBSCR17 | 2228 | 10950221 | A | 0.25 | 0.01269 | 0.41 |
| WBSCR17 | 2234 | 17593300 | G | 0.35 | 0.01932 | 0.46 |
| WBSCR17 | 2236 | 12666361 | A | 0.35 | 0.02424 | 0.47 |
| PCLO | 2333 | 4129230 | T | 0.23 | 0.01020 | 0.39 |
| PCLO | 2334 | 10240976 | T | 0.33 | 0.01650 | 0.44 |
| SEMA3E | 2338 | 2722963 | G | 0.42 | 0.01335 | 2.35 |
| SEMA3E | 2339 | 2713174 | C | 0.50 | 0.04125 | 1.96 |
| SEMA3E | 2340 | 2249188 | G | 0.38 | 0.04895 | 0.52 |
| SEMA3E | 2342 | 17504676 | G | 0.31 | 0.01401 | 0.43 |
| PON1 | 2381 | 3917538 | T | 0.33 | 0.01804 | 2.39 |
| PON1 | 2382 | 2057681 | G | 0.40 | 0.03807 | 2.03 |
| DYNCII1 | 2389 | 2171555 | A | 0.23 | 0.03512 | 2.42 |
| ZNF3 | 2398 | 7778571 | G | 0.37 | 0.01729 | 2.32 |
| EMID2 | 2402 | 6947185 | C | 0.25 | 0.01964 | 0.42 |
| CUX1 | 2407 | 12537077 | T | 0.52 | 0.01748 | 2.18 |
| CUX1 | 2408 | 2694158 | T | 0.46 | 0.01228 | 2.33 |
| GRM8 | 2433 | 1419508 | A | 0.65 | 0.00083 | 3.05 |
| GRM8 | 2434 | 17627206 | T | 0.63 | 0.00136 | 2.90 |
| GRM8 | 2435 | 954661 | T | 0.63 | 0.00136 | 2.90 |
| GRM8 | 2436 | 2157752 | T | 0.19 | 0.00623 | 0.35 |
| GRM8 | 2437 | 10954112 | C | 0.29 | 0.04545 | 0.50 |
| GRM8 | 2438 | 2283079 | G | 0.62 | 0.00542 | 2.51 |
| GRM8 | 2443 | 2299500 | A | 0.25 | 0.00346 | 0.35 |
| GRM8 | 2445 | 13222700 | T | 0.65 | 0.00056 | 3.17 |
| GRM8 | 2446 | 11763603 | C | 0.65 | 0.00079 | 3.07 |
| GRM8 | 2449 | 6975541 | T | 0.62 | 0.00542 | 2.51 |
| CNTNAP2 | 2495 | 6464776 | C | 0.46 | 0.02746 | 2.09 |
| CNTNAP2 | 2511 | 2530309 | G | 0.27 | 0.00696 | 2.97 |
| CSMD1 | 2549 | 2938236 | T | 0.12 | 0.00734 | 5.78 |
| DLC1 | 2575 | 17788913 | T | 0.56 | 0.04725 | 1.92 |
| DLC1 | 2578 | 536147 | G | 0.06 | 0.00596 | 0.20 |
| SLC7A2 | 2599 | 2248010 | G | 0.48 | 0.02122 | 2.15 |
| UNCSD | 2636 | 2589344 | T | 0.31 | 0.03607 | 2.18 |
| TOX | 2683 | 4738731 | C | 0.60 | 0.00624 | 2.46 |
| TOX | 2684 | 12115074 | C | 0.60 | 0.00749 | 2.42 |
| NKAIN3 | 2690 | 4604422 | C | 0.63 | 0.00333 | 2.65 |
| NKAIN3 | 2692 | 1993125 | C | 0.27 | 0.00753 | 0.39 |
| NKAIN3 | 2693 | 7835901 | C | 0.60 | 0.01415 | 2.24 |
| NKAIN3 | 2696 | 827683 | C | 0.27 | 0.02869 | 2.37 |
| NKAIN3 | 2699 | 1480194 | C | 0.27 | 0.03678 | 2.27 |
| NKAIN3 | 2700 | 16929287 | G | 0.29 | 0.00878 | 2.79 |
| NKAIN3 | 2701 | 936616 | T | 0.29 | 0.01777 | 2.50 |
| NKAIN3 | 2708 | 16929568 | A | 0.17 | 0.04280 | 2.64 |
| NKAIN3 | 2716 | 4739023 | C | 0.38 | 0.04353 | 0.50 |
| NKAIN3 | 2722 | 11989852 | A | 0.14 | 0.02493 | 3.47 |
| NKAIN3 | 2724 | 17279355 | G | 0.13 | 0.02869 | 3.37 |
| KCNB2 | 2754 | 2981104 | C | 0.23 | 0.03512 | 2.42 |
| BAALC | 2785 | 1845430 | A | 0.27 | 0.02098 | 0.44 |
| BAALC | 2786 | 3956319 | T | 0.00 | 0.04448 | 0.00 |
| ADCY8 | 2894 | 4736722 | C | 0.58 | 0.01611 | 2.20 |
| ADCY8 | 2896 | 7461448 | A | 0.04 | 0.04502 | 0.24 |
| COL22A1 | 2912 | 2318340 | T | 0.35 | 0.01777 | 2.35 |
| COL22A1 | 2916 | 2318348 | G | 0.27 | 0.02869 | 2.37 |
| ZC3H3 | 2920 | 7464822 | A | 0.40 | 0.03075 | 2.13 |
| SMARCA2 | 2928 | 7872216 | T | 0.27 | 0.01266 | 2.72 |
| TEK | 2988 | 664461 | C | 0.27 | 0.01966 | 0.44 |
| PIP5K1B | 2997 | 4745375 | T | 0.08 | 0.02797 | 0.31 |
| TRPM3 | 3020 | 1932701 | C | 0.04 | 0.01421 | 0.19 |
| TRPM6 | 3046 | 7859201 | C | 0.52 | 0.00672 | 2.45 |
| TRPM6 | 3048 | 4300062 | C | 0.35 | 0.02477 | 2.24 |
| TRPM6 | 3049 | 7024831 | A | 0.35 | 0.02477 | 2.24 |
| PCSK5 | 3055 | 1029034 | G | 0.48 | 0.00086 | 3.14 |
| NFIL3 | 3091 | 6479339 | T | 0.23 | 0.00993 | 3.05 |
| SVEP1 | 3124 | 963143 | A | 0.26 | 0.01788 | 0.41 |
| TTLL11 | 3146 | 10120546 | C | 0.50 | 0.03239 | 2.02 |
| TTLL11 | 3147 | 12551572 | G | 0.46 | 0.03023 | 2.06 |
| ABCA2 | 3201 | 12337910 | G | 0.25 | 0.00053 | 4.63 |
| ARMC3 | 3250 | 10828389 | T | 0.38 | 0.04099 | 2.04 |
| ARHGAP21 | 3256 | 4415644 | T | 0.37 | 0.04307 | 2.03 |
| ARHGAP21 | 3257 | 7897303 | A | 0.37 | 0.04928 | 2.00 |
| ARHGAP21 | 3258 | 11593521 | C | 0.35 | 0.01247 | 2.47 |
| MYO3A | 3261 | 10828909 | A | 0.38 | 0.04067 | 0.51 |
| MYO3A | 3263 | 11014853 | T | 0.38 | 0.04067 | 0.51 |
| MYO3A | 3267 | 993397 | A | 0.46 | 0.02746 | 2.09 |
| MYO3A | 3280 | 1999240 | C | 0.50 | 0.04892 | 1.91 |
| JMJD1C | 3297 | 10995505 | A | 0.15 | 0.03454 | 0.41 |
| KCNMA1 | 3317 | 10762731 | G | 0.60 | 0.01762 | 2.19 |
| KCNMA1 | 3318 | 10762732 | T | 0.60 | 0.01762 | 2.19 |
| KCNMA1 | 3333 | 2673436 | A | 0.33 | 0.00587 | 0.40 |
| NRG3 | 3342 | 2644208 | T | 0.25 | 0.04263 | 0.47 |
| SORCS3 | 3372 | 10786808 | T | 0.04 | 0.00852 | 0.17 |
| ATRNL1 | 3406 | 11197045 | G | 0.15 | 0.02514 | 0.39 |
| ATRNL1 | 3407 | 11197046 | G | 0.15 | 0.02514 | 0.39 |
| ATRNL1 | 3408 | 2420071 | C | 0.17 | 0.03243 | 0.42 |
| ATE1 | 3446 | 10732824 | A | 0.00 | 0.00915 | 0.00 |
| ATE1 | 3447 | 3750835 | A | 0.00 | 0.01186 | 0.00 |
| ATE1 | 3451 | 11200260 | T | 0.04 | 0.02999 | 0.22 |
| ATE1 | 3452 | 7906462 | A | 0.04 | 0.03826 | 0.23 |
| STIM1 | 3462 | 10835406 | G | 0.50 | 0.03012 | 2.05 |
| STIM1 | 3463 | 10835407 | G | 0.50 | 0.04125 | 1.96 |
| STIM1 | 3464 | 10767767 | T | 0.50 | 0.04301 | 1.96 |
| MICAL2 | 3481 | 2270511 | G | 0.27 | 0.01113 | 2.76 |
| DLG2 | 3537 | 10898192 | G | 0.02 | 0.04533 | 0.16 |
| DLG2 | 3540 | 11233954 | A | 0.02 | 0.02001 | 0.13 |
| DLG2 | 3551 | 7101454 | C | 0.58 | 0.02649 | 2.07 |
| ELMOD1 | 3556 | 6588969 | A | 0.62 | 0.03256 | 2.03 |
| OPCML | 3575 | 7107264 | C | 0.21 | 0.04981 | 2.34 |
| OPCML | 3576 | 1941219 | G | 0.23 | 0.03512 | 2.42 |
| OPCML | 3578 | 6590699 | T | 0.30 | 0.02929 | 2.31 |
| OPCML | 3579 | 6590700 | C | 0.33 | 0.00553 | 2.82 |
| OPCML | 3580 | 6590701 | G | 0.37 | 0.01729 | 2.32 |
| OPCML | 3581 | 7935298 | A | 0.31 | 0.03607 | 2.18 |
| TMEM16B | 3596 | 9645764 | C | 0.10 | 0.00498 | 0.26 |
| TMEM16B | 3597 | 4930809 | T | 0.10 | 0.01395 | 0.30 |
| TMEM16B | 3599 | 11610348 | A | 0.10 | 0.00195 | 14.36 |
| TMEM16B | 3600 | 3782638 | G | 0.10 | 0.00195 | 14.36 |
| TMEM16B | 3601 | 1860961 | G | 0.10 | 0.00195 | 14.36 |
| TMEM16B | 3602 | 12578500 | A | 0.10 | 0.00833 | 7.13 |
| ITPR2 | 3635 | 1049376 | G | 0.13 | 0.01493 | 0.35 |
| ITPR2 | 3636 | 2570 | C | 0.21 | 0.03297 | 0.45 |
| CNOT2 | 3646 | 17107924 | T | 0.19 | 0.04706 | 2.46 |
| CNOT2 | 3651 | 17108018 | G | 0.19 | 0.04706 | 2.46 |
| LMO7 | 3740 | 9573627 | G | 0.46 | 0.04582 | 1.94 |
| LMO7 | 3743 | 9530467 | G | 0.42 | 0.04202 | 0.51 |
| LMO7 | 3749 | 11617789 | G | 0.21 | 0.03308 | 2.54 |
| GPC6 | 3805 | 4085921 | G | 0.23 | 0.03640 | 0.46 |
| GPC6 | 3808 | 4394948 | C | 0.25 | 0.01318 | 0.41 |
| GPC6 | 3811 | 885192 | G | 0.16 | 0.03428 | 0.41 |
| GPC6 | 3813 | 9524268 | T | 0.17 | 0.03524 | 0.43 |
| GPC6 | 3815 | 9524290 | G | 0.06 | 0.02341 | 0.26 |
| GPC6 | 3816 | 9524298 | A | 0.06 | 0.02512 | 0.26 |
| GPC6 | 3817 | 9516323 | T | 0.06 | 0.02740 | 0.27 |
| ITGBL1 | 3839 | 1335589 | T | 0.12 | 0.03133 | 0.37 |
| ITGBL1 | 3847 | 1436261 | C | 0.12 | 0.03498 | 0.38 |
| LRFN5 | 3904 | 1033877 | G | 0.19 | 0.04082 | 0.45 |
| NRXN3 | 3959 | 8022988 | T | 0.27 | 0.03678 | 2.27 |
| MAGEL2 | 4004 | 6576608 | A | 0.29 | 0.01477 | 0.43 |
| RYR3 | 4025 | 2077268 | T | 0.04 | 0.02999 | 0.22 |
| RYR3 | 4032 | 8036377 | A | 0.42 | 0.04594 | 1.96 |
| RYR3 | 4033 | 2043054 | G | 0.38 | 0.04062 | 2.03 |
| C15ORF41 | 4039 | 6495868 | G | 0.27 | 0.01966 | 0.44 |
| TBC1D2B | 4088 | 1108478 | C | 0.10 | 0.02526 | 0.33 |
| TBC1D2B | 4092 | 8030999 | G | 0.23 | 0.03895 | 2.38 |
| A2BP1 | 4122 | 8055711 | T | 0.17 | 0.01639 | 0.38 |
| WWOX | 4153 | 11641213 | G | 0.63 | 0.00580 | 2.51 |
| WWOX | 4154 | 16947095 | A | 0.62 | 0.00946 | 2.36 |
| CDH13 | 4200 | 13337699 | A | 0.35 | 0.03829 | 0.50 |
| USP10 | 4210 | 744839 | T | 0.54 | 0.02481 | 2.09 |
| DNAH9 | 4221 | 12601236 | A | 0.21 | 0.00624 | 0.36 |
| RAB11FIP4 | 4225 | 315431 | T | 0.17 | 0.01292 | 0.37 |
| CA10 | 4251 | 12945099 | C | 0.58 | 0.01047 | 2.36 |
| CACNG4 | 4261 | 9303513 | G | 0.52 | 0.01329 | 2.26 |
| DNAH17 | 4268 | 9302876 | A | 0.40 | 0.00851 | 2.54 |
| DLGAP1 | 4277 | 3915772 | G | 0.08 | 0.04077 | 0.33 |
| KIAA0802 | 4304 | 10502383 | A | 0.15 | 0.02191 | 0.38 |
| KIAA0802 | 4305 | 17407982 | C | 0.15 | 0.02191 | 0.38 |
| KIAA0802 | 4306 | 17408213 | T | 0.16 | 0.03428 | 0.41 |
| CHST9 | 4315 | 9949654 | C | 0.13 | 0.03741 | 0.40 |
| CHST9 | 4316 | 1426879 | C | 0.21 | 0.02111 | 0.42 |
| CHST9 | 4317 | 4800797 | G | 0.21 | 0.02244 | 0.42 |
| RNF24 | 4394 | 2143235 | A | 0.12 | 0.03993 | 3.42 |
| PRNT | 4401 | 7269641 | C | 0.13 | 0.03741 | 0.40 |
| PRNT | 4402 | 6107527 | G | 0.48 | 0.01356 | 2.30 |
| PRNT | 4403 | 6037950 | T | 0.13 | 0.03741 | 0.40 |
| PLCB1 | 4412 | 6055513 | G | 0.25 | 0.00057 | 0.30 |
| PLCB1 | 4432 | 6118375 | C | 0.44 | 0.00111 | 3.06 |
| PLCB1 | 4433 | 4295085 | A | 0.38 | 0.02536 | 0.48 |
| PLCB4 | 4438 | 6056653 | G | 0.21 | 0.03653 | 2.50 |
| JAG1 | 4439 | 3748477 | T | 0.00 | 0.03317 | 0.00 |
| PTPRT | 4492 | 6029924 | A | 0.40 | 0.03807 | 2.03 |
| PTPRT | 4493 | 6016664 | C | 0.42 | 0.04594 | 1.96 |
| PTPRT | 4495 | 4812571 | C | 0.46 | 0.04972 | 1.92 |
| PTPRT | 4496 | 7263976 | C | 0.50 | 0.00816 | 2.40 |
| PTPRT | 4498 | 746413 | C | 0.35 | 0.01178 | 0.43 |
| PTPRT | 4499 | 1126101 | G | 0.60 | 0.00181 | 2.80 |
| PTPRT | 4501 | 6016688 | T | 0.13 | 0.00325 | 0.29 |
| SLC37A1 | 4560 | 401396 | C | 0.23 | 0.04041 | 0.47 |
| RIBC2 | 4616 | 5765398 | A | 0.28 | 0.02449 | 0.45 |
| RIBC2 | 4617 | 2092101 | G | 0.29 | 0.04017 | 0.49 |

| **TABLE 14: Alleles Influencing Discontinuation for Lack of Efficacy for Perphenazine** | | | | | | |
|---|---|---|---|---|---|---|
| **Gene** | **Seq ID** | **NCBI RS#** | **Allele** | **Freq. in Discontinuers** | **P** | **Odds Ratio** |
| EPHB2 | 20 | 10799762 | C | 0.02 | 0.0049860 | 0.09 |
| SCP2 | 41 | 7546714 | T | 0.21 | 0.0474200 | 2.52 |
| LRP8 | 52 | 2297660 | A | 0.57 | 0.0165700 | 2.28 |
| LRP8 | 54 | 12035926 | G | 0.53 | 0.0389800 | 2.00 |
| PRKACB | 73 | 10493750 | C | 0.36 | 0.0414600 | 0.50 |
| PRKACB | 74 | 2642186 | G | 0.44 | 0.0210700 | 2.27 |
| PRKACB | 75 | 11163905 | G | 0.63 | 0.0085300 | 2.48 |
| PRKACB | 76 | 2134648 | C | 0.59 | 0.0217800 | 2.16 |
| PRKACB | 77 | 2134646 | T | 0.59 | 0.0302300 | 2.07 |
| OLFML2B | 110 | 7537366 | T | 0.09 | 0.0464400 | 0.36 |
| SLC35F3 | 191 | 10495346 | T | 0.02 | 0.0227000 | 0.13 |
| FMN2 | 240 | 4659569 | T | 0.36 | 0.0098790 | 2.64 |
| FMN2 | 241 | 10754696 | T | 0.36 | 0.0098790 | 2.64 |
| FMN2 | 242 | 10754697 | A | 0.32 | 0.0414500 | 2.20 |
| PLD5 | 269 | 12061610 | C | 0.29 | 0.0422200 | 0.49 |
| C2ORF46 | 280 | 2914424 | T | 0.18 | 0.0276700 | 3.19 |
| DDEF2 | 283 | 10593 | G | 0.26 | 0.0207800 | 2.70 |
| SLC8A1 | 346 | 417591 | G | 0.09 | 0.0333800 | 0.34 |
| SLC8A1 | 349 | 385561 | G | 0.11 | 0.0391000 | 0.37 |
| C2ORF34 | 368 | 11888605 | A | 0.26 | 0.0066990 | 0.38 |
| C2ORF34 | 369 | 3862996 | C | 0.26 | 0.0066990 | 0.38 |
| C2ORF34 | 370 | 10445928 | C | 0.19 | 0.0077750 | 0.36 |
| C2ORF34 | 371 | 734016 | A | 0.19 | 0.0077750 | 0.36 |
| C2ORF34 | 372 | 6544767 | A | 0.19 | 0.0077750 | 0.36 |
| C2ORF34 | 374 | 3738980 | C | 0.05 | 0.0297800 | 0.26 |
| PRKCE | 379 | 10865208 | C | 0.52 | 0.0087580 | 2.49 |
| PSME4 | 400 | 805438 | A | 0.59 | 0.0154300 | 2.26 |
| PSME4 | 401 | 805391 | T | 0.43 | 0.0453100 | 0.50 |
| CCDC85A | 420 | 1159916 | G | 0.36 | 0.0364900 | 2.16 |
| CCDC85A | 428 | 1030091 | C | 0.00 | 0.0353000 | 0.00 |
| CTNNA2 | 466 | 1930 | T | 0.59 | 0.0217800 | 2.16 |
| NAP5 | 477 | 1437909 | G | 0.28 | 0.0244500 | 0.45 |
| NAP5 | 480 | 2217747 | T | 0.57 | 0.0266600 | 2.11 |
| RAB3GAP1 | 488 | 10174944 | T | 0.23 | 0.0386700 | 2.54 |
| NXPH2 | 500 | 12470031 | G | 0.38 | 0.0209800 | 2.32 |
| KYNU | 532 | 1371516 | C | 0.28 | 0.0307700 | 2.43 |
| KYNU | 533 | 6430001 | A | 0.38 | 0.0313900 | 2.18 |
| PKP4 | 598 | 3771645 | C | 0.17 | 0.0244400 | 0.40 |
| PKP4 | 601 | 2528593 | A | 0.15 | 0.0300900 | 0.39 |
| PARD3B | 665 | 849221 | T | 0.13 | 0.0245200 | 4.43 |
| COL4A3 | 698 | 6741679 | A | 0.41 | 0.0477000 | 2.01 |
| CNTN4 | 766 | 12496950 | G | 0.48 | 0.0048920 | 2.65 |
| ATP2B2 | 792 | 6763274 | C | 0.33 | 0.0054240 | 3.04 |
| CACNA2D3 | 876 | 10865992 | G | 0.19 | 0.0402600 | 0.45 |
| ERC2 | 878 | 815453 | G | 0.31 | 0.0239400 | 2.43 |
| ERC2 | 880 | 11130482 | C | 0.29 | 0.0270100 | 2.49 |
| IL17RD | 903 | 2035654 | C | 0.19 | 0.0327500 | 2.91 |
| STX19 | 1000 | 9810986 | T | 0.07 | 0.0098710 | 0.36 |
| PLCXD2 | 1017 | 1913495 | T | 0.07 | 0.0069750 | 0.24 |
| STXBP5L | 1026 | 471031 | G | 0.59 | 0.0207900 | 2.18 |
| STXBP5L | 1027 | 657578 | **G** | 0.59 | 0.0302300 | 2.07 |
| STXBP5L | 1028 | 603635 | T | 0.36 | 0.0198100 | 2.41 |
| STXBP5L | 1030 | 1191296 | A | 0.34 | 0.0221100 | 2.38 |
| STXBP5L | 1032 | 1108170 | T | 0.57 | 0.0462000 | 1.99 |
| STXBP5L | 1034 | 6787048 | T | 0.33 | 0.0324900 | 2.26 |
| STXBP5L | 1035 | 12186062 | C | 0.41 | 0.0329200 | 2.15 |
| RAB6B | 1062 | 1880663 | C | 0.09 | 0.0464400 | 0.36 |
| HTR3D | 1128 | 939334 | G | 0.41 | 0.0038100 | 2.86 |
| HTR3D | 1129 | 6779545 | A | 0.54 | 0.0371400 | 2.04 |
| HTR3D | 1130 | 1000952 | C | 0.24 | 0.0101100 | 0.39 |
| SLIT2 | 1188 | 10516357 | A | 0.41 | 0.0079380 | 2.61 |
| SLIT2 | 1190 | 2168802 | T | 0.31 | 0.0239400 | 2.43 |
| PCDH7 | 1202 | 2310225 | A | 0.38 | 0.0310500 | 2.22 |
| SHROOM3 | 1257 | 344141 | C | 0.28 | 0.0333300 | 0.47 |
| SCD5 | 1273 | 17354301 | T | 0.14 | 0.0397400 | 0.41 |
| CAMK2D | 1364 | 6853484 | T | 0.05 | 0.0244300 | 0.39 |
| CAMK2D | 1365 | 10488959 | C | 0.05 | 0.0244300 | 2.85 |
| CAMK2D | 1366 | 7438925 | G | 0.21 | 0.0474200 | 2.52 |
| MAML3 | 1391 | 11734419 | G | 0.16 | 0.0404600 | 0.42 |
| IL15 | 1403 | 6841939 | A | 0.55 | 0.0078430 | 2.48 |
| IL15 | 1404 | 2139383 | T | 0.34 | 0.0060710 | 0.39 |
| IL15 | 1406 | 4254850 | C | 0.31 | 0.0023750 | 0.35 |
| IL15 | 1407 | 1493015 | A | 0.41 | 0.0477000 | 2.01 |
| IL15 | 1408 | 6821171 | G | 0.19 | 0.0402600 | 0.45 |
| INPP4B | 1410 | 2667101 | G | 0.39 | 0.0458500 | 2.05 |
| INPP4B | 1412 | 336378 | T | 0.05 | 0.0234000 | 2.49 |
| INPP4B | 1413 | 1353603 | A | 0.05 | 0.0275500 | 2.05 |
| INPP4B | 1419 | 7697702 | C | 0.07 | 0.0419200 | 7.31 |
| DCLK2 | 1443 | 4696645 | A | 0.05 | 0.0419800 | 0.28 |
| CTSO | 1446 | 6536124 | A | 0.38 | 0.0381700 | 2.12 |
| FSTL5 | 1450 | 17041096 | C | 0.05 | 0.0102200 | 0.22 |
| FSTL5 | 1451 | 7657675 | A | 0.05 | 0.0162900 | 0.23 |
| FSTL5 | 1455 | 6825091 | A | 0.38 | 0.0106300 | 2.58 |
| FSTL5 | 1456 | 7442468 | C | 0.40 | 0.0145300 | 2.43 |
| FSTL5 | 1463 | 1023957 | C | 0.32 | 0.0171800 | 2.56 |
| FSTL5 | 1464 | 17459954 | T | 0.33 | 0.0115500 | 2.71 |
| FSTL5 | 1465 | 6814301 | T | 0.36 | 0.0243900 | 2.30 |
| TLL1 | 1476 | 2322443 | T | 0.34 | 0.0463700 | 0.50 |
| PALLD | 1487 | 17054309 | A | 0.12 | 0.0453800 | 0.40 |
| SEMA5A | 1541 | 11134354 | A | 0.29 | 0.0088690 | 3.12 |
| SEMA5A | 1542 | 4702625 | G | 0.26 | 0.0346000 | 2.44 |
| CDH10 | 1574 | 7731953 | T | 0.04 | 0.0229400 | 0.20 |
| PDE4D | 1623 | 2936193 | G | 0.07 | 0.0443900 | 0.32 |
| PDE4D | 1635 | 295963 | C | 0.07 | 0.0370100 | 0.31 |
| TNPO1 | 1639 | 6452685 | T | 0.05 | 0.0022400 | 0.17 |
| TNPO1 | 1640 | 6884453 | G | 0.07 | 0.0061530 | 0.23 |
| TNPO1 | 1642 | 12187110 | T | 0.09 | 0.0117600 | 0.28 |
| TNPO1 | 1644 | 2548331 | A | 0.59 | 0.0217800 | 2.16 |
| FCHO2 | 1645 | 478575 | C | 0.31 | 0.0051750 | 0.37 |
| FCHO2 | 1647 | 6895786 | G | 0.17 | 0.0008214 | 0.27 |
| FCHO2 | 1648 | 7727222 | C | 0.17 | 0.0017100 | 0.29 |
| FCHO2 | 1649 | 7709974 | A | 0.17 | 0.0017100 | 0.29 |
| VCAN | 1665 | 11741157 | T | 0.57 | 0.0006516 | 3.21 |
| VCAN | 1666 | 11749904 | C | 0.55 | 0.0011520 | 3.05 |
| VCAN | 1667 | 4558964 | G | 0.12 | 0.0277900 | 0.37 |
| GPR98 | 1681 | 688956 | C | 0.59 | 0.0032750 | 2.71 |
| GPR98 | 1682 | 168743 | G | 0.19 | 0.0330500 | 0.43 |
| GPR98 | 1683 | 16868901 | C | 0.09 | 0.0408800 | 0.35 |
| GPR98 | 1689 | 1878878 | A | 0.50 | 0.0290000 | 2.10 |
| FBXL17 | 1700 | 10463584 | G | 0.26 | 0.0062720 | 3.37 |
| ODZ2 | 1791 | 11952245 | G | 0.40 | 0.0391900 | 2.09 |
| ODZ2 | 1792 | 1529681 | A | 0.23 | 0.0367500 | 0.45 |
| ODZ2 | 1796 | 1560654 | T | 0.14 | 0.0149000 | 0.35 |
| GABRP | 1804 | 732157 | C | 0.60 | 0.0459700 | 1.96 |
| SLC22A23 | 1809 | 11967828 | T | 0.26 | 0.0117900 | 3.00 |
| SLC22A23 | 1810 | 17136440 | T | 0.26 | 0.0207800 | 2.70 |
| SLC22A23 | 1811 | 7747259 | T | 0.26 | 0.0243200 | 2.63 |
| JARID2 | 1826 | 9396578 | T | 0.14 | 0.0172900 | 0.36 |
| RIMS1 | 1902 | 1010326 | A | 0.36 | 0.0098790 | 2.64 |
| SLC22A16 | 1926 | 9400390 | T | 0.21 | 0.0397600 | 0.45 |
| TRDN | 1951 | 7453704 | C | 0.40 | 0.0145300 | 2.43 |
| TRDN | 1952 | 2169092 | T | 0.53 | 0.0199600 | 2.19 |
| PDE7B | 1987 | 6938424 | T | 0.10 | 0.0072410 | 10.96 |
| PDE7B | 1988 | 17065190 | A | 0.07 | 0.0470000 | 7.04 |
| PARK2 | 2024 | 9365311 | C | 0.40 | 0.0459700 | 0.51 |
| PDE10A | 2064 | 7761766 | G | 0.41 | 0.0152800 | 2.37 |
| SDK1 | 2074 | 663466 | A | 0.09 | 0.0464400 | 0.36 |
| SKAP2 | 2118 | 774250 | T | 0.09 | 0.0295100 | 0.33 |
| CREBS | 2122 | 1544469 | G | 0.16 | 0.0296000 | 0.40 |
| PDE1C | 2154 | 2191876 | T | 0.07 | 0.0113200 | 0.25 |
| PDE1C | 2157 | 17336016 | G | 0.19 | 0.0077750 | 0.36 |
| BMPER | 2167 | 4140982 | A | 0.40 | 0.0268900 | 2.21 |
| WBSCR17 | 2240 | 1859526 | T | 0.33 | 0.0130000 | 2.63 |
| WBSCR17 | 2244 | 12699040 | A | 0.21 | 0.0394900 | 0.45 |
| LIMK1 | 2249 | 810532 | G | 0.05 | 0.0249400 | 0.25 |
| MAGI2 | 2277 | 12531031 | T | 0.21 | 0.0474200 | 2.52 |
| MAGI2 | 2279 | 848913 | C | 0.04 | 0.0142100 | 0.18 |
| MAGI2 | 2286 | 10255710 | C | 0.21 | 0.0243700 | 2.93 |
| MAGI2 | 2300 | 10248584 | G | 0.05 | 0.0249400 | 0.25 |
| MAGI2 | 2301 | 4730829 | C | 0.31 | 0.0147600 | 2.64 |
| PCLO | 2330 | 976714 | T | 0.47 | 0.0202300 | 2.23 |
| SEMA3E | 2342 | 17504676 | G | 0.48 | 0.0278100 | 2.13 |
| ABCB4 | 2349 | 2097937 | G | 0.29 | 0.0428000 | 2.29 |
| PON1 | 2383 | 2299257 | C | 0.26 | 0.0332100 | 0.46 |
| DYNC1I1 | 2390 | 4357216 | C | 0.19 | 0.0289000 | 2.98 |
| GRM8 | 2448 | 17684592 | A | 0.36 | 0.0439300 | 2.10 |
| EXOC4 | 2454 | 1345938 | C | 0.17 | 0.0298100 | 0.42 |
| EXOC4 | 2456 | 7783217 | C | 0.17 | 0.0216500 | 0.40 |
| EXOC4 | 2457 | 6961517 | T | 0.16 | 0.0153600 | 0.37 |
| EXOC4 | 2458 | 10488165 | C | 0.48 | 0.0064380 | 2.58 |
| EXOC4 | 2460 | 972420 | C | 0.69 | 0.0108100 | 2.42 |
| DGK1 | 2476 | 12535157 | A | 0.18 | 0.0019130 | 0.29 |
| DGK1 | 2477 | 4728414 | C | 0.24 | 0.0197800 | 0.43 |
| DGK1 | 2478 | 1528103 | C | 0.24 | 0.0197800 | 0.43 |
| DGK1 | 2479 | 10268638 | A | 0.22 | 0.0205800 | 0.42 |
| TBXAS1 | 2483 | 2270163 | G | 0.30 | 0.0340200 | 0.47 |
| CNTNAP2 | 2495 | 6464776 | C | 0.12 | 0.0453800 | 0.40 |
| PTPRN2 | 2522 | 4716883 | G | 0.03 | 0.0282100 | 0.21 |
| PTPRN2 | 2532 | 10265417 | T | 0.41 | 0.0413100 | 0.50 |
| PTPRN2 | 2535 | 2878355 | A | 0.16 | 0.0296000 | 0.40 |
| MCPH1 | 2563 | 3020213 | T | 0.50 | 0.0404600 | 2.00 |
| PSD3 | 2615 | 1506894 | C | 0.02 | 0.0289900 | 0.14 |
| GFRA2 | 2618 | 17427734 | A | 0.26 | 0.0095710 | 0.40 |
| PEBP4 | 2630 | 2457426 | C | 0.43 | 0.0498900 | 0.52 |
| ENTPD4 | 2631 | 9644075 | G | 0.24 | 0.0386800 | 2.46 |
| SLC25A37 | 2632 | 10503726 | C | 0.41 | 0.0000072 | 6.07 |
| SLC25A37 | 2633 | 7826247 | G | 0.43 | 0.0001645 | 4.09 |
| SLC25A37 | 2634 | 11778179 | T | 0.36 | 0.0000056 | 7.95 |
| SFRP1 | 2649 | 10106678 | C | 0.57 | 0.0351300 | 2.03 |
| SNTG1 | 2667 | 2623207 | C | 0.12 | 0.0319900 | 4.16 |
| NKAIN3 | 2715 | 10957255 | G | 0.41 | 0.0099140 | 2.52 |
| NKAIN3 | 2716 | 4739023 | C | 0.41 | 0.0279200 | 0.47 |
| NKAIN3 | 2719 | 4382471 | T | 0.38 | 0.0313900 | 2.18 |
| NKAIN3 | 2720 | 7835051 | G | 0.43 | 0.0266600 | 0.48 |
| NKAIN3 | 2721 | 10100456 | T | 0.38 | 0.0313900 | 2.18 |
| DEPDC2 | 2727 | 10097733 | T | 0.14 | 0.0373800 | 0.40 |
| CRISPLD1 | 2756 | 1455795 | T | 0.03 | 0.0406700 | 0.23 |
| CRISPLD1 | 2758 | 13248650 | G | 0.38 | 0.0065000 | 2.77 |
| GRHL2 | 2770 | 13263539 | G | 0.64 | 0.0112200 | 2.36 |
| GRHL2 | 2771 | 17397776 | T | 0.53 | 0.0183100 | 2.23 |
| GRHL2 | 2772 | 17397811 | A | 0.53 | 0.0199600 | 2.19 |
| NCALD | 2784 | 7813132 | C | 0.33 | 0.0077350 | 2.85 |
| ZFPM2 | 2797 | 7003445 | C | 0.59 | 0.0002598 | 3.61 |
| ZFPM2 | 2799 | 3779776 | G | 0.22 | 0.0250600 | 2.79 |
| ZFPM2 | 2800 | 1442326 | G | 0.45 | 0.0002781 | 3.78 |
| ZFPM2 | 2801 | 12546300 | G | 0.47 | 0.0025700 | 2.96 |
| ZFPM2 | 2807 | 2028553 | A | 0.31 | 0.0087100 | 2.87 |
| SAMD12 | 2823 | 11774993 | G | 0.38 | 0.0275800 | 0.48 |
| SAMD12 | 2824 | 2514588 | T | 0.41 | 0.0229200 | 2.24 |
| SAMD12 | 2825 | 1607924 | G | 0.41 | 0.0229200 | 2.24 |
| SAMD12 | 2826 | 10505340 | A | 0.17 | 0.0146800 | 3.79 |
| FER1L6 | 2836 | 11784115 | A | 0.22 | 0.0383600 | 0.46 |
| FER1L6 | 2842 | 7832499 | C | 0.05 | 0.0355900 | 0.27 |
| FER1L6 | 2844 | 13254498 | A | 0.05 | 0.0355900 | 0.27 |
| MTSS1 | 2855 | 1978661 | A | 0.40 | 0.0179500 | 2.35 |
| COL22A1 | 2908 | 13271565 | T | 0.40 | 0.0268900 | 2.21 |
| COL22A1 | 2915 | 1879047 | T | 0.07 | 0.0039890 | 0.22 |
| SMARCA2 | 2922 | 16937123 | C | 0.19 | 0.0146900 | 3.51 |
| PTPRD | 2942 | 1500326 | T | 0.26 | 0.0473300 | 0.49 |
| PTPRD | 2953 | 4742533 | C | 0.18 | 0.0444300 | 0.44 |
| TRPM6 | 3044 | 2274923 | C | 0.20 | 0.0014970 | 5.88 |
| GNAQ | 3065 | 11145659 | T | 0.31 | 0.0087100 | 2.87 |
| GNAQ | 3068 | 10781482 | T | 0.31 | 0.0105500 | 2.80 |
| GNAQ | 3070 | 6560625 | C | 0.33 | 0.0324900 | 2.26 |
| DAPK1 | 3083 | 1927973 | C | 0.57 | 0.0442700 | 1.98 |
| RP11-35N6.1 | 3109 | 10989207 | T | 0.48 | 0.0346100 | 2.05 |
| SVEP1 | 3123 | 7043896 | A | 0.21 | 0.0394900 | 0.45 |
| SVEP1 | 3125 | 1571236 | T | 0.21 | 0.0435800 | 0.46 |
| STOM | 3142 | 2196311 | G | 0.07 | 0.0416700 | 0.32 |
| ABL1 | 3169 | 3824400 | A | 0.19 | 0.0327500 | 2.91 |
| TSC1 | 3180 | 7858160 | T | 0.26 | 0.0117900 | 3.00 |
| PNPLA7 | 3204 | 10867123 | G | 0.12 | 0.0278700 | 0.37 |
| PITRM1 | 3214 | 7094698 | A | 0.22 | 0.0148200 | 0.40 |
| PITRM1 | 3215 | 3740607 | G | 0.41 | 0.0107600 | 0.42 |
| PITRM1 | 3216 | 2388557 | A | 0.21 | 0.0435800 | 0.46 |
| CUGBP2 | 3231 | 658469 | C | 0.38 | 0.0275800 | 0.48 |
| CACNB2 | 3234 | 7898858 | A | 0.62 | 0.0136900 | 2.31 |
| CDH23 | 3310 | 9415998 | G | 0.59 | 0.0049580 | 2.58 |
| CDH23 | 3311 | 9415047 | C | 0.39 | 0.0322000 | 0.48 |
| CDH23 | 3312 | 3802711 | T | 0.34 | 0.0001127 | 5.09 |
| CDH23 | 3314 | 4747197 | T | 0.38 | 0.0051710 | 2.84 |
| CDH23 | 3315 | 2070968 | A | 0.31 | 0.0026060 | 3.48 |
| KCNMA1 | 3327 | 7897566 | C | 0.50 | 0.0150500 | 2.31 |
| SORBS1 | 3359 | 7079293 | T | 0.26 | 0.0188200 | 0.43 |
| SORBS1 | 3360 | 4918918 | T | 0.25 | 0.0235300 | 0.43 |
| SORBS1 | 3363 | 11188299 | C | 0.12 | 0.0104800 | 6.45 |
| SORBS1 | 3367 | 540746 | T | 0.31 | 0.0298900 | 0.47 |
| SORCS3 | 3372 | 10786808 | T | 0.31 | 0.0026060 | 3.48 |
| SORCS3 | 3382 | 2177744 | C | 0.24 | 0.0386800 | 2.46 |
| VTI1A | 3403 | 11196083 | T | 0.17 | 0.0298100 | 0.42 |
| ATRNL1 | 3420 | 10885833 | C | 0.21 | 0.0068830 | 3.91 |
| ARNTL | 3483 | 7112233 | T | 0.10 | 0.0075890 | 0.29 |
| ARNTL | 3489 | 4414197 | T | 0.14 | 0.0105300 | 0.34 |
| ARNTL | 3490 | 11022753 | C | 0.14 | 0.0149000 | 0.35 |
| ARNTL | 3491 | 10766073 | A | 0.16 | 0.0267100 | 0.39 |
| SPON1 | 3497 | 2049723 | C | 0.33 | 0.0209500 | 2.44 |
| SPONI | 3514 | 4756787 | C | 0.21 | 0.0270900 | 2.87 |
| NELL1 | 3516 | 12799803 | A | 0.29 | 0.0366100 | 2.34 |
| KCNA4 | 3518 | 605765 | G | 0.56 | 0.0079790 | 2.50 |
| 81.99 mb | 3524 | 1939640 | A | 0.21 | 0.0290900 | 0.43 |
| ELMOD1 | 3559 | 683266 | A | 0.38 | 0.0085320 | 2.65 |
| OPCML | 3575 | 7107264 | C | 0.21 | 0.0474200 | 2.52 |
| OPCML | 3580 | 6590701 | G | 0.33 | 0.0387300 | 2.21 |
| TSPAN9 | 3594 | 4304861 | G | 0.60 | 0.0124100 | 2.32 |
| CNOT2 | 3652 | 3817487 | A | 0.47 | 0.0410600 | 2.01 |
| NBEA | 3703 | 17081356 | G | 0.05 | 0.0244300 | 2.21 |
| TRPC4 | 3729 | 1556541 | C | 0.19 | 0.0019590 | 0.29 |
| LMO7 | 3741 | 502171 | G | 0.52 | 0.0466300 | 1.95 |
| LMO7 | 3742 | 7335067 | A | 0.18 | 0.0096220 | 0.35 |
| LMO7 | 3743 | 9530467 | G | 0.33 | 0.0365100 | 0.49 |
| GPC5 | 3766 | 655169 | T | 0.47 | 0.0076730 | 2.54 |
| GPC5 | 3768 | 9560814 | C | 0.38 | 0.0377100 | 2.14 |
| GPC5 | 3769 | 7987869 | C | 0.57 | 0.0133300 | 2.30 |
| GPC5 | 3788 | 7997883 | G | 0.31 | 0.0406600 | 0.49 |
| GPC5 | 3790 | 2148226 | G | 0.22 | 0.0282600 | 0.44 |
| ITGBL1 | 3838 | 7998372 | T | 0.55 | 0.0443400 | 1.96 |
| ITGBL1 | 3848 | 9300690 | C | 0.60 | 0.0442200 | 1.98 |
| NOVA1 | 3862 | 2144335 | A | 0.11 | 0.0240600 | 0.34 |
| NOVA1 | 3863 | 1950420 | G | 0.12 | 0.0199300 | 0.35 |
| NPAS3 | 3864 | 11851667 | C | 0.21 | 0.0435300 | 0.46 |
| NPAS3 | 3868 | 1579701 | C | 0.28 | 0.0044010 | 0.37 |
| NPAS3 | 3869 | 1579702 | G | 0.28 | 0.0062430 | 0.38 |
| NPAS3 | 3871 | 10133530 | A | 0.22 | 0.0136200 | 3.18 |
| GNG2 | 3906 | 8017705 | A | 0.30 | 0.0404500 | 0.48 |
| PPP2R5E | 3925 | 8008684 | G | 0.09 | 0.0464400 | 0.36 |
| RGS6 | 3949 | 10149207 | G | 0.23 | 0.0154200 | 0.41 |
| CCDC88C | 3982 | 4904764 | G | 0.22 | 0.0282600 | 0.44 |
| RYR3 | 4035 | 11072734 | G | 0.36 | 0.0439300 | 2.10 |
| RYR3 | 4036 | 891343 | T | 0.38 | 0.0456700 | 2.06 |
| MEIS2 | 4049 | 1562804 | T | 0.53 | 0.0094870 | 2.41 |
| RASGRP1 | 4052 | 17574546 | C | 0.09 | 0.0042040 | 0.24 |
| RASGRP1 | 4053 | 16967120 | T | 0.07 | 0.0026770 | 0.20 |
| CGNL1 | 4068 | 4774257 | G | 0.43 | 0.0284600 | 2.15 |
| RORA | 4082 | 11071548 | G | 0.59 | 0.0104800 | 2.39 |
| TBC1D2B | 4097 | 4473152 | G | 0.43 | 0.0163500 | 2.34 |
| TBC1D2B | 4098 | 12903461 | T | 0.43 | 0.0194300 | 2.27 |
| SH3GL3 | 4106 | 11633764 | A | 0.13 | 0.0418200 | 0.39 |
| PCSK6 | 4110 | 11247300 | A | 0.21 | 0.0143000 | 3.32 |
| A2BP1 | 4130 | 2346943 | G | 0.59 | 0.0292500 | 2.10 |
| CRISPLD2 | 4215 | 8049317 | G | 0.53 | 0.0281400 | 2.09 |
| GAS7 | 4219 | 10491088 | C | 0.14 | 0.0051100 | 0.31 |
| DNAH9 | 4220 | 4141189 | A | 0.33 | 0.0268400 | 0.47 |
| CA10 | 4250 | 9303606 | C | 0.64 | 0.0009870 | 3.07 |
| CA10 | 4251 | 12945099 | C | 0.66 | 0.0018140 | 2.90 |
| DLGAP1 | 4278 | 7245298 | G | 0.37 | 0.0454100 | 2.12 |
| ZFP161 | 4282 | 990072 | T | 0.54 | 0.0033140 | 2.78 |
| ZFP161 | 4283 | 11665417 | T | 0.34 | 0.0452600 | 0.50 |
| ZFP161 | 4284 | 9945680 | A | 0.34 | 0.0452600 | 0.50 |
| PTPRM | 4293 | 1866854 | G | 0.45 | 0.0383900 | 2.06 |
| NEDD4L | 4328 | 4940385 | C | 0.53 | 0.0094870 | 2.41 |
| NEDD4L | 4329 | 10513914 | G | 0.38 | 0.0313900 | 2.18 |
| NEDD4L | 4330 | 2075405 | G | 0.34 | 0.0422200 | 2.13 |
| DOK6 | 4381 | 4438406 | C | 0.55 | 0.0011520 | 3.05 |
| DOK6 | 4382 | 9962570 | T | 0.36 | 0.0364900 | 2.16 |
| MACROD2 | 4441 | 994567 | C | 0.38 | 0.0456700 | 2.06 |
| MACROD2 | 4442 | 6135115 | G | 0.29 | 0.0272100 | 2.43 |
| MACROD2 | 4443 | 204606 | G | 0.38 | 0.0456700 | 2.06 |
| MACROD2 | 4444 | 6079395 | G | 0.62 | 0.0275800 | 2.10 |
| SEC23B | 4489 | 963435 | G | 0.47 | 0.0410600 | 2.01 |
| KCNB1 | 4518 | 13042686 | G | 0.02 | 0.0225300 | 0.13 |
| CDH4 | 4531 | 6121764 | G | 0.52 | 0.0311800 | 2.08 |
| CDH4 | 4532 | 1317385 | C | 0.52 | 0.0338600 | 2.05 |
| CDH4 | 4533 | 6089669 | A | 0.40 | 0.0330100 | 2.15 |
| CDH4 | 4536 | 17811544 | G | 0.33 | 0.0365100 | 0.49 |
| CDH4 | 4537 | 6121844 | C | 0.17 | 0.0071560 | 4.69 |
| PDE9A | 4564 | 12626774 | C | 0.19 | 0.0017080 | 0.30 |

| **TABLE 15: Alleles Influencing Discontinuation for Lack of Efficacy for Ziprasidone** | | | | | | |
|---|---|---|---|---|---|---|
| **Gene** | **Seq ID** | **NCBI RS#** | **Allele** | **Freq. in Discontinuers** | **P** | **Odds Ratio** |
| PIK3CD | 1 | 10779729 | A | 0.17 | 0.03768 | 0.33 |
| RP1-21O18.1 | 7 | 6663699 | G | 0.43 | 0.04776 | 2.49 |
| RP1-21O18.1 | 13 | 10803343 | G | 0.07 | 0.03146 | 3.67 |
| AGBL4-C1ORF165 | 28 | 11205538 | C | 0.18 | 0.00368 | 0.22 |
| AGBL4-C1ORF165 | 33 | 4285747 | C | 0.21 | 0.00472 | 0.24 |
| AGBL4-C1ORF165 | 34 | 3121522 | A | 0.20 | 0.00239 | 0.22 |
| AGBL4-C1ORF165 | 35 | 12043418 | T | 0.23 | 0.00649 | 0.27 |
| AGBL4-C1ORF165 | 36 | 2355693 | T | 0.17 | 0.00620 | 0.24 |
| AGBL4-C1ORF165 | 37 | 3122291 | T | 0.30 | 0.00854 | 0.30 |
| KCNN3 | 96 | 6664817 | G | 0.07 | 0.03146 | 4.58 |
| ATF6 | 99 | 1875762 | T | 0.21 | 0.00932 | 5.91 |
| ATF6 | 101 | 10918029 | A | 0.13 | 0.04796 | 5.08 |
| RYR2 | 198 | 637520 | T | 0.60 | 0.04589 | 2.42 |
| RYR2 | 199 | 604735 | A | 0.60 | 0.01027 | 3.14 |
| RYR2 | 214 | 2249847 | C | 0.57 | 0.00892 | 3.24 |
| FMN2 | 244 | 3795677 | A | 0.46 | 0.01047 | 3.40 |
| FMN2 | 245 | 10157105 | G | 0.43 | 0.02019 | 2.95 |
| FMN2 | 246 | 12122068 | C | 0.33 | 0.03497 | 2.90 |
| FMN2 | 247 | 10926223 | C | 0.33 | 0.03497 | 2.90 |
| RGS7 | 255 | 191735 | A | 0.23 | 0.00948 | 0.29 |
| RGS7 | 257 | 4659585 | A | 0.23 | 0.00187 | 0.23 |
| RGS7 | 258 | 10802917 | G | 0.73 | 0.00136 | 4.44 |
| RGS7 | 259 | 670659 | T | 0.50 | 0.02311 | 2.78 |
| C2ORF46 | 274 | 182971 | C | 0.30 | 0.01499 | 3.74 |
| NTSR2 | 288 | 4669767 | C | 0.27 | 0.02258 | 0.34 |
| KLHL29 | 289 | 747345 | A | 0.20 | 0.01128 | 0.28 |
| KLHL29 | 294 | 10172684 | G | 0.17 | 0.01797 | 0.29 |
| KCNK3 | 306 | 1275982 | C | 0.57 | 0.02803 | 2.68 |
| KCNK3 | 307 | 11126666 | A | 0.43 | 0.03939 | 2.65 |
| BRE | 324 | 12468596 | A | 0.33 | 0.04203 | 2.80 |
| CRIM1 | 329 | 3770919 | G | 0.13 | 0.01918 | 0.26 |
| SLC8A1 | 344 | 2110922 | T | 0.53 | 0.02358 | 2.74 |
| PRKCE | 387 | 6748375 | C | 0.57 | 0.01031 | 3.14 |
| PSME4 | 402. | 805399 | A | 0.43 | 0.02019 | 2.95 |
| PSME4 | 403 | 805404 | C | 0.43 | 0.03166 | 2.70 |
| PSME4 | 404 | 805415 | C | 0.43 | 0.03166 | 2.70 |
| PSME4 | 405 | 805319 | G | 0.43 | 0.03166 | 2.70 |
| PSME4 | 406 | 805360 | T | 0.53 | 0.03460 | 2.56 |
| PSME4 | 407 | 3731967 | A | 0.57 | 0.01031 | 3.14 |
| PSME4 | 408 | 10183655 | T | 0.57 | 0.01576 | 2.93 |
| PSME4 | 409 | 17045488 | G | 0.40 | 0.04485 | 2.57 |
| PSME4 | 410 | 1363061 | T | 0.30 | 0.01499 | 3.74 |
| ACYP2 | 411 | 17039547 | C | 0.40 | 0.04485 | 2.57 |
| ACYP2 | 412 | 918357 | G | 0.30 | 0.00854 | 0.30 |
| CTNNA2 | 444 | 1971766 | A | 0.60 | 0.01027 | 3.14 |
| CTNNA2 | 445 | 2566542 | C | 0.29 | 0.01526 | 4.00 |
| NAPS | 478 | 1437904 | T | 0.13 | 0.01918 | 0.26 |
| NAPS | 479 | 10176860 | C | 0.07 | 0.02497 | 0.20 |
| NAPS | 481 | 1434227 | A | 0.07 | 0.00408 | 0.14 |
| NAPS | 484 | 1975218 | A | 0.03 | 0.03959 | 0.15 |
| NXPH2 | 499 | 11686876 | T | 0.23 | 0.00948 | 0.29 |
| LRP1B | 509 | 10201482 | C | 0.07 | 0.04085 | 0.22 |
| LRP1B | 522 | 11691595 | G | 0.50 | 0.04995 | 2.40 |
| LRP1B | 523 | 1900936 | T | 0.63 | 0.03077 | 2.62 |
| CACNB4 | 571 | 13429172 | C | 0.13 | 0.01369 | 0.25 |
| FMNL2 | 576 | 11684450 | G | 0.57 | 0.04807 | 2.40 |
| FMNL2 | 587 | 1561267 | C | 0.23 | 0.01379 | 4.72 |
| FMNL2 | 588 | 2272535 | G | 0.20 | 0.03335 | 4.00 |
| FMNL2 | 589 | 3817620 | G | 0.20 | 0.03335 | 4.00 |
| PDE1A | 642 | 16823124 | A | 0.13 | 0.04935 | 0.32 |
| PDE1A | 650 | 2887208 | C | 0.57 | 0.03096 | 2.62 |
| ALS2 | 651 | 3219171 | T | 0.17 | 0.00447 | 13.00 |
| ALS2 | 652 | 10469756 | T | 0.20 | 0.00448 | 8.25 |
| ALS2 | 653 | 12470229 | C | 0.13 | 0.04796 | 5.08 |
| NRP2 | 672 | 849565 | C | 0.30 | 0.00729 | 4.43 |
| COL4A3 | 700 | 2178631 | C | 0.63 | 0.02157 | 2.79 |
| COL4A3 | 702 | 4263106 | T | 0.27 | 0.03801 | 3.17 |
| COL4A3 | 703 | 6750210 | C | 0.33 | 0.02476 | 3.17 |
| COL4A3 | 704 | 12468691 | C | 0.30 | 0.01499 | 3.74 |
| COL4A3 | 705 | 6436674 | T | 0.20 | 0.03335 | 4.00 |
| COL4A3 | 708 | 7587228 | C | 0.20 | 0.03335 | 4.00 |
| COL4A3 | 709 | 10188531 | G | 0.20 | 0.03335 | 4.00 |
| TRIP12 | 728 | 17324331 | G | 0.20 | 0.02246 | 0.32 |
| ECEL1 | 729 | 12616972 | C | 0.23 | 0.02700 | 0.34 |
| CNTN6 | 737 | 3772339 | T | 0.57 | 0.03396 | 2.56 |
| CNTN6 | 745 | 2055738 | C | 0.63 | 0.01481 | 2.97 |
| CNTN6 | 746 | 3772290 | T | 0.63 | 0.01481 | 2.97 |
| CNTN4 | 759 | 11915265 | T | 0.53 | 0.02359 | 2.79 |
| CNTN4 | 760 | 1153486 | T | 0.53 | 0.03460 | 2.56 |
| CNTN4 | 761 | 1685456 | C | 0.63 | 0.00912 | 3.23 |
| CNTN4 | 762 | 1502582 | T | 0.30 | 0.04823 | 2.81 |
| CNTN4 | 765 | 9876071 | T | 0.27 | 0.00756 | 0.29 |
| CNTN4 | 766 | 12496950 | G | 0.17 | 0.00895 | 0.25 |
| CNTN4 | 767 | 12494838 | G | 0.13 | 0.02656 | 0.28 |
| FBXL2 | 811 | 4234254 | C | 0.03 | 0.00374 | 0.08 |
| FBXL2 | 812 | 6783644 | G | 0.03 | 0.00636 | 0.09 |
| CLASP2 | 815 | 4629282 | G | 0.03 | 0.00758 | 0.10 |
| ULK4 | 827 | 11707955 | C | 0.67 | 0.02840 | 2.69 |
| CACNA2D3 | 868 | 13082611 | G | 0.20 | 0.01128 | 0.28 |
| CACNA2D3 | 869 | 17140 | T | 0.13 | 0.01918 | 0.26 |
| ERC2 | 877 | 3796232 | G | 0.17 | 0.00189 | 0.20 |
| ERC2 | 893 | 17825409 | C | 0.13 | 0.04935 | 0.32 |
| IL17RD | 904 | 747088 | A | 0.03 | 0.02878 | 0.13 |
| PTPRG | 935 | 4447755 | C | 0.33 | 0.04203 | 2.80 |
| PRICKLE2 | 966 | 4994608 | A | 0.20 | 0.02389 | 0.32 |
| FAM19A1 | 982 | 6801913 | A | 0.40 | 0.03564 | 2.72 |
| FOXP1 | 986 | 2166780 | T | 0.50 | 0.04995 | 2.40 |
| HTR1F | 999 | 1027689 | T | 0.23 | 0.01166 | 4.87 |
| PLCXD2 | 1012 | 13074817 | G | 0.64 | 0.01277 | 3.15 |
| PLCXD2 | 1013 | 13079085 | C | 0.63 | 0.02157 | 2.79 |
| PLCXD2 | 1014 | 6768713 | A | 0.63 | 0.02157 | 2.79 |
| PLCXD2 | 1015 | 12490166 | A | 0.63 | 0.02936 | 2.66 |
| STXBP5L | 1033 | 2331921 | A | 0.57 | 0.02343 | 2.73 |
| STXBP5L | 1035 | 12186062 | C | 0.57 | 0.02102 | 2.80 |
| KALRN | 1040 | 634265 | A | 0.57 | 0.04088 | 2.50 |
| CPNE4 | 1055 | 9289393 | T | 0.57 | 0.04445 | 2.45 |
| CPNE4 | 1056 | 9289395 | G | 0.50 | 0.01500 | 3.00 |
| CPNE4 | 1057 | 876519 | G | 0.63 | 0.04302 | 2.47 |
| CLSTN2 | 1076 | 6439927 | C | 0.47 | 0.01391 | 3.09 |
| TNIK | 1101 | 9824475 | G | 0.43 | 0.04776 | 2.49 |
| SLC2A9 | 1159 | 3733591 | A | 0.07 | 0.04693 | 0.23 |
| LDB2 | 1184 | 13110882 | G | 0.23 | 0.00948 | 0.29 |
| SLIT2 | 1188 | 10516357 | A | 0.47 | 0.03353 | 2.63 |
| LPHN3 | 1232 | 950313 | C | 0.07 | 0.03405 | 0.21 |
| LPHN3 | 1233 | 1124974 | A | 0.07 | 0.03436 | 0.21 |
| SCDS | 1265 | 6811012 | T | 0.07 | 0.04693 | 0.23 |
| FAM13A1 | 1286 | 1398938 | T | 0.10 | 0.04921 | 7.44 |
| COL25A1 | 1317 | 2305438 | A | 0.23 | 0.01934 | 0.32 |
| ANK2 | 1345 | 967099 | T | 0.57 | 0.01031 | 3.14 |
| ANK2 | 1348 | 362496 | G | 0.47 | 0.00490 | 3.70 |
| ANK2 | 1351 | 29306 | G | 0.40 | 0.01771 | 3.11 |
| ANK2 | 1353 | 29311 | A | 0.40 | 0.01771 | 3.11 |
| MAML3 | 1395 | 11729794 | T | 0.37 | 0.04302 | 0.41 |
| MAML3 | 1397 | 17051001 | T | 0.53 | 0.02358 | 2.74 |
| DCLK2 | 1437 | 10001651 | C | 0.17 | 0.04589 | 0.34 |
| DCLK2 | 1438 | 11735949 | C | 0.23 | 0.01934 | 0.32 |
| FSTL5 | 1461 | 45426 | T | 0.20 | 0.00239 | 0.22 |
| TLL1 | 1478 | 17505781 | T | 0.13 | 0.03639 | 0.30 |
| PALLD | 1503 | 17614715 | T | 0.53 | 0.01774 | 2.92 |
| PALLD | 1504 | 13116423 | T | 0.53 | 0.02056 | 2.83 |
| PALLD | 1505 | 924511 | T | 0.53 | 0.02358 | 2.74 |
| ODZ3 | 1515 | 6820811 | G | 0.50 | 0.00940 | 3.25 |
| AHRR | 1534 | 2251843 | C | 0.30 | 0.04890 | 0.40 |
| SEMA5A | 1535 | 985723 | A | 0.27 | 0.02372 | 3.64 |
| DNAH5 | 1550 | 6554811 | G | 0.63 | 0.01481 | 2.97 |
| DNAH5 | 1551 | 2401810 | G | 0.57 | 0.02803 | 2.68 |
| DNAH5 | 1552 | 2401809 | A | 0.57 | 0.03096 | 2.62 |
| DNAH5 | 1561 | 1530498 | G | 0.17 | 0.03768 | 0.33 |
| ITGA1 | 1606 | 10513001 | G | 0.13 | 0.03639 | 0.30 |
| ITGA1 | 1611 | 923837 | T | 0.70 | 0.03581 | 2.63 |
| PDE4D | 1627 | 2910641 | A | 0.40 | 0.04485 | 2.57 |
| GPR98 | 1685 | 4916681 | T | 0.67 | 0.00934 | 3.23 |
| GPR98 | 1687 | 16868980 | C | 0.60 | 0.00359 | 3.71 |
| GPR98 | 1688 | 2366926 | G | 0.60 | 0.00418 | 3.60 |
| GPR98 | 1691 | 2438359 | T | 0.63 | 0.04302 | 2.47 |
| GPR98 | 1693 | 2950852 | A | 0.17 | 0.01503 | 6.60 |
| FBXL17 | 1702 | 288144 | A | 0.37 | 0.04060 | 2.70 |
| FBXL17 | 1703 | 289227 | C | 0.37 | 0.04060 | 2.70 |
| FBXL17 | 1711 | 7706429 | A | 0.03 | 0.00027 | 0.05 |
| SNX2 | 1758 | 4343835 | C | 0.60 | 0.01547 | 2.94 |
| ODZ2 | 1787 | 2337017 | C | 0.03 | 0.03959 | 0.15 |
| PHACTR1 | 1822 | 4714990 | C | 0.53 | 0.04006 | 2.51 |
| ATXN1 | 1838 | 3812206 | T | 0.47 | 0.03353 | 2.63 |
| RNF144B | 1842 | 9477749 | C | 0.53 | 0.04468 | 2.45 |
| RNF144B | 1843 | 531481 | T | 0.10 | 0.03664 | 0.27 |
| SLC17A1 | 1857 | 4712976 | T | 0.30 | 0.04823 | 2.81 |
| BTNL2 | 1878 | 2076530 | G | 0.27 | 0.03146 | 0.36 |
| RIMS1 | 1903 | 9446563 | G | 0.60 | 0.02274 | 2.75 |
| GABRR2 | 1918 | 282128 | A | 0.33 | 0.01999 | 0.35 |
| SLC22A16 | 1929 | 723685 | C | 0.00 | 0.03653 | 0.00 |
| NKAIN2 | 1957 | 173067 | A | 0.17 | 0.01277 | 0.27 |
| NKAIN2 | 1958 | 163284 | A | 0.20 | 0.02389 | 0.32 |
| NKAIN2 | 1959 | 12195023 | T | 0.10 | 0.01931 | 0.23 |
| NKAIN2 | 1960 | 163295 | A | 0.23 | 0.04101 | 0.37 |
| NKAIN2 | 1971 | 11154271 | C | 0.37 | 0.00515 | 4.20 |
| SYNE1 | 2007 | 4472361 | G | 0.40 | 0.04485 | 2.57 |
| SYNE1 | 2008 | 7776230 | T | 0.40 | 0.04485 | 2.57 |
| CARD11 | 2069 | 4722277 | T | 0.63 | 0.03077 | 2.62 |
| FERD3L | 2103 | 10228726 | G | 0.23 | 0.02613 | 3.84 |
| FERD3L | 2104 | 7780145 | G | 0.37 | 0.01461 | 3.36 |
| CREB5 | 2121 | 6977628 | T | 0.50 | 0.02002 | 3.00 |
| CREB5 | 2126 | 886750 | A | 0.43 | 0.04776 | 2.49 |
| PDE1C | 2155 | 6952633 | G | 0.23 | 0.03717 | 0.36 |
| PDE1C | 2158 | 30568 | C | 0.33 | 0.01104 | 3.75 |
| PDE1A | 2159 | 30589 | G | 0.37 | 0.00079 | 5.98 |
| CDC2L5 | 2186 | 3800802 | T | 0.50 | 0.02977 | 2.67 |
| ABCA13 | 2193 | 17661898 | A | 0.00 | 0.04995 | 0.00 |
| ABCA13 | 2196 | 17548783 | C | 0.29 | 0.02100 | 0.33 |
| WBSCR17 | 2229 | 7777301 | C | 0.67 | 0.00253 | 3.91 |
| WBSCR17 | 2230 | 7808758 | C | 0.67 | 0.00401 | 3.67 |
| WBSCR17 | 2233 | 10950240 | A | 0.70 | 0.01192 | 3.20 |
| PCLO | 2330 | 976714 | T | 0.20 | 0.02246 | 0.32 |
| ABCB4 | 2351 | 31662 | A | 0.00 | 0.04995 | 0.00 |
| ADAM22 | 2360 | 2299199 | G | 0.30 | 0.04943 | 0.40 |
| ZNF3 | 2398 | 7778571 | G | 0.17 | 0.04663 | 0.34 |
| SLC13A1 | 2429 | 6963735 | C | 0.17 | 0.00895 | 0.25 |
| SLC13A1 | 2430 | 2140516 | C | 0.43 | 0.01233 | 3.24 |
| GRM8 | 2451 | 2106183 | T | 0.13 | 0.04935 | 0.32 |
| CNTNAP2 | 2507 | 6958824 | A | 0.33 | 0.00550 | 4.36 |
| CNTNAP2 | 2508 | 1637864 | T | 0.30 | 0.01499 | 3.74 |
| CNTNAP2 | 2509 | 1637840 | T | 0.30 | 0.01499 | 3.74 |
| PTPRN2 | 2519 | 4909289 | A | 0.13 | 0.02396 | 0.27 |
| PTPRN2 | 2526 | 2335836 | C | 0.18 | 0.03901 | 0.33 |
| CSMD1 | 2543 | 13260434 | A | 0.07 | 0.04693 | 0.23 |
| SGCZ | 2580 | 2898389 | C | 0.57 | 0.02343 | 2.73 |
| SGCZ | 2583 | 1551816 | G | 0.27 | 0.02258 | 0.34 |
| SGCZ | 2584 | 4428672 | T | 0.27 | 0.03146 | 0.36 |
| SGCZ | 2585 | 12545852 | C | 0.27 | 0.03146 | 0.36 |
| SGCZ | 2586 | 728845 | A | 0.30 | 0.03569 | 0.38 |
| SGCZ | 2589 | 1903597 | G | 0.27 | 0.03146 | 0.36 |
| SGCZ | 2591 | 1461868 | A | 0.13 | 0.04935 | 0.32 |
| SGCZ | 2592 | 17119601 | G | 0.13 | 0.04935 | 0.32 |
| SLC7A2 | 2597 | 2588233 | C | 0.23 | 0.01488 | 0.30 |
| SLC25A37 | 2635 | 10092233 | G | 0.13 | 0.04796 | 5.08 |
| UNCSD | 2642 | 7826624 | T | 0.07 | 0.03436 | 0.21 |
| SNTG1 | 2665 | 2467203 | A | 0.40 | 0.04485 | 2.57 |
| SNTG1 | 2668 | 2625758 | G | 0.40 | 0.04485 | 2.57 |
| PLAG1 | 2682 | 13273123 | G | 0.32 | 0.01744 | 3.55 |
| DEPDC2 | 2732 | 7007570 | G | 0.54 | 0.02220 | 2.85 |
| BAALC | 2788 | 11785583 | G | 0.07 | 0.00914 | 0.16 |
| BAALC | 2789 | 4734695 | C | 0.17 | 0.01797 | 0.29 |
| CSMD3 | 2815 | 4354335 | G | 0.53 | 0.00617 | 3.59 |
| MTSS1 | 2848 | 17371123 | G | 0.67 | 0.00934 | 3.23 |
| DDEF1 | 2862 | 12546206 | A | 0.53 | 0.03460 | 2.56 |
| DDEF1 | 2865 | 12675390 | C | 0.43 | 0.00396 | 3.96 |
| DDEF1 | 2866 | 1469286 | T | 0.39 | 0.00810 | 3.75 |
| DDEF1 | 2868 | 16904231 | C | 0.37 | 0.01461 | 3.36 |
| DDEF1 | 2870 | 16904239 | A | 0.37 | 0.01461 | 3.36 |
| DDEF1 | 2871 | 16904241 | G | 0.37 | 0.01461 | 3.36 |
| DDEF1 | 2872 | 4733783 | T | 0.37 | 0.01461 | 3.36 |
| DDEF1 | 2873 | 4733788 | G | 0.37 | 0.01461 | 3.36 |
| DDEF1 | 2875 | 2791349 | C | 0.10 | 0.01382 | 0.22 |
| DDEF1 | 2876 | 6470814 | A | 0.37 | 0.01461 | 3.36 |
| DDEF1 | 2877 | 1123205 | G | 0.53 | 0.02358 | 2.74 |
| DDEF1 | 2878 | 11786705 | G | 0.37 | 0.01461 | 3.36 |
| DDEF1 | 2879 | 4574884 | G | 0.10 | 0.01575 | 0.22 |
| DDEF1 | 2880 | 17287702 | T | 0.10 | 0.02672 | 0.25 |
| DDEF1 | 2881 | 16904260 | C | 0.10 | 0.02672 | 0.25 |
| DDEF1 | 2882 | 10086454 | C | 0.13 | 0.03639 | 0.30 |
| ST3GAL1 | 2905 | 17722401 | A | 0.67 | 0.01855 | 2.92 |
| ST3GAL1 | 2906 | 10112722 | G | 0.67 | 0.01999 | 2.86 |
| COL22A1 | 2910 | 4909444 | T | 0.17 | 0.04150 | 0.33 |
| COL22A1 | 2911 | 12674962 | C | 0.23 | 0.00649 | 0.27 |
| COL22A1 | 2918 | 1316335 | A | 0.10 | 0.04978 | 0.29 |
| COL22A1 | 2919 | 9657434 | T | 0.10 | 0.04978 | 0.29 |
| SMARCA2 | 2928 | 7872216 | T | 0.27 | 0.03801 | 3.17 |
| TEK | 2987 | 4601425 | T | 0.50 | 0.02311 | 2.78 |
| TEK | 2989 | 10511803 | C | 0.37 | 0.00799 | 3.80 |
| MAMDC2 | 3012 | 943537 | T | 0.13 | 0.00466 | 0.21 |
| MAMDC2 | 3013 | 4531114 | A | 0.07 | 0.03436 | 0.21 |
| MAMDC2 | 3015 | 11142029 | T | 0.13 | 0.00675 | 0.22 |
| MAMDC2 | 3016 | 1927098 | C | 0.07 | 0.03436 | 0.21 |
| MAMDC2 | 3017 | 2773362 | T | 0.07 | 0.03522 | 0.21 |
| MAMDC2 | 3018 | 3858107 | T | 0.13 | 0.03639 | 0.30 |
| MAMDC2 | 3019 | 1180079 | T | 0.07 | 0.04085 | 0.22 |
| TRPM3 | 3020 | 1932701 | C | 0.00 | 0.04995 | 0.00 |
| TMC1 | 3040 | 1663763 | T | 0.42 | 0.04056 | 2.72 |
| TRPM6 | 3044 | 2274923 | C | 0.18 | 0.03027 | 4.71 |
| PCSK5 | 3053 | 4745488 | C | 0.47 | 0.02199 | 2.84 |
| RP11-35N6.1 | 3109 | 10989207 | T | 0.50 | 0.04995 | 2.40 |
| FKTN | 3115 | 635712 | C | 0.50 | 0.01500 | 3.00 |
| PALM2 | 3116 | 17202273 | C | 0.10 | 0.03664 | 0.27 |
| PALM2 | 3117 | 17202329 | C | 0.10 | 0.03664 | 0.27 |
| PALM2 | 3119 | 7849027 | G | 0.10 | 0.03664 | 0.27 |
| PALM2 | 3121 | 7032940 | A | 0.07 | 0.00516 | 0.14 |
| PALM2 | 3122 | 7036157 | G | 0.07 | 0.00643 | 0.15 |
| SVEP1 | 3128 | 4978939 | C | 0.63 | 0.03077 | 2.62 |
| ZNF618 | 3129 | 4978561 | T | 0.10 | 0.02672 | 0.25 |
| ZNF618 | 3130 | 4979326 | A | 0.10 | 0.03664 | 0.27 |
| DAB2IP | 3143 | 10985431 | T | 0.00 | 0.00727 | 0.00 |
| RALGPS1 | 3152 | 2025955 | T | 0.47 | 0.01391 | 3.09 |
| RALGPS1 | 3153 | 10760473 | G | 0.47 | 0.01391 | 3.09 |
| RALGPS1 | 3155 | 13298677 | G | 0.33 | 0.02037 | 3.28 |
| RALGPS1 | 3157 | 12350252 | A | 0.25 | 0.00393 | 0.25 |
| RALGPS1 | 3158 | 1028866 | G | 0.30 | 0.00569 | 0.28 |
| RALGPS1 | 3159 | 1028865 | T | 0.27 | 0.00756 | 0.29 |
| ABL1 | 3170 | 7021981 | T | 0.23 | 0.02613 | 3.84 |
| ABL1 | 3171 | 11792273 | G | 0.23 | 0.02613 | 3.84 |
| NOTCH1 | 3200 | 10870085 | T | 0.10 | 0.04978 | 0.29 |
| ABCA2 | 3201 | 12337910 | G | 0.00 | 0.01981 | 0.00 |
| PITRM1 | 3217 | 4242746 | T | 0.47 | 0.03567 | 2.63 |
| CACNB2 | 3240 | 10828726 | G | 0.53 | 0.04946 | 2.39 |
| MYO3A | 3262 | 10741104 | C | 0.70 | 0.02563 | 2.79 |
| MYO3A | 3264 | 10828912 | A | 0.70 | 0.02563 | 2.79 |
| MYO3A | 3285 | 12764197 | G | 0.07 | 0.03005 | 0.21 |
| MYO3A | 3287 | 3781117 | C | 0.07 | 0.01800 | 0.18 |
| CDH23 | 3308 | 1227051 | C | 0.38 | 0.03748 | 2.90 |
| KCNMA1 | 3322 | 1907729 | T | 0.03 | 0.03959 | 0.15 |
| KCNMA1 | 3328 | 10824519 | T | 0.30 | 0.02563 | 0.36 |
| KCNMA1 | 3329 | 10824520 | T | 0.30 | 0.03569 | 0.38 |
| KCNMA1 | 3332 | 11002137 | C | 0.30 | 0.03569 | 0.38 |
| NRG3 | 3339 | 17099789 | G | 0.07 | 0.04593 | 0.23 |
| NRG3 | 3340 | 1937959 | G | 0.07 | 0.01065 | 0.16 |
| NRG3 | 3341 | 7902584 | G | 0.07 | 0.01800 | 0.18 |
| HSPA12A | 3426 | 3010476 | G | 0.57 | 0.01576 | 2.93 |
| GRK5 | 3430 | 10787948 | C | 0.57 | 0.02343 | 2.73 |
| ATE1 | 3431 | 1693688 | C | 0.20 | 0.01128 | 0.28 |
| ATE1 | 3435 | 1219505 | T | 0.30 | 0.04890 | 0.40 |
| ARNTL | 3485 | 4372426 | C | 0.60 | 0.02093 | 2.80 |
| ARNTL | 3488 | 900144 | G | 0.61 | 0.04410 | 2.50 |
| SPON1 | 3514 | 4756787 | C | 0.03 | 0.03959 | 0.15 |
| OPCML | 3577 | 7110672 | C | 0.07 | 0.01288 | 0.17 |
| OPCML | 3579 | 6590700 | C | 0.10 | 0.03664 | 0.27 |
| OPCML | 3583 | 12419575 | A | 0.07 | 0.02497 | 0.20 |
| OPCML | 3585 | 7936263 | G | 0.17 | 0.03768 | 0.33 |
| OPCML | 3588 | 7108751 | T | 0.13 | 0.04096 | 0.31 |
| OPCML | 3589 | 4936195 | G | 0.03 | 0.02534 | 0.13 |
| TSPAN9 | 3592 | 4766079 | C | 0.07 | 0.01800 | 0.18 |
| STYK1 | 3615 | 10845198 | T | 0.47 | 0.04942 | 2.43 |
| ITPR2 | 3639 | 7955200 | C | 0.13 | 0.04935 | 0.32 |
| SLC46A3 | 3693 | 1617234 | C | 0.30 | 0.04890 | 0.40 |
| SLC46A3 | 3694 | 715704 | G | 0.53 | 0.04946 | 2.39 |
| NBEA | 3704 | 2322289 | C | 0.04 | 0.03687 | 0.14 |
| NBEA | 3714 | 4500591 | A | 0.10 | 0.00775 | 0.19 |
| NBEA | 3721 | 7327540 | A | 0.33 | 0.02840 | 0.37 |
| NBEA | 3722 | 3818423 | C | 0.13 | 0.01918 | 0.26 |
| NBEA | 3724 | 3794388 | C | 0.07 | 0.03436 | 0.21 |
| GPC5 | 3762 | 9523312 | G | 0.17 | 0.00620 | 0.24 |
| GPC5 | 3763 | 9523313 | G | 0.17 | 0.01797 | 0.29 |
| GPC5 | 3779 | 1972350 | T | 0.47 | 0.02199 | 2.84 |
| GPC6 | 3812 | 9301916 | C | 0.47 | 0.03353 | 2.63 |
| GPC6 | 3813 | 9524268 | T | 0.47 | 0.03353 | 2.63 |
| GPC6 | 3819 | 4468463 | G | 0.03 | 0.03959 | 0.15 |
| ITGBL1 | 3839 | 1335589 | T | 0.20 | 0.04238 | 0.36 |
| NOVA1 | 3863 | 1950420 | G | 0.43 | 0.03166 | 2.70 |
| SLC25A21 | 3881 | 712377 | C | 0.27 | 0.02292 | 0.34 |
| SLC25A21 | 3885 | 1367029 | T | 0.37 | 0.02034 | 0.35 |
| GNG2 | 3908 | 10138800 | A | 0.27 | 0.02258 | 0.34 |
| SAMD4A | 3918 | 17128004 | A | 0.37 | 0.03069 | 2.90 |
| SAMD4A | 3919 | 10483639 | C | 0.37 | 0.04060 | 2.70 |
| RGS6 | 3953 | 917426 | G | 0.57 | 0.03396 | 2.56 |
| KCNK10 | 3961 | 1956541 | T | 0.30 | 0.04823 | 2.81 |
| KCNK10 | 3965 | 17698533 | C | 0.47 | 0.04942 | 2.43 |
| KCNK13 | 3968 | 12892344 | A | 0.30 | 0.03581 | 0.38 |
| ATXN3 | 3989 | 10140469 | G | 0.40 | 0.01304 | 3.33 |
| ATXN3 | 3990 | 12588287 | C | 0.40 | 0.01771 | 3.11 |
| ATXN3 | 3991 | 10146087 | G | 0.40 | 0.01771 | 3.11 |
| ATXN3 | 3992 | 11850101 | A | 0.40 | 0.01771 | 3.11 |
| ATXN3 | 3993 | 10146249 | A | 0.40 | 0.01771 | 3.11 |
| ATXN3 | 3994 | 1048755 | A | 0.40 | 0.01771 | 3.11 |
| ATXN3 | 3995 | 8020781 | C | 0.40 | 0.01771 | 3.11 |
| ATXN3 | 3996 | 1072958 | G | 0.40 | 0.01771 | 3.11 |
| ATXN3 | 3997 | 1072957 | C | 0.40 | 0.01771 | 3.11 |
| ATP10A | 4017 | 4906654 | T | 0.23 | 0.04150 | 3.47 |
| RYR3 | 4024 | 8043532 | C | 0.53 | 0.03460 | 2.56 |
| RYR3 | 4031 | 2132208 | A | 0.20 | 0.00448 | 8.25 |
| CGNL1 | 4069 | 4774940 | C | 0.10 | 0.01931 | 0.23 |
| CGNL1 | 4070 | 7182648 | T | 0.07 | 0.04693 | 0.23 |
| TBC1D2B | 4096 | 3803380 | G | 0.30 | 0.02800 | 3.21 |
| ARNT2 | 4105 | 7180510 | G | 0.50 | 0.02977 | 2.67 |
| A2BP1 | 4117 | 8059039 | T | 0.27 | 0.02372 | 3.64 |
| A2BP1 | 4118 | 1155959 | C | 0.40 | 0.04485 | 2.57 |
| A2BP1 | 4133 | 7498500 | C | 0.17 | 0.00895 | 0.25 |
| A2BP1 | 4138 | 7191615 | A | 0.13 | 0.04796 | 5.08 |
| EEF2K | 4148 | 4275840 | T | 0.37 | 0.00515 | 4.20 |
| PLCG2 | 4161 | 8043845 | C | 0.10 | 0.01382 | 0.22 |
| MPHOSPH6 | 4163 | 8052512 | T | 0.07 | 0.04085 | 0.22 |
| CDH13 | 4193 | 1424189 | T | 0.13 | 0.00967 | 0.23 |
| CDH13 | 4195 | 723920 | A | 0.20 | 0.03105 | 0.34 |
| CDH13 | 4197 | 11864731 | A | 0.20 | 0.00783 | 0.27 |
| CDH13 | 4198 | 10514577 | G | 0.23 | 0.02700 | 0.34 |
| RAB11FIP4 | 4224 | 757378 | T | 0.53 | 0.04006 | 2.51 |
| CA10 | 4227 | 8076197 | A | 0.30 | 0.02563 | 0.36 |
| CA10 | 4228 | 8076496 | A | 0.30 | 0.02563 | 0.36 |
| CA10 | 4231 | 203010 | T | 0.73 | 0.00507 | 3.70 |
| CA10 | 4233 | 203042 | A | 0.27 | 0.01595 | 0.32 |
| CA10 | 4234 | 4794315 | G | 0.67 | 0.01999 | 2.86 |
| CA10 | 4236 | 9896168 | G | 0.63 | 0.02793 | 2.70 |
| CA10 | 4240 | 1503058 | A | 0.70 | 0.02535 | 2.82 |
| CA10 | 4241 | 11652641 | G | 0.37 | 0.02505 | 3.00 |
| CA10 | 4242 | 12150128 | C | 0.29 | 0.04460 | 3.00 |
| DNAH17 | 4266 | 11649879 | T | 0.13 | 0.04796 | 5.08 |
| OSBPL1A | 4309 | 6508259 | A | 0.07 | 0.01250 | 0.17 |
| NEDD4L | 4335 | 11152073 | A | 0.07 | 0.00309 | 0.13 |
| CDH7 | 4351 | 976882 | A | 0.33 | 0.01781 | 0.34 |
| CDH7 | 4352 | 12970791 | T | 0.33 | 0.01999 | 0.35 |
| CDH7 | 4353 | 10871590 | A | 0.53 | 0.04946 | 2.39 |
| CDH7 | 4359 | 7244664 | G | 0.10 | 0.00808 | 0.23 |
| CDH7 | 4362 | 9950064 | A | 0.10 | 0.00808 | 2.70 |
| CDH7 | 4364 | 9945997 | A | 0.10 | 0.00808 | 2.49 |
| CDH7 | 4366 | 1976073 | C | 0.07 | 0.03146 | 0.29 |
| CDH7 | 4367 | 2587438 | C | 0.07 | 0.03146 | 3.36 |
| CDH7 | 4368 | 205084 | C | 0.07 | 0.03146 | 0.29 |
| PLCB1 | 4413 | 8123568 | A | 0.37 | 0.04060 | 2.70 |
| PLCB1 | 4415 | 2235212 | A | 0.37 | 0.02505 | 3.00 |
| PLCB1 | 4425 | 718711 | A | 0.07 | 0.00063 | 0.10 |
| PLCB1 | 4426 | 6133621 | C | 0.07 | 0.00096 | 0.11 |
| KIF16B | 4464 | 2144883 | T | 0.27 | 0.01108 | 0.30 |
| KIF16B | 4465 | 2328019 | C | 0.27 | 0.01108 | 0.30 |
| KIF16B | 4466 | 2144886 | T | 0.33 | 0.02615 | 0.36 |
| KIF16B | 4467 | 2180443 | A | 0.33 | 0.02840 | 0.37 |
| KIF16B | 4468 | 2144891 | A | 0.33 | 0.02840 | 0.37 |
| KIF16B | 4469 | 6111075 | T | 0.33 | 0.02840 | 0.37 |
| KIF16B | 4470 | 6111076 | C | 0.33 | 0.02840 | 0.37 |
| KIF16B | 4471 | 1884598 | G | 0.33 | 0.02840 | 0.37 |
| KIF16B | 4472 | 6034464 | A | 0.33 | 0.02840 | 0.37 |
| KIF16B | 4475 | 7263619 | T | 0.39 | 0.04638 | 2.64 |
| PTPRT | 4501 | 6016688 | T | 0.33 | 0.04203 | 2.80 |
| PTPRT | 4502 | 6124439 | A | 0.40 | 0.03564 | 2.72 |
| ARVCF | 4567 | 1110477 | C | 0.07 | 0.03146 | 0.22 |
| ARVCF | 4568 | 887200 | C | 0.10 | 0.00808 | 2.39 |
| ARVCF | 4570 | 887203 | A | 0.07 | 0.03146 | 2.63 |

| **TABLE 16: Alleles Influencing Discontinuation from Adverse Events for Olanzapine** | | | | | | |
|---|---|---|---|---|---|---|
| **Gene** | **Seq ID** | **NCBI RS #** | **Allele** | **Freq. in Discontinuers** | **P** | **Odds Ratio** |
| RP1-21O18.1 | 10 | 6693853 | T | 0.58 | 0.037160 | 1.99 |
| RP1-21O18.1 | 11 | 6665012 | G | 0.58 | 0.037160 | 1.99 |
| AGBL4-C1orf165 | 38 | 3121518 | A | 0.27 | 0.037710 | 0.47 |
| SEC16B | 126 | 1889981 | T | 0.37 | 0.042350 | 2.06 |
| SEC16B | 127 | 1415085 | A | 0.12 | 0.045540 | 0.39 |
| USH2A | 154 | 7548730 | C | 0.40 | 0.030090 | 2.12 |
| GNG4 | 192 | 10925976 | A | 0.58 | 0.022620 | 2.13 |
| RYR2 | 204 | 4376724 | C | 0.29 | 0.041420 | 0.49 |
| RYR2 | 205 | 3924864 | C | 0.27 | 0.001269 | 3.92 |
| CHRM3 | 226 | 479933 | A | 0.32 | 0.030190 | 2.27 |
| CHRM3 | 227 | 663927 | A | 0.33 | 0.031270 | 2.22 |
| FMN2 | 248 | 11799544 | T | 0.21 | 0.000617 | 5.46 |
| FMN2 | 249 | 10926257 | T | 0.21 | 0.000617 | 5.46 |
| BRE | 321 | 6732163 | G | 0.31 | 0.042990 | 2.14 |
| CRIM1 | 328 | 3770939 | T | 0.42 | 0.029140 | 2.11 |
| PRKCE | 377 | 556650 | T | 0.27 | 0.018600 | 2.58 |
| PRKCE | 387 | 6748375 | C | 0.38 | 0.038590 | 0.50 |
| EPAS1 | 391 | 17034950 | A | 0.44 | 0.011130 | 2.38 |
| ACYP2 | 412 | 918357 | G | 0.63 | 0.015920 | 2.25 |
| ACYP2 | 413 | 10186140 | C | 0.04 | 0.037230 | 0.23 |
| CCDC85A | 421 | 2869529 | C | 0.48 | 0.042890 | 1.97 |
| CCDC85A | 423 | 214047 | C | 0.04 | 0.028760 | 0.22 |
| CCDC85A | 424 | 214041 | T | 0.58 | 0.003955 | 2.60 |
| CCDC85A | 426 | 1990758 | C | 0.25 | 0.016630 | 2.71 |
| CTNNA2 | 449 | 2974169 | C | 0.33 | 0.016640 | 0.44 |
| CTNNA2 | 450 | 2916519 | T | 0.33 | 0.021340 | 0.46 |
| NAP5 | 487 | 11695364 | T | 0.38 | 0.011400 | 2.45 |
| LRP1B | 517 | 11681928 | A | 0.42 | 0.029140 | 2.11 |
| LRP I B | 522 | 11691595 | G | 0.44 | 0.036940 | 2.03 |
| ARHGAP15 | 537 | 354714 | T | 0.31 | 0.030730 | 0.47 |
| ARHGAP15 | 548 | 2381466 | A | 0.26 | 0.022920 | 0.44 |
| ARHGAP15 | 559 | 16823114 | A | 0.19 | 0.006185 | 0.35 |
| ARHGAP15 | 560 | 10928200 | T | 0.19 | 0.008093 | 0.36 |
| ARHGAP15 | 561 | 13031917 | C | 0.19 | 0.008668 | 0.36 |
| CACNB4 | 566 | 6736449 | C | 0.60 | 0.012000 | 2.30 |
| CACNB4 | 568 | 7582231 | C | 0.60 | 0.026450 | 2.09 |
| KCNJ3 | 591 | 2937609 | C | 0.23 | 0.024360 | 0.42 |
| CERKL | 632 | 895901 | A | 0.48 | 0.009179 | 2.41 |
| NRP2 | 668 | 849540 | T | 0.60 | 0.020530 | 2.16 |
| NRP2 | 669 | 849538 | T | 0.58 | 0.022620 | 2.13 |
| NRP2 | 670 | 849526 | A | 0.37 | 0.043550 | 0.51 |
| NRP2 | 671 | 849525 | A | 0.36 | 0.049730 | 0.51 |
| ERBB4 | 678 | 12998535 | T | 0.40 | 0.016090 | 2.31 |
| ERBB4 | 679 | 3828242 | G | 0.40 | 0.019260 | 2.27 |
| ERBB4 | 680 | 1992029 | G | 0.42 | 0.029140 | 2.11 |
| DNER | 714 | 10170426 | T | 0.00 | 0.032350 | 0.00 |
| CNTN6 | 739 | 3732517 | C | 0.19 | 0.044470 | 0.45 |
| CNTN4 | 758 | 17029515 | G | 0.31 | 0.003594 | 0.37 |
| CNTN4 | 759 | 11915265 | T | 0.29 | 0.004547 | 0.36 |
| CNTN4 | 760 | 1153486 | T | 0.33 | 0.021340 | 0.46 |
| CNTN4 | 761 | 1685456 | C | 0.36 | 0.025010 | 0.47 |
| ITPR1 | 774 | 304073 | G | 0.52 | 0.043680 | 2.00 |
| IRAK2 | 786 | 11717636 | C | 0.42 | 0.036420 | 0.50 |
| ATP2B2 | 787 | 241508 | A | 0.56 | 0.049230 | 1.95 |
| ATP2B2 | 791 | 11719939 | G | 0.40 | 0.005456 | 2.66 |
| ATP2B2 | 792 | 6763274 | C | 0.40 | 0.006565 | 2.61 |
| ULK4 | 833 | 10510719 | A | 0.15 | 0.032150 | 0.41 |
| FLNB | 907 | 9834312 | A | 0.18 | 0.039760 | 0.43 |
| FHIT | 920 | 9311767 | G | 0.56 | 0.001187 | 2.97 |
| FHIT | 930 | 12638466 | G | 0.42 | 0.029140 | 2.11 |
| PRICKLE2 | 967 | 696027 | T | 0.37 | 0.016110 | 2.37 |
| PLCXD2 | 1010 | 1513331 | C | 0.21 | 0.049150 | 2.37 |
| STXBP5L | 1029 | 2331538 | T | 0.35 | 0.024240 | 0.47 |
| STXBP5L | 1031 | 17249426 | A | 0.33 | 0.031120 | 2.27 |
| STXBP5L | 1032 | 1108170 | T | 0.35 | 0.032280 | 0.47 |
| STXBP5L | 1036 | 2169302 | G | 0.21 | 0.049150 | 2.37 |
| KALRN | 1039 | 9880957 | G | 0.35 | 0.014620 | 0.44 |
| KALRN | 1042 | 661798 | C | 0.62 | 0.018480 | 2.19 |
| CPNE4 | 1049 | 6792146 | A | 0.21 | 0.022010 | 2.81 |
| CPNE4 | 1051 | 6808429 | C | 0.23 | 0.004598 | 3.54 |
| CPNE4 | 1052 | 6802747 | G | 0.13 | 0.039290 | 3.16 |
| CPNE4 | 1058 | 6806898 | T | 0.27 | 0.013610 | 2.73 |
| RAB6B | 1061 | 6765093 | G | 0.50 | 0.040170 | 1.98 |
| SERPINI2 | 1088 | 9841174 | C | 0.54 | 0.044170 | 1.94 |
| SERPINI2 | 1089 | 17246389 | C | 0.44 | 0.003797 | 2.71 |
| TNIK | 1099 | 4955765 | G | 0.65 | 0.008359 | 2.44 |
| PLD1 | 1105 | 4894760 | T | 0.40 | 0.016090 | 2.31 |
| NLGN1 | 1118 | 9878812 | C | 0.04 | 0.031720 | 0.22 |
| HTR3C | 1131 | 6443938 | A | 0.50 | 0.001657 | 2.94 |
| IL1RAP | 1144 | 6788050 | G | 0.10 | 0.026700 | 4.63 |
| SLC2A9 | 1157 | 1568318 | C | 0.13 | 0.028860 | 0.38 |
| SLC2A9 | 1158 | 938563 | G | 0.14 | 0.030440 | 0.39 |
| SLC2A9 | 1160 | 4529048 | C | 0.12 | 0.004553 | 0.28 |
| SLC2A9 | 1163 | 7669607 | T | 0.12 | 0.013260 | 0.32 |
| SLC2A9 | 1164 | 10023068 | A | 0.06 | 0.003129 | 0.18 |
| SLC2A9 | 1165 | 9291640 | C | 0.06 | 0.003129 | 0.18 |
| SLC2A9 | 1166 | 9291642 | C | 0.06 | 0.027060 | 0.27 |
| SLC2A9 | 1167 | 12509955 | T | 0.13 | 0.028860 | 0.38 |
| PPARGC1A | 1193 | 8192678 | A | 0.44 | 0.036940 | 2.03 |
| CCKAR | 1197 | 2040342 | C | 0.33 | 0.000003 | 7.29 |
| CCKAR | 1198 | 2130250 | A | 0.44 | 0.005535 | 2.59 |
| KIAA1239 | 1210 | 11727634 | C | 0.28 | 0.017050 | 2.63 |
| LIMCH1 | 1226 | 4861131 | T | 0.20 | 0.049210 | 0.46 |
| NPFFR2 | 1235 | 11936367 | G | 0.48 | 0.001589 | 2.98 |
| CXCL9 | 1253 | 2869460 | A | 0.08 | 0.027240 | 0.30 |
| FAM13A1 | 1288 | 1996139 | A | 0.13 | 0.019840 | 3.83 |
| FAM13A1 | 1289 | 1513811 | T | 0.13 | 0.039290 | 3.16 |
| COL25A1 | 1319 | 13104307 | T | 0.23 | 0.004186 | 0.35 |
| COL25A1 | 1335 | 7675657 | A | 0.19 | 0.035530 | 0.44 |
| ANK2 | 1339 | 313941 | G | 0.04 | 0.025670 | 2.98 |
| ANK2 | 1346 | 11942005 | G | 0.27 | 0.018600 | 2.58 |
| ANK2 | 1351 | 29306 | G | 0.13 | 0.013790 | 0.34 |
| ANK2 | 1353 | 29311 | A | 0.12 | 0.008824 | 0.30 |
| NDST3 | 1372 | 2389498 | T | 0.06 | 0.027060 | 0.27 |
| NDST3 | 1373 | 4558930 | C | 0.06 | 0.044670 | 0.30 |
| MAML3 | 1399 | 7698089 | T | 0.25 | 0.003842 | 0.35 |
| MAML3 | 1400 | 7656823 | A | 0.25 | 0.003842 | 0.35 |
| MAML3 | 1401 | 4863720 | C | 0.25 | 0.007220 | 0.38 |
| IL15 | 1405 | 10519613 | A | 0.21 | 0.049150 | 2.37 |
| FSTL5 | 1452 | 4501178 | C | 0.35 | 0.015790 | 2.42 |
| FSTL5 | 1462 | 17459658 | C | 0.04 | 0.014570 | 0.19 |
| FSTL5 | 1466 | 13110363 | G | 0.21 | 0.011500 | 3.17 |
| FSTL5 | 1469 | 7375994 | A | 0.31 | 0.005990 | 2.90 |
| FSTL5 | 1470 | 13113862 | A | 0.27 | 0.022870 | 2.55 |
| FSTL5 | 1473 | 4691039 | T | 0.31 | 0.041440 | 2.19 |
| AHRR | 1530 | 3734145 | T | 0.42 | 0.025630 | 2.15 |
| DNAH5 | 1548 | 6862469 | C | 0.60 | 0.011470 | 2.32 |
| DNAH5 | 1549 | 6876673 | T | 0.58 | 0.017380 | 2.20 |
| MYO10 | 1569 | 388887 | T | 0.30 | 0.026150 | 0.46 |
| CDH10 | 1575 | 3822429 | T | 0.29 | 0.012420 | 0.42 |
| SLC1A3 | 1585 | 6878201 | T | 0.31 | 0.042990 | 2.14 |
| ITGA1 | 1599 | 13189973 | T | 0.08 | 0.002099 | 0.21 |
| ITGAI | 1600 | 4865747 | T | 0.04 | 0.004332 | 0.15 |
| ITGA1 | 1602 | 17209947 | A | 0.04 | 0.005148 | 0.15 |
| PDE4D | 1622 | 10461657 | G | 0.04 | 0.026570 | 0.21 |
| PDE4D | 1631 | 294492 | T | 0.17 | 0.005492 | 4.26 |
| PDE4D | 1632 | 294494 | A | 0.33 | 0.013880 | 2.53 |
| PDE4D | 1634 | 295955 | G | 0.17 | 0.021490 | 3.14 |
| PDE4D | 1635 | 295963 | C | 0.17 | 0.023690 | 3.09 |
| GPR98 | 1682 | 168743 | G | 0.17 | 0.045620 | 0.44 |
| FBXL17 | 1705 | 12515105 | C | 0.32 | 0.023730 | 0.46 |
| FBXL17 | 1716 | 6875297 | C | 0.67 | 0.038610 | 2.06 |
| FBXL17 | 1717 | 4957554 | C | 0.16 | 0.000345 | 0.23 |
| FBXL17 | 1718 | 2024528 | A | 0.65 | 0.012500 | 2.41 |
| FBXL17 | 1719 | 2193976 | T | 0.63 | 0.021020 | 2.17 |
| SEMA6A | 1734 | 34971 | G | 0.44 | 0.039310 | 2.02 |
| SNCAIP | 1750 | 17149128 | G | 0.23 | 0.044370 | 2.36 |
| ODZ2 | 1794 | 10055910 | T | 0.35 | 0.015790 | 2.42 |
| GABRP | 1803 | 11134650 | C | 0.19 | 0.008509 | 3.57 |
| ATXN1 | 1835 | 235147 | A | 0.32 | 0.030020 | 0.47 |
| RNF144B | 1839 | 1408267 | G | 0.10 | 0.033690 | 0.35 |
| BTN3A3 | 1874 | 2237236 | G | 0.23 | 0.009517 | 0.39 |
| BTNL2 | 1876 | 3806156 | T | 0.23 | 0.012340 | 0.40 |
| KLC4 | 1883 | 3793017 | C | 0.37 | 0.022690 | 2.26 |
| SLC22A16 | 1931 | 17071722 | T | 0.22 | 0.026790 | 2.68 |
| SLC22A16 | 1932 | 7768422 | C | 0.21 | 0.031910 | 2.59 |
| TRDN | 1949 | 2317707 | T | 0.27 | 0.028460 | 0.46 |
| NKAIN2 | 1954 | 342623 | T | 0.25 | 0.021300 | 2.62 |
| NKAIN2 | 1965 | 9388339 | A | 0.33 | 0.035850 | 2.18 |
| PLAGL1 | 1990 | 9321951 | T | 0.27 | 0.037250 | 0.48 |
| SYNE1 | 2005 | 2763015 | A | 0.62 | 0.038590 | 1.99 |
| CARD11 | 2068 | 3735131 | C | 0.10 | 0.032970 | 4.41 |
| CREB5 | 2120 | 7791555 | G | 0.16 | 0.043170 | 0.42 |
| ABCA13 | 2194 | 17132289 | T | 0.02 | 0.044780 | 0.16 |
| ABCA13 | 2195 | 7802110 | G | 0.16 | 0.021480 | 0.38 |
| ABCA13 | 2197 | 6966506 | A | 0.13 | 0.022690 | 0.37 |
| ABCA13 | 2199 | 7778020 | A | 0.04 | 0.037230 | 0.23 |
| LIMK1 | 2249 | 810532 | G | 0.10 | 0.049050 | 0.37 |
| MAGI2 | 2271 | 10238177 | A | 0.25 | 0.024210 | 0.44 |
| MAGI2 | 2295 | 848942 | C | 0.52 | 0.044370 | 1.97 |
| PCLO | 2321 | 2715148 | C | 0.40 | 0.032390 | 0.49 |
| PCLO | 2325 | 2888019 | T | 0.60 | 0.030960 | 2.09 |
| PCLO | 2326 | 1986742 | G | 0.60 | 0.033770 | 2.02 |
| CUX1 | 2400 | 940482 | T | 0.06 | 0.012400 | 0.23 |
| KCND2 | 2412 | 802372 | C | 0.04 | 0.048060 | 0.24 |
| GRM8 | 2439 | 2283061 | G | 0.56 | 0.014440 | 2.25 |
| EXOC4 | 2453 | 7786489 | A | 0.12 | 0.044870 | 0.39 |
| PTPRN2 | 2516 | 221242 | A | 0.16 | 0.040950 | 2.86 |
| PTPRN2 | 2523 | 10277112 | A | 0.17 | 0.007146 | 0.32 |
| PTPRN2 | 2534 | 10266729 | C | 0.62 | 0.047940 | 1.94 |
| MCPH1 | 2560 | 2442497 | G | 0.21 | 0.019710 | 2.85 |
| SGCZ | 2579 | 9325685 | C | 0.30 | 0.007190 | 0.39 |
| PSD3 | 2613 | 3758141 | C | 0.27 | 0.007317 | 3.00 |
| LYN | 2674 | 2719268 | G | 0.35 | 0.021410 | 2.33 |
| NKAIN3 | 2705 | 975688 | T | 0.23 | 0.040790 | 0.47 |
| DEPDC2 | 2728 | 1434764 | A | 0.25 | 0.019020 | 0.43 |
| CSMD3 | 2815 | 4354335 | G | 0.40 | 0.021160 | 2.29 |
| CSMD3 | 2816 | 1513524 | C | 0.35 | 0.042990 | 2.08 |
| CSMD3 | 2817 | 2954892 | G | 0.35 | 0.042990 | 2.08 |
| FBXO32 | 2830 | 6990407 | G | 0.04 | 0.048060 | 0.24 |
| MTSS1 | 2853 | 9771920 | A | 0.21 | 0.012920 | 3.11 |
| MTSS1 | 2854 | 4870913 | T | 0.21 | 0.035420 | 2.55 |
| DDEF1 | 2861 | 6984815 | A | 0.21 | 0.027150 | 0.43 |
| DDEF1 | 2862 | 12546206 | A | 0.46 | 0.045530 | 1.96 |
| DDEF1 | 2877 | 1123205 | G | 0.50 | 0.013310 | 2.41 |
| ADCY8 | 2891 | 7464362 | G | 0.48 | 0.020830 | 2.18 |
| ADCY8 | 2892 | 11781997 | A | 0.65 | 0.003414 | 2.69 |
| ADCY8 | 2893 | 7459573 | G | 0.54 | 0.034640 | 2.01 |
| ADCY8 | 2894 | 4736722 | C | 0.33 | 0.034380 | 0.49 |
| PTPRD | 2953 | 4742533 | C | 0.38 | 0.020570 | 2.29 |
| PTPRD | 2954 | 10815933 | C | 0.37 | 0.031300 | 2.15 |
| K1AA1797 | 2959 | 12336110 | A | 0.35 | 0.018390 | 2.37 |
| KIAA1797 | 2960 | 7848159 | G | 0.04 | 0.028760 | 0.22 |
| PIP5K1B | 2995 | 963707 | G | 0.44 | 0.036940 | 2.03 |
| PIP5K1B | 2998 | 1541082 | A | 0.02 | 0.018550 | 0.12 |
| TMC1 | 3032 | 13285999 | A | 0.62 | 0.014200 | 2.26 |
| NTRK2 | 3071 | 11140721 | C | 0.15 | 0.029140 | 3.14 |
| ZNF618 | 3134 | 2269454 | G | 0.40 | 0.005456 | 2.66 |
| LHX6 | 3149 | 7849627 | G | 0.50 | 0.040170 | 1.98 |
| ABL1 | 3165 | 2855160 | T | 0.27 | 0.046630 | 0.49 |
| EXOSC2 | 3166 | 4740366 | G | 0.10 | 0.033690 | 0.35 |
| ABL1 | 3172 | 10901297 | A | 0.13 | 0.045020 | 3.34 |
| MYO3A | 3267 | 993397 | A | 0.56 | 0.005913 | 2.49 |
| MYO3A | 3277 | 2096176 | T | 0.29 | 0.026170 | 0.46 |
| MYO3A | 3280 | 1999240 | C | 0.56 | 0.031870 | 2.03 |
| MYO3A | 3281 | 3758447 | C | 0.29 | 0.045580 | 0.49 |
| MYO3A | 3289 | 2050440 | A | 0.35 | 0.030820 | 0.48 |
| SLC16A9 | 3291 | 1171648 | A | 0.12 | 0.017090 | 0.33 |
| SLC16A9 | 3292 | 1171643 | G | 0.12 | 0.021930 | 0.35 |
| JMJD1C | 3298 | 10740118 | C | 0.50 | 0.013410 | 2.28 |
| JMJD1C | 3301 | 9629895 | A | 0.10 | 0.020610 | 0.32 |
| JMJD1C | 3302 | 3999089 | C | 0.54 | 0.032810 | 2.03 |
| CDH23 | 3314 | 4747197 | T | 0.46 | 0.004661 | 2.63 |
| CDH23 | 3315 | 2070968 | A | 0.31 | 0.019160 | 2.46 |
| NRG3 | 3339 | 17099789 | G | 0.06 | 0.019680 | 0.25 |
| NRG3 | 3341 | 7902584 | G | 0.02 | 0.015140 | 0.12 |
| NRG3 | 3345 | 474496 | G | 0.46 | 0.013080 | 2.32 |
| NRG3 | 3347 | 495978 | A | 0.42 | 0.045280 | 1.98 |
| ATRNL1 | 3420 | 10885833 | C | 0.17 | 0.040800 | 2.72 |
| ATRNL1 | 3421 | 180675 | A | 0.28 | 0.029530 | 2.38 |
| ATRNL1 | 3422 | 180635 | T | 0.35 | 0.042990 | 2.08 |
| ATE1 | 3431 | 1693688 | C | 0.54 | 0.011740 | 2.31 |
| ATE1 | 3433 | 7086628 | C | 0.62 | 0.013770 | 2.28 |
| ATE1 | 3434 | 2132902 | T | 0.54 | 0.015650 | 2.23 |
| ATE1 | 3435 | 1219505 | T | 0.58 | 0.017380 | 2.20 |
| MICAL2 | 3472 | 2015963 | T | 0.58 | 0.037160 | 1.99 |
| 81.99 mb | 3523 | 2186693 | T | 0.17 | 0.015130 | 0.38 |
| DLG2 | 3533 | 10898148 | A | 0.32 | 0.016760 | 2.49 |
| DLG2 | 3534 | 7950988 | T | 0.31 | 0.039880 | 2.18 |
| DLG2 | 3549 | 11234225 | T | 0.58 | 0.011850 | 2.31 |
| DLG2 | 3550 | 6592211 | A | 0.60 | 0.008591 | 2.40 |
| DLG2 | 3551 | 7101454 | C | 0.56 | 0.030440 | 2.05 |
| OPCML | 3573 | 11223425 | A | 0.27 | 0.028460 | 0.46 |
| OPCML | 3574 | 4937764 | T | 0.67 | 0:034380 | 2.06 |
| OPCML | 3575 | 7107264 | C | 0.21 | 0.019710 | 2.85 |
| OPCML | 3576 | 1941219 | G | 0.23 | 0.023090 | 2.65 |
| TMEM16B | 3598 | 16933813 | A | 0.18 | 0.016520 | 3.29 |
| TMEM16B | 3602 | 12578500 | A | 0.17 | 0.021490 | 3.14 |
| STYK1 | 3609 | 1078437 | G | 0.56 | 0.031990 | 2.05 |
| STYK1 | 3613 | 6488316 | T | 0.62 | 0.007691 | 2.46 |
| STYK1 | 3614 | 7350597 | A | 0.60 | 0.008762 | 2.42 |
| STYK1 | 3615 | 10845198 | T | 0.54 | 0.025310 | 2.10 |
| STYK1 | 3616 | 10743918 | C | 0.22 | 0.010240 | 0.38 |
| CNOT2 | 3641 | 2118427 | T | 0.19 | 0.043910 | 2.53 |
| CNOT2 | 3648 | 10506586 | A | 0.15 | 0.007767 | 4.40 |
| CNOT2 | 3649 | 2870879 | G | 0.15 | 0.016660 | 3.64 |
| CNOT2 | 3650 | 3817486 | G | 0.15 | 0.029140 | 3.14 |
| NAV3 | 3676 | 392085 | G | 0.08 | 0.030900 | 0.31 |
| SLC46A3 | 3695 | 952648 | G | 0.31 | 0.047380 | 0.50 |
| NBEA | 3722 | 3818423 | C | 0.06 | 0.034830 | 0.28 |
| FNDC3A | 3733 | 9535073 | C | 0.30 | 0.030100 | 2.31 |
| FNDC3A | 3734 | 7321178 | G | 0.37 | 0.048800 | 2.02 |
| PCDH20 | 3738 | 3812872 | C | 0.38 | 0.016200 | 2.34 |
| GPC5 | 3771 | 553717 | A | 0.21 | 0.006283 | 3.55 |
| GPC5 | 3772 | 342678 | A | 0.21 | 0.007109 | 3.49 |
| NALCN | 3834 | 499685 | T | 0.56 | 0.031870 | 2.03 |
| ITGBL1 | 3847 | 1436261 | C | 0.10 | 0.042770 | 0.36 |
| ITGBL1 | 3851 | 1469855 | G | 0.27 | 0.045860 | 2.21 |
| ITGBL1 | 3852 | 1469853 | G | 0.27 | 0.045860 | 2.21 |
| NPAS3 | 3870 | 1315139 | T | 0.56 | 0.017950 | 2.19 |
| LRFNS | 3895 | 1597045 | T | 0.40 | 0.009054 | 2.57 |
| LRFNS | 3896 | 8015480 | C | 0.42 | 0.006701 | 2.57 |
| GNG2 | 3907 | 4898721 | C | 0.29 | 0.015270 | 2.60 |
| RGS6 | 3941 | 2238199 | T | 0.56 | 0.047580 | 1.95 |
| RGS6 | 3943 | 2214965 | T | 0.52 | 0.029010 | 2.06 |
| RGS6 | 3951 | 2239219 | C | 0.60 | 0.041900 | 1.97 |
| RGS6 | 3952 | 2238192 | A | 0.54 | 0.020630 | 2.15 |
| PSMC1 | 3971 | 6575121 | C | 0.13 | 0.039290 | 3.16 |
| RPS6KA5 | 3973 | 941847 | T | 0.14 | 0.005797 | 0.30 |
| RPS6KA5 | 3978 | 1075014 | C | 0.23 | 0.020310 | 0.42 |
| RPS6KA5 | 3979 | 11159988 | C | 0.23 | 0.020310 | 0.42 |
| BCL11B | 3998 | 807450 | C | 0.19 | 0.044470 | 0.45 |
| RYR3 | 4028 | 16957065 | G | 0.52 | 0.025210 | 2.11 |
| PCSK6 | 4109 | 755867 | A | 0.10 | 0.026420 | 0.33 |
| A2BP1 | 4117 | 8059039 | T | 0.30 | 0.016110 | 2.60 |
| A2BP1 | 4134 | 7191740 | C | 0.08 | 0.014440 | 0.27 |
| A2BP1 | 4137 | 8054765 | G | 0.15 | 0.025300 | 0.39 |
| MPHOSPH6 | 4173 | 2967391 | G | 0.06 | 0.005492 | 0.20 |
| MPHOSPH6 | 4174 | 2967340 | C | 0.06 | 0.007233 | 0.21 |
| MPHOSPH6 | 4176 | 902542 | A | 0.06 | 0.008486 | 0.21 |
| MPHOSPH6 | 4177 | 2967337 | C | 0.06 | 0.020940 | 0.25 |
| MPHOSPH6 | 4179 | 2967333 | G | 0.08 | 0.021700 | 0.29 |
| MPHOSPH6 | 4180 | 2967328 | C | 0.08 | 0.024080 | 0.30 |
| CDH13 | 4186 | 4598906 | T | 0.23 | 0.040790 | 0.47 |
| CDH13 | 4193 | 1424189 | T | 0.58 | 0.005441 | 2.52 |
| CDH13 | 4195 | 723920 | A | 0.58 | 0.028050 | 2.07 |
| CDH13 | 4196 | 10514579 | C | 0.43 | 0.043520 | 2.04 |
| CDH13 | 4198 | 10514577 | G | 0.58 | 0.035950 | 2.01 |
| DNAH17 | 4266 | 11649879 | T | 0.02 | 0.036340 | 0.15 |
| KIAA0802 | 4305 | 17407982 | C | 0.10 | 0.033790 | 0.35 |
| CHST9 | 4316 | 1426879 | C | 0.18 | 0.010190 | 0.36 |
| CHST9 | 4317 | 4800797 | G | 0.19 | 0.017440 | 0.40 |
| CDH7 | 4350 | 4455070 | T | 0.58 | 0.003955 | 2.60 |
| CDH7 | 4351 | 976882 | A | 0.58 | 0.003955 | 2.60 |
| CDH7 | 4352 | 12970791 | T | 0.58 | 0.003955 | 2.60 |
| PRNT | 4398 | 2065704 | A | 0.27 | 0.017580 | 0.43 |
| PRNT | 4402 | 6107527 | G | 0.60 | 0.003612 | 2.63 |
| PRNT | 4404 | 6052833 | C | 0.27 | 0.017580 | 0.43 |
| PRNT | 4407 | 6084876 | T | 0.28 | 0.034420 | 0.47 |
| PLCB1 | 4422 | 6039249 | G | 0.38 | 0.041780 | 2.05 |
| JAG1 | 4439 | 3748477 | T | 0.08 | 0.032450 | 5.48 |
| JAG1 | 4440 | 3748478 | T | 0.08 | 0.036660 | 5.30 |
| MACROD2 | 4448 | 459322 | G | 0.29 | 0.027170 | 0.45 |
| MACROD2 | 4449 | 459874 | C | 0.31 | 0.037940 | 0.49 |
| MACROD2 | 4453 | 175805 | T | 0.14 | 0.030440 | 0.39 |
| MACROD2 | 4454 | 2236008 | T | 0.15 | 0.022950 | 0.39 |
| KIF16B | 4482 | 7352372 | G | 0.35 | 0.015790 | 2.42 |
| KIF16B | 4483 | 6075069 | T | 0.29 | 0.029600 | 2.33 |
| PTPRT | 4497 | 6065432 | T | 0.15 | 0.046150 | 0.43 |
| PTPRT | 4500 | 6065434 | T | 0.19 | 0.047650 | 0.45 |
| PTPRT | 4501 | 6016688 | T | 0.12 | 0.027980 | 0.36 |
| SNAI1 | 4524 | 6020157 | A | 0.04 | 0.006507 | 0.16 |
| SNAI1 | 4525 | 4647957 | T | 0.04 | 0.044900 | 0.24 |
| SNAI1 | 4527 | 1543442 | A | 0.46 | 0.045210 | 1.97 |
| CDH4 | 4530 | 4812313 | G | 0.17 | 0.045620 | 0.44 |
| NCAM2 | 4551 | 986917 | A | 0.27 | 0.022450 | 0.44 |
| TTLL1 | 4593 | 5996268 | C | 0.10 | 0.031900 | 4.43 |

| **TABLE 17: Alleles Influencing Discontinuation from Adverse Events for Risperidone** | | | | | | |
|---|---|---|---|---|---|---|
| **Gene Name** | **Seq ID** | **NCBI RS#** | **Allele** | **Freq. in Discontinuers** | **P** | **Odds Ratio** |
| ELOVL5 | 1895 | 9474507 | A | 0.40 | 0.00003 | 5.77 |
| ELOVL5 | 1890 | 974323 | T | 0.43 | 0.01484 | 2.68 |
| ELOVL5 | 1893 | 9357760 | G | 0.43 | 0.01677 | 2.63 |
| ELOVL5 | 1894 | 9370196 | C | 0.43 | 0.02108 | 2.54 |
| ELOVL5 | 1897 | 9367529 | G | 0.43 | 0.01027 | 2.82 |
| EPHB2 | 22 | 309500 | T | 0.77 | 0.00038 | 4.64 |
| EPHB2 | 23 | 309499 | T | 0.73 | 0.00064 | 4.19 |
| AGBL4-C1orf165 | 24 | 319965 | A | 0.63 | 0.01910 | 2.57 |
| SCP2 | 39 | 7528867 | A | 0.17 | 0.00230 | 6.20 |
| KCNK2 | 141 | 2123331 | G | 0.23 | 0.01638 | 0.34 |
| KCNK2 | 142 | 12038094 | T | 0.20 | 0.01409 | 0.32 |
| KCNK2 | 144 | 4655272 | A | 0.43 | 0.02761 | 0.42 |
| KCNK2 | 146 | 12039875 | A | 0.23 | 0.02608 | 0.37 |
| KCNK2 | 147 | 2363564 | C | 0.23 | 0.01638 | 0.34 |
| KCNK2 | 148 | 10779647 | T | 0.23 | 0.02241 | 0.36 |
| USH2A | 158 | 301760 | C | 0.20 | 0.04605 | 0.39 |
| ESRRG | 177 | 10218694 | A | 0.53 | 0.02252 | 2.47 |
| ESRRG | 178 | 12088947 | A | 0.53 | 0.02705 | 2.39 |
| RYR2 | 200 | 2275287 | A | 0.50 | 0.04452 | 2.22 |
| RGS7 | 261 | 12038803 | A | 0.07 | 0.00676 | 0.16 |
| PLD5 | 267 | 924775 | A | 0.07 | 0.03049 | 0.22 |
| C2orf46 | 278 | 10168400 | A | 0.20 | 0.02607 | 0.35 |
| DDEF2 | 281 | 2666218 | G | 0.46 | 0.04821 | 2.38 |
| KCNK3 | 306 | 1275982 | C | 0.17 | 0.02448 | 0.33 |
| PRKCE | 382 | 4953288 | T | 0.20 | 0.04436 | 0.39 |
| PRKCE | 385 | 2595222 | A | 0.50 | 0.04981 | 2.18 |
| PRKCE | 389 | 1020445 | C | 0.13 | 0:02699 | 0.30 |
| ACYP2 | 417 | 17045754 | C | 0.23 | 0.04732 | 2.63 |
| CTNNA2 | 444 | 1971766 | A | 0.46 | 0.03393 | 2.39 |
| NAP5 | 486 | 7559239 | T | 0.23 | 0.03500 | 0.39 |
| LRP1B | 506 | 825429 | C | 0.70 | 0.02783 | 2.51 |
| KYNU | 531 | 6429997 | C | 0.20 | 0.04605 | 0.39 |
| FMNL2 | 579 | 2346182 | G | 0.17 | 0.02911 | 0.34 |
| FMNL2 | 580 | 2577180 | G | 0.07 | 0.03477 | 0.23 |
| PKP4 | 595 | 4664966 | A | 0.23 | 0.04641 | 0.41 |
| PLA2R1 | 602 | 3828323 | C | 0.73 | 0.01120 | 2.96 |
| PLA2R1 | 605 | 3828324 | C | 0.27 | 0.01331 | 0.35 |
| PLA2R1 | 607 | 2715921 | G | 0.27 | 0.04121 | 2.58 |
| PLA2R1 | 608 | 2667011 | G | 0.37 | 0.00620 | 3.17 |
| PLA2R1 | 609 | 2667012 | C | 0.70 | 0.00320 | 3.38 |
| PLA2R1 | 610 | 2715936 | G | 0.50 | 0.00093 | 3.85 |
| PLA2R1 | 613 | 4665145 | T | 0.40 | 0.01053 | 2.86 |
| PLA2R1 | 614 | 16844715 | T | 0.17 | 0.01169 | 0.29 |
| SCN9A | 625 | 6746030 | A | 0.30 | 0.01662 | 2.92 |
| SCN9A | 626 | 2226711 | C | 0.30 | 0.01662 | 2.92 |
| COL4A3 | 706 | 4619602 | C | 0.27 | 0.03951 | 0.41 |
| COL4A3 | 707 | 2070735 | A | 0.20 | 0.01791 | 3.48 |
| COL4A3 | 708 | 7587228 | C | 0.17 | 0.03155 | 3.40 |
| COL4A3 | 710 | 11681636 | C | 0.27 | 0.04554 | 0.42 |
| CNTN6 | 733 | 11919853 | C | 0.47 | 0.04077 | 2.27 |
| CNTN6 | 736 | 11925058 | C | 0.63 | 0.00087 | 3.76 |
| CNTN4 | 772 | 1039255 | C | 0.67 | 0.03408 | 2.39 |
| ATP2B2 | 790 | 11710165 | C | 0.10 | 0.01529 | 0.24 |
| ATP2B2 | 793 | 34923 | G | 0.07 | 0.00416 | 0.15 |
| RARB | 804 | 1286654 | T | 0.50 | 0.04190 | 2.24 |
| RARB | 807 | 17526942 | T | 0.20 | 0.02770 | 3.17 |
| ZNF445 | 854 | 6766334 | C | 0.17 | 0.00844 | 0.28 |
| CACNA2D3 | 866 | 9825455 | A | 0.07 | 0.01258 | 0.18 |
| FHIT | 915 | 2630167 | G | 0.20 | 0.04080 | 2.90 |
| FHIT | 916 | 212016 | C | 0.33 | 0.04923 | 2.33 |
| CADPS | 944 | 9311842 | T | 0.37 | 0.04663 | 2.30 |
| CADPS | 945 | 1148827 | A | 0.17 | 0.03827 | 3.27 |
| CADPS | 948 | 17357618 | C | 0.40 | 0.03887 | 2.33 |
| SYNPR | 954 | 1505600 | C | 0.33 | 0.04270 | 2.39 |
| SYNPR | 955 | 4616634 | G | 0.33 | 0.04923 | 2.33 |
| FAM19A1 | 983 | 9310118 | G | 0.50 | 0.03501 | 2.31 |
| GBE1 | 988 | 11922101 | T | 0.13 | 0.00579 | 6.15 |
| GBE1 | 997 | 3755713 | C | 0.11 | 0.02588 | 0.26 |
| EPHA6 | 1005 | 4621347 | A | 0.10 | 0.04537 | 4.33 |
| CDGAP | 1023 | 9855065 | A | 0.30 | 0.01662 | 2.92 |
| KALRN | 1041 | 17289013 | T | 0.57 | 0.01923 | 2.52 |
| KALRN | 1042 | 661798 | C | 0.70 | 0.02783 | 2.51 |
| KALRN | 1043 | 3772756 | G | 0.21 | 0.01221 | 3.74 |
| RAB6B | 1061 | 6765093 | G | 0.27 | 0.03951 | 0.41 |
| NLGN1 | 1122 | 10936780 | G | 0.23 | 0.03707 | 2.78 |
| NLGN1 | 1127 | 9828801 | T | 0.20 | 0.04080 | 2.90 |
| PPARGC1A | 1193 | 8192678 | A | 0.13 | 0.01734 | 0.28 |
| CCKAR | 1198 | 2130250 | A | 0.13 | 0.01090 | 0.26 |
| KIAA1239 | 1206 | 17575883 | A | 0.30 | 0.04286 | 2.46 |
| KIAA1239 | 1208 | 2928297 | A | 0.77 | 0.00182 | 3.91 |
| KIAA1239 | 1209 | 2973226 | C | 0.70 | 0.01451 | 2.77 |
| KIAA1239 | 1211 | 1382979 | A | 0.50 | 0.00546 | 3.00 |
| NPFFR2 | 1247 | 4694116 | A | 0.10 | 0.00095 | 17.89 |
| NPFFR2 | 1251 | 1513895 | T | 0.10 | 0.00526 | 9.00 |
| SHROOM3 | 1260 | 9307184 | G | 0.57 | 0.01387 | 2.72 |
| HERC3 | 1274 | 4312724 | C | 0.40 | 0.04325 | 2.29 |
| CAMK2D | 1365 | 10488959 | C | 0.00 | 0.04342 | 0.00 |
| CAMK2D | 1367 | 7680434 | T | 0.00 | 0.04342 | 0.00 |
| GPR103 | 1383 | 7688947 | C | 0.43 | 0.01320 | 2.72 |
| GPR103 | 1384 | 11732033 | A | 0.43 | 0.01320 | 2.72 |
| GPR103 | 1385 | 17438900 | C | 0.43 | 0.01320 | 2.72 |
| MAML3 | 1394 | 2271391 | T | 0.57 | 0.00646 | 2.93 |
| MAML3 | 1399 | 7698089 | T | 0.30 | 0.03766 | 0.42 |
| MAML3 | 1400 | 7656823 | A | 0.30 | 0.03777 | 0.42 |
| MAML3 | 1401 | 4863720 | C | 0.30 | 0.03799 | 0.42 |
| INPP4B | 1422 | 2130685 | T | 0.30 | 0.01930 | 2.87 |
| POU4F2 | 1428 | 6835151 | A | 0.23 | 0.03442 | 2.82 |
| DCLK2 | 1437 | 10001651 | C | 0.00 | 0.00193 | 0.00 |
| DCLK2 | 1438 | 11735949 | C | 0.13 | 0.03114 | 0.31 |
| DCLK2 | 1439 | 17503402 | A | 0.13 | 0.00133 | 0.19 |
| DCLK2 | 1440 | 11727182 | G | 0.13 | 0.00281 | 0.21 |
| DCLK2 | 1442 | 6535725 | A | 0.20 | 0.01445 | 0.32 |
| TLL1 | 1481 | 1391334 | G | 0.54 | 0.02492 | 2.49 |
| TLL1 | 1482 | 1497947 | T | 0.63 | 0.04990 | 2.21 |
| DNAH5 | 1546 | 1971860 | A | 0.33 | 0.03466 | 0.42 |
| DNAH5 | 1567 | 168131 | G | 0.20 | 0.04011 | 0.38 |
| SLCIA3 | 1584 | 2032892 | C | 0.03 | 0.01907 | 2.39 |
| TNPO1 | 1644 | 2548331 | A | 0.27 | 0.02529 | 0.38 |
| CMYA5 | 1652 | 1129770 | A | 0.30 | 0.01191 | 3.09 |
| VCAN | 1664 | 33612 | C | 0.17 | 0.03863 | 0.36 |
| FBXL17 | 1705 | 12515105 | C | 0.60 | 0.02317 | 2.47 |
| FBXL17 | 1706 | 4957550 | G | 0.40 | 0.03712 | 0.44 |
| SNX2 | 1759 | 12109789 | G | 0.17 | 0.04433 | 0.37 |
| SNX24 | 1764 | 6595416 | T | 0.13 | 0.04980 | 0.34 |
| TRPC7 | 1774 | 17170027 | C | 0.20 | 0.04605 | 0.39 |
| SLC17A3 | 1863 | 629444 | T | 0.20 | 0.04080 | 2.90 |
| | | | | | | |
| SLC22A16 | 1927 | 9320331 | A | 0.17 | 0.03655 | 0.35 |
| SLC22A16 | 1928 | 9481067 | G | 0.30 | 0.02783 | 0.40 |
| SLC22A16 | 1930 | 7740547 | C | 0.07 | 0.04709 | 0.25 |
| TRDN | 1938 | 1367674 | T | 0.03 | 0.04564 | 0.16 |
| TRDN | 1950 | 6915835 | C | 0.12 | 0.04099 | 0.29 |
| NKAIN2 | 1960 | 163295 | A | 0.60 | 0.00945 | 2.81 |
| NKAIN2 | 1963 | 7751055 | A | 0.23 | 0.03500 | 0.39 |
| EYA4 | 1974 | 17301622 | G | 0.71 | 0.02285 | 2.69 |
| EYA4 | 1976 | 7454561 | G | 0.70 | 0.02379 | 2.57 |
| SYNE1 | 2006 | 1844356 | G | 0.63 | 0.02268 | 2.50 |
| TFB1M | 2009 | 3822964 | C | 0.27 | 0.03951 | 0.41 |
| TFB1M | 2012 | 162978 | A | 0.54 | 0.04178 | 2.29 |
| PARK2 | 2025 | 9355924 | A | 0.57 | 0.04551 | 2.21 |
| PARK2 | 2030 | 9295173 | G | 0.30 | 0.03243 | 0.41 |
| CARD11 | 2067 | 3735124 | C | 0.30 | 0.03944 | 2.50 |
| NPY | 2114 | 16129 | T | 0.25 | 0.01422 | 0.33 |
| NPY | 2115 | 16124 | A | 0.29 | 0.02665 | 0.38 |
| CREBS | 2119 | 2237361 | C | 0.30 | 0.03810 | 0.42 |
| CHN2 | 2128 | 7781003 | T | 0.17 | 0.03863 | 0.36 |
| SCRN1 | 2136 | 1465327 | T | 0.43 | 0.01822 | 2.67 |
| PLEKHA8 | 2137 | 10488086 | A | 0.30 | 0.00832 | 3.27 |
| PLEKHA8 | 2138 | 17158623 | G | 0.07 | 0.00089 | 3.48 |
| PLEKHA8 | 2139 | 7806238 | A | 0.27 | 0.03062 | 2.74 |
| FLJ22374 | 2150 | 17159526 | G | 0.25 | 0.04469 | 2.67 |
| FLJ22374 | 2151 | 10253781 | T | 0.32 | 0.00290 | 3.84 |
| BMPER | 2169 | 969363 | C | 0.54 | 0.02311 | 2.59 |
| IGFBP3 | 2189 | 10282088 | A | 0.33 | 0.04664 | 2.36 |
| ABCA13 | 2190 | 17132163 | T | 0.20 | 0.01791 | 3.48 |
| ABCA13 | 2191 | 12113721 | A | 0.20 | 0.01791 | 3.48 |
| WBSCR17 | 2238 | 6971383 | A | 0.18 | 0.02082 | 0.32 |
| PPP1R9A | 2377 | 854532 | T | 0.23 | 0.04641 | 0.41 |
| DYNCII1 | 2397 | 12666041 | A | 0.37 | 0.00854 | 3.03 |
| CADPS2 | 2424 | 2501443 | C | 0.30 | 0.03777 | 0.42 |
| EXOC4 | 2462 | 6966403 | A | 0.20 | 0.03113 | 0.36 |
| EXOC4 | 2466 | 2346442 | A | 0.23 | 0.01663 | 0.34 |
| EXOC4 | 2469 | 10224683 | A | 0.20 | 0.03484 | 0.37 |
| EXOC4 | 2470 | 7792546 | A | 0.20 | 0.03484 | 0.37 |
| DGK1 | 2474 | 1731951 | T | 0.30 | 0.01220 | 0.35 |
| DGKI | 2480 | 12707359 | T | 0.67 | 0.00496 | 3.13 |
| CNTNAP2 | 2503 | 3915305 | C | 0.43 | 0.03940 | 2.29 |
| CNTNAP2 | 2504 | 10952682 | G | 0.43 | 0.03940 | 2.29 |
| CNTNAP2 | 2505 | 4726875 | A | 0.57 | 0.04551 | 2.21 |
| CNTNAP2 | 2506 | 1528507 | G | 0.57 | 0.04551 | 2.21 |
| MCPH1 | 2551 | 1530408 | C | 0.33 | 0.03909 | 2.43 |
| SGCZ | 2579 | 9325685 | C | 0.27 | 0.03499 | 0.40 |
| PSD3 | 2603 | 13275817 | A | 0.43 | 0.04370 | 2.26 |
| PEBP4 | 2629 | 17757261 | G | 0.37 | 0.03754 | 2.39 |
| UNC5D | 2640 | 2579898 | G | 0.50 | 0.03736 | 2.28 |
| SNTG1 | 2652 | 10504093 | G | 0.13 | 0.04839 | 3.45 |
| LYN | 2675 | 868541 | A | 0.65 | 0.02167 | 2.69 |
| LYN | 2680 | 2668005 | G | 0.43 | 0.00771 | 2.94 |
| LYN | 2681 | 2668003 | C | 0.40 | 0.01375 | 2.75 |
| NKAIN3 | 2688 | 16928883 | C | 0.30 | 0.00181 | 4.14 |
| NKAIN3 | 2689 | 16928902 | C | 0.27 | 0.00970 | 3.36 |
| NKAIN3 | 2695 | 1901409 | C | 0.60 | 0.02660 | 2.42 |
| NKAIN3 | 2703 | 16929381 | T | 0.27 | 0.03810 | 2.62 |
| NKAIN3 | 2706 | 4739003 | A | 0.23 | 0.03707 | 2.78 |
| NKAIN3 | 2709 | 1156385 | A | 0.53 | 0.00769 | 2.87 |
| NKAIN3 | 2710 | 1383154 | A | 0.33 | 0.03909 | 2.43 |
| NKAIN3 | 2711 | 10504354 | A | 0.50 | 0.01120 | 2.73 |
| KCNB2 | 2738 | 12541900 | G | 0.63 | 0.02155 | 2.52 |
| KCNB2 | 2741 | 2249770 | C | 0.30 | 0.02264 | 2.77 |
| CRISPLD1 | 2755 | 2383928 | T | 0.20 | 0.04362 | 2.87 |
| CRISPLD1 | 2760 | 10100216 | T | 0.13 | 0.00797 | 0.25 |
| GRHL2 | 2775 | 13275653 | C | 0.57 | 0.02411 | 2.50 |
| GRHL2 | 2777 | 4734573 | C | 0.43 | 0.04370 | 2.26 |
| GRHL2 | 2778 | 11994917 | T | 0.50 | 0.02393 | 2.45 |
| NCALD | 2780 | 2387620 | G | 0.47 | 0.02910 | 2.39 |
| NCALD | 2781 | 2226401 | C | 0.37 | 0.02985 | 2.48 |
| BAALC | 2786 | 3956319 | T | 0.00 | 0.02195 | 0.00 |
| ZFPM2 | 2804 | 16873707 | T | 0.17 | 0.02020 | 3.80 |
| ZFPM2 | 2808 | 1372614 | G | 0.20 | 0.04080 | 2.90 |
| DDEF1 | 2862 | 12546206 | A | 0.23 | 0.04641 | 0.41 |
| DDEF1 | 2877 | 1123205 | G | 0.23 | 0.04553 | 0.41 |
| ADCY8 | 2888 | 2860416 | T | 0.27 | 0.02630 | 2.83 |
| ADCY8 | 2895 | 6986982 | A | 0.03 | 0.01092 | 0.11 |
| ADCY8 | 2896 | 7461448 | A | 0.03 | 0.01268 | 0.11 |
| ADCY8 | 2897 | 17327900 | A | 0.00 | 0.02195 | 0.00 |
| ADCY8 | 2898 | 11991124 | T | 0.03 | 0.01377 | 0.12 |
| ADCY8 | 2899 | 10505569 | G | 0.00 | 0.03313 | 0.00 |
| KCNQ3 | 2900 | 977939 | A | 0.27 | 0.03951 | 0.41 |
| SMARCA2 | 2933 | 10116703 | A | 0.67 | 0.02993 | 2.43 |
| PTPRD | 2951 | 7027844 | C | 0.67 | 0.02137 | 2.56 |
| PTPRD | 2952 | 6477333 | A | 0.60 | 0.01340 | 2.67 |
| ADAMTSL1 | 2957 | 574090 | T | 0.20 | 0.01092 | 3.85 |
| ADAMTSL1 | 2958 | 563805 | C | 0.23 | 0.01647 | 3.26 |
| IFT74 | 2983 | 3429 | A | 0.00 | 0.02520 | 0.00 |
| IFT74 | 2984 | 17694631 | C | 0.00 | 0.02421 | 0.00 |
| TEK | 2986 | 10967731 | A | 0.17 | 0.04607 | 0.37 |
| APBAI | 3000 | 1761287 | G | 0.73 | 0.00082 | 4.08 |
| APBAI | 3001 | 2781542 | G | 0.50 | 0.00322 | 3.21 |
| APBAI | 3004 | 11139084 | A | 0.57 | 0.01590 | 2.59 |
| APBA1 | 3005 | 10867730 | T | 0.57 | 0.01590 | 2.59 |
| APBAI | 3006 | 7872660 | T | 0.37 | 0.00620 | 3.17 |
| APBA1 | 3007 | 7033141 | G | 0.33 | 0.03058 | 2.54 |
| TRPM3 | 3021 | 10780950 | T | 0.33 | 0.02355 | 2.65 |
| NTRK2 | 3072 | 1212171 | C | 0.67 | 0.00739 | 2.97 |
| NTRK2 | 3073 | 1187329 | A | 0.60 | 0.03818 | 2.29 |
| NTRK2 | 3074 | 7867174 | G | 0.50 | 0.00422 | 3.10 |
| NTRK2 | 3075 | 7038866 | A | 0.43 | 0.01677 | 2.63 |
| NTRK2 | 3076 | 13289538 | G | 0.10 | 0.01746 | 5.96 |
| NTRK2 | 3077 | 2799482 | C | 0.57 | 0.00050 | 3.92 |
| NTRK2 | 3078 | 2799483 | C | 0.57 | 0.00050 | 3.92 |
| NTRK2 | 3079 | 1899641 | G | 0.40 | 0.03136 | 2.42 |
| DAPKI | 3081 | 11141876 | T | 0.21 | 0.04177 | 2.88 |
| SHC3 | 3085 | 9410299 | C | 0.30 | 0.00082 | 0.25 |
| GABBR2 | 3101 | 10985765 | C | 0.27 | 0.02822 | 2.78 |
| FKTN | 3113 | 17251166 | C | 0.13 | 0.04715 | 0.34 |
| FKTN | 3114 | 7851777 | T | 0.13 | 0.04715 | 0.34 |
| SVEP1 | 3127 | 7875389 | C | 0.73 | 0.00439 | 3.36 |
| TTLL11 | 3145 | 10985483 | T | 0.20 | 0.03113 | 0.36 |
| TTLL11 | 3146 | 10120546 | C | 0.20 | 0.04605 | 0.39 |
| RALGPS1 | 3154 | 10987552 | T | 0.53 | 0.03236 | 2.33 |
| VAV2 | 3183 | 7865299 | G | 0.30 | 0.02264 | 2.77 |
| ABCA2 | 3201 | 12337910 | G | 0.21 | 0.02903 | 3.13 |
| MY03A | 3259 | 7896978 | C | 0.30 | 0.02476 | 2.73 |
| MYO3A | 3279 | 3737316 | T | 0.33 | 0.00478 | 3.41 |
| MYO3A | 3285 | 12764197 | G | 0.36 | 0.00480 | 3.47 |
| NRG3 | 3343 | 17744778 | C | 0.40 | 0.03887 | 2.33 |
| NRG3 | 3347 | 495978 | A | 0.14 | 0.01097 | 0.26 |
| SORCS3 | 3379 | 7099585 | T | 0.43 | 0.02281 | 2.57 |
| ATRN1 | 3410 | 10787571 | G | 0.11 | 0.03701 | 0.29 |
| ATRNL1 | 3415 | 1590733 | A | 0.29 | 0.04766 | 0.42 |
| ATE1 | 3436 | 1874664 | C | 0.30 | 0.01662 | 2.92 |
| ATE1 | 3437 | 3763753 | T | 0.30 | 0.01824 | 2.88 |
| GALNTL4 | 3470 | 11021734 | C | 0.37 | 0.03487 | 2.43 |
| ARNTL | 3491 | 10766073 | A | 0.13 | 0.04343 | 0.33 |
| ARNTL | 3492 | 10832020 | C | 0.03 | 0.00807 | 0.10 |
| ARNTL | 3494 | 1044432 | T | 0.33 | 0.00688 | 3.23 |
| ARNTL | 3495 | 1044431 | A | 0.32 | 0.01280 | 3.06 |
| DLG2 | 3535 | 10898155 | T | 0.33 | 0.03494 | 0.42 |
| DLG2 | 3536 | 1400313 | A | 0.37 | 0.04233 | 0.44 |
| STYK1 | 3616 | 10743918 | C | 0.54 | 0.02196 | 2.54 |
| PIK3C2G | 3634 | 10743268 | G | 0.60 | 0.01279 | 2.69 |
| KCNC2 | 3656 | 4131955 | T | 0.50 | 0.02118 | 2.49 |
| KCNC2 | 3657 | 7952767 | C | 0.50 | 0.01359 | 2.73 |
| KCNC2 | 3658 | 1526821 | C | 0.50 | 0.02118 | 2.49 |
| KCNC2 | 3659 | 10785167 | T | 0.50 | 0.02578 | 2.42 |
| NAV3 | 3666 | 1677914 | G | 0.18 | 0.03894 | 3.22 |
| NAV3 | 3669 | 1479027 | G | 0.20 | 0.04362 | 2.87 |
| NAV3 | 3671 | 1731743 | G | 0.20 | 0.04362 | 2.87 |
| GAS2L3 | 3682 | 17030333 | T | 0.13 | 0.01380 | 4.89 |
| N4BP2L2 | 3699 | 206336 | G | 0.64 | 0.02909 | 2.48 |
| LMO7 | 3747 | 9593132 | T | 0.13 | 0.04839 | 3.45 |
| GPC5 | 3785 | 17188557 | G | 0.10 | 0.00821 | 0.21 |
| GPC5 | 3787 | 913005 | T | 0.70 | 0.00078 | 3.97 |
| GPC5 | 3789 | 6492630 | A | 0.70 | 0.00707 | 3.06 |
| GPC5 | 3790 | 2148226 | G | 0.13 | 0.00281 | 0.21 |
| GPC5 | 3791 | 9523825 | G | 0.27 | 0.01569 | 0.35 |
| GPC6 | 3795 | 16948547 | G | 0.43 | 0.01320 | 2.72 |
| GPC6 | 3800 | 1323975 | A | 0.13 | 0.04715 | 0.34 |
| GPC6 | 3803 | 3899317 | A | 0.27 | 0.01846 | 0.36 |
| ITGBL1 | 3845 | 7338172 | G | 0.03 | 0.03464 | 0.15 |
| ITGBL1 | 3846 | 3783215 | C | 0.03 | 0.03464 | 0.15 |
| LRFN5 | 3892 | 10149327 | T | 0.17 | 0.00995 | 0.28 |
| PPP2R5E | 3920 | 6573508 | C | 0.07 | 0.01965 | 0.20 |
| PPP2R5E | 3921 | 10137202 | C | 0.00 | 0.00811 | 0.00 |
| PPP2R5E | 3929 | 7154718 | G | 0.10 | 0.02065 | 5.74 |
| RGS6 | 3950 | 2239221 | C | 0.60 | 0.04465 | 2.23 |
| ATP10A | 4010 | 7175557 | C | 0.14 | 0.03709 | 3.69 |
| RYR3 | 4026 | 16973353 | A | 0.30 | 0.04357 | 0.43 |
| RASGRP1 | 4051 | 12324402 | G | 0.10 | 0.02382 | 0.26 |
| GLDN | 4063 | 17648140 | G | 0.17 | 0.01940 | 0.31 |
| CGNL1 | 4067 | 12595188 | G | 0.30 | 0.01662 | 2.92 |
| CGNL1 | 4070 | 7182648 | T | 0.03 | 0.00749 | 0.10 |
| CGNL1 | 4072 | 8034215 | G | 0.07 | 0.00754 | 0.17 |
| TBC1D2B | 4090 | 16969397 | T | 0.23 | 0.02425 | 3.02 |
| ARNT2 | 4101 | 4778800 | T | 0.37 | 0.02342 | 2.59 |
| ARNT2 | 4104 | 4778836 | T | 0.17 | 0.04433 | 0.37 |
| PCSK6 | 4111 | 12595154 | G | 0.60 | 0.04353 | 2.24 |
| A2BP1 | 4131 | 1507031 | A | 0.23 | 0.04036 | 0.40 |
| TMC5 | 4140 | 8059797 | A | 0.50 | 0.04981 | 2.18 |
| MPHOSPH6 | 4178 | 1011433 | C | 0.27 | 0.02037 | 2.95 |
| MPHOSPH6 | 4180 | 2967328 | C | 0.27 | 0.03810 | 2.62 |
| USP10 | 4207 | 7188512 | C | 0.50 | 0.01120 | 2.73 |
| USP10 | 4208 | 964453 | G | 0.37 | 0.02342 | 2.59 |
| USP10 | 4210 | 744839 | T | 0.47 | 0.00756 | 2.90 |
| CA10 | 4232 | 12603762 | C | 0.63 | 0.00320 | 3.24 |
| CA10 | 4235 | 1501253 | G | 0.53 | 0.01613 | 2.59 |
| CA10 | 4237 | 1393735 | G | 0.63 | 0.00399 | 3.16 |
| CA10 | 4238 | 2970016 | G | 0.33 | 0.02168 | 0.39 |
| MSI2 | 4256 | 792402 | A | 0.03 | 0.02702 | 0.14 |
| MSI2 | 4257 | 8066677 | G | 0.13 | 0.02117 | 0.29 |
| HRNBP3 | 4272 | 7212305 | A | 0.00 | 0.02520 | 0.00 |
| HRNBP3 | 4273 | 907898 | C | 0.00 | 0.02782 | 0.00 |
| DLGAP1 | 4278 | 7245298 | G | 0.03 | 0.02018 | 0.13 |
| CDH7 | 4357 | 8093720 | A | 0.20 | 0.04011 | 0.38 |
| CDH7 | 4358 | 1484725 | T | 0.20 | 0.04145 | 0.38 |
| CDH7 | 4361 | 2046407 | G | 0.43 | 0.02108 | 2.54 |
| CDH7 | 4365 | 2628210 | G | 0.37 | 0.02342 | 2.59 |
| ATRN | 4390 | 151511 | A | 0.57 | 0.04551 | 2.21 |
| PLCB1 | 4421 | 2662990 | T | 0.03 | 0.03012 | 0.14 |
| PLCB1 | 4424 | 6086591 | A | 0.43 | 0.04766 | 2.22 |
| MACROD2 | 4451 | 2193028 | C | 0.27 | 0.01064 | 3.32 |
| MACROD2 | 4455 | 6043616 | C | 0.23 | 0.03311 | 2.86 |
| MAPRE1 | 4491 | 2070090 | T | 0.43 | 0.00448 | 3.15 |
| CDH4 | 4534 | 2427144 | A | 0.67 | 0.04706 | 2.26 |
| NCAM2 | 4548 | 2826841 | T | 0.37 | 0.04663 | 2.30 |
| ASPHD2 | 4573 | 16982107 | T | 0.17 | 0.00073 | 7.90 |
| HPS4 | 4574 | 5761552 | T | 0.67 | 0.02553 | 2.49 |
| HPS4 | 4575 | 739291 | C | 0.30 | 0.00564 | 3.48 |
| HPS4 | 4576 | 3747134 | G | 0.30 | 0.00564 | 3.48 |
| HPS4 | 4577 | 5761557 | A | 0.25 | 0.03046 | 2.88 |
| TTLLI | 4593 | 5996268 | C | 0.17 | 0.04629 | 3.08 |

| **TABLE 18: Alleles Influencing Discontinuation from Adverse Events for Quetiapine** | | | | | | |
|---|---|---|---|---|---|---|
| **Gene Name** | **Seq ID** | **NCBI RS#** | **Allele** | **Freq. in Discontinuers** | **P** | **Odds Ratio** |
| KIF1B | 2 | 4846198 | A | 0.30 | 0.0258 | 0.44 |
| RPI-21O18.1 | 18 | 10803354 | A | 0.60 | 0.0111 | 2.45 |
| LRP8 | 56 | 11206139 | G | 0.36 | 0.0387 | 0.48 |
| ELTD1 | 64 | 1352555 | A | 0.45 | 0.0407 | 2.05 |
| ELTD1 | 65 | 4650627 | A | 0.11 | 0.0229 | 0.33 |
| NGF | 80 | 6327 | A | 0.57 | 0.0436 | 2.01 |
| MR1 | 134 | 3845422 | A | 0.32 | 0.0285 | 2.33 |
| KCNK2 | 138 | 10864143 | A | 0.48 | 0.0140 | 2.40 |
| USH2A | 151 | 1339411 | T | 0.32 | 0.0481 | 2.15 |
| ESRRG | 183 | 12033378 | A | 0.27 | 0.0380 | 2.35 |
| RYR2 | 206 | 2010045 | G | 0.62 | 0.0307 | 2.16 |
| RYR2 | 207 | 2618687 | T | 0.52 | 0.0452 | 2.00 |
| CHRM3 | 226 | 479933 | A | 0.11 | 0.0209 | 0.32 |
| CHRM3 | 227 | 663927 | A | 0.11 | 0.0255 | 0.33 |
| FMN2 | 228 | 12118961 | T | 0.61 | 0.0326 | 2.11 |
| FMN2 | 229 | 12145060 | A | 0.55 | 0.0142 | 2.37 |
| FMN2 | 231 | 12123407 | A | 0.57 | 0.0288 | 2.13 |
| FMN2 | 248 | 11799544 | T | 0.00 | 0.0478 | 0.00 |
| FMN2 | 249 | 10926257 | T | 0.00 | 0.0478 | 0.00 |
| FMN2 | 250 | 4659973 | T | 0.25 | 0.0477 | 0.47 |
| RGS7 | 258 | 10802917 | G | 0.36 | 0.0317 | 0.47 |
| DDEF2 | 284 | 10929580 | C | 0.59 | 0:0083 | 2.50 |
| KLHL29 | 289 | 747345 | A | 0.61 | 0.0022 | 2.90 |
| KLHL29 | 290 | 737565 | A | 0.64 | 0.0048 | 2.69 |
| KLHL29 | 291 | 747344 | G | 0.64 | 0.0050 | 2.67 |
| KLHL29 | 292 | 6727901 | G | 0.64 | 0.0065 | 2.60 |
| KLHL29 | 293 | 7570872 | G | 0.50 | 0.0365 | 2.06 |
| KLHL29 | 294 | 10172684 | G | 0.45 | 0.0321 | 2.13 |
| BRE | 322 | 10209422 | T | 0.29 | 0.0034 | 3.49 |
| BRE | 323 | 6747817 | G | 0.27 | 0.0387 | 2.33 |
| CCDC85A | 418 | 4672096 | G | 0.00 | 0.0316 | 0.00 |
| CCDC85A | 424 | 214041 | T | 0.48 | 0.0225 | 2.22 |
| CTNNA2 | 457 | 2165975 | T | 0.23 | 0.0317 | 2.61 |
| CTNNA2 | 458 | 6547299 | A | 0.18 | 0.0023 | 0.29 |
| CTNNA2 | 462 | 17018919 | C | 0.18 | 0.0266 | 2.98 |
| CTNNA2 | 464 | 318362 | C | 0.25 | 0.0338 | 0.44 |
| ARHGAP15 | 539 | 13032052 | C | 0.07 | 0.0416 | 0.29 |
| ARHGAP15 | 544 | 16858944 | A | 0.09 | 0.0435 | 0.34 |
| ARHGAP15 | 545 | 7605182 | C | 0.07 | 0.0498 | 0.30 |
| ARHGAP15 | 546 | 17814502 | A | 0.09 | 0.0067 | 0.24 |
| KIF5C | 562 | 7355478 | T | 0.02 | 0.0262 | 0.14 |
| FMNL2 | 580 | 2577180 | G | 0.32 | 0.0207 | 2.46 |
| FMNL2 | 585 | 11682487 | C | 0.41 | 0.0412 | 2.08 |
| FMNL2 | 586 | 6434113 | C | 0.34 | 0.0325 | 2.24 |
| PLA2R1 | 604 | 949753 | G | 0.34 | 0.0080 | 2.77 |
| ALS2 | 654 | 4675226 | T | 0.11 | 0.0311 | 0.35 |
| CUL3 | 686 | 16866061 | G | 0.48 | 0.0361 | 2.08 |
| IRS1 | 690 | 10205233 | T | 0.11 | 0.0412 | 3.56 |
| IRS1 | 691 | 1560251 | T | 0.18 | 0.0423 | 2.69 |
| COL4A4 | 693 | 10933165 | C | 0.55 | 0.0113 | 2.40 |
| COL4A4 | 695 | 10166736 | A | 0.57 | 0.0114 | 2.40 |
| DNER | 712 | 207671 | G | 0.57 | 0.0231 | 2.19 |
| CNTN6 | 742 | 265799 | G | 0.70 | 0.0137 | 2.45 |
| CNTN6 | 744 | 265767 | A | 0.67 | 0.0054 | 2.73 |
| CNTN6 | 745 | 2055738 | C | 0.71 | 0.0002 | 3.89 |
| CNTN6 | 746 | 3772290 | T | 0.68 | 0.0003 | 3.57 |
| CNTN6 | 754 | 17038701 | G | 0.14 | 0.035 | 3.26 |
| CNTN4 | 770 | 12497768 | T | 0.57 | 0:0436 | 2.01 |
| IRAK2 | 784 | 6804954 | A | 0.34 | 0.0443 | 0.49 |
| IRAK2 | 785 | 708035 | T | 0.50 | 0.0165 | 2.30 |
| IRAK2 | 786 | 11717636 | C | 0.57 | 0.0407 | 2.03 |
| RARB | 805 | 17525900 | C | 0.18 | 0.0266 | 2.98 |
| CACNA2D2 | 861 | 2298955 | C | 0.20 | 0.0158 | 3.11 |
| CACNA2D2 | 862 | 743757 | C | 0.18 | 0.0423 | 2.69 |
| CACNA2D3 | 867 | 4928040 | A | 0.20 | 0.0314 | 0.42 |
| FLNB | 907 | 9834312 | A | 0.25 | 0.0218 | 0.42 |
| FLNB | 911 | 12632456 | A | 0.23 | 0.0493 | 0.46 |
| FHIT | 921 | 11919495 | A | 0.50 | 0.0024 | 2.89 |
| FHIT | 922 | 9871875 | C | 0.50 | 0.0024 | 2.89 |
| FHIT | 923 | 9834182 | A | 0.50 | 0.0107 | 2.43 |
| FHIT | 929 | 815718 | C | 0.08 | 0.0413 | 0.29 |
| PTPRG | 938 | 1388613 | C | 0.07 | 0.0053 | 0.20 |
| CADPS | 944 | 9311842 | T | 0.07 | 0.0216 | 0.26 |
| MAGI1 | 974 | 9840574 | T | 0.43 | 0.0339 | 2.12 |
| FAM19A1 | 981 | 11707519 | G | 0.25 | 0.0445 | 2.33 |
| GBE1 | 992 | 3772899 | G | 0.34 | 0.0101 | 0.40 |
| HTR1F | 999 | 1027689 | T | 0.16 | 0.0445 | 2.84 |
| PLCXD2 | 1016 | 9288930 | T | 0.30 | 0.0462 | 0.48 |
| ZXDC | 1047 | 1687462 | C | 0.30 | 0.0461 | 0.47 |
| CLSTN2 | 1078 | 6775380 | A | 0.45 | 0.0254 | 2.22 |
| CLSTN2 | 1080 | 347975 | G | 0.45 | 0.0166 | 2.33 |
| CLSTN2 | 1081 | 347973 | A | 0.43 | 0.0359 | 2.18 |
| TNIK | 1100 | 9870583 | C | 0.36 | 0.0230 | 0.44 |
| NLGN1 | 1111 | 10936764 | G | 0.16 | 0.0416 | 0.41 |
| HTR3D | 1129 | 6779545 | A | 0.55 | 0.0409 | 2.03 |
| LEPREL1 | 1139 | 893054 | T | 0.36 | 0.0232 | 2.33 |
| LEPREL1 | 1140 | 573533 | A | 0.36 | 0.0273 | 2.27 |
| SLC2A9 | 1164 | 10023068 | A | 0.36 | 0.0149 | 2.48 |
| SLC2A9 | 1166 | 9291642 | C | 0.27 | 0.0084 | 3.00 |
| SLC2A9 | 1168 | 6820230 | T | 0.18 | 0.0401 | 0.42 |
| LDB2 | 1182 | 1990280 | G | 0.55 | 0.0080 | 2.50 |
| LDB2 | 1184 | 13110882 | G | 0.52 | 0.0111 | 2.41 |
| KIAA1239 | 1206 | 17575883 | A | 0.02 | 0.0104 | 0.11 |
| KIAA1239 | 1207 | 17575897 | T | 0.05 | 0.0147 | 0.19 |
| TBC1D1 | 1212 | 13110318 | A | 0.18 | 0.0105 | 3.67 |
| LIMCH1 | 1225 | 7674006 | C | 0.09 | 0.0317 | 4.70 |
| CXCL9 | 1254 | 10518143 | C | 0.14 | 0.0030 | 0.26 |
| SCD5 | 1262 | 7684732 | C | 0.34 | 0.0429 | 2.14 |
| SCD5 | 1265 | 6811012 | T | 0.30 | 0.0333 | 2.33 |
| SCDS | 1269 | 6848340 | T | 0.25 | 0.0142 | 2.87 |
| FAM13A1 | 1280 | 7657817 | T | 0.30 | 0.0240 | 2.46 |
| FAM13A1 | 1287 | 1708673 | T | 0.02 | 0.0212 | 0.13 |
| FAM13A1 | 1288 | 1996139 | A | 0.02 | 0.0262 | 0.14 |
| PDLIM5 | 1291 | 1353044 | T | 0.48 | 0.0002 | 3.78 |
| PDLIM5 | 1292 | 2433325 | A | 0.48 | 0.0002 | 3.78 |
| PDLIM5 | 1293 | 2510777 | C | 0.30 | 0.0054 | 0.36 |
| PDLIM5 | 1295 | 2452600 | T | 0.41 | 0.0141 | 2.42 |
| PDLIM5 | 1297 | 7662466 | A | 0.32 | 0.0185 | 0.43 |
| PDLIM5 | 1299 | 7690296 | G | 0.32 | 0.0499 | 0.49 |
| PDLIM5 | 1300 | 17021918 | T | 0.41 | 0.0217 | 2.29 |
| DKK2 | 1304 | 419764 | T | 0.23 | 0.0280 | 0.42 |
| COL25A1 | 1320 | 11942576 | C | 0.50 | 0.0180 | 2.27 |
| COL25A1 | 1336 | 4256292 | G | 0.41 | 0.0412 | 2.08 |
| ANK2 | 1347 | 10019154 | A | 0.25 | 0.0071 | 0.36 |
| ANK2 | 1348 | 362496 | G | 0.48 | 0.0029 | 2.84 |
| ANK2 | 1349 | 29329 | T | 0.59 | 0.0226 | 2.21 |
| MAML3 | 1387 | 2246759 | G | 0.25 | 0.0035 | 0.33 |
| MAML3 | 1389 | 4863683 | C | 0.36 | 0.0467 | 2.08 |
| MAML3 | 1390 | 6831959 | A | 0.68 | 0.0075 | 2.60 |
| MAML3 | 1391 | 11734419 | G | 0.48 | 0.0090 | 2.50 |
| INPP4B | 1416 | 17015544 | T | 0.05 | 0.0221 | 0.21 |
| INPP4B | 1420 | 336391 | T | 0.18 | 0.0311 | 0.41 |
| INPP4B | 1422 | 2130685 | T | 0.09 | 0.0334 | 0.32 |
| FSTL5 | 1450 | 17041096 | C | 0.43 | 0.0032 | 3.01 |
| FSTL5 | 1451 | 7657675 | A | 0.40 | 0.0060 | 2.77 |
| FSTL5 | 1452 | 4501178 | C | 0.25 | 0.0314 | 2.49 |
| PALLD | 1483 | 6552873 | C | 0.18 | 0.0171 | 0.37 |
| PALLD | 1486 | 9312333 | C | 0.18 | 0.0209 | 0.38 |
| PALLD | 1488 | 10031058 | G | 0.18 | 0.0209 | 0.38 |
| PALLD | 1489 | 10009813 | G | 0.50 | 0.0230 | 2.20 |
| PALLD | 1490 | 6840767 | C | 0.14 | 0.0387 | 0.38 |
| PALLD | 1493 | 2319900 | T | 0.23 | 0.0496 | 0.46 |
| PALLD | 1506 | 4364224 | T | 0.55 | 0.0229 | 2.23 |
| ODZ3 | 1513 | 2675534 | T | 0.09 | 0.0358 | 0.32 |
| ENPP6 | 1525 | 4444870 | T | 0.57 | 0.0145 | 2.33 |
| AHRR | 1531 | 2721004 | T | 0.45 | 0.0193 | 2.28 |
| AHRR | 1532 | 2721012 | G | 0.45 | 0.0039 | 2.84 |
| DNAH5 | 1546 | 1971860 | A | 0.34 | 0.0048 | 0.37 |
| EGFLAM | 1592 | 9292705 | A | 0.14 | 0.0447 | 3.09 |
| EGFLAM | 1596 | 2731979 | C | 0.25 | 0.0017 | 4.03 |
| EGFLAM | 1597 | 2731968 | C | 0.18 | 0.0043 | 4.35 |
| EGFLAM | 1598 | 16903982 | A | 0.14 | 0.0065 | 5.29 |
| ITGA1 | 1604 | 2860025 | T | 0.11 | 0.0458 | 0.37 |
| ITGA1 | 1610 | 2279587 | T | 0.00 | 0.0137 | 0.00 |
| ITGA1 | 1612 | 12520591 | G | 0.00 | 0.0169 | 0.00 |
| ITGA2 | 1620 | 3212576 | G | 0.07 | 0.0175 | 0.25 |
| ITGA2 | 1621 | 7719848 | A | 0.17 | 0.0428 | 0.41 |
| PDE4D | 1635 | 295963 | C | 0.20 | 0.0233 | 2.92 |
| TNPO1 | 1639 | 6452685 | T | 0.26 | 0.0347 | 2.44 |
| VCAN | 1667 | 4558964 | G | 0.14 | 0.0125 | 0.32 |
| ODZ2 | 1788 | 11134468 | G | 0.02 | 0.0397 | 0.15 |
| SLC22A23 | 1812 | 17136575 | G | 0.05 | 0.0182 | 0.20 |
| ATXN1 | 1837 | 3812192 | T | 0.26 | 0.0050 | 0.35 |
| SLC17A3 | 1859 | 1165161 | A | 0.39 | 0.0300 | 2.20 |
| RIMS1 | 1904 | 2463702 | G | 0.34 | 0.0300 | 0.46 |
| RIMS1 | 1915 | 2815743 | T | 0.62 | 0.0382 | 2.09 |
| NKAIN2 | 1954 | 342623 | T | 0.25 | 0.0215 | 2.67 |
| NKAIN2 | 1967 | 1031881 | C | 0.34 | 0.0016 | 3.40 |
| NKAIN2 | 1968 | 6906607 | A | 0.32 | 0.0078 | 2.85 |
| NKAIN2 | 1969 | 609783 | T | 0.32 | 0.0207 | 2.46 |
| PDE7B | 1982 | 4382284 | T | 0.57 | 0.0122 | 2.39 |
| STX11 | 1996 | 4896708 | A | 0.18 | 0.0011 | 0.26 |
| SYNE1 | 2006 | 1844356 | G | 0.52 | 0.0184 | 2.26 |
| SYNE1 | 2008 | 7776230 | T | 0.39 | 0.0441 | 2.08 |
| PDE10A | 2059 | 9459418 | A | 0.36 | 0.0360 | 2.17 |
| NXPH1 | 2083 | 10238726 | G | 0.59 | 0.0009 | 3.18 |
| NXPH1 | 2086 | 7777974 | A | 0.50 | 0.0273 | 2.16 |
| NXPH1 | 2087 | 10257265 | C | 0.45 | 0.0070 | 2.60 |
| NXPH1 | 2088 | 970526 | G | 0.48 | 0.0079 | 2.58 |
| PDE1C | 2160 | 4551232 | A | 0.25 | 0.0212 | 0.42 |
| PDE1C | 2161 | 10225488 | A | 0.25 | 0.0212 | 0.42 |
| BMPER | 2168 | 12672492 | A | 0.09 | 0.0358 | 0.32 |
| BMPER | 2169 | 969363 | C | 0.45 | 0.0169 | 2.38 |
| CDC2L5 | 2184 | 10272641 | G | 0.55 | 0.0233 | 2.19 |
| CDC2L5 | 2185 | 12536726 | G | 0.14 | 0.0284 | 3.53 |
| ABCA13 | 2192 | 6974064 | A | 0.20 | 0.0391 | 2.59 |
| ABCA13 | 2210 | 1918602 | T | 0.12 | 0.0430 | 3.54 |
| WBSCR17 | 2239 | 1012127 | T | 0.43 | 0.0135 | 2.41 |
| WBSCR17 | 2242 | 4585627 | T | 0.48 | 0.0451 | 2.01 |
| HIP1 | 2258 | 757364 | T | 0.33 | 0.0323 | 0.46 |
| MAGI2 | 2267 | 2428920 | C | 0.11 | 0.0209 | 0.32 |
| MAGI2 | 2280 | 1121066 | A | 0.61 | 0.0218 | 2.22 |
| MAGI2 | 2290 | 6954793 | C | 0.23 | 0.0152 | 2.96 |
| PCLO | 2334 | 10240976 | T | 0.66 | 0.0036 | 2.80 |
| SEMA3E | 2342 | 17504676 | G | 0.60 | 0.0335 | 2.12 |
| SEMA3E | 2346 | 1972459 | C | 0.50 | 0.0453 | 2.00 |
| DYNCIII | 2396 | 42066 | A | 0.05 | 0.0204 | 0.20 |
| CADPS2 | 2425 | 2428769 | G | 0.25 | 0.0259 | 0.43 |
| GRM8 | 2433 | 1419508 | A | 0.27 | 0.0050 | 0.35 |
| GRM8 | 2434 | 17627206 | T | 0.27 | 0.0080 | 0.38 |
| GRM8 | 2435 | 954661 | T | 0.27 | 0.0080 | 0.38 |
| GRM8 | 2438 | 2283079 | G | 0.30 | 0.0170 | 0.42 |
| GRM8 | 2440 | 10234189 | T | 0.59 | 0.0030 | 2.80 |
| GRM8 | 2441 | 1008907 | T | 0.59 | 0.0039 | 2.72 |
| GRM8 | 2447 | 6951643 | G | 0.27 | 0.0050 | 0.35 |
| GRM8 | 2449 | 6975541 | T | 0.30 | 0.0170 | 0.42 |
| GRM8 | 2450 | 1419442 | T | 0.20 | 0.0244 | 0.40 |
| GRM8 | 2451 | 2106183 | T | 0.41 | 0.0322 | 2.16 |
| CNTNAP2 | 2488 | 1639471 | G | 0.20 | 0.0244 | 0.40 |
| CNTNAP2 | 2492 | 6978796 | A | 0.20 | 0.0258 | 0.41 |
| CSMD1 | 2537 | 622765 | A | 0.27 | 0.0387 | 2.33 |
| MCPH1 | 2551 | 1530408 | C | 0.27 | 0.0192 | 2.63 |
| MCPH1 | 2553 | 2440416 | G | 0.29 | 0.0296 | 2.44 |
| MCPH1 | 2558 | 2920660 | A | 0.25 | 0.0347 | 2.45 |
| MCPH1 | 2566 | 4841224 | T | 0.50 | 0.0213 | 2.23 |
| MCPH1 | 2567 | 3739391 | A | 0.14 | 0.0421 | 0.39 |
| PEBP4 | 2626 | 4872539 | C | 0.14 | 0.0047 | 0.28 |
| PEBP4 | 2627 | 7829416 | G | 0.14 | 0.0059 | 0.29 |
| PEBP4 | 2628 | 6558183 | T | 0.11 | 0.0378 | 0.36 |
| SNTG1 | 2653 | 10957739 | G | 0.36 | 0.0399 | 2.15 |
| SNTG1 | 2671 | 1484128 | T | 0.16 | 0.0132 | 0.34 |
| SNTG1 | 2672 | 1484129 | T | 0.20 | 0.0487 | 0.45 |
| NKAIN3 | 2705 | 975688 | T | 0.45 | 0.0166 | 2.33 |
| NKAIN3 | 2709 | 1156385 | A | 0.45 | 0.0216 | 2.24 |
| NKAIN3 | 2711 | 10504354 | A | 0.45 | 0.0166 | 2.33 |
| KCNB2 | 2743 | 2256675 | A | 0.14 | 0.0245 | 3.63 |
| KCNB2 | 2746 | 2255053 | A | 0.14 | 0.0245 | 3.63 |
| MMP16 | 2765 | 1915023 | T | 0.02 | 0.0138 | 0.12 |
| MMP16 | 2766 | 7821897 | C | 0.02 | 0.0161 | 0.12 |
| GRHL2 | 2773 | 4734553 | G | 0.52 | 0.0406 | 2.03 |
| CSMD3 | 2809 | 6981814 | A | 0.41 | 0.0412 | 2.08 |
| MTSS1 | 2852 | 4871506 | T | 0.39 | 0.0218 | 0.45 |
| SMARCA2 | 2931 | 2147263 | T | 0.30 | 0.0042 | 0.35 |
| SLC1A1 | 2935 | 9886720 | T | 0.36 | 0.0460 | 2.10 |
| SLC1A1 | 2936 | 4742008 | A | 0.39 | 0.0498 | 2.04 |
| RP11-35N6.1 | 3108 | 7853653 | A | 0.48 | 0.0329 | 2.12 |
| VAV2 | 3185 | 10993832 | A | 0.36 | 0.0467 | 2.08 |
| VAV2 | 3189 | 2810473 | T | 0.39 | 0.0498 | 2.04 |
| KCNT1 | 3195 | 497547 | G | 0.09 | 0.0110 | 7.10 |
| KCNT1 | 3196 | 11103147 | G | 0.02 | 0.0442 | 0.16 |
| KCNT1 | 3197 | 11103150 | A | 0.02 | 0.0489 | 0.16 |
| KCNT1 | 3198 | 10858158 | G | 0.02 | 0.0489 | 0.16 |
| KCNT1 | 3199 | 11103157 | C | 0.02 | 0.0489 | 0.16 |
| ABCA2 | 3201 | 12337910 | G | 0.02 | 0.0247 | 0.13 |
| CACNA1B | 3205 | 7391010 | C | 0.07 | 0.0175 | 0.25 |
| VTI1A | 3391 | 3740143 | G | 0.18 | 0.0454 | 0.43 |
| HSPA12A | 3427 | 10787720 | C | 0.20 | 0.0174 | 3.06 |
| ATE1 | 3446 | 10732824 | A | 0.19 | 0.0061 | 4.00 |
| ATE1 | 3447 | 3750835 | A | 0.17 | 0.0200 | 3.40 |
| SPON1 | 3500 | 10832163 | T | 0.25 | 0.0381 | 0.45 |
| SPONI | 3506 | 10832170 | T | 0.36 | 0.0317 | 0.47 |
| SPONI | 3507 | 1406356 | A | 0.36 | 0.0258 | 0.46 |
| SPONI | 3510 | 11023088 | T | 0.64 | 0.0154 | 2.38 |
| DLG2 | 3528 | 2060147 | G | 0.36 | 0.0005 | 3.89 |
| DLG2 | 3530 | 983590 | T | 0.34 | 0.0007 | 3.80 |
| DLG2 | 3532 | 12225388 | A | 0.30 | 0.0030 | 3.36 |
| DLG2 | 3533 | 10898148 | A | 0.31 | 0.0054 | 3.09 |
| DLG2 | 3534 | 7950988 | T | 0.29 | 0.0084 | 2.99 |
| DLG2 | 3543 | 4451754 | A | 0.45 | 0.0443 | 2.02 |
| ELMOD1 | 3556 | 6588969 | A | 0.32 | 0.0094 | 0.39 |
| OPCML | 3566 | 4936168 | C | 0.45 | 0.0216 | 2.24 |
| OPCML | 3579 | 6590700 | C | 0.09 | 0.0435 | 0.34 |
| OPCML | 3582 | 4937780 | G | 0.09 | 0.0400 | 0.33 |
| TSPAN9 | 3593 | 544668 | G | 0.52 | 0.0233 | 2.19 |
| KCNC2 | 3662 | 2471664 | C | 0.09 | 0.0358 | 0.32 |
| PCDH17 | 3735 | 7324697 | A | 0.14 | 0.0167 | 0.33 |
| LMO7 | 3746 | 9318373 | A | 0.39 | 0.0407 | 0.49 |
| GPC6 | 3808 | 4394948 | C | 0.52 | 0.0429 | 2.02 |
| GPC6 | 3822 | 9561551 | C | 0.34 | 0.0482 | 2.11 |
| ITGBL1 | 3849 | 17686597 | C | 0.02 | 0.0305 | 0.14 |
| ITGBL1 | 3850 | 2281991 | G | 0.02 | 0.0377 | 0.15 |
| ITGBL1 | 3852 | 1469853 | G | 0.02 | 0.0323 | 0.14 |
| SLC25A21 | 3888 | 7153409 | A | 0.14 | 0.0245 | 3.63 |
| SLC25A21 | 3889 | 4900678 | C | 0.14 | 0.0284 | 3.53 |
| LRFN5 | 3902 | 10131436 | A | 0.18 | 0.0310 | 0.40 |
| LRFN5 | 3904 | 1033877 | G | 0.45 | 0.0126 | 2.41 |
| GNG2 | 3905 | 8018469 | T | 0.57 | 0.0436 | 2.01 |
| RGS6 | 3950 | 2239221 | C | 0.34 | 0.0365 | 0.48 |
| CCDC88C | 3987 | 11160006 | C | 0.26 | 0.0287 | 0.43 |
| BCL11B | 4001 | 1257446 | C | 0.16 | 0.0215 | 0.37 |
| BCL11H | 4002 | 1257416 | G | 0.20 | 0.0298 | 0.42 |
| KIAA1370 | 4064 | 4776065 | C | 0.18 | 0.0311 | 0.41 |
| TBC1D2B | 4089 | 10519181 | C | 0.16 | 0.0318 | 0.39 |
| TBC1D2B | 4091 | 2241885 | C | 0.30 | 0.0210 | 0.43 |
| TBC1D2B | 4093 | 11634607 | T | 0.20 | 0.0089 | 0.35 |
| TBC1D2B | 4095 | 2867985 | A | 0.18 | 0.0112 | 0.35 |
| A2BP1 | 4114 | 1420058 | C | 0.69 | 0.0127 | 2.49 |
| A2BP1 | 4116 | 8047133 | C | 0.66 | 0.0300 | 2.16 |
| A2BP1 | 4118 | 1155959 | C | 0.61 | 0.0218 | 2.22 |
| A2BP1 | 4119 | 12926215 | G | 0.39 | 0.0087 | 2.64 |
| A2BP1 | 4121 | 4289011 | C | 0.60 | 0.0067 | 2.60 |
| A2BP1 | 4122 | 8055711 | T | 0.50 | 0.0012 | 3.11 |
| WWOX | 4153 | 11641213 | G | 0.32 | 0.0185 | 0.43 |
| WWOX | 4156 | 4887935 | C | 0.48 | 0.0186 | 2.28 |
| CDH13 | 4185 | 7196979 | G | 0.64 | 0.0283 | 2.20 |
| RAB11FIP4 | 4225 | 315431 | T | 0.43 | 0.0430 | 2.05 |
| CA10 | 4232 | 12603762 | C | 0.30 | 0.0462 | 0.48 |
| CA10 | 4234 | 4794315 | G | 0.30 | 0.0462 | 0.48 |
| CA10 | 4241 | 11652641 | G | 0.09 | 0.0435 | 0.34 |
| CA10 | 4243 | 16951177 | C | 0.02 | 0.0053 | 0.09 |
| MSI2 | 4255 | 1007462 | C | 0.64 | 0.0164 | 2.32 |
| HRNBP3 | 4272 | 7212305 | A | 0.25 | 0.0445 | 2.33 |
| PTPRM | 4287 | 503716 | T | 0.00 | 0.0478 | 0.00 |
| NEDD4L | 4325 | 11663936 | T | 0.32 | 0.0028 | 3.27 |
| NEDD4L | 4327 | 12457509 | A | 0.43 | 0.0044 | 2.77 |
| NEDD4L | 4329 | 10513914 | G | 0.43 | 0.0085 | 2.56 |
| NEDD4L | 4330 | 2075405 | G | 0.36 | 0.0107 | 2.59 |
| NEDD4L | 4331 | 4940679 | G | 0.61 | 0.0051 | 2.65 |
| NEDD4L | 4332 | 7242486 | G | 0.61 | 0.0055 | 2.65 |
| CCBE1 | 4336 | 17065761 | C | 0.02 | 0.0262 | 0.14 |
| CCBE1 | 4337 | 12969965 | A | 0.32 | 0.0320 | 2.29 |
| ZNF667 | 4387 | 10423617 | T | 0.33 | 0.0092 | 2.77 |
| RNF24 | 4393 | 6084530 | C | 0.16 | 0.0234 | 0.37 |
| PLCB1 | 4412 | 6055513 | G | 0.61 | 0.0139 | 2.36 |
| PLCB1 | 4414 | 6055577 | A | 0.07 | 0.0325 | 0.28 |
| PLEB1 | 4433 | 4295085 | A | 0.66 | 0.0299 | 2.16 |
| PLCB4 | 4434 | 6086686 | A | 0.66 | 0.0300 | 2.16 |
| MACROD2 | 4447 | 1657969 | T | 0.23 | 0.0430 | 0.45 |
| KIF16H | 4462 | 6043875 | C | 0.52 | 0.0345 | 2.08 |
| PTPRT | 4498 | 746413 | C | 0.66 | 0.0127 | 2.42 |
| PTPRT | 4501 | 6016688 | T | 0.43 | 0.0203 | 2.28 |
| KCNB1 | 4519 | 496511 | C | 0.60 | 0.0273 | 2.18 |
| MIRN155 | 4554 | 2828908 | T | 0.11 | 0.0281 | 0.34 |

| **TABLE 19: Alleles Influencing Discontinuation from Adverse Events for Perphenazine** | | | | | | |
|---|---|---|---|---|---|---|
| **Gene Name** | **Seq ID** | **NCBI RS#** | **Allele** | **Freq. in Discontinuers** | **P** | **Odds Ratio** |
| EPHB2 | 23 | 309499 | T | 0.63 | 0.0425 | 2.31 |
| ELTD1 | 67 | 4625238 | T | 0.33 | 0.0444 | 2.45 |
| PRKACB | 74 | 2642186 | G | 0.11 | 0.0060 | 0.20 |
| NGF | 83 | 11102925 | T | 0.07 | 0.0337 | 0.22 |
| KCNK2 | 139 | 2841598 | G | 0.37 | 0.0315 | 0.41 |
| USH2A | 170 | 12030122 | C | 0.23 | 0.0051 | 4.41 |
| ESRRG | 180 | 11117642 | T | 0.23 | 0.0269 | 3.13 |
| RYR2 | 213 | 2685297 | G | 0.07 | 0.0072 | 0.16 |
| RYR2 | 214 | 2249847 | C | 0.18 | 0.0255 | 0.32 |
| FMN2 | 234 | 11801848 | C | 0.53 | 0.0116 | 2.79 |
| FMN2 | 235 | 10926168 | G | 0.67 | 0.0148 | 2.77 |
| FMN2 | 236 | 7520065 | G | 0.67 | 0.0148 | 2.77 |
| RGS7 | 256 | 10802916 | T | 0.20 | 0.0315 | 0.36 |
| PLD5 | 270 | 6673239 | T | 0.20 | 0.0315 | 0.36 |
| HPCAL1 | 285 | 16856020 | T | 0.23 | 0.0297 | 3.07 |
| KCNK3 | 306 | 1275982 | C | 0.23 | 0.0316 | 0.37 |
| KCNK3 | 307 | 11126666 | A | 0.03 | 0.0009 | 0.07 |
| KCNK3 | 308 | 7584568 | G | 0.33 | 0.0185 | 0.37 |
| KCNK3 | 309 | 1627854 | G | 0.37 | 0.0425 | 0.43 |
| BRE | 321 | 6732163 | G | 0.07 | 0.0281 | 0.21 |
| BRE | 323 | 6747817 | G | 0.10 | 0.0451 | 0.29 |
| CDC42EP3 | 343 | 7607934 | G | 0.10 | 0.0248 | 0.26 |
| SLC8A1 | 355 | 17026039 | G | 0.30 | 0.0227 | 2.89 |
| PRKCE | 376 | 666334 | T | 0.50 | 0.0053 | 3.13 |
| ACYP2 | 414 | 2090182 | T | 0.03 | 0.0414 | 0.19 |
| CTNNA2 | 468 | 1444539 | C | 0.20 | 0.0225 | 3.56 |
| NAPS | 485 | 13392755 | T | 0.57 | 0.0130 | 2.75 |
| LRP1B | 504 | 10469593 | C | 0.10 | 0.0106 | 0.22 |
| LRP1B | 505 | 12479163 | A | 0.10 | 0.0451 | 0.29 |
| LRP1B | 507 | 4447563 | G | 0.10 | 0.0005 | 0.14 |
| KYNU | 534 | 3768844 | C | 0.30 | 0.0227 | 2.89 |
| ARHGAP15 | 550 | 6732131 | A | 0.17 | 0.0082 | 0.27 |
| ARHGAP15 | 553 | 6750323 | C | 0.17 | 0.0082 | 0.27 |
| ARHGAP15 | 554 | 4334442 | C | 0.10 | 0.0248 | 0.26 |
| ARHGAP15 | 555 | 10197021 | T | 0.23 | 0.0422 | 0.39 |
| ARHGAP15 | 557 | 11693600 | C | 0.57 | 0.0333 | 2.38 |
| FMNL2 | 579 | 2346182 | G | 0.50 | 0.0286 | 2.44 |
| FMNL2 | 586 | 6434113 | C | 0.07 | 0.0171 | 0.19 |
| SCN9A | 624 | 7589835 | A | 0.27 | 0.0228 | 3.05 |
| CERKL | 632 | 895901 | A | 0.50 | 0.0453 | 2.26 |
| PDE1A | 648 | 9332425 | T | 0.23 | 0.0351 | 0.38 |
| PARD3B | 655 | 1861763 | C | 0.33 | 0.0328 | 2.60 |
| PARD3B | 656 | 1019357 | G | 0.33 | 0.0328 | 2.60 |
| PARD3B | 667 | 1079943 | G | 0.17 | 0.0066 | 0.26 |
| COL4A3 | 698 | 6741679 | A | 0.50 | 0.0172 | 2.65 |
| TRIP12 | 728 | 17324331 | G | 0.47 | 0.0411 | 2.32 |
| CNTN6 | 746 | 3772290 | T | 0.57 | 0.0412 | 2.30 |
| CNTN4 | 768 | 13077493 | T | 0.07 | 0.0404 | 0.23 |
| CNTN4 | 773 | 11920374 | A | 0.50 | 0.0332 | 2.39 |
| ATP2B2 | 791 | 11719939 | G | 0.07 | 0.0281 | 0.21 |
| ATP2B2 | 792 | 6763274 | C | 0.07 | 0.0310 | 0.22 |
| ARPP-21 | 820 | 1963934 | C | 0.40 | 0.0257 | 2.59 |
| ARPP-21 | 821 | 4678802 | C | 0.67 | 0.0334 | 2.45 |
| CACNA2D3 | 865 | 4928009 | A | 0.40 | 0.0165 | 2.78 |
| CACNA2D3 | 866 | 9825455 | A | 0.33 | 0.0444 | 2.45 |
| ERC2 | 888 | 1485672 | T | 0.10 | 0.0045 | 0.19 |
| ERC2 | 890 | 9873480 | C | 0.11 | 0.0025 | 0.17 |
| ERC2 | 891 | 11708280 | A | 0.10 | 0.0053 | 0.20 |
| ERC2 | 892 | 6765986 | C | 0.10 | 0.0061 | 0.20 |
| ERC2 | 894 | 4974206 | G | 0.17 | 0.0153 | 0.30 |
| ERC2 | 895 | 13064525 | C | 0.13 | 0.0154 | 0.27 |
| FLNB | 908 | 2259091 | C | 0.50 | 0.0202 | 2.59 |
| PRICKLE2 | 960 | 17664984 | T | 0.04 | 0.0296 | 0.14 |
| PRICKLE2 | 962 | 153729 | A | 0.32 | 0.0193 | 2.98 |
| MAGI1 | 971 | 7649739 | G | 0.18 | 0.0334 | 0.34 |
| MAGI1 | 977 | 6445525 | C | 0.33 | 0.0345 | 0.41 |
| MAGI1 | 978 | 4688632 | G | 0.33 | 0.0409 | 0.42 |
| FAM19A1 | 980 | 6548986 | T | 0.53 | 0.0313 | 2.40 |
| RAB6B | 1061 | 6765093 | G | 0.60 | 0.0140 | 2.73 |
| EPHB1 | 1069 | 13314927 | G | 0.40 | 0.0294 | 2.53 |
| LEPREL1 | 1134 | 1666486 | A | 0.37 | 0.0318 | 2.54 |
| LEPREL1 | 1135 | 1447935 | T | 0.33 | 0.0496 | 2.41 |
| LRRC15 | 1148 | 4974514 | A | 0.53 | 0.0313 | 2.40 |
| SLC2A9 | 1155 | 1401438 | T | 0.30 | 0.0454 | 2.52 |
| PPARGC1A | 1195 | 6448226 | G | 0.27 | 0.0458 | 0.41 |
| SOD3 | 1196 | 17622933 | T | 0.47 | 0.0093 | 2.94 |
| NPFFR2 | 1239 | 4574375 | G | 0.07 | 0.0044 | 2.61 |
| NPFFR2 | 1248 | 9790741 | G | 0.17 | 0.0498 | 0.37 |
| SHROOM3 | 1255 | 3943037 | G | 0.43 | 0.0021 | 3.83 |
| SHROOM3 | 1256 | 13138237 | G | 0.43 | 0.0058 | 3.19 |
| SHROOM3 | 1258 | 4276307 | A | 0.23 | 0.0422 | 0.39 |
| FAM13A1 | 1286 | 1398938 | T | 0.20 | 0.0225 | 3.56 |
| FAM13A1 | 1287 | 1708673 | T | 0.20 | 0.0347 | 3.19 |
| FAM13A1 | 1288 | 1996139 | A | 0.20 | 0.0347 | 3.19 |
| COL25A1 | 1320 | 11942576 | C | 0.20 | 0.0261 | 0.35 |
| COL25A1 | 1329 | 794149 | G | 0.50 | 0.0453 | 2.26 |
| CAMK2D | 1368 | 17532826 | A | 0.33 | 0.0328 | 2.60 |
| GPR103 | 1383 | 7688947 | C | 0.07 | 0.0483 | 0.24 |
| MAML3 | 1401 | 4863720 | C | 0.63 | 0.0208 | 2.61 |
| IL15 | 1406 | 4254850 | C | 0.67 | 0.0148 | 2.77 |
| INPP4B | 1423 | 2627802 | T | 0.33 | 0.0278 | 0.40 |
| DCLK2 | 1434 | 10002404 | A | 0.60 | 0.0380 | 2.35 |
| TLL1 | 1479 | 3756016 | C | 0.20 | 0.0473 | 0.39 |
| PALLD | 1500 | 13122019 | A | 0.64 | 0.0298 | 2.54 |
| PALLD | 1507 | 1071738 | C | 0.57 | 0.0081 | 3.02 |
| PALLD | 1508 | 13116548 | T | 0.50 | 0.0008 | 4.00 |
| PALLD | 1509 | 11733251 | A | 0.50 | 0.0022 | 3.52 |
| PALLD | 1510 | 10518037 | T | 0.50 | 0.0027 | 3.43 |
| PALLD | 1511 | 6843588 | G | 0.57 | 0.0167 | 2.65 |
| PALLD | 1512 | 2331450 | A | 0.50 | 0.0027 | 3.43 |
| ITGA1 | 1606 | 10513001 | G | 0.03 | 0.0469 | 0.16 |
| THBS4 | 1654 | 385771 | C | 0.03 | 0.0071 | 0.10 |
| THBS4 | 1655 | 366471 | G | 0.03 | 0.0071 | 0.10 |
| THBS4 | 1656 | 256447 | C | 0.03 | 0.0087 | 0.10 |
| THBS4 | 1657 | 256444 | C | 0.07 | 0.0233 | 0.21 |
| THBS4 | 1661 | 7736549 | A | 0.04 | 0.0160 | 0.12 |
| MEF2C | 1676 | 700592 | G | 0.63 | 0.0054 | 3.14 |
| MEF2C | 1677 | 304151 | C | 0.50 | 0.0022 | 3.52 |
| MEF2C | 1678 | 4518438 | T | 0.65 | 0.0271 | 2.66 |
| MEF2C | 1679 | 3850651 | C | 0.57 | 0.0333 | 2.38 |
| FBXL17 | 1717 | 4957554 | C | 0.20 | 0.0261 | 0.35 |
| HSD17B4 | 1742 | 246965 | G | 0.27 | 0.0089 | 0.32 |
| LOC340069 | 1746 | 328694 | A | 0.30 | 0.0334 | 0.40 |
| TRPC7 | 1774 | 17170027 | C | 0.17 | 0.0394 | 0.35 |
| ODZ2 | 1790 | 13158058 | T | 0.17 | 0.0026 | 0.23 |
| PHACTR1 | 1821 | 12526453 | C | 0.17 | 0.0498 | 0.37 |
| ATXN1 | 1835 | 235147 | A | 0.20 | 0.0144 | 0.31 |
| SLC17A4 | 1846 | 9348694 | T | 0.40 | 0.0218 | 2.67 |
| SLC17A4 | 1848 | 1892248 | T | 0.40 | 0.0300 | 2.51 |
| SLC17A2 | 1865 | 3799371 | T | 0.53 | 0.0087 | 2.91 |
| SLC17A2 | 1866 | 6938233 | T | 0.53 | 0.0087 | 2.91 |
| SLC17A2 | 1867 | 2071297 | C | 0.53 | 0.0087 | 2.91 |
| SLC17A2 | 1868 | 442601 | C | 0.13 | 0.0274 | 0.30 |
| SLC17A2 | 1869 | 9467646 | G | 0.53 | 0.0104 | 2.84 |
| SLC17A2 | 1870 | 9467652 | G | 0.53 | 0.0116 | 2.79 |
| SLC17A2 | 1871 | 199739 | T | 0.13 | 0.0330 | 0.31 |
| BTNL2 | 1876 | 3806156 | T | 0.23 | 0.0291 | 0.37 |
| RIMS1 | 1898 | 530028 | G | 0.64 | 0.0390 | 2.41 |
| RIMS1 | 1899 | 511211 | C | 0.63 | 0.0425 | 2.31 |
| TRDN | 1936 | 2873479 | A | 0.20 | 0.0205 | 3.63 |
| TRDN | 1943 | 12178740 | G | 0.00 | 0.0450 | 0.00 |
| TRDN | 1944 | 4509144 | T | 0.00 | 0.0450 | 0.00 |
| TRDN | 1946 | 6902416 | G | 0.00 | 0.0450 | 0.00 |
| TRDN | 1947 | 9490794 | T | 0.00 | 0.0450 | 0.00 |
| NKAIN2 | 1955 | 9490937 | C | 0.07 | 0.0391 | 8.64 |
| EYA4 | 1977 | 9385647 | C | 0.20 | 0.0379 | 0.37 |
| PARK2 | 2016 | 2064419 | G | 0.33 | 0.0278 | 0.40 |
| PARK2 | 2018 | 3765474 | C | 0.27 | 0.0320 | 0.39 |
| PARK2 | 2019 | 992037 | T | 0.17 | 0.0124 | 0.29 |
| PARK2 | 2022 | 6455738 | A | 0.17 | 0.0186 | 0.31 |
| PARK2 | 2034 | 6930880 | C | 0.18 | 0.0334 | 0.34 |
| PARK2 | 2035 | 2023079 | C | 0.17 | 0.0394 | 0.35 |
| PARK2 | 2047 | 9365473 | T | 0.33 | 0.0368 | 2.55 |
| PDE10A | 2055 | 484842 | G | 0.03 | 0.0469 | 0.16 |
| CARD11 | 2066 | 2679258 | A | 0.70 | 0.0486 | 2.33 |
| SDK1 | 2073 | 663486 | T | 0.30 | 0.0326 | 2.70 |
| SDK1 | 2074 | 663466 | A | 0.30 | 0.0227 | 2.89 |
| NXPH1 | 2090 | 6962955 | A | 0.25 | 0.0134 | 0.32 |
| SCIN | 2094 | 849760 | C | 0.20 | 0.0379 | 0.37 |
| CREB5 | 2121 | 6977628 | T | 0.07 | 0.0247 | 0.21 |
| CREB5 | 2126 | 886750 | A | 0.10 | 0.0451 | 0.29 |
| SCRN1 | 2129 | 9912 | T | 0.40 | 0.0119 | 2.93 |
| FLJ22374 | 2145 | 7781249 | A | 0.23 | 0.0042 | 0.28 |
| FU22374 | 2147 | 10258097 | A | 0.63 | 0.0230 | 2.56 |
| CDC2L5 | 2186 | 3800802 | T | 0.46 | 0.0309 | 2.49 |
| CDC2L5 | 2187 | 10277422 | C | 0.37 | 0.0168 | 2.84 |
| GTF2IRD1 | 2253 | 6964833 | C | 0.43 | 0.0360 | 2.40 |
| HIP1 | 2257 | 1018945 | G | 0.30 | 0.0182 | 0.36 |
| MAG12 | 2282 | 10259665 | A | 0.10 | 0.0106 | 0.22 |
| CACNA2D1 | 2303 | 12535840 | G | 0.30 | 0.0490 | 0.43 |
| PCLO | 2311 | 17156675 | T | 0.47 | 0.0290 | 2.46 |
| ABCB4 | 2348 | 31659 | C | 0.23 | 0.0051 | 4.41 |
| ABCB4 | 2351 | 31662 | A | 0.23 | 0.0096 | 3.89 |
| DYNC1I1 | 2389 | 2171555 | A | 0.17 | 0.0467 | 3.29 |
| DYNC1I1 | 2394 | 2299277 | T | 0.37 | 0.0118 | 3.01 |
| KCND2 | 2411 | 10276880 | G | 0.10 | 0.0045 | 13.67 |
| KCND2 | 2417 | 12154976 | T | 0.37 | 0.0234 | 2.68 |
| KCND2 | 2418 | 2189974 | C | 0.37 | 0.0458 | 2.39 |
| SLC13A1 | 2426 | 1404836 | C | 0.07 | 0.0310 | 0.22 |
| DGKI | 2477 | 4728414 | C | 0.57 | 0.0076 | 2.96 |
| DGKI | 2478 | 1528103 | C | 0.57 | 0.0076 | 2.96 |
| DGKI | 2479 | 10268638 | A | 0.50 | 0.0361 | 2.35 |
| CNTNAP2 | 2487 | 1639481 | G | 0.64 | 0.0383 | 2.42 |
| CNTNAP2 | 2496 | 7802708 | G | 0.17 | 0.0153 | 0.30 |
| MCPH1 | 2574 | 7844367 | C | 0.17 | 0.0467 | 3.29 |
| KCNB2 | 2754 | 2981104 | C | 0.23 | 0.0413 | 2.84 |
| NCALD | 2780 | 2387620 | G | 0.50 | 0.0453 | 2.26 |
| FERIL6 | 2833 | 4242352 | C | 0.43 | 0.0461 | 2.29 |
| MTSS1 | 2852 | 4871506 | T | 0.33 | 0.0341 | 0.41 |
| KCNQ3 | 2901 | 2597363 | G | 0.17 | 0.0261 | 3.87 |
| NTRK2 | 3074 | 7867174 | G | 0.10 | 0.0027 | 0.18 |
| NTRK2 | 3075 | 7038866 | A | 0.07 | 0.0025 | 0.13 |
| NTRK2 | 3077 | 2799482 | C | 0.04 | 0.0005 | 0.06 |
| NTRK2 | 3078 | 2799483 | C | 0.07 | 0.0012 | 0.12 |
| NTRK2 | 3079 | 1899641 | G | 0.07 | 0.0020 | 0.13 |
| STOM | 3142 | 2196311 | G | 0.27 | 0.0308 | 2.86 |
| RALGPS1 | 3160 | 10987576 | A | 0.03 | 0.0469 | 0.16 |
| ABLI | 3167 | 2855190 | C | 0.03 | 0.0469 | 0.16 |
| ABCA2 | 3201 | 12337910 | G | 0.20 | 0.0205 | 3.63 |
| PRKCQ | 3226 | 806399 | A | 0.40 | 0.0447 | 2.35 |
| PRKCQ | 3228 | 2236380 | T | 0.47 | 0.0472 | 2.27 |
| PRKCQ | 3230 | 4748667 | A | 0.23 | 0.0164 | 0.34 |
| CACNB2 | 3239 | 729245 | A | 0.47 | 0.0411 | 2.32 |
| MYO3A | 3267 | 993397 | A | 0.53 | 0.0178 | 2.63 |
| MYO3A | 3288 | 3006844 | G | 0.25 | 0.0295 | 0.37 |
| JMJD1C | 3299 | 10761747 | G | 0.40 | 0.0300 | 2.51 |
| JMJD1C | 3301 | 9629895 | A | 0.37 | 0.0318 | 2.54 |
| JMJD1C | 3303 | 2893922 | C | 0.37 | 0.0007 | 4.55 |
| CDH23 | 3313 | 4747195 | T | 0.40 | 0.0342 | 2.46 |
| CDH23 | 3314 | 4747197 | T | 0.40 | 0.0394 | 2.40 |
| VT11A | 3401 | 17585548 | G | 0.37 | 0.0359 | 2.49 |
| ATE1 | 3439 | 7075881 | T | 0.03 | 0.0414 | 0.20 |
| ATE1 | 3444 | 10749429 | T | 0.03 | 0.0414 | 2.37 |
| CTBP2 | 3454 | 769160 | C | 0.00 | 0.0173 | 0.00 |
| GALNTL4 | 3468 | 4910288 | A | 0.53 | 0.0484 | 2.23 |
| MICAL2 | 3473 | 1159649 | C | 0.07 | 0.0016 | 0.13 |
| STYK1 | 3609 | 1078437 | G | 0.53 | 0.0313 | 2.40 |
| STYK1 | 3611 | 3759259 | A | 0.53 | 0.0342 | 2.37 |
| STYK1 | 3612 | 10845187 | C | 0.53 | 0.0428 | 2.29 |
| STYK1 | 3615 | 10845198 | T | 0.60 | 0.0140 | 2.73 |
| LOC729025 | 3630 | 11056890 | C | 0.07 | 0.0193 | 0.20 |
| MTIF3 | 3689 | 1885990 | A | 0.30 | 0.0454 | 2.52 |
| UBL3 | 3698 | 9578147 | G | 0.23 | 0.0053 | 0.29 |
| PCDH17 | 3736 | 4884285 | T | 0.37 | 0.0234 | 2.68 |
| GPCS | 3778 | 1330469 | T | 0.60 | 0.0109 | 2.83 |
| GPC6 | 3794 | 1008993 | T | 0.03 | 0.0106 | 0.11 |
| GPC6 | 3795 | 16948547 | G | 0.07 | 0.0192 | 0.20 |
| NALCN | 3824 | 625331 | C | 0.03 | 0.0327 | 0.14 |
| NALCN | 3825 | 676602 | A | 0.00 | 0.0220 | 0.00 |
| NALCN | 3837 | 3916910 | C | 0.10 | 0.0262 | 0.26 |
| ITGBL1 | 3840 | 875116 | C | 0.10 | 0.0148 | 0.23 |
| ITGBL1 | 3841 | 915343 | T | 0.07 | 0.0373 | 0.23 |
| ITGBL1 | 3842 | 1296174 | A | 0.07 | 0.0447 | 0.24 |
| ITGBL1 | 3846 | 3783215 | C | 0.07 | 0.0281 | 0.21 |
| ITGBL1 | 3847 | 1436261 | C | 0.07 | 0.0483 | 0.24 |
| NOVA1 | 3862 | 2144335 | A | 0.36 | 0.0240 | 2.78 |
| SLC25A21 | 3882 | 2022601 | A | 0.36 | 0.0313 | 0.40 |
| SLC25A21 | 3885 | 1367029 | T | 0.36 | 0.0248 | 0.38 |
| SLC25A21 | 3886 | 10140136 | A | 0.13 | 0.0164 | 0.27 |
| SLC25A21 | 3887 | 9806037 | G | 0.17 | 0.0250 | 0.32 |
| SAMD4A | 3910 | 11626634 | A | 0.23 | 0.0028 | 5.00 |
| PPP2R5E | 3922 | 7140704 | G | 0.30 | 0.0099 | 3.37 |
| PPP2R5E | 3924 | 10152067 | G | 0.29 | 0.0442 | 2.65 |
| GLDN | 4062 | 17648128 | A | 0.57 | 0.0315 | 2.40 |
| GLDN | 4063 | 17648140 | G | 0.57 | 0.0333 | 2.38 |
| A2BP1 | 4118 | 1155959 | C | 0.53 | 0.0313 | 2.40 |
| A2BP1 | 4119 | 12926215 | G | 0.07 | 0.0131 | 0.18 |
| A2BP1 | 4120 | 1492382 | C | 0.07 | 0.0175 | 0.19 |
| A2BP1 | 4121 | 4289011 | C | 0.20 | 0.0453 | 0.38 |
| TMC5 | 4142 | 2353696 | T | 0.47 | 0.0472 | 2.27 |
| EEF2K | 4144 | 8059561 | C | 0.10 | 0.0217 | 0.25 |
| EEF2K | 4145 | 10521118 | A | 0.20 | 0.0205 | 3.63 |
| CDH13 | 4191 | 1862832 | T | 0.27 | 0.0458 | 0.41 |
| CDH13 | 4201 | 1833965 | A | 0.17 | 0.0186 | 0.31 |
| CRISPLD2 | 4214 | 2934477 | A | 0.27 | 0.0458 | 0.41 |
| CA10 | 4226 | 11867674 | G | 0.27 | 0.0056 | 0.30 |
| CA10 | 4229 | 985758 | G | 0.13 | 0.0154 | 0.27 |
| CA10 | 4230 | 4794295 | T | 0.27 | 0.0056 | 0.30 |
| SDK2 | 4263 | 7219778 | A | 0.27 | 0.0173 | 0.35 |
| HRNBP3 | 4274 | 4313839 | C | 0.20 | 0.0453 | 0.38 |
| OSBPL1A | 4308 | 2077984 | C | 0.03 | 0.0414 | 0.14 |
| NEDD4L | 4321 | 1008899 | A | 0.03 | 0.0273 | 0.14 |
| NEDD4L | 4322 | 292456 | G | 0.13 | 0.0395 | 0.32 |
| NEDD4L | 4324 | 4941375 | C | 0.03 | 0.0306 | 0.14 |
| CDH7 | 4355 | 7237421 | T | 0.53 | 0.0248 | 2.49 |
| CDH7 | 4356 | 8092259 | G | 0.50 | 0.0405 | 2.37 |
| PRNT | 4399 | 2065705 | C | 0.21 | 0.0485 | 0.38 |
| PLCB1 | 4412 | 6055513 | G | 0.67 | 0.0282 | 2.51 |
| PLCB1 | 4417 | 16994533 | G | 0.30 | 0.0451 | 2.55 |
| KIF16B | 4463 | 4814474 | A | 0.60 | 0.0224 | 2.54 |
| KIF16B | 4465 | 2328019 | C | 0.23 | 0.0240 | 0.36 |
| KIF16B | 4474 | 12624938 | A | 0.32 | 0.0275 | 2.79 |
| SEC23B | 4485 | 1022681 | A | 0.03 | 0.0079 | 0.10 |
| SEC23B | 4486 | 1555354 | C | 0.07 | 0.0212 | 0.20 |
| PTPRT | 4502 | 6124439 | A | 0.10 | 0.0377 | 0.28 |
| CDH4 | 4539 | 6089573 | A | 0.73 | 0.0096 | 3.30 |
| PDE9A | 4563 | 2245730 | C | 0.70 | 0.0093 | 3.03 |
| PDE9A | 4564 | 12626774 | C | 0.17 | 0.0226 | 0.32 |
| ARVCF | 4569 | 2073746 | A | 0.43 | 0.0080 | 3.06 |
| ARFGAP3 | 4579 | 2024645 | T | 0.63 | 0.0050 | 3.17 |
| ARFGAP3 | 4580 | 5758965 | A | 0.63 | 0.0116 | 2.83 |
| ARFGAP3 | 4583 | 4822208 | T | 0.63 | 0.0276 | 2.49 |
| PACSIN2 | 4584 | 12166809 | A | 0.64 | 0.0031 | 3.51 |
| PACSIN2 | 4585 | 5759013 | A | 0.63 | 0.0041 | 3.25 |
| PACSIN2 | 4586 | 4820494 | C | 0.63 | 0.0041 | 3.25 |
| PACSIN2 | 4587 | 1071960 | C | 0.43 | 0.0210 | 2.62 |
| PACSIN2 | 4588 | 7286299 | A | 0.60 | 0.0469 | 2.27 |

| **TABLE 20: Alleles Influencing Discontinuation from Adverse Events for Ziprasidone** | | | | | | |
|---|---|---|---|---|---|---|
| **Gene Name** | **Seq ID** | **NCBI #** | **Correct** | **Freq. in Discontinuers** | **P** | **Odds Ratio** |
| RP1-21O18.1 | 12 | 2175771 | C | 0.09 | 0.0354 | 0.22 |
| AGBL4-C1 orf165 | 28 | 11205538 | C | 0.59 | 0.0415 | 2.72 |
| SCP2 | 42 | 6588458 | A | 0.14 | 0.0427 | 5.68 |
| FAM78B | 112 | 6662013 | T | 0.14 | 0.0239 | 0.24 |
| FAM78B | 117 | 1002160 | A | 0.41 | 0.0067 | 4.09 |
| FAM78B | 118 | 7521995 | C | 0.40 | 0.0177 | 3.56 |
| FAM78B | 119 | 715421 | A | 0.45 | 0.0071 | 3.91 |
| FAM78B | 120 | 10800198 | C | 0.45 | 0.0181 | 3.28 |
| DPT | 121 | 6427142 | G | 0.00 | 0.0466 | 0.00 |
| PLA2G4A | 137 | 12720662 | T | 0.41 | 0.0019 | 5.15 |
| USH2A | 159 | 1805049 | G | 0.59 | 0.0042 | 4.04 |
| USH2A | 160 | 2274117 | G | 0.59 | 0.0042 | 4.04 |
| USH2A | 162 | 773498 | G | 0.59 | 0.0042 | 4.04 |
| USH2A | 167 | 773042 | T | 0.50 | 0.0074 | 3.80 |
| ESRRG | 171 | 1833036 | A | 0.68 | 0.0127 | 3.47 |
| ESRRG | 172 | 12757165 | G | 0.59 | 0.0035 | 4.18 |
| ESRRG | 173 | 6658528 | T | 0.64 | 0.0448 | 2.68 |
| ESRRG | 174 | 11117612 | G | 0.59 | 0.0042 | 4.04 |
| RYR2 | 197 | 1806641 | C | 0.27 | 0.0463 | 0.36 |
| RYR2 | 202 | 12048201 | G | 0.00 | 0.0236 | 0.00 |
| RYR2 | 209 | 3766871 | A | 0.14 | 0.0427 | 5.68 |
| CHRM3 | 219 | 7513746 | G | 0.23 | 0.0303 | 0.31 |
| CHRM3 | 220 | 6429154 | G | 0.23 | 0.0391 | 0.33 |
| CHRM3 | 222 | 1431719 | G | 0.23 | 0.0391 | 0.33 |
| CHRM3 | 225 | 10802801 | A | 0.23 | 0.0391 | 0.33 |
| FMN2 | 228 | 12118961 | T | 0.73 | 0.0273 | 3.14 |
| FMN2 | 230 | 6698474 | A | 0.68 | 0.0311 | 2.95 |
| FMN2 | 231 | 12123407 | A | 0.68 | 0.0408 | 2.79 |
| FMN2 | 233 | 11591010 | C | 0.00 | 0.0378 | 0.00 |
| PLD5 | 271 | 1548160 | C | 0.14 | 0.0424 | 0.27 |
| PLD5 | 272 | 12128054 | A | 0.14 | 0.0308 | 0.26 |
| DDEF2 | 283 | 10593 | G | 0.18 | 0.0224 | 5.41 |
| BRE | 313 | 7572644 | C | 0.00 | 0.0373 | 0.00 |
| PLEKHH2 | 360 | 12621654 | T | 0.14 | 0.0498 | 0.29 |
| PRKCE | 378 | 1464572 | T | 0.68 | 0.0120 | 3.52 |
| PRKCE | 386 | 13003856 | G | 0.59 | 0.0477 | 2.62 |
| PRKCE | 387 | 6748375 | C | 0.18 | 0.0315 | 0.29 |
| EPAS1 | 393 | 7589621 | A | 0.41 | 0.0115 | 3.69 |
| CCDC85A | 424 | 214041 | T | 0.18 | 0.0404 | 0.31 |
| CTNNA2 | 451 | 53915 | G | 0.50 | 0.0173 | 3.22 |
| CTNNA2 | 454 | 1006607 | G | 0.27 | 0.0068 | 5.33 |
| CTNNA2 | 456 | 2120408 | C | 0.27 | 0.0068 | 5.33 |
| CTNNA2 | 457 | 2165975 | T | 0.27 | 0.0068 | 5.33 |
| NAP5 | 476 | 13414473 | C | 0.59 | 0.0135 | 3.33 |
| UBXD2 | 496 | 6430585 | A | 0.09 | 0.0448 | 0.23 |
| UBXD2 | 497 | 1042712 | C | 0.05 | 0.0174 | 0.12 |
| PLA2R1 | 610 | 2715936 | G | 0.00 | 0.0373 | 0.00 |
| PDE1A | 640 | 833119 | C | 0.27 | 0.0481 | 3.19 |
| PARD3H | 657 | 2878473 | C | 0.32 | 0.0418 | 3.08 |
| ERBB4 | 683 | 4672668 | G | 0.27 | 0.0358 | 0.34 |
| DNER | 711 | 2396644 | A | 0.45 | 0.0224 | 3.13 |
| CNTN6 | 735 | 6798321 | C | 0.36 | 0.0339 | 0.35 |
| CNTN6 | 736 | 11925058 | C | 0.25 | 0.0064 | 0.23 |
| CNTN6 | 737 | 3772339 | T | 0.18 | 0.0142 | 0.25 |
| CNTN6 | 738 | 4286413 | T | 0.17 | 0.0079 | 0.19 |
| CNTN4 | 767 | 12494838 | G | 0.45 | 0.0465 | 2.69 |
| CNTN4 | 771 | 11129386 | A | 0.23 | 0.0391 | 0.33 |
| SLC6A6 | 798 | 11713355 | A | 0.32 | 0.0146 | 3.97 |
| STAC | 823 | 4678874 | G | 0.36 | 0.0350 | 3.05 |
| ULK4 | 839 | 7649806 | A | 0.27 | 0.0463 | 0.36 |
| ERC2 | 902 | 17216615 | C | 0.14 | 0.0427 | 5.68 |
| FHIT | 917 | 802778 | A | 0.00 | 0.0297 | 0.00 |
| FHIT | 929 | 815718 | C | 0.32 | 0.0418 | 3.08 |
| CADPS | 949 | 11925708 | A | 0.64 | 0.0448 | 2.68 |
| CADPS | 950 | 968808 | T | 0.65 | 0.0235 | 3.18 |
| CADPS | 952 | 9825965 | C | 0.64 | 0.0448 | 2.68 |
| SYNPR | 953 | 1812207 | A | 0.68 | 0.0226 | 3.14 |
| SYNPR | 956 | 7618831 | T | 0.60 | 0.0359 | 2.89 |
| CPNE4 | 1059 | 2178385 | A | 0.59 | 0.0264 | 2.95 |
| RAB6B | 1061 | 6765093 | G | 0.14 | 0.0185 | 0.23 |
| EPHB1 | 1063 | 7650345 | G | 0.14 | 0.0393 | 0.27 |
| EPHB1 | 1068 | 1870196 | T | 0.09 | 0.0448 | 0.23 |
| CLSTN2 | 1073 | 6439926 | A | 0.23 | 0.0500 | 0.34 |
| SPSB4 | 1084 | 4683550 | C | 0.14 | 0.0143 | 10.89 |
| SPSB4 | 1086 | 2086180 | G | 0.14 | 0.0427 | 5.68 |
| NLGN1 | 1121 | 692457 | A | 0.64 | 0.0186 | 3.18 |
| NLGN1 | 1122 | 10936780 | G | 0.20 | 0.0050 | 9.00 |
| NLGN1 | 1123 | 692486 | G | 0.64 | 0.0186 | 3.18 |
| NLGN1 | 1124 | 12634066 | A | 0.18 | 0.0074 | 8.22 |
| NLGN1 | 1125 | 11713211 | C | 0.59 | 0.0477 | 2.62 |
| NLGN1 | 1127 | 9828801 | T | 0.14 | 0.0389 | 5.84 |
| HTR3C | 1131 | 6443938 | A | 0.64 | 0.0305 | 2.92 |
| LDB2 | 1177 | 157613 | G | 0.14 | 0.0389 | 5.84 |
| LDB2 | 1179 | 158278 | G | 0.14 | 0.0389 | 5.84 |
| LDB2 | 1180 | 150261 | C | 0.14 | 0.0101 | 11.84 |
| SLIT2 | 1191 | 9992591 | C | 0.64 | 0.0095 | 3.57 |
| SOD3 | 1196 | 17622933 | T | 0.10 | 0.0427 | 0.23 |
| KIAA1239 | 1205 | 1435378 | G | 0.09 | 0.0497 | 0.24 |
| UBE2K | 1214 | 1003212 | T | 0.23 | 0.0313 | 4.06 |
| NPFFR2 | 1234 | 1520503 | T | 0.18 | 0.0187 | 0.26 |
| CXCL9 | 1254 | 10518143 | C | 0.05 | 0.0080 | 0.10 |
| SCD5 | 1261 | 4693474 | C | 0.55 | 0.0263 | 2.95 |
| SCD5 | 1264 | 993380 | A | 0.55 | 0.0263 | 2.95 |
| FAM13A1 | 1281 | 4544678 | C | 0.32 | 0.0311 | 0.34 |
| FAM13A1 | 1282 | 7691186 | G | 0.32 | 0.0311 | 0.34 |
| FAM13A1 | 1283 | 6814344 | G | 0.36 | 0.0448 | 0.37 |
| FAM13A1 | 1284 | 13131633 | T | 0.36 | 0.0448 | 0.37 |
| COL25A1 | 1317 | 2305438 | A | 0.59 | 0.0477 | 2.62 |
| COL25A1 | 1327 | 3096507 | C | 0.15 | 0.0403 | 0.27 |
| ANK2 | 1345 | 967099 | T | 0.18 | 0.0315 | 0.29 |
| ANK2 | 1348 | 362496 | G | 0.09 | 0.0278 | 0.20 |
| GPR103 | 1381 | 13110738 | G | 0.00 | 0.0047 | 0.00 |
| GPR103 | 1382 | 2013332 | A | 0.05 | 0.0361 | 0.14 |
| IL15 | 1405 | 10519613 | A | 0.00 | 0.0466 | 0.00 |
| INPP4B | 1416 | 17015544 | T | 0.23 | 0.0356 | 3.94 |
| INPP4B | 1424 | 1391099 | G | 0.00 | 0.0345 | 0.00 |
| DCLK2 | 1433 | 10014161 | C | 0.80 | 0.0026 | 5.50 |
| DCLK2 | 1436 | 7665011 | A | 0.77 | 0.0052 | 4.43 |
| DCLK2 | 1438 | 11735949 | C | 0.64 | 0.0134 | 3.37 |
| DCLK2 | 1442 | 6535725 | A | 0.68 | 0.0311 | 2.95 |
| CTSO | 1449 | 10517625 | C | 0.32 | 0.0418 | 3.08 |
| FSTL5 | 1471 | 1994770 | A | 0.23 | 0.0303 | 0.31 |
| ENPP6 | 1522 | 7696176 | C | 0.23 | 0.0097 | 0.25 |
| ENPP6 | 1523 | 6831986 | C | 0.23 | 0.0176 | 0.28 |
| ENPP6 | 1524 | 17679120 | A | 0.32 | 0.0408 | 0.36 |
| DNAHS | 1560 | 1445823 | C | 0.59 | 0.0120 | 3.41 |
| CDH10 | 1574 | 7731953 | T | 0.00 | 0.0378 | 0.00 |
| CDH10 | 1576 | 983446 | T | 0.05 | 0.0058 | 0.09 |
| EGFLAM | 1589 | 2434504 | A | 0.64 | 0.0339 | 2.84 |
| EGFLAM | 1591 | 1428263 | C | 0.55 | 0.0396 | 2.82 |
| ITGA1 | 1599 | 13189973 | T | 0.41 | 0.0420 | 2.82 |
| ITGA1 | 1606 | 10513001 | G | 0.45 | 0.0328 | 2.89 |
| VCAN | 1672 | 3096172 | C | 0.05 | 0.0455 | 0.15 |
| GPR98 | 1684 | 10062714 | T | 0.27 | 0.0358 | 0.34 |
| GPR98 | 1687 | 16868980 | C | 0.10 | 0.0032 | 0.13 |
| GPR98 | 1688 | 2366926 | G | 0.14 | 0.0060 | 0.19 |
| FBXL17 | 1708 | 286769 | T | 0.10 | 0.0341 | 0.21 |
| HSD17B4 | 1738 | 2636968 | G | 0.77 | 0.0003 | 6.66 |
| HSD17B4 | 1740 | 32662 | T | 0.23 | 0.0071 | 0.24 |
| HSD17B4 | 1741 | 463098 | A | 0.77 | 0.0005 | 6.28 |
| HSD17B4 | 1742 | 246965 | G | 0.77 | 0.0005 | 6.17 |
| HSD17B4 | 1743 | 246968 | A | 0.68 | 0.0008 | 5.26 |
| LOC340069 | 1746 | 328694 | A | 0.68 | 0.0234 | 3.11 |
| SNCAIP | 1753 | 304379 | C | 0.27 | 0.0114 | 0.27 |
| SNCAIP | 1755 | 1841972 | C | 0.36 | 0.0350 | 3.05 |
| SNCAIP | 1756 | 11241644 | G | 0.36 | 0.0350 | 3.05 |
| SNX2 | 1758 | 4343835 | C | 0.23 | 0.0391 | 0.33 |
| SNX2 | 1760 | 2407403 | G | 0.59 | 0.0191 | 3.13 |
| SNX24 | 1762 | 246286 | T | 0.64 | 0.0059 | 3.88 |
| SNX24 | 1763 | 246266 | C | 0.64 | 0.0062 | 3.92 |
| CTNNAI | 1780 | 1828187 | G | 0.50 | 0.0213 | 3.11 |
| CTNNAI | 1782 | 176382 | G | 0.50 | 0.0251 | 3.00 |
| CTNNAI | 1783 | 10038162 | A | 0.50 | 0.0251 | 3.00 |
| CTNNAI | 1784 | 9327829 | T | 0.50 | 0.0251 | 3.00 |
| CTNNAI | 1785 | 3763016 | G | 0.50 | 0.0251 | 3.00 |
| CTNNAI | 1786 | 10045605 | C | 0.50 | 0.0251 | 3.00 |
| GABRP | 1805 | 3792811 | G | 0.59 | 0.0191 | 3.13 |
| SERPINB6 | 1806 | 1358869 | C | 0.23 | 0.0391 | 0.33 |
| PHACTRI | 1823 | 12527257 | C | 0.05 | 0.0104 | 0.10 |
| PHACTRI | 1824 | 9367368 | C | 0.05 | 0.0111 | 0.10 |
| RNF144B | 1840 | 6927687 | C | 0.05 | 0.0365 | 0.14 |
| RNF144B | 1843 | 531481 | T | 0.41 | 0.0284 | 3.07 |
| RNF144B | 1844 | 527984 | T | 0.40 | 0.0411 | 2.95 |
| TJAPI | 1887 | 1096699 | A | 0.50 | 0.0307 | 2.90 |
| GABRR2 | 1918 | 282128 | A | 0.73 | 0.0207 | 3.29 |
| GABRR2 | 1920 | 6900958 | T | 0.14 | 0.0308 | 0.26 |
| KLHL32 | 1925 | 1206149 | A | 0.59 | 0.0446 | 2.67 |
| NKAIN2 | 1958 | 163284 | A | 0.59 | 0.0120 | 3.41 |
| NKAIN2 | 1959 | 12195023 | T | 0.50 | 0.0028 | 4.43 |
| NKAIN2 | 1961 | 163291 | A | 0.59 | 0.0304 | 2.89 |
| EYA4 | 1977 | 9385647 | C | 0.64 | 0.0066 | 3.79 |
| UTRN | 1997 | 601873 | A | 0.23 | 0.0401 | 0.33 |
| UTRN | 1999 | 760855 | T | 0.09 | 0.0098 | 0.16 |
| UTRN | 2001 | 7765923 | A | 0.09 | 0.0183 | 0.18 |
| UTRN | 2002 | 6906465 | G | 0.45 | 0.0101 | 3.84 |
| SLC22A3 | 2015 | 2048327 | G | 0.50 | 0.0251 | 3.00 |
| PARK2 | 2032 | 6455807 | G | 0.14 | 0.0239 | 0.24 |
| PARK2 | 2034 | 6930880 | C | 0.18 | 0.0404 | 0.31 |
| CARD11 | 2066 | 2679258 | A | 0.27 | 0.0463 | 0.36 |
| CARD11 | 2069 | 4722277 | T | 0.23 | 0.0098 | 0.25 |
| SDKI | 2076 | 10951446 | A | 0.27 | 0.0469 | 0.35 |
| SDKI | 2080 | 651245 | A | 0.55 | 0.0491 | 2.60 |
| NXPHI | 2086 | 7777974 | A | 0.64 | 0.0012 | 4.90 |
| NXPHI | 2087 | 10257265 | C | 0.50 | 0.0143 | 3.35 |
| NXPHI | 2088 | 970526 | G | 0.50 | 0.0212 | 3.22 |
| SCIN | 2092 | 2240571 | G | 0.64 | 0.0448 | 2.68 |
| NPY | 2114 | 16129 | T | 0.27 | 0.0274 | 0.32 |
| NPY | 2115 | 16124 | A | 0.27 | 0.0358 | 0.34 |
| BMPER | 2163 | 11978741 | T | 0.09 | 0.0448 | 0.23 |
| BMPER | 2164 | 6975236 | T | 0.59 | 0.0063 | 3.78 |
| GRB10 | 2215 | 2244347 | C | 0.14 | 0.0141 | 0.22 |
| WBSCR17 | 2233 | 10950240 | A | 0.30 | 0.0337 | 0.33 |
| WBSCR17 | 2237 | 11768233 | A | 0.55 | 0.0363 | 2.77 |
| MAGI2 | 2266 | 6466021 | G | 0.36 | 0.0482 | 2.86 |
| MAGI2 | 2283 | 7789445 | A | 0.23 | 0.0276 | 4.18 |
| CACNA2DI | 2312 | 2368020 | A | 0.45 | 0.0057 | 4.04 |
| CACNA2DI | 2314 | 1468400 | A | 0.45 | 0.0042 | 4.31 |
| CACNA2DI | 2315 | 7788848 | A | 0.45 | 0.0094 | 3.69 |
| PCLO | 2327 | 17282763 | C | 0.18 | 0.0404 | 0.31 |
| PCLO | 2328 | 12539066 | T | 0.55 | 0.0037 | 4.20 |
| PCLO | 2330 | 976714 | T | 0.64 | 0.0030 | 4.30 |
| PCLO | 2331 | 7778238 | C | 0.77 | 0.0071 | 4.22 |
| PCLO | 2332 | 2190045 | C | 0.19 | 0.0030 | 0.19 |
| SEMA3E | 2340 | 2249188 | G | 0.68 | 0.0408 | 2.79 |
| SEMA3E | 2343 | 215274 | C | 0.23 | 0.0176 | 0.28 |
| SEMA3E | 2345 | 3801562 | C | 0.23 | 0.0232 | 0.29 |
| PPP1R9A | 2365 | 6465455 | T | 0.27 | 0.0207 | 0.30 |
| PPP1R9A | 2369 | 961262 | C | 0.27 | 0.0155 | 0.29 |
| DYNCIII | 2379 | 1478984 | G | 0.09 | 0.0261 | 0.20 |
| PONI | 2385 | 13223537 | C | 0.41 | 0.0420 | 2.82 |
| DYNCIII | 2386 | 1382620 | A | 0.09 | 0.0216 | 0.19 |
| DYNCIII | 2387 | 983350 | T | 0.09 | 0.0216 | 0.19 |
| DYNCIII | 2388 | 983352 | T | 0.09 | 0.0261 | 0.20 |
| CUXI | 2403 | 425964 | T | 0.27 | 0.0068 | 5.33 |
| CUX1 | 2405 | 427039 | T | 0.27 | 0.0278 | 3.70 |
| SLC13A1 | 2427 | 2222510 | A | 0.14 | 0.0048 | 0.18 |
| SLC13A1 | 2428 | 2462152 | G | 0.14 | 0.0060 | 0.19 |
| SLC13A1 | 2429 | 6963735 | C | 0.68 | 0.0003 | 6.00 |
| PTPRN2 | 2518 | 6943534 | T | 0.50 | 0.0488 | 2.62 |
| PTPRN2 | 2519 | 4909289 | A | 0.45 | 0.0475 | 2.70 |
| PTPRN2 | 2524 | 6960916 | A | 0.50 | 0.0488 | 2.62 |
| PTPRN2 | 2525 | 6966663 | G | 0.50 | 0.0488 | 2.62 |
| PTPRN2 | 2532 | 10265417 | T | 0.64 | 0.0429 | 2.72 |
| PTPRN2 | 2533 | 10081235 | A | 0.64 | 0.0448 | 2.68 |
| CSMD1 | 2538 | 4875250 | G | 0.41 | 0.0004 | 6.82 |
| PSD3 | 2608 | 6992325 | C | 0.41 | 0.0115 | 3.69 |
| PSD3 | 2611 | 1492289 | C | 0.64 | 0.0095 | 3.57 |
| PSD3 | 2612 | 4921968 | A | 0.55 | 0.0263 | 2.95 |
| PSD3 | 2614 | 11778310 | G | 0.45 | 0.0328 | 2.89 |
| PEBP4 | 2626 | 4872539 | C | 0.50 | 0.0074 | 3.75 |
| PEBP4 | 2627 | 7829416 | G | 0.50 | 0.0093 | 3.63 |
| PEBP4 | 2628 | 6558183 | T | 0.45 | 0.0009 | 5.50 |
| UNC5D | 2641 | 1439158 | G | 0.14 | 0.0498 | 0.29 |
| PRKDC | 2650 | 4873770 | T | 0.09 | 0.0079 | 3.19 |
| SNTGI | 2654 | 6999728 | A | 0.32 | 0.0036 | 5.44 |
| SNTGI | 2655 | 2106156 | A | 0.05 | 0.0130 | 0.11 |
| SNTGI | 2656 | 1000638 | T | 0.05 | 0.0174 | 0.12 |
| SNTGI | 2660 | 12548940 | C | 0.05 | 0.0284 | 0.13 |
| SNTGI | 2670 | 7832799 | A | 0.41 | 0.0497 | 2.72 |
| NKAIN3 | 2691 | 2084799 | C | 0.27 | 0.0359 | 0.34 |
| NKAIN3 | 2693 | 7835901 | C | 0.64 | 0.0186 | 3.18 |
| GRHL2 | 2776 | 4734037 | C | 0.27 | 0.0481 | 3.19 |
| GRHL2 | 2778 | 11994917 | T | 0.55 | 0.0233 | 3.03 |
| GRHL2 | 2779 | 1131862 | G | 0.36 | 0.0222 | 3.38 |
| NCALD | 2780 | 2387620 | G | 0.45 | 0.0328 | 2.89 |
| NCALD | 2781 | 2226401 | C | 0.36 | 0.0075 | 4.25 |
| BAALC | 2787 | 13279226 | A | 0.32 | 0.0418 | 3.08 |
| SAMD12 | 2829 | 2460983 | G | 0.32 | 0.0418 | 3.08 |
| DDEF1 | 2858 | 3871 | C | 0.45 | 0.0465 | 2.69 |
| DDEF1 | 2863 | 7838372 | T | 0.27 | 0.0170 | 4.25 |
| DDEF1 | 2864 | 6991714 | A | 0.05 | 0.0361 | 0.14 |
| DDEF1 | 2867 | 2670886 | C | 0.30 | 0.0281 | 3.64 |
| DDEF1 | 2874 | 2670882 | G | 0.27 | 0.0481 | 3.19 |
| COL22A1 | 2907 | 4475485 | T | 0.18 | 0.0224 | 5.41 |
| COL22A1 | 2914 | 13279213 | A | 0.41 | 0.0420 | 2.82 |
| SMARCA2 | 2929 | 7048976 | G | 0.36 | 0.0448 | 0.37 |
| PTPRD | 2941 | 12555585 | G | 0.32 | 0.0256 | 3.47 |
| PTPRD | 2943 | 10124689 | T | 0.14 | 0.0498 | 0.29 |
| TRPM6 | 3045 | 7045338 | T | 0.18 | 0.0315 | 0.29 |
| TRPM6 | 3049 | 7024831 | A | 0.09 | 0.0129 | 0.17 |
| PCSKS | 3058 | 10124834 | T | 0.05 | 0.0455 | 0.15 |
| NTRK2 | 3079 | 1899641 | G | 0.09 | 0.0448 | 0.23 |
| DAPK1 | 3082 | 6560011 | C | 0.18 | 0.0142 | 0.25 |
| SVEP1 | 3124 | 963143 | A | 0.59 | 0.0446 | 2.67 |
| SVEP1 | 3126 | 10759422 | C | 0.36 | 0.0411 | 2.95 |
| DAB2IP | 3143 | 10985431 | T | 0.32 | 0.0076 | 4.60 |
| TSCI | 3181 | 11243940 | G | 0.50 | 0.0354 | 2.80 |
| TSCI | 3182 | 7035308 | A | 0.50 | 0.0354 | 2.80 |
| KCNT1 | 3195 | 497547 | G | 0.18 | 0.0224 | 5.41 |
| ABCA2 | 3201 | 12337910 | G | 0.25 | 0.0217 | 4.47 |
| PITRM1 | 3215 | 3740607 | G | 0.32 | 0.0311 | 0.34 |
| PITRM1 | 3217 | 4242746 | T | 0.09 | 0.0087 | 0.16 |
| PITRM1 | 3220 | 3829918 | G | 0.14 | 0.0239 | 0.24 |
| MYO3A | 3259 | 7896978 | C | 0.09 | 0.0354 | 0.22 |
| MYO3A | 3260 | 11014839 | A | 0.09 | 0.0354 | 0.22 |
| MY03A | 3265 | 6482521 | C | 0.14 | 0.0101 | 11.84 |
| MYO3A | 3266 | 872754 | G | 0.14 | 0.0101 | 11.84 |
| MYO3A | 3270 | 1339814 | T | 0.36 | 0.0411 | 2.95 |
| MYO3A | 3271 | 11014917 | A | 0.05 | 0.0174 | 0.12 |
| MYO3A | 3273 | 5012431 | C | 0.41 | 0.0358 | 0.36 |
| MYO3A | 3274 | 12773856 | C | 0.41 | 0.0358 | 0.36 |
| MYO3A | 3276 | 2139795 | G | 0.41 | 0.0358 | 0.36 |
| MYO3A | 3278 | 3758449 | C | 0.41 | 0.0358 | 0.36 |
| MYO3A | 3287 | 3781117 | C | 0.05 | 0.0284 | 0.13 |
| CDH23 | 3307 | 10999947 | A | 0.45 | 0.0328 | 2.89 |
| VTI1A | 3395 | 2044366 | C | 0.00 | 0.0070 | 0.00 |
| VTI1A | 3396 | 11196051 | G | 0.05 | 0.0104 | 0.10 |
| VTI1A | 3397 | 10885371 | A | 0.05 | 0.0321 | 0.14 |
| ATRNL1 | 3418 | 2068463 | C | 0.27 | 0.0207 | 0.30 |
| ATE1 | 3431 | 1693688 | C | 0.68 | 0.0013 | 4.94 |
| ATE1 | 3432 | 2935718 | C | 0.64 | 0.0186 | 3.18 |
| ATE1 | 3433 | 7086628 | C | 0.64 | 0.0448 | 2.68 |
| ATE1 | 3435 | 1219505 | T | 0.68 | 0.0127 | 3.47 |
| MICAL2 | 3475 | 2010463 | A | 0.64 | 0.0002 | 6.07 |
| MICAL2 | 3476 | 10831750 | T | 0.50 | 0.0115 | 3.47 |
| MICAL2 | 3477 | 7117171 | G | 0.50 | 0.0074 | 3.75 |
| MICAL2 | 3478 | 17477949 | T | 0.25 | 0:0120 | 0.26 |
| MICAL2 | 3479 | 954428 | G | 0.64 | 0.0030 | 4.30 |
| DLG2 | 3540 | 11233954 | A | 0.00 | 0.0186 | 0.00 |
| DLG2 | 3547 | 11234221 | A | 0.59 | 0.0191 | 3.13 |
| DLG2 | 3548 | 11234222 | A | 0.59 | 0.0191 | 3.13 |
| OPCML | 3578 | 6590699 | T | 0.41 | 0.0185 | 3.36 |
| OPCML | 3581 | 7935298 | A | 0.41 | 0.0185 | 3.36 |
| OPCML | 3582 | 4937780 | G | 0.41 | 0.0185 | 3.36 |
| OPCML | 3586 | 12792779 | T | 0.35 | 0.0228 | 3.55 |
| OPCML | 3587 | 7108670 | C | 0.41 | 0.0185 | 3.36 |
| OPCML | 3589 | 4936195 | G | 0.40 | 0.0007 | 6.57 |
| TSPAN9 | 3595 | 10774140 | C | 0.09 | 0.0354 | 0.22 |
| LOC729025 | 3618 | 10219568 | A | 0.64 | 0.0448 | 2.68 |
| PIK3C2G | 3633 | 11043979 | C | 0.60 | 0.0474 | 2.72 |
| ITPR2 | 3637 | 16930669 | C | 0.20 | 0.0380 | 4.38 |
| ITPR2 | 3639 | 7955200 | C | 0.45 | 0.0224 | 3.13 |
| CNOT2 | 3642 | 10879096 | G | 0.05 | 0.0455 | 0.15 |
| KCNC2 | 3661 | 10735985 | G | 0.64 | 0.0134 | 3.37 |
| MTIF3 | 3688 | 3759433 | T | 0.32 | 0.0256 | 3.47 |
| SLC46A3 | 3693 | 1617234 | C | 0.64 | 0.0448 | 2.68 |
| UBL3 | 3697 | 9551767 | C | 0.14 | 0.0081 | 0.20 |
| UBL3 | 3698 | 9578147 | G | 0.14 | 0.0081 | 0.20 |
| NBEA | 3719 | 7320580 | G | 0.50 | 0.0143 | 3.35 |
| NBEA | 3720 | 7988705 | A | 0.50 | 0.0173 | 3.22 |
| NBEA | 3722 | 3818423 | C | 0.50 | 0.0173 | 3.22 |
| NBEA | 3723 | 17769661 | T | 0.36 | 0.0411 | 2.95 |
| TRPC4 | 3732 | 1538146 | T | 0.23 | 0.0232 | 0.29 |
| GPC5 | 3792 | 17267453 | C | 0.14 | 0.0427 | 5.68 |
| GPC6 | 3812 | 9301916 | C | 0.09 | 0.0098 | 0.16 |
| GPC6 | 3813 | 9524268 | T | 0.09 | 0.0098 | 0.16 |
| NALCN | 3823 | 16958230 | G | 0.23 | 0.0046 | 7.16 |
| NALCN | 3824 | 625331 | C | 0.27 | 0.0278 | 3.70 |
| NALCN | 3830 | 2390576 | G | 0.41 | 0.0477 | 0.38 |
| NALCN | 3832 | 1538240 | C | 0.80 | 0.0215 | 3.79 |
| NALCN | 3833 | 518212 | G | 0.18 | 0.0244 | 0.27 |
| ITGBL1 | 3838 | 7998372 | T | 0.82 | 0.0079 | 4.50 |
| NOVA1 | 3857 | 1473722 | G | 0.05 | 0.0284 | 0.13 |
| NOVA1 | 3858 | 17111388 | C | 0.05 | 0.0321 | 0.14 |
| NOVA1 | 3859 | 3794485 | C | 0.05 | 0.0284 | 0.13 |
| NPAS3 | 3867 | 243284 | G | 0.59 | 0.0477 | 2.62 |
| NPAS3 | 3871 | 10133530 | A | 0.27 | 0.0278 | 3.70 |
| GNG2 | 3907 | 4898721 | C | 0.05 | 0.0361 | 0.14 |
| GNG2 | 3908 | 10138800 | A | 0.64 | 0.0339 | 2.84 |
| RGS6 | 3931 | 36340 | A | 0.25 | 0.0460 | 0.33 |
| RGS6 | 3935 | 11623588 | T | 0.73 | 0.0111 | 3.70 |
| RGS6 | 3936 | 10151019 | A | 0.09 | 0.0448 | 0.23 |
| RGS6 | 3938 | 1076317 | C | 0.77 | 0.0132 | 3.78 |
| RGS6 | 3939 | 1568404 | T | 0.77 | 0.0176 | 3.58 |
| RGS6 | 3942 | 1520332 | T | 0.18 | 0.0244 | 0.27 |
| RGS6 | 3946 | 1099968 | G | 0.14 | 0.0424 | 0.27 |
| RGS6 | 3948 | 2190508 | C | 0.14 | 0.0498 | 0.29 |
| KCNK10 | 3962 | 12587003 | C | 0.36 | 0.0311 | 3.17 |
| CCDC88C | 3987 | 11160006 | C | 0.23 | 0.0391 | 0.33 |
| CCDC88C | 3988 | 10139287 | A | 0.61 | 0.0048 | 4.40 |
| ATP10A | 4015 | 4906620 | C | 0.05 | 0.0135 | 0.11 |
| RYR3 | 4018 | 2251107 | G | 0.59 | 0.0191 | 3.13 |
| RYR3 | 4029 | 4780144 | C | 0.09 | 0.0079 | 0.38 |
| GLDN | 4057 | 16964316 | A | 0.14 | 0.0389 | 5.84 |
| GLDN | 4059 | 16964319 | T | 0.14 | 0.0389 | 5.84 |
| GLDN | 4060 | 10163098 | G | 0.14 | 0.0389 | 5.84 |
| GLDN | 4061 | 12443092 | C | 0.14 | 0.0389 | 5.84 |
| CGNL1 | 4068 | 4774257 | G | 0.05 | 0.0135 | 0.11 |
| RORA | 4078 | 12914584 | G | 0.59 | 0.0027 | 4.33 |
| RORA | 4080 | 1657792 | A | 0.32 | 0.0311 | 0.34 |
| SH3GL3 | 4107 | 17158811 | A | 0.14 | 0.001 | 2.89 |
| EEF2K | 4148 | 4275840 | T | 0.05 | 0.0409 | 0.15 |
| USP10 | 4209 | 3829554 | G | 0.18 | 0.0315 | 0.29 |
| CA10 | 4231 | 203010 | T | 0.32 | 0.0311 | 0.34 |
| CA10 | 4233 | 203042 | A | 0.64 | 0.0448 | 2.68 |
| CA10 | 4240 | 1503058 | A | 0.30 | 0.0192 | 0.29 |
| ZFP161 | 4285 | 12605370 | C | 0.18 | 0.0224 | 5.41 |
| ZFP161 | 4286 | 12604181 | T | 0.23 | 0.0276 | 4.18 |
| CCBE1 | 4337 | 12969965 | A | 0.36 | 0.0222 | 3.38 |
| CDH7 | 4349 | 7241038 | T | 0.73 | 0.0152 | 3.50 |
| CDH7 | 4351 | 976882 | A | 0.73 | 0.0199 | 3.36 |
| CDH7 | 4352 | 12970791 | T | 0.73 | 0.0207 | 3.29 |
| DOK6 | 4379 | 11662555 | G | 0.27 | 0.0358 | 0.34 |
| DOK6 | 4380 | 12959721 | A | 0.27 | 0.0463 | 0.36 |
| MACROD2 | 4451 | 2193028 | C | 0.00 | 0.0466 | 0.00 |
| SEC23B | 4487 | 2273525 | T | 0.14 | 0.0389 | 5.84 |
| PTPRT | 4505 | 6065520 | T | 0.05 | 0.0104 | 0.10 |
| PTPRT | 4512 | 6072869 | G | 0.32 | 0.0418 | 3.08 |
| SNAI1 | 4524 | 6020157 | A | 0.32 | 0.0418 | 3.08 |
| SNAI1 | 4527 | 1543442 | A | 0.23 | 0.0500 | 0.34 |
| SLC37A1 | 4560 | 401396 | C | 0.45 | 0.0224 | 3.13 |
| EFCAB6 | 4601 | 4823125 | A | 0.45 | 0.0181 | 3.28 |
| EFCAB6 | 4603 | 137733 | T | 0.32 | 0.0311 | 0.34 |
| EFCAB6 | 4606 | 5764214 | C | 0.27 | 0.0358 | 0.34 |
| EFCAB6 | 4611 | 8141695 | A | 0.45 | 0.0465 | 2.69 |

| **TABLE A: Summary of SNPs (NCBI Human Genome Reference Assembly Build 36.3)** | | | | | | | |
|---|---|---|---|---|---|---|---|
| **Seq ID** | **Gene** | **Chr** | **Position (BP)** | **Seq ID** | **Gene** | **Chr** | **Position (BP)** |
| 1 | PIK3CD | 1 | 9,713,582 | 2 | KFF1B | 1 | 10,204,773 |
| 3 | KIF1B | 1 | 10,230,040 | 4 | KIF1B | 1 | 10,27,299 |
| 5 | KIF1B | 1 | 10,308,058 | 6 | KIF1B | 1 | 10,314,123 |
| 7 | RP1-21O18.1 | 1 | 15,160,805 | 8 | RP1-21O18.1 | 1 | 15,178,638 |
| 9 | RP1-21O18.1 | 1 | 15,204,190 | 10 | RPl.21O18.1 | 1 | 15,225,545 |
| 11 | RP1-21O18.1 | 1 | 15,227,828 | 12 | RP1-21O18.1 | 1 | 15,230,092 |
| 13 | RP1-21O18.1 | 1 | 15,265,882 | 14 | RPl-21O18.1 | 1 | 15,278,076 |
| 15 | RP1-21O18.1 | 1 | 15,281,743 | 16 | RP1-21O18.1 | 1 | 15,285,942 |
| 17 | RP1-21O18.1 | 1 | 15,287,000 | 18 | RPl-21O18.1 | 1 | 15,311,577 |
| 19 | EPHB2 | 1 | 22,976,971 | 20 | EPHB2 | 1 | 22,984,956 |
| 21 | EPHB2 | 1 | 23,015,083 | 22 | EPHB2 | 1 | 23,106,187 |
| 23 | EPHB2 | 1 | 23,107,533 | 24 | AGBL4-ClORF165 | 1 | 48,833,568 |
| 25 | AGBL4-ClORF165 | 1 | 48,864,626 | 26 | AGBL4-ClORF165 | 1 | 48,885,834 |
| 27 | AGBL4-ClORF165 | 1 | 49,006,963 | 28 | AGBL4-ClORF165 | 1 | 49,010181 |
| 29 | AGBL4-ClORF165 | 1 | 49,012,555 | 30 | AGBL4-ClORF165 | 1 | 49,046,634 |
| 31 | AGBL4-ClORF165 | 1 | 49,108,184 | 32 | AGBL4-ClORF165 | 1 | 49,109,043 |
| 33 | AGBL4-ClORF165 | 1 | 49,124,764 | 34 | AGBL4-ClORF165 | 1 | 49,132,460 |
| 35 | AGBL4-ClORF165 | 1 | 49,133,207 | 36 | AGBL4-ClORF165 | 1 | 49,150,727 |
| 37 | AGBL4-ClORF165 | 1 | 49,214,610 | 38 | AGBL4-ClORF165 | 1 | 49,243,353 |
| 39 | SCP2 | 1 | 53,226,518 | 40 | SCP2 | 1 | 53,228,487 |
| 41 | SCP2 | 1 | 53,261,152 | 42 | SCP2 | 1 | 53,283,563 |
| 43 | SCP2 | 1 | 53,284,873 | 44 | SCP2 | 1 | 53,285,107 |
| 45 | SCP2 | 1 | 53,286,570 | 46 | SCP2 | 1 | 53,290,585 |
| 47 | SCP2 | 1 | 53,290,637 | 48 | LRP8 | 1 | 53,485,315 |
| 49 | LRP8 | 1 | 53,486,137 | 50 | LRP8 | 1 | 53,495,778 |
| 51 | LRP8 | 1 | 53,495,937 | 52 | LRP8 | 1 | 53,504,903 |
| 53 | LRP8 | 1 | 53,519,183 | 54 | LRP8 | 1 | 53,524,722 |
| 55 | LRP8 | 1 | 53,527,479 | 56 | LRP8 | 1 | 53,535,369 |
| 57 | LRP8 | 1 | 53,558,737 | 58 | LRP8 | 1 | 53,581,246 |
| 59 | LRP8 | 1 | 53,581,281 | 60 | LRP8 | 1 | 53,581,618 |
| 61 | ELTD1 | 1 | 79,151,010 | 62 | ELTD1 | 1 | 79,153,054 |
| 63 | ELTD1 | 1 | 79,165,344 | 64 | ELTD1 | 1 | 79,175,923 |
| 65 | ELTD1 | 1 | 79,246,223 | 66 | ELTD1 | 1 | 79,256,985 |
| 67 | ELTD1 | 1 | 79,272,148 | 68 | PRKACB | 1 | 84,299,506 |
| 69 | PRKACB | 1 | 84,301,022 | 70 | PRKACB | 1 | 94,306,260 |
| 71 | PRKACB | 1 | 84,306,347 | 72 | PRKACB | 1 | 84,323,437 |
| 73 | PRKACB | 1 | 84,334,853 | 74 | PRKACB | 1 | 84,343,593 |
| 75 | PRKACB | 1 | 84,366,987 | 76 | PRKACB | 1 | 84,435,529 |
| 77 | PRKACB | 1 | 84,450,848 | 78 | SYT6 | 1 | 114,452,599 |
| 79 | SYT6 | 1 | 114,478,552 | 80 | NGF | 1 | 115,631,509 |
| 81 | NGF | 1 | 115,658,774 | 82 | NGF | 1 | 115,664,140 |
| 83 | NGF | 1 | 115,672,877 | 84 | NGF | 1 | 115,679,147 |
| 85 | SLC22A15 | 1 | 116,329,613 | 86 | SLC22A15 | 1 | 116,353,502 |
| 87 | PTGFRN | 1 | 117,246,888 | 88 | PTGFRN | 1 | 117,267,288 |
| 89 | PTGFRN | 1 | 117,271,304 | 90 | PTGFRN | 1 | 117,272,017 |
| 91 | PTGFRN | 1 | 117,273,572 | 92 | PTGFRN | 1 | 117,277,238 |
| 93 | PTGFRN | 1 | 117,284,884 | 94 | PTGFRN | 1 | 117,316,638 |
| 95 | CGN | 1 | 149,743,817 | 96 | KCNN3 | 1 | 152,950,730 |
| 97 | KCNN3 | 1 | 152,954,821 | 98 | ATF6 | 1 | 160,027,900 |
| 99 | ATF6 | 1 | 160,052,935 | 100 | ATF6 | 1 | 160,062,017 |
| 101 | ATF6 | 1 | 160,062,520 | 102 | ATF6 | 1 | 160,101,070 |
| 103 | ATF6 | 1 | 160,102,111 | 104 | ATF6 | 1 | 160,107,168 |
| 105 | ATF6 | 1 | 160,108,831 | 106 | ATF6 | 1 | 160,118,977 |
| 107 | ATF6 | 1 | 160,143,072 | 108 | ATF6 | 1 | 160,147,921 |
| 109 | ATF6 | 1 | 160,193,261 | 110 | OLFML2B | 1 | 160,222,129 |
| III | OLFML2B | 1 | 160,247,126 | 112 | FAM78B | 1 | 164,309,282 |
| 113 | FAM78B | 1 | 164,314,302 | 114 | FAM78B | 1 | 164,348,193 |
| 115 | FAM78B | 1 | 164,348,737 | 116 | FAM78B | 1 | 164,350,112 |
| 117 | FAM78B | 1 | 164,364,382 | 118 | FAM78B | 1 | 164,365,940 |
| 119 | FAM78B | 1 | 164,388,390 | 120 | FAM78B | 1 | 164,392,047 |
| 121 | DPT | 1 | 166,937,301 | 122 | DPT | 1 | 166,944,971 |
| 123 | DPT | 1 | 166,954,638 | 124 | DPT | 1 | 166,961,651 |
| 125 | DPT | 1 | 166,964,218 | 126 | SEC16B | 1 | 176,201,673 |
| 127 | SEC16B | 1 | 176,201,898 | 128 | SEC16B | 1 | 176,210,214 |
| 129 | SEC16B | 1 | 176,210,435 | 130 | SEC16B | 1 | 176,215,337 |
| 131 | RALGPS2 | 1 | 176,976,589 | 132 | RALGPS2 | 1 | 177,012,234 |
| 133 | RALGPS2 | 1 | 177,114,846 | 134 | MR1 | 1 | 179,279,594 |
| 135 | PLA2G4A | 1 | 185,173,669 | 136 | PLA2G4A | 1 | 185,178,610 |
| 137 | PLA2G4A | 1 | 185,200,715 | 138 | KCNK2 | 1 | 213,266,926 |
| 139 | KCNK2 | 1 | 213,267,433 | 140 | KCNK2 | 1 | 213,269,771 |
| 141 | KCNK2 | 1 | 213,286,968 | 142 | KCNK2 | 1 | 213,302,819 |
| 143 | KCNK2 | 1 | 213,304,166 | 144 | KCNK2 | 1 | 213,312,765 |
| 145 | KCNK2 | 1 | 213,332,299 | 146 | KCNK2 | 1 | 213,336,427 |
| 147 | KCNK2 | 1 | 213,347,737 | 148 | KCNK2 | 1 | 213,393,108 |
| 149 | KCNK2 | 1 | 213,426,881 | 150 | KCNK2 | 1 | 213,474,680 |
| 151 | USH2A | 1 | 213,537,370 | 152 | USH2A | 1 | 213,895,122 |
| 153 | USH2A | 1 | 213,896,517 | 154 | USH2A | 1 | 213,911,770 |
| 155 | USH2A | 1 | 213,954,742 | 156 | USH2A | 1 | 213,955,280 |
| 157 | USH2A | 1 | 214,209,606 | 158 | USH2A | 1 | 214,404,156 |
| 159 | USH2A | 1 | 214,415,387 | 160 | USH2A | 1 | 214,429,760 |
| 161 | USH2A | 1 | 214,464,725 | 162 | USH2A | 1 | 214,466,781 |
| 163 | USH2A | 1 | 214,476,504 | 164 | USH2A | 1 | 214,498,585 |
| 165 | USH2A | 1 | 214,498,863 | 166 | USH2A | 1 | 214,498,944 |
| 167 | USH2A | 1 | 214,506,295 | 168 | USH2A | 1 | 214,506,786 |
| 169 | USH2A | 1 | 214,509,837 | 170 | USH2A | 1 | 214,572,091 |
| 171 | ESRRG | 1 | 214,773,635 | 172 | ESRRG | 1 | 214,783,160 |
| 173 | ESRRG | 1 | 214,788,713 | 174 | ESRRG | 1 | 214,788,951 |
| 175 | ESRRG | 1 | 214,789,245 | 176 | ESRRG | 1 | 214,811,868 |
| 177 | ESRRG | 1 | 214,827,730 | 178 | ESRRG | 1 | 214,836,783 |
| 179 | ESRRG | 1 | 214,886,050 | 180 | ESRRG | 1 | 214,891,848 |
| 181 | ESRRG | 1 | 214,950,266 | 182 | ESRRG | 1 | 214,951,990 |
| 183 | ESRRG | 1 | 214,952,115 | 184 | ESRRG | 1 | 214,962,423 |
| 185 | SLC35F3 | 1 | 232,371,799 | 186 | SLC35F3 | 1 | 232,375,209 |
| 187 | SLC35F3 | 1 | 232,397,501 | 188 | SLC35F3 | 1 | 232,500,781 |
| 189 | SLC35F3 | 1 | 232,515,592 | 190 | SLC35F3 | 1 | 232,524,008 |
| 191 | SLC35F3 | 1 | 232,525,097 | 192 | GNG4 | 1 | 233,780,076 |
| 193 | GNG4 | 1 | 233,794,439 | 194 | GNG4 | 1 | 233,795,319 |
| 195 | GNG4 | 1 | 233,825,193 | 196 | RYR2 | 1 | 235,343,646 |
| 197 | RYR2 | 1 | 235,413,480 | 198 | RYR2 | 1 | 235,435,907 |
| 199 | RYR2 | 1 | 235,441,114 | 200 | RYR2 | 1 | 235,500,248 |
| 201 | RYR2 | 1 | 235,513,475 | 202 | RYR2 | 1 | 235,568,481 |
| 203 | RYR2 | 1 | 235,614,223 | 204 | RYR2 | 1 | 235,616,144 |
| 205 | RYR2 | 1 | 235,661,657 | 206 | RYR2 | 1 | 235,725,107 |
| 207 | RYR2 | 1 | 235,743,969 | 208 | RYR2 | 1 | 235,793,994 |
| 209 | RYR2 | 1 | 235,844,707 | 210 | RYR2 | 1 | 235,887,079 |
| 211 | RYR2 | 1 | 235,891,567 | 212 | RYR2 | 1 | 235,942,911 |
| 213 | RYR2 | 1 | 235,964,467 | 214 | RYR2 | 1 | 235,999,612 |
| 215 | RYR2 | 1 | 236,061,906 | 216 | CHRM3 | 1 | 237,894,973 |
| 217 | CHRM3 | 1 | 237,906,730 | 218 | CHRM3 | 1 | 237,924,147 |
| 219 | CHRM3 | 1 | 237,929,034 | 220 | CHRM3 | 1 | 237,943,889 |
| 221 | CHRM3 | 1 | 237,944,344 | 222 | CHRM3 | 1 | 237,947,826 |
| 223 | CHRM3 | 1 | 237,952,433 | 224 | CHRM3 | 1 | 237,958,134 |
| 225 | CHRM3 | 1 | 237,969,464 | 226 | CHRM3 | 1 | 237,996,528 |
| 227 | CHRM3 | 1 | 238,001,974 | 228 | FMN2 | 1 | 238,340,274 |
| 229 | FMN2 | 1 | 238,349,704 | 230 | FMN2 | 1 | 238,361,987 |
| 231 | FMN2 | 1 | 238,362,705 | 232 | FMN2 | 1 | 238,386,530 |
| 233 | FMN2 | 1 | 238,407,239 | 234 | FMN2 | 1 | 238,426,833 |
| 235 | FMN2 | 1 | 238,443,154 | 236 | FMN2 | 1 | 238,455,099 |
| 237 | FMN2 | 1 | 238,486,712 | 238 | FMN2 | 1 | 238,494,888 |
| 239 | FMN2 | 1 | 238,512,719 | 240 | FMN2 | 1 | 238,516,765 |
| 241 | FMN2 | 1 | 238,519,250 | 242 | FMN2 | 1 | 238,520,251 |
| 243 | FMN2 | 1 | 238,525,003 | 244 | FMN2 | 1 | 238,559,357 |
| 245 | FMN2 | 1 | 238,559,913 | 246 | FMN2 | 1 | 238,564,221 |
| 247 | FMN2 | 1 | 238,564,870 | 248 | FMN2 | 1 | 238,669,725 |
| 249 | FMN2 | 1 | 238,670,023 | 250 | FMN2 | 1 | 238,703,639 |
| 251 | FMN2 | 1 | 238,704,194 | 252 | RGS7 | 1 | 239,024,094 |
| 253 | RGS7 | 1 | 239,026,696 | 254 | RGS7 | 1 | 239,049,800 |
| 255 | RGS7 | 1 | 239,145,433 | 256 | RGS7 | 1 | 239,198,639 |
| 257 | RGS7 | 1 | 239,202,133 | 258 | RGS7 | 1 | 239,215,583 |
| 259 | RGS7 | 1 | 239,228,398 | 260 | RGS7 | 1 | 239,576,418 |
| 261 | RGS7 | 1 | 239,588,948 | 262 | PLD5 | 1 | 240,358,903 |
| 263 | PLD5 | 1 | 240,452,068 | 264 | PLD5 | 1 | 240,454,472 |
| 265 | PLD5 | 1 | 240,454,888 | 266 | PLD5 | 1 | 240,458,406 |
| 267 | PLD5 | 1 | 240,632,975 | 268 | PLD5 | 1 | 240,640,372 |
| 269 | PLD5 | 1 | 240,643,721 | 270 | PLD5 | 1 | 240,694,244 |
| 271 | PLD5 | 1 | 240,708,546 | 272 | PLD5 | 1 | 240,723,055 |
| 273 | C2ORF46 | 2 | 8,262,682 | 274 | C2ORF46 | 2 | 8,321,006 |
| 275 | C2ORF46 | 2 | 8,342,299 | 276 | C2ORF46 | 2 | 8,354,700 |
| 277 | C2ORF46 | 2 | 8,355,126 | 278 | C2ORF46 | 2 | 8,357,838 |
| 279 | C2ORF46 | 2 | 8,383,937 | 280 | C2ORF46 | 2 | 8,384,366 |
| 281 | DDEF2 | 2 | 9,320,439 | 282 | DDEF2 | 2 | 9,458,300 |
| 283 | DDEF2 | 2 | 9,463,481 | 284 | DDEF2 | 2 | 9,464,814 |
| 285 | HPCAL1 | 2 | 10,301,740 | 286 | KCNF1 | 2 | 10,952,881 |
| 287 | KCNF1 | 2 | 10,982,079 | 288 | NTSR2 | 2 | 11,726,315 |
| 289 | KLHL29 | 2 | 23,504,556 | 290 | KLHL29 | 2 | 23,504,709 |
| 291 | KLHL29 | 2 | 23,504,805 | 292 | KLHL29 | 2 | 23,507,416 |
| 293 | KLHL29 | 2 | 23,519,201 | 294 | KLHL29 | 2 | 23,533,545 |
| 295 | KLHL29 | 2 | 23,634,723 | 296 | KLHL29 | 2 | 23,641,228 |
| 297 | KLHL29 | 2 | 23,648,425 | 298 | KLHL29 | 2 | 23,782,141 |
| 299 | KLHL29 | 2 | 23,784,540 | 300 | KLHL29 | 2 | 23,794,852 |
| 301 | KLHL29 | 2 | 23,801,093 | 302 | ASXL2 | 2 | 25,824,008 |
| 303 | ASXL2 | 2 | 25,830,860 | 304 | ASXL2 | 2 | 25,889,540 |
| 305 | ASXL2 | 2 | 25,909,377 | 306 | KCNK3 | 2 | 26,772,593 |
| 307 | KCNK3 | 2 | 26,782,315 | 308 | KCNK3 | 2 | 26,798,797 |
| 309 | KCNK3 | 2 | 26,807,044 | 310 | DPYSL5 | 2 | 26,936,993 |
| 311 | DPYSL5 | 2 | 26,991,120 | 312 | DPYSL5 | 2 | 27,023,044 |
| 313 | BRE | 2 | 28,173,537 | 314 | BRE | 2 | 28,173,685 |
| 315 | BRE | 2 | 28,178,705 | 316 | BRE | 2 | 28,179,762 |
| 317 | BRE | 2 | 28,195,657 | 318 | BRE | 2 | 28,212,255 |
| 319 | BRE | 2 | 28,212,485 | 320 | BRE | 2 | 28,243,350 |
| 321 | BRE | 2 | 28,281,970 | 322 | BRE | 2 | 28,284,035 |
| 323 | BRE | 2 | 28,285,741 | 324 | BRE | 2 | 28,357,445 |
| 325 | BRE | 2 | 28,396,438 | 326 | CRIM1 | 2 | 36,443,527 |
| 327 | CRIM1 | 2 | 36,467,658 | 328 | CRIM1 | 2 | 36,471,032 |
| 329 | CRIM1 | 2 | 36,485,964 | 330 | CRIM1 | 2 | 36,532,532 |
| 331 | CRIM | 2 | 36,535,123 | 332 | CRIM1 | 2 | 36,537,212 |
| 333 | CRIM | 2 | 36,611,604 | 334 | CRIM1 | 2 | 36,632,391 |
| 335 | FEZ2 | 2 | 36,634,284 | 336 | FEZ2 | 2 | 36,634,905 |
| 337 | FEZ2 | 2 | 36,645,798 | 338 | FEZ2 | 2 | 36,657,394 |
| 339 | FEZ2 | 2 | 36,663,000 | 340 | CDC42EP3 | 2 | 37,717,087 |
| 341 | CDC42EP3 | 2 | 37,725,643 | 342 | CDC42EP3 | 2 | 37,726,375 |
| 343 | CDC42EP3 | 2 | 37,741,421 | 344 | SLC8A1 | 2 | 40,217,148 |
| 345 | SLC8A1 | 2 | 40,230,588 | 346 | SLC8A1 | 2 | 40,253,816 |
| 347 | SLC8A1 | 2 | 40,267,644 | 348 | SLC8A1 | 2 | 40,270,423 |
| 349 | SLC8A1 | 2 | 40,279,466 | 350 | SLC8A1 | 2 | 40,414,134 |
| 351 | SLC8A1 | 2 | 40,430,629 | 352 | SLC8A1I | 2 | 40,461,774 |
| 353 | SLC8A1 | 2 | 40,492,801 | 354 | SLC8A1 | 2 | 40,494,296 |
| 355 | SLC8A1 | 2 | 40,524,198 | 356 | HAAO | 2 | 42,872,648 |
| 357 | HAAO | 2 | 42,918,059 | 358 | PLEKHH2 | 2 | 43,780,963 |
| 359 | PLEKHH2 | 2 | 43,783,487 | 360 | PLEKHH2 | 2 | 43,785,627 |
| 361 | PLEKHH2 | 2 | 43,839,444 | 362 | PLEKHH2 | 2 | 43,846,952 |
| 363 | C2ORF34 | 2 | 44,500,542 | 364 | C2ORF34 | 2 | 44,501,551 |
| 365 | C2ORF34 | 2 | 44,529,327 | 366 | C2ORF34 | 2 | 44,529,730 |
| 367 | C2ORF34 | 2 | 44,536,723 | 368 | C2ORF34 | 2 | 44,593,376 |
| 369 | C2ORF34 | 2 | 44,599,767 | 370 | C2ORF34 | 2 | 44,686,360 |
| 371 | C2ORF34 | 2 | 44,722,640 | 372 | C2ORF34 | 2 | 44,726,913 |
| 373 | C2ORF34 | 2 | 44,739,819 | 374 | C2ORF34 | 2 | 44,834,623 |
| 375 | C2ORF34 | 2 | 44,846,991 | 376 | PRKCE | 2 | 45,737,717 |
| 377 | PRKCE | 2 | 45,755,958 | 378 | PRKCE | 2 | 45,807,414 |
| 379 | PRKCE | 2 | 45,910,006 | 380 | PRKCE | 2 | 45,919,740 |
| 381 | PRKCE | 2 | 45,927,156 | 382 | PRKCE | 2 | 45,979,475 |
| 383 | PRKCE | 2 | 46,065,162 | 384 | PRKCE | 2 | 46,085,029 |
| 385 | PRKCE | 2 | 46,085,876 | 386 | PRKCE | 2 | 46,108,254 |
| 387 | PRKCE | 2 | 46,130,026 | 388 | PRKCE | 2 | 46,139,028 |
| 389 | PRKCE | 2 | 46,226,779 | 390 | PRKCE | 2 | 46,257,075 |
| 391 | EPAS1 | 2 | 46,392,298 | 392 | EPAS1 | 2 | 46,401,568 |
| 393 | EPAS1 | 2 | 46,435,886 | 394 | EPAS1 | 2 | 46,438,356 |
| 395 | EPAS1 | 2 | 46,443,888 | 396 | EPAS1 | 2 | 46,458,097 |
| 397 | EPAS1 | 2 | 46,459,163 | 398 | FBXO11 | 2 | 47,947,841 |
| 399 | FBXO11 | 2 | 47,952,212 | 400 | PSME4 | 2 | 53,909,692 |
| 401 | PSME4 | 2 | 53,957,822 | 402 | PSME4 | 2 | 53,968,471 |
| 403 | PSME4 | 2 | 53,970,304 | 404 | PSME4 | 2 | 53,978,295 |
| 405 | PSME4 | 2 | 53,988,933 | 406 | PSME4 | 2 | 54,018,352 |
| 407 | PSME4 | 2 | 54,055,952 | 408 | PSME4 | 2 | 54,064,285 |
| 409 | PSME4 | 2 | 54,136,333 | 410 | PSME4 | 2 | 54,137,945 |
| 411 | ACYP2 | 2 | 54,187,233 | 412 | ACYP2 | 2 | 54,189,467 |
| 413 | ACYP2 | 2 | 54,216,479 | 414 | ACYP2 | 2 | 54,245,303 |
| 415 | ACYP2 | 2 | 54,247,074 | 416 | ACYP2 | 2 | 54,312,954 |
| 417 | ACYP2 | 2 | 54,350,261 | 418 | CCDC85A | 2 | 56,266,849 |
| 419 | CCDC85A | 2 | 56,271,283 | 420 | CCDC85A | 2 | 56,312,732 |
| 421 | CCDC85A | 2 | 56,325,663 | 422 | CCDC85A | 2 | 56,325,994 |
| 423 | CCDC85A | 2 | 56,344,184 | 424 | CCDC85A | 2 | 56,356,533 |
| 425 | CCDC85A | 2 | 56,416,774 | 426 | CCDC85A | 2 | 56,424,062 |
| 427 | CCDC85A | 2 | 56,425,867 | 428 | CCDC85A | 2 | 56,493,023 |
| 429 | COMMD1 | 2 | 62,036,964 | 430 | COMMD1 | 2 | 62,193,184 |
| 431 | OTX1 | 2 | 63,151,654 | 432 | AAK1 | 2 | 69,546,510 |
| 433 | AAK1 | 2 | 69,569,349 | 434 | AAK1 | 2 | 69,639,692 |
| 435 | AAK1 | 2 | 69,650,512 | 436 | AAK1 | 2 | 69,673,078 |
| 437 | AAK1 | 2 | 69,708,598 | 438 | AAK1 | 2 | 69,716,927 |
| 439 | AAK1 | 2 | 69,740,261 | 440 | AAK1 | 2 | 69,768,980 |
| 441 | AAK1 | 2 | 69,834,530 | 442 | CTNNA2 | 2 | 79,613,746 |
| 443 | CTNNA2 | 2 | 79,635,253 | 444 | CTNNA2 | 2 | 79,644,485 |
| 445 | CTNNA2 | 2 | 79,648,869 | 446 | CTNNA2 | 2 | 79,827,051 |
| 447 | CTNNA2 | 2 | 79,934,690 | 448 | CTNNA2 | 2 | 79,950,787 |
| 449 | CTNNA2 | 2 | 79,998,204 | 450 | CTNNA2 | 2 | 79,998,431 |
| 451 | CTNNA2 | 2 | 80,091,949 | 452 | CTNNA2 | 2 | 80,246,403 |
| 453 | CTNNA2 | 2 | 80,252,218 | 454 | CTNNA2 | 2 | 80,302,393 |
| 455 | CTNNA2 | 2 | 80,303,744 | 456 | CTNNA2 | 2 | 80,303,795 |
| 457 | CTNNA2 | 2 | 80,305,615 | 458 | CTNNA2 | 2 | 80,305,672 |
| 459 | CTNNA2 | 2 | 80,323,975 | 460 | CTNNA2 | 2 | 80,331,785 |
| 461 | CTNNA2 | 2 | 80,341,439 | 462 | CTNNA2 | 2 | 90,341,671 |
| 463 | CTNNA2 | 2 | 80,342,285 | 464 | CTNNA2 | 2 | 80,346,411 |
| 465 | CTNNA2 | 2 | 80,348,223 | 466 | CTNNA2 | 2 | 80,368,183 |
| 467 | CTNNA2 | 2 | 80,377,795 | 468 | CTNNA2 | 2 | 80,498,999 |
| 469 | CTNNA2 | 2 | 80,598,484 | 470 | CTNNA2 | 2 | 80,657,667 |
| 471 | CTNNA2 | 2 | 80,670,274 | 472 | CTNNA2 | 2 | 80,682,874 |
| 473 | CTNNA2 | 2 | 80,703,322 | 474 | INPP4A | 2 | 98,449,370 |
| 475 | INPP4A | 2 | 98,538,676 | 476 | NAPS | 2 | 133,368,245 |
| 477 | NAPS | 2 | 133,513,639 | 478 | NAPS | 2 | 133,547,260 |
| 479 | NAPS | 2 | 133,558,451 | 480 | NAPS | 2 | 133,567,502 |
| 481 | NAPS | 2 | 133,613,763 | 482 | NAPS | 2 | 133,619,788 |
| 483 | NAPS | 2 | 133,620,453 | 484 | NAPS | 2 | 133,628,562 |
| 485 | NAP5 | 2 | 133,692,413 | 486 | NAPS | 2 | 133,970,443 |
| 487 | NAPS | 2 | 134,029,768 | 488 | RAB3GAP1 | 2 | 135,514,217 |
| 489 | RAB3GAP1 | 2 | 135,649,190 | 490 | ZRANB3 | 2 | 135,701,191 |
| 491 | ZRANB3 | 2 | 135,867,882 | 492 | ZRANB3 | 2 | 135,868,244 |
| 493 | ZRANB3 | 2 | 135,903,815 | 494 | ZRANB3 | 2 | 135,981,101 |
| 495 | ZRANB3 | 2 | 136,029,379 | 496 | UBXD2 | 2 | 136,223,397 |
| 497 | UBXD2 | 2 | 136,262,314 | 498 | NXPH2 | 2 | 139,205,858 |
| 499 | NXPH2 | 2 | 139,218,623 | 500 | NXPH2 | 2 | 139,301,576 |
| 501 | LRP1B | 2 | 140,708,533 | 502 | LRP1B | 2 | 140,710,220 |
| 503 | LRP1B | 2 | 140,711,565 | 504 | LRP1B | 2 | 140,740,113 |
| 505 | LRP1B | 2 | 140,749,675 | 506 | LRP1B | 2 | 140,836,079 |
| 507 | LRP1B | 2 | 140,917,629 | 508 | LRP1B | 2 | 141,253,166 |
| 509 | LRP1B | 2 | 141,446,369 | 510 | LRP1B | 2 | 141,470,624 |
| 511 | LRP1B | 2 | 141,472,804 | 512 | LRP1B | 2 | 141,702,891 |
| 513 | LRP1B | 2 | 141,715,030 | 514 | LRP1B | 2 | 141,896,198 |
| 515 | LRP1B | 2 | 142,007,651 | 516 | LRP1B | 2 | 142,065,965 |
| 517 | LRP1B | 2 | 142,066,600 | 518 | LRP1B | 2 | 142,371,205 |
| 519 | LRP1B | 2 | 142,371,483 | 520 | LRP1B | 2 | 142,395,682 |
| 521 | LRP1B | 2 | 142,529,079 | 522 | LRP1B | 2 | 142,573,134 |
| 523 | LRP1B | 2 | 142,591,970 | 524 | LRP1B | 2 | 142,598,638 |
| 525 | KYNU | 2 | 143,355,132 | 526 | KYNU | 2 | 143,368,826 |
| 527 | KYNU | 2 | 143,405,309 | 528 | KYNU | 2 | 143,430,832 |
| 529 | KYNU | 2 | 143,434,516 | 530 | KYNU | 2 | 143,439,309 |
| 531 | KYNU | 2 | 143,455,211 | 532 | KYNU | 2 | 143,465,263 |
| 533 | KYNU | 2 | 143,504,218 | 534 | KYNU | 2 | 143,513,164 |
| 535 | KYNU | 2 | 143,516,377 | 536 | KYNU | 2 | 143,598,044 |
| 537 | ARHGAP15 | 2 | 143,606,219 | 538 | ARHGAP15 | 2 | 143,685,981 |
| 539 | ARHGAP15 | 2 | 143,692,950 | 540 | ARHGAP15 | 2 | 143,698,840 |
| 541 | ARHGAP15 | 2 | 143,729,953 | 542 | ARHGAP15 | 2 | 143,729,996 |
| 543 | ARHGAP15 | 2 | 143,738,248 | 544 | ARHGAP15 | 2 | 143,739,424 |
| 545 | ARHGAP15 | 2 | 143,757,220 | 546 | ARHGAP15 | 2 | 143,769,771 |
| 547 | ARHGAP15 | 2 | 143,848,173 | 548 | ARHGAP15 | 2 | 143,849,979 |
| 549 | ARHGAP15 | 2 | 143,943,051 | 550 | ARHGAP15 | 2 | 143,999,263 |
| 551 | ARHGAP15 | 2 | 144,018,166 | 552 | ARHGAP15 | 2 | 144,025,496 |
| 553 | ARHGAP15 | 2 | 144,034,639 | 554 | ARHGAP15 | 2 | 144,040,124 |
| 555 | ARHGAP15 | 2 | 144,064,434 | 556 | ARHGAP15 | 2 | 144,068,893 |
| 557 | ARHGAP15 | 2 | 144,097,012 | 558 | ARHGAP15 | 2 | 144,273,491 |
| 559 | ARHGAP15 | 2 | 144,283,468 | 560 | ARHGAP15 | 2 | 144,294,622 |
| 561 | ARHGAP15 | 2 | 144,295,364 | 562 | KIF5C | 2 | 149,333,649 |
| 563 | KIF5C | 2 | 149,521,137 | 564 | KIF5C | 2 | 149,539,190 |
| 565 | CACNB4 | 2 | 152,527,508 | 566 | CACNB4 | 2 | 152,578,170 |
| 567 | CACNB4 | 2 | 152,587,594 | 568 | CACNB4 | 2 | 152,607,303 |
| 569 | CACNB4 | 2 | 152,632,727 | 570 | CACNB4 | 2 | 152,638,370 |
| 571 | CACNB4 | 2 | 152,655,949 | 572 | CACNB4 | 2 | 152,659,379 |
| 573 | CACNB4 | 2 | 152,684,248 | 574 | FMNL2 | 2 | 152,981,465 |
| 575 | FMNL2 | 2 | 152,996,148 | 576 | FMNL2 | 2 | 153,007,734 |
| 577 | FMNL2 | 2 | 153,075,073 | 578 | FMNL2 | 2 | 153,096,733 |
| 579 | FMNL2 | 2 | 153,097,194 | 580 | FMNL2 | 2 | 153,102,193 |
| 581 | FMNL2 | 2 | 153,146,684 | 582 | FMNL2 | 2 | 153,146,906 |
| 583 | FMNL2 | 2 | 153,147,303 | 584 | FMNL2 | 2 | 153,147,664 |
| 585 | FMNL2 | 2 | 153,159,182 | 586 | FMNL2 | 2 | 153,166,996 |
| 587 | FMNL2 | 2 | 153,201,156 | 588 | FMNL2 | 2 | 153,201,532 |
| 589 | FMNL2 | 2 | 153,207,856 | 590 | KCNJ3 | 2 | 155,289,884 |
| 591 | KCNJ3 | 2 | 155,329,292 | 592 | KCNJ3 | 2 | 155,397,929 |
| 593 | KCNJ3 | 2 | 155,403,085 | 594 | PKP4 | 2 | 159,035,950 |
| 595 | PKP4 | 2 | 159,058,381 | 596 | PKP4 | 2 | 159,099,329 |
| 597 | PKP4 | 2 | 159,132,118 | 598 | PKP4 | 2 | 159,175,274 |
| 599 | PKP4 | 2 | 159,214,299 | 600 | PKP4 | 2 | 159,219,409 |
| 601 | PKP4 | 2 | 159,240,155 | 602 | PLA2R1 | 2 | 160,516,321 |
| 603 | PLA2R1 | 2 | 160,516,560 | 604 | PLA2R1 | 2 | 160,519,430 |
| 605 | PLA2R1 | 2 | 160,543,237 | 606 | PLA2R1 | 2 | 160,543,645 |
| 607 | PLA2R1 | 2 | 160,550,074 | 608 | PLA2R1 | 2 | 160,575,305 |
| .609 | PLA2R1 | 2 | 160,575,799 | 610 | PLA2R1 | 2 | 160,584,453 |
| 611 | PLA2R1 | 2 | 160,599,255 | 612 | PLA2R1 | 2 | 160,612,267 |
| 613 | PLA2R1 | 2 | 160,616,115 | 614 | PLA2R1 | 2 | 160,623,352 |
| 615 | SCN3A | 2 | 165,671,518 | 616 | SCN3A | 2 | 165,685,811 |
| 617 | SCN3A | 2 | 165,708,114 | 618 | SCN3A | 2 | 165,732,374 |
| 619 | SCN1A | 2 | 166,596,685 | 620 | SCN1A | 2 | 166,606,110 |
| 621 | SCN1A | 2 | 166,617,790 | 622 | SCN1A | 2 | 166,632,718 |
| 623 | SCN9A | 2 | 166,764,674 | 624 | SCN9A | 2 | 166,781,097 |
| 625 | SCN9A | 2 | 166,807,404 | 626 | SCN9A | 2 | 166,817,960 |
| 627 | SCN9A | 2 | 166,857,449 | 628 | SCN9A | 2 | 166,871,909 |
| 629 | SCN9A | 2 | 166,874,107 | 630 | SCN7A | 2 | 166,970,599 |
| 631 | CERKL | 2 | 182,130,703 | 632 | CERKL | 2 | 182,145,339 |
| 633 | CERKL | 2 | 182,164,093 | 634 | CERKL | 2 | 182,228,354 |
| 635 | CERKL | 2 | 182,239,099 | 636 | PDE1A | 2 | 182,767,445 |
| 637 | PDE1A | 2 | 182,821,396 | 638 | PDE1A | 2 | 182,826,618 |
| 639 | PDE1A | 2 | 182,840,603 | 640 | PDE1A | 2 | 182,886,451 |
| 641 | PDE1A | 2 | 182,918,516 | 642 | PDE1A | 2 | 182,932,372 |
| 643 | PDE1A | 2 | 182,971,998 | 644 | PDE1A | 2 | 183,013,022 |
| 645 | PDE1A | 2 | 183,013,344 | 646 | PDE1A | 2 | 183,050,970 |
| 647 | PDE1A | 2 | 183,061,827 | 648 | PDE1A | 2 | 183,067,065 |
| 649 | PDE1A | 2 | 183,086,558 | 650 | PDE1A | 2 | 183,088,998 |
| 651 | ALS2 | 2 | 202,278,237 | 652 | ALS2 | 2 | 202,288,513 |
| 653 | ALS2 | 2 | 202,305,680 | 654 | ALS2 | 2 | 202,341,183 |
| 655 | PARD3B | 2 | 205,237,235 | 656 | PARD3B | 2 | 205,244,163 |
| 657 | PARD3B | 2 | 205,347,353 | 658 | PARD3B | 2 | 205,620,648 |
| 659 | PARD3B | 2 | 205,625,989 | 660 | PARD3B | 2 | 205,639,010 |
| 661 | PARD3B | 2 | 205,650,805 | 662 | PARD3B | 2 | 205,659,848 |
| 663 | PARD3B | 2 | 205,705,702 | 664 | PARD3B | 2 | 205,725,077 |
| 665 | PARD3B | 2 | 205,907,031 | 666 | PARD3B | 2 | 205,967,382 |
| 667 | PARD3B | 2 | 206,172,710 | 668 | NRP2 | 2 | 206,294,712 |
| 669 | NRP2 | 2 | 206,296,221 | 670 | NRP2 | 2 | 206,300,940 |
| 671 | NRP2 | 2 | 206,301,486 | 672 | NRP2 | 2 | 206,318,296 |
| 673 | NRP2 | 2 | 206,441,948 | 674 | NRP2 | 2 | 206,442,661 |
| 675 | NRP2 | 2 | 206,443,765 | 676 | PIP5K3 | 2 | 208,955,629 |
| 677 | PIP5K3 | 2 | 208,956,492 | 678 | ERBB4 | 2 | 211,975,964 |
| .679 | ERBB4 | 2 | 211,983,182 | 680 | ERBB4 | 2 | 212,022,377 |
| 681 | ERBB4 | 2 | . 213,497,122 | 682 | ERBB4 | 2 | 213,505,758 |
| 683 | ERBB4 | 2 | 213,507,896 | 684 | ERBB4 | 2 | 213,508,611 |
| 685 | CUL3 | 2 | 225,109,321 | 686 | CUL3 | 2 | 225,148,088 |
| 687 | CUL3 | 2 | 225,149,052 | 688 | IRS1 | 2 | 227,304,756 |
| 689 | IRS1 | 2 | 227,307,777 | 690 | IRS1 | 2 | 227,322,249 |
| 691 | IRS1 | 2 | 227,362,192 | 692 | COL4A4 | 2 | 227,583,201 |
| 693 | COL4A4 | 2 | 227,591,892 | 694 | COL4A4 | 2 | 227,606,678 |
| 695 | COL4A4 | 2 | 227,608,663 | 696 | COL4A4 | 2 | 227,622,869 |
| 697 | COL4A4 | 2 | 227,630,259 | 698 | COL4A3 | 2 | 227,768,751 |
| 699 | COL4A3 | 2 | 227,795,487 | 700 | COL4A3 | 2 | 227,795,722 |
| 701 | COL4A3 | 2 | 227,800,650 | 702 | COL4A3 | 2 | 227,801,143 |
| 703 | COL4A3 | 2 | 227,818,028 | 704 | COL4A3 | 2 | 227,848,813 |
| 705 | COL4A3 | 2 | 227,864,848 | 706 | COL4A3 | 2 | 227,875,258 |
| 707 | COL4A3 | 2 | 227,885,145 | 708 | COL4A3 | 2 | 227,885,723 |
| 709 | COL4A3 | 2 | 227,886,069 | 710 | COL4A3 | 2 | 227,889,666 |
| 711 | DNER | 2 | 229,942,770 | 712 | DNER | 2 | 229,964,830 |
| 713 | DNER | 2 | 229,972,935 | 714 | DNER | 2 | 230,110,447 |
| 715 | DNER | 2 | 230,162,171 | 716 | DNER | 2 | 230,170,094 |
| 717 | DNER | 2 | 230,176,862 | 718 | DNER | 2 | 230,262,864 |
| 719 | DNER | 2 | 230,270,658 | 720 | DNER | 2 | 230,270,700 |
| 721 | DNER | 2 | 230,271,362 | 722 | DNER | 2 | 230,271,569 |
| 723 | TRIP12 | 2 | 230,296,640 | 724 | TRIP12 | 2 | 230,317,393 |
| 725 | TRIP12 | 2 | 230,340,512 | 726 | TRIP12 | 2 | 230,422,269 |
| 727 | TRIP12 | 2 | 230,442,325 | 728 | TRIP12 | 2 | 230,447,453 |
| 729 | ECEL1 | 2 | 233,048,959 | 730 | CHRND | 2 | 233,091,660 |
| 731 | SAG | 2 | 233,903,639 | 732 | SAG | 2 | 233,909,134 |
| 733 | CNTN6 | 3 | 1,222,723 | 734 | CNTN6 | 3 | 1,231,380 |
| 735 | CNTN6 | 3 | 1,247,244 | 736 | CNTN6 | 3 | 1,251,092 |
| 737 | CNTN6 | 3 | 1,268,504 | 738 | CNTN6 | 3 | 1,292,727 |
| 739 | CNTN6 | 3 | 1,295,271 | 740 | CNTN6 | 3 | 1,311,397 |
| 741 | CNTN6 | 3 | 1,313,259 | 742 | CNTN6 | 3 | 1,336,323 |
| 743 | CNTN6 | 3 | 1,341,846 | 744 | CNTN6 | 3 | 1,350,581 |
| 745 | CNTN6 | 3 | 1,357,151 | 746 | CNTN6 | 3 | 1,357,358 |
| 747 | CNTN6 | 3 | 1,372,414 | 748 | CNTN6 | 3 | 1,375,419 |
| 749 | CNTN6 | 3 | 1,378,076 | 750 | CNTN6 | 3 | 1,396,673 |
| 751 | CNTN6 | 3 | 1,399,850 | 752 | CNTN6 | 3 | 1,400,997 |
| 753 | CNTN6 | 3 | 1,403,635 | 754 | CNTN6 | 3 | 1,414,974 |
| 755 | CNTN4 | 3 | 2,072,897 | 756 | CNTN4 | 3 | 2,267,019 |
| 757 | CNTN4 | 3 | 2,285,875 | 758 | CNTN4 | 3 | 2,287,398 |
| 759 | CNTN4 | 3 | 2,287,451 | 760 | CNTN4 | 3 | 2,298,242 |
| 761 | CNTN4 | 3 | 2,321,860 | 762 | CNTN4 | 3 | 2,326,831 |
| 763 | CNTN4 | 3 | 2,336,802 | 764 | CNTN4 | 3 | 2,375,967 |
| 765 | CNTN4 | 3 | 2,384,532 | 766 | CNTN4 | 3 | 2,386,061 |
| 767 | CNTN4 | 3 | 2,734,283 | 768 | CNTN4 | 3 | 2,736,667 |
| 769 | CNTN4 | 3 | 2,758,344 | 770 | CNTN4 | 3 | 3,046,742 |
| 771 | CNTN4 | 3 | 3,047,912 | 772 | CNTN4 | 3 | 3,048,509 |
| 773 | CNTN4 | 3 | 3,067,546 | 774 | ITPR1 | 3 | 4,511,386 |
| 775 | ITPR1 | 3 | 4,527,175 | 776 | ITPRI | 3 | 4,688,162 |
| 777 | ITPR1 | 3 | 4,720,230 | 778 | ITPR1 | 3 | 4,725,489 |
| 779 | ITPR1 | 3 | 4,725,558 | 780 | ITPR1 | 3 | 4,725,674 |
| 781 | ITPR1 | 3 | 4,729,125 | 782 | ITPRI | 3 | 4,841,416 |
| 783 | ITPRI | 3 | 4,846,598 | 784 | IRAK2 | 3 | 10,234,114 |
| 785 | IRAK2 | 3 | 10,251,163 | 786 | IRAK2 | 3 | 10,252,129 |
| 787 | ATP2B2 | 3 | 10,358,996 | 788 | ATP2B2 | 3 | 10,412,280 |
| 789 | ATP2B2 | 3 | 10,416,147 | 790 | ATP2B2 | 3 | 10,418,090 |
| 791 | ATP2B2 | 3 | 10,432,572 | 792 | ATP2B2 | 3 | 10,432,857 |
| 793 | ATP2B2 | 3 | 10,439,753 | 794 | SLC6A6 | 3 | 14,421,894 |
| 795 | SLC6A6 | 3 | 14,422,977 | 796 | SLC6A6 | 3 | 14,464,931 |
| 797 | SLC6A6 | 3 | 14,495,168 | 798 | SLC6A6 | 3 | 14,503,422 |
| 799 | DAZL | 3 | 16,599,020 | 800 | DAZL | 3 | 16,602,617 |
| 801 | DAZL | 3 | 16,611,406 | 802 | DAZL | 3 | 16,611,469 |
| 803 | DAZL | 3 | 16,627,901 | 804 | RARB | 3 | 25,506,269 |
| 805 | RARB | 3 | 25,544,885 | 806 | RARB | 3 | 25,589,536 |
| 807 | RARB | 3 | 25,592,407 | 808 | RARB | 3 | 25,593,163 |
| 809 | RARB | 3 | 25,620,267 | 810 | RARB | 3 | 25,620,425 |
| 811 | FBXL2 | 3 | 33,317,991 | 812 | FBXL2 | 3 | 33,400,034 |
| 813 | CLASP2 | 3 | 33,516,308 | 814 | CLASP2 | 3 | 33,516,362 |
| 815 | CLASP2 | 3 | 33,592,794 | 816 | CLASP2 | 3 | 33,752,897 |
| 817 | CLASP2 | 3 | 33,761,745 | 818 | CLASP2 | 3 | 33,780,565 |
| 819 | ARPP-21 | 3 | 35,697,969 | 820 | ARPP-21 | 3 | 35,742,792 |
| 821 | ARPP-21 | 3 | 35,750,253 | 822 | ARPP-21 | 3 | 35,760,612 |
| 823 | STAC | 3 | 36,432,376 | 824 | STAC | 3 | 36,433,107 |
| 825 | STAC | 3 | 36,445,311 | 826 | STAC | 3 | 36,459,827 |
| 827 | ULK4 | 3 | 41,283,352 | 828 | ULK4 | 3 | 41,329,536 |
| 829 | ULK4 | 3 | 41,344,053 | 830 | ULK4 | 3 | 41,348,228 |
| 831 | ULK4 | 3 | 41,350,671 | 832 | ULK4 | 3 | 41,363,412 |
| 833 | ULK4 | 3 | 41,365,143 | 834 | ULK4 | 3 | 41,369,174 |
| 835 | ULK4 | 3 | 41,395,796 | 836 | ULK4 | 3 | 41,421,630 |
| 837 | ULK4 | 3 | 41.438,204 | 838 | ULK4 | 3 | 41,455,051 |
| 839 | ULK4 | 3 | 41,468,595 | 840 | ULK4 | 3 | 41,469,435 |
| 841 | ULK4 | 3 | 41,469,609 | 842 | ULK4 | 3 | 41,476,741 |
| 843 | ULK4 | 3 | 41,482,456 | 844 | ULK4 | 3 | 41,507,368 |
| 845 | ULK4 | 3 | 41,507,554 | 846 | ULK4 | 3 | 41,508,106 |
| 847 | ULK4 | 3 | 41,584,470 | 848 | ULK4 | 3 | 41,736,832 |
| 849 | ULK4 | 3 | 41,785,368 | 850 | ULK4 | 3 | 41,833,735 |
| 851 | ULK4 | 3 | 41,852,418 | 852 | ULK4 | 3 | 41,900,305 |
| 853 | ULK4 | 3 | 41,971,140 | 854 | ZNF445 | 3 | 44,542,069 |
| 855 | GPX1 | 3 | 49,429,116 | 856 | GPX1 | 3 | 49,545,204 |
| 857 | GPX1 | 3 | 49,624,993 | 858 | GPX1 | 3 | 49,853,117 |
| 859 | GPX1 | 3 | 49,865,617 | 860 | SEMA3F | 3 | 50,180,966 |
| 861 | CACNA2D2 | 3 | 50,406,198 | 862 | CACNA2D2 | 3 | 50,451,382 |
| 863 | CACNA2D2 | 3 | 50,523,899 | 864 | CACNA2D3 | 3 | 54,127,859 |
| 865 | CACNA2D3 | 3 | 54,216,922 | 866 | CACNA2D3 | 3 | 54,217,081 |
| 867 | CACNA2D3 | 3 | 54,331,793 | 868 | CACNA2D3 | 3 | 54,494,308 |
| 869 | CACNA2D3 | 3 | 54,540,948 | 870 | CACNA2D3 | 3 | 54,774,489 |
| 871 | CACNA2D3 | 3 | 54,918,069 | 872 | CACNA2D3 | 3 | 54,953,081 |
| 873 | CACNA2D3 | 3 | 54,955,060 | 874 | CACNA2D3 | 3 | 54,996,262 |
| 875 | CACNA2D3 | 3 | 55,019,916 | 876 | CACNA2D3 | 3 | 55,094,024 |
| 877 | ERC2 | 3 | 55,519,913 | 878 | ERC2 | 3 | 55,743,028 |
| 879 | ERC2 | 3 | 55,757,809 | 880 | ERC2 | 3 | 55,760,546 |
| 881 | ERC2 | 3 | 55,858,272 | 882 | ERC2 | 3 | 55,877,492 |
| 883 | ERC2 | 3 | 55,902,605 | 884 | ERC2 | 3 | 55,954,550 |
| 885 | ERC2 | 3 | 55,985,742 | 886 | ERC2 | 3 | 56,002,843 |
| 887 | ERC2 | 3 | 56,119,301 | 888 | ERC2 | 3 | 56,164,173 |
| 889 | ERC2 | 3 | 56,175,003 | 890 | ERC2 | 3 | 56,245,378 |
| 891 | ERC2 | 3 | 56,304,151 | 892 | ERC2 | 3 | 56,337,250 |
| 893 | ERC2 | 3 | 56,347,766 | 894 | ERC2 | 3 | 56,368,109 |
| 895 | ERC2 | 3 | 56,393,831 | 896 | ERC2 | 3 | 56,422,909 |
| 897 | ERC2 | 3 | 56,462,620 | 898 | ERC2 | 3 | 56,464,816 |
| 899 | ERC2 | 3 | 56,491,685 | 900 | ERC2 | 3 | 56,496,204 |
| 901 | ERC2 | 3 | 56,496,259 | 902 | ERC2 | 3 | 56,510,277 |
| 903 | IL17RD | 3 | 57,115,148 | 904 | IL17RD | 3 | 57,128,722 |
| 905 | IL17RD | 3 | 57,128,909 | 906 | IL17RD | 3 | 57,143,381 |
| 907 | FLNB | 3 | 58,057,749 | 908 | FLNB | 3 | 58,069,197 |
| 909 | FLNB | 3 | 58,075,809 | 910 | FLNB | 3 | 58,083,555 |
| 911 | FLNB | 3 | 58,093,595 | 912 | FLNB | 3 | 58,139,576 |
| 913 | FLNB | 3 | 58,143,446 | 914 | FHIT | 3 | 59,824,587 |
| 915 | FHIT | 3 | 59,949,555 | 916 | FHIT | 3 | 59,956,565 |
| 917 | FHIT | 3 | 59,966,929 | 918 | FHIT | 3 | 60,339,716 |
| 919 | FHIT | 3 | 60,367,407 | 920 | FHIT | 3 | 60,478,148 |
| 921 | FHIT | 3 | 61,027,539 | 922 | FHIT | 3 | 61,027,560 |
| 923 | FHIT | 3 | 61,027,682 | 924 | FHIT | 3 | 61,135,266 |
| 925 | FHIT | 3 | 61,137,444 | 926 | FHIT | 3 | 61,159,361 |
| 927 | FHIT | 3 | 61,213,258 | 928 | FHIT | 3 | 61,214,250 |
| 929 | FHIT | 3 | 61,228,928 | 930 | FHIT | 3 | 61,234,576 |
| 931 | PTPRG | 3 | 61,484,177 | 932 | PTPRG | 3 | 61,488,535 |
| 933 | PTPRG | 3 | 61,493,156 | 934 | PTPRG | 3 | 61,723,402 |
| 935 | PTPRG | 3 | 62,142,228 | 936 | PTPRG | 3 | 62,196,081 |
| 937 | PTPRG | 3 | 62,219,484 | 938 | PTPRG | 3 | 62,219,610 |
| 939 | PTPRG | 3 | 62,221,462 | 940 | PTPRG | 3 | 62,245,596 |
| 941 | PTPRG | 3 | 62,256,476 | 942 | CADPS | 3 | 62,473,418 |
| 943 | CADPS | 3 | 62,487,886 | 944 | CADPS | 3 | 62,498,262 |
| 945 | CADPS | 3 | 62,548,478 | 946 | CADPS | 3 | 62,720,200 |
| 947 | CADPS | 3 | 62,748,989 | 948 | CADPS | 3 | 62,789,812 |
| 949 | CADPS | 3 | 62,801,974 | 950 | CADPS | 3 | 62,836,232 |
| 951 | CADPS | 3 | 62,846,542 | 952 | CADPS | 3 | 62,855,968 |
| 953 | SYNPR | 3 | 63,451,187 | 954 | SYNPR | 3 | 63,493,882 |
| 955 | SYNPR | 3 | 63,500,043 | 956 | SYNPR | 3 | 63,531,349 |
| 957 | PRICKLE2 | 3 | 64,058,457 | 958 | PRICKLE2 | 3 | 64,091,416 |
| 959 | PRICKLE2 | 3 | 64,123,328 | 960 | PRICKLE2 | 3 | 64,136,626 |
| 961 | PRICKLE2 | 3 | 64,140,665 | 962 | PRICKLE2 | 3 | 64,153,423 |
| 963 | PRICKLE2 | 3 | 64,153,968 | 964 | PRICKLE2 | 3 | 64,159,437 |
| 965 | PRICKLE2 | 3 | 64,160,166 | 966 | PRICKLE2 | 3 | 64,176,516 |
| 967 | PRICKLE2 | 3 | 64,178,866 | 968 | PRICKLE2 | 3 | 64,181,567 |
| 969 | PRICKLE2 | 3 | 64,184,717 | 970 | PRICKLE2 | 3 | 64,186,645 |
| 971 | MAGI1 | 3 | 65,373,938 | 972 | MAGI1 | 3 | 65,407,523 |
| 973 | MAGI1 | 3 | 65,496,864 | 974 | MAGI1 | 3 | 65,505,289 |
| 975 | MAGI1 | 3 | 65,576,735 | 976 | MAGI1 | 3 | 65,839,896 |
| 977 | MAGI1 | 3 | 65,968,144 | 978 | MAGI1 | 3 | 65,975,330 |
| 979 | FAM19A1 | 3 | 68,196,007 | 980 | FAM19A1 | 3 | 68,232,575 |
| 981 | FAM19A1 | 3 | 68,409,920 | 982 | FAM19A1 | 3 | 68,614,345 |
| 983 | FAM19A1 | 3 | 68,673,127 | 984 | FOXP1 | 3 | 70,972,978 |
| 985 | FOXP1 | 3 | 71,021,299 | 986 | FOXP1 | 3 | 71,295,488 |
| 987 | FOXP1 | 3 | 71,476,030 | 988 | GBE1 | 3 | 81,687,469 |
| 989 | GBE1 | 3 | 81,694,225 | 990 | GBE1 | 3 | 81,702,278 |
| 991 | GBE1 | 3 | 81,727,601 | 992 | GBE1 | 3 | 81,816,787 |
| 993 | GBE1 | 3 | 81,841,418 | 994 | GBE1 | 3 | 81,848,716 |
| 995 | GBE1 | 3 | 81,859,450 | 996 | GBE1 | 3 | 81,885,108 |
| 997 | GBE1 | 3 | 81,893,914 | 998 | GBE1 | 3 | 81,894,474 |
| 999 | HTR1F | 3 | 88,105,976 | 1000 | STX19 | 3 | 95,301,818 |
| 1001 | EPHA6 | 3 | 97,947,064 | 1002 | EPHA6 | 3 | 97,998,729 |
| 1003 | EPHA6 | 3 | 98,070,623 | 1004 | EPHA6 | 3 | 98,108,626 |
| 1005 | EPHA6 | 3 | 98,164,249 | 1006 | EPHA6 | 3 | 98,267,154 |
| 1007 | EPHA6 | 3 | 98,686,182 | 1008 | EPHA6 | 3 | 98,787,645 |
| 1009 | EPHA6 | 3 | 98,810,974 | 1010 | PLCXD2 | 3 | 112,883,336 |
| 1011 | PLCXD2 | 3 | 112,941,527 | 1012 | PLCXD2 | 3 | 112,949,845 |
| 1013 | PLCXD2 | 3 | 112,953,907 | 1014 | PLCXD2 | 3 | 112,960,464 |
| 1015 | PLCXD2 | 3 | 112,964,847 | 1016 | PLCXD2 | 3 | 113,021,589 |
| 1017 | PLCXD2 | 3 | 113,072,668 | 1018 | CDGAP | 3 | 120,517,200 |
| 1019 | CDGAP | 3 | 120,525,701 | 1020 | CDGAP | 3 | 120,527,813 |
| 1021 | CDGAP | 3 | 120,551,002 | 1022 | CDGAP | 3 | 120,606,504 |
| 1023 | CDGAP | 3 | 120,612,831 | 1024 | CDGAP | 3 | 120,615,873 |
| 1025 | CDGAP | 3 | 120,621,365 | 1026 | STXBP5L | 3 | 122,127,643 |
| 1027 | STXBP5L | 3 | 122,140,467 | 1028 | STXBP5L | 3 | 122,173,450 |
| 1029 | STXBP5L | 3 | 122,346,648 | 1030 | STXBP5L | 3 | 122,406,894 |
| 1031 | STXBP5L | 3 | 122,476,483 | 1032 | STXBP5L | 3 | 122,522,839 |
| 1033 | STXBP5L | 3 | 122,608,097 | 1034 | STXBP5L | 3 | 122,623,726 |
| 1035 | STXBP5L | 3 | 122,626,569 | 1036 | STXBP5L | 3 | 122,628,751 |
| 1037 | KALRN | 3 | 125,461,439 | 1038 | KALRN | 3 | 125,461,555 |
| 1039 | KALRN | 3 | 125,462,341 | 1040 | KALRN | 3 | 125,586,787 |
| 1041 | KALRN | 3 | 125,602,730 | 1042 | KALRN | 3 | 125,610,651 |
| 1043 | KALRN | 3 | 125,632,871 | 1044 | KALRN | 3 | 125,876,300 |
| 1045 | KALRN | 3 | 125,928,454 | 1046 | KALRN | 3 | 125,928,594 |
| 1047 | ZXDC | 3 | 127,665,610 | 1048 | CPNE4 | 3 | 132,725,192 |
| 1049 | CPNE4 | 3 | 132,741,385 | 1050 | CPNE4 | 3 | 132,749,292 |
| 1051 | CPNE4 | 3 | 132,772,981 | 1052 | CPNE4 | 3 | 132,802,779 |
| 1053 | CPNE4 | 3 | 132,865,495 | 1054 | CPNE4 | 3 | 132,885,425 |
| 1055 | CPNE4 | 3 | 132,886,287 | 1056 | CPNE4 | 3 | 132,907,690 |
| 1057 | CPNE4 | 3 | 132,918,068 | 1058 | CPNE4 | 3 | 133,145,979 |
| 1059 | CPNE4 | 3 | 133,235,987 | 1060 | CPNE4 | 3 | 133,241,957 |
| 1061 | RAB6B | 3 | 135,041,388 | 1062 | RAB6B | 3 | 135,064,149 |
| 1063 | EPHB1 | 3 | 136,138,136 | 1064 | EPHB1 | 3 | 136,151,931 |
| 1065 | EPHB1 | 3 | 136,154,975 | 1066 | EPHB1 | 3 | 136,156,084 |
| 1067 | EPNB1 | 3 | 136,165,323 | 1068 | EPHB1 | 3 | 136,172,644 |
| 1069 | EPHB1 | 3 | 136,306,915 | 1070 | EPHB1 | 3 | 136,390,869 |
| 1071 | EPHB1 | 3 | 136,505,913 | 1072 | EPHB1 | 3 | 136,509,166 |
| 1073 | CLSTN2 | 3 | 141,659,332 | 1074 | CLSTN2 | 3 | 141,661,175 |
| 1075 | CLSTN2 | 3 | 141,662,920 | 1076 | CLSTN2 | 3 | 141,663,200 |
| 1077 | CLSTN2 | 3 | 141,764,774 | 1078 | CLSTN2 | 3 | 141,770,820 |
| 1079 | CLSTN2 | 3 | 141,773,663 | 1080 | CLSTN2 | 3 | 141,774,103 |
| 1081 | CLSTN2 | 3 | 141,775,226 | 1082 | SPSB4 | 3 | 142,241,400 |
| 1083 | SPSB4 | 3 | 142,258,988 | 1084 | SPSB4 | 3 | 142,286,988 |
| 1085 | SPSB4 | 3 | 142,287,637 | 1086 | SPSB4 | 3 | 142,328,274 |
| 1087 | SERPINI2 | 3 | 168,658,115 | 1088 | SERPINI2 | 3 | 168,667,572 |
| 1089 | SERPINI2 | 3 | 168,672,301 | 1090 | SERPINI2 | 3 | 168,681,607 |
| 1091 | SERPINI1 | 3 | 168,954,239 | 1092 | SERPINI1 | 3 | 168,985,871 |
| 1093 | SLC7A14 | 3 | 171,682,206 | 1094 | SLC7A14 | 3 | 171,736,501 |
| 1095 | SLC7A14 | 3 | 171,743,763 | 1096 | SLC7A14 | 3 | 171,815,650 |
| 1097 | TNIK | 3 | 172,281,618 | 1098 | TNIK | 3 | 172,294,982 |
| 1099 | TNIK | 3 | 172,313,766 | 1100 | TNIK | 3 | 172,381,296 |
| 1101 | TNIK | 3 | 172,402,113 | 1102 | TNIK | 3 | 172,428,696 |
| 1103 | TNIK | 3 | 172,715,462 | 1104 | PLD1 | 3 | 172,791,538 |
| 1105 | PLD1 | 3 | 172,935,130 | 1106 | PLD1 | 3 | 172,970,915 |
| 1107 | PLD1 | 3 | 172,984,008 | 1108 | PLD1 | 3 | 172,990,075 |
| 1109 | PLD1 | 3 | 172,991,671 | 1110 | NLGN1 | 3 | 174,810,790 |
| 1111 | NLGN1 | 3 | 174,819,343 | 1112 | NLGN1 | 3 | 174,822,098 |
| 1113 | NLGN1 | 3 | 174,845,437 | 1114 | NLGN1 | 3 | 174,861,258 |
| 1115 | NLGN1 | 3 | 174,879,091 | 1116 | NLGN1 | 3 | 174,967,790 |
| 1117 | NLGN1 | 3 | 174,995,392 | 1118 | NLGN1 | 3 | 175,026,145 |
| 1119 | NLGN1 | 3 | 175,384,416 | 1120 | NLGN1 | 3 | 175,458,980 |
| 1121 | NLGN1 | 3 | 175,478,622 | 1122 | NLGN1 | 3 | 175,493,649 |
| 1123 | NLGN1 | 3 | 175,497,804 | 1124 | NLGN1 | 3 | 175,515,979 |
| 1125 | NLGN1 | 3 | 175,516,080 | 1126 | NLGN1 | 3 | 175,541,931 |
| 1127 | NLGN1 | 3 | 175,543,000 | 1128 | HTR3D | 3 | 185,232,425 |
| 1129 | HTR3D | 3 | 185,236,739 | 1130 | HTR3D | 3 | 185,238,516 |
| 1131 | HTR3C | 3 | 185,276,148 | 1132 | LEPREL1 | 3 | 191,189,805 |
| 1133 | LEPREL1 | 3 | 191,218,155 | 1134 | LEPREL1 | 3 | 191,237,107 |
| 1135 | LEPREL1 | 3 | 191,244,878 | 1136 | LEPREL1 | 3 | 191,331,953 |
| 1137 | LEPREL1 | 3 | 191,356,159 | 1138 | LEPREL1 | 3 | 191,356,831 |
| 1139 | LEPREL1 | 3 | 191,356,896 | 1140 | LEPREL1 | 3 | 191,358,612 |
| 1141 | IL1RAP | 3 | 191,716,532 | 1142 | IL1RAP | 3 | 191,719,452 |
| 1143 | IL1RAP | 3 | 191,825,136 | 1144 | IL1RAP | 3 | 191,859,482 |
| 1145 | LRRC15 | 3 | 195,561,991 | 1146 | LRRC15 | 3 | 195,562,211 |
| 1147 | LRRC15 | 3 | 195,566,153 | 1148 | LRRC15 | 3 | 195,567,372 |
| 1149 | LRRC15 | 3 | 195,569,243 | 1150 | LRRC15 | 3 | 195,586,726 |
| 1151 | UBXD7 | 3 | 197,576,308 | 1152 | UBXD7 | 3 | 197,632,452 |
| 1153 | UBXD7 | 3 | 197,636,526 | 1154 | UBXD7 | 3 | 197,642,729 |
| 1155 | SLC2A9 | 4 | 9,423,554 | 1156 | SLC2A9 | 4 | 9,476,600 |
| 1157 | SLC2A9 | 4 | 9,480,639 | 1158 | SLC2A9 | 4 | 9,500,096 |
| 1159 | SLC2A9 | 4 | 9,531,228 | 1160 | SLC2A9 | 4 | 9,606,210 |
| 1161 | SLC2A9 | 4 | 9,606,401 | 1162 | SLC2A9 | 4 | 9,606,822 |
| 1163 | SLC2A9 | 4 | 9,606,899 | 1164 | SLC2A9 | 4 | 9,613,930 |
| 1165 | SLC2A9 | 4 | 9,616,184 | 1166 | SLC2A9 | 4 | 9,616,373 |
| 1167 | SLC2A9 | 4 | 9,633,401 | 1168 | SLC2A9 | 4 | 9,636,640 |
| 1169 | LDB2 | 4 | 16,106,226 | 1170 | LDB2 | 4 | 16,119,229 |
| 1171 | LDB2 | 4 | 16,128,817 | 1172 | LDB2 | 4 | 16,197,827 |
| 1173 | LDB2 | 4 | 16,200,696 | 1174 | LDB2 | 4 | 16,362,961 |
| 1175 | LDB2 | 4 | 16,379,333 | 1176 | LDB2 | 4 | 16,386,273 |
| 1177 | LDB2 | 4 | 16,387,385 | 1178 | LDB2 | 4 | 16,390,278 |
| 1179 | LDB2 | 4 | 16,410,139 | 1180 | LDB2 | 4 | 16,417,600 |
| 1181 | LDB2 | 4 | 16,498,396 | 1182 | LDB2 | 4 | 16,513,031 |
| 1183 | LDB2 | 4 | 16,515,486 | 1184 | LDB2 | 4 | 16,525,999 |
| 1185 | SLIT2 | 4 | 19,864,404 | 1186 | SLIT2 | 4 | 19,872,156 |
| 1187 | SLIT2 | 4 | 20,162,402 | 1188 | SLIT2 | 4 | 20,172,206 |
| 1189 | SLIT2 | 4 | 20,177,664 | 1190 | SLIT2 | 4 | 20,194,981 |
| 1191 | SLIT2 | 4 | 20,202,844 | 1192 | PPARGC1A | 4 | 23,422,850 |
| 1193 | PPARGC1A | 4 | 23,424,760 | 1194 | PPARGC1A | 4 | 23,439,336 |
| 1195 | PPARGC1A | 4 | 23,445,751 | 1196 | SOD3 | 4 | 24,399,778 |
| 1197 | CCKAR | 4 | 26,098,368 | 1198 | CCKAR | 4 | 26,113,255 |
| 1199 | PCDH7 | 4 | 30,383,181 | 1200 | PCDH7 | 4 | 30,417,378 |
| 1201 | PCDH7 | 4 | 30,420,872 | 1202 | PCDH7 | 4 | 30,440,719 |
| 1203 | PCDH7 | 4 | 30,441,417 | 1204 | KIAA1239 | 4 | 36,941,226 |
| 1205 | KIAA1239 | 4 | 36,945,500 | 1206 | KIAA1239 | 4 | 37,005,415 |
| 1207 | KIAA1239 | 4 | 37,007,192 | 1208 | KIAA1239 | 4 | 37,133,996 |
| 1209 | KIAA1239 | 4 | 37,134,494 | 1210 | KIAA1239 | 4 | 37,140,169 |
| 1211 | KIAA1239 | 4 | 37,157,082 | 1212 | TBC1D1 | 4 | 37,815,251 |
| 1213 | UBE2K | 4 | 39,386,342 | 1214 | UBE2K | 4 | 39,494,511 |
| 1215 | N4BP2 | 4 | 39,864,889 | 1216 | LIMCH1 | 4 | 41,179,983 |
| 1217 | LIMCH1 | 4 | 41,257,923 | 1218 | LIMCH1 | 4 | 41,260,644 |
| 1219 | LIMCH1 | 4 | 41,285,436 | 1220 | LIMCH1 | 4 | 41,294,746 |
| 1221 | LIMCH1 | 4 | 41,308,688 | 1222 | LIMCH1 | 4 | 41,314,999 |
| 1223 | LIMCH1 | 4 | 41,354,243 | 1224 | LIMCH1 | 4 | 41,368,361 |
| 1225 | LIMCH1 | 4 | 41,394,864 | 1226 | LIMCH1 | 4 | 41,421,570 |
| 1227 | LIMCH1 | 4 | 41,429,386 | 1228 | LIMCH1 | 4 | 41,433,490 |
| 1229 | LOC389207 | 4 | 42,562,218 | 1230 | LOC389207 | 4 | 42,597,411 |
| 1231 | LPHN3 | 4 | 62,501,063 | 1232 | LPHN3 | 4 | 62,708,418 |
| 1233 | LPHN3 | 4 | 62,719,919 | 1234 | NPFFR2 | 4 | 73,054,817 |
| 1235 | NPFFR2 | 4 | 73,103,842 | 1236 | NPFFR2 | 4 | 73,115,321 |
| 1237 | NPFFR2 | 4 | 73,122,046 | 1238 | NPFFR2 | 4 | 73,122,182 |
| 1239 | NPFFR2 | 4 | 73,143,891 | 1240 | NPFFR2 | 4 | 73,145,571 |
| 1241 | NPFFR2 | 4 | 73,147,681 | 1242 | NPFFR2 | 4 | 73,153,403 |
| 1243 | NPFFR2 | 4 | 73,153,496 | 1244 | NPFFR2 | 4 | 73,154,786 |
| 1245 | NPFFR2 | 4 | 73,160,673 | 1246 | NPFFR2 | 4 | 73,161,337 |
| 1247 | NPFFR2 | 4 | 73,163,818 | 1248 | NPFFR2 | 4 | 73,219,749 |
| 1249 | NPFFR2 | 4 | 73,236,822 | 1250 | NPFFR2 | 4 | 73,263,574 |
| 1251 | NPFFR2 | 4 | 73,279,891 | 1252 | NPFFR2 | 4 | 73,291,379 |
| 1253 | CXCL9 | 4 | 77,140,838 | 1254 | CXCL9 | 4 | 77,152,469 |
| 1255 | SHROOM3 | 4 | 77,819,898 | 1256 | SHROOM3 | 4 | 77,835,586 |
| 1257 | SHROOM3 | 4 | 77,879,755 | 1258 | SHROOM3 | 4 | 77,883,172 |
| 1259 | SHROOM3 | 4 | 77,888,752 | 1260 | SHROOM3 | 4 | 77,944,496 |
| 1261 | SCD5 | 4 | 83,782,853 | 1262 | SCDS | 4 | 83,794,856 |
| 1263 | SCDS | 4 | 83,794,931 | 1264 | SCD5 | 4 | 83,803,520 |
| 1265 | SCD5 | 4 | 83,809,123 | 1266 | SCD5 | 4 | 83,816,306 |
| 1267 | SCD5 | 4 | 83,832,656 | 1268 | SCDS | 4 | 83,843,002 |
| 1269 | SCD5 | 4 | 83,845,049 | 1270 | SCD5 | 4 | 83,847,997 |
| 1271 | SCD5 | 4 | 83,920,815 | 1272 | SCD5 | 4 | 83,923,596 |
| 1273 | SCD5 | 4 | 83,944,297 | 1274 | HERC3 | 4 | 89,711,547 |
| 1275 | HERC3 | 4 | 89,741,644 | 1276 | HERC3 | 4 | 89,751,705 |
| 1277 | HERC3 | 4 | 89,786,790 | 1278 | HERC3 | 4 | 89,803,447 |
| 1279 | HERC3 | 4 | 89,822,108 | 1280 | FAM13A1 | 4 | 89,887,882 |
| 1281 | FAM13A1 | 4 | 89,950,302 | 1282 | FAM13A1 | 4 | 89,950,932 |
| 1283 | FAM13A1 | 4 | 89,952,905 | 1284 | FAM13A1 | 4 | 89,958,502 |
| 1285 | FAM13A1 | 4 | 90,116,396 | 1286 | FAM13A1 | 4 | 90,156,345 |
| 1287 | FAM13A1 | 4 | 90,160,944 | 1288 | FAM13A1 | 4 | 90,213,209 |
| 1289 | FAM13A1 | 4 | 90,214,821 | 1290 | PDLIM5 | 4 | 95,638,542 |
| 1291 | PDLIM5 | 4 | 95,660,333 | 1292 | PDLIM5 | 4 | 95,660,615 |
| 1293 | PDLIM5 | 4 | 95,679,972 | 1294 | PDLIM5 | 4 | 95,701,843 |
| 1295 | PDLIM5 | 4 | 95,715,905 | 1296 | PDLIM5 | 4 | 95,716,6,77 |
| 1297 | PDLIM5 | 4 | 95,728,786 | 1298 | PDLIM5 | 4 | 95,747,196 |
| 1299 | PDLIM5 | 4 | 95,780,482 | 1300 | PDLIM5 | 4 | 95,781,900 |
| 1301 | PDLIM5 | 4 | 95,791,722 | 1302 | PDLIM5 | 4 | 95,810,999 |
| 1303 | TSPAN5 | 4 | 99,862,201 | 1304 | DKK2 | 4 | 108,063,606 |
| 1305 | DKK2 | 4 | 108,084,600 | 1306 | DKK2 | 4 | 108,236,721 |
| 1307 | PAPSS1 | 4 | 108,757,215 | 1308 | PAPSS1 | 4 | 108,791,600 |
| 1309 | PAPSS1 | 4 | 108,824,661 | 1310 | PAPSS1 | 4 | 108,829,312 |
| 1311 | PAPSS1 | 4 | 108,831,390 | 1312 | PAPSS1 | 4 | 108,846,299 |
| 1313 | PAPSS1 | 4 | 108,901,540 | 1314 | PAPSS1 | 4 | 108,902,359 |
| 1315 | COL25A1 | 4 | 109,933,621 | 1316 | COL25A1 | 4 | 109,934,307 |
| 1317 | COL25A1 | 4 | 109,954,162 | 1318 | COL25A1 | 4 | 109,959,534 |
| 1319 | COL25A1 | 4 | 109,967,676 | 1320 | COL25A1 | 4 | 109,982,367 |
| 1321 | COL25A1 | 4 | 109,987,393 | 1322 | COL25A1 | 4 | 110,046,425 |
| 1323 | COL25A1 | 4 | 110,050,277 | 1324 | COL25A1 | 4 | 110,066,070 |
| 1325 | COL25A1 | 4 | 110,067,552 | 1326 | COL25A1 | 4 | 110,067,736 |
| 1327 | COL25A1 | 4 | 110,166,630 | 1328 | COL25A1 | 4 | 110,173,221 |
| 1329 | COL25A1 | 4 | 110,179,691 | 1330 | COL25A1 | 4 | 110,195,082 |
| 1331 | COL25A1 | 4 | 110,198,580 | 1332 | COL25A1 | 4 | 110,320,625 |
| 1333 | COL25A1 | 4 | 110,322,934 | 1334 | COL25A1 | 4 | 110,351,151 |
| 1335 | COL25A1 | 4 | 110,397,623 | 1336 | COL25A1 | 4 | 110,409,320 |
| 1337 | COL25A1 | 4 | 110,469,277 | 1338 | COL25A1 | 4 | 110,497,932 |
| 1339 | ANK2 | 4 | 114,184,993 | 1340 | ANK2 | 4 | 114,298,380 |
| 1341 | ANK2 | 4 | 114,298,464 | 1342 | ANK2 | 4 | 114,298,576 |
| 1343 | ANK2 | 4 | 114,318,660 | 1344 | ANK2 | 4 | 114,329,441 |
| 1345 | ANK2 | 4 | 114,338,099 | 1346 | ANK2 | 4 | 114,340,609 |
| 1347 | ANK2 | 4 | 114,348,644 | 1348 | ANK2 | 4 | 114,388,294 |
| 1349 | ANK2 | 4 | 114,424,380 | 1350 | ANK2 | 4 | 114,429,212 |
| 1351 | ANK2 | 4 | 114,436,352 | 1352 | ANK2 | 4 | 114,437,446 |
| 1353 | ANK2 | 4 | 114,445,280 | 1354 | ANK2 | 4 | 114,474,019 |
| 1355 | ANK2 | 4 | 114,480,090 | 1356 | ANK2 | 4 | 114,504,770 |
| 1357 | CAMK2D | 4 | 114,620,118 | 1358 | CAMK2D | 4 | 114,648,856 |
| 1359 | CAMK2D | 4 | 114,651,522 | 1360 | CAMK2D | 4 | 114,715,574 |
| 1361 | CAMK2D | 4 | 114,727,378 | 1362 | CAMK2D | 4 | 114,729,956 |
| 1363 | CAMK2D | 4 | 114,766,153 | 1364 | CAMK2D | 4 | 114,772,042 |
| 1365 | CAMK2D | 4 | 114,781,182 | 1366 | CAMK2D | 4 | 114,782,182 |
| 1367 | CAMK2D | 4 | 114,799,352 | 1368 | CAMK2D | 4 | 114,923,416 |
| 1369 | NDST3 | 4 | 119,169,101 | 1370 | NDST3 | 4 | 119,169,326 |
| 1371 | NDST3 | 4 | 119,186,644 | 1372 | NDST3 | 4 | 119,186,984 |
| 1373 | NDST3 | 4 | 119,210,639 | 1374 | NDST3 | 4 | 119,235,665 |
| 1375 | NDST3 | 4 | 119,256,090 | 1376 | NDST3 | 4 | 119,257,098 |
| 1377 | PRSS12 | 4 | 119,462,492 | 1378 | PRSS12 | 4 | 119,488,872 |
| 1379 | PRSS12 | 4 | 119,498,546 | 1380 | GPR103 | 4 | 122,484,922 |
| 1381 | GPR103 | 4 | 122,487,863 | 1382 | GPR103 | 4 | 122,510,972 |
| 1383 | GPR103 | 4 | 122,517,665 | 1384 | GPR103 | 4 | 122,518,528 |
| 1385 | GPR103 | 4 | 122,521,072 | 1386 | MAML3 | 4 | 140,868,325 |
| 1387 | MAML3 | 4 | 140,870,180 | 1388 | MAML3 | 4 | 140,878,048 |
| 1389 | MAML3 | 4 | 140,895,835 | 1390 | MAML3 | 4 | 140,896,430 |
| 1391 | MAML3 | 4 | 140,902,213 | 1392 | MAML3 | 4 | 140,983,797 |
| 1393 | MAML3 | 4 | 141,004,965 | 1394 | MAML3 | 4 | 141,007,145 |
| 1395 | MAML3 | 4 | 141,030,150 | 1396 | MAML3 | 4 | 141,043,992 |
| 1397 | MAML3 | 4 | 141,044,880 | 1398 | MAML3 | 4 | 141,051,231 |
| 1399 | MAML3 | 4 | 141,130,298 | 1400 | MAML3 | 4 | 141,138,533 |
| 1401 | MAML3 | 4 | 141,142,480 | 1402 | IL15 | 4 | 142,734,966 |
| 1403 | IL15 | 4 | 142,737,011 | 1404 | IL15 | 4 | 142,764,901 |
| 1405 | IL15 | 4 | 142,873,534 | 1406 | IL15 | 4 | 142,907,840 |
| 1407 | IL15 | 4 | 142,911,413 | 1408 | IL15 | 4 | 142,916,618 |
| 1409 | IL15 | 4 | 142,924,868 | 1410 | INPP4B | 4 | 143,195,583 |
| 1411 | INPP4B | 4 | 143,201,848 | 1412 | INPP4B | 4 | 143,221,023 |
| 1413 | INPP4B | 4 | 143,222,465 | 1414 | INPP4B | 4 | 143,223,756 |
| 1415 | INPP4B | 4 | 143,242,213 | 1416 | INPP4B | 4 | 143,262,085 |
| 1417 | INPP4B | 4 | 143,269,904 | 1418 | INPP4B | 4 | 143,269,935 |
| 1419 | INPP4B | 4 | 143,275,746 | 1420 | INPP4B | 4 | 143,295,856 |
| 1421 | INPP4B | 4 | 143,380,957 | 1422 | INPP4B | 4 | 143,436,768 |
| 1423 | INPP4B | 4 | 143,476,047 | 1424 | INPP4B | 4 | 143,593,360 |
| 1425 | POU4F2 | 4 | 147,727,101 | 1426 | POU4F2 | 4 | 147,731,368 |
| 1427 | POU4F2 | 4 | 147,739,219 | 1428 | POU4F2 | 4 | 147,748,659 |
| 1429 | POU4F2 | 4 | 147,758,283 | 1430 | POU4F2 | 4 | 147,768,753 |
| 1431 | POU4F2 | 4 | 147,799,157 | 1432 | DCLK2 | 4 | 151,229,912 |
| 1433 | DCLK2 | 4 | 151,230,058 | 1434 | DCLK2 | 4 | 151,230,190 |
| 1435 | DCLK2 | 4 | 151,236,783 | 1436 | DCLK2 | 4 | 151,259,013 |
| 1437 | DCLK2 | 4 | 151,291,338 | 1438 | DCLK2 | 4 | 151,294,657 |
| 1439 | DCLK2 | 4 | 151,355,610 | 1440 | DCLK2 | 4 | 151,366,761 |
| 1441 | DCLK2 | 4 | 151,372,390 | 1442 | DCLK2 | 4 | 151,378,402 |
| 1443 | DCLK2 | 4 | 151,401,865 | 1444 | CTSO | 4 | 157,072,641 |
| 1445 | CTSO | 4 | 157,090,787 | 1446 | CTSO | 4 | 157,091,152 |
| 1447 | CTSO | 4 | 157,119,171 | 1448 | CTSO | 4 | 157,124,684 |
| 1449 | CTSO | 4 | 157,125,805 | 1450 | FSTL5 | 4 | 162,602,916 |
| 1451 | FSTL5 | 4 | 162,608,107 | 1452 | FSTL5 | 4 | 162,696,532 |
| 1453 | FSTL5 | 4 | 162,784,518 | 1454 | FSTL5 | 4 | 162,786,977 |
| 1455 | FSTL5 | 4 | 162,828,621 | 1456 | FSTL5 | 4 | 162,852,707 |
| 1457 | FSTL5 | 4 | 162,863,519 | 1458 | FSTL5 | 4 | 162,901,935 |
| 1459 | FSTL5 | 4 | 162,923,589 | 1460 | FSTL5 | 4 | 162,953,212 |
| 1461 | FSTL5 | 4 | 163,113,553 | 1462 | FSTL5 | 4 | 163,171,406 |
| 1463 | FSTL5 | 4 | 163,172,602 | 1464 | FSTL5 | 4 | 163,204,852 |
| 1465 | FSTL5 | 4 | 163,224,500 | 1466 | FSTL5 | 4 | 163,262,634 |
| 1467 | FSTL5 | 4 | 163,274,178 | 1468 | FSTL5 | 4 | 163,275,058 |
| 1469 | FSTL5 | 4 | 163,282,207 | 1470 | FSTL5 | 4 | 163,306,761 |
| 1471 | FSTL5 | 4 | 163,316,581 | 1472 | FSTLS | 4 | 163,369,235 |
| 1473 | FSTL5 | 4 | 163,379,495 | 1474 | TLL1 | 4 | 166,984,039 |
| 1475 | TLL1 | 4 | 167,012,651 | 1476 | TLL1 | 4 | 167,017,110 |
| 1477 | TLL1 | 4 | 167,022,978 | 1478 | TLL1 | 4 | 167,186,954 |
| 1479 | TLL1 | 4 | 167,200,417 | 1480 | TLL1 | 4 | 167,215,864 |
| 1481 | TLL1 | 4 | 167,247,157 | 1482 | TLL1 | 4 | 167,250,203 |
| 1483 | PALLD | 4 | 169,678,354 | 1484 | PALLD | 4 | 169,678,426 |
| 1485 | PALLD | 4 | 169,680,592 | 1486 | PALLD | 4 | 169,685,203 |
| 1487 | PALLD | 4 | 169,690,892 | 1488 | PALLD | 4 | 169,693,362 |
| 1489 | PALLD | 4 | 169,693,682 | 1490 | PALLD | 4 | 169,701,141 |
| 1491 | PALLD | 4 | 169,702,565 | 1492 | PALLD | 4 | 169,713,776 |
| 1493 | PALLD | 4 | 169,721,943 | 1494 | PALLD | 4 | 169,766,914 |
| 1495 | PALLD | 4 | 169,828,987 | 1496 | PALLD | 4 | 169,831,329 |
| 1497 | PALLD | 4 | 169,831,979 | 1498 | PALLD | 4 | 169,854,703 |
| 1499 | PALLD | 4 | 169,866,028 | 1500 | PALLD | 4 | 169,894,890 |
| 1501 | PALLD | 4 | 169,910,252 | 1502 | PALLD | 4 | 169,910,404 |
| 1503 | PALLD | 4 | 169,938,153 | 1504 | PALLD | 4 | 169,938,977 |
| 1505 | PALLD | 4 | 169,939,539 | 1506 | PALLD | 4 | 170,077,542 |
| 1507 | PALLD | 4 | 170,085,964 | 1508 | PALLD | 4 | 170,111,195 |
| 1509 | PALLD | 4 | 170,118,492 | 1510 | PALLD | 4 | 170,135,598 |
| 1511 | PALLD | 4 | 170,136,535 | 1512 | PALLD | 4 | 170,137,800 |
| 1513 | ODZ3 | 4 | 183,476,755 | 1514 | ODZ3 | 4 | 183,478,510 |
| 1515 | ODZ3 | 4 | 183,764,260 | 1516 | ODZ3 | 4 | 183,790,373 |
| 1517 | ODZ3 | 4 | 183,876,156 | 1518 | ODZ3 | 4 | 183,917,862 |
| 1519 | ODZ3 | 4 | 183,917,955 | 1520 | ENPP6 | 4 | 185,234,241 |
| 1521 | ENPP6 | 4 | 185,239,625 | 1522 | ENPP6 | 4 | 185,251,384 |
| 1523 | ENPP6 | 4 | 185,254,744 | 1524 | ENPP6 | 4 | 185,258,135 |
| 1525 | ENPP6 | 4 | 185,260,510 | 1526 | ENPP6 | 4 | 185,263,371 |
| 1527 | ENPP6 | 4 | 185,269,255 | 1528 | ENPP6 | 4 | 185,314,773 |
| 1529 | CASP3 | 4 | 185,792,230 | 1530 | AHRR | 5 | 399,636 |
| 1531 | AHRR | 5 | 401,598 | 1532 | AHRR | 5 | 431,413 |
| 1533 | AHRR | 5 | 465,694 | 1534 | AHRR | 5 | 510,955 |
| 1535 | SEMA5A | 5 | 9,066,544 | 1536 | SEMA5A | 5 | 9,066,608 |
| 1537 | SEMA5A | 5 | 9,071,565 | 1538 | SEMA5A | 5 | 9,093,141 |
| 1539 | SEMA5A | 5 | 9,103,410 | 1540 | SEMA5A | 5 | 9,119,802 |
| 1541 | SEMASA | 5 | 9,474,794 | 1542 | SEMA5A | 5 | 9,477,130 |
| 1543 | SEMA5A | 5 | 9,488,305 | 1544 | SEMA5A | 5 | 9,489,697 |
| 1545 | SEMA5A | 5 | 9,613,249 | 1546 | DNAHS | 5 | 13,711,926 |
| 1547 | DNAHS | 5 | 13,776,956 | 1548 | DNAH5 | 5 | 13,780,181 |
| 1549 | DNAHS | 5 | 13,780,350 | 1550 | DNAH5 | 5 | 13,795,858 |
| 1551 | DNAH5 | 5 | 13,802,952 | 1552 | DNAH5 | 5 | 13,807,394 |
| 1553 | DNAHS | 5 | 13,822,974 | 1554 | DNAH5 | 5 | 13,832,743 |
| 1555 | DNAH5 | 5 | 13,841,886 | 1556 | DNAH5 | 5 | 13,842,420 |
| 1557 | DNAHS | 5 | 13,896,485 | 1558 | DNAH5 | 5 | 13,897,249 |
| 1559 | DNAH5 | 5 | 13,897,274 | 1560 | DNAH5 | 5 | 13,953,345 |
| 1561 | DNAH5 | 5 | 13,955,220 | 1562 | DNAH5 | 5 | 13,957,400 |
| 1563 | DNAH5 | 5 | 13,988,491 | 1564 | DNAH5 | 5 | 13,988,683 |
| 1565 | DNAH5 | 5 | 13,988,747 | 1566 | DNAH5 | 5 | 13,989,500 |
| 1567 | DNAH5 | 5 | 14,007,463 | 1568 | DNAH5 | 5 | 14,032,397 |
| 1569 | MYO10 | 5 | 16,711,524 | 1570 | MYO10 | 5 | 16,822,273 |
| 1571 | MYO10 | 5 | 16,836,327 | 1572 | MYO10 | 5 | 16,837,873 |
| 1573 | MYO10 | 5 | 16,838,077 | 1574 | CDH10 | 5 | 24,500,153 |
| 1575 | CDH10 | 5 | 24,533,855 | 1576 | CDH10 | 5 | 24,663,859 |
| 1577 | CDH10 | 5 | 24,727,740 | 1578 | SLC45A2 | 5 | 33,979,974 |
| 1579 | SLC45A2 | 5 | 33,990,637 | 1580 | CIQTNF3 | 5 | 34,083,721 |
| 1581 | SLC1A3 | 5 | 36,644,918 | 1582 | SLC1A3 | 5 | 36,670,315 |
| 1583 | SLC1A3 | 5 | 36,675,215 | 1584 | SLC1A3 | 5 | 36,712,840 |
| 1585 | SLC1A3 | 5 | 36,755,577 | 1586 | SLC1A3 | 5 | 36,755,736 |
| 1587 | EGFLAM | 5 | 38,246,651 | 1588 | EGFLAM | 5 | 38,307,878 |
| 1589 | EGFLAM | 5 | 38,337,535 | 1590 | EGFLAM | 5 | 38,357,270 |
| 1591 | EGFLAM | 5 | 38,357,424 | 1592 | EGFLAM | 5 | 38,382,422 |
| 1593 | EGFLAM | 5 | 38,386,400 | 1594 | EGFLAM | 5 | 38,402,419 |
| 1595 | EGFLAM | 5 | 38,450,087 | 1596 | EGFLAM | 5 | 38,485,966 |
| 1597 | EGFLAM | 5 | 38,492,523 | 1598 | EGFLAM | 5 | 38,502,504 |
| 1599 | ITGA1 | 5 | 52,114,759 | 1600 | ITGA1 | 5 | 52,130,669 |
| 1601 | ITGA1 | 5 | 52,133,521 | 1602 | ITGA1 | 5 | 52,134,676 |
| 1603 | ITGA1 | 5 | 52,146,916 | 1604 | ITGA1 | 5 | 52,149,807 |
| 1605 | ITGA1 | 5 | 52,155,113 | 1606 | ITGA1 | 5 | 52,185,286 |
| 1607 | ITGA1 | 5 | 52,194,858 | 1608 | ITGA1 | 5 | 52,223,785 |
| 1609 | ITGA1 | 5 | 52,231,264 | 1610 | ITGA1 | 5 | 52,250,338 |
| 1611 | ITGA1 | 5 | 52,259,107 | 1612 | ITGA1 | 5 | 52,265,502 |
| 1613 | ITGA1 | 5 | 52,277,790 | 1614 | ITGA1 | 5 | 52,278,143 |
| 1615 | ITGA1 | 5 | 52,285,233 | 1616 | ITGA1 | 5 | 52,289,413 |
| 1617 | ITGA2 | 5 | 52,369,177 | 1618 | ITGA2 | 5 | 52,376,821 |
| 1619 | ITGA2 | 5 | 52,388,590 | 1620 | ITGA2 | 5 | 52,402,662 |
| 1621 | ITGA2 | 5 | 52,425,212 | 1622 | PDE4D | 5 | 58,3 3,749 |
| 1623 | PDE4D | 5 | 58,367,198 | 1624 | PDE4D | 5 | 58,406,595 |
| 1625 | PDE4D | 5 | 58,429,089 | 1626 | PDE4D | 5 | 58,431,868 |
| 1627 | PDE4D | 5 | 58,432,005 | 1628 | PDE4D | 5 | 58,681,562 |
| 1629 | PDE4D | 5 | 58,712,773 | 1630 | PDE4D | 5 | 58,715,241 |
| 1631 | PDE4D | 5 | 58,895,884 | 1632 | PDE4D | 5 | 58,917,427 |
| 1633 | PDE4D | 5 | 58,935,951 | 1634 | PDE4D | 5 | 58,936,088 |
| 1635 | PDE4D | 5 | 58,941,002 | 1636 | ELOVL7 | 5 | 60,152,637 |
| 1637 | ELOVL7 | 5 | 60,152,959 | 1638 | ELOVL7 | 5 | 60,171,742 |
| 1639 | TNPO1 | 5 | 72,120,843 | 1640 | TNPO1 | 5 | 72,172,369 |
| 1641 | TNPO1 | 5 | 72,182,236 | 1642 | TNPO1 | 5 | 72,185,181 |
| 1643 | TNPO1 | 5 | 72,217,160 | 1644 | TNPO1 | 5 | 72,292,936 |
| 1645 | FCHO2 | 5 | 72,374,865 | 1646 | FCHO2 | 5 | 72,384,028 |
| 1647 | FCHO2 | 5 | 72,426,767 | 1648 | FCHO2 | 5 | 72,429,851 |
| 1649 | FCHO2 | 5 | 72,430,491 | 1650 | CMYAS | 5 | 79,029,508 |
| 1651 | CMYA5 | 5 | 79,058,467 | 1652 | CMYAS | 5 | 79,122,639 |
| 1653 | CMYA5 | 5 | 79,136,873 | 1654 | THBS4 | 5 | 79,356,433 |
| 1655 | THBS4 | 5 | 79,356,634 | 1656 | THBS4 | 5 | 79,375,707 |
| 1657 | THBS4 | 5 | 79,376,918 | 1658 | THBS4 | 5 | 79,387,300 |
| 1659 | THBS4 | 5 | 79,397,021 | 1660 | THBS4 | 5 | 79,411,480 |
| 1661 | THBS4 | 5 | 79,415,294 | 1662 | THBS4 | 5 | 79,425,956 |
| 1663 | VCAN | 5 | 82,795,773 | 1664 | VCAN | 5 | 82,798,333 |
| 1665 | VCAN | 5 | 82,806,912 | 1666 | VCAN | 5 | 82,818,286 |
| 1667 | VCAN | 5 | 82,822,305 | 1668 | VCAN | 5 | 82,851,164 |
| 1669 | VCAN | 5 | 82,869,125 | 1670 | VCAN | 5 | 82,873,387 |
| 1671 | VCAN | 5 | 82,875,330 | 1672 | VCAN | 5 | 82,900,222 |
| 1673 | VCAN | 5 | 82,914,275 | 1674 | MEF2C | 5 | 88,077,688 |
| 1675 | MEF2C | 5 | 88,091,517 | 1676 | MEF2C | 5 | 88,138,619 |
| 1677 | MEF2C | 5 | 88,161,609 | 1678 | MEF2C | 5 | 88,193,308 |
| 1679 | MEF2C | 5 | 88,216,865 | 1680 | GPR98 | 5 | 89,882,699 |
| 1681 | GPR98 | 5 | 89,899,035 | 1682 | GPR98 | 5 | 89,923,439 |
| 1683 | GPR98 | 5 | 89,976,245 | 1684 | GPR98 | 5 | 89,988,287 |
| 1685 | GPR98 | 5 | 89,992,773 | 1686 | GPR98 | 5 | 90,015,345 |
| 1687 | GPR98 | 5 | 90,020,637 | 1688 | GPR98 | 5 | 90,024,260 |
| 1689 | GPR98 | 5 | 90,026,080 | 1690 | GPR98 | 5 | 90,071,845 |
| 1691 | GPR98 | 5 | 90,078,258 | 1692 | GPR98 | 5 | 90,203,767 |
| 1693 | GPR98 | 5 | 90,444,837 | 1694 | CAST | 5 | 96,112,090 |
| 1695 | CAST | 5 | 96,112,771 | 1696 | CAST | 5 | 96,114,802 |
| 1697 | FBXL17 | 5 | 107,223,636 | 1698 | FBXL17 | 5 | 107,224,897 |
| 1699 | FBXL17 | 5 | 107,256,661 | 1700 | FBXL17 | 5 | 107,283,768 |
| 1701 | FBXL17 | 5 | 107,381,014 | 1702 | FBXL17 | 5 | 107,423,133 |
| 1703 | FBXL17 | 5 | 107,433,375 | 1704 | FBXL17 | 5 | 107,441,485 |
| 1705 | FBXL17 | 5 | 107,513,060 | 1706 | FBXL17 | 5 | 107,527,862 |
| 1707 | FBXL17 | 5 | 107,540,044 | 1708 | FBXL17 | 5 | 107,546,460 |
| 1709 | FBXL17 | 5 | 107,584,601 | 1710 | FBXL17 | 5 | 107,598,684 |
| 1711 | FBXL17 | 5 | 107,614,682 | 1712 | FBXL17 | 5 | 107,641,934 |
| 1713 | FBXL17 | 5 | 107,681,849 | 1714 | FBXL17 | 5 | 107,705,110 |
| 1715 | FHXL17 | 5 | 107,715,515 | 1716 | FBXL17 | 5 | 107,716,753 |
| 1717 | FBXL17 | 5 | 107,773,275 | 1718 | FBXL17 | 5 | 107,810,930 |
| 1719 | FBXL17 | 5 | 107,833,753 | 1720 | PJA2 | 5 | 108,713,575 |
| 1721 | PJA2 | 5 | 108,727,060 | 1722 | PJA2 | 5 | 108,742,197 |
| 1723 | PJA2 | 5 | 108,787,364 | 1724 | KCNN2 | 5 | 113,739,288 |
| 1725 | KCNN2 | 5 | 113,759,422 | 1726 | KCNN2 | 5 | 113,763,996 |
| 1727 | KCNN2 | 5 | 113,766,613 | 1728 | KCNN2 | 5 | 113,784,704 |
| 1729 | KCNN2 | 5 | 113,836,263 | 1730 | KCNN2 | 5 | 113,858,701 |
| 1731 | KCNN2 | 5 | 113,879,949 | 1732 | SEMA6A | 5 | 115,803,210 |
| 1733 | SEMA6A | 5 | 115,816,311 | 1734 | SEMA6A | 5 | 115,823,087 |
| 1735 | SEMA6A | 5 | 115,914,844 | 1736 | SEMA6A | 5 | 115,919,012 |
| 1737 | SEMA6A | 5 | 115,967,240 | 1738 | HSD17B4 | 5 | 118,865,967 |
| 1739 | HSD17B4 | 5 | 118,872,179 | 1740 | HSD17B4 | 5 | 118,872,385 |
| 1741 | HSD17B4 | 5 | 118,915,055 | 1742 | HSD17B4 | 5 | 118,918,814 |
| 1743 | HSD17B4 | 5 | 118,927,674 | 1744 | LOC340069 | 5 | 118,989,273 |
| 1745 | LOC340069 | 5 | 118,992,036 | 1746 | LOC340069 | 5 | 118,997,859 |
| 1747 | FTMT | 5 | 121,238,114 | 1748 | SNCAIP | 5 | 121,656,692 |
| 1749 | SNCAIP | 5 | 121,699,768 | 1750 | SNCAIP | 5 | 121,757,816 |
| 1751 | SNCAIP | 5 | 121,768,808 | 1752 | SNCAIP | 5 | 121,778,228 |
| 1753 | SNCAIP | 5 | 121,796,570 | 1754 | SNCAIP | 5 | 121,807,289 |
| 1755 | SNCAIP | 5 | 121,824,037 | 1756 | SNCAIP | 5 | 121,831,417 |
| 1757 | SNCAIP | 5 | 121,854,840 | 1758 | SNX2 | 5 | 122,116,630 |
| 1759 | SNX2 | 5 | 122,174,518 | 1760 | SNX2 | 5 | 122,201,930 |
| 1761 | SNX24 | 5 | 122,237,091 | 1762 | SNX24 | 5 | 122,273,273 |
| 1763 | SNX24 | 5 | 122,283,900 | 1764 | SNX24 | 5 | 122,295,903 |
| 1765 | SNX24 | 5 | 122,309,550 | 1766 | ADAMTS19 | 5 | 128,850,315 |
| 1767 | ADAMTS19 | 5 | 129,039,887 | 1768 | ADAMTS19 | 5 | 129,043,351 |
| 1769 | ADAMTS19 | 5 | 129,078,440 | 1770 | VDAC1 | 5 | 133,352,457 |
| 1771 | VDAC1 | 5 | 133,392,157 | 1772 | VDAC1 | 5 | 133,405,061 |
| 1773 | TRPC7 | 5 | 135,551,643 | 1774 | TRPC7 | 5 | 135,641,325 |
| 1775 | TRPC7 | 5 | 135,680,121 | 1776 | TRPC47 | 5 | 135,683,035 |
| 1777 | TRPC7 | 5 | 135,705,746 | 1778 | TRPC7 | 5 | 135,708,380 |
| 1779 | TRPC7 | 5 | 135,713,994 | 1780 | CTNNA1 | 5 | 138,200,119 |
| 1781 | CTNNA1 | 5 | 138,216,610 | 1782 | CTNNA1 | 5 | 138,254,666 |
| 1783 | CTNNA1 | 5 | 138,264,654 | 1784 | CTNNA1 | 5 | 138,289,570 |
| 1785 | CTNNA1 | 5 | 138,298,631 | 1786 | CTNNA1 | 5 | 138,300,046 |
| 1787 | ODZ2 | 5 | 167,085,900 | 1788 | ODZ2 | 5 | 167,105,972 |
| 1789 | ODZ2 | 5 | 167,191,250 | 1790 | ODZ2 | 5 | 167,391,524 |
| 1791 | ODZ2 | 5 | 167,400,913 | 1792 | ODZ2 | 5 | 167,485,949 |
| 1793 | ODZ2 | 5 | 167,486,071 | 1794 | ODZ2 | 5 | 167,486,458 |
| 1795 | ODZ2 | 5 | 167,491,001 | 1796 | ODZ2 | 5 | 167,527,141 |
| 1797 | ODZ2 | 5 | 167,576,871 | 1798 | ODZ2 | 5 | 167,606,948 |
| 1799 | ODZ2 | 5 | 167,634,311 | 1800 | WWC1 | 5 | 167,705,652 |
| 1801 | WWC1 | 5 | 167,720,663 | 1802 | WWC1 | 5 | 167,819,115 |
| 1803 | GABRP | 5 | 170,134,117 | 1804 | GABRP | 5 | 170,143,829 |
| 1805 | GABRP | 5 | 170,147,972 | 1806 | SERPINB6 | 6 | 2,926,223 |
| 1807 | SLC22A23 | 6 | 3,215,063 | 1808 | SLC22A23 | 6 | 3,222,297 |
| 1809 | SLC22A23 | 6 | 3,281,912 | 1810 | SLC22A23 | 6 | 3,282,785 |
| 1811 | SLC22A23 | 6 | 3,284,105 | 1812 | SLC22A23 | 6 | 3,395,265 |
| 1813 | SLC22A23 | 6 | 3,398,658 | 1814 | NEDD9 | 6 | 11,294,417 |
| 1815 | NEDD9 | 6 | 11,302,962 | 1816 | PHACTR1 | 6 | 12,845,008 |
| 1817 | PHACTR1 | 6 | 12,858,919 | 1818 | PHACTR1 | 6 | 12,989,841 |
| 1819 | PHACTR1 | 6 | 13,002,890 | 1820 | PHACTR1 | 6 | 13,006,278 |
| 1821 | PHACTR1 | 6 | 13,035,530 | 1822 | PHACTR1 | 6 | 13,035,831 |
| 1823 | PHACTR1 | 6 | 13,286,048 | 1824 | PHACTR1 | 6 | 13,297,254 |
| 1825 | PHACTR1 | 6 | 13,373,352 | 1826 | JARID2 | 6 | 15,388,186 |
| 1827 | JARID2 | 6 | 15,449,354 | 1828 | JARID2 | 6 | 15,499,187 |
| 1829 | JARID2 | 6 | 15,512,229 | 1830 | JARID2 | 6 | 15,516,507 |
| 1831 | JARID2 | 6 | 15,537,710 | 1832 | JARID2 | 6 | 15,567,913 |
| 1833 | JARID2 | 6 | 15,593,357 | 1834 | JARID2 | 6 | 15,624,264 |
| 1835 | ATXN1 | 6 | 16,595,385 | 1836 | ATXN1 | 6 | 16,755,970 |
| 1837 | ATXN1 | 6 | 16,785,550 | 1838 | ATXN1 | 6 | 16,808,077 |
| 1839 | RNF144B | 6 | 18,530,528 | 1840 | RNF144B | 6 | 18,546,855 |
| 1841 | RNF144B | 6 | 18,563,593 | 1842 | RNF144B | 6 | 18,566,740 |
| 1843 | RNF144B | 6 | 18,568,182 | 1844 | RNF144B | 6 | 18,574,729 |
| 1845 | SLC17A4 | 6 | 25,848,481 | 1846 | SLC17A4 | 6 | 25;861,619 |
| 1847 | SLC17A4 | 6 | 25,872,171 | 1848 | SLC17A4 | 6 | 25,876,893 |
| 1849 | SLC17A4 | 6 | 25,880,026 | 1850 | SLC17A4 | 6 | 25,887,371 |
| 1851 | SLC17A4 | 6 | 25,890,293 | 1852 | SLC17A4 | 6 | 25,900,229 |
| 1853 | SLC17A1 | 6 | 25,909,950 | 1854 | SLC17A1 | 6 | 25,921,129 |
| 1855 | SLC17A1 | 6 | 25,937,612 | 1856 | SLC17A1 | 6 | 25,937,638 |
| 1857 | SLC17A1 | 6 | 25,950,182 | 1858 | SLC17A3 | 6 | 25,971,584 |
| 1859 | SLC17A3 | 6 | 25,972,341 | 1860 | SLC17A3 | 6 | 25,972,877 |
| 1861 | SLC17A3 | 6 | 25,973,245 | 1862 | SLC17A3 | 6 | 25,978,521 |
| 1863 | SLC17A3 | 6 | 25,993,793 | 1864 | SLC17A2 | 6 | 26,009,112 |
| 1865 | SLC17A2 | 6 | 26,020,795 | 1866 | SLC17A2 | 6 | 26,022,056 |
| 1867 | SLC17A2 | 6 | 26,032,492 | 1868 | SLC17A2 | 6 | 26,042,503 |
| 1869 | SLC17A2 | 6 | 26,065,387 | 1870 | SLC17A2 | 6 | 26,067,106 |
| 1871 | SLC17A2 | 6 | 26,068,488 | 1872 | BTN3A1 | 6 | 26,506,534 |
| 1873 | BTN3A1 | 6 | 26,525,105 | 1874 | BTN3A3 | 6 | 26,559,532 |
| 1875 | MSH5 | 6 | 31,838,993 | 1876 | BTNL2 | 6 | 32,469,155 |
| 1877 | BTNL2 | 6 | 32,471,430 | 1878 | BTNL2 | 6 | 32,471,794 |
| 1879 | BRD2 | 6 | 32,961,584 | 1880 | LRFN2 | 6 | 40,462,772 |
| 1881 | LRFN2 | 6 | 40,635,612 | 1882 | PPP2R5D | 6 | 43,066,878 |
| 1883 | KLC4 | 6 | 43,173,891 | 1884 | PARC | 6 | 43,277,865 |
| 1885 | ABCC10 | 6 | 43,504,834 | 1886 | TJAP1 | 6 | 43,604,640 |
| 1887 | TJAP1 | 6 | 43,636,419 | 1888 | ELOVL5 | 6 | 53,221,300 |
| 1889 | ELOVLS | 6 | 53,238,244 | 1890 | ELOVL5 | 6 | 53,267,506 |
| 1891 | ELOVL5 | 6 | 53,280,718 | 1892 | ELOVL5 | 6 | 53,296,096 |
| 1893 | ELOVL5 | 6 | 53,298,093 | 1894 | ELOVL5 | 6 | 53,298,209 |
| 1895 | ELOVL5 | 6 | 53,350,763 | 1896 | ELOVL5 | 6 | 53,372,444 |
| 1897 | ELOVL5 | 6 | 53,386,201 | 1898 | RIMS1 | 6 | 72,772,376 |
| 1899 | RIMS1 | 6 | 72,784,876 | 1900 | RIMS 1 | 6 | 72,799,111 |
| 1901 | RIMS1 | 6 | 72,806,741 | 1902 | RIMS1 | 6 | 72,843,114 |
| 1903 | RIMS1 | 6 | 72,860,627 | 1904 | RIMS1 | 6 | 72,910,499 |
| 1905 | RIMS1 | 6 | 72,939,135 | 1906 | RIMS1 | 6 | 72,967,122 |
| 1907 | RIMS1 | 6 | 72,982,413 | 1908 | RIMS1 | 6 | 73,021,641 |
| 1909 | RIMS1 | 6 | 73,031,069 | 1910 | RIMS1 | 6 | 73,052,051 |
| 1911 | RIMS1 | 6 | 73,063,804 | 1912 | RIMS1 | 6 | 73,113,269 |
| 1913 | RIMS1 | 6 | 73,114,364 | 1914 | RIMS1 | 6 | 73,118,107 |
| 1915 | RIMS1 | 6 | 73,148,099 | 1916 | RIMS1 | 6 | 73,166,669 |
| 1917 | RIMS1 | 6 | 73,170,109 | 1918 | GABRR2 | 6 | 90,024,574 |
| 1919 | GABRR2 | 6 | 90,034,625 | 1920 | GABRR2 | 6 | 90,069,593 |
| 1921 | KLHL32 | 6 | 97,549,988 | 1922 | KLHL32 | 6 | 97,602,178 |
| 1923 | KLHL32 | 6 | 97,632,117 | 1924 | KLHL32 | 6 | 97,643,903 |
| 1925 | KLHL32 | 6 | 97,673,659 | 1926 | SLC22A16 | 6 | 110,850,843 |
| 1927 | SLC22A16 | 6 | 110,853,761 | 1928 | SLC22A16 | 6 | 110,857,245 |
| 1929 | SLC22A16 | 6 | 110,870,568 | 1930 | SLC22A16 | 6 | 110,897,601 |
| 1931 | SLC22A16 | 6 | 110,900,785 | 1932 | SLC22A16 | 6 | 110,903,098 |
| 1933 | SLC22A16 | 6 | 110,914,475 | 1934 | HS3ST5 | 6 | 114,502,447 |
| 1935 | TRDN | 6 | 123,553,000 | 1936 | TRDN | 6 | 123,728,987 |
| 1937 | TRDN | 6 | 123,738,465 | 1938 | TRDN | 6 | 123,743,637 |
| 1939 | TRDN | 6 | 123,749,431 | 1940 | TRDN | 6 | 123,753,816 |
| 1941 | TRDN | 6 | 123,766,181 | 1942 | TRDN | 6 | 123,774,707 |
| 1943 | TRDN | 6 | 123,862,152 | 1944 | TRDN | 6 | 123,865,649 |
| 1945 | TRDN | 6 | 123,871,263 | 1946 | TRDN | 6 | 123,875,156 |
| 1947 | TRDN | 6 | 123,877,575 | 1948 | TRDN | 6 | 123,878,251 |
| 1949 | TRDN | 6 | 123,931,045 | 1950 | TRDN | 6 | 123,991,308 |
| 1951 | TRDN | 6 | 124,006,657 | 1952 | TRDN | 6 | 124,017,012 |
| 1953 | NKAIN2 | 6 | 124,086,439 | 1954 | NKAIN2 | 6 | 124,145,158 |
| 1955 | NKAIN2 | 6 | 124,173,325 | 1956 | NKAIN2 | 6 | 124,174,594 |
| 1957 | NKAIN2 | 6 | 124,462,813 | 1958 | NKAIN2 | 6 | 124,473,210 |
| 1959 | NKAIN2 | 6 | 124,481,807 | 1960 | NKAIN2 | 6 | 124,488,366 |
| 1961 | NKAIN2 | 6 | 124,492,154 | 1962 | NKAIN2 | 6 | 124,658,216 |
| 1963 | NKAIN2 | 6 | 124,742,885 | 1964 | NKAIN2 | 6 | 124,850,664 |
| 1965 | NKAIN2 | 6 | 124,859,016 | 1966 | NKAIN2 | 6 | 124,953,233 |
| 1967 | NKAIN2 | 6 | 124,962,839 | 1968 | NKAIN2 | 6 | 124,977,809 |
| 1969 | NKAIN2 | 6 | 124,987,301 | 1970 | NKAIN2 | 6 | 125,020,491 |
| 1971 | NKAIN2 | 6 | 125,198,148 | 1972 | EYA4 | 6 | 133,617,346 |
| 1973 | EYA4 | 6 | 133,625,496 | 1974 | EYA4 | 6 | 133,672,254 |
| 1975 | EYA4 | 6 | 133,674,026 | 1976 | EYA4 | 6 | 133,678,316 |
| 1977 | EYA4 | 6 | 133,790,074 | 1978 | EYA4 | 6 | 133,876,222 |
| 1979 | PDE7B | 6 | 136,206,400 | 1980 | PDE7B | 6 | 136,244,527 |
| 1981 | PDE7B | 6 | 136,251,409 | 1982 | PDE7B | 6 | 136,262,109 |
| 1983 | PDE7B | 6 | 136,263,312 | 1984 | PDE7B | 6 | 136,308,009 |
| 1985 | PDE7B | 6 | 136,313,220 | 1986 | PDE7B | 6 | 136,326,169 |
| 1987 | PDE7B | 6 | 136,346,863 | 1988 | PDE7B | 6 | 136,357,577 |
| 1989 | PDE7B | 6 | 136,408,668 | 1990 | PLAGL1 | 6 | 144,319,261 |
| 1991 | PLAGAL1 | 6 | 144,326,195 | 1992 | PLAGL1 | 6 | 144,345,108 |
| 1993 | PLAGL1 | 6 | 144,355,380 | 1994 | STX11 | 6 | 144,498,222 |
| 1995 | STX11 | 6 | 144,554,694 | 1996 | STX11 | 6 | 144,584,781 |
| 1997 | UTRN | 6 | 144,651,202 | 1998 | UTRN | 6 | 144,665,805 |
| 1999 | UTRN | 6 | 144,698,346 | 2000 | UTRN | 6 | 144,723,376 |
| 2001 | UTRN | 6 | 144,728,467 | 2002 | UTRN | 6 | 144,943,208 |
| 2003 | SYNE1 | 6 | 152,485,437 | 2004 | SYNE1 | 6 | 152,511,829 |
| 2005 | SYNE1 | 6 | 152,549,890 | 2006 | SYNE1 | 6 | 152,683,133 |
| 2007 | SYNE1 | 6 | 152,683,299 | 2008 | SYNE1 | 6 | 152,685,563 |
| 2009 | TFB1M | 6 | 155,621,092 | 2010 | TFB1M | 6 | 155,621,249 |
| 2011 | TFB1M | 6 | 155,663,514 | 2012 | TFB1M | 6 | 155,686,995 |
| 2013 | SLC22A3 | 6 | 160,754,505 | 2014 | SLC22A3 | 6 | 160,762,527 |
| 2015 | SLC22A3 | 6 | 160,783,522 | 2016 | PARK2 | 6 | 161,894,294 |
| 2017 | PARK2 | 6 | 161,894,696 | 2018 | PARK2 | 6 | 161,910,473 |
| 2019 | PARK2 | 6 | 161,921,426 | 2020 | PARK2 | 6 | 161,941,630 |
| 2021 | PARK2 | 6 | 161,952,077 | 2022 | PARK2 | 6 | 161,960,395 |
| 2023 | PARK2 | 6 | 162,001,625 | 2024 | PARK2 | 6 | 162,001,753 |
| 2025 | PARK2 | 6 | 162,026,087 | 2026 | PARK2 | 6 | 162,194,371 |
| 2027 | PARK2 | 6 | 162,199,188 | 2028 | PARK2 | 6 | 162,199,563 |
| 2029 | PARK2 | 6 | 162,201,523 | 2030 | PARK2 | 6 | 162,204,134 |
| 2031 | PARK2 | 6 | 162,651,333 | 2032 | PARK2 | 6 | 162,656,326 |
| 2033 | PARK2 | 6 | 162,666,256 | 2034 | PARK2 | 6 | 162,666,349 |
| 2035 | PARK2 | 6 | 162,709,903 | 2036 | PARK2 | 6 | 162,719,593 |
| 2037 | PARK2 | 6 | 162,724,935 | 2038 | PARK2 | 6 | 162,725,125 |
| 2039 | PARK2 | 6 | 162,728,999 | 2040 | PARK2 | 6 | 162,770,133 |
| 2041 | PARK2 | 6 | 162,782,182 | 2042 | PARK2 | 6 | 162,795,324 |
| 2043 | PARK2 | 6 | 162,871,284 | 2044 | PARK2 | 6 | 162,891,538 |
| 2045 | PARK2 | 6 | 162,902,279 | 2046 | PARK2 | 6 | 162,916,186 |
| 2047 | PARK2 | 6 | 162,947,100 | 2048 | PARK2 | 6 | 162,960,031 |
| 2049 | PARK2 | 6 | 163,103,553 | 2050 | PACRG | 6 | 163,515,155 |
| 2051 | PACRG | 6 | 163,520,215 | 2052 | PACRG | 6 | 163,520,791 |
| 2053 | PACRG | 6 | 163,523,252 | 2054 | PACRG | 6 | 163,571,731 |
| 2055 | PDE10A | 6 | 165,679,905 | 2056 | PDE10A | 6 | 165,711,808 |
| 2057 | PDE10A | 6 | 165,714,078 | 2058 | PDE10A | 6 | 165,741,392 |
| 2059 | PDE10A | 6 | 165,787,781 | 2060 | PDE10A | 6 | 165,834,350 |
| 2061 | PDE10A | 6 | 165,835,911 | 2062 | PDE10A | 6 | 165,840,748 |
| 2063 | PDE10A | 6 | 165,868,541 | 2064 | PDE10A | 6 | 165,883,564 |
| 2065 | PDE10A | 6 | 166,023,863 | 2066 | CARD11 | 7 | 2,917,872 |
| 2067 | CARD11 | 7 | 2,923,531 | 2068 | CARD11 | 7 | 2,928,819 |
| 2069 | CARD11 | 7 | 2,985,3 79 | 2070 | SDK1 | 7 | 4,118,110 |
| 2071 | SDK1 | 7 | 4,118,615 | 2072 | SDK1 | 7 | 4,140,139 |
| 2073 | SDK1 | 7 | 4,152,211 | 2074 | SDK1 | 7 | 4,152,228 |
| 2075 | SDK1 | 7 | 4,153,102 | 2076 | SDK1 | 7 | 4,158,337 |
| 2077 | SDK1 | 7 | 4,162,572 | 2078 | SDK1 | 7 | 4,174,523 |
| 2079 | SDK1 | 7 | 4,174,875 | 2080 | SDK1 | 7 | 4,214,383 |
| 2081 | SDK1 | 7 | 4,230,185 | 2082 | SDK1 | 7 | 4,272,360 |
| 2083 | NXPH1 | 7 | 8,578,640 | 2084 | NXPH1 | 7 | 8,589,612 |
| 2085 | NXPH1 | 7 | 8,600,283 | 2086 | NXPH1 | 7 | 8,608,391 |
| 2087 | NXPH1 | 7 | 8,647,759 | 2088 | NXPH1 | 7 | 8,662,332 |
| 2089 | NXPH1 | 7 | 8,700,069 | 2090 | NXPH1 | 7 | 8,780,133 |
| 2091 | SCIN | 7 | 12,576,204 | 2092 | SCIN | 7 | 12,576,513 |
| 2093 | SCIN | 7 | 12,579,777 | 2094 | SCIN | 7 | 12,634,312 |
| 2095 | ETV1 | 7 | 13,906,357 | 2096 | ETV1 | 7 | 13,945,334 |
| 2097 | ETV1 | 7 | 13,982,894 | 2098 | ETV1 | 7 | 13,987,646 |
| 2099 | ETV1 | 7 | 13,996,042 | 2100 | SNX13 | 7 | 17,861,458 |
| 2101 | SNX13 | 7 | 17,937,235 | 2102 | SNX13 | 7 | 17,974,835 |
| 2103 | FERD3L | 7 | 19,153,324 | 2104 | FERD3L | 7 | 19,164,657 |
| 2105 | FERD3L | 7 | 19,167,340 | 2106 | FERD3L | 7 | 19,346,900 |
| 2107 | FERD3L | 7 | 19,353,440 | 2108 | FERD3L | 7 | 19,353,457 |
| 2109 | FERD3L | 7 | 19,354,351 | 2110 | STK31 | 7 | 23,742,002 |
| 2111 | STK31 | 7 | 23,760,407 | 2112 | STK31 | 7 | 23,859,818 |
| 2113 | STK31 | 7 | 23,906,389 | 2114 | NPY | 7 | 24,297,290 |
| 2115 | NPY | 7 | 24,298,324 | 2116 | SKAP2 | 7 | 26,917,430 |
| 2117 | SKAP2 | 7 | 26,969,979 | 2118 | SKAP2 | 7 | 26,982,204 |
| 2119 | CREB5 | 7 | 28,798,822 | 2120 | CREB5 | 7 | 28,804,778 |
| 2121 | CREB5 | 7 | 28,811,671 | 2122 | CREB5 | 7 | 28,812,569 |
| 2123 | CREB5 | 7 | 28,815,049 | 2124 | CREB5 | 7 | 28,818,987 |
| 2125 | CREB5 | 7 | 28,870,041 | 2126 | CREBS | 7 | 28,906,935 |
| 2127 | CHN2' | 7 | 29,215,509 | 2128 | CHN2 | 7 | 29,483,175 |
| 2129 | SCRN1 | 7 | 29,926,736 | 2130 | SCRN1 | 7 | 29,929,11 |
| 2131 | SCRN1 | 7 | 29,929,459 | 2132 | SCRN1 | 7 | 30,010,342 |
| 2133 | SCRN1 | 7 | 30,011,939 | 2134 | PLEKHA8 | 7 | 30,034,151 |
| 2135 | PLEKHA8 | 7 | 30,037,069 | 2136 | SCRN1 | 7 | 30,052,432 |
| 2137 | PLEKHA8 | 7 | 30,083,942 | 2138 | PLEKHA8 | 7 | 30,097,689 |
| 2139 | PLEKHA8 | 7 | 30,099,727 | 2140 | CRHR2 | 7 | 30,663,882 |
| 2141 | CRHR2 | 7 | 30,665,429 | 2142 | CRHR2 | 7 | 30,676,000 |
| 2143 | CRHR2 | 7 | 30,775,386 | 2144 | FLJ22374 | 7 | 30,787,631 |
| 2145 | FLJ22374 | 7 | 30,803,801 | 2146 | FLJ22374 | 7 | 30,832,290 |
| 2147 | FLJ22374 | 7 | 30,842,957 | 2148 | FLJ22374 | 7 | 30,843,167 |
| 2149 | FLJ22374 | 7 | 30,860,754 | 2150 | FLJ22374 | 7 | 30,865,222 |
| 2151 | FLJ22374 | 7 | 30,880,636 | 2152 | PDE1C | 7 | 31,794,059 |
| 2153 | PDE1C | 7 | 31,815,231 | 2154 | PDE1C | 7 | 31,931,223 |
| 2155 | PDE1A | 7 | 31,968,183 | 2156 | PDE1C | 7 | 31,979,118 |
| 2157 | PDE1A | 7 | 32,001,981 | 2158 | PDE1C | 7 | 32,037,670 |
| 2159 | PDE1A | 7 | 32,064,508 | 2160 | PDE1C | 7 | 32,094,250 |
| 2161 | PDE1C | 7 | 32,095,407 | 2162 | PDE1C | 7 | 32,167,820 |
| 2163 | BMPER | 7 | 33,934,543 | 2164 | BMPER | 7 | 33,942,093 |
| 2165 | BMPER | 7 | 34,056,348 | 2166 | BMPER | 7 | 34,067,083 |
| 2167 | BMPER | 7 | 34,077,080 | 2168 | BMPER | 7 | 34,077,746 |
| 2169 | BMPER | 7 | 34,107,862 | 2170 | BMPER | 7 | 34,184,882 |
| 2171 | EEPD1 | 7 | 36,280,985 | 2172 | EEPD1 | 7 | 36,306,659 |
| 2173 | VPS41 | 7 | 38,745,317 | 2174 | VPS41 | 7 | 38,758,751 |
| 2175 | VPS41 | 7 | 38,759,185 | 2176 | VPS41 | 7 | 38,768,570 |
| 2177 | VPS41 | 7 | 38,768,817 | 2178 | VPS41 | 7 | 38,812,386 |
| 2179 | VPS41 | 7 | 38,855,826 | 2180 | VPS41 | 7 | 38,866,716 |
| 2181 | VPS41 | 7 | 38,936,691 | 2182 | CDC2L5 | 7 | 39,994,009 |
| 2183 | CDC2L5 | 7 | 40,051,522 | 2184 | CDC2L5 | 7 | 40,056,457 |
| 2185 | CDC2L5 | 7 | 40,063,896 | 2186 | CDC2L5 | 7 | 40,067,860 |
| 2187 | CDC2L5 | 7 | 40,083,623 | 2188 | CDC2L5 | 7 | 40,124,213 |
| 2189 | IGFBP3 | 7 | 45,916,971 | 2190 | ABCA13 | 7 | 48,253,360 |
| 2191 | ABCA13 | 7 | 48,256,966 | 2192 | ABCA13 | 7 | 48,351,224 |
| 2193 | ABCA13 | 7 | 48,388,584 | 2194 | ABCA13 | 7 | 48,399,261 |
| 2195 | ABCA13 | 7 | 48,415,696 | 2196 | ABCA13 | 7 | 48,420,703 |
| 2197 | ABCA13 | 7 | 48,424,446 | 2198 | ABCA13 | 7 | 48,425,312 |
| 2199 | ABCA13 | 7 | 48,427,057 | 2200 | ABCA13 | 7 | 48,471,003 |
| 2201 | ABCA13 | 7 | 48,472,098 | 2202 | ABCA13 | 7 | 48,477,112 |
| 2203 | ABCA13 | 7 | 48,516,335 | 2204 | ABCA13 | 7 | 48,521,048 |
| 2205 | ABCA13 | 7 | 48,537,470 | 2206 | ABCA13 | 7 | 48,537,723 |
| 2207 | ABCA13 | 7 | 48,538,115 | 2208 | ABCA13 | 7 | 48,549,904 |
| 2209 | ABCA13 | 7 | 48,553,490 | 2210 | ABCA13 | 7 | 48,557,23 |
| 2211 | ABCA13 | 7 | 48,622,770 | 2212 | ABCA13 | 7 | 48,656,578 |
| 2213 | ABCA13 | 7 | 48,707,129 | 2214 | ABCA13 | 7 | 48,714,222 |
| 2215 | GRB10 | 7 | 50,707,082 | 2216 | GRB10 | 7 | 50,738,123 |
| 2217 | GRB10 | 7 | 50,745,597 | 2218 | GRB10 | 7 | 50,751,377 |
| 2219 | GRB10 | 7 | 50,752,942 | 2220 | GRB10 | 7 | 50,767,383 |
| 2221 | GRB10 | 7 | 50,790,805 | 2222 | GRB10 | 7 | 50,791,895 |
| 2223 | GRB10 | 7 | 50,797,467 | 2224 | GRB10 | 7 | 50,814,098 |
| 2225 | GRB10 | 7 | 50,839,403 | 2226 | GRB10 | 7 | 50,878,465 |
| 2227 | WBSCR17 | 7 | 69,708,664 | 2228 | WBSCR17 | 7 | 69,982,663 |
| 2229 | WBSCR17 | 7 | 70,003,525 | 2230 | WBSCR17 | 7 | 70,008,405 |
| 2231 | WBSCR17 | 7 | 70,009,819 | 2232 | WBSCR17 | 7 | 70,020,032 |
| 2233 | WBSCR17 | 7 | 70,196,421 | 2234 | WBSCR17 | 7 | 70,318,305 |
| 2235 | WBSCR17 | 7 | 70,327,601 | 2236 | WBSCR17 | 7 | 70,328,803 |
| 2237 | WBSCR17 | 7 | 70,438,836 | 2238 | WBSCR17 | 7 | 70,486,737 |
| 2239 | WBSCR17 | 7 | 70,508,901 | 2240 | WBSCR17 | 7 | 70,527,975 |
| 2241 | WBSCR17 | 7 | 70,537,333 | 2242 | WBSCR17 | 7 | 70,542,497 |
| 2243 | WBSCR17 | 7 | 70,553,758 | 2244 | WBSCR17 | 7 | 70,605,584 |
| 2245 | WBSCR17 | 7 | 70,610,898 | 2246 | WBSCR17 | 7 | 70,622,039 |
| 2247 | WBSCR17 | 7 | 70,661,813 | 2248 | WBSCR17 | 7 | 70,677,555 |
| 2249 | LIMK1 | 7 | 73,143,124 | 2250 | LIMK1 | 7 | 73,155,050, |
| 2251 | GTF2IRD1 | 7 | 73,536,522 | 2252 | GTF2IRD1 | 7 | 73,552,324 |
| 2253 | GTF2IRD1 | 7 | 73,739,845 | 2254 | HIP1 | 7 | 74,641,558 |
| 2255 | HIP1 | 7 | 75,014,321 | 2256 | HIP1 | 7 | 75,071,583 |
| 2257 | HIP1 | 7 | 75,093,684 | 2258 | HIP1 | 7 | 75,125,346 |
| 2259 | HIP1 | 7 | 75,132,211 | 2260 | HIP1 | 7 | 75,134,148 |
| 2261 | HIP1 | 7 | 75,137,795 | 2262 | HIP1 | 7 | 75,146,409 |
| 2263 | HIP1 | 7 | 75,149,973 | 2264 | HIP1 | 7 | 75,186,242 |
| 2265 | HIP1 | 7 | 75,198,775 | 2266 | MAGI2 | 7 | 77,062,170 |
| 2267 | MAGI2 | 7 | 77,491,345 | 2268 | MAGI2 | 7 | 77,497,177 |
| 2269 | MAGI2 | 7 | 77,562,324 | 2270 | MAGI2 | 7 | 77,596,871 |
| 2271 | MAGI2 | 7 | 77,707,944 | 2272 | MAGI2 | 7 | 77,807,229 |
| 2273 | MAGI2 | 7 | 77,811,392 | 2274 | MAGI2 | 7 | 77,818,446 |
| 2275 | MAGI2 | 7 | 78,085,231 | 2276 | MAGI2 | 7 | 78,087,129 |
| 2277 | MAGI2 | 7 | 78,091,049 | 2278 | MAGI2 | 7 | 78,305,682 |
| 2279 | MAGI2 | 7 | 78,331,358 | 2280 | MAGI2 | 7 | 78,350,745 |
| 2281 | MAGI2 | 7 | 78,378,995 | 2282 | MAGI2 | 7 | 78,393,425 |
| 2283 | MAGI2 | 7 | 78,548,747 | 2284 | MAGI2 | 7 | 78,554,879 |
| 2285 | MAGI2 | 7 | 78,581,862 | 2286 | MAGI2 | 7 | 78,589,166 |
| 2287 | MAGI2 | 7 | 78,621,037 | 2288 | MAGI2 | 7 | 78,634,193 |
| 2289 | MAGI2 | 7 | 78,639,527 | 2290 | MAGI2 | 7 | 78,753,565 |
| 2291 | MAGI2 | 7 | 78,760,470 | 2292 | MAGI2 | 7 | 78,779,395 |
| 2293 | MAGI2 | 7 | 78,817,132 | 2294 | MAGI2 | 7 | 78,829,240 |
| 2295 | MAGI2 | 7 | 78,847,570 | 2296 | MAGI2 | 7 | 78,865,894 |
| 2297 | MAGI2 | 7 | 78,867,313 | 2298 | MAGI2 | 7 | 78,872,510 |
| 2299 | MAGI2 | 7 | 78,884,537 | 2300 | MAGI2 | 7 | 78,894,096 |
| 2301 | MAGI2 | 7 | 78,902,124 | 2302 | GNAI1 | 7 | 79,129,442 |
| 2303 | CACNA2D1 | 7 | 81,000,419 | 2304 | CACNA2D1 | 7 | 81,262,844 |
| 2305 | CACNA2D1 | 7 | 81,397,331 | 2306 | CACNA2D1 | 7 | 81,409,867 |
| 2307 | CACNA2D1 | 7 | 81,410,981 | 2308 | CACNA2D1 | 7 | 81,501,211 |
| 2309 | CACNA2D1 | 7 | 81,551,628 | 2310 | PCLO | 7 | 81,734,489 |
| 2311 | PCLO | 7 | 81,754,524 | 2312 | CACNA2D1 | 7 | 81,819,131 |
| 2313 | CACNA2D1 | 7 | 81,829,947 | 2314 | CACNA2D1 | 7 | 81,842,488 |
| 2315 | CACNA2D1 | 7 | 81,846,503 | 2316 | PCLO | 7 | 81,895,238 |
| 2317 | CACNA2D1 | 7 | 81,925,395 | 2318 | PCLO | 7 | 81,935,063 |
| 2319 | PCLO | 7 | 81,953,190 | 2320 | PCLO | 7 | 82,252,869 |
| 2321 | PCLO | 7 | 82,287,971 | 2322 | PCLO | 7 | 82,291,644 |
| 2323 | PCLO | 7 | 82,292,608 | 2324 | PCLO | 7 | 82,317,658 |
| 2325 | PCLO | 7 | 82,341,345 | 2326 | PCLO | 7 | 82,341,378 |
| 2327 | PCLO | 7 | 82,358,102 | 2328 | PCLO | 7 | 82,391,885 |
| 2329 | PCLO | 7 | 82,401,452 | 2330 | PCLO | 7 | 82,419,795 |
| 2331 | PCLO | 7 | 82,454,771 | 2332 | PCLO | 7 | 82,581,867 |
| 2333 | PCLO | 7 | 82,636,908 | 2334 | PCLO | 7 | 82,659,120 |
| 2335 | SEMA3E | 7 | 82,881,204 | 2336 | SEMA3E | 7 | 82,882,893 |
| 2337 | SEMA3E | 7 | 82,883,620 | 2338 | SEMA3E | 7 | 82,895,964 |
| 2339 | SEMA3E | 7 | 82,913,893 | 2340 | SEMA3E | 7 | 82,935,623 |
| 2341 | SEMA3E | 7 | 82,946,973 | 2342 | SEMA3E | 7 | 82,970,533 |
| 2343 | SEMA3E | 7 | 83,029,806 | 2344 | SEMA3E | 7 | 83,047,248 |
| 2345 | SEMA3E | 7 | 83,061,152 | 2346 | SEMA3E | 7 | 83,125,543 |
| 2347 | SEMA3A | 7 | 83,697,056 | 2348 | ABCB4 | 7 | 86,372,139 |
| 2349 | ABCB4 | 7 | 86,868,839 | 2350 | ABCB4 | 7 | 86,877,077 |
| 2351 | ABCB4 | 7 | 86,883,520 | 2352 | ABCB4 | 7 | 86,892,183 |
| 2353 | ABCB4 | 7 | 86,897,339 | 2354 | ABCB4 | 7 | 86,941,606 |
| 2355 | ADAM22 | 7 | 87,441,679 | 2356 | ADAM22 | 7 | 87,471,187 |
| 2357 | ADAM22 | 7 | 87,471,440 | 2358 | ADAM22 | 7 | 87,554,236 |
| 2359 | ADAM22 | 7 | 87,557,024 | 2360 | ADAM22 | 7 | 87,579,450 |
| 2361 | ADAM22 | 7 | 87,606,110 | 2362 | GTPBP10 | 7 | 89,357,529 |
| 2363 | GTPBP10 | 7 | 89,852,344 | 2364 | GTPBP10 | 7 | 89,857,773 |
| 2365 | PPP1R9A | 7 | 94,064,776 | 2366 | PON1 | 7 | 94,266,443 |
| 2367 | PPP1R9A | 7 | 94,453,195 | 2368 | PPP1R9A | 7 | 94,534,075 |
| 2369 | PPP1R9A | 7 | 94,569,490 | 2370 | PPP1R9A | 7 | 94,607,167 |
| 2371 | PPP1R9A | 7 | 94,632,721 | 2372 | PPP1R9A | 7 | 94,632,764 |
| 2373 | PPP1R9A | 7 | 94,681,040 | 2374 | PPP1R9A | 7 | 94,681,285 |
| 2375 | PPP1R9A | 7 | 94,717,161 | 2376 | PPP1R9A | 7 | 94,733,892 |
| 2377 | PPP1R9A | 7 | 94,746,827 | 2378 | PPP1R9A | 7 | 94,747,598 |
| 2379 | DYNCII1 | 7 | 94,748,308 | 2380 | PPP1R9A | 7 | 94,759,427 |
| 2381 | PON1 | 7 | 94,775,829 | 2382 | PON1 | 7 | 94,776,193 |
| 2383 | PON1 | 7 | 94,780,701 | 2384 | DYNCII1 | 7 | 94,786,218 |
| 2385 | PON1 | 7 | 94,804,103 | 2386 | DYNCII1 | 7 | 95,242,508 |
| 2387 | DYNC1I1 | 7 | 95,242,854 | 2388 | DYNCII1 | 7 | 95,242,972 |
| 2389 | DYNCII1 | 7 | 95,325,994 | 2390 | DYNCII1 | 7 | 95,369,751 |
| 2391 | DYNC1I1 | 7 | 95,384,186 | 2392 | DYNCII1 | 7 | 95,451,958 |
| 2393 | DYNCII1 | 7 | 95,452,403 | 2394 | DYNCII1 | 7 | 95,503,995 |
| 2395 | DYNCII1 | 7 | 95,548,598 | 2396 | DYNCII1 | 7 | 95,556,260 |
| 2397 | DYNCII1 | 7 | 95,581,854 | 2398 | ZNF3 | 7 | 99,024,937 |
| 2399 | ZNF3 | 7 | 99,480,681 | 2400 | CUX1 | 7 | 100,785,884 |
| 2401 | EMID2 | 7 | 100,787,736 | 2402 | EMID2 | 7 | 100,828,478 |
| 2403 | CUX1 | 7 | 100,962,370 | 2404 | CUX1 | 7 | 101,306,594 |
| 2405 | CUX1 | 7 | 101,391,246 | 2406 | CUX1 | 7 | 101,533,073 |
| 2407 | CUX1 | 7 | 101,612,087 | 2408 | CUX1 | 7 | 101,694,544 |
| 2409 | CUX1 | 7 | 101,704,241 | 2410 | CUX1 | 7 | 101,704,367 |
| 2411 | KCND2 | 7 | 119,301,300 | 2412 | KCND2 | 7 | 119,564,389 |
| 2413 | KCND2 | 7 | 120,060,200 | 2414 | KCND2 | 7 | 120,110,963 |
| 2415 | KCND2 | 7 | 120,117,902 | 2416 | KCND2 | 7 | 120,130,284 |
| 2417 | KCND2 | 7 | 120,197,437 | 2418 | KCND2 | 7 | 120,197,820 |
| 2419 | CADPS2 | 7 | 121,805,395 | 2420 | CADPS2 | 7 | 121,865,650 |
| 2421 | CADPS2 | 7 | 122,066,038 | 2422 | CADPS2 | 7 | 122,074,175 |
| 2423 | CADPS2 | 7 | 122,146,928 | 2424 | CADPS2 | 7 | 122,329,673 |
| 2425 | CADPS2 | 7 | 122,329,881 | 2426 | SLC13A1 | 7 | 122,523,202 |
| 2427 | SLC13A1 | 7 | 122,572,907 | 2428 | SLC13A1 | 7 | 122,585,872 |
| 2429 | SLC13A1 | 7 | 122,587,919 | 2430 | SLC13A1 | 7 | 122,596,470 |
| 2431 | SLC13A1 | 7 | 122,613,192 | 2432 | SLC13A1 | 7 | 122,631,110 |
| 2433 | GRM8 | 7 | 125,769,933 | 2434 | GRM8 | 7 | 125,816,605 |
| 2435 | GRM8 | 7 | 125,837,771 | 2436 | GRM8 | 7 | 125,855,816 |
| 2437 | GRM8 | 7 | 125,871,585 | 2438 | GRM8 | 7 | 125,879,584 |
| 2439 | GRM8 | 7 | 125,888,362 | 2440 | GRM8 | 7 | 126,095,504 |
| 2441 | GRM8 | 7 | 126,114,635 | 2442 | GRM8 | 7 | 126,156,034 |
| 2443 | GRM8 | 7 | 126,159,625 | 2444 | GRM8 | 7 | 126,163,369 |
| 2445 | GRM8 | 7 | 126,167,389 | 2446 | GRM8 | 7 | 126,170,542 |
| 2447 | GRM8 | 7 | 126,216,753 | 2448 | GRM8 | 7 | 126,295,139 |
| 2449 | GRM8 | 7 | 126,299,256 | 2450 | GRM8 | 7 | 126,521,407 |
| 2451 | GRM8 | 7 | 126,552,824 | 2452 | GRM8 | 7 | 126,623,193 |
| 2453 | EXOC4 | 7 | 132,456,559 | 2454 | EXOC4 | 7 | 132,476,956 |
| 2455 | EXOC4 | 7 | 132,754,129 | 2456 | EXOC4 | 7 | 132,754,657 |
| 2457 | EXOC4 | 7 | 132,781,480 | 2458 | EXOC4 | 7 | 132,788,184 |
| 2459 | EXOC4 | 7 | 132,789,020 | 2460 | EXOC4 | 7 | 132,797,614 |
| 2461 | EXOC4 | 7 | 132,917,865 | 2462 | EXOC4 | 7 | 132,953,563 |
| 2463 | EXOC4 | 7 | 132,991,170 | 2464 | EXOC4 | 7 | 132,996,175 |
| 2465 | EXOC4 | 7 | 133,002,858 | 2466 | EXOC4 | 7 | 133,045,046 |
| 2467 | EXOC4 | 7 | 133,081,578 | 2468 | EXOC4 | 7 | 133,240,386 |
| 2469 | EXOC4 | 7 | 133,241,477 | 2470 | EXOC4 | 7 | 133,246,042 |
| 2471 | EXOC4 | 7 | 133,352,637 | 2472 | EXOC4 | 7 | 133,378,037 |
| 2473 | DGKI | 7 | 136,578,863 | 2474 | DGKI | 7 | 136,726,387 |
| 2475 | DGKI | 7 | 136,730,489 | 2476 | DGKI | 7 | 136,757,487 |
| 2477 | DGKI | 7 | 136,769,909 | 2478 | DGKI | 7 | 136,770,651 |
| 2479 | DGKI | 7 | 136,772,491 | 2480 | DGKI | 7 | 136,782,783 |
| 2481 | CREB3L2 | 7 | 137,343,428 | 2482 | TBXAS1 | 7 | 138,950,079 |
| 2483 | TBXAS1 | 7 | 139,135,304 | 2484 | TBXAS1 | 7 | 139,225,646 |
| 2485 | TEXAS1 | 7 | 139,311,144 | 2486 | CNTNAP2 | 7 | 145,425,744 |
| 2487 | CNTNAP2 | 7 | 145,431,076 | 2488 | CNTNAP2 | 7 | 145,485,790 |
| 2489 | CNTNAP2 | 7 | 145,686,573 | 2490 | CNTNAP2 | 7 | 145,710,838 |
| 2491 | CNTNAP2 | 7 | 145,724,542 | 2492 | CNTNAP2 | 7 | 145,743,361 |
| 2493 | CNTNAP2 | 7 | 145,865,999 | 2494 | CNTNAP2 | 7 | 145,906,353 |
| 2495 | CNTNAP2 | 7 | 146,204,639 | 2496 | CNTNAP2 | 7 | 146,414,565 |
| 2497 | CNTNAP2 | 7 | 146,671,711 | 2498 | CNTNAP2 | 7 | 146,671,930 |
| 2499 | CNTNAP2 | 7 | 146,689,924 | 2500 | CNTNAP2 | 7 | 146,724,262 |
| 2501 | CNTNAP2 | 7 | 146,762,507 | 2502 | CNTNAP2 | 7 | 146,788,354 |
| 2503 | CNTNAP2 | 7 | 146,795,593 | 2504 | CNTNAP2 | 7 | 146,800,067 |
| 2505 | CNTNAP2 | 7 | 146,816,270 | 2506 | CNTNAP2 | 7 | 146,816,456 |
| 2507 | CNTNAP2 | 7 | 147,669,284 | 2508 | CNTNAP2 | 7 | 147,695,819 |
| 2509 | CNTNAP2 | 7 | 147,712,266 | 2510 | CNTNAP2 | 7 | 147,748,438 |
| 2511 | CNTNAP2 | 7 | 147,749,182 | 2512 | PTPRN2 | 7 | 156,566,641 |
| 2513 | PTPRN2 | 7 | 156,598,397 | 2514 | PTPRN2 | 7 | 157,007,427 |
| 2515 | PTPRN2 | 7 | 157,007,523 | 2516 | PTPRN2 | 7 | 157,017,352 |
| 2517 | PTPRN2 | 7 | 157,044,149 | 2518 | PTPRN2 | 7 | 157,149,210 |
| 2519 | PTPRN2 | 7 | 157,175,770 | 2520 | PTPRN2 | 7 | 157,421,786 |
| 2521 | PTPRN2 | 7 | 157,499,807 | 2522 | PTPRN2 | 7 | 157,511,759 |
| 2523 | PTPRN2 | 7 | 157,609,674 | 2524 | PTPRN2 | 7 | 157,619,954 |
| 2525 | PTPRN2 | 7 | 157,621,221 | 2526 | PTPRN2 | 7 | 157,662,662 |
| 2527 | PTPRN2 | 7 | 157,875,149 | 2528 | PTPRN2 | 7 | 157,904,553 |
| 2529 | PTPRN2 | 7 | 157,908,408 | 2530 | PTPRN2 | 7 | 157,919,551 |
| 2531 | PTPRN2 | 7 | 157,920,856 | 2532 | PTPRN2 | 7 | 157,973,350 |
| 2533 | PTPRN2 | 7 | 158,015,477 | 2534 | PTPRN2 | 7 | 158,029,235 |
| 2535 | PTPRN2 | 7 | 158,077,879 | 2536 | CSMD1 | 8 | 2,819,792 |
| 2537 | CSMD1 | 8 | 2,849,233 | 2538 | CSMD1 | 8 | 2,958,438 |
| 2539 | CSMD1 | 8 | 3,207,377 | 2540 | CSMD1 | 8 | 3,209,023 |
| 2541 | CSMD1 | 8 | 3,209,202 | 2542 | CSMD1 | 8 | 3,211,642 |
| 2543 | CSMD1 | 8 | 3,213,631 | 2544 | CSMD1 | 8 | 3,216,613 |
| 2545 | CSMD1 | 8 | 3,230,805 | 2546 | CSMD1 | 8 | 3,232,022 |
| 2547 | CSMD1 | 8 | 3,232,222 | 2548 | CSMD1 | 8 | 3,250,935 |
| 2549 | CSMD1 | 8 | 3,275,090 | 2550 | CSMD1 | 8 | 3,340,467 |
| 2551 | MCPH1 | 8 | 6,266,787 | 2552 | MCPH1 | 8 | 6,282,480 |
| 2553 | MCPH1 | 8 | 6,288,880 | 2554 | MCPH1 | 8 | 6,289,562 |
| 2555 | MCPH1 | 8 | 6,290,919 | 2556 | MCPH1 | 8 | 6,298,146 |
| 2557 | MCPH1 | 8 | 6,306,930 | 2558 | MCPH1 | 8 | 6,307,055 |
| 2559 | MCPH1 | 8 | 6,307,171 | 2560 | MCPH1 | 8 | 6,314,156 |
| 2561 | MCPH1 | 8 | 6,314,682 | 2562 | MCPH1 | 8 | 6,316,969 |
| 2563 | MCPH1 | 8 | 6,357,338 | 2564 | MCPH1 | 8 | 6,362,452 |
| 2565 | MCPH1 | 8 | 6,373,849 | 2566 | MCPH1 | 8 | 6,379,955 |
| 2567 | MCPH1 | 8 | 6,407,942 | 2568 | MCPH1 | 8 | 6,461,123 |
| 2569 | MCPH1 | 8 | 6,461,154 | 2570 | MCPH1 | 8 | 6,461,166 |
| 2571 | MCPH1 | 8 | 6,466,450 | 2572 | MCPH1 | 8 | 6,475,913 |
| 2573 | MCPH1 | 8 | 6,475,985 | 2574 | MCPH1 | 8 | 6,509,698 |
| 2575 | DLC1 | 8 | 13,006,870 | 2576 | DLC1 | 8 | 13,013,028 |
| 2577 | DLC1 | 8 | 13,014,125 | 2578 | DLC1 | 8 | 13,016,678 |
| 2579 | SGCZ | 8 | 13,962,387 | 2580 | SGCZ | 8 | 14,242,631 |
| 2581 | SGCZ | 8 | 14,247,697 | 2582 | SGCZ | 8 | 14,292,030 |
| 2583 | SGCZ | 8 | 14,303,019 | 2584 | SGCZ | 8 | 14,313,399 |
| 2585 | SGCZ | 8 | 14,316,760 | 2586 | SGCZ | 8 | 14,330,923 |
| 2587 | SGCZ | 8 | 14,351,624 | 2588 | SGCZ | 8 | 14,357,736 |
| 2589 | SGCZ | 8 | 14,362,495 | 2590 | SGCZ | 8 | 14,390,316 |
| 2591 | SGCZ | 8 | 14,390,686 | 2592 | SGCZ | 8 | 14,420,466 |
| 2593 | SGCZ | 8 | 14,594,583 | 2594 | SGCZ | 8 | 14,623,703 |
| 2595 | SGCZ | 8 | 14,915,797 | 2596 | SGCZ | 8 | 15,179,623 |
| 2597 | SLC7A2 | 8 | 17,436,984 | 2598 | SLC7A2 | 8 | 17,454,671 |
| 2599 | SLC7A2 | 8 | 17,460,770 | 2600 | SLC7A2 | 8 | 17,471,816 |
| 2601 | SLC7A2 | 8 | 17,472,551 | 2602 | PSD3 | 8 | 18,428,754 |
| 2603 | PSD3 | 8 | 18,431,654 | 2604 | PSD3 | 8 | 18,529,675 |
| 2605 | PSD3 | 8 | 18,559,152 | 2606 | PSD3 | 8 | 18,560,182 |
| 2607 | PSD3 | 8 | 18,709,420 | 2608 | PSD3 | 8 | 18,734,189 |
| 2609 | PSD3 | 8 | 18,737,733 | 2610 | PSD3 | 8 | 18,737,939 |
| 2611 | PSD3 | 8 | 18,757,428 | 2612 | PSD3 | 8 | 18,765,584 |
| 2613 | PSD3 | 8 | 18,772,229 | 2614 | PSD3 | 8 | 18,819,182 |
| 2615 | PSD3 | 8 | 18,953,685 | 2616 | ATP6V1B2 | 8 | 20,124,602 |
| 2617 | ATP6VIB2 | 8 | 20,137,423 | 2618 | GFRA2 | 8 | 21,554,368 |
| 2619 | GFRA2 | 8 | 21,642,357 | 2620 | XPO7 | 8 | 21,886,104 |
| 2621 | XPO7 | 8 | 21,892,330 | 2622 | XPO7 | 8 | 21,901,374 |
| 2623 | XPO7 | 8 | 21,916,371 | 2624 | XPO7 | 8 | 21,924,970 |
| 2625 | PPP3CC | 8 | 22,380,522 | 2626 | PEBP4 | 8 | 22,632,507 |
| 2627 | PEBP4 | 8 | 22,634,532 | 2628 | PEBP4 | 8 | 22,648,953 |
| 2629 | PEBP4 | 8 | 22,828,329 | 2630 | PEBP4 | 8 | 22,842,095 |
| 2631 | ENTPD4 | 8 | 23,415,906 | 2632 | SLC25A37 | 8 | 23,453,211 |
| 2633 | SLC25A37 | 8 | 23,454,180 | 2634 | SLC25A37 | 8 | 23,471,600 |
| 2635 | SLC25A37 | 8 | 23,490,677 | 2636 | UNC5D | 8 | 35,516,694 |
| 2637 | UNC5D | 8 | 35,526,631 | 2638 | UNC5D | 8 | 35,529,087 |
| 2639 | UNC5D | 8 | 35,531,300 | 2640 | UNC5D | 8 | 35,544,036 |
| 2641 | UNC5D | 8 | 35,574,119 | 2642 | UNC5D | 8 | 35,586,829 |
| 2643 | UNC5D | 8 | 35,752,828 | 2644 | UNC5D | 8 | 36,047,709 |
| 2645 | SFRP1 | 8 | 41,244,318 | 2646 | SFRP1 | 8 | 41,254,926 |
| 2647 | SFRP1 | 8 | 41,256,599 | 2648 | SFRP1 | 8 | 41,262,183 |
| 2649 | SFRP1 | 8 | 41,276,396 | 2650 | PRKDC | 8 | 49,008,811 |
| 2651 | SNTG1 | 8 | 50,996,988 | 2652 | SNTG1 | 8 | 51,028,383 |
| 2653 | SNTG1 | 8 | 51,082,766 | 2654 | SNTG1 | 8 | 51,106,589 |
| 2655 | SNTG1 | 8 | 51,127,991 | 2656 | SNTG1 | 8 | 51,142,873 |
| 2657 | SNTG1 | 8 | 51,234,566 | 2658 | SNTG1 | 8 | 51,276,836 |
| 2659 | SNTG1 | 8 | 51,289,749 | 2660 | SNTG1 | 8 | 51,320,466 |
| 2661 | SNTG1 | 8 | 51,329,479 | 2662 | SNTG1 | 8 | 51,333,190 |
| 2663 | SNTG1 | 8 | 51,557,371 | 2664 | SNTG1 | 8 | 51,603,877 |
| 2665 | SNTG1 | 8 | 51,605,982 | 2666 | SNTG1 | 8 | 51,616,502 |
| 2667 | SNTG1 | 8 | 51,620,897 | 2668 | SNTG1 | 8 | 51,624,716 |
| 2669 | SNTG1 | 8 | 51,624,769 | 2670 | SNTG1 | 8 | 51,859,715 |
| 2671 | SNTG1 | 8 | 51,888,421 | 2672 | SNTG1 | 8 | 51,900,540 |
| 2673 | LYN | 8 | 56,987,448 | 2674 | LYN | 8 | 57,034,636 |
| 2675 | LYN | 8 | 57,039,314 | 2676 | LYN | 8 | 57,053,118 |
| 2677 | LYN | 8 | 57,054,784 | 2678 | LYN | 8 | 57,107,537 |
| 2679 | LYN | 8 | 57,108,529 | 2680 | LYN | 8 | 57,108,914 |
| 2681 | LYN | 8 | 57,110,114 | 2682 | PLAG1 | 8 | 57,263,345 |
| 2683 | TOX | 8 | 59,972,394 | 2684 | TOX | 8 | 59,980,027 |
| 2685 | TOX | 8 | 60,043,593 | 2686 | NKAIN3 | 8 | 63,450,713 |
| 2687 | NKAIN3 | 8 | 63,461,008 | 2688 | NKAIN3 | 8 | 63,496,176 |
| 2689 | NKAIN3 | 8 | 63,498,893 | 2690 | NKAIN3 | 8 | 63,502,193 |
| 2691 | NKAIN3 | 8 | 63,552,090 | 2692 | NKAIN3 | 8 | 63,555,581 |
| 2693 | NKAIN3 | 8 | 63,557,252 | 2694 | NKAIN3 | 8 | 63,608,657 |
| 2695 | NKAIN3 | 8 | 63,649,765 | 2696 | NKAIN3 | 8 | 63,651,711 |
| 2697 | NKAIN3 | 8 | 63,659,439 | 2698 | NKAIN3 | 8 | 63,662,642 |
| 2699 | NKAIN3 | 8 | 63,670,367 | 2700 | NKAIN3 | 8 | 63,692,734 |
| 2701 | NKAIN3 | 8 | 63,693,165 | 2702 | NKAIN3 | 8 | 63,711,876 |
| 2703 | NKAIN3 | 8 | 63,750,511 | 2704 | NKAIN3 | 8 | 63,781,261 |
| 2705 | NKAIN3 | 8 | 63,821,921 | 2706 | NKAIN3 | 8 | 63,822,189 |
| 2707 | NKAIN3 | 8 | 63,825,859 | 2708 | NKAIN3 | 8 | 63,862,125 |
| 2709 | NKAIN3 | 8 | 63,868,884 | 2710 | NKAIN3 | 8 | 63,897,057 |
| 2711 | NKAIN3 | 8 | 63,900,353 | 2712 | NKAIN3 | 8 | 63,930,295 |
| 2713 | NKAIN3 | 8 | 63,935,531 | 2714 | NKAIN3 | 8 | 63,936,341 |
| 2715 | NKAIN3 | 8 | 63,989,029 | 2716 | NKAIN3 | 8 | 63,994,228 |
| 2717 | NKAIN3 | 8 | 64,008,938 | 2718 | NKAIN3 | 8 | 64,011,110 |
| 2719 | NKAIN3 | 8 | 64,020,373 | 2720 | NKAIN3 | 8 | 64,030,047 |
| 2721 | NKAIN3 | 8 | 64,033,505 | 2722 | NKAIN3 | 8 | 64,056,708 |
| 2723 | NKAIN3 | 8 | 64,068,304 | 2724 | NKAIN3 | 8 | 64,084,956 |
| 2725 | DEPDC2 | 8 | 69,043,977 | 2726 | DEPDC2 | 8 | 69,121,712 |
| 2727 | DEPDC2 | 8 | 69,176,372 | 2728 | DEPDC2 | 8 | 69,176,632 |
| 2729 | DEPDC2 | 8 | 69,207,400 | 2730 | DEPDC2 | 8 | 69,208,963 |
| 2731 | DEPDC2 | 8 | 69,209,296 | 2732 | DEPDC2 | 8 | 69,269,021 |
| 2733 | DEPDC2 | 8 | 69,308,908 | 2734 | DEPDC2 | 8 | 69,314,054 |
| 2735 | KCNB2 | 8 | 73,601,624 | 2736 | KCNB2 | 8 | 73,614,029 |
| 2737 | KCNB2 | 8 | 73,618,324 | 2738 | KCNB2 | 8 | 73,628,966 |
| 2739 | KCNB2 | 8 | 73,638,413 | 2740 | KCNB2 | 8 | 73,651,827 |
| 2741 | KCNB2 | 8 | 73,740,108 | 2742 | KCNB2 | 8 | 73,742,670 |
| 2743 | KCNB2 | 8 | 73,754,776 | 2744 | KCNB2 | 8 | 73,762,984 |
| 2745 | KCNB2 | 8 | 73,776,211 | 2746 | KCNB2 | 8 | 73,780,051 |
| 2747 | KCNB2 | 8 | 73,780,154 | 2748 | KCNB2 | 8 | 73,798,241 |
| 2749 | KCNB2 | 8 | 73,818,016 | 2750 | KCNB2 | 8 | 73,821,159 |
| 2751 | KCNB2 | 8 | 73,901,806 | 2752 | KCNB2 | 8 | 73,975,854 |
| 2753 | KCNB2 | 8 | 74,014,801 | 2754 | KCNB2 | 8 | 74,026,549 |
| 2755 | CRISPLD1 | 8 | 76,072,659 | 2756 | CRISPLD1 | 8 | 76,086,655 |
| 2757 | CRISPLD1 | 8 | 76,103,499 | 2758 | CRISPLD1 | 8 | 76,109,250 |
| 2759 | CRISPLD1 | 8 | 76,158,048 | 2760 | CRISPLD1 | 8 | 76,167,652 |
| 2761 | MMP16 | 8 | 89,112,004 | 2762 | MMP16 | 8 | 89,163,898 |
| 2763 | MMP16 | 8 | 89,276,696 | 2764 | MMP16 | 8 | 89,337,659 |
| 2765 | MMP16 | 8 | 89,358,861 | 2766 | MMP16 | 8 | 89,399,547 |
| 2767 | MMP16 | 8 | 89,413,738 | 2768 | MMP16 | 8 | 89,414,303 |
| 2769 | MMP16 | 8 | 89,421,430 | 2770 | GRHL2 | 8 | 102,613,455 |
| 2771 | GRHL2 | 8 | 102,617,910 | 2772 | GRHL2 | 8 | 102,618,040 |
| 2773 | GRHL2 | 8 | 102,690,095 | 2774 | GRHL2 | 8 | 102,697,131 |
| 2775 | GRHL2 | 8 | 102,718,857 | 2776 | GRHL2 | 8 | 102,740,588 |
| 2777 | GRHL2 | 8 | 102,746,981 | 2778 | GRHL2 | 8 | 102,759,164 |
| 2779 | GRHL2 | 8 | 102,769,504 | 2780 | NCALD | 8 | 102,777,547 |
| 2781 | NCALD | 8 | 102,812,988 | 2782 | NCALD | 8 | 102,868,670 |
| 2783 | NCALD | 8 | 102,903,699 | 2784 | NCALD | 8 | 102,907,196 |
| 2785 | BAALC | 8 | 104,238,041 | 2786 | BAALC | 8 | 104,265,315 |
| 2787 | BAALC | 8 | 104,271,601 | 2788 | BAALC | 8 | 104,319,015 |
| 2789 | BAALC | 8 | 104,325,901 | 2790 | ZFPM2 | 8 | 106,413,317 |
| 2791 | ZFPM2 | 8 | 106,488,930 | 2792 | ZFPM2 | 8 | 106,504,399 |
| 2793 | ZFPM2 | 8 | 106,505,157 | 2794 | ZFPM2 | 8 | 106,507,154 |
| 2795 | ZFPM2 | 8 | 106,507,756 | 2796 | ZFPM2 | 8 | 106,530,047 |
| 2797 | ZFPM2 | 8 | 106,794,362 | 2798 | ZFPM2 | 8 | 106,800,179 |
| 2799 | ZFPM2 | 8 | 106,801,553 | 2800 | ZFPM2 | 8 | 106,824,935 |
| 2801 | ZFPM2 | 8 | 106,828,892 | 2802 | ZFPM2 | 8 | 106,847,240 |
| 2803 | ZFPM2 | 8 | 106,864,559 | 2804 | ZFPM2 | 8 | 106,880,858 |
| 2805 | ZFPM2 | 8 | 106,880,874 | 2806 | ZFPM2 | 8 | 106,888,057 |
| 2807 | ZFPM2 | 8 | 106,910,116 | 2808 | ZFPM2 | 8 | 106,922,815 |
| 2809 | CSMD3 | 8 | 113,479,682 | 2810 | CSMD3 | 8 | 113,505,578 |
| 2811 | CSMD3 | 8 | 114,209,523 | 2812 | CSMD3 | 8 | 114,259,566 |
| 2813 | CSMD3 | 8 | 114,319,937 | 2814 | CSMD3 | 8 | 114,458,866 |
| 2815 | CSMD3 | 8 | 114,475,512 | 2816 | CSMD3 | 8 | 114,519,694 |
| 2817 | CSMD3 | 8 | 114,552,699 | 2818 | SAMD12 | 8 | 119,448,826 |
| 2819 | SAMD12 | 8 | 119,531,636 | 2820 | SAMD12 | 8 | 119,532,372 |
| 2821 | SAMD12 | 8 | 119,532,971 | 2822 | SAMD12 | 8 | 119,536,976 |
| 2823 | SAMD12 | 8 | 119,622,273 | 2824 | SAMD12 | 8 | 119,669,482 |
| 2825 | SAMD12 | 8 | 119,671,788 | 2826 | SAMD12 | 8 | 119,676,462 |
| 2827 | SAMD12 | 8 | 119,719,731 | 2828 | SAMD12 | 8 | 119,740,378 |
| 2829 | SAMD12 | 8 | 119,741,315 | 2830 | FBXO32 | 8 | 124,583,159 |
| 2831 | FBXO32 | 8 | 124,595,944 | 2832 | FER1L6 | 8 | 125,020,334 |
| 2833 | FER1L6 | 8 | 125,032,627 | 2834 | FER1L6 | 8 | 125,053,616 |
| 2835 | FER1L6 | 8 | 125,102,788 | 2836 | FER1L6 | 8 | 125,108,933 |
| 2837 | FER1L6 | 8 | 125,122,813 | 2838 | FER1L6 | 8 | 125,123,546 |
| 2839 | FER1L6 | 8 | 125,142,993 | 2840 | FER1L6 | 8 | 125,144,097 |
| 2841 | FER1L6 | 8 | 125,145,770 | 2842 | FER1L6 | 8 | 125,147,531 |
| 2843 | FER1L6 | 8 | 125,157,817 | 2844 | FER1L6 | 8 | 125,158,437 |
| 2845 | FER1L6 | 8 | 125,171,480 | 2846 | FER1L6 | 8 | 125,186,054 |
| 2847 | FER1L6 | 8 | 125,223,811 | 2848 | MTSS1 | 8 | 125,643,333 |
| 2849 | MTSS1 | 8 | 125,648,174 | 2850 | MTSS1 | 8 | 125,649,171 |
| 2851 | MTSS1 | 8 | 125,651,280 | 2852 | MTSS1 | 8 | 125,669,999 |
| 2853 | MTSS1 | 8 | 125,690,229 | 2854 | MTSS1 | 8 | 125,700,462 |
| 2855 | MTSS1 | 8 | 125,802,301 | 2856 | MTSS1 | 8 | 125,954,015 |
| 2857 | FAM84B | 8 | 127,836,839 | 2858 | DDEF1 | 8 | 131,125,920 |
| 2859 | DDEF1 | 8 | 131,179,288 | 2860 | DDEF1 | 8 | 131,199,776 |
| 2861 | DDEF1 | 8 | 131,225,253 | 2862 | DDEF1 | 8 | 131,236,230 |
| 2863 | DDEF1 | 8 | 131,247,872 | 2864 | DDEF1 | 8 | 131,254,886 |
| 2865 | DDEF1 | 8 | 131,286,874 | 2866 | DDEF1 | 8 | 131,296,201 |
| 2867 | DDEF1 | 8 | 131,325,860 | 2868 | DDEF1 | 8 | 131,350,781 |
| 2869 | DDEF1 | 8 | 131,365,205 | 2870 | DDEF1 | 8 | 131,373,060 |
| 2871 | DDEF1 | 8 | 131,379,036 | 2872 | DDEF1 | 8 | 131,394,209 |
| 2873 | DDEF1 | 8 | 131,406,218 | 2874 | DDEF1 | 8 | 131,407,018 |
| 2875 | DDEF1 | 8 | 131,415,088 | 2876 | DDEF1 | 8 | 131,415,248 |
| 2877 | DDEF1 | 8 | 131,418,292 | 2878 | DDEF1 | 8 | 131,423,273 |
| 2879 | DDEF1 | 8 | 131,436,663 | 2880 | DDEF1 | 8 | 131,465,743 |
| 2881 | DDEF1 | 8 | 131,469,150 | 2882 | DDEF1 | 8 | 131,480,431 |
| 2883 | DDEF1 | 8 | 131,490,170 | 2884 | ADCY8 | 8 | 131,862,883 |
| 2885 | ADCY8 | 8 | 131,872,404 | 2886 | ADCY8 | 8 | 131,876,740 |
| 2887 | ADCY8 | 8 | 131,930,184 | 2888 | ADCY8 | 8 | 131,958,850 |
| 2889 | ADCY8 | 8 | 131,961,234 | 2890 | ADCY8 | 8 | 131,972,316 |
| 2891 | ADCY8 | 8 | 131,983,404 | 2892 | ADCY8 | 8 | 131,985,500 |
| 2893 | ADCY8 | 8 | 131,997,583 | 2894 | ADCY8 | 8 | 132,016,683 |
| 2895 | ADCY8 | 8 | 132,056,557 | 2896 | ADCY8 | 8 | 132,057,791 |
| 2897 | ADCY8 | 8 | 132,070,010 | 2898 | ADCY8 | 8 | 132,071,996 |
| 2899 | ADCY8 | 8 | 132,151,934 | 2900 | KCNQ3 | 8 | 133,208,937 |
| 2901 | KCNQ3 | 8 | 133,575,550 | 2902 | ST3GAL1 | 8 | 134,608,818 |
| 2903 | ST3GAL1 | 8 | 134,609,061 | 2904 | ST3GAL1 | 8 | 134,610,724 |
| 2905 | ST3GAL1 | 8 | 134,664,388 | 2906 | ST3GAL1 | 8 | 134,664,414 |
| 2907 | COL22A1 | 8 | 139,668,834 | 2908 | COL22A1 | 8 | 139,744,740 |
| 2909 | COL22A1 | 8 | 139,763,602 | 2910 | COL22A1 | 8 | 139,770,391 |
| 2911 | COL22A1 | 8 | 139,777,590 | 2912 | COL22A1 | 8 | 139,834,014 |
| 2913 | COL22A1 | 8 | 139,871,695 | 2914 | COL22A1 | 8 | 139,901,545 |
| 2915 | COL22A1 | 8 | 139,976,026 | 2916 | COL22A1 | 8 | 139,980,721 |
| 2917 | COL22A1 | 8 | 139,992,260 | 2918 | COL22A1 | 8 | 139,992,787 |
| 2919 | COL22A1 | 8 | 139,994,229 | 2920 | ZC3H3 | 8 | 144,670,321 |
| 2921 | ZC3H3 | 8 | 144,684,823 | 2922 | SMARCA2 | 9 | 2,053,356 |
| 2923 | SMARCA2 | 9 | 2,083,551 | 2924 | SMARCA2 | 9 | 2,115,231 |
| 2925 | SMARCA2 | 9 | 2,120,828 | 2926 | SMARCA2 | 9 | 2,120,917 |
| 2927 | SMARCA2 | 9 | 2,123,189 | 2928 | SMARCA2 | 9 | 2,147,097 |
| 2929 | SMARCA2 | 9 | 2,163,296 | 2930 | SMARCA2 | 9 | 2,175,324 |
| 2931 | SMARCA2 | 9 | 2,177,750 | 2932 | SMARCA2 | 9 | 2,178,335 |
| 2933 | SMARCA2 | 9 | 2,178,383 | 2934 | SMARCA2 | 9 | 2,181,309 |
| 2935 | SLC1A1 | 9 | 4,550,348 | 2936 | SLC1A1 | 9 | 4,552,472 |
| 2937 | SLC1A1 | 9 | 4,567,130 | 2938 | SLC1A1 | 9 | 4,576,808 |
| 2939 | PTPRD | 9 | 8,283,622 | 2940 | PTPRD | 9 | 8,299,450 |
| 2941 | PTPRD | 9 | 8,365,585 | 2942 | PTPRD | 9 | 8,372,966 |
| 2943 | PTPRD | 9 | 8,389,766 | 2944 | PTPRD | 9 | 8,390,681 |
| 2945 | PTPRD | 9 | 8,393,640 | 2946 | PTPRD | 9 | 8,417,170 |
| 2947 | PTPRD | 9 | 8,418,856 | 2948 | PTPRD | 9 | 8,420,033 |
| 2949 | PTPRD | 9 | 8,420,742 | 2950 | PTPRD | 9 | 8,422,445 |
| 2951 | PTPRD | 9 | 8,423,160 | 2952 | PTPRD | 9 | 8,727,316 |
| 2953 | PTPRD | 9 | 8,728,267 | 2954 | PTPRD | 9 | 8,752,705 |
| 2955 | PTPRD | 9 | 8,764,062 | 2956 | PTPRD | 9 | 8,765,087 |
| 2957 | ADAMTSL1 | 9 | 18,714,225 | 2958 | ADAMTSL1 | 9 | 18,718,689 |
| 2959 | KIAA1797 | 9 | 20,678,117 | 2960 | KIAA1797 | 9 | 20,686,043 |
| 2961 | KIAA1797 | 9 | 20,732,210 | 2962 | KIAA1797 | 9 | 20,752,645 |
| 2963 | KIAA1797 | 9 | 20,754,870 | 2964 | KIAA1797 | 9 | 20,791,991 |
| 2965 | KIAA1797 | 9 | 20,794,446 | 2966 | KIAA1797 | 9 | 20,833,841 |
| 2967 | KIAA1797 | 9 | 20,872,525 | 2968 | KIAA1797 | 9 | 20,882,952 |
| 2969 | KIAA1797 | 9 | 20,886,938 | 2970 | KIAA1797 | 9 | 20,898,513 |
| 2971 | KIAA1797 | 9 | 20,914,474 | 2972 | KIAA1797 | 9 | 20,921,349 |
| 2973 | KIAA 1797 | 9 | 20,935,207 | 2974 | KIAA 1797 | 9 | 20,935,372 |
| 2975 | KIAA1797 | 9 | 20,943,335 | 2976 | KIAA1797 | 9 | 20,943,750 |
| 2977 | KIAA1797 | 9 | 20,951,906 | 2978 | KIAA1797 | 9 | 20,977,703 |
| 2979 | KIAA 1797 | 9 | 20,978,426 | 2980 | KIAA 1797 | 9 | 20,986,709 |
| 2981 | KIAA1797 | 9 | 20,989,770 | 2982 | IFT74 | 9 | 26,999,100 |
| 2983 | IFT74 | 9 | 27,052,721 | 2984 | IFT74 | 9 | 27,056,070 |
| 2985 | IFT74 | 9 | 27,063,650 | 2986 | TEK | 9 | 27,129,817 |
| 2987 | TEK | 9 | 27,142,292 | 2988 | TEK | 9 | 27,142,308 |
| 2989 | TEK | 9 | 27,144,617 | 2990 | TEK | 9 | 27,175,101 |
| 2991 | PIP5K1B | 9 | 70,499,527 | 2992 | PIP5K1B | 9 | 70,515,001 |
| 2993 | PIP5K1B | 9 | 70,515,728 | 2994 | PIP5K1B | 9 | 70,520,963 |
| '2995 | PIP5K1B | 9 | 70,545,037 | 2996 | PIP5K1B | 9 | 70,670,116 |
| 2997 | PIP5K1B | 9 | 70,703,782 | 2998 | PIP5K1B | 9 | 70,811,920 |
| 2999 | PIP5K1B | 9 | 70,814,515 | 3000 | APBA1 | 9 | 71,276,235 |
| 3001 | APBA1 | 9 | 71,280,626 | 3002 | APBA1 | 9 | 71,298,607 |
| 3003 | APBA1 | 9 | 71,324,247 | 3004 | APBA1 | 9 | 71,327,356 |
| 3005 | APBA1 | 9 | 71,332,520 | 3006 | ALPHA1 | 9 | 71,343,317 |
| 3007 | APBA1 | 9 | 71,415,835 | 3008 | MAMDC2 | 9 | 71,887,859 |
| 3009 | MAMDC2 | 9 | 71,892,836 | 3010 | MAMDC2 | 9 | 71,896,167 |
| 3011 | MAMDC2 | 9 | 71,913,927 | 3012 | MAMDC2 | 9 | 71,930,214 |
| 3013 | MAMDC2 | 9 | 71,979,450 | 3014 | MAMDC2 | 9 | 71,991,846 |
| 3015 | MAMDC2 | 9 | 71,998,663 | 3016 | MAMDC2 | 9 | 72,025,025 |
| 3017 | MAMDC2 | 9 | 72,051,438 | 3018 | MAMDC2 | 9 | 72,051,513 |
| 3019 | MAMDC2 | 9 | 72,053,813 | 3020 | TRPM3 | 9 | 72,346,211 |
| 3021 | TRPM3 | 9 | 72,383,248 | 3022 | TRPM3 | 9 | 72,384,667 |
| 3023 | TRPM3 | 9 | 72,616,673 | 3024 | TRPM3 | 9 | 72,617,325 |
| 3025 | TRPM3 | 9 | 72,673,741 | 3026 | TRPM3 | 9 | 72,946,351 |
| 3027 | TMC1 | 9 | 74,339,851 | 3028 | TMC1 | 9 | 74,345,877 |
| 3029 | TMC1 | 9 | 74,363,736 | 3030 | TMC1 | 9 | 74,379,155 |
| 3031 | TMC1 | 9 | 74,383,637 | 3032 | TMC1 | 9 | 74,415,525 |
| 3033 | TMC1 | 9 | 74,434,104 | 3034 | TMC1 | 9 | 74,470,431 |
| 3035 | TMC1 | 9 | 74,485,413 | 3036 | TMC1 | 9 | 74,507,002 |
| 3037 | TMC1 | 9 | 74,507,694 | 3038 | TMC1 | 9 | 74,537,387 |
| 3039 | TMC1 | 9 | 74,555,746 | 3040 | TMC1 | 9 | 74,558,826 |
| 3041 | TMC1 | 9 | 74,574,594 | 3042 | TMC1 | 9 | 74,579,921 |
| 3043 | TMC1 | 9 | 74,701,976 | 3044 | TRPM6 | 9 | 76,580,880 |
| 3045 | TRPM6 | 9 | 76,604,375 | 3046 | TRPM6 | 9 | 76,605,104 |
| 3047 | TRPM6 | 9 | 76,616,041 | 3048 | TRPM6 | 9 | 76,616,993 |
| 3049 | TRPM6 | 9 | 76,617,856 | 3050 | PCSK5 | 9 | 77,687,086 |
| 3051 | PCSK5 | 9 | 77,798,692 | 3052 | PCSK5 | 9 | 77,810,526 |
| 3053 | PCSK5 | 9 | 77,824,856 | 3054 | PCSK5 | 9 | 77,896,771 |
| 3055 | PCSK5 | 9 | 77,897,682 | 3056 | PCSK5 | 9 | 77,915,932 |
| 3057 | PCSK5 | 9 | 77,932,517 | 3058 | PCSK5 | 9 | 77,974,918 |
| 3059 | GNAQ | 9 | 79,595,252 | 3060 | GNAQ | 9 | 79,600,333 |
| 3061 | GNAQ | 9 | 79,659,567 | 3062 | GNAQ | 9 | 79,734,072 |
| 3063 | GNAQ | 9 | 79,809,631 | 3064 | GNAQ | 9 | 79,812,915 |
| 3065 | GNAQ | 9 | 79,845,641 | 3066 | GNAQ | 9 | 79,856,574 |
| 3067 | GNAQ | 9 | 79,868,777 | 3068 | GNAQ | 9 | 79,874,660 |
| 3069 | GNAQ | 9 | 79,908,815 | 3070 | GNAQ | 9 | 79,946,188 |
| 3071 | NTRK2 | 9 | 86,464,568 | 3072 | NTRK2 | 9 | 86,472,347 |
| 3073 | NTRK2 | 9 | 86,479,100 | 3074 | NTRK2 | 9 | 86,571,296 |
| 3075 | NTRK2 | 9 | 86,577,727 | 3076 | NTRK2 | 9 | 86,590,037 |
| 3077 | NTRK2 | 9 | 86,598,770 | 3078 | NTRK2 | 9 | 86,598,798 |
| 3079 | NTRK2 | 9 | 86,599,187 | 3080 | NTRK2 | 9 | 86,627,979 |
| 3081 | DAPK1 | 9 | 89,356,618 | 3082 | DAPK1 | 9 | 89,446,457 |
| 3083 | DAPK1 | 9 | 89,510,774 | 3084 | DAPK1 | 9 | 89,516,915 |
| 3085 | SHC3 | 9 | 90,829,608 | 3086 | DIRAS2 | 9 | 92,422,258 |
| 3087 | DIRAS2 | 9 | 92,426,243 | 3088 | DIRAS2 | 9 | 92,432,978 |
| 3089 | NFIL3 | 9 | 93,218,808 | 3090 | NFIL3 | 9 | 93,220,448 |
| 3091 | NFIL3 | 9 | 93,276,485 | 3092 | NFIL3 | 9 | 93,283,388 |
| 3093 | NFIL3 | 9 | 93,286,276 | 3094 | NFIL3 | 9 | 93,286,497 |
| 3095 | NFIL3 | 9 | 93,286,593 | 3096 | NFIL3 | 9 | 93,286,700 |
| 3097 | ZNF169 | 9 | 96,102,802 | 3098 | ZNF169 | 9 | 96,255,984 |
| 3099 | H1ATL1 | 9 | 96,261,255 | 3100 | H1ATL1 | 9 | 96,268,739 |
| 3101 | GABBR2 | 9 | 100,095,943 | 3102 | GABBR2 | 9 | 100,110,308 |
| 3103 | GABBR2 | 9 | 100,111,343 | 3104 | GABBR2 | 9 | 100,114,693 |
| 3105 | GABBR2 | 9 | 100,116,448 | 3106 | GABBR2 | 9 | 100,118,041 |
| 3107 | GABBR2 | 9 | 100,429,660 | 3108 | RP11-35N6.1 | 9 | 102,447,217 |
| 3109 | RP11-35N6.1 | 9 | 102,472,272 | 3110 | GRIN3A | 9 | 103,636,342 |
| 3111 | FKTN | 9 | 107,402,666 | 3112 | FKTN | 9 | 107,449,221 |
| 3113 | FKTN | 9 | 107,449,435 | 3114 | FKTN | 9 | 107,449,527 |
| 3115 | FKTN | 9 | 107,455,377 | 3116 | PALM2 | 9 | 111,500,859 |
| 3117 | PALM2 | 9 | 111,531,505 | 3118 | PALM2 | 9 | 111,545,842 |
| 3119 | PALM2 | 9 | 111,552,566 | 3120 | PALM2 | 9 | 111,555,049 |
| 3121 | PALM2 | 9 | 111,985,226 | 3122 | PALM2 | 9 | 111,985,595 |
| 3123 | SVEP1 | 9 | 112,145,525 | 3124 | SVEP1 | 9 | 112,152,393 |
| 3125 | SVEP1 | 9 | 112,154,879 | 3126 | SVEP1 | 9 | 112,166,117 |
| 3127 | SVEP1 | 9 | 112,167,878 | 3128 | SVEP1 | 9 | 112,395,423 |
| 3129 | ZNF618 | 9 | 115,804,104 | 3130 | ZNF618 | 9 | 115,820,286 |
| 3131 | ZNF618 | 9 | 115,839,747 | 3132 | ZNF618 | 9 | 115,840,427 |
| 3133 | ZNF618 | 9 | 115,848,968 | 3134 | ZNF618 | 9 | 115,862,471 |
| 3135 | CDK5RAP2 | 9 | 122,190,274 | 3136 | CDK5RAP2 | 9 | 122,245,733 |
| 3137 | CDK5RAP2 | 9 | 122,247,523 | 3138 | CDK5RAP2 | 9 | 122,341,167 |
| 3139 | CDK5RAP2 | 9 | 122,362,134 | 3140 | CDK5RAP2 | 9 | 122,370,634 |
| 3141 | RAB14 | 9 | 123,011,433 | 3142 | STOM | 9 | 123,210,311 |
| 3143 | DAB21P | 9 | 123,691,611 | 3144 | TTLL11 | 9 | 123,780,890 |
| 3145 | TTL11 | 9 | 123,816,925 | 3146 | TTLL11 | 9 | 123,894,371 |
| 3147 | TTLL11 | 9 | 123,904,206 | 3148 | TTLL11 | 9 | 123,908,883 |
| 3149 | LHX6 | 9 | 124,033,326 | 3150 | PTGS1 | 9 | 124,226,030 |
| 3151 | RALGPS1 | 9 | 128,740,226 | 3152 | RALGPS1 | 9 | 128,789,924 |
| 3153 | RALGPS1 | 9 | 128,809,594 | 3154 | RALGPS1 | 9 | 128,851,222 |
| 3155 | RALGPS1 | 9 | 128,902,817 | 3156 | RALGPS1 | 9 | 128,946,477 |
| 3157 | RALGPS1 | 9 | 128,955,452 | 3158 | RALGPS1 | 9 | 128,972,837 |
| 3159 | RALGPS1 | 9 | 128,973,145 | 3160 | RALGPS1 | 9 | 129,028,297 |
| 3161 | SLC25A25 | 9 | 129,895,358 | 3162 | SLC25A25 | 9 | 129,899,153 |
| 3163 | FREQ | 9 | 131,997,035 | 3164 | EXOSC2 | 9 | 132,557,580 |
| 3165 | ABL1 | 9 | 132,607,062 | 3166 | EXOSC2 | 9 | 132,620,443 |
| 3167 | ABL1 | 9 | 132,698,182 | 3168 | ABL1 | 9 | 132,722,573 |
| 3169 | ABL1 | 9 | 132,723,896 | 3170 | ABL1 | 9 | 132,729,556 |
| 3171 | ABL1 | 9 | 132,743,853 | 3172 | ABL1 | 9 | 132,757,571 |
| 3173 | NTNG2 | 9 | 134,032,706 | 3174 | NTNG2 | 9 | 134,048,486 |
| 3175 | NTNG2 | 9 | 134,061,758 | 3176 | NTNG2 | 9 | 134,063,788 |
| 3177 | NTNG2 | 9 | 134,063,884 | 3178 | NTNG2 | 9 | 134,072,800 |
| 3179 | TSC1 | 9 | 134,776,725 | 3180 | TSC1 | 9 | 134,786,003 |
| 3181 | TSC1 | 9 | 134,811,193 | 3182 | TSC1 | 9 | 134,812,111 |
| 3183 | VAV2 | 9 | 135,652,749 | 3184 | VAV2 | 9 | 135,708,866 |
| 3185 | VAV2 | 9 | 135,712,953 | 3186 | VAV2 | 9 | 135,714,835 |
| 3187 | VAV2 | 9 | 135,725,041 | 3188 | VAV2 | 9 | 135,735,578 |
| 3189 | VAV2 | 9 | 135,750,672 | 3190 | VAV2 | 9 | 135,797,591 |
| 3191 | VAV2 | 9 | 135,800,678 | 3192 | VAV2 | 9 | 135,816,278 |
| 3193 | OLFM1 | 9 | 137,163,836 | 3194 | OLFM1 | 9 | 137,166,820 |
| 3195 | KCNT1 | 9 | 137,739,699 | 3196 | KCNT1 | 9 | 137,760,712 |
| 3197 | KCNT1 | 9 | 137,761,268 | 3198 | KCNT1 | 9 | 137,771,812 |
| 3199 | KCNT1 | 9 | 137,780,294 | 3200 | NOTCH1 | 9 | 138,588,006 |
| 3201 | ABCA2 | 9 | 139,049,256 | 3202 | NPDC1 | 9 | 139,079,674 |
| 3203 | GRIN1 | 9 | 139,123,742 | 3204 | PNPLA7 | 9 | 139,552,139 |
| 3205 | CACNA1B | 9 | 139,875,052 | 3206 | CACNA1B | 9 | 140,019,421 |
| 3207 | CACNA1B | 9 | 140,019,503 | 3208 | CACNA1B | 9 | 140,029,466 |
| 3209 | CACNA1B | 9 | 140,075,368 | 3210 | CACNA1B | 9 | 140,078,171 |
| 3211 | CACNA1B | 9 | 140,089,212 | 3212 | CACNA1B | 9 | 140,094,586 |
| 3213 | CACNA1B | 9 | 140,121,687 | 3214 | PITRM1 | 10 | 3,173,747 |
| 3215 | PITRM1 | 10 | 3,179,380 | 3216 | PITRM1 | 10 | 3,181,527 |
| 3217 | PITRM1 | 10 | 3,192,065 | 3218 | PITRM1 | 10 | 3,196,365 |
| 3219 | PITRM1 | 10 | 3,197,632 | 3220 | PITRM1 | 10 | 3,198,140 |
| 3221 | PITRM1 | 10 | 3,198,512 | 3222 | PITRM1 | 10 | 3,209,794 |
| 3223 | PITRM1 | 10 | 3,213,179 | 3224 | PITRM1 | 10 | 3,782,844 |
| 3225 | PITRM1 | 10 | 3,786,569 | 3226 | PRKCQ | 10 | 6,508,611 |
| 3227 | PRKCQ | 10 | 6,509,161 | 3228 | PRKCQ | 10 | 6,509,823 |
| 3229 | PRKCQ | 10 | 6,512,253 | 3230 | PRKCQ | 10 | 7,151,877 |
| 3231 | CUGBP2 | 10 | 11,261,372 | 3232 | CUGBP2 | 10 | 11,397,516 |
| 3233 | CACNB2 | 10 | 18,499,288 | 3234 | CACNB2 | 10 | 18,693,867 |
| 3235 | CACNB2 | 10 | 18,737,688 | 3236 | CACNB2 | 10 | 18,751,294 |
| 3237 | CACNB2 | 10 | 18,756,352 | 3238 | CACNB2 | 10 | 18,767,907 |
| 3239 | CACNB2 | 10 | 18,781,800 | 3240 | CACNB2 | 10 | 18,784,954 |
| 3241 | CACNB2 | 10 | 18,850,680 | 3242 | CACNB2 | 10 | 18,875,360 |
| 3243 | ARMC3 | 10 | 23,192,732 | 3244 | ARMC3 | 10 | 23,204,056 |
| 3245 | ARMC3 | 10 | 23,243,088 | 3246 | ARMC3 | 10 | 23,249,050 |
| 3247 | ARMC3 | 10 | 23,264,868 | 3248 | ARMC3 | 10 | 23,277,574 |
| 3249 | ARMC3 | 10 | 23,289,115 | 3250 | ARMC3 | 10 | 23,318,053 |
| 3251 | ARMC3 | 10 | 23,337,258 | 3252 | ARMC3 | 10 | 23,343,422 |
| 3253 | ARHGAP21 | 10 | 24,973,706 | 3254 | ARHGAP21 | 10 | 25,007,098 |
| 3255 | ARHGAP21 | 10 | 25,017,790 | 3256 | ARHGAP21 | 10 | 25,037,439 |
| 3257 | ARHGAP21 | 10 | 25,046,553 | 3258 | ARHGAP21 | 10 | 25,121,861 |
| 3259 | MYO3A | 10 | 26,224,703 | 3260 | MY03A | 10 | 26,227,755 |
| 3261 | MY03A | 10 | 26,246,862 | 3262 | MYO3A | 10 | 26,247,055 |
| 3263 | MY03A | 10 | 26,247,564 | 3264 | MY03A | 10 | 26,252,100 |
| 3265 | MYO3A | 10 | 26,265,542 | 3266 | MYO3A | 10 | 26,266,216 |
| 3267 | MY03A | 10 | 26,326,995 | 3268 | MYO3A | 10 | 26,344,078 |
| 3269 | MY03A | 10 | 26,350,685 | 3270 | MYO3A | 10 | 26,352,830 |
| 3271 | MYO3A | 10 | 26,355,103 | 3272 | MYO3A | 10 | 26,385,171 |
| 3273 | MYO3A | 10 | 26,389,693 | 3274 | MYO3A | 10 | 26,396,941 |
| 3275 | MYO3A | 10 | 26,398,640 | 3276 | MYO3A | 10 | 26,443,297 |
| 3277 | MY03A | 10 | 26,473,372 | 3278 | MYO3A | 10 | 26,486,318 |
| 3279 | MY03A | 10 | 26,499,719 | 3280 | MYO3A | 10 | 26,503,136 |
| 3281 | MY03A | 10 | 26,503,593 | 3282 | MYO3A | 10 | 26,511,966 |
| 3283 | MYO3A | 10 | 26,523,271 | 3284 | MYO3A | 10 | 26,523,510 |
| 3285 | MYO3A | 10 | 26,524,160 | 3286 | MYO3A | 10 | 26,526,934 |
| 3287 | MYO3A | 10 | 26,552,050 | 3288 | MYO3A | 10 | 26,677,147 |
| 3289 | MYO3A | 10 | 26,679,306 | 3290 | SLC16A9 | 10 | 61,084,017 |
| 3291 | SLC16A9 | 10 | 61,120,938 | 3292 | SLC16A9 | 10 | 61,122,758 |
| 3293 | SLC16A9 | 10 | 61,177,020 | 3294 | SLC16A9 | 10 | 61,178,790 |
| 3295 | SLC16A9 | 10 | 61,187,929 | 3296 | JMJD1C | 10 | 64,732,014 |
| 3297 | JMJD1C | 10 | 64,761,165 | 3298 | JMJD1C | 10 | 64,771,213 |
| 3299 | JMJD1C | 10 | 64,778,162 | 3300 | JMJD1C | 10 | 64,791,571 |
| 3301 | JMJD1C | 10 | 64,792,793 | 3302 | JMJD1C | 10 | 64,873,814 |
| 3303 | JMJD1C | 10 | 64,917,025 | 3304 | CDH23 | 10 | 72,828,713 |
| 3305 | CDH23 | 10 | 72,997,429 | 3306 | CDH23 | 10 | 73,084,016 |
| 3307 | CDH23 | 10 | 73,104,912 | 3308 | CDH23 | 10 | 73,171,562 |
| 3309 | CDH23 | 10 | 73,192,700 | 3310 | CDH23 | 10 | 73,209,936 |
| 3311 | CDH23 | 10 | 73,211,015 | 3312 | CDH23 | 10 | 73,214,092 |
| 3313 | CDH23 | 10 | 73,228,958 | 3314 | CDH23 | 10 | 73,236,209 |
| 3315 | CDH23 | 10 | 73,251,566 | 3316 | KCNMA1 | 10 | 78,240,799 |
| 3317 | KCNMA1 | 10 | 78,297,136 | 3318 | KCNMA1 | 10 | 78,300,503 |
| 3319 | KCNMA1 | 10 | 78,319,405 | 3320 | KCNMA1 | 10 | 78,324,802 |
| 3321 | KCNMA1 | 10 | 78,337,197 | 3322 | KCNMA1 | 10 | 78,348,173 |
| 3323 | KCNMA1 | 10 | 78,426,444 | 3324 | KCNMA1 | 10 | 78,426,609 |
| 3325 | KCNMA1 | 10 | 78,446,565 | 3326 | KCNMA1 | 10 | 78,616,033 |
| 3327 | KCNMA1 | 10 | 78,626,486 | 3328 | KCNMA1 | 10 | 78,738,696 |
| 3329 | KCNMA1 | 10 | 78,738,970 | 3330 | KCNMA1 | 10 | 78,756,964 |
| 3331 | KCNMA1 | 10 | 78,757,037 | 3332 | KCNMA1 | 10 | 78,794,600 |
| 3333 | KCNMA1 | 10 | 79,033,394 | 3334 | KCNMA1 | 10 | 79,073,940 |
| 3335 | KCNMA1 | 10 | 79,094,637 | 3336 | KCNMA1 | 10 | 79,162,833 |
| 3337 | KCNMA1 | 10 | 79,168,796 | 3338 | KCNMA1 | 10 | 79,176,445 |
| 3339 | NRG3 | 10 | 84,078,553 | 3340 | NRG3 | 10 | 84,136,852 |
| 3341 | NRG3 | 10 | 84,197,554 | 3342 | NRG3 | 10 | 84,479,001 |
| 3343 | NRG3 | 10 | 84,479,024 | 3344 | NRG3 | 10 | 84,487,647 |
| 3345 | NRG3 | 10 | 84,604,364 | 3346 | NRG3 | 10 | 84,613,706 |
| 3347 | NRG3 | 10 | 84,616,939 | 3348 | NRG3 | 10 | 84,679,040 |
| 3349 | NRG3 | 10 | 84,680,434 | 3350 | NRG3 | 10 | 84,681,866 |
| 3351 | NRG3 | 10 | 84,683,470 | 3352 | NRG3 | 10 | 84,688,185 |
| 3353 | NRG3 | 10 | 84,820,632 | 3354 | SORBS1 | 10 | 97,064,414 |
| 3355 | SORBS1 | 10 | 97,075,121 | 3356 | SORBS1 | 10 | 97,076,023 |
| 3357 | SORBS1 | 10 | 97,095,565 | 3358 | SORBS1 | 10 | 97,097,088 |
| 3359 | SORBS1 | 10 | 97,109,583 | 3360 | SORBS1 | 10 | 97,112,231 |
| 3361 | SORBS1 | 10 | 97,112,420 | 3362 | SORBS1 | 10 | 97,122,546 |
| 3363 | SORBS1 | 10 | 97,122,783 | 3364 | SORBS1 | 10 | 97,150,503 |
| 3365 | SORBS1 | 10 | 97,200,805 | 3366 | SORBS1 | 10 | 97,204,408 |
| 3367 | SORBS1 | 10 | 97,216,419 | 3368 | SORBS1 | 10 | 97,216,500 |
| 3369 | SORBS1 | 10 | 97,329,428 | 3370 | SORCS3 | 10 | 106,367,232 |
| 3371 | SORCS3 | 10 | 106,371,034 | 3372 | SORCS3 | 10 | 106,374,605 |
| 3373 | SORCS3 | 10 | 106,590,333 | 3374 | SORCS3 | 10 | 106,614,338 |
| 3375 | SORCS3 | 10 | 106,636,964 | 3376 | SORCS3 | 10 | 106,646,127 |
| 3377 | SORCS3 | 10 | 106,664,021 | 3378 | SORCS3 | 10 | 106,684,869 |
| 3379 | SORCS3 | 10 | 106,684,970 | 3380 | SORCS3 | 10 | 106,689,202 |
| 3381 | SORCS3 | 10 | 106,701,972 | 3382 | SORCS3 | 10 | 106,703,623 |
| 3383 | SORCS3 | 10 | 106,784,111 | 3384 | SORCS3 | 10 | 106,897,228 |
| 3385 | SORCS3 | 10 | 106,906,037 | 3386 | SORCS3 | 10 | 106,906,641 |
| 3387 | SORCS3 | 10 | 106,935,226 | 3388 | SORCS3 | 10 | 107,025,958 |
| 3389 | VTI1A | 10 | 114,212,444 | 3390 | VTI1A | 10 | 114,214,053 |
| 3391 | VTI1A | 10 | 114,214,492 | 3392 | VTI1A | 10 | 114,218,153 |
| 3393 | VTI1A | 10 | 114,226,855 | 3394 | VTI1A | 10 | 114,228,361 |
| 3395 | VTI1A | 10 | 114,404,784 | 3396 | VTI1A | 10 | 114,424,611 |
| 3397 | VTI1A | 10 | 114,426,056 | 3398 | VTI1A | 10 | 114,454,027 |
| 3399 | VTI1A | 10 | 114,465,945 | 3400 | VTI1A | 10 | 114,469,252 |
| 3401 | VTI1A | 10 | 114,471,012 | 3402 | VTI1A | 10 | 114,473,068 |
| 3403 | VTI1A | 10 | 114,489,994 | 3404 | VTI1A | 10 | 114,506,363 |
| 3405 | VTI1A | 10 | 114,508,308 | 3406 | ATRNL1 | 10 | 116,818,582 |
| 3407 | ATRNL1 | 10 | 116,818,761 | 3408 | ATRNL1 | 10 | 116,826,992 |
| 3409 | ATRNL1 | 10 | 117,051,783 | 3410 | ATRNL1 | 10 | 117,186,878 |
| 3411 | ATRNL1 | 10 | 117,208,818 | 3412 | ATRNL1 | 10 | 117,226,168 |
| 3413 | ATRNL1 | 10 | 117,257,300 | 3414 | ATRNL1 | 10 | 117,342,514 |
| 3415 | ATRNL1 | 10 | 117,348,932 | 3416 | ATRNL1 | 10 | 117,350,294 |
| 3417 | ATRNL1 | 10 | 117,404,353 | 3418 | ATRNL1 | 10 | 117,429,854 |
| 3419 | ATRNL1 | 10 | 117,485,967 | 3420 | ATRNL1 | 10 | 117,697,723 |
| 3421 | ATRNL1 | 10 | 117,743,056 | 3422 | ATRNL1 | 10 | 117,761,145 |
| 3423 | HSPA12A | 10 | 118,432,901 | 3424 | HSPA12A | 10 | 118,434,753 |
| 3425 | HSPA12A | 10 | 118,438,068 | 3426 | HSPA12A | 10 | 118,446,140 |
| 3427 | HSPA12A | 10 | 118,463,943 | 3428 | HSPA12A | 10 | 118,527,758 |
| 3429 | GRK5 | 10 | 120,961,454 | 3430 | GRK5 | 10 | 121,055,451 |
| 3431 | ATE1 | 10 | 123,426,686 | 3432 | ATE1 | 10 | 123,427,443 |
| 3433 | ATE1 | 10 | 123,432,831 | 3434 | ATE1 | 10 | 123,440,223 |
| 3435 | ATE1 | 10 | 123,450,896 | 3436 | ATE1 | 10 | 123,491,722 |
| 3437 | ATE1 | 10 | 123,491,947 | 3438 | ATE1 | 10 | 123,521,812 |
| 3439 | ATE1 | 10 | 123,529,351 | 3440 | ATE1 | 10 | 123,529,439 |
| 3441 | ATE1 | 10 | 123,531,179 | 3442 | ATE1 | 10 | 123,531,856 |
| 3443 | ATE1 | 10 | 123,539,198 | 3444 | ATE1 | 10 | 123,546,416 |
| 3445 | ATE1 | 10 | 123,604,845 | 3446 | ATE1 | 10 | 123,644,015 |
| 3447 | ATE | 10 | 123,648,608 | 3448 | ATE1 | 10 | 123,651,712 |
| 3449 | ATE1 | 10 | 123,663,196 | 3450 | ATE1 | 10 | 123,666,737 |
| 3451 | ATE1 | 10 | 123,669,674 | 3452 | ATE1 | 10 | 123,697,603 |
| 3453 | CTBP2 | 10 | 126,677,518 | 3454 | CTBP2 | 10 | 127,123,254 |
| 3455 | CTBP2 | 10 | 127,125,130 | 3456 | CTBP2 | 10 | 127,127,898 |
| 3457 | STIM1 | 11 | 3,847,536 | 3458 | STIM1 | 11 | 3,867,621 |
| 3459 | STIM1 | 11 | 3,915,404 | 3460 | STIM1 | 11 | 3,927,655 |
| 3461 | STIM1 | 11 | 3,931,164 | 3462 | STIM1 | 11 | 3,949,530 |
| 3463 | STIM1 | 11 | 3,949,807 | 3464 | STIM1 | 11 | 3,960,254 |
| 3465 | STIM1 | 11 | 4,033,624 | 3466 | STIM1 | 11 | 4,042,105 |
| 3467 | STIM1 | 11 | 4,085,062 | 3468 | GALNTL4 | 11 | 11,222,747 |
| 3469 | GALNTL4 | 11 | 11,228,193 | 3470 | GALNTL4 | 11 | 11,246,167 |
| 3471 | GALNTL4 | 11 | 11,497,136 | 3472 | MICAL2 | 11 | 12,084,055 |
| 3473 | MICAL2 | 11 | 12,092,180 | 3474 | MICAL2 | 11 | 12,139,036 |
| 3475 | MICAL2 | 11 | 12,142,872 | 3476 | MICAL2 | 11 | 12,150,320 |
| 3477 | MICAL2 | 11 | 12,156,032 | 3478 | MICAL2 | 11 | 12,175,779 |
| 3479 | MICAL2 | 11 | 12,191,280 | 3480 | MICAL2 | 11 | 12,212,874 |
| 3481 | MICAL2 | 11 | 12,222,118 | 3482 | ARNTL | 11 | 13,212,114 |
| 3483 | ARNTL | 11 | 13,212,547 | 3484 | ARNTL | 11 | 13,222,791 |
| 3485 | ARNTL | 11 | 13,231,816 | 3486 | ARNTL | 11 | 13,234,603 |
| 3487 | ARNTL | 11 | 13,249,824 | 3488 | ARNTL | 11 | 13,250,844 |
| 3489 | ARNTL | 11 | 13,262,887 | 3490 | ARNTL | 11 | 13,267,430 |
| 3491 | ARNTL | 11 | 13,274,540 | 3492 | ARNTL | 11 | 13,277,919 |
| 3493 | ARNTL | 11 | 13,302,169 | 3494 | ARNTL | 11 | 13,366,374 |
| 3495 | ARNTL | 11 | 13,366,419 | 3496 | SPON1 | 11 | 13,877,677 |
| 3497 | SPON1 | 11 | 13,922,920 | 3498 | SPON1 | 11 | 13,990,100 |
| 3499 | SPON1 | 11 | 13,994,054 | 3500 | SPON1 | 11 | 14,004,879 |
| 3501 | SPON1 | 11 | 14,005,230 | 3502 | SPON1 | 11 | 14,005,260 |
| 3503 | SPON1 | 11 | 14,008,775 | 3504 | SPON1 | 11 | 14,014,762 |
| 3505 | SPON1 | 11 | 14,014,854 | 3506 | SPON1 | 11 | 14,023,125 |
| 3507 | SPON1 | 11 | 14,034,087 | 3508 | SPON1 | 11 | 14,058,326 |
| 3509 | SPON1 | 11 | 14,070,335 | 3510 | SPON1 | 11 | 14,075,677 |
| 3511 | SPON1 | 11 | 14,115,440 | 3512 | SPON1 | 11 | 14,169,786 |
| 3513 | SPON1 | 11 | 14,215,231 | 3514 | SPON1 | 11 | 14,248,953 |
| 3515 | IGSF22 | 11 | 18,646,965 | 3516 | NELL1 | 11 | 20,753,829 |
| 3517 | KCNA4 | 11 | 30,011,330 | 3518 | KCNA4 | 11 | 30,174,079 |
| 3519 | C11ORF49 | 11 | 47,114,253 | 3520 | C11ORF49 | 11 | 47,130,872 |
| 3521 | C11ORF49 | 11 | 47,132,581 | 3522 | C11ORF49 | 11 | 47,144,987 |
| 3523 | 81.99 mb | 11 | 8,921,684 | 3524 | 81.99 mb | 11 | 81,928,698 |
| 3525 | 81.99 mb | 11 | 81,935,230 | 3526 | DLG2 | 11 | 82,720,706 |
| 3527 | DLG2 | 11 | 82,925,435 | 3528 | DLG2 | 11 | 83,018,964 |
| 3529 | DLG2 | 11 | 83,021,566 | 3530 | DLG2 | 11 | 83,036,277 |
| 3531 | DLG2 | 11 | 83,055,612 | 3532 | DLG2 | 11 | 83,083,527 |
| 3533 | DLG2 | 11 | 83,086,000 | 3534 | DLG2 | 11 | 83,099,929 |
| 3535 | DLG2 | 11 | 83,189,802 | 3536 | DLG2 | 11 | 83,212,271 |
| 3537 | DLG2 | 11 | 83,418,483 | 3538 | DLG2 | 11 | 83,452,078 |
| 3539 | DLG2 | 11 | 83,517,920 | 3540 | DLG2 | 11 | 83,609,529 |
| 3541 | DLG2 | 11 | 84,136,207 | 3542 | DLG2 | 11 | 84,140,727 |
| 3543 | DLG2 | 11 | 84,147,518 | 3544 | DLG2 | 11 | 84,170,883 |
| 3545 | DLG2 | 11 | 84,171,952 | 3546 | DLG2 | 11 | 84,214,566 |
| 3547 | DLG2 | 11 | 84,251,688 | 3548 | DLG2 | 11 | 84,251,805 |
| 3549 | DLG2 | 11 | 84,273,766 | 3550 | DLG2 | 11 | 84,283,321 |
| 3551 | DLG2 | 11 | 84,283,477 | 3552 | DLG2 | 11 | 84,340,897 |
| 3553 | DLG2 | 11 | 84,399,900 | 3554 | DLG2 | 11 | 84,425,370 |
| 3555 | DLG2 | 11 | 84,428,334 | 3556 | ELMOD1 | 11 | 106,964,269 |
| 3557 | ELMOD1 | 11 | 107,036,969 | 3558 | ELMOD1 | 11 | 107,051,899 |
| 3559 | ELMOD1 | 11 | 107,077,060 | 3560 | OPCML | 11 | 131,718,194 |
| 3561 | OPCML | 11 | 131,734,280 | 3562 | OPCML | 11 | 131,771,635 |
| 3563 | OPCML | 11 | 131,776,950 | 3564 | OPCML | 11 | 131,778,201 |
| 3565 | OPCML | 11 | 131,792,538 | 3566 | OPCML | 11 | 131,819,986 |
| 3567 | OPCML | 11 | 132,037,815 | 3568 | OPCML | 11 | 132,058,790 |
| 3569 | OPCML | 11 | 132,071,379 | 3570 | OPCML | 11 | 132,086,652 |
| 3571 | OPCML | 11 | 132,087,137 | 3572 | OPCML | 11 | 132,639,204 |
| 3573 | OPCML | 11 | 132,649,186 | 3574 | OPCML | 11 | 132,665,988 |
| 3575 | OPCML | 11 | 132,803,817 | 3576 | OPCML | 11 | 132,813,942 |
| 3577 | OPCML | 11 | 132,820,428 | 3578 | OPCML | 11 | 132,820,536 |
| 3579 | OPCML | 11 | 132,820,701 | 3580 | OPCML | 11 | 132,821,079 |
| 3581 | OPCML | 11 | 132,856,014 | 3582 | OPCML | 11 | 132,868,027 |
| 3583 | OPCML | 11 | 132,873,386 | 3584 | OPCML | 11 | 132,874,542 |
| 3585 | OPCML | 11 | 132,884,694 | 3586 | OPCML | 11 | 132,895,402 |
| 3587 | OPCML | 11 | 132,895,679 | 3588 | OPCML | 11 | 132,895,944 |
| 3589 | OPCML | 11 | 132,897,912 | 3590 | WNT5B | 12 | 1,617,734 |
| 3591 | TSPAN9 | 12 | 3,065,463 | 3592 | TSPAN9 | 12 | 3,216,860 |
| 3593 | TSPAN9 | 12 | 3,258,265 | 3594 | TSPAN9 | 12 | 3,310,366 |
| 3595 | TSPAN9 | 12 | 3,331,863 | 3596 | TMEM16B | 12 | 5,505,585 |
| 3597 | TMEM16B | 12 | 5,507,133 | 3598 | TMEM16B | 12 | 5,694,683 |
| 3599 | TMEM16B | 12 | 5,697,894 | 3600 | TMEM16B | 12 | 5,704,425 |
| 3601 | TMEM16B | 12 | 5,711,994 | 3602 | TMEM16B | 12 | 5,717,063 |
| 3603 | TMEM16B | 12 | 5,793,894 | 3604 | TMEM16B | 12 | 5,870,387 |
| 3605 | TMEM16B | 12 | 5,892,756 | 3606 | TMEM16B | 12 | 5,894,743 |
| 3607 | TMEM16B | 12 | 5,900,666 | 3608 | STYK1 | 12 | 10,665,965 |
| 3609 | STYK1 | 12 | 10,666,462 | 3610 | STYK1 | 12 | 10,667,780 |
| 3611 | STYK1 | 12 | 10,673,382 | 3612 | STYK1 | 12 | 10,676,279 |
| 3613 | STYK1 | 12 | 10,696,179 | 3614 | STYK1 | 12 | 10,697,767 |
| 3615 | STYK1 | 12 | 10,708,924 | 3616 | STYK1 | 12 | 10,711,189 |
| 3617 | LOC729025 | 12 | 16,235,442 | 3618 | LOC729025 | 12 | 16,236,368 |
| 3619 | LOC729025 | 12 | 16,250,464 | 3620 | LOC729025 | 12 | 16,264,150 |
| 3621 | LOC729025 | 12 | 16,288,678 | 3622 | LOC729025 | 12 | 16,297,957 |
| 3623 | LOC729025 | 12 | 16,298,403 | 3624 | LOC729025 | 12 | 16,302,981 |
| 3625 | LOC729025 | 12 | 16,310,672 | 3626 | LOC729025 | 12 | 16,319,824 |
| 3627 | LOC729025 | 12 | 16,328,662 | 3628 | LOC729025 | 12 | 16,328,782 |
| 3629 | LOC729025 | 12 | 16,334,812 | 3630 | LOC729025 | 12 | 16,384,311 |
| 3631 | PIK3C2G | 12 | 18,237,964 | 3632 | PIK3C2G | 12 | 18,274,187 |
| 3633 | PIK3C2G | 12 | 18,291,303 | 3634 | PIK3C2G | 12 | 18,301,821 |
| 3635 | ITPR2 | 12 | 26,382,742 | 3636 | ITPR2 | 12 | 26,383,105 |
| 3637 | ITPR2 | 12 | 26,475,978 | 3638 | ITPR2 | 12 | 26,476,845 |
| 3639 | ITPR2 | 12 | 26,477,096 | 3640 | ITPR2 | 12 | 26,624,329 |
| 3641 | CNOT2 | 12 | 68,794,819 | 3642 | CNOT2 | 12 | 68,819,144 |
| 3643 | CNOT2 | 12 | 68,831,198 | 3644 | CNOT2 | 12 | 68,841,926 |
| 3645 | CNOT2 | 12 | 68,893,334 | 3646 | CNOT2 | 12 | 68,960,922 |
| 3647 | CNOT2 | 12 | 68,977,572 | 3648 | CNOT2 | 12 | 69,001,757 |
| 3649 | CNOT2 | 12 | 69,005,612 | 3650 | CNOT2 | 12 | 69,015,087 |
| 3651 | CNOT2 | 12 | 69,023,213 | 3652 | SNOT2 | 12 | 69,026,461 |
| 3653 | CNOT2 | 12 | 69,027,002 | 3654 | CNOT2 | 12 | 69,063,143 |
| 3655 | KCNC2 | 12 | 73,596,702 | 3656 | KCNC2 | 12 | 73,597,602 |
| 3657 | KCNC2 | 12 | 73,620,979 | 3658 | KCNC2 | 12 | 73,625,557 |
| 3659 | KCNC2 | 12 | 73,723,470 | 3660 | KCNC2 | 12 | 73,877,027 |
| 3661 | KCNC2 | 12 | 73,886,479 | 3662 | KCNC2 | 12 | 73,898,155 |
| 3663 | NAV3 | 12 | 76,718,256 | 3664 | NAV3 | 12 | 76,748,290 |
| 3665 | NAV3 | 12 | 76,793,282 | 3666 | NAV3 | 12 | 76,798,556 |
| 3667 | NAV3 | 12 | 76,817,706 | 3668 | NAV3 | 12 | 76,824,819 |
| 3669 | NAV3 | 12 | 76,839,838 | 3670 | NAV3 | 12 | 76,845,308 |
| 3671 | NAV3 | 12 | 76,847,522 | 3672 | NAV3 | 12 | 76,854,382 |
| 3673 | NAV3 | 12 | 76,862,517 | 3674 | NAV3 | 12 | 76,872,382 |
| 3675 | NAV3 | 12 | 76,891,682 | 3676 | NAV3 | 12 | 77,014,895 |
| 3677 | NAV3 | 12 | 77,064,747 | 3678 | NAV3 | 12 | 77,070,387 |
| 3679 | NAV3 | 12 | 77,126,330 | 3680 | GAS2L3 | 12 | 99,495,794 |
| 3681 | GAS2L3 | 12 | 99,498,724 | 3682 | GAS2L3 | 12 | 99,519,980 |
| 3683 | CHST11 | 12 | 103,426,320 | 3684 | CHST11 | 12 | 103,448,913 |
| 3685 | ATXN2 | 12 | 110,487,733 | 3686 | ATXN2 | 12 | 110,547,258 |
| 3687 | PLA2G1B | 12 | 119,247,148 | 3688 | MTIF3 | 13 | 26,895,999 |
| 3689 | MTIF3 | 13 | 26,938,502 | 3690 | MTIF3 | 13 | 26,939,055 |
| 3691 | MTIF3 | 13 | 26,945,908 | 3692 | MTIF3 | 13 | 26,979,535 |
| 3693 | SLC46A3 | 13 | 28,207,069 | 3694 | SLC46A3 | 13 | 28,267,317 |
| 3695 | SLC46A3 | 13 | 28,270,443 | 3696 | UBL3 | 13 | 29,334,889 |
| 3697 | UBL3 | 13 | 29,360,712 | 3698 | UBL3 | 13 | 29,362,035 |
| 3699 | N4BP2L2 | 13 | 31,898,581 | 3700 | N4BP2L2 | 13 | 31,915,043 |
| 3701 | N4BP2L2 | 13 | 31,934,759 | 3702 | N4BP2L2 | 13 | 32,021,516 |
| 3703 | NBEA | 13 | 33,950,130 | 3704 | NBEA | 13 | 34,391,285 |
| 3705 | NBEA | 13 | 34,694,247 | 3706 | NBEA | 13 | 34,706,182 |
| 3707 | NBEA | 13 | 34,784,368 | 3708 | NBEA | 13 | 34,784,675 |
| 3709 | NBEA | 13 | 34,835,455 | 3710 | NBEA | 13 | 34,844,029 |
| 3711 | NBEA | 13 | 34,878,006 | 3712 | NBEA | 13 | 34,964,578 |
| 3713 | NBEA | 13 | 34,969,786 | 3714 | NBEA | 13 | 34,979,260 |
| 3715 | NBEA | 13 | 34,980,142 | 3716 | NBEA | 13 | 34,988,680 |
| 3717 | NBEA | 13 | 35,037,473 | 3718 | NBEA | 13 | 35,070,282 |
| 3719 | NBEA | 13 | 35,086,809 | 3720 | NBEA | 13 | 35,087,894 |
| 3721 | NBEA | 13 | 35,112,563 | 3722 | NBEA | 13 | 35,118,281 |
| 3723 | NBEA | 13 | 35,130,015 | 3724 | NBEA | 13 | 35,142,667 |
| 3725 | TRPC4 | 13 | 37,134,885 | 3726 | TRPC4 | 13 | 37,148,211 |
| 3727 | TRPC4 | 13 | 37,178,430 | 3728 | TRPC4 | 13 | 37,187,961 |
| 3729 | TRPC4 | 13 | 37,203,414 | 3730 | TRPC4 | 13 | 37,247,236 |
| 3731 | TRPC4 | 13 | 37,259,744 | 3732 | TRPC4 | 13 | 37,343,288 |
| 3733 | FNDC3A | 13 | 48,210,811 | 3734 | FNDC3A | 13 | 48,211,076 |
| 3735 | PCDH17 | 13 | 57,157,493 | 3736 | PCDH17 | 13 | 57,209,470 |
| 3737 | PCDH20 | 13 | 60,876,811 | 3738 | PCDH20 | 13 | 60,884,919 |
| 3739 | PCDH20 | 13 | 60,887,176 | 3740 | LMO7 | 13 | 75,185,966 |
| 3741 | LMO7 | 13 | 75,218,115 | 3742 | LMO7 | 13 | 75,241,934 |
| 3743 | LMO7 | 13 | 75,245,817 | 3744 | LMO7 | 13 | 75,260,740 |
| 3745 | LMO7 | 13 | 75,261,656 | 3746 | LMO7 | 13 | 75,261,722 |
| 3747 | LMO7 | 13 | 75,293,621 | 3748 | LMO7 | 13 | 75,308,726 |
| 3749 | LMO7 | 13 | 75,311,839 | 3750 | LMO7 | 13 | 75,315,102 |
| 3751 | LMO7 | 13 | 75,319,632 | 3752 | LMO7 | 13 | 75,321,249 |
| 3753 | LMO7 | 13 | 75,334,096 | 3754 | SLAIN1 | 13 | 77,130,842 |
| 3755 | SLAIN1 | 13 | 77,151,328 | .3756 | SLAIN1 | 13 | 77,192,452 |
| 3757 | SLAIN1 | 13 | 77,192,550 | 3758 | SLAIN1 | 13 | 77,211,551 |
| 3759 | SLAIN1 | 13 | 77,232,218 | 3760 | SLAIN1 | 13 | 77,290,759 |
| 3761 | GPC5 | 13 | 90,547,957 | 3762 | GPC5 | 13 | 90,811,945 |
| 3763 | GPC5 | 13 | 90,818,043 | 3764 | GPC5 | 13 | 90,832,063 |
| 3765 | GPC5 | 13 | 90,924,773 | 3766 | GPC5 | 13 | 90,925,132 |
| 3767 | GPC5 | 13 | 90,931,610 | 3768 | GPC5 | 13 | 90,931,942 |
| 3769 | GPC5 | 13 | 90,932,220 | 3770 | GPC5 | 13 | 91,109,203 |
| 3771 | GPC5 | 13 | 91,143,580 | 3772 | GPC5 | 13 | 91,145,267 |
| 3773 | GPCS | 13 | 91,149,048 | 3774 | GPC5 | 13 | 91,159,705 |
| 3775 | GPC5 | 13 | 91,160,283 | 3776 | GPC5 | 13 | 91,222,210 |
| 3777 | GPC5 | 13 | 91,293,729 | 3778 | GPC5 | 13 | 91,346,842 |
| 3779 | GPC5 | 13 | 91,356,566 | 3780 | GPC5 | 13 | 91,382,163 |
| 3781 | GPC5 | 13 | 91,389,873 | 3782 | GPCS | 13 | 91,399,197 |
| 3783 | GPC5 | 13 | 92,117,688 | 3784 | GPC5 | 13 | 92,117,738 |
| 3785 | GPC5 | 13 | 92,121,270 | 3786 | GPC5 | 13 | 92,164,430 |
| 3787 | GPC5 | 13 | 92,275,844 | 3788 | GPCS | 13 | 92,310,485 |
| 3789 | GPC5 | 13 | 92,332,949 | 3790 | GPC5 | 13 | 92,347,342 |
| 3791 | GPC5 | 13 | 92,354,245 | 3792 | GPCS | 13 | 92,362,008 |
| 3793 | GPC6 | 13 | 92,673,304 | 3794 | GPC6 | 13 | 92,693,343 |
| 3795 | GPC6 | 13 | 92,712,064 | 3796 | GPC6 | 13 | 92,775,697 |
| 3797 | GPC6 | 13 | 92,805,108 | 3798 | GPC6 | 13 | 92,807,207 |
| 3799 | GPC6 | 13 | 92,807,479 | 3800 | GPC6 | 13 | 92,807,544 |
| 3801 | GPC6 | 13 | 92,809,170 | 3802 | GPC6 | 13 | 92,916,576 |
| 3803 | GPC6 | 13 | 93,081,095 | 3804 | GPC6 | 13 | 93,088,009 |
| 3805 | GPC6 | 13 | 93,091,677 | 3806 | GPC6 | 13 | 93,115,588 |
| 3807 | GPC6 | 13 | 93,183,430 | 3808 | GPC6 | 13 | 93,185,878 |
| 3809 | GPC6 | 13 | 93,196,895 | 3810 | GPC6 | 13 | 93,196,981 |
| 3811 | GPC6 | 13 | 93,235,896 | 3812 | GPC6 | 13 | 93,340,807 |
| 3813 | GPC6 | 13 | 93,341,695 | 3814 | GPC6 | 13 | 93,416,682 |
| 3815 | GPC6 | 13 | 93,417,250 | 3816 | GPC6 | 13 | 93,430,821 |
| 3817 | GPC6 | 13 | 93,434,569 | 3818 | GPC6 | 13 | 93,497,436 |
| 3819 | GPC6 | 13 | 93,540,169 | 3820 | GPC6 | 13 | 93,667,409 |
| 3821 | GPC6 | 13 | 93,668,359 | 3822 | GPC6 | 13 | 93,851,561 |
| 3823 | NALCN | 13 | 100,517,778 | 3824 | NALCN | 13 | 100,522,814 |
| 3825 | NALCN | 13 | 100,523,120 | 3826 | NALCN | 13 | 100,535,252 |
| 3827 | NALCN | 13 | 100,611,732 | 3828 | NALCN | 13 | 100,681,085 |
| 3829 | NALCN | 13 | 100,688,436 | 3830 | NALCN | 13 | 100,690,246 |
| 3831 | NALCN | 13 | 100,700,656 | 3832 | NALCN | 13 | 100,766,311 |
| 3833 | NALCN | 13 | 100,774,404 | 3834 | NALCN | 13 | 100,777,500 |
| 3835 | NALCN | 13 | 100,849,091 | 3836 | NALCN | 13 | 100,853,208 |
| 3837 | NALCN | 13 | 100,906,309 | 3838 | ITGBL1 | 13 | 100,939,679 |
| 3839 | ITGBL1 | 13 | 100,956,004 | 3840 | ITGBL1 | 13 | 100,973,899 |
| 3841 | ITGBL1 | 13 | 101,003,300 | 3842 | ITGBL1 | 13 | 101,022,894 |
| 3843 | ITGBL1 | 13 | 101,028,277 | 3844 | ITGBL1 | 13 | 101,066,640 |
| 3845 | ITGBL1 | 13 | 101,070,551 | 3846 | ITGBL1 | 13 | 101,075,206 |
| 3847 | ITGBL1 | 13 | 101,088,453 | 3848 | ITGBL1 | 13 | 101,144,060 |
| 3849 | ITGBL1 | 13 | 101,163,997 | 3850 | ITGBL1 | 13 | 101,164,826 |
| 3851 | ITGBLI | 13 | 101,165,404 | 3852 | ITGBL1 | 13 | 101,165,489 |
| 3853 | WDR23 | 14 | 23,656,673 | 3854 | WDR23 | 14 | 23,664,573 |
| 3855 | WDR23 | 14 | 23,671,158 | 3856 | NOVA1 | 14 | 26,027,844 |
| 3857 | NOVA1 | 14 | 26,060,196 | 3858 | NOVA1 | 14 | 26,107,933 |
| 3859 | NOVA1 | 14 | 26,129,186 | 3860 | NOVA1 | 14 | 26,159,322 |
| 3861 | NOVA1 | 14 | 26,193,254 | 3862 | NOVA1 | 14 | 26,201,964 |
| 3863 | NOVA1 | 14 | 26,210,860 | 3864 | NPAS3 | 14 | 32,658,581 |
| 3865 | NPAS3 | 14 | 32,661,665 | 3866 | NPAS3 | 14 | 32,777,738 |
| 3867 | NPAS3 | 14 | 32,782,805 | 3868 | NPAS3 | 14 | 32,887,201 |
| 3869 | NPAS3 | 14 | 32,887,304 | 3870 | NPAS3 | 14 | 32,894,144 |
| 3871 | NPAS3 | 14 | 32,983,731 | 3872 | NPAS3 | 14 | 33,099,951 |
| 3873 | NPAS3 | 14 | 33,099,995 | 3874 | NPAS3 | 14 | 33,192,243 |
| 3875 | NPAS3 | 14 | 33,192,848 | 3876 | NPAS3 | 14 | 33,200,625 |
| 3877 | NPAS3 | 14 | 33,206,451 | 3878 | SLC25A21 | 14 | 36,227,370 |
| 3879 | SLC25A21 | 14 | 36,242,267 | 3880 | SLC25A21 | 14 | 36,246,679 |
| 3881 | SLC25A21 | 14 | 36,406,723 | 3882 | SLC25A21 | 14 | 36,411,454 |
| 3883 | SLC25A21 | 14 | 36,411,583 | 3884 | SLC25A21 | 14 | 36,419,219 |
| 3885 | SLC25A21 | 14 | 36,419,536 | 3886 | SLC25A21 | 14 | 36,557,939 |
| 3887 | SLC25A21 | 14 | 36,558,759 | 3888 | SLC25A21 | 14 | 36,657,448 |
| 3889 | SLC25A21 | 14 | 36,664,123 | 3890 | SLC25A21 | 14 | 36,727,597 |
| 3891 | LRFN5 | 14 | 41,011,370 | 3892 | LRFN5 | 14 | 41,019,623 |
| 3893 | LRFN5 | 14 | 41,023,512 | 3894 | LRFN5 | 14 | 41,066,681 |
| 3895 | LRFN5 | 14 | 41,246,521 | 3896 | LRFN5 | 14 | 41,252,963 |
| 3897 | LRFN5 | 14 | 41,299,858 | 3898 | LRFN5 | 14 | 41,357,595 |
| 3899 | LRFN5 | 14 | 41,372,302 | 3900 | LRFN5 | 14 | 41,379,353 |
| 3901 | LRFN5 | 14 | 41,384,489 | 3902 | LRFN5 | 14 | 41,388,962 |
| 3903 | LRFN5 | 14 | 41,389,331 | 3904 | LRFN5 | 14 | 41,484,830 |
| 3905 | GNG2 | 14 | 51,340,131 | 3906 | GNG2 | 14 | 51,340,221 |
| 3907 | GNG2 | 14 | 51,402,742 | 3908 | GNG2 | 14 | 51,409,101 |
| 3909 | GNG2 | 14 | 51,428,644 | 3910 | SAMD4A | 14 | 54,111,220 |
| 3911 | SAMD4A | 14 | 54,112,365 | 3912 | SAMD4A | 14 | 54,113,270 |
| 3913 | SAMD4A | 14 | 54,319,095 | 3914 | SAMD4A | 14 | 54,327,340 |
| 3915 | SAMD4A | 14 | 54,335,279 | 3916 | SAMD4A | 14 | 54,335,321 |
| 3917 | SAMD4A | 14 | 54,373,811 | 3918 | SAMD4A | 14 | 54,375,990 |
| 3919 | SAMD4A | 14 | 54,376,207 | 3920 | PPP2R5E | 14 | 62,856,493 |
| 3921 | PPP2R5E | 14 | 62,858,991 | 3922 | PPP2R5E | 14 | 62,949,791 |
| 3923 | PPP2RSE | 14 | 62,985,912 | 3924 | PPP2R5E | 14 | 62,986,716 |
| 3925 | PPP2R5E | 14 | 63,014,544 | 3926 | PPP2RSE | 14 | 63,044,719 |
| 3927 | PPP2R5E | 14 | 63,091,961 | 3928 | PPP2R5E | 14 | 63,099,534 |
| 3929 | PPP2R5E | 14 | 63,213,578 | 3930 | RGS6 | 14 | 71,459,842 |
| 3931 | RGS6 | 14 | 71,461,797 | 3932 | RGS6 | 14 | 71,524,688 |
| 3933 | RGS6 | 14 | 71,527,435 | 3934 | RGS6 | 14 | 71,532,362 |
| 3935 | RGS6 | 14 | 71,532,447 | 3936 | RGS6 | 14 | 71,730,259 |
| 3937 | RGS6 | 14 | 71,789,160 | 3938 | RGS6 | 14 | 71,802,071 |
| 3939 | RGS6 | 14 | 71,806,722 | 3940 | RGS6 | 14 | 71,850,274 |
| 3941 | RGS6 | 14 | 71,868,756 | 3942 | RGS6 | 14 | 71,872,419 |
| 3943 | RGS6 | 14 | 71,886,146 | 3944 | RGS6 | 14 | 71,886,379 |
| 3945 | RGS6 | 14 | 71,889,437 | 3946 | RGS6 | 14 | 71,896,348 |
| 3947 | RGS6 | 14 | 71,903,671 | 3948 | RGS6 | 14 | 71,904,219 |
| 3949 | RGS6 | 14 | 71,907,687 | 3950 | RGS6 | 14 | 71,921,862 |
| 3951 | RGS6 | 14 | 71,922,573 | 3952 | RGS6 | 14 | 71,925,692 |
| 3953 | RGS6 | 14 | 72,020,234 | 3954 | RGS6 | 14 | 72,129,058 |
| 3955 | NRXN3 | 14 | 77,481,947 | 3956 | NRXN3 | 14 | 79,336,370 |
| 3957 | NRXN3 | 14 | 79,340,786 | 3958 | NRXN3 | 14 | 79,343,602 |
| 3959 | NRXN3 | 14 | 79,403,317 | 3960 | KCNK10 | 14 | 87,720,759 |
| 3961 | KCNK10 | 14 | 87,768,396 | 3962 | KCNK10 | 14 | 87,843,799 |
| 3963 | KCNK10 | 14 | 87,846,012 | 3964 | KCNK10 | 14 | 87,874,519 |
| 3965 | KCNK10 | 14 | 87,881,781 | 3966 | KCNK10 | 14 | 87,887,777 |
| 3967 | KCNK13 | 14 | 89,669,023 | 3968 | KCNK13 | 14 | 89,728,563 |
| 3969 | KCNK13 | 14 | 89,740,891 | 3970 | PSMC1 | 14 | 89,806,078 |
| 3971 | PSMC1 | 14 | 89,810,027 | 3972 | RPS6KA5 | 14 | 90,431,677 |
| 3973 | RPS6KA5 | 14 | 90,501,524 | 3974 | RPS6KA5 | 14 | 90,502,182 |
| 3975 | RPS6KA5 | 14 | 90,527,909 | 3976 | RPS6KA5 | 14 | 90,536,260 |
| 3977 | RPS6KA5 | 14 | 90,572,871 | 3978 | RPS6KA5 | 14 | 90,600,783 |
| 3979 | RPS6KA5 | 14 | 90,600,927 | 3980 | RPS6KA5 | 14 | 90,616,889 |
| 3981 | CCDC88C | 14 | 90,843,247 | 3982 | CCDC88C | 14 | 90,864,783 |
| 3983 | CCDC88C | 14 | 90,916,433 | 3984 | CCDC88C | 14 | 90,918,598 |
| 3985 | CCDC88C | 14 | 90,919,949 | 3986 | CCDC88C | 14 | 90,920,049 |
| 3987 | CCDC88C | 14 | 90,960,430 | 3988 | CCDC88C | 14 | 90,966,287 |
| 3989 | ATXN3 | 14 | 91,603,867 | 3990 | ATXN3 | 14 | 91,606,712 |
| 3991 | ATXN3 | 14 | 91,610,714 | 3992 | ATXN3 | 14 | 91,610,975 |
| 3993 | ATXN3 | 14 | 91,611,093 | 3994 | ATXN3 | 14 | 91,618,538 |
| 3995 | ATXN3 | 14 | 91,621,082 | 3996 | ATXN3 | 14 | 91,621,815 |
| 3997 | ATXN3 | 14 | 91,621,825 | 3998 | BCL11B | 14 | 98,713,688 |
| 3999 | BCL11B | 14 | 98,742,719 | 4000 | BCL11B | 14 | 98,746,376 |
| 4001 | BCL11B | 14 | 98,757,526 | 4002 | BCL11B | 14 | 98,765,857 |
| 4003 | BCL11B | 14 | 98,816,027 | 4004 | MAGEL2 | 15 | 21,460,719 |
| 4005 | ATP10A | 15 | 23,469,206 | 4006 | ATP10A | 15 | 23,471,534 |
| 4007 | ATP10A | 15 | 23,474,886 | 4008 | ATP10A | 15 | 23,476,768 |
| 4009 | ATP10A | 15 | 23,478,440 | 4010 | ATP10A | 15 | 23,486,252 |
| 4011 | ATP10A | 15 | 23,486,388 | 4012 | ATP10A | 15 | 23,493,398 |
| 4013 | ATP10A | 15 | 23,496,935 | 4014 | ATP10A | 15 | 23,497,677 |
| 4015 | ATP10A | 15 | 23,498,058 | 4016 | ATP10A | 15 | 23,504,535 |
| 4017 | ATP10A | 15 | 23,825,206 | 4018 | RYR3 | 15 | 31,385,694 |
| 4019 | RYR3 | 15 | 31,386,142 | 4020 | RYR3 | 15 | 31,397,903 |
| 4021 | RYR3 | 15 | 31,406,234 | 4022 | RYR3 | 15 | 31,410,065 |
| 4023 | RYR3 | 15 | 31,424,553 | 4024 | RYR3 | 15 | 31,507,982 |
| 4025 | RYR3 | 15 | 31,661,043 | 4026 | RYR3 | 15 | 31,701,532 |
| 4027 | RYR3 | 15 | 31,707,341 | 4028 | RYR3 | 15 | 31,716,682 |
| 4029 | RYR3 | 15 | 31,741,944 | 4030 | RYR3 | 15 | 31,744,674 |
| 4031 | RYR3 | 15 | 31,750,983 | 4032 | RYR3 | 15 | 31,815,285 |
| 4033 | RYR3 | 15 | 31,899,901 | 4034 | RYR3 | 15 | 31,900,545 |
| 4035 | RYR3 | 15 | 31,922,164 | 4036 | RYR3 | 15 | 31,926,584 |
| 4037 | C15ORF41 | 15 | 34,692,069 | 4038 | C15ORF41 | 15 | 34,733,595 |
| 4039 | C15ORF41 | 15 | 34,791,082 | 4040 | C15ORF41 | 15 | 34,796,937 |
| 4041 | C15ORF41 | 15 | 34,833,829 | 4042 | C15ORF41 | 15 | 34,863,762 |
| 4043 | C15ORF41 | 15 | 34,863,820 | 4044 | C15ORF41 | 15 | 34,890,144 |
| 4045 | C15ORF41 | 15 | 34,901,333 | 4046 | C15ORF41 | 15 | 34,901,385 |
| 4047 | C15ORF41 | 15 | 34,902,489 | 4048 | MEIS2 | 15 | 34,979,253 |
| 4049 | MEIS2 | 15 | 35,010,253 | 4050 | MEIS2 | 15 | 35,073,394 |
| 4051 | RASGRP1 | 15 | 36,581,858 | 4052 | RASGRP1 | 15 | 36,689,768 |
| 4053 | RASGRP1 | 15 | 36,707,739 | 4054 | RASGRP1 | 15 | 37,051,959 |
| 4055 | GLDN | 15 | 49,444,605 | 4056 | GLDN | 15 | 49,450,678 |
| 4057 | GLDN | 15 | 49,457,033 | 4058 | GLDN | 15 | 49,457,768 |
| 4059 | GLDN | 15 | 49,459,928 | 4060 | GLDN | 15 | 49,460,922 |
| 4061 | GLDN | 15 | 49,461,033 | 4062 | GLDN | 15 | 49,477,064 |
| 4063 | GLDN | 15 | 49,481,784 | 4064 | KIAA1370 | 15 | 50,717,008 |
| 4065 | KIAA1370 | 15 | 50,742,531 | 4066 | KIAA1370 | 15 | 50,746,979 |
| 4067 | CGNL1 | 15 | 55,403,598 | 4068 | CGNL1 | 15 | 55,423,011 |
| 4069 | CGNL1 | 15 | 55,516,581 | 4070 | CGNL1 | 15 | 35,518,865 |
| 4071 | CGNL1 | 15 | 55,520,686 | 4072 | CGNL1 | 15 | 55,521,306 |
| 4073 | CGNL1 | 15 | 55,527,416 | 4074 | CGNL1 | 15 | 55,627,080 |
| 4075 | CGNL1 | 15 | 55,627,123 | 4076 | CGNL1 | 15 | 55,632,176 |
| 4077 | CGNL1 | 15 | 55,633,731 | 4078 | RORA | 15 | 58,605,378 |
| 4079 | RORA | 15 | 58,680,723 | 4080 | RORA | 15 | 58,710,721 |
| 4081 | RORA | 15 | 58,718,548 | 4082 | RORA | 15 | 58,721,610 |
| 4083 | RORA | 15 | 59,614,183 | 4084 | CLK3 | 15 | 72,697,052 |
| 4085 | CLK3 | 15 | 72,702,941 | 4086 | CLK3 | 15 | 72,707,585 |
| 4087 | CLK3 | 15 | 72,727,323 | 4088 | TBC1D2B | 15 | 75,981,714 |
| 4089 | TBC1D2B | 15 | 76,076,030 | 4090 | TBC1D2B | 15 | 76,083,890 |
| 4091 | TBC1D2B | 15 | 76,096,608 | 4092 | TBC1D2B | 15 | 76,098,398 |
| 4093 | TBC1D2B | 15 | 76,121,710 | 4094 | TBC1D2B | 15 | 76,124,994 |
| 4095 | TBC1D2B | 15 | 76,150,830 | 4096 | TBC1D2B | 15 | 76,157,798 |
| 4097 | TBC1D2B | 15 | 76,168,715 | 4098 | TBC1D2B | 15 | 76,170,084 |
| 4099 | ARNT2 | 15 | 78,539,124 | 4100 | ARNT2 | 15 | 78,542,039 |
| 4101 | ARNT2 | 15 | 78,566,813 | 4102 | ARNT2 | 15 | 78,700,178 |
| 4103 | ARNT2 | 15 | 78,704,322 | 4104 | ARNT2 | 15 | 78,713,205 |
| 4105 | ARNT2 | 15 | 78,718,605 | 4106 | SH3GL3 | 15 | 81,924,484 |
| 4107 | SH3GL3 | 15 | 82,039,470 | 4108 | SH3GL3 | 15 | 82,089,602 |
| 4109 | PCSK6 | 15 | 99,687,851 | 4110 | PCSK6 | 15 | 99,842,741 |
| 4111 | PCSK6 | 15 | 99,970,111 | 4112 | A2BP1 | 16 | 6,521,767 |
| 4113 | A2BP1 | 16 | 6,523,126 | 4114 | A2BP1 | 16 | 6,526,273 |
| 4115 | A2BP1 | 16 | 6,528,853 | 4116 | A2BP1 | 16 | 6,531,472 |
| 4117 | A2BP1 | 16 | 6,676,476 | 4118 | A2BP1 | 16 | 6,691,358 |
| 4119 | A2BP1 | 16 | 6,963,875 | 4120 | A2BP1 | 16 | 6,970,310 |
| 4121 | A2BP1 | 16 | 6,974,269 | 4122 | A2BP1 | 16 | 6,980,934 |
| 4123 | A2BP1 | 16 | 7,223,623 | 4124 | A2BP1 | 16 | 7,223,826 |
| 4125 | A2BP1 | 16 | 7,224,553 | 4126 | A2BP1 | 16 | 7,226,822 |
| 4127 | A2BP1 | 16 | 7,234,640 | 4128 | A2BP1 | 16 | 7,247,111 |
| 4129 | A2BP1 | 16 | 7,253,357 | 4130 | A2BP1 | 16 | 7,435,187 |
| 4131 | A2BP1 | 16 | 7,551,379 | 4132 | A2BP1 | 16 | 7,556,763 |
| 4133 | A2BP1 | 16 | 7,560,608 | 4134 | A2BP1 | 16 | 7,629,108 |
| 4135 | A2BP1 | 16 | 7,630,072 | 4136 | A2BP1 | 16 | 7,632,187 |
| 4137 | A2BP1 | 16 | 7,633,275 | 4138 | A2BP1 | 16 | 7,700,000 |
| 4139 | TMC5 | 16 | 19,254,444 | 4140 | TMC5 | 16 | 19,311,395 |
| 4141 | TMCS | 16 | 19,321,487 | 4142 | TMCS | 16 | 19,413,314 |
| 4143 | GDE1 | 16 | 19,455,004 | 4144 | EEF2K | 16 | 22,107,225 |
| 4145 | EEF2K | 16 | 22,114,775 | 4146 | EEF2K | 16 | 22,169,198 |
| 4147 | EEF2K | 16 | 22,170,015 | 4148 | EEF2K | 16 | 22,249,604 |
| 4149 | KIFC3 | 16 | 56,410,585 | 4150 | HYDIN | 16 | 69,818,152 |
| 4151 | WWOX | 16 | 76,643,771 | 4152 | WWOX | 16 | 76,655,506 |
| 4153 | WWOX | 16 | 76,672,696 | 4154 | WWOX | 16 | 76,673,124 |
| 4155 | WWOX | 16 | 76,674,781 | 4156 | WWOX | 16 | 76,691,956 |
| 4157 | WWOX | 16 | 77,016,847 | 4158 | WWOX | 16 | 77,024,259 |
| 4159 | WWOX | 16 | 77,041,675 | 4160 | WWOX | 16 | 77,045,378 |
| 4161 | PLCG2 | 16 | 80,379,032 | 4162 | MPHOSPH6 | 16 | 80,705,905 |
| 4163 | MPHOSPH6 | 16 | 80,722,907 | 4164 | MPHOSPH6 | 16 | 80,723,078 |
| 4165 | MPHOSPH6 | 16 | 80,728,226 | 4166 | MPHOSPH6 | 16 | 80,728,868 |
| 4167 | MPHOSPH6 | 16 | 80,737,839 | 4168 | MPHOSPH6 | 16 | 80,744,073 |
| 4169 | MPHOSPH6 | 16 | 80,750,997 | 4170 | MPHOSPH6 | 16 | 80,757,604 |
| 4171 | MPHOSPH6 | 16 | 80,759,877 | 4172 | MPHOSPH6 | 16 | 80,765,783 |
| 4173 | MPHOSPH6 | 16 | 80,787,893 | 4174 | MPHOSPH6 | 16 | 80,815,052 |
| 4175 | MPHOSPH6 | 16 | 80,815,095 | 4176 | MPHOSPH6 | 16 | 80,817,367 |
| 4177 | MPHOSPH6 | 16 | 80,820,057 | 4178 | MPHOSPH6 | 16 | 80,831,050 |
| 4179 | MPHOSPH6 | 16 | 80,831,708 | 4180 | MPHOSPH6 | 16 | 80,833,378 |
| 4181 | MPHOSPH6 | 16 | 80,833,755 | 4182 | CDH13 | 16 | 81,340,088 |
| 4183 | CDH13 | 16 | 81,348,352 | 4184 | CDH13 | 16 | 81,374,752 |
| 4185 | CDH13 | 16 | 81,380,017 | 4186 | CDH13 | 16 | 81,390,450 |
| 4187 | CDH13 | 16 | 81,443,634 | 4188 | CDH13 | 16 | 81,464,521 |
| 4189 | CDH13 | 16 | 81,470,072 | 4190 | CDH13 | 16 | 81,813,878 |
| 4191 | CDH13 | 16 | 81,884,064 | 4192 | CDH13 | 16 | 81,887,115 |
| 4193 | CDH13 | 16 | 81,888,111 | 4194 | CDH13 | 16 | 81,898,337 |
| 4195 | CDH13 | 16 | 81,899,890 | 4196 | CDH13 | 16 | 81,917,154 |
| 4197 | CDH13 | 16 | 81,919,816 | 4198 | CDH13 | 16 | 81,925,901 |
| 4199 | CDH13 | 16 | 81,931,464 | 4200 | CDH13 | 16 | 81,933,244 |
| 4201 | CDH13 | 16 | 82,194,302 | 4202 | CDH13 | 16 | 82,357,323 |
| 4203 | CDH13 | 16 | 82,394,233 | 4204 | CDH13 | 16 | 82,410,620 |
| 4205 | CDH13 | 16 | 82,420,137 | 4206 | USP10 | 16 | 83,305,397 |
| 4207 | USP10 | 16 | 83,311,158 | 4208 | USP10 | 16 | 83,325,736 |
| 4209 | USP10 | 16 | 83,328,544 | 4210 | USP10 | 16 | 83,331,937 |
| 4211 | USP10 | 16 | 83,371,654 | 4212 | CRISPLD2 | 16 | 83,443,531 |
| 4213 | CRISPLD2 | 16 | 83,475,493 | 4214 | CRISPLD2 | 16 | 83,480,748 |
| 4215 | CRISPLD2 | 16 | 83,495,998 | 4216 | CRISPLD2 | 16 | 83,497,517 |
| 4217 | CRISPLD2 | 16 | 83,540,736 | 4218 | ALOX12B | 17 | 7,981,019 |
| 4219 | GAS7 | 17 | 9,808,904 | 4220 | DNAH9 | 17 | 11,444,122 |
| 4221 | DNAH9 | 17 | 11,650,220 | 4222 | DNAH9 | 17 | 11,662,069 |
| 4223 | DNAH9 | 17 | 11,715,142 | 4224 | RAB11FIP4 | 17 | 26,746,745 |
| 4225 | RAB11FIP4 | 17 | 27,005,299 | 4226 | CA10 | 17 | 47,089,473 |
| 4227 | CA10 | 17 | 47,092,582 | 4228 | CA10 | 17 | 47,092,738 |
| 4229 | CA10 | 17 | 47,098,311 | 4230 | CA10 | 17 | 47,099,992 |
| 4231 | CA10 | 17 | 47,382,993 | 4232 | CA10 | 17 | 47,412,115 |
| 4233 | CA10 | 17 | 47,427,244 | 4234 | CA10 | 17 | 47,434,848 |
| 4235 | CA10 | 17 | 47,435,142 | 4236 | CA10 | 17 | 47,453,436 |
| 4237 | CA10 | 17 | 47,528,183 | 4238 | CA10 | 17 | 47,531,938 |
| 4239 | CA10 | 17 | 47,547,164 | 4240 | CA10 | 17 | 47,566,855 |
| 4241 | CA10 | 17 | 47,599,744 | 4242 | CA10 | 17 | 47,674,724 |
| 4243 | CA10 | 17 | 47,675,666 | 4244 | CA10 | 17 | 48,115,311 |
| 4245 | CA10 | 17 | 48,122,i55 | 4246 | CA10 | 17 | 48,191,994 |
| 4247 | CA10 | 17 | 48,209,418 | 4248 | CA10 | 17 | 48,233,607 |
| 4249 | CA10 | 17 | 48,242,213 | 4250 | CA10 | 17 | 48,243,428 |
| 4251 | CA10 | 17 | 48,264,257 | 4252 | CA10 | 17 | 48,275,354 |
| 4253 | CA10 | 17 | 48,292,028 | 4254 | CA10 | 17 | 48,292,853 |
| 4255 | MSI2 | 17 | 52,558,314 | 4256 | MSI2 | 17 | 52,773,732 |
| 4257 | MSI2 | 17 | 52,817,244 | 4258 | MSI2 | 17 | 53,002,826 |
| 4259 | MSI2 | 17 | 53,007,065 | 4260 | MSI2 | 17 | 53,093,648 |
| 4261 | CACNG4 | 17 | 62,462,327 | 4262 | SDK2 | 17 | 68,829,410 |
| 4263 | SDK2 | 17 | 68,954,425 | 4264 | DNAH17 | 17 | 73,942,963 |
| 4265 | DNAH17 | 17 | 73,950,638 | 4266 | DNAH17 | 17 | 73,950,704 |
| 4267 | DNAH17 | 17 | 73,954,236 | 4268 | DNAH17 | 17 | 73,990,984 |
| 4269 | DNAH17 | 17 | 74,017,503 | 4270 | DNAH17 | 17 | 74,071,124 |
| 4271 | HRNBP3 | 17 | 74,776,568 | 4272 | HRNBP3 | 17 | 74,845,144 |
| 4273 | HRNBP3 | 17 | 74,857,410 | 4274 | HRNBP3 | 17 | 75,313,214 |
| 4275 | TGIF1 | 18 | 3,319,448 | 4276 | DLGAP1 | 18 | 3,829,680 |
| 4277 | DLGAP1 | 18 | 3,838,826 | 4278 | DLGAP1 | 18 | 3,840,002 |
| 4279 | DLGAP1 | 18 | 3,927,944 | 4280 | ZFP161 | 18 | 5,251,246 |
| 4281 | ZFP161 | 18 | 5,269,052 | 4282 | ZFP161 | 18 | 5,280,198 |
| 4283 | ZFP161 | 18 | 5,283,943 | 4284 | ZFP161 | 18 | 5,284,323 |
| 4285 | ZFP161 | 18 | 5,398,997 | 4286 | ZFP161 | 18 | 5,399,208 |
| 4287 | PTPRM | 18 | 7,523,566 | 4288 | PTPRM | 18 | 7,563,510 |
| 4289 | PTPRM | 18 | 7,571,167 | 4290 | PTPRM | 18 | 7,585,927 |
| 4291 | PTPRM | 18 | 7,598,911 | 4292 | PTPRM | 18 | 7,599,137 |
| 4293 | PTPRM | 18 | 7,892,147 | 4294 | PTPRM | 18 | 8,000,444 |
| 4295 | PTPRM | 18 | 8,040,886 | 4296 | PTPRM | 18 | 8,042,261 |
| 4297 | PTPRM | 18 | 8,059,868 | 4298 | PTPRM | 18 | 8,068,553 |
| 4299 | PTPRM | 18 | 8,086,608 | 4300 | PTPRM | 18 | 8,087,133 |
| 4301 | PTPRM | 18 | 8,288,088 | 4302 | PTPRM | 18 | 8,288,633 |
| 4303 | PTPRM | 18 | 8,377,680 | 4304 | KIAA0802 | 18 | 8,839,087 |
| 4305 | KIAA0802 | 18 | 8,840,062 | 4306 | KIAA0802 | 18 | 8,844,132 |
| 4307 | KIAA0802 | 18 | 8,884,977 | 4308 | OSBPL1A | 18 | 20,006,364 |
| 4309 | OSBPL1A | 18 | 20,042,294 | 4310 | OSBPL1A | 18 | 20,093,929 |
| 4311 | CHST9 | 18 | 22,804,050 | 4312 | CHST9 | 18 | 22,854,839 |
| 4313 | CHST9 | 18 | 22,874,628 | 4314 | CHST9 | 18 | 22,886,548 |
| 4315 | CHST9 | 18 | 22,965,754 | 4316 | CHST9 | 18 | 23,010,213 |
| 4317 | CHST9 | 18 | 23,012,354 | 4318 | LOXHD1 | 18 | 42,342,838 |
| 4319 | LOXHD1 | 18 | 42,382,501 | 4320 | NEDD4L | 18 | 54,010,852 |
| 4321 | NEDD4L | 18 | 54,013,845 | 4322 | NEDD4L | 18 | 54,039,159 |
| 4323 | NEDD4L | 18 | 54,047,200 | 4324 | NEDD4L | 18 | 54,095,115 |
| 4325 | NEDD4L | 18 | 54,100,941 | 4326 | NEDD4L | 18 | 54,155,894 |
| 4327 | NEDD4L | 18 | 54,165,956 | 4328 | NEDD4L | 18 | 54,173,333 |
| 4329 | NEDD4L | 18 | 54,179,311 | 4330 | NEDD4L | 18 | 54,188,829 |
| 4331 | NEDD4L | 18 | 54,202,520 | 4332 | NEDD4L | 18 | 54,202,587 |
| 4333 | NEDD4L | 18 | 54,238,628 | 4334 | NEDD4L | 18 | 54,250,387 |
| 4335 | NEDD4L | 18 | 54,259,246 | 4336 | CCBE1 | 18 | 55,220,303 |
| 4337 | CCBE1 | 18 | 55,241,702 | 4338 | CCBE1 | 18 | 55,332,724 |
| 4339 | CCBE1 | 18 | 55,333,626 | 4340 | CCBE1 | 18 | 55,372,586 |
| 4341 | CCBE1 | 18 | 55,372,710 | 4342 | CCBE1 | 18 | 55,372,725 |
| 4343 | CCBE1 | 18 | 55,375,168 | 4344 | CCBE1 | 18 | 55,377,089 |
| 4345 | CCBE1 | 18 | 55,494,237 | 4346 | CCBE1 | 18 | 55,507,128 |
| 4347 | CCBE1 | 18 | 55,510,412 | 4348 | CDH20 | 18 | 57,031,422 |
| 4349 | CDH7 | 18 | 61,289,294 | 4350 | CDH7 | 18 | 61,305,341 |
| 4351 | CDH7 | 18 | 61,305,944 | 4352 | CDH7 | 18 | 61,316,783 |
| 4353 | CDH7 | 18 | 61,469,322 | 4354 | CDH7 | 18 | 61,566,553 |
| 4355 | CDH7 | 18 | 61,586,240 | 4356 | CDH7 | 18 | 61,590,121 |
| 4357 | CDH7 | 18 | 61,595,280 | 4358 | CDH7 | 18 | 61,595,696 |
| 4359 | CDH7 | 18 | 61,626,782 | 4360 | CDH7 | 18 | 61,668,497 |
| 4361 | CDH7 | 18 | 61,690,373 | 4362 | CDH7 | 18 | 61,691,931 |
| 4363 | CDH7 | 18 | 61,693,003 | 4364 | CDH7 | 18 | 61,697,352 |
| 4365 | CDH7 | 18 | 61,697,973 | 4366 | CDH7 | 18 | 61,716,015 |
| 4367 | CDH7 | 18 | 61,716,853 | 4368 | CDH7 | 18 | 61,736,852 |
| 4369 | CDH7 | 18 | 61,739,825 | 4370 | TXNDC10 | 18 | 64,477,750 |
| 4371 | TXNDC10 | 18 | 64,483,360 | 4372 | TXNDC10 | 18 | 64,483,488 |
| 4373 | TXNOC10 | 18 | 64,536,945 | 4374 | DOK6 | 18 | 65,224,346 |
| 4375 | DOK6 | 18 | 65,312,795 | 4376 | DOK6 | 18 | 65,312,915 |
| 4377 | DOK6 | 18 | 65,324,331 | 4378 | DOK6 | 18 | 65,437,835 |
| 4379 | DOK6 | 18 | 65,452,233 | 4380 | DOK6 | 18 | 65,453,592 |
| 4381 | DOK6 | 18 | 65,637,817 | 4382 | DOK6 | 18 | 65,649,653 |
| 4383 | MBP | 18 | 72,823,708 | 4384 | LDLR | 19 | 11,102,877 |
| 4385 | LDLR | 19 | 11,163,807 | 4386 | ZNF667 | 19 | 61,678,984 |
| 4387 | ZNF667 | 19 | 61,723,565 | 4388 | ZNF544 | 19 | 63,458,980 |
| 4389 | ZNF544 | 19 | 63,476,544 | 4390 | ATRN | 20 | 3,443,074 |
| 4391 | ATRN | 20 | 3,560,688 | 4392 | ATRN | 20 | 3,587,019 |
| 4393 | RNF24 | 20 | 3,882,766 | 4394 | RNF24 | 20 | 3,941,491 |
| 4395 | RNF24 | 20 | 4,034,314 | 4396 | PRNT | 20 | 4,659,767 |
| 4397 | PRNT | 20 | 4,665,262 | 4398 | PRNT | 20 | 4,667,344 |
| 4399 | PRNT | 20 | 4,667,407 | 4400 | PRNT | 20 | 4,676,075 |
| 4401 | PRNT | 20 | 4,678,559 | 4402 | PRNT | 20 | 4,678,670 |
| 4403 | PRNT | 20 | 4,679,022 | 4404 | PRNT | 20 | 4,679,142 |
| 4405 | PRNT | 20 | 4,690,080 | 4406 | PRNT | 20 | 4,690,705 |
| 4407 | PRNT | 20 | 4,690,937 | 4408 | FERMT1 | 20 | 6,003,038 |
| 4409 | FERMT1 | 20 | 6,037,797 | 4410 | FERMT1 | 20 | 6,048,088 |
| 4411 | FERMT1 | 20 | 6,162,234 | 4412 | PLCB1 | 20 | 8,000,158 |
| 4413 | PLCB1 | 20 | 8,001,312 | 4414 | PLCB1 | 20 | 8,082,140 |
| 4415 | PLCB1 | 20 | 8,092,135 | 4416 | PLCB1 | 20 | 8,159,200 |
| 4417 | PLCB1 | 20 | 8,166,965 | 4418 | PLCB1 | 20 | 8,185,056 |
| 4419 | PLCB1 | 20 | 8,186,289 | 4420 | PLCB1 | 20 | 8,195,326 |
| 4421 | PLCB1 | 20 | 8,322,803 | 4422 | PLCB1 | 20 | 8,677,119 |
| 4423 | PLCB1 | 20 | 8,691,637 | 4424 | PLCB1 | 20 | 8,710,195 |
| 4425 | PLCB1 | 20 | 8,713,736 | 4426 | PLCB1 | 20 | 8,714,952 |
| 4427 | PLCB1 | 20 | 8,756,294 | 4428 | PLCB1 | 20 | 8,759,482 |
| 4429 | PLCB1 | 20 | 8,761,509 | 4430 | PLCB1 | 20 | 8,761,544 |
| 4431 | PLCB1 | 20 | 8,794,006 | 4432 | PLCB1 | 20 | 8,824,090 |
| 4433 | PLCB1 | 20 | 8,837,613 | 4434 | PLCB4 | 20 | 8,837,897 |
| 4435 | PLCB1 | 20 | 8,842,975 | 4436 | PLCB4 | 20 | 8,846,570 |
| 4437 | PLCB4 | 20 | 9,313,241 | 4438 | PLCB4 | 20 | 9,433,807 |
| 4439 | JAG1 | 20 | 10,589,470 | 4440 | JAG1 | 20 | 10,589,575 |
| 4441 | MACROD2 | 20 | 14,218,096 | 4442 | MACROD2 | 20 | 14,242,180 |
| 4443 | MACROD2 | 20 | 14,265,679 | 4444 | MACROD2 | 20 | 14,275,899 |
| 4445 | MACROD2 | 20 | 15,066,513 | 4446 | MACROD2 | 20 | 15,068,323 |
| 4447 | MACROD2 | 20 | 15,073,972 | 4448 | MACROD2 | 20 | 15,085,117 |
| 4449 | MACROD2 | 20 | 15,087,547 | 4450 | MACROD2 | 20 | 15,094,357 |
| 4451 | MACROD2 | 20 | 15,557,292 | 4452 | MACROD2 | 20 | 15,559,316 |
| 4453 | MACROD2 | 20 | 15,780,282 | 4454 | MACROD2 | 20 | 15,839,419 |
| 4455 | MACROD2 | 20 | 15,846,812 | 4456 | MACROD2 | 20 | 15,890,876 |
| 4457 | MACROD2 | 20 | 15,908,144 | 4458 | MACROD2 | 20 | 15,943,358 |
| 4459 | MACROD2 | 20 | 15,981,022 | 4460 | MACROD2 | 20 | 15,993,679 |
| 4461 | KIF16B | 20 | 16,199,083 | 4462 | KIF16B | 20 | 16,221,665 |
| 4463 | KIF16B | 20 | 16,270,199 | 4464 | KIF16B | 20 | 16,273,140 |
| 4465 | KIF16B | 20 | 16,275,825 | 4466 | KIF16B | 20 | 16,288,458 |
| 4467 | KIF16B | 20 | 16,288,836 | 4468 | KIF16B | 20 | 16,293,233 |
| 4469 | KIF16B | 20 | 16,294,439 | 4470 | KIF16B | 20 | 16,294,523 |
| 4471 | KIF16B | 20 | 16,301,934 | 4472 | KIF16B | 20 | 16,307,567 |
| 4473 | KIF16B | 20 | 16,314,615 | 4474 | KIF16B | 20 | 16,371,330 |
| 4475 | KIF16B | 20 | 16,395,367 | 4476 | KIF16B | 20 | 16,435,785 |
| 4477 | KIF16B | 20 | 16,436,083 | 4478 | KIF16B | 20 | 16,442,919 |
| 4479 | KIF16B | 20 | 16,445,469 | 4480 | KIF16B | 20 | 16,454,298 |
| 4481 | KIF16B | 20 | 16,454,369 | 4482 | KIF16B | 20 | 16,459,991 |
| 4483 | KIF16B | 20 | 16,476,914 | 4484 | KIF16B | 20 | 16,591,934 |
| 4485 | SEC23B | 20 | 18,435,014 | 4486 | SEC23B | 20 | 18,454,825 |
| 4487 | SEC23B | 20 | 18,470,933 | 4488 | SEC23B | 20 | 18,471,002 |
| 4489 | SEC23B | 20 | 18,517,599 | 4490 | MAPRE1 | 20 | 30,888,730 |
| 4491 | MAPRE1 | 20 | 30,891,296 | 4492 | PTPRT | 20 | 40,069,405 |
| 4493 | PTPRT | 20 | 40,087,340 | 4494 | PTPRT | 20 | 40,104,368 |
| 4495 | PTPRT | 20 | 40,111,844 | 4496 | PTPRT | 20 | 40,141,351 |
| 4497 | PTPRT | 20 | 40,141,648 | 4498 | PTPRT | 20 | 40,143,404 |
| 4499 | PTPRT | 20 | 40,151,573 | 4500 | PTPRT | 20 | 40,153,367 |
| 4501 | PTPRT | 20 | 40,176,580 | 4502 | PTPRT | 20 | 40,353,349 |
| 4503 | PTPRT | 20 | 40,525,385 | 4504 | PTPRT | 20 | 40,541,070 |
| 4505 | PTPRT | 20 | 40,715,840 | 4506 | PTPRT | 20 | 40,724,350 |
| 4507 | PTPRT | 20 | 40,745,754 | 4508 | PTPRT | 20 | 40,759,541 |
| 4509 | PTPRT | 20 | 40,759,651 | 4510 | PTPRT | 20 | 40,766,955 |
| 4511 | PTPRT | 20 | 40,781,649 | 4512 | PTPRT | 20 | 40,832,585 |
| 4513 | PTPRT | 20 | 41,380,590 | 4514 | PTPRT | 20 | 41,380,842 |
| 4515 | PTPRT | 20 | 41,381,326 | 4516 | ZSWIM3 | 20 | 43,929,395 |
| 4517 | KCNB1 | 20 | 47,457,043 | 4518 | KCNB1 | 20 | 47,468,865 |
| 4519 | KCNB1 | 20 | 47,543,084 | 4520 | KCNB1 | 20 | 47,550,362 |
| 4521 | KCNB1 | 20 | 47,554,184 | 4522 | PTGIS | 20 | 47,563,735 |
| 4523 | PTGIS | 20 | 47,596,551 | 4524 | SNAI1 | 20 | 48,025,165 |
| 4525 | SNAI1 | 20 | 48,034,331 | 4526 | SNAI1 | 20 | 48,036,030 |
| 4527 | SNAI1 | 20 | 48,038,206 | 4528 | SNAI1 | 20 | 48,051,852 |
| 4529 | CDH4 | 20 | 59,287,591 | 4530 | CDH4 | 20 | 59,359,672 |
| 4531 | CDH4 | 20 | 59,410,397 | 4532 | CDH4 | 20 | 59,411,482 |
| 4533 | CDH4 | 20 | 59,456,145 | 4534 | CDH4 | 20 | 59,516,281 |
| 4535 | CDH4 | 20 | 59,708,754 | 4536 | CDH4 | 20 | 59,854,564 |
| 4537 | CDH4 | 20 | 59,923,377 | 4538 | CDH4 | 20 | 59,936,745 |
| 4539 | CDH4 | 20 | 59,972,047 | 4540 | C21ORF37 | 21 | 17,711,037 |
| 4541 | C2IORF37 | 21 | 17,738,145 | 4542 | NCAM2 | 21 | 20,822,764 |
| 4543 | NCAM2 | 21 | 20,823,179 | 4544 | NCAM2 | 21 | 21,399,247 |
| 4545 | NCAM2 | 21 | 21,668,058 | 4546 | NCAM2 | 21 | 21,717,043 |
| 4547 | NCAM2 | 21 | 21,722,038 | 4548 | NCAM2 | 21 | 21,734,222 |
| 4549 | NCAM2 | 21 | 21,757,884 | 4550 | NCAM2 | 21 | 21,763,517 |
| 4551 | NCAM2 | 21 | 21,791,467 | 4552 | NCAM2 | 21 | 21,849,510 |
| 4553 | MIRN155 | 21 | 24,246,806 | 4554 | MIRN155 | 21 | 24,492,909 |
| 4555 | MIRN155 | 21 | 24,857,632 | 4556 | PCP4 | 21 | 40,147,412 |
| 4557 | PCP4 | 21 | 40,194,909 | 4558 | PCP4 | 21 | 40,196,193 |
| 4559 | PCP4 | 21 | 40,196,614 | 4560 | SLC37A1 | 21 | 42,871,651 |
| 4561 | PDE9A | 21 | 42,977,018 | 4562 | PDE9A | 21 | 42,997,501 |
| 4563 | PDE9A | 21 | 42,998,397 | 4564 | PDE9A | 21 | 43,012,117 |
| 4565 | PDE9A | 21 | 43,061,181 | 4566 | PDE9A | 21 | 43,101,819 |
| 4567 | ARVCF | 22 | 18,342,380 | 4568 | ARVCF | 22 | 18,343,666 |
| 4569 | ARVCF | 22 | 18,358,558 | 4570 | ARVCF | 22 | 18,376,255 |
| 4571 | SGSM1 | 22 | 23,675,537 | 4572 | ASPHD2 | 22 | 25,130,199 |
| 4573 | ASPHD2 | 22 | 25,171,643 | 4574 | HPS4 | 22 | 25,200,502 |
| 4575 | HPS4 | 22 | 25,207,539 | 4576 | HPS4 | 22 | 25,207,981 |
| 4577 | HPS4 | 22 | 25,208,024 | 4578 | ARFGAP3 | 22 | 41,526,978 |
| 4579 | ARFGAP3 | 22 | 41,542,725 | 4580 | ARFGAP3 | 22 | 41,556,543 |
| 4581 | ARFGAP3 | 22 | 41,562,917 | 4582 | ARFGAP3 | 22 | 41,590,371 |
| 4583 | ARFGAP3 | 22 | 41,591,167 | 4584 | PACSIN2 | 22 | 41,617,234 |
| 4585 | PACSIN2 | 22 | 41,619,417 | 4586 | PACSIN2 | 22 | 41,635,805 |
| 4587 | PACSIN2 | 22 | 41,679,576 | 4588 | PACSIN2 | 22 | 41,722,800 |
| 4589 | PACSIN2 | 22 | 41,727,507 | 4590 | PACSIN2 | 22 | 41,765,612 |
| 4591 | TTLL1 | 22 | 41,776,832 | 4592 | TTLL1 | 22 | 41,789,790 |
| 4593 | TTLL1 | 22 | 41,800,470 | 4594 | TTLL1 | 22 | 41,822,583 |
| 4595 | TTLL1 | 22 | 41,822,906 | 4596 | EFCAB6 | 22 | 42,262,997 |
| 4597 | EFCAB6 | 22 | 42,278,436 | 4598 | EFCAB6 | 22 | 42,307,729 |
| 4599 | EFCAB6 | 22 | 42,320,777 | 4600 | EFCAB6 | 22 | 42,341,477 |
| 4601 | EFCAB6 | 22 | 42,354,506 | 4602 | EFCAB6 | 22 | 42,358,549 |
| 4603 | EFCAB6 | 22 | 42,364,511 | 4604 | EFCAB6 | 22 | 42,387,277 |
| 4605 | EFCAB6 | 22 | 42,411,013 | 4606 | EFCAB6 | 22 | 42,414,775 |
| 4607 | EFCAB6 | 22 | 42,426,302 | 4608 | EFCAB6 | 22 | 42,433,349 |
| 4609 | EFCAB6 | 22 | 42,451,208 | 4610 | EFCAB6 | 22 | 42,494,726 |
| 4611 | EFCAB6 | 22 | 42,496,931 | 4612 | EFCAB6 | 22 | 42,500,759 |
| 4613 | EFCAB6 | 22 | 42,521,911 | 4614 | EFCAB6 | 22 | 42,527,300 |
| 4615 | EFCAB6 | 22 | 42,584,422 | 4616 | RIBC2 | 22 | 44,257,735 |
| 4617 | RIBC2 | 22 | 44,258,667 | | | | |

## Claims

1. A method of predicting a human patient's likely response to a treatment for schizophrenia (SZ), the method comprising:
determining the identity of at least one allele of a single nucleotide polymorphism (SNP) in the elongation of very long chain fatty acids family member 5 (ELOVL5) gene selected from the group consisting of: rs9474507, rs974323, rs9357760, rs9370196, rs9367529, rs2562898, rs1429146, rs209500, and rs1429143; and
identifying a human patient having an A allele at rs9474507 as having an increased likelihood of having a quicker time to discontinuation of treatment with risperidone or an increased likelihood of discontinuation of treatment with risperidone due to an adverse event as compared to human patient not having an A allele at rs9474507;
identifying a human patient having a T allele at rs974323 as having an increased likelihood of discontinuation of treatment with risperidone due to an adverse event as compared to a human patient not having a T allele at rs974323;
identifying a human patient having a G allele at rs9357760 as having an increased likelihood of having a greater improvement in symptom burden in response to treatment with ziprasidone, an increased likelihood of having a quicker time to discontinuation of treatment with risperidone due to lack of efficacy, or an increased likelihood of discontinuation of treatment with risperidone due to an adverse event as compared to a human patient not having a G allele at rs9357760;
identifying a human patient having a C allele at rs9370196 as having an increased likelihood of a greater improvement in symptom burden in response to treatment with ziprasidone, an increased likelihood of having a quicker time to discontinuation of treatment with risperidone due to lack of efficacy, or an increased likelihood of discontinuation of treatment with risperidone due to an adverse event as compared to a human patient not having a C allele at rs9370196;
identifying a human patient having a G allele at rs9367529 as having an increased likelihood of discontinuation of treatment with risperidone due to an adverse event as compared to a human patient not having a G allele at rs9367529;
identifying a human patient having an A allele at rs2562898 as having an increased likelihood of having a decreased improvement in symptom burden in response to treatment with quetiapine, or an increased likelihood of having a quicker time to discontinuation of treatment with quetiapine due to lad of efficacy as compared to a human patient not having an A allele at rs2562898;
identifying a human patient having a T allele at rs1429146 as having an increased likelihood of having an increased improvement in symptom burden in response to treatment with ziprasidone, or an increased likelihood of having a quicker time to discontinuation of treatment with risperidone due to lack of efficacy as compared to a human patient not having a T allele at rs 1429146;
identifying a human patient having a T allele at rs209500 as having an increased likelihood of having a decreased improvement in symptom burden in response to treatment with ziprasidone as compared to a human subject not having a T allele at rs209500; and
identifying a human patient having a G allele at rs1429143 as having an increased likelihood of having a quicker time to discontinuation of treatment with risperidone due to lack of efficacy as compared to a human subject not having a G allele at rs1429143.

2. A method of selecting a treatment for schizophrenia (SZ) for administration to a human patient, the method comprising:
determining the identity in the subject of at least one allele of a single nucleotide polymorphism (SNP) in the elongation of very long chain fatty acids family member 5 (ELOVL5) gene selected from the group consisting of: rs9474507, rs974323, rs9357760, rs9370196, rs9367529, rs2562898, rs1429146, rs209500, and rs1429143; and
selecting a treatment not including risperidone for a subject having an A allele at rs9474507, a T allele at rs974323, a G allele at rs9357760, a C allele at rs9370196, a G allele at rs9367529, a T allele at rs1429146, or a G allele at rs1429143;
selecting a treatment including ziprasidone for a subject having a G allele at rs9357760; a C allele at rs9370196, a T allele at rs1429146;
selecting a treatment not including quetiapine for a human patient having an A allele at rs2562898; or
selecting a treatment not including ziprasidone for a human patient having a T allele at rs209500.

3. The method of claim 1 or 2, wherein the method further comprises obtaining a sample containing genomic DNA from the human patient.

4. The method of claim 3, wherein determining the identity of at least one allele comprises contacting the sample with a probe specific for a selected allele of rs9474507, rs974323, rs9357760, rs9370196, rs9367529, rs2562898, rs1429146, rs209500, or rs1429143, and detecting the formation of complexes between the probe and the selected allele, wherein the formation of complexes between the probe and the selected allele indicates the presence of the selected allele in the sample.

5. The method of claim 1 or 2, wherein:
determining the identity of an allele at rs9474507 comprises determining the identity of the nucleotide at position 31 of SEQ ID NO: 1895;
determining the identity of an allele at rs974323 comprises determining the identity of the nucleotide at position 31 of SEQ ID NO: 1890;
determining the identity of an allele at rs9357760 comprises determining the identity of the nucleotide at position 31 of SEQ ID NO: 1893;
determining the identity of an allele at rs9370196 comprises determining the identity of the nucleotide at position 31 of SEQ ID NO: 1894;
determining the identity of an allele at rs9367529 comprises determining the identity of the nucleotide at position 31 of SEQ ID NO: 1897;
determining the identity of an allele at rs2562898 comprises determining the identity of the nucleotide at position 31 of SEQ ID NO: 1888;
determining the identity of an allele at rs 1429146 comprises determining the identity of the nucleotide at position 31 of SEQ ID NO: 1889;
determining the identity of an allele at rs209500 comprises determining the identity of the nucleotide at position 31 of SEQ ID NO: 1892; or
determining the identity of an allele at rs1429143 comprises determining the identity of the nucleotide at position 31 of SEQ ID NO: 1896.

6. The method of claim 1 or 2, wherein the human patient has schizophrenia.

7. The method of claim 1 or 2, wherein the human patient is at risk of developing schizophrenia.

8. The method of claim 1 or 2, wherein the subject has a symptom of schizophrenia.

9. The method of claim 1 or 2, further comprising recording the identity of the at least one allele in a tangible medium.

10. The method of claim 9, wherein the tangible medium comprises a computer-readable disk, a solid state memory device, or an optical storage device.

11. The method of claim 1 or 2, further comprising selecting or excluding a human patient for enrollment in a clinical trial based on the identity of the at least one allele.

12. The method of claim 1 or 2, further comprising stratifying a human patient population for analysis of a clinical trial based on the identity of the at least one allele in the human patients.

## Patentansprüche

1. Ein Verfahren zur Vorhersage einer wahrscheinlichen Antwort eines menschlichen Patienten auf eine Behandlung für Schizophrenie, das Verfahren umfassend:
Bestimmen der Identität von mindestens einem Allel von einem Einzelnukleotid-Polymorphismus (single nucleotide polymorphism - SNP) in dem Gen für Familienmitglied 5 der Verlängerung von sehr langen Fettsäure-Ketten (elongation of very long chain fatty acids family member 5 - ELOVL5), der ausgewählt wird aus der Gruppe bestehend aus: rs9474507, rs974323, rs9357760, rs9370196, rs9367529, rs2562898, rs1429146, rs209500 und rs1429143; und
Identifizieren eines menschlichen Patienten, der ein A Allel bei rs9474507 hat, als einen menschlichen Patienten, der eine erhöhte Wahrscheinlichkeit für eine kürzere Zeit bis zur Einstellung einer Behandlung mit Risperidon,
oder der eine erhöhte Wahrscheinlichkeit für eine Einstellung einer Behandlung mit Risperidon wegen eines unerwünschten Ereignisses hat im Vergleich zu einem menschlichen Patienten, der kein A Allel bei rs9474507 hat;
Identifizieren eines menschlichen Patienten, der ein T Allel bei rs974323 hat, als einen menschlichen Patienten, der eine erhöhte Wahrscheinlichkeit für eine Einstellung einer Behandlung mit Risperidon wegen eines unerwünschten Ereignisses hat im Vergleich zu einem menschlichen Patienten, der kein T Allel bei rs974323 hat;
Identifizieren eines menschlichen Patienten, der ein G Allel bei rs9357760 hat, als einen menschlichen Patienten, der eine erhöhte Wahrscheinlichkeit für eine größere Verbesserung in einer Symptombelastung in Antwort auf eine Behandlung mit Ziprasidon, der eine erhöhte Wahrscheinlichkeit für eine kürzere Zeit bis zur Einstellung einer Behandlung mit Risperidon wegen eines Mangels an Wirksamkeit, oder der eine erhöhte Wahrscheinlichkeit für eine Einstellung einer Behandlung mit Risperidon wegen eines unerwünschten Ereignisses hat im Vergleich zu einem menschlichen Patienten, der kein G Allel bei rs9357760 hat;
Identifizieren eines menschlichen Patienten, der ein C Allel bei rs9370196 hat, als einen menschlichen Patienten, der eine erhöhte Wahrscheinlichkeit für eine größere Verbesserung in einer Symptombelastung in Antwort auf eine Behandlung mit Ziprasidon, der eine erhöhte Wahrscheinlichkeit für eine kürzere Zeit bis zur Einstellung einer Behandlung mit Risperidon wegen eines Mangels an Wirksamkeit, oder der eine erhöhte Wahrscheinlichkeit für eine Einstellung einer Behandlung mit Risperidon wegen eines unerwünschten Ereignisses hat im Vergleich zu einem menschlichen Patienten, der kein C Allel bei rs9370196 hat;
Identifizieren eines menschlichen Patienten, der ein G Allel bei rs9367529 hat, als einen menschlichen Patienten, der eine erhöhte Wahrscheinlichkeit für eine Einstellung einer Behandlung mit Risperidon wegen eines unerwünschten Ereignisses hat im Vergleich zu einem menschlichen Patienten, der kein G Allel bei rs9367529 hat;
Identifizieren eines menschlichen Patienten, der ein A Allel bei rs2562898 hat, als einen menschlichen Patienten, der eine erhöhte Wahrscheinlichkeit für eine verminderte Verbesserung in einer Symptombelastung in Antwort auf eine Behandlung mit Quetiapin, oder der eine erhöhte Wahrscheinlichkeit für eine kürzere Zeit bis zur Einstellung einer Behandlung mit Quetiapin wegen eines Mangels an Wirksamkeit hat im Vergleich zu einem menschlichen Patienten, der kein A Allel bei rs2562898 hat;
Identifizieren eines menschlichen Patienten, der ein T Allel bei rs 1429146 hat, als einen menschlichen Patienten, der eine erhöhte Wahrscheinlichkeit für eine größere Verbesserung in einer Symptombelastung in Antwort auf eine Behandlung mit Ziprasidon, oder der der eine erhöhte Wahrscheinlichkeit für eine kürzere Zeit bis zur Einstellung einer Behandlung mit Risperidon wegen eines Mangels an Wirksamkeit hat im Vergleich zu einem menschlichen Patienten, der kein T Allel bei rs1429146 hat;
Identifizieren eines menschlichen Patienten, der ein T Allel bei rs209500 hat, als einen menschlichen Patienten, der eine erhöhte Wahrscheinlichkeit für eine verminderte Verbesserung in einer Symptombelastung in Antwort auf eine Behandlung mit Ziprasidon hat im Vergleich zu einem menschlichen Patienten, der kein T Allel bei rs209500 hat; und
Identifizieren eines menschlichen Patienten, der ein G Allel bei rs 1429143 hat, als einen menschlichen Patienten, der eine erhöhte Wahrscheinlichkeit für eine kürzere Zeit bis zur Einstellung einer Behandlung mit Risperidon wegen eines Mangels an Wirksamkeit hat im Vergleich zu einem menschlichen Patienten, der kein G Allel bei rs 1429143 hat.

2. Ein Verfahren zum Auswählen einer Behandlung für Schizophrenie (SZ) zur Verabreichung an einen menschlichen Patienten, das Verfahren umfassend:
Bestimmen, in dem Subjekt, der Identität von mindestens einem Allel von einem Einzelnukleotid-Polymorphismus (single nucleotide polymorphism - SNP) in dem Gen für Familienmitglied 5 der Verlängerung von sehr langen Fettsäure-Ketten (elongation of very long chain fatty acids family member 5 - ELOVL5), der ausgewählt wird aus der Gruppe bestehend aus: rs9474507, rs974323, rs9357760, rs9370196, rs9367529, rs2562898, rs1429146, rs209500 und rs1429143; und
Auswählen einer Behandlung, die nicht Risperidon umfasst, für ein Subjekt, das ein A Allel bei rs9474507, ein T Allel bei rs974323, ein G Allel bei rs9357760, ein C Allel bei rs9370196, ein G Allel bei rs9367529, ein T Allel bei rs1429146 oder ein G Allel bei rs1429143 hat;
Auswählen einer Behandlung, die Ziprasidon umfasst, für ein Subjekt, das ein G Allel bei rs9357760, ein C Allel bei rs9370196 oder ein T Allel bei rs1429146 hat;
Auswählen einer Behandlung, die nicht Quetiapin umfasst, für einen menschlichen Patienten, der ein A Allel bei rs2562898 hat;
Auswählen einer Behandlung, die nicht Ziprasidon umfasst, für einen menschlichen Patienten, der ein T Allel bei rs209500 hat.

3. Das Verfahren nach Anspruch 1 oder 2, wobei das Verfahren weiter umfasst Erhalten einer Probe, die genomische DNA von einem menschlichen Patienten enthält.

4. Das Verfahren nach Anspruch 3, wobei Bestimmen der Identität von mindestens einem Allel umfasst Kontaktieren der Probe mit eine Sonde, die spezifisch ist für ein ausgewähltes Allel von rs9474507, rs974323, rs9357760, rs9370196, rs9367529, rs2562898, rs1429146, rs209500 und rs1429143, und Detektieren der Bildung eines Komplexes zwischen der Sonde und dem ausgewählten Allel, wobei die Bildung des Komplexes zwischen der Sonde und dem ausgewählten Allel das Vorhandensein des ausgewählten Allels in der Probe anzeigt.

5. Das Verfahren nach Anspruch 1 oder 2, wobei:
Bestimmen der Identität von einem Allel bei rs9474507 Bestimmen der Identität des Nukleotids bei der Position 31 von SEQ ID NO: 1895 umfasst;
Bestimmen der Identität von einem Allel bei rs974323 Bestimmen der Identität des Nukleotids bei der Position 31 von SEQ ID NO: 1890 umfasst;
Bestimmen der Identität von einem Allel bei rs9357760 Bestimmen der Identität des Nukleotids bei der Position 31 von SEQ ID NO: 1893 umfasst;
Bestimmen der Identität von einem Allel bei rs9370196 Bestimmen der Identität des Nukleotids bei der Position 31 von SEQ ID NO: 1894 umfasst;
Bestimmen der Identität von einem Allel bei rs9367529 Bestimmen der Identität des Nukleotids bei der Position 31 von SEQ ID NO: 1897 umfasst;
Bestimmen der Identität von einem Allel bei rs2562898 Bestimmen der Identität des Nukleotids bei der Position 31 von SEQ ID NO: 1888 umfasst;
Bestimmen der Identität von einem Allel bei rs1429146 Bestimmen der Identität des Nukleotids bei der Position 31 von SEQ ID NO: 1889 umfasst;
Bestimmen der Identität von einem Allel bei rs209500 Bestimmen der Identität des Nukleotids bei der Position 31 von SEQ ID NO: 1892 umfasst; oder
Bestimmen der Identität von einem Allel bei rs1429143 Bestimmen der Identität des Nukleotids bei der Position 31 von SEQ ID NO: 1896 umfasst.

6. Das Verfahren nach Anspruch 1 oder 2, wobei der menschliche Patient Schizophrenie hat.

7. Das Verfahren nach Anspruch 1 oder 2, wobei der menschliche Patient ein Risiko hat, Schizophrenie zu entwickeln.

8. Das Verfahren nach Anspruch 1 oder 2, wobei das Subjekt ein Schizophrenie-Symptom hat.

9. Das Verfahren nach Anspruch 1 oder 2, weiter umfassend Aufnehmen der Identität von mindestens einem Allel auf einem materiellen Medium.

10. Das Verfahren nach Anspruch 9, wobei das materielle Medium eine computer-lesbare Disk, eine Solid-State-Speichervorrichtung oder eine optische Speichervorrichtung umfasst.

11. Das Verfahren nach Anspruch 1 oder 2, weiter umfassend Auswählen oder Ausschließen eines menschlichen Patienten für die Einschreibung in einer klinischen Versuchsreihe basierend auf der Identität von mindestens einem Allel.

12. Das Verfahren nach Anspruch 1 oder 2, weiter umfassend Stratifizieren einer Population von menschlichen Patienten zur Analyse von einer klinischen Versuchsreihe basierend auf der Identität von mindestens einem Allel in den menschlichen Patienten.

## Revendications

1. Procédé de prédiction de la réponse probable d'un patient humain à un traitement pour la schizophrénie (SZ), le procédé comprenant :
la détermination de l'identité d'au moins un allèle d'un polymorphisme de nucléotide simple (SNP) dans le gène du membre 5 de la famille d'allongement d'acides gras à très longue chaîne (ELOVL5) choisi dans le groupe constitué de : rs9474507, rs974323, rs9357760, rs9370196, rs9367529, rs2562898, rs1429146, rs209500, et rs1429143 ; et
l'identification d'un patient humain ayant un allèle A à rs9474507 comme ayant une probabilité accrue d'avoir un temps plus court avant l'interruption du traitement avec la rispéridone ou une probabilité accrue d'interruption du traitement avec la rispéridone due à un événement indésirable par rapport à un patient humain n'ayant pas un allèle A à rs9474507 ;
l'identification d'un patient humain ayant un allèle T à rs974323 comme ayant une probabilité accrue d'interruption du traitement avec la rispéridone due à un événement indésirable par rapport à un patient humain n'ayant pas un allèle T à rs974323 ;
l'identification d'un patient humain ayant un allèle G à rs9357760 comme ayant une probabilité accrue d'avoir une plus forte amélioration de la charge symptomatique en réponse au traitement avec la ziprasidone, une probabilité accrue d'avoir un temps plus court avant l'interruption du traitement avec la rispéridone due à un manque d'efficacité, ou une probabilité accrue d'interruption du traitement avec la rispéridone due à un événement indésirable par rapport à un patient humain n'ayant pas un allèle G à rs9357760 ;
l'identification d'un patient humain ayant un allèle C à rs9370196 comme ayant une probabilité accrue d'une plus grande amélioration de la charge symptomatique en réponse au traitement avec la ziprasidone, une probabilité accrue d'avoir un temps plus court avant l'interruption du traitement avec la rispéridone due à un manque d'efficacité, ou une probabilité accrue d'interruption du traitement avec la rispéridone due à un événement indésirable par rapport à un patient humain n'ayant pas un allèle C à rs9370196 ;
l'identification d'un patient humain ayant un allèle G à rs9367529 comme ayant une probabilité accrue d'interruption du traitement avec la rispéridone due à un événement indésirable par rapport à un patient humain n'ayant pas un allèle G à rs9367529 ;
l'identification d'un patient humain ayant un allèle A à rs2562898 comme ayant une probabilité accrue d'avoir une amélioration réduite de la charge symptomatique en réponse au traitement avec la quétiapine, ou une probabilité accrue d'avoir un temps plus court avant l'interruption du traitement avec la quétiapine en raison d'un manque d'efficacité par rapport à un patient humain n'ayant pas un allèle A à rs2562898 ;
l'identification d'un patient humain ayant un allèle T à rs1429146 comme ayant une probabilité accrue d'avoir une amélioration accrue de la charge symptomatique en réponse au traitement avec la ziprasidone, ou une probabilité accrue d'avoir un temps plus court avant l'interruption du traitement avec la rispéridone en raison d'un manque d'efficacité par rapport à un patient humain n'ayant pas un allèle T à rs1429146 ;
l'identification d'un patient humain ayant un allèle T à rs209500 comme ayant une probabilité accrue d'avoir une amélioration réduite de la charge symptomatique en réponse au traitement avec la ziprasidone par rapport à un sujet humain n'ayant pas un allèle T à rs209500 ; et
l'identification d'un patient humain ayant un allèle G à rs1429143 comme ayant une probabilité accrue d'avoir un temps plus court avant l'interruption du traitement avec la rispéridone en raison d'un manque d'efficacité par rapport à un sujet humain n'ayant pas un allèle G à rs1429143.

2. Procédé de sélection d'un traitement pour la schizophrénie (SZ) pour administration à un patient humain, le procédé comprenant : la détermination de l'identité chez le sujet d'au moins un allèle d'un polymorphisme de nucléotide simple (SNP) dans le gène du membre 5 de la famille d'allongement d'acides gras à très longue chaîne (ELOVL5) choisi dans le groupe constitué de : rs9474507, rs974323, rs9357760, rs9370196, rs9367529, rs2562898, rs1429146, rs209500, et rs1429143 ; et
la sélection d'un traitement ne comprenant pas la rispéridone pour un sujet ayant un allèle A à rs9474507, un allèle T à rs974323, un allèle G à rs9357760, un allèle C à rs9370196, un allèle G à rs9367529, un allèle T à rs1429146, ou un allèle G à rs1429143 ;
la sélection d'un traitement comprenant la ziprasidone pour un sujet ayant un allèle G à rs9357760 ; un allèle C à rs9370196, un allèle T à rs1429146 ;
la sélection d'un traitement ne comprenant pas la quétiapine pour un patient humain ayant un allèle A à rs2562898 ; ou
la sélection d'un traitement ne comprenant pas la ziprasidone pour un patient humain ayant un allèle T à rs209500.

3. Procédé de la revendication 1 ou 2, le procédé comprenant en outre l'obtention d'un échantillon contenant de l'ADN génomique du patient humain.

4. Procédé de la revendication 3, dans lequel la détermination de l'identité d'au moins un allèle comprend la mise en contact de l'échantillon avec une sonde spécifique pour un allèle sélectionné de rs9474507, rs974323, rs9357760, rs9370196, rs9367529, rs2562898, rs1429146, rs209500, ou rs1429143, et la détection de la formation de complexes entre la sonde et l'allèle sélectionné, la formation de complexes entre la sonde et l'allèle sélectionné indique la présence de l'allèle sélectionné dans l'échantillon.

5. Procédé de la revendication 1 ou 2, dans lequel :
la détermination de l'identité d'un allèle à rs9474507 comprend la détermination de l'identité du nucléotide à la position 31 de SEQ ID NO: 1895 ;
la détermination de l'identité d'un allèle à rs974323 comprend la détermination de l'identité du nucléotide à la position 31 de SEQ ID NO: 1890 ;
la détermination de l'identité d'un allèle à rs9357760 comprend la détermination de l'identité du nucléotide à la position 31 de SEQ ID NO: 1893 ;
la détermination de l'identité d'un allèle à rs9370196 comprend la détermination de l'identité du nucléotide à la position 31 de SEQ ID NO: 1894 ;
la détermination de l'identité d'un allèle à rs9367529 comprend la détermination de l'identité du nucléotide à la position 31 de SEQ ID NO: 1897 ;
la détermination de l'identité d'un allèle à rs2562898 comprend la détermination de l'identité du nucléotide à la position 31 de SEQ ID NO: 1888 ;
la détermination de l'identité d'un allèle à rs1429146 comprend la détermination de l'identité du nucléotide à la position 31 de SEQ ID NO: 1889 ;
la détermination de l'identité d'un allèle à rs209500 comprend la détermination de l'identité du nucléotide à la position 31 de SEQ ID NO: 1892 ;
ou la détermination de l'identité d'un allèle à rs1429143 comprend la détermination de l'identité du nucléotide à la position 31 de SEQ ID NO: 1896.

6. Procédé de la revendication 1 ou 2, dans lequel le patient humain est atteint de schizophrénie.

7. Procédé de la revendication 1 ou 2, dans lequel le patient humain présente un risque de développer la schizophrénie.

8. Procédé de la revendication 1 ou 2, dans lequel le sujet à un symptôme de schizophrénie.

9. Procédé de la revendication 1 ou 2, comprenant en outre l'enregistrement de l'identité de l'au moins un allèle dans un support matériel.

10. Procédé de la revendication 9, dans lequel le support matériel comprend un disque lisible par ordinateur, un dispositif de mémoire à semi-conducteur, ou un dispositif de stockage optique.

11. Procédé de la revendication 1 ou 2, comprenant en outre la sélection ou l'exclusion d'un patient humain pour recrutement dans un essai clinique sur la base de l'identité de l'au moins un allèle.

12. Procédé de la revendication 1 ou 2, comprenant en outre la stratification d'une population de patients humains pour l'analyse d'un essai clinique sur la base de l'identité de l'au moins un allèle chez les patients humains.
